(19) Europäisches Patentamt
European Patent Office
Office européen des brevets

(11) EP 4 653 016 A1

(12) **EUROPEAN PATENT APPLICATION**
published in accordance with Art. 153(4) EPC

(43) Date of publication:
**26.11.2025 Bulletin 2025/48**

(21) Application number: **24744298.1**

(22) Date of filing: **17.01.2024**

(51) International Patent Classification (IPC):
***A61K 47/68*** *(2017.01)* ***C07K 16/28*** *(2006.01)*
***C07D 307/20*** *(2006.01)* ***A61P 35/00*** *(2006.01)*

(52) Cooperative Patent Classification (CPC):
**A61K 31/357; A61K 31/365; A61K 31/381; A61K 31/4025; A61K 31/438; A61K 31/4433; A61K 31/453; A61K 47/68; A61P 35/00; C07D 307/20; C07K 16/28**

(86) International application number:
**PCT/CN2024/072749**

(87) International publication number:
**WO 2024/153123 (25.07.2024 Gazette 2024/30)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **17.01.2023 CN 202310082754**
**18.05.2023 CN 202310563948**
**11.01.2024 CN 202410045426**

(71) Applicant: **SystImmune, Inc.**
**Redmond WA 98052 (US)**

(72) Inventors:
- **ZHU, Yi**
**Chengdu, Sichuan 611130 (CN)**
- **WAN, Weili**
**Chengdu, Sichuan 611130 (CN)**
- **LAI, Weirong**
**Chengdu, Sichuan 611130 (CN)**
- **ZHU, Guili**
**Chengdu, Sichuan 611130 (CN)**
- **XUE, Fanglin**
**Chengdu, Sichuan 611130 (CN)**

(74) Representative: **Mathys & Squire**
**The Shard**
**32 London Bridge Street**
**London SE1 9SG (GB)**

Remarks:
The complete document including Reference Table(s) and the Sequence Listing(s) can be downloaded from the EPO website

(54) **CONJUGATE OF ERIBULIN DRUG**

(57) A conjugate of the Eribulin drug or a pharmaceutically acceptable salt or solvate thereof, and a preparation method therefor and the use thereof; a linker-drug conjugate or an isomer, mesomer, racemate, or enantiomer thereof, or a mixture thereof, or a pharmaceutically acceptable salt or solvate thereof, and a preparation method therefor and the use thereof; a cytotoxin or an isomer, mesomer, racemate, or an enantiomer thereof, or a mixture thereof, or a pharmaceutically acceptable salt or solvate thereof, and a preparation method therefor and the use thereof.

EP 4 653 016 A1

## Description

### CROSS-REFERENCE TO RELATED APPLICATIONS

The present disclosure is based on and claims priority to CN patent application No. 202310082754.5, filed on January 17, 2023, CN patent application No. 202310563948.7, filed on May 18, 2023, and CN patent application No. 2024100454262, filed on January 11, 2024. The entire content of each of these applications is hereby incorporated by reference.

### TECHNICAL FIELD

The present invention relates to the technical field of medicine, in particular to a ligand-drug conjugate, a linker-drug compound, and preparation methods and uses thereof.

### BACKGROUND

As a specific treatment that can target cancer cells, the development of ADCs (antibody-drug conjugates) has made significant progress since 2000. Currently, there are 15 ADC drugs on the market around the world, namely Mylotarg, Besponsa, Kadcyla, Polivy, Lumoxiti, Enhertu, Adcetris, Padcev, Tivdak, Blenrep, Trodelvy, Akalux, Zynlonta, Disitamab Vedotin and Elahere. The treatment fields are mainly focused on hematological tumors and solid tumors. An ADC consists of three key components: a monoclonal antibody or an antibody fragment that selectively binds to an antigen on tumor cell surface, a cleavable and non-cleavable linker, and a small molecule cytotoxin. The ADC makes full use of the specificity of the antibody binding to the tumor cell antigen and the high efficiency of small molecule drug.

Microtubules are hollow fiber filaments composed of $\alpha$-tubulin and $\beta$-tubulin. They are one of the components of the cytoskeleton and play an important role in cell signal transduction, intracellular migration and transport, and cell shape maintenance. Moreover, during mitosis, microtubule rearranges to form spindles, which are crucial for the movement and separation of sister chromatids. Most of the biological functions of microtubule in cells are regulated by polymerization kinetics, and interfering with the dynamic behavior of microtubule can have a significant impact on spindle formation and cell division. Because cancer cells divide rapidly, they are more sensitive than normal cells to drugs that affect the normal function of tubulin. Currently, important microtubule inhibitors used as ADC payloads include Auristatin and Eribulin, which are derived from marine species, and Maytansinoids derived from plants.

Eribulin is a simplified synthetic derivative of macrocyclic compound Halichondrin B. In November 2010, Eribulin (methanesulfonate) was first approved by the US FDA for clinical treatment of metastatic breast cancer patients who have received at least two chemotherapy regimens (including anthracycline and taxane chemotherapy drugs). Its trade name is Halaven™, developed and marketed by Eisai. Eribulin can selectively bind to the $\beta$-tubulin (+) end to inhibit the elongation of microtubules. In addition to directly killing cancer cells, it can also transform the tumor microenvironment and serve as a small molecule toxin to enhance the "bystander effect" of the ADC. The most common adverse reactions of Eribulin are fatigue, neutropenia, hair loss, peripheral neuropathy, nausea, constipation, etc. At present, there are few studies on ADCs with Eribulin as the warhead, and all of them have the linker introduced on the amino group at C35 of Eribulin. There are no instances of the linker being introduced on the hydroxyl group at C34. Moreover, there is no research on using the precursor of Eribulin having dihydroxyl groups as the warhead.

To sum up, although Eribulin can be used clinically as a single drug, designing a stable ADC molecule containing Eribulin or its derivatives for targeted delivery to tumor cells can make the treatment safer and more effective and better meet clinical needs.

### SUMMARY OF THE INVENTION

Based on a comprehensive understanding of ADC drugs, the inventors of the application creatively designed and synthesized a series of ADC molecules containing Eribulin or its derivatives. Through experiments, it was found that the designed ADC molecules exhibited good anti-tumor activity.

In a first aspect, the present application relates to a ligand-drug conjugate of formula I or a pharmaceutically acceptable salt or solvate thereof,

$$\text{Ab-(L-D)}_n \qquad \text{Formula I}$$

wherein,

Ab is a ligand unit;

L is a linker for covalently connecting Ab to D;
n is selected from integers or decimals between 1 and 40;
-D is as shown in formula II or formula III:

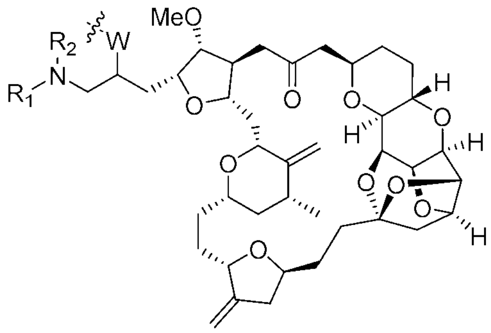

Formula II

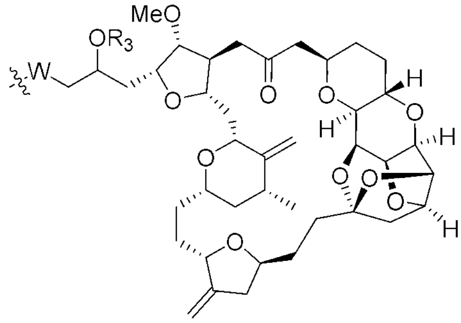

Formula III

wherein W is an oxygen atom or a sulfur atom,
$R_1$ and $R_2$ are the same or different, and are each independently selected from: hydrogen atom, alkyl, alkoxy, alkenyl, cycloalkyl, aryl, heteroaryl, heterocyclyl, $-C(O)-Q_1-Q_2$ and $-SO_2-Q_1-Q_2$, wherein $Q_1$ is selected from the group consisting of O, N, S atoms and a chemical bond, $Q_2$ is selected from the group consisting of alkyl, cycloalkyl, heterocyclyl, spirocyclyl, bridged ring, alkenyl, aryl and heteroaryl; optionally, the alkyl, alkoxy, alkenyl, cycloalkyl, heterocyclyl, spirocyclyl, bridged ring, aryl and heteroaryl are each independently substituted by one or more substituents selected from the group consisting of hydrogen atom, alkyl, alkoxy, halogen, deuterium, amino, cyano, hydroxy, mercapto, azido, nitro, carboxyl, acyl, carbonyl, hydroxyalkyl, cycloalkyl, heterocyclyl, aryl and heteroaryl; or $R_1$ and $R_2$, together with a nitrogen atom connected thereto, form a 3- to 8-membered heterocyclyl; optionally, the heterocyclyl is substituted by one or more substituents selected from the group consisting of hydrogen atom, alkyl, alkoxy, halogen, deuterium, amino, cyano, hydroxy, mercapto, azido, nitro, carboxyl, acyl, carbonyl, hydroxyalkyl, cycloalkyl, heterocyclyl, aryl and heteroaryl; $R_3$ is selected from the group consisting of hydrogen atom, alkyl, acyl, sulfonyl, cycloalkyl, heterocyclyl, aryl and heteroaryl; optionally, the alkyl, acyl, sulfonyl, cycloalkyl, heterocyclyl, aryl and heteroaryl are each independently substituted by one or more substituents selected from the group consisting of hydrogen atom, alkyl, alkoxy, halogen, deuterium, amino, cyano, hydroxy, mercapto, azido, nitro, carboxyl, acyl, carbonyl, hydroxyalkyl, cycloalkyl, heterocyclyl, aryl and heteroaryl.

In some embodiments, L has a structure shown in formula IV:

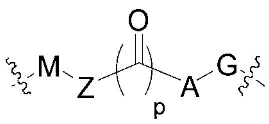

Formula IV

wherein,

M is a linking unit covalently connected to Ab;
Z is selected from the group consisting of -C1-C10 alkylene-, -C3-C8 carbocycle-, - arylene-, -3- to 8-membered heterocyclyl-, $-(CH_2CH_2O)_r-$, sulfonyl, amide, a chemical bond,

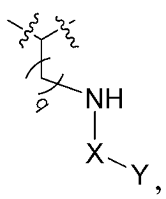

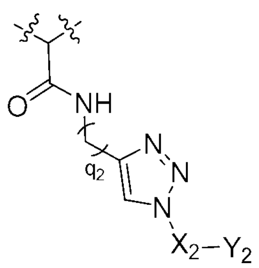

and any combinations thereof, wherein $X_1$ and $X_2$ are selected from the group consisting of -C1-C10 alkylene-, -C3-C8 carbocycle-, -arylene-, -3- to 8-membered heterocyclyl-, $-(CH_2CH_2O)_r-$, $-NR_4-$, carbonyl and any combinations thereof, wherein $R_4$ is selected from the group consisting of hydrogen atom, deuterium atom, alkyl and substituted alkyl; optionally, the -C1-C10 alkylene-, -C3-C8 carbocycle- and -3- to 8-membered heterocyclyl- are each independently substituted with one or more substituents selected from the group consisting of hydrogen atom, deuterium atom, halogen, hydroxyl, cyano, nitro, amino, alkyl, heteroalkyl, substituted alkyl, alkoxy, carboxy and cycloalkyl; each heterocyclyl independently contains 1 to 3 atoms selected from the group consisting of N, O and S; $Y_1$ is a hydrophilic structure selected from the group consisting of carboxyl, phosphoric acid, polyphosphoric acid, phosphorous acid, sulfonic acid, sulfinic acid, polyethylene glycol (PEG), and any combinations thereof; $Y_2$ is selected from the following structures:

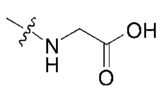 , 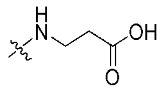 , 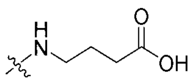 , 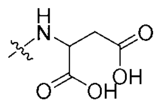 , 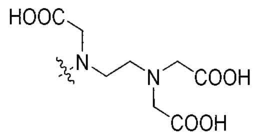 ,

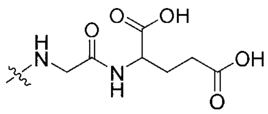 , 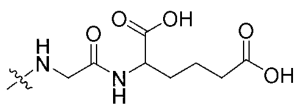 , 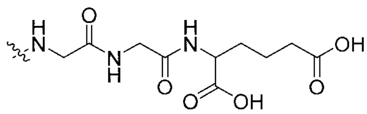 , 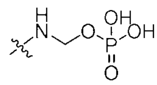 ,

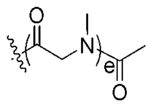 , 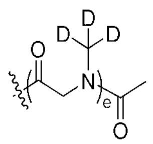 and 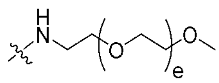 ;

r is selected from integers between 1 and 10; $q_1$ and $q_2$ are selected from integers between 1 and 8; e is selected from integers between 1 and 20;
A is a peptide residue consisting of 2 to 7 amino acids;
G is a spacer unit connected to D; and
p is 0 or1.

In some embodiments, a wavy line on the left in

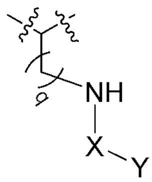 or 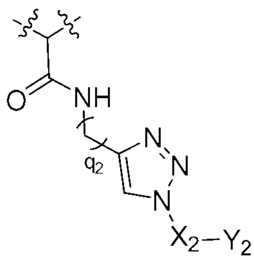

indicates connecting to a linking site on M, and a wavy line on the right indicates connecting to a carbonyl.

In some embodiments, the linking unit M has a succinimide structure as represented by formula a, or a structure as represented by formula b or formula c in which the ring in succinimide group is in open form:

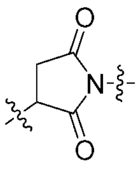

Formula a

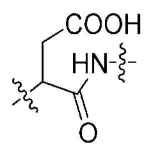

Formula b

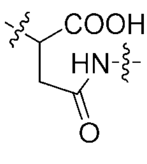

Formula c

In formula a, formula b or formula c, a wavy line on the left indicates connecting to a linking site on Ab, and a wavy

line on the right indicates connecting to a linking site on Z.

In some embodiments, A is a polypeptide residue composed of 2 to 7 amino acids selected from the group consisting of phenylalanine, glycine, valine, lysine, citrulline, serine, glutamic acid, aspartic acid, and cysteic acid.

In some embodiments, the spacer unit G is selected from the group consisting of structures represented by formulas Va, Vb, Vc and Vd and any combinations thereof:

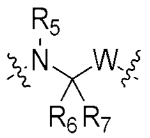

Formula Va

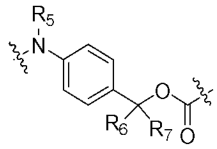

Formula Vb

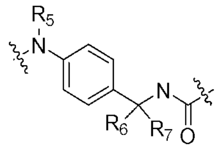

Formula Vc

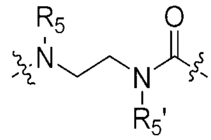

Formula Vd

wherein a wavy line on the left represents a linking site between a nitrogen atom and peptide residue A, a wavy line on the right represents a linking site between an oxygen atom and drug D, and W is an oxygen atom or a sulfur atom and is a joint group between the drug D and the spacer unit G; and

$R_5$, $R_5$', $R_6$ and $R_7$ are each independently selected from the group consisting of hydrogen atom, deuterium atom, alkyl and substituted alkyl.

In some embodiments, the ligand-drug conjugate or the pharmaceutically acceptable salt or solvate thereof of the present application has a structure represented by formula VI or formula VII:

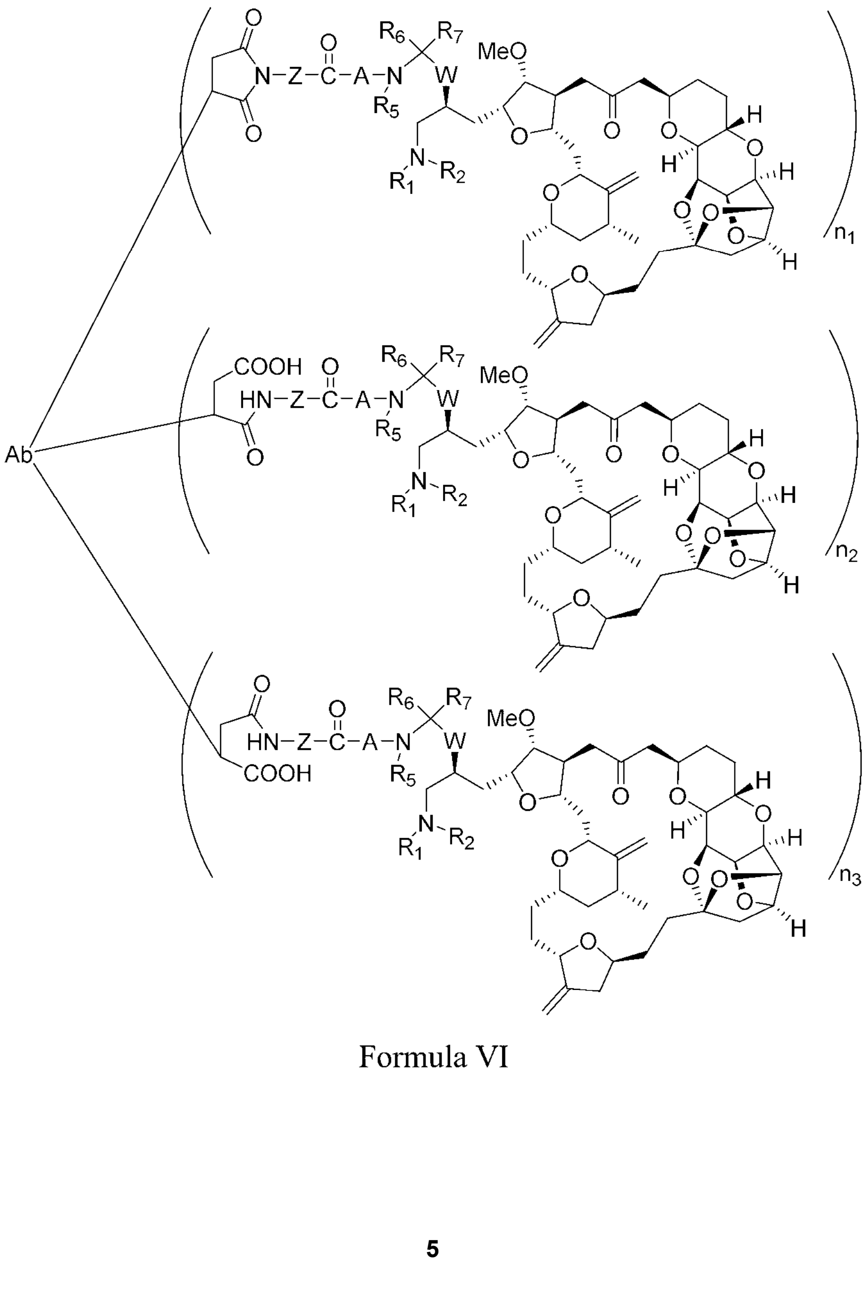

Formula VI

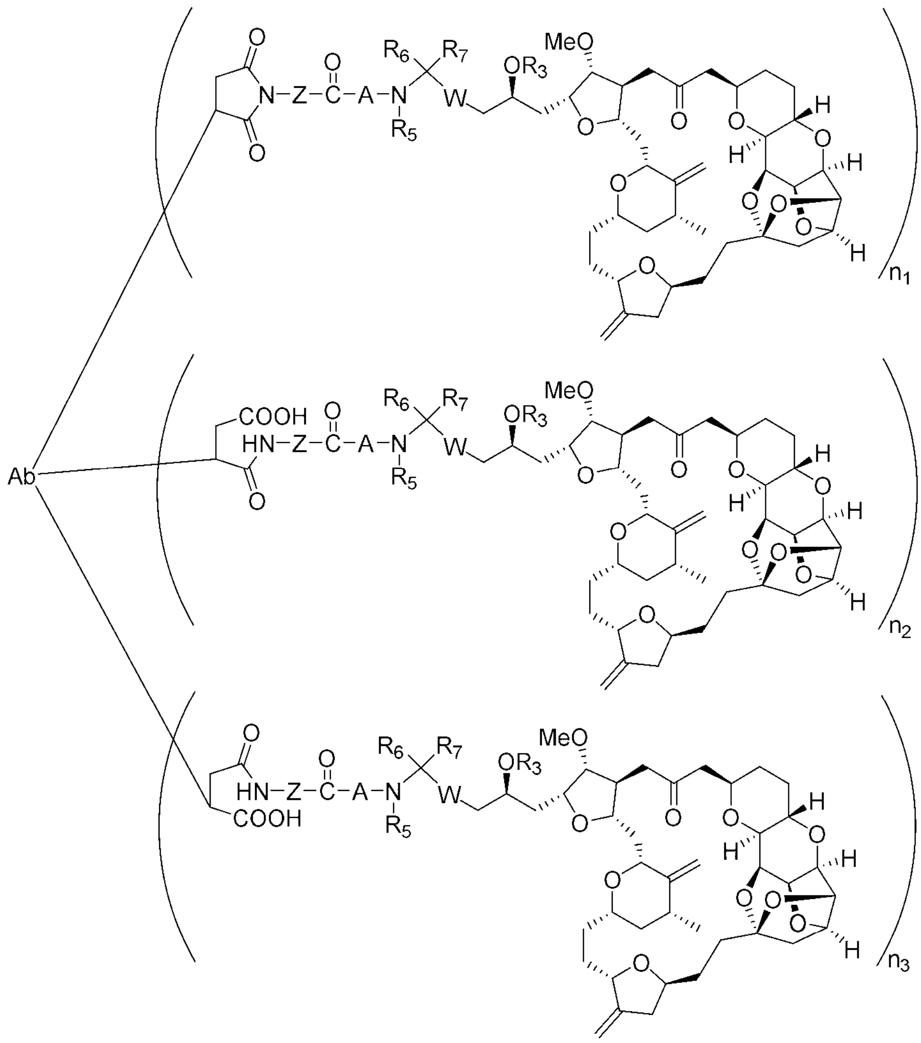

Formula VII

wherein, the Ab, Z, A, $R_5$, $R_6$, $R_7$, $R_3$ and W are as defined above; $n_1$, $n_2$ and $n_3$ are independently selected from integers or decimals between 0 and 40 (e.g. 0-5, 5-10, 10-15, 15-20, 20-25, 25-30, 30-35 or 35-40), $n_1$, $n_2$ and $n_3$ are not 0 at the same time, and $n_1+n_2+n_3 \leq 40$.

In some embodiments, Z is selected from the group consisting of -C1-C10 alkylene-, - $(CH_2CH_2O)_r$-, amide,

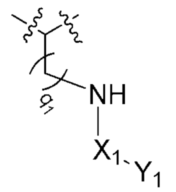 , 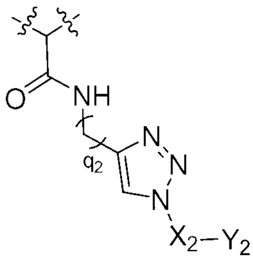 ,

and any combinations thereof;

In some embodiments, Z is -C2-alkylene or -C5-alkylene;

In some embodiments, Z is

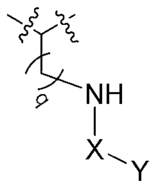,

wherein, $q_1$ is selected from integers between 1 and 8 (e.g. 1, 2, 3, 4, 5, 6, 7 and 8).

In some embodiments, $q_1$ is 1.

In some embodiments, $q_1$ is 1, and $-NH-X_1-Y_1$ composes the hydrophilic structural unit $Ac_1$, the $Ac_1$ is selected, without limitation, from the group consisting of (D/L) alanine, (D/L) leucine, (D/L) isoleucine, (D/L) valine, (D/L) phenylalanine, (D/L) proline, (D/L) tryptophan, (D/L) serine, (D/L) tyrosine, (D/L) cysteine, (D/L) cystine, (D/L) arginine, (D/L) histidine, (D/L) methionine, (D/L) asparagine, (D/L) glutamine, (D/L) threonine, (D/L) aspartic acid, (D/L) glutamic acid, natural or unnatural amino acid derivatives and the following structures:

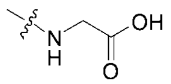, 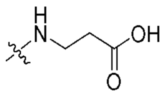, 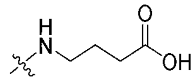, 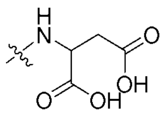, 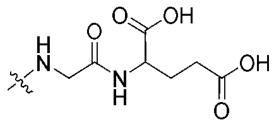,

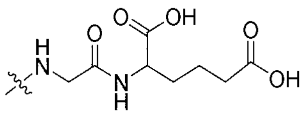, 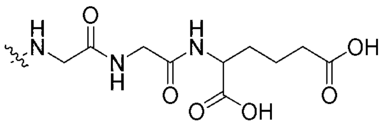, 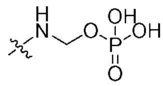, 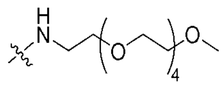

and

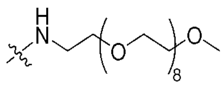.

In some embodiments, $q_1$ is 1, and $-NH-X_1-Y_1$ composes the hydrophilic structural unit $Ac_1$, the $Ac_1$ is selected from the following structures:

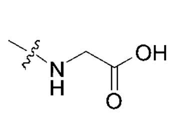, 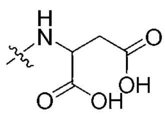,

and

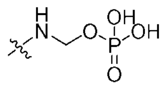.

In some embodiments, Z is

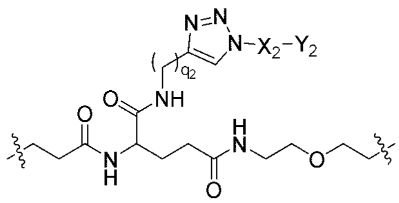,

wherein, $q_2$ is selected from integers between 1 and 8 (e.g. 1, 2, 3, 4, 5, 6, 7 and 8); preferably, $q_2$ is 1; preferably, $X_2$ is -(C1-C10 alkylene)-$(CH_2CH_2O)$r-(C=O)- or -(C1-C10 alkylene)-$(CH_2CH_2O)_r$-$NR_4$-.

In some embodiments, $q_2$ is 1, and $-X_2-Y_2$ composes the hydrophilic structural unit $Ac_2$, the $Ac_2$ is selected, without limitation, from

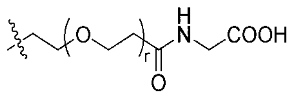, 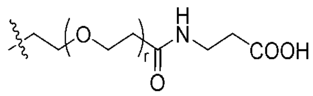,

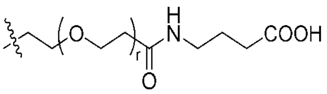, 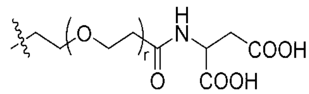, 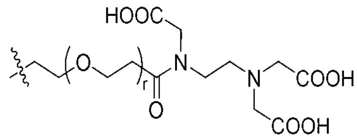,

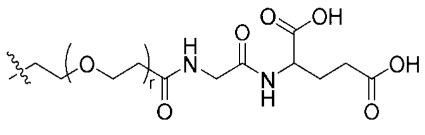, 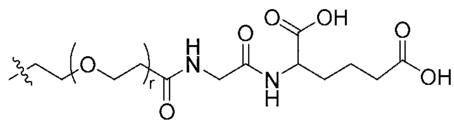,

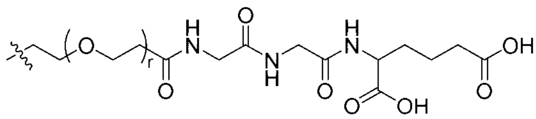, 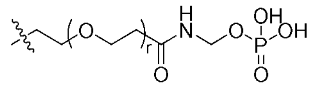, 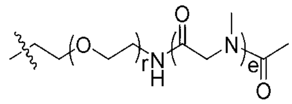

or

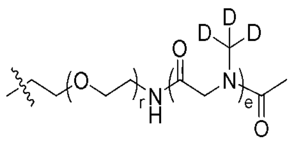;

r is selected from integers between 1 and 10 (e.g. 1, 2, 3, 4, 5, 6, 7, 8, 9 and 10); e is selected from integers between 1 and 20 (e.g. 1, 2, 3, 4, 5, 6, 7, 8, 9, 10, 11, 12, 13, 14, 15, 16, 17, 18, 19 and 20).

In some embodiments, $q_2$ is 1, and $-X_2-Y_2$ composes the hydrophilic structural unit $Ac_2$, the $Ac_2$ is selected from the following structures:

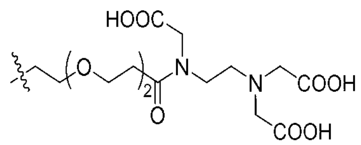,

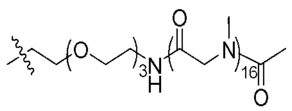 and 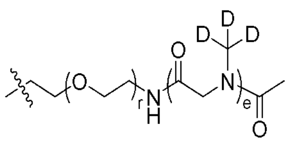.

In some embodiments, A is a polypeptide residue composed of 2 to 5 amino acids selected from the group consisting of phenylalanine, glycine, valine, lysine, citrulline, serine, glutamic acid, aspartic acid, and cysteic acid.

In some embodiments, A is a peptide residue formed by 2 to 4 amino acids selected from phenylalanine and glycine.

In some embodiments, A is a tetrapeptide residue composed of glycine (G), glycine (G), phenylalanine (F), and glycine (G).

In some embodiments, A is -GGFG-.

In some embodiments, W is an oxygen atom or a sulfur atom, $R_1$ and $R_2$ are the same or different, and are each independently selected from the group consisting of: hydrogen atom, alkyl, alkoxy, alkenyl, cycloalkyl, $-C(O)-Q_1-Q_2$ and

$-SO_2-Q_1-Q_2$, wherein $Q_1$ is selected from the group consisting of O and a chemical bond, and $Q_2$ is selected from the group consisting of alkyl, heterocyclyl, alkenyl, aryl, heteroaryl and cycloalkyl; optionally, the alkyl, heterocyclyl, alkenyl, aryl, heteroaryl and cycloalkyl are each independently substituted with one or more substituents selected from the group consisting of hydrogen atom, alkyl, hydroxyalkyl, halogen, deuterium, mercapto, amino, hydroxy, cycloalkyl and heterocyclyl; or $R_1$ and $R_2$ together with a nitrogen atom connected thereto, form a 3- to 6-membered heterocyclyl, and optionally the heterocyclyl is substituted with one or more substituents selected from the group consisting of hydrogen atom, alkyl, alkoxy, halogen, deuterium, amino, aryl, and hydroxyl; $R_3$ is selected from the group consisting of hydrogen atom and alkyl, and optionally the alkyl is substituted with one or more substituents selected from the group consisting of hydrogen atom, alkyl, alkoxy, halogen, deuterium, amino, hydroxyl, cycloalkyl and heterocyclyl.

In some embodiments, W is an oxygen atom, $R_1$ and $R_2$ are the same or different, and are each independently selected from the group consisting of: hydrogen atom, alkyl, alkenyl, haloalkyl, $-C(O)-Q_1-Q_2$ and $-SO_2-Q_1-Q_2$, wherein $Q_1$ is selected from the group consisting of O and a chemical bond, $Q_2$ is selected from the group consisting of alkyl, alkenyl, aryl and 3- to 8-membered cycloalkyl, and optionally, the alkyl, alkenyl, aryl and cycloalkyl are each independently substituted with one or more substituents selected from the group consisting of hydrogen atom, alkyl, hydroxyalkyl, halogen, aryl, and hydroxyl; or $R_1$ and $R_2$ together with a nitrogen atom connected thereto, form a 3- to 6-membered heterocyclyl containing one to two nitrogen atoms and optionally one oxygen atom; optionally, the 3- to 6-membered heterocyclyl is substituted with one or more substituents selected from the group consisting of hydrogen atom, alkyl, and haloalkyl; $R_3$ is selected from the group consisting of hydrogen atom and alkyl, and the alkyl is substituted with one or more substituents selected from the group consisting of hydrogen atom, alkyl, halogen, deuterium, amino and hydroxyl.

In some embodiments, W is an oxygen atom, $R_1$ and $R_2$ are the same or different, and are each independently selected from the group consisting of: hydrogen atom, $C_1$-$C_4$ alkyl (such as methyl, ethyl), $C_2$-$C_4$ alkenyl (such as allyl), $C_1$-$C_4$ haloalkyl (such as difluoroethyl) and the following structures:

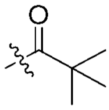, 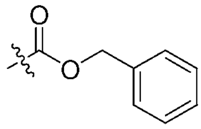, 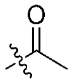, 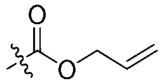, 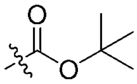, 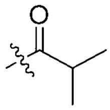, 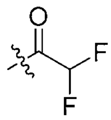, 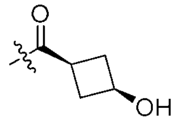,

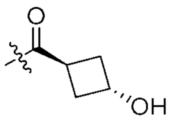, 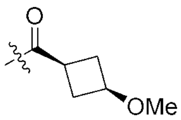, 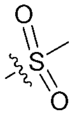, 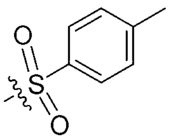, 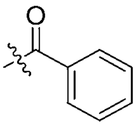, 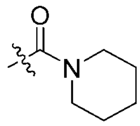, 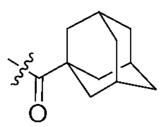,

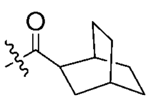, 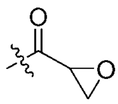, 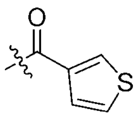, 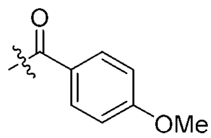, 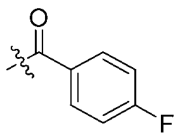, 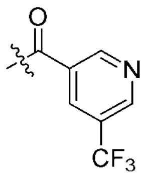,

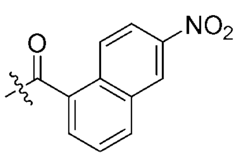, 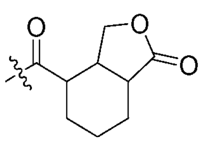, 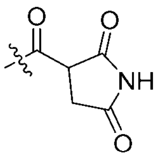, 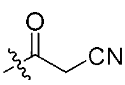, 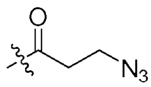, 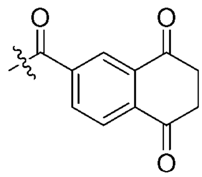,

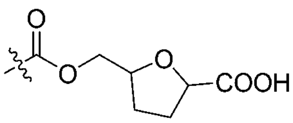, 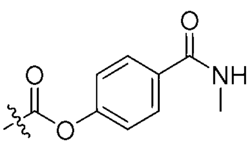, 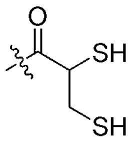, 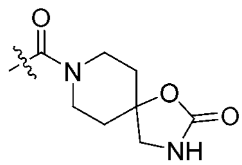;

or $R_1$ and $R_2$, together with a nitrogen atom connected thereto, form a 3- to 6-membered heterocyclyl containing one to two nitrogen atoms and optionally one oxygen atom (such as azyclopropyl, piperidinyl, piperazinyl, morpholinyl); optionally,

the 3- to 6-membered heterocyclyl is substituted with one or more substituents selected from the group consisting of hydrogen atom, $C_1$-$C_4$ alkyl (e.g., methyl), and $C_1$-$C_4$ haloalkyl (e.g., trifluoromethyl); and $R_3$ is selected from the group consisting of hydrogen atom and methyl.

In some embodiments, $R_5$, $R_6$ and $R_7$ are each independently selected from the group consisting of hydrogen atom, deuterium atom, alkyl, haloalkyl, deuterated alkyl and hydroxyalkyl.

In some embodiments, $R_5$, $R_6$ and $R_7$ are simultaneously hydrogen atoms.

In some embodiments, the ligand-drug conjugate or the pharmaceutically acceptable salt or solvate thereof of the present application has a structure represented by formula VIa or formula VIIa:

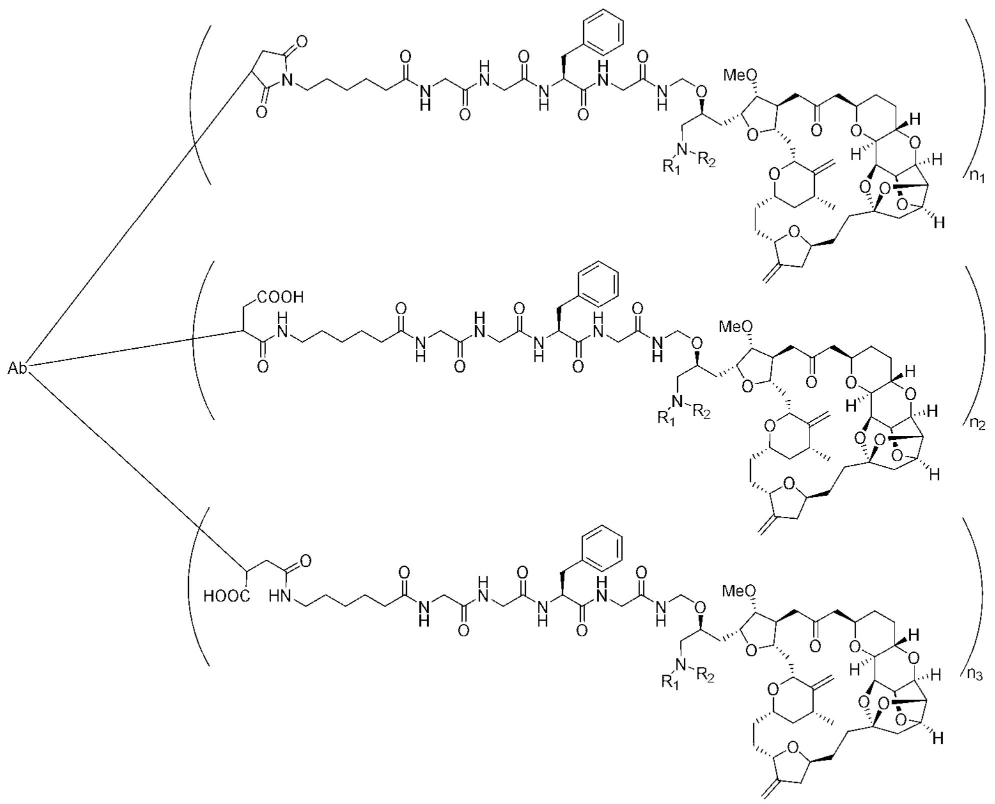

Formula VIa

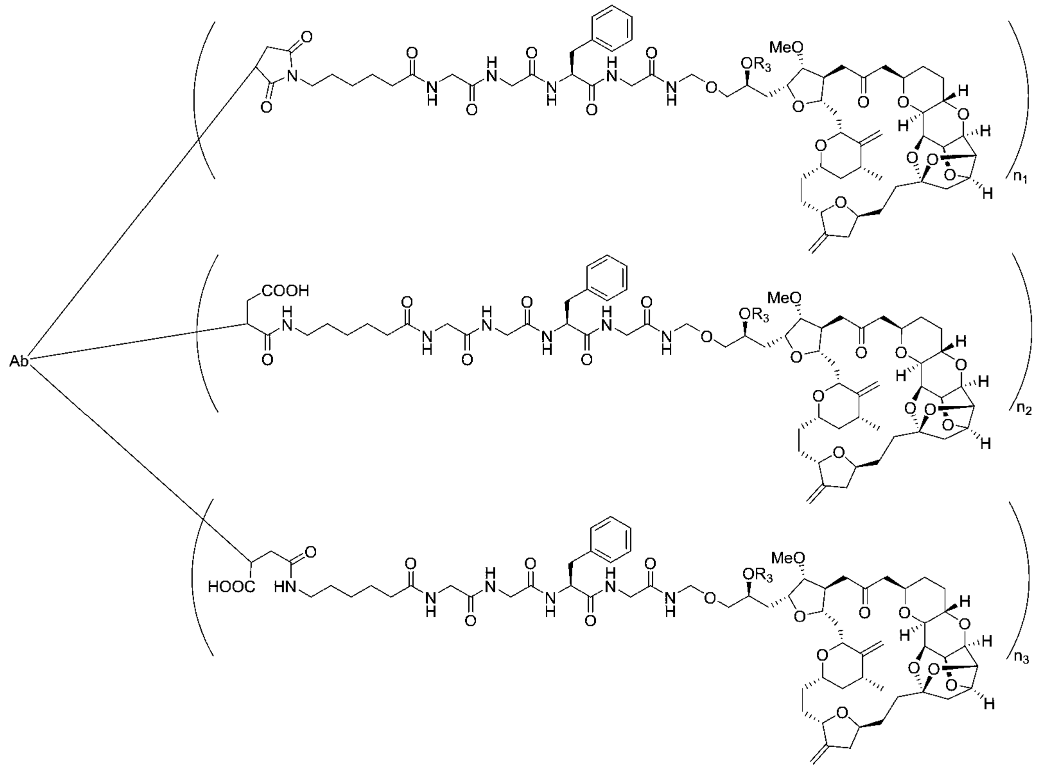

Formula VIIa

wherein, the $R_1$, $R_2$, $R_3$, $n_1$, $n_2$ and $n_3$ are as defined above.

In some embodiments, the ligand-drug conjugate or the pharmaceutically acceptable salt or solvate thereof of the present application has a structure represented by formula VIb or formula VIIb:

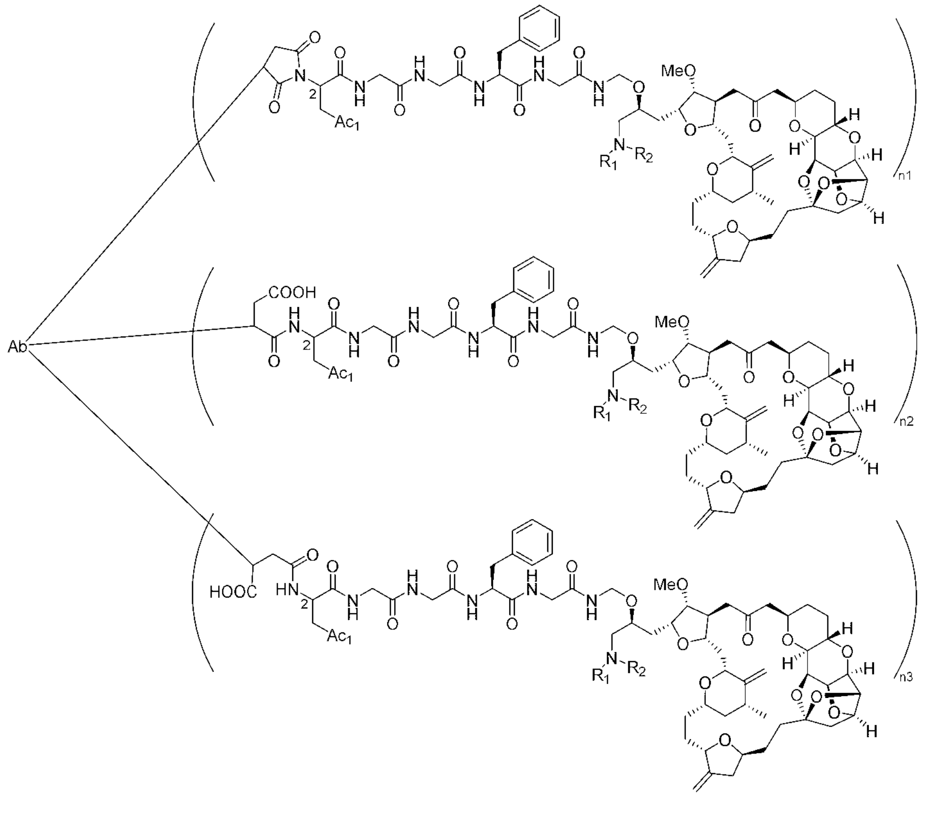

Formula VIb

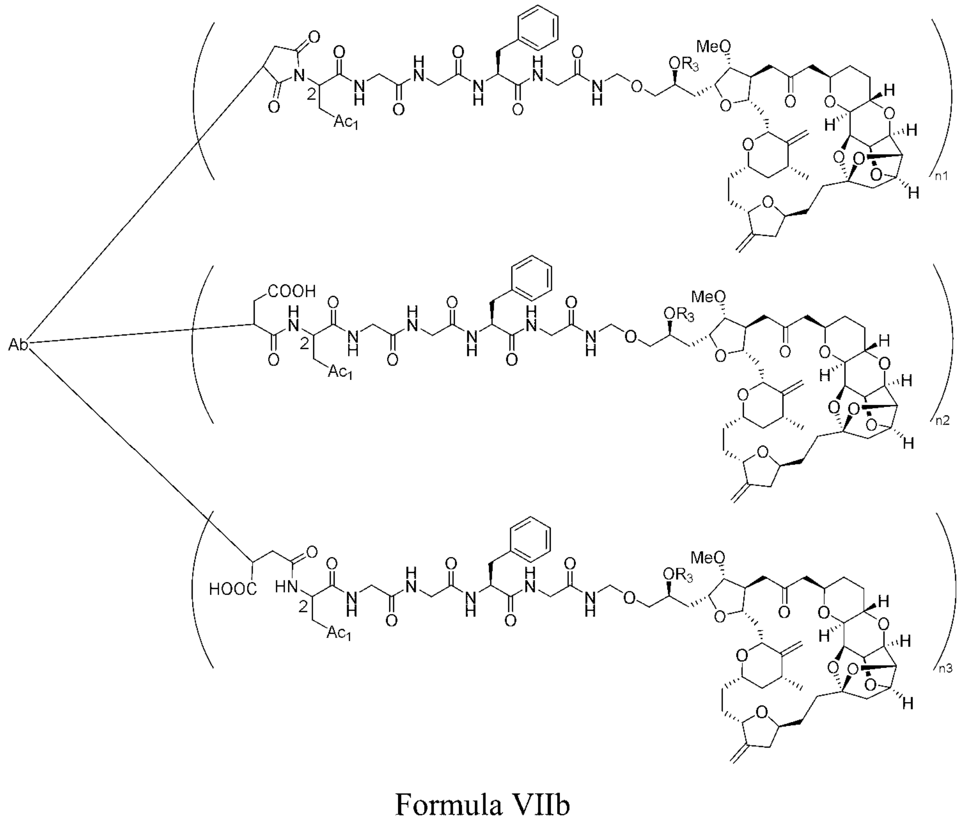

Formula VIIb

wherein, the $R_1$, $R_2$, $R_3$, $n_1$, $n_2$ and $n_3$ are as defined above;

$Ac_1$ is a hydrophilic structural unit having the structure shown in formula d:

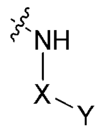

Formula d

the $X_1$ and $Y_1$ are as defined above.

In some embodiments, the $Ac_1$ is selected, without limitation, from the group consisting of (D/L) alanine, (D/L) leucine, (D/L) isoleucine, (D/L) valine, (D/L) phenylalanine, (D/L) proline, (D/L) tryptophan, (D/L) serine, (D/L) tyrosine, (D/L) cysteine, (D/L) cystine, (D/L) arginine, (D/L) histidine, (D/L) methionine, (D/L) asparagine, (D/L) glutamine, (D/L) threonine, (D/L) aspartic acid, (D/L) glutamic acid, natural or unnatural amino acid derivatives and the following structures:

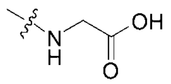 , 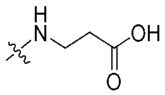 , 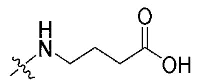 , 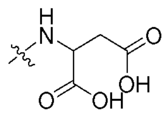 , 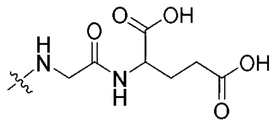 ,

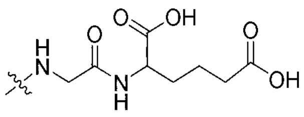 , 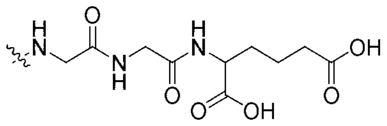 , 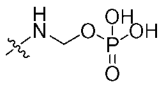 , 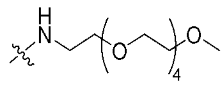

and

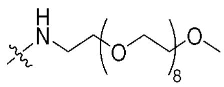 .

In some embodiments, $Ac_1$ is selected from the following structures:

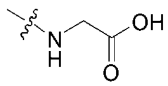 ,

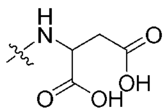 , and 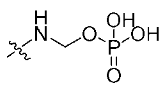 .

In some embodiments, the ligand-drug conjugate or the pharmaceutically acceptable salt or solvate thereof of the present application has a structure represented by formula VIc:

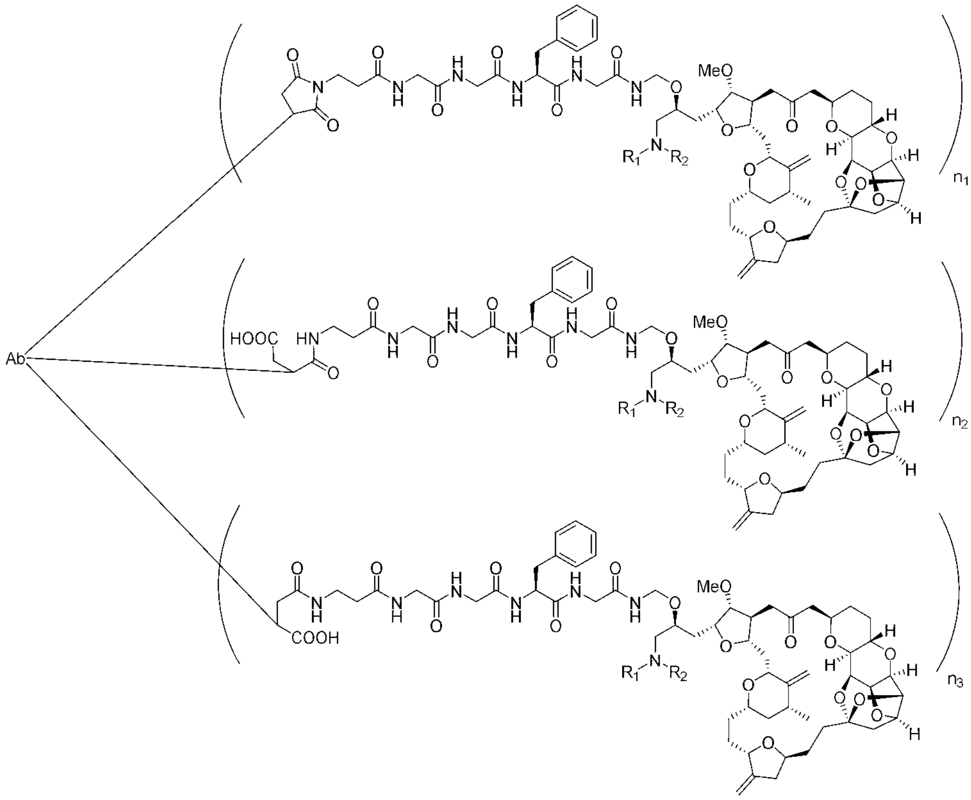

Formula VIc

wherein, the $R_1$, $R_2$, $n_1$, $n_2$ and $n_3$ are as defined above.

In some embodiments, the ligand-drug conjugate or the pharmaceutically acceptable salt or solvate thereof of the present application has a structure represented by formula VId:

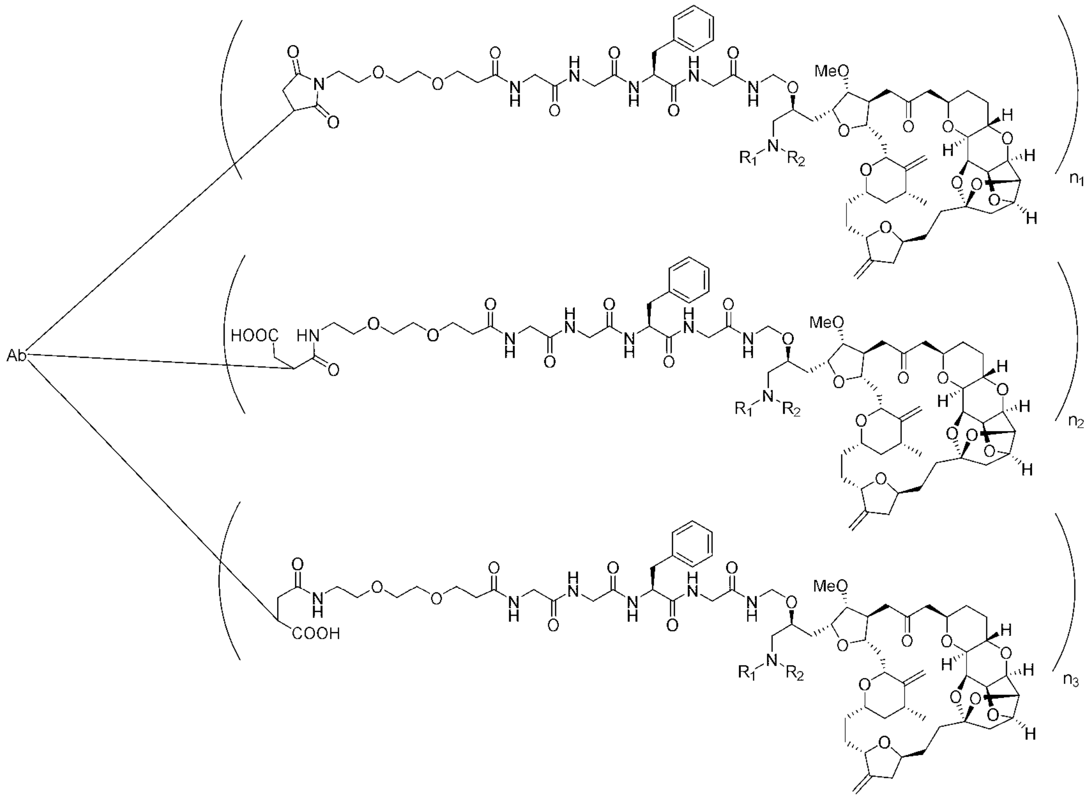

Formula VId

wherein the $R_1$, $R_2$, $n_1$, $n_2$ and $n_3$ are as defined above.

In some embodiments, the ligand-drug conjugate or the pharmaceutically acceptable salt or solvate thereof of the present application has a structure represented by formula VIe:

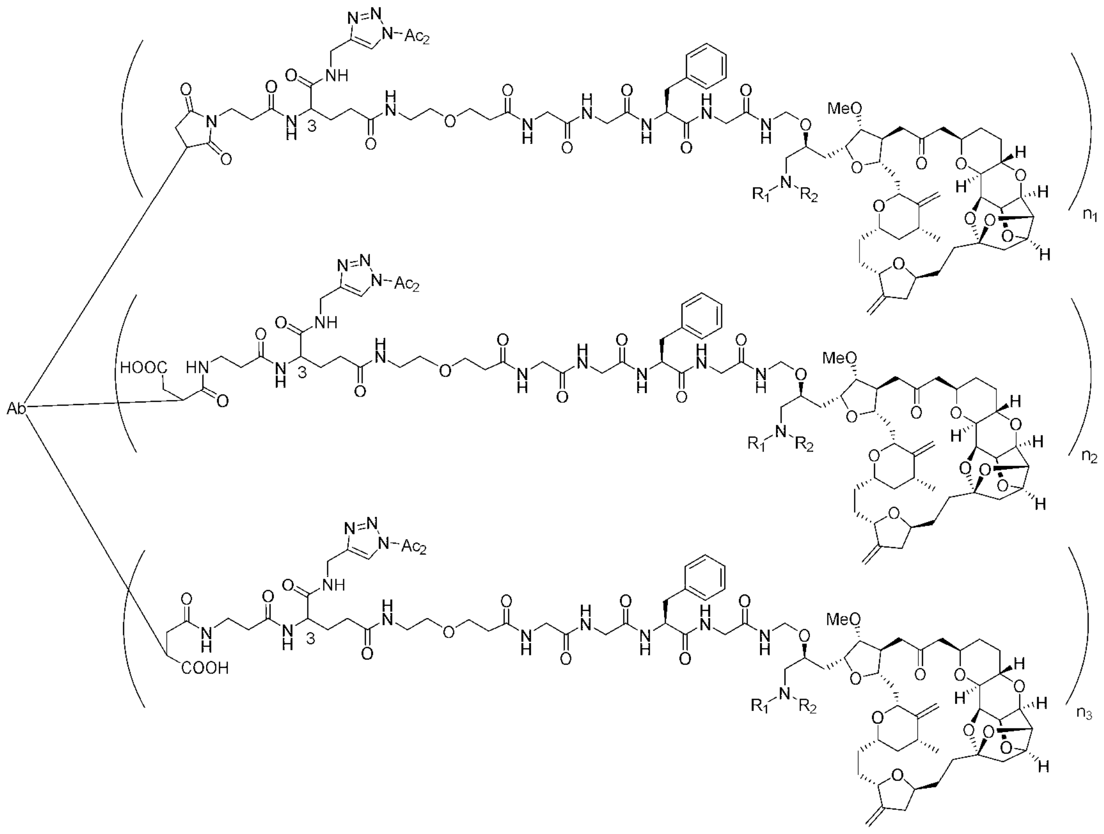

Formula VIe

wherein the $R_1$, $R_2$, $n_1$, $n_2$ and $n_3$ are as defined above;

$Ac_2$ is a hydrophilic structural unit composed of $-X_2-Y_2$, and the $X_2$ and $Y_2$ are as defined above.

In some embodiments, the $Ac_2$ is selected, without limitation, from

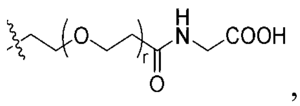

,

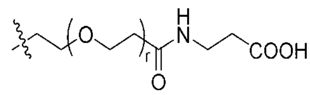

,

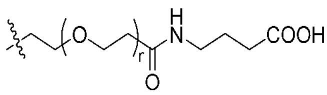

,

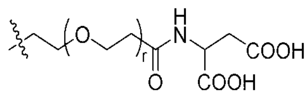

,

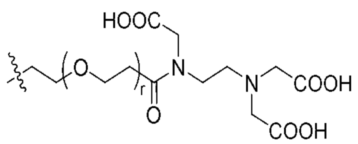

,

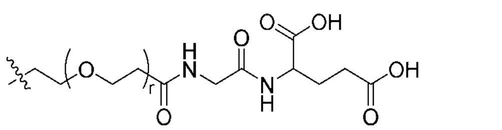

,

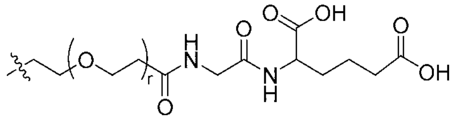

,

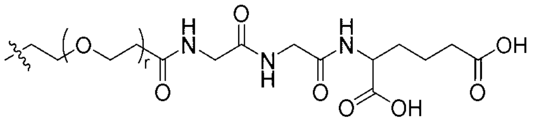

,

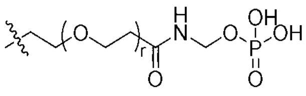 , 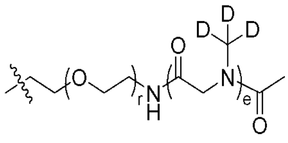 or 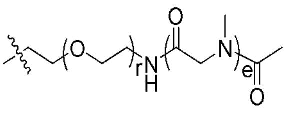 ;

wherein, r is selected from integers between 1 and 10; e is selected from integers between 1 and 20.

In some embodiments, $Ac_2$ is selected from the following structures:

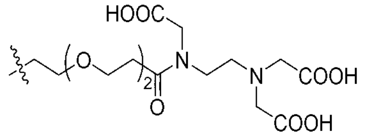 and 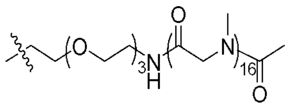 .

In some embodiments, the ligand-drug conjugate or the pharmaceutically acceptable salt or solvate thereof of the present application has a structure represented by formula VIf:

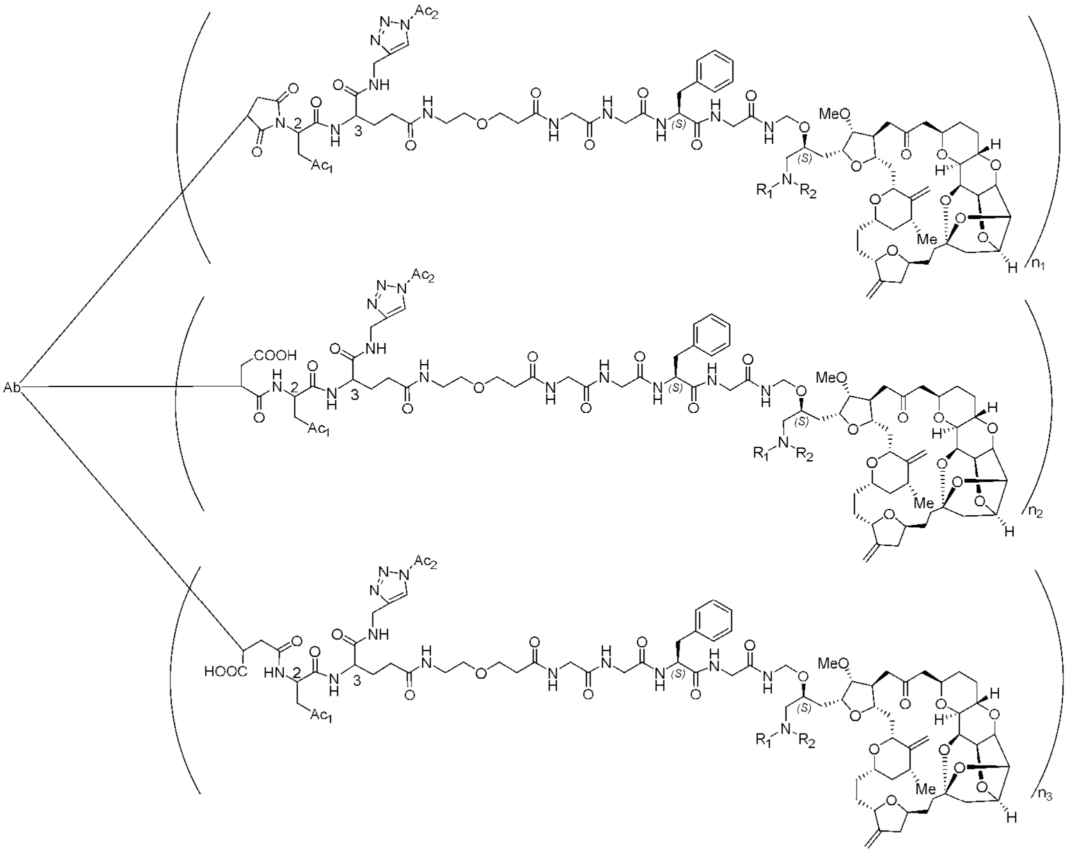

Formula VIf

wherein the $R_1$, $R_2$, $n_1$, $n_2$ and $n_3$ are as defined above;

$Ac_1$ is a hydrophilic structural unit having the structure shown in formula d:

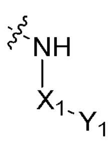

Formula d

the $X_1$ and $Y_1$ are as defined above;

In some embodiments, the $Ac_1$ is selected, without limitation, from the group consisting of (D/L) alanine, (D/L) leucine, (D/L) isoleucine, (D/L) valine, (D/L) phenylalanine, (D/L) proline, (D/L) tryptophan, (D/L) serine, (D/L) tyrosine, (D/L) cysteine, (D/L) cystine, (D/L) arginine, (D/L) histidine, (D/L) methionine, (D/L) asparagine, (D/L) glutamine, (D/L) threonine, (D/L) aspartic acid, (D/L) glutamic acid, natural or unnatural amino acid derivatives and the following structures:

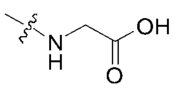 , 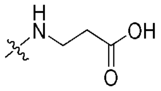 , 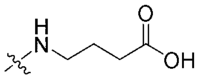 , 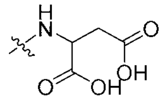 , 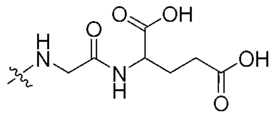 ,

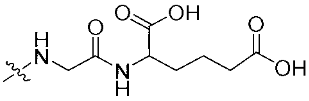 , 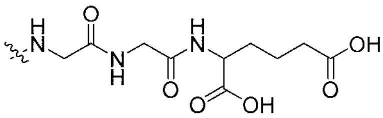 , 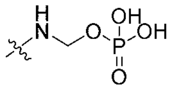 , 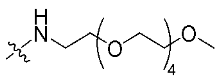

and

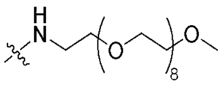 .

In some embodiments, $Ac_1$ is selected from the following structures:

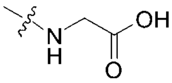 ,

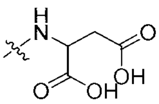 , and 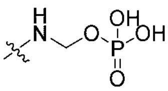 .

In some embodiments, $Ac_1$ is:

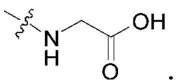 .

$Ac_2$ is a hydrophilic structural unit composed of $-X_2-Y_2$, and the $X_2$ and $Y_2$ are as defined above.

In some embodiments, the $Ac_2$ is selected, without limitation, from

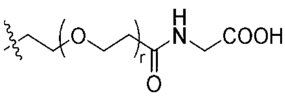 ,

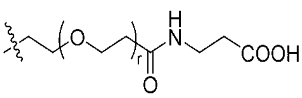 , 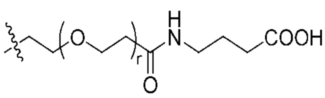 , 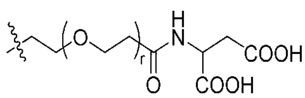 ,

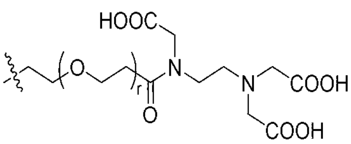 , 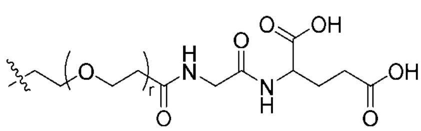 ,

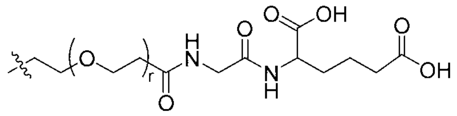,
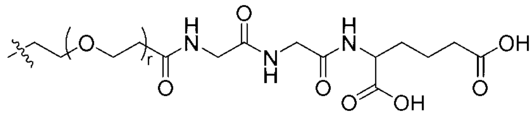,

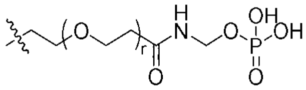, 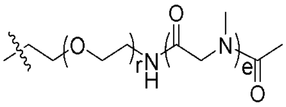 or 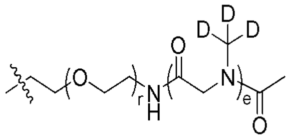;

wherein, r is selected from integers between 1 and 10; e is selected from integers between 1 and 20.

In some embodiments, $Ac_2$ is selected from the following structures:

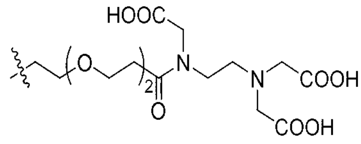, 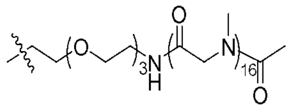 and 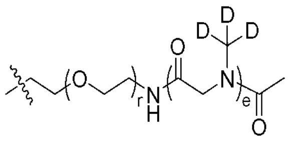.

In some embodiments, the ligand-drug conjugate or the pharmaceutically acceptable salt or solvate thereof of the present application has a structure represented by formula VIg:

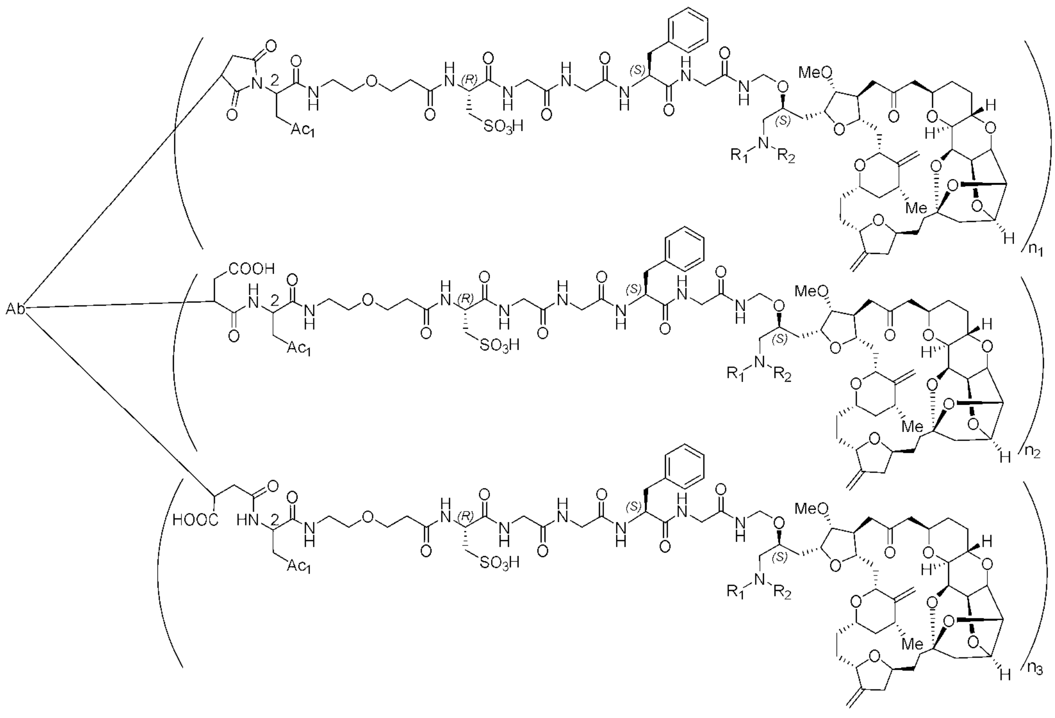

Formula VIg

wherein the $R_1$, $R_2$, $n_1$, $n_2$ and $n_3$ are as defined above;

$Ac_1$ is a hydrophilic structural unit having the structure shown in formula d:

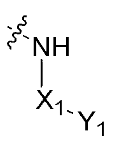

Formula d

the $X_1$ and $Y_1$ are as defined above;

In some embodiments, the $Ac_1$ is selected, without limitation, from the group consisting of (D/L) alanine, (D/L) leucine, (D/L) isoleucine, (D/L) valine, (D/L) phenylalanine, (D/L) proline, (D/L) tryptophan, (D/L) serine, (D/L) tyrosine, (D/L) cysteine, (D/L) cystine, (D/L) arginine, (D/L) histidine, (D/L) methionine, (D/L) asparagine, (D/L) glutamine, (D/L) threonine, (D/L) aspartic acid, (D/L) glutamic acid, natural or unnatural amino acid derivatives and the following structures:

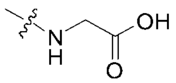, 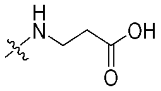, 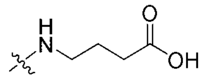, 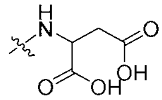, 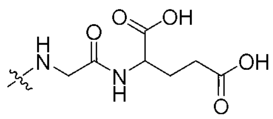,

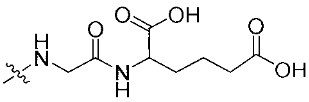, 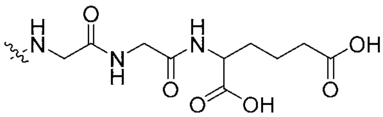, 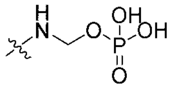, 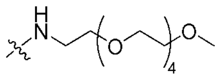

and

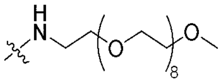.

In some embodiments, $Ac_1$ is selected from the following structures:

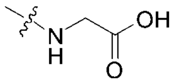,

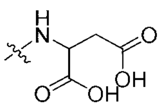, and 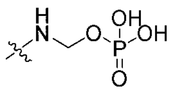.

In some embodiments, $Ac_1$ is:

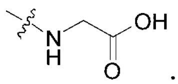.

In some embodiments, the ligand-drug conjugate or the pharmaceutically acceptable salt or solvate thereof of the present application is selected from the following structures:

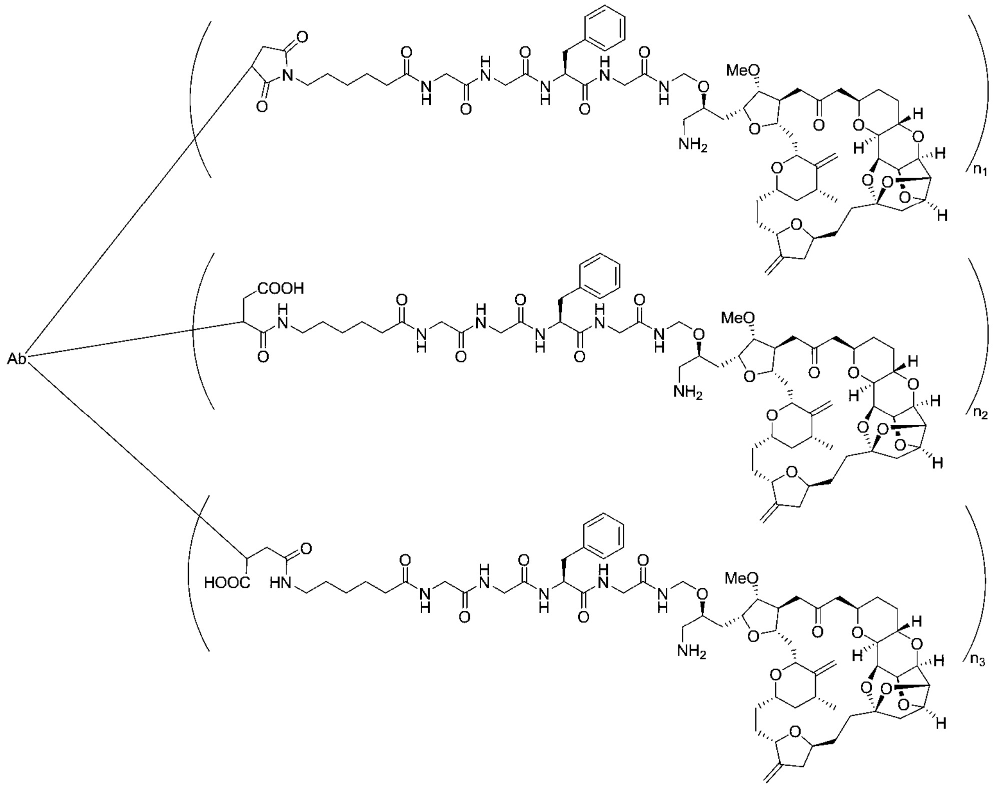

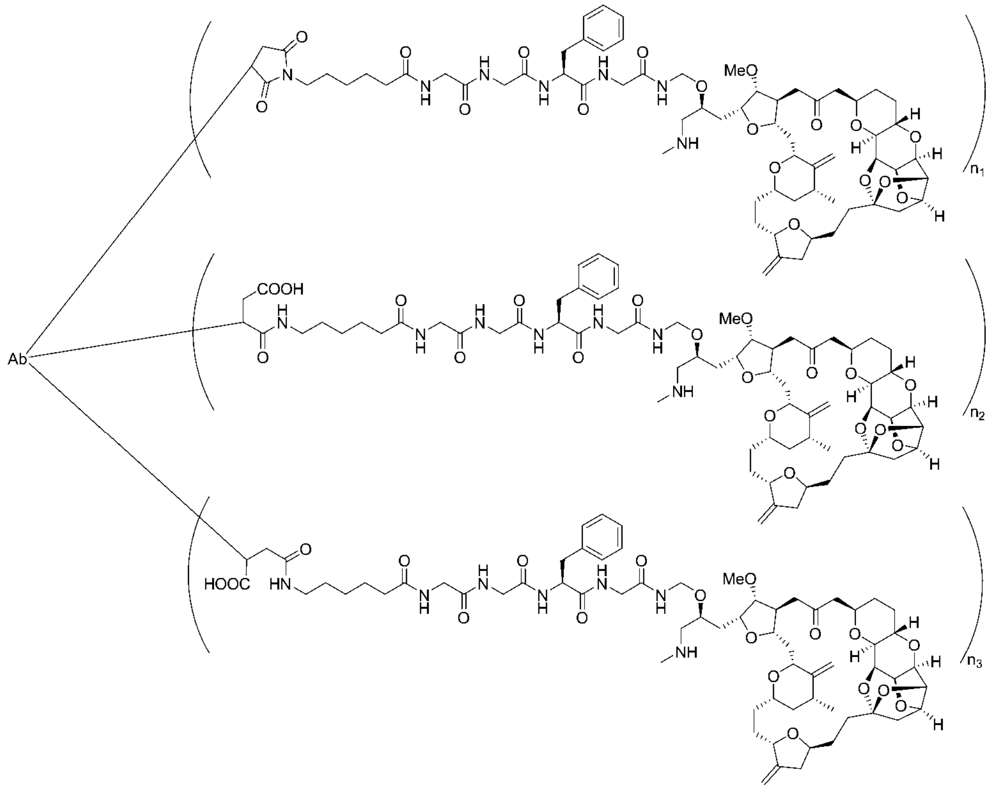

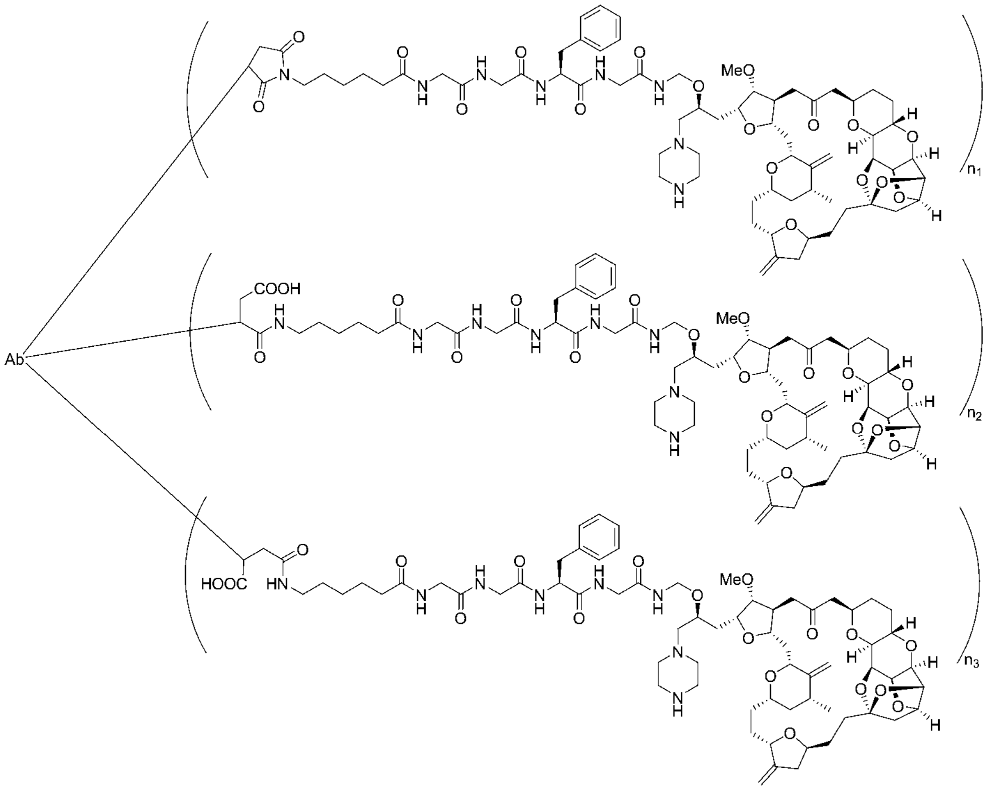

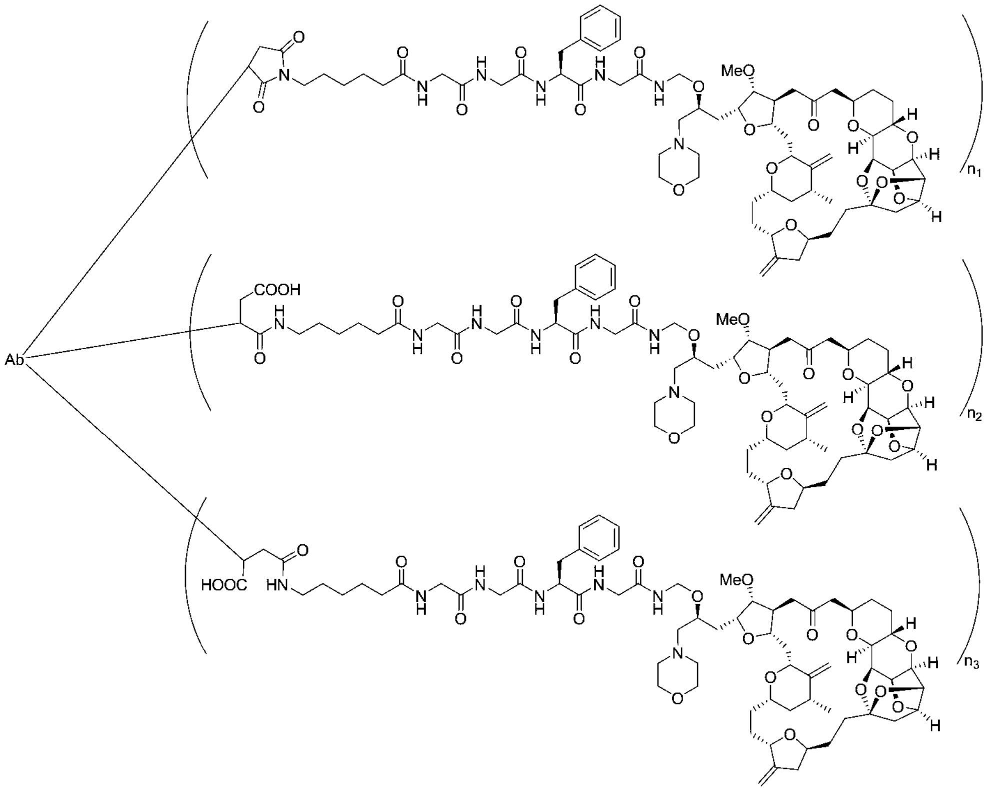

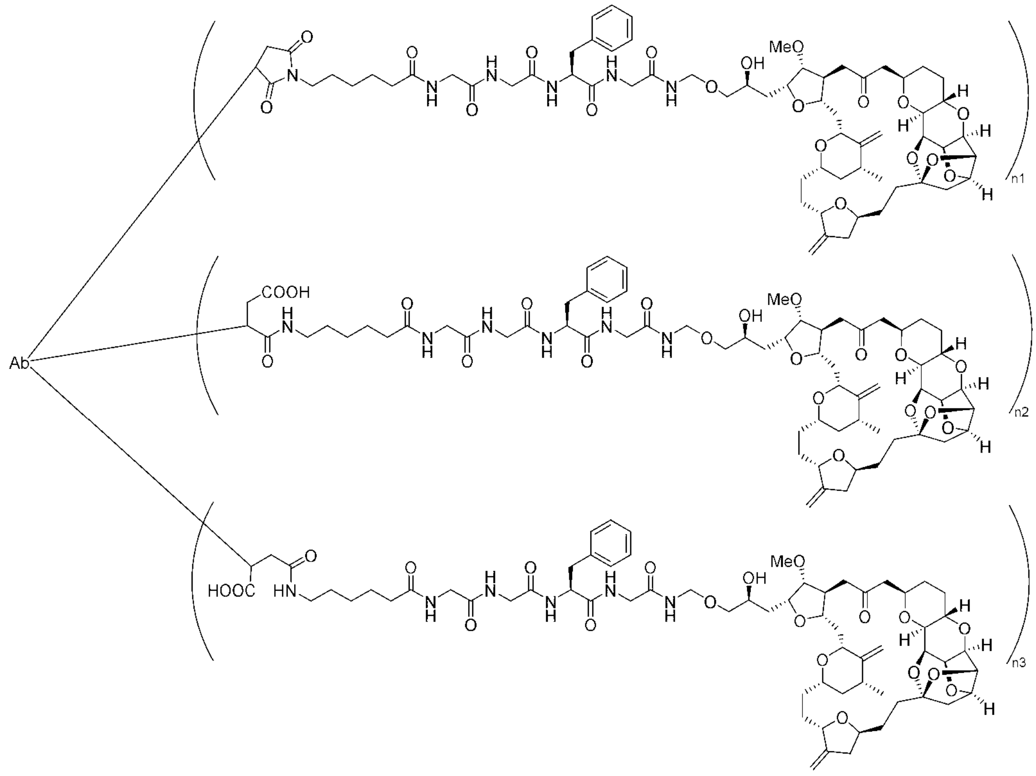

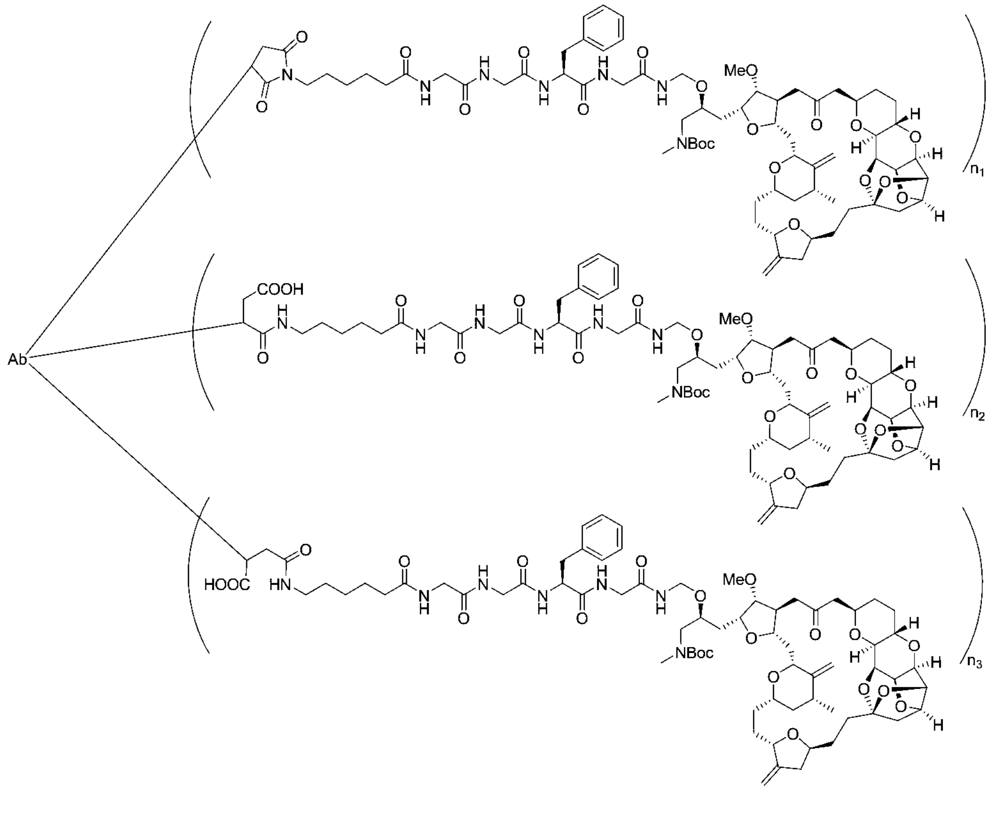

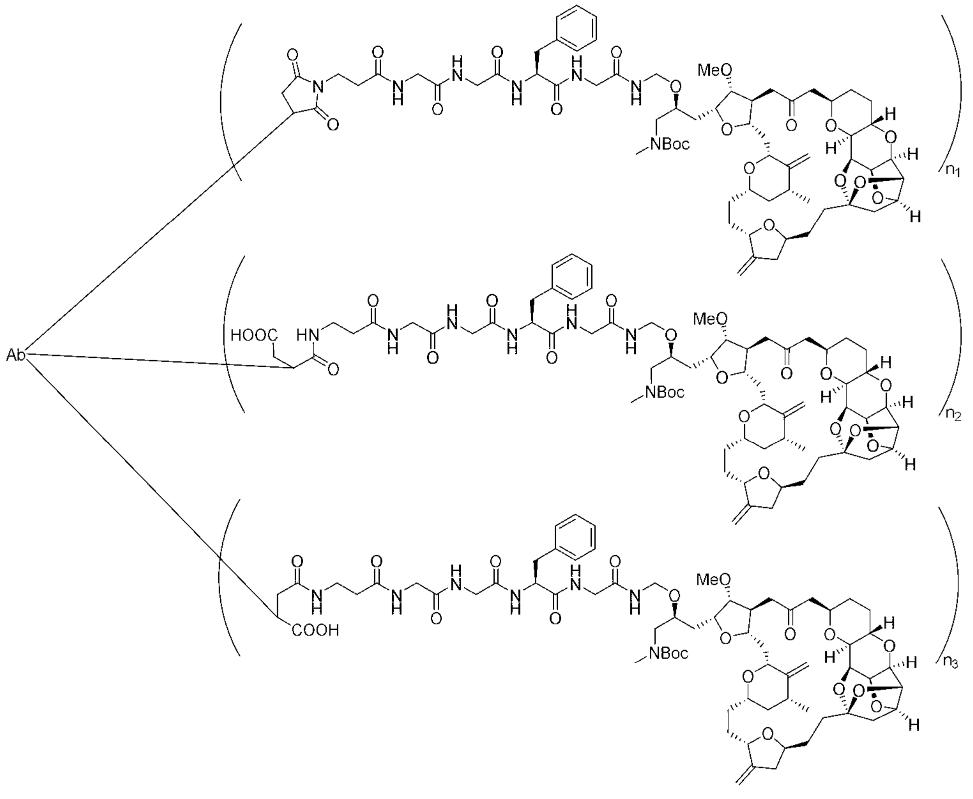

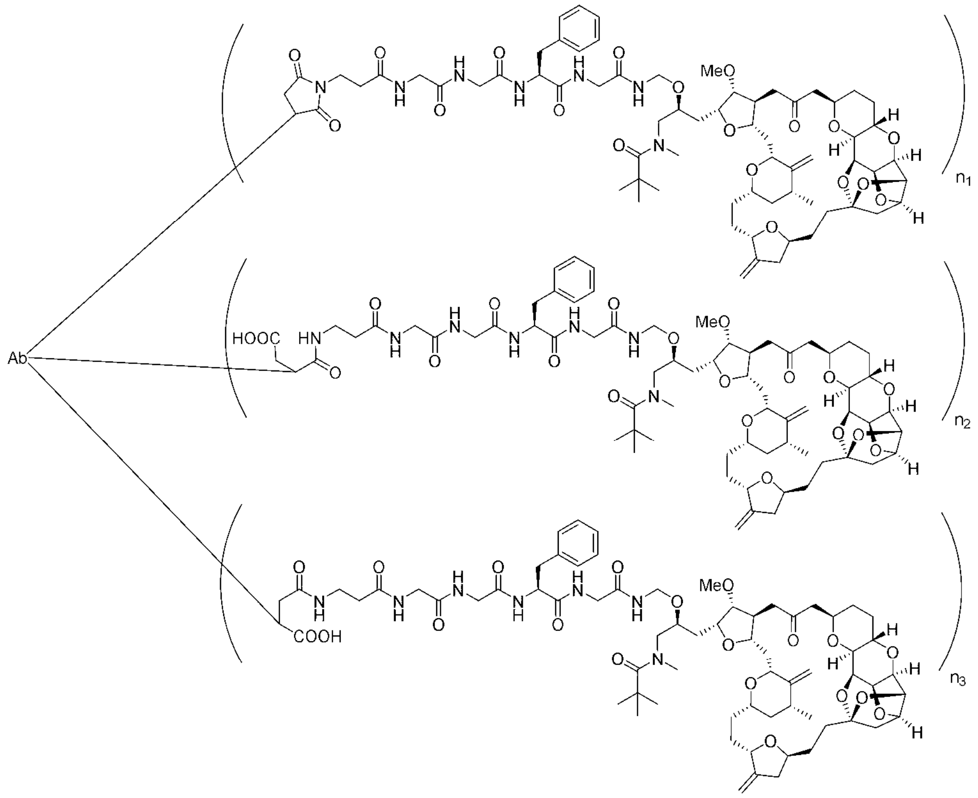

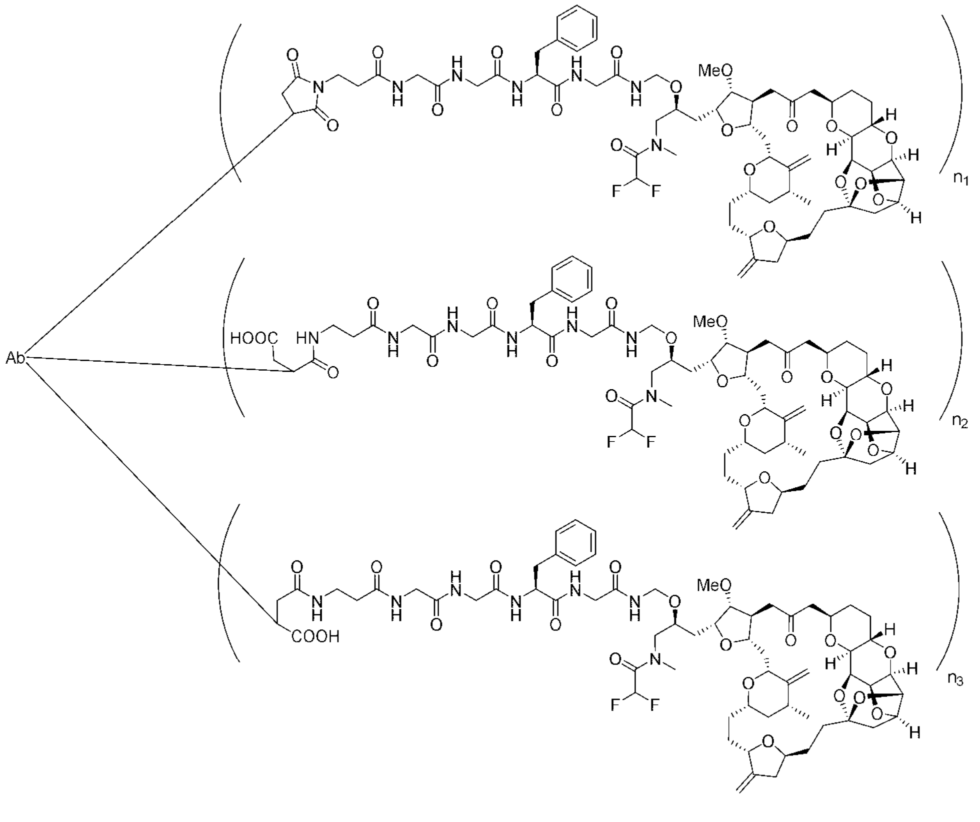

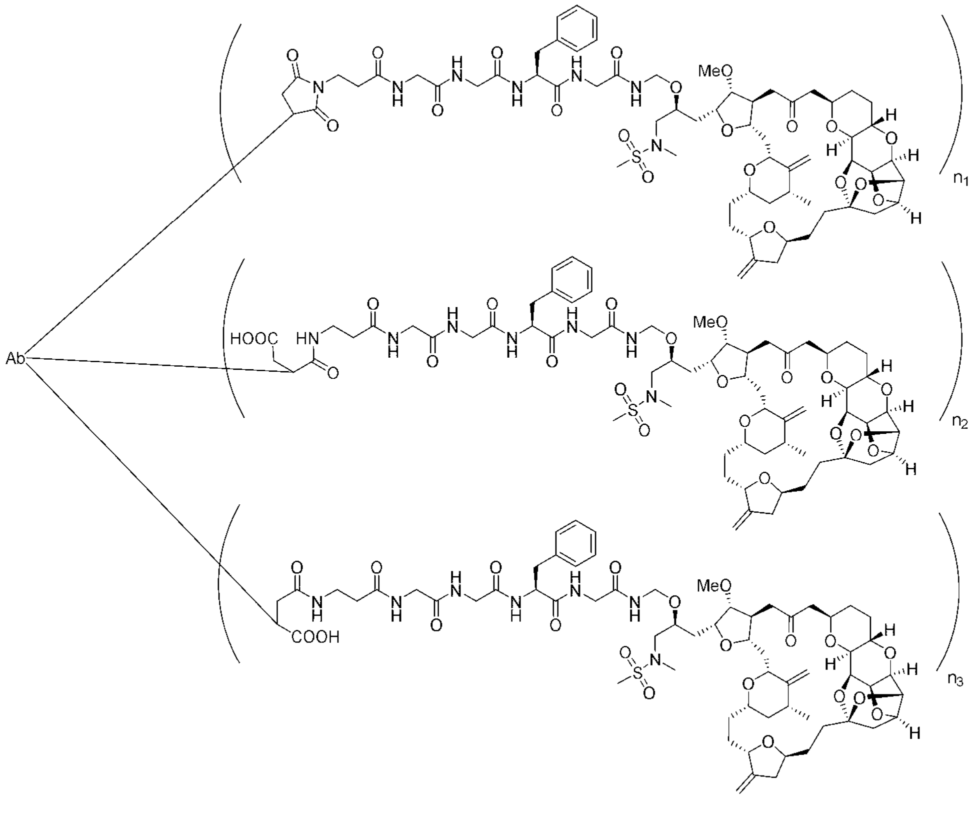

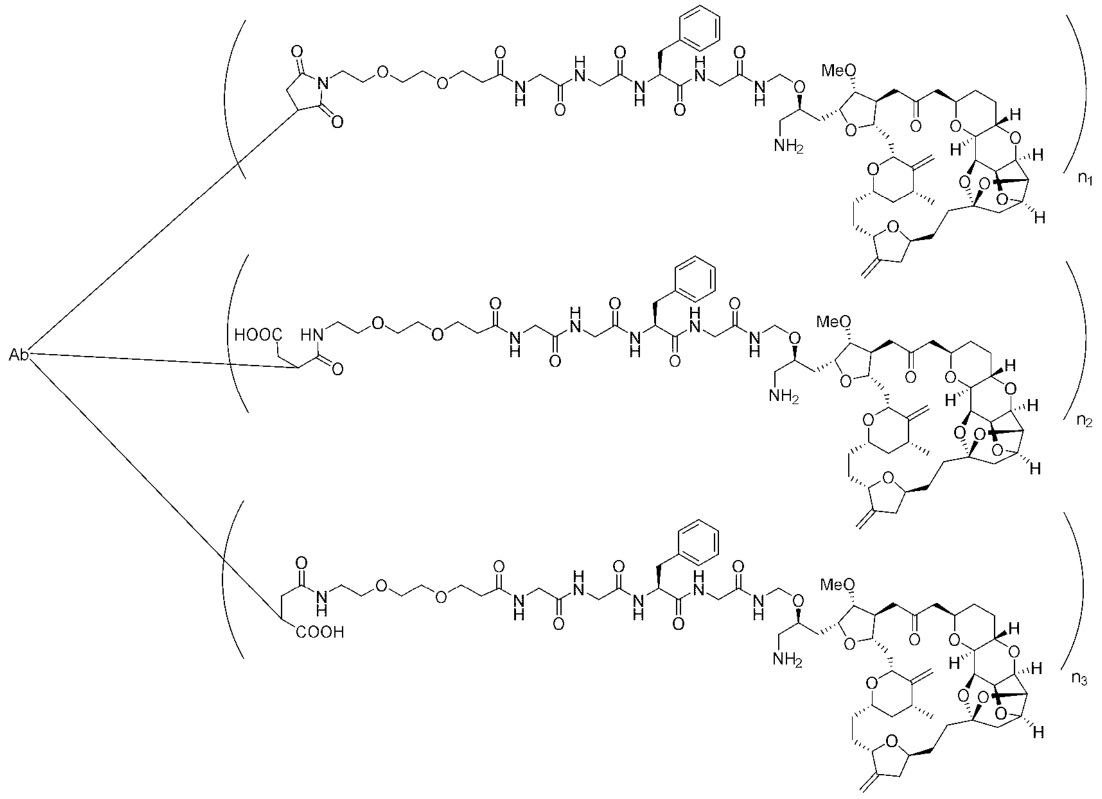

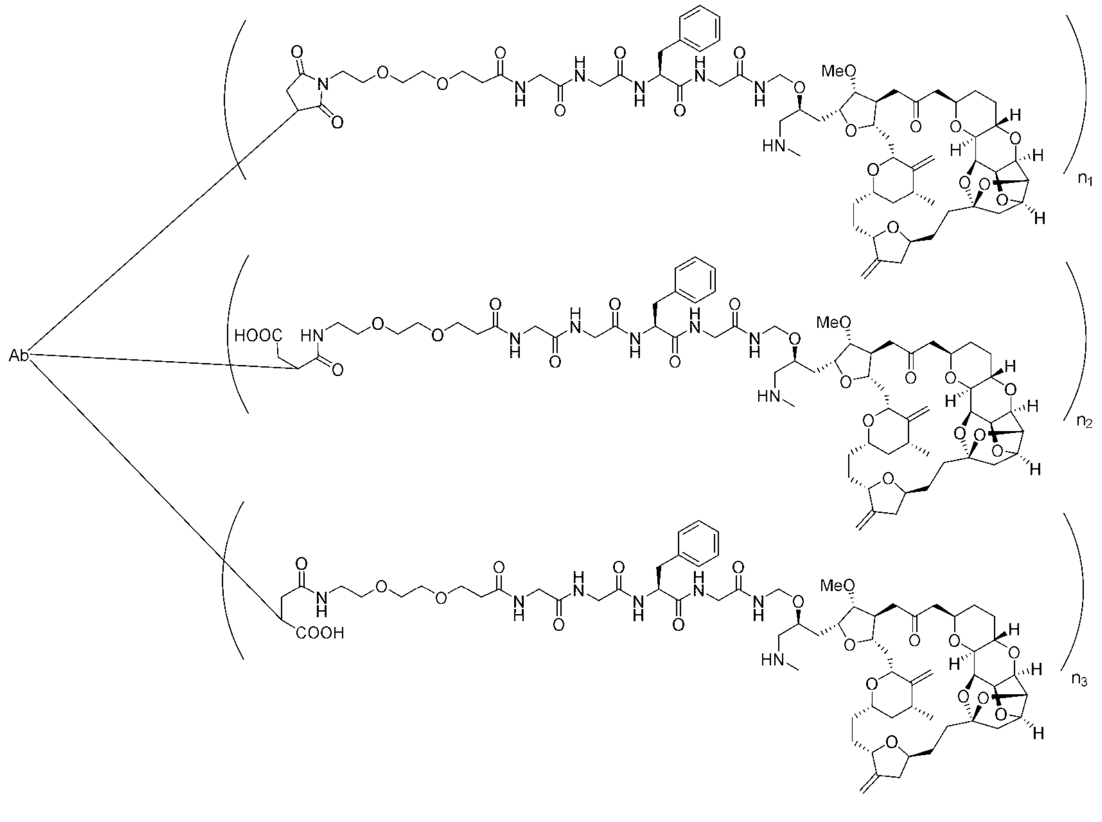

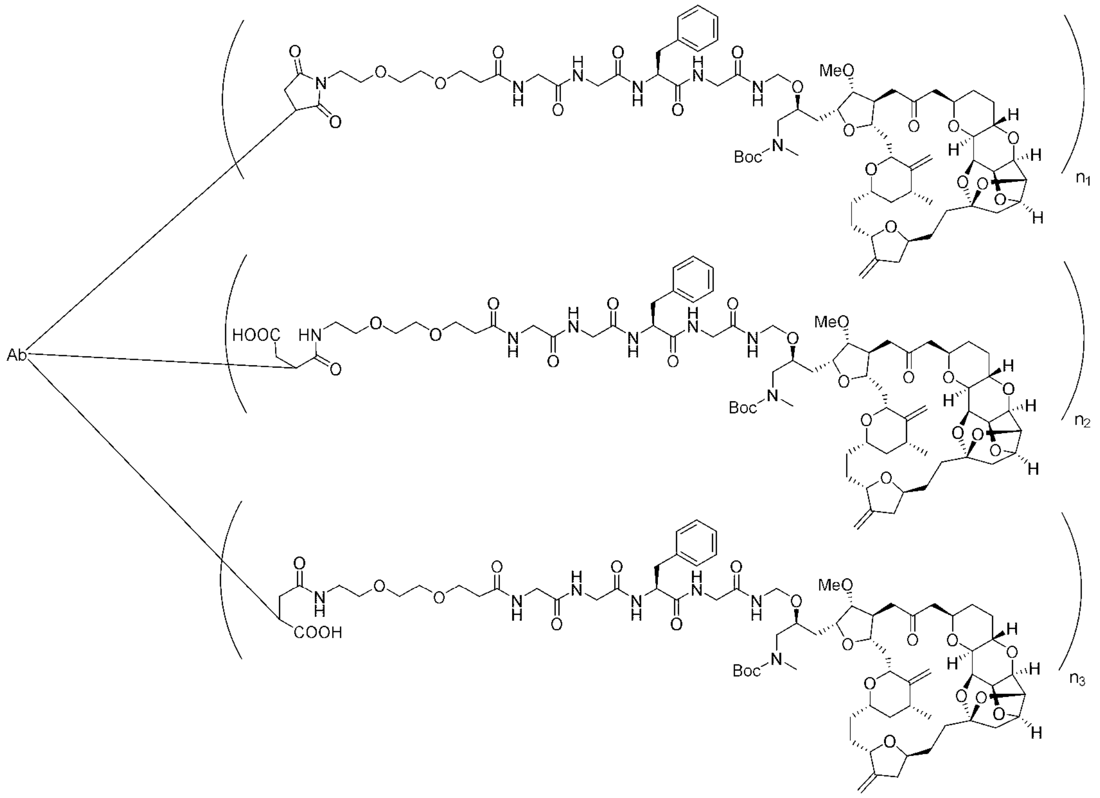

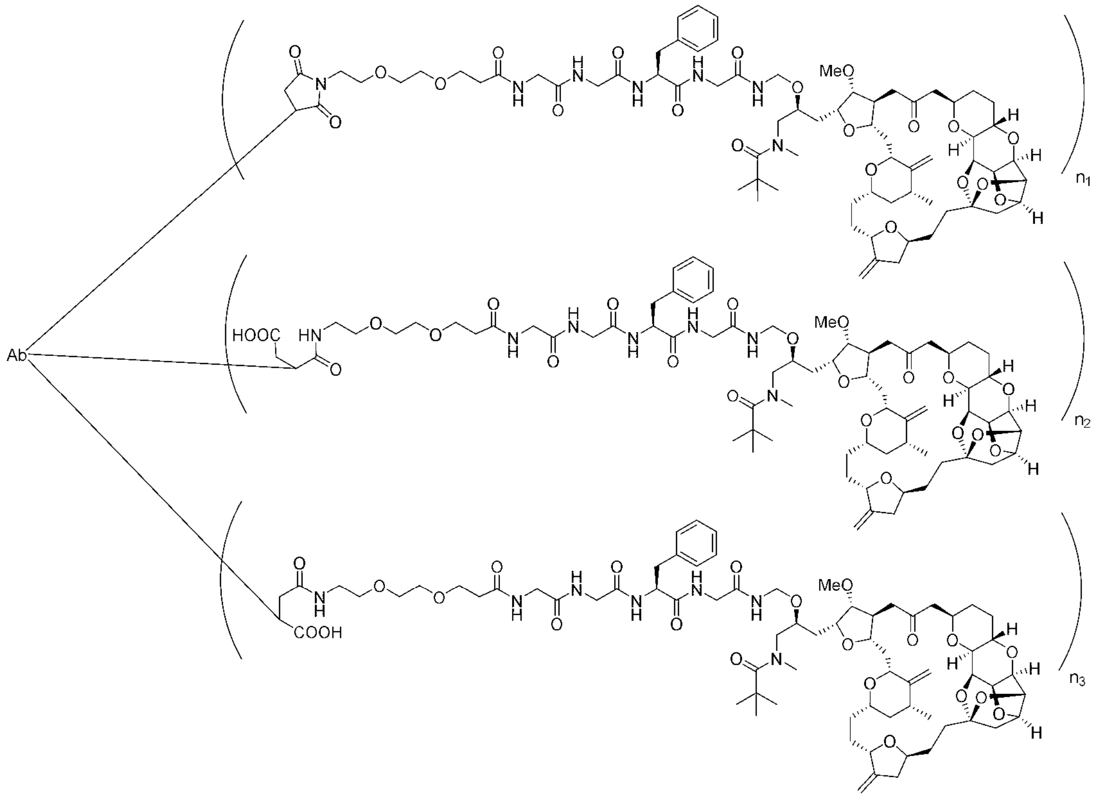

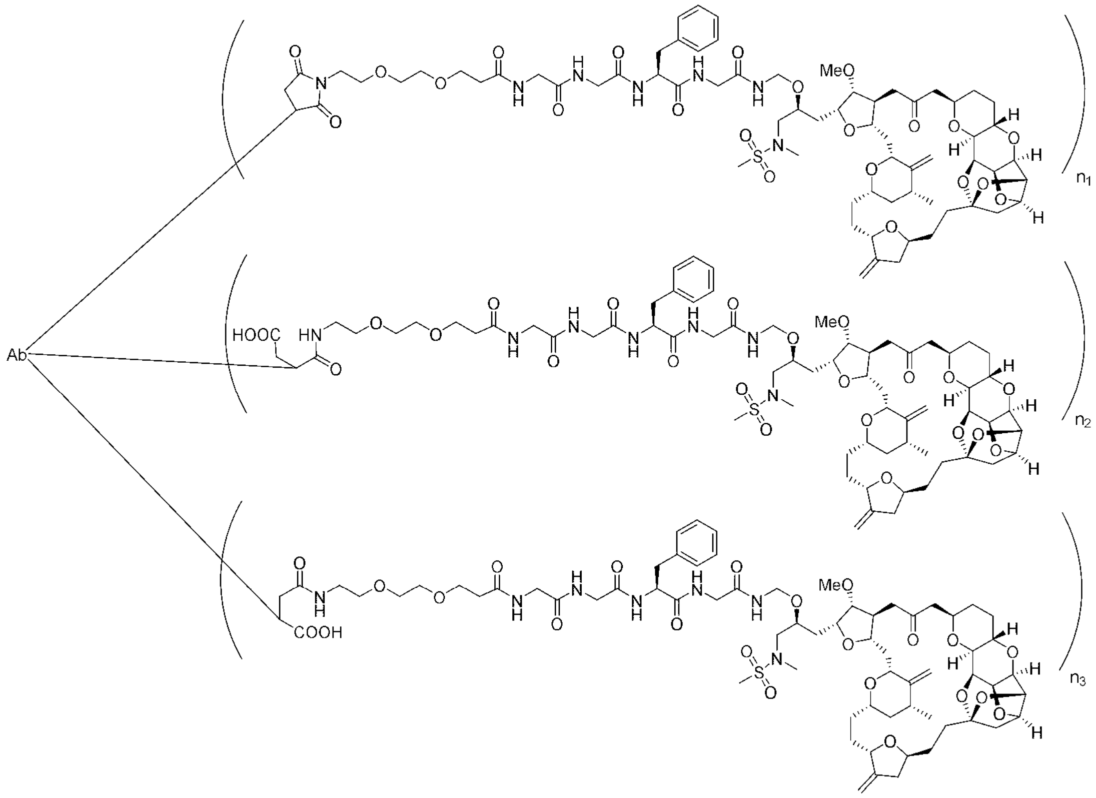

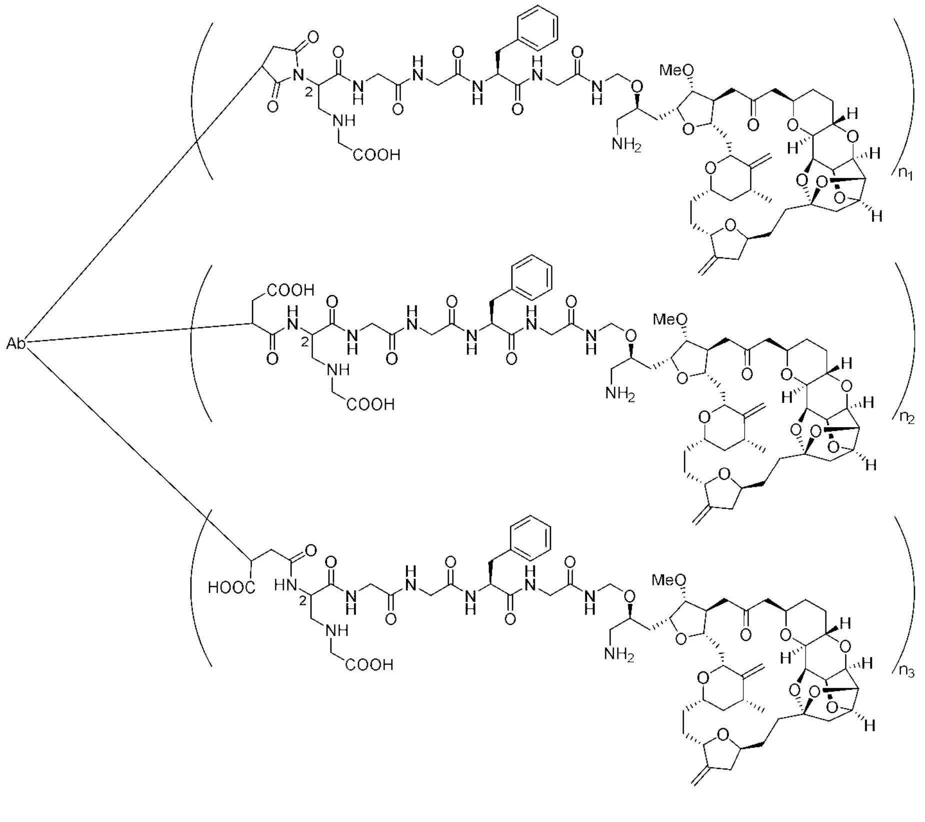

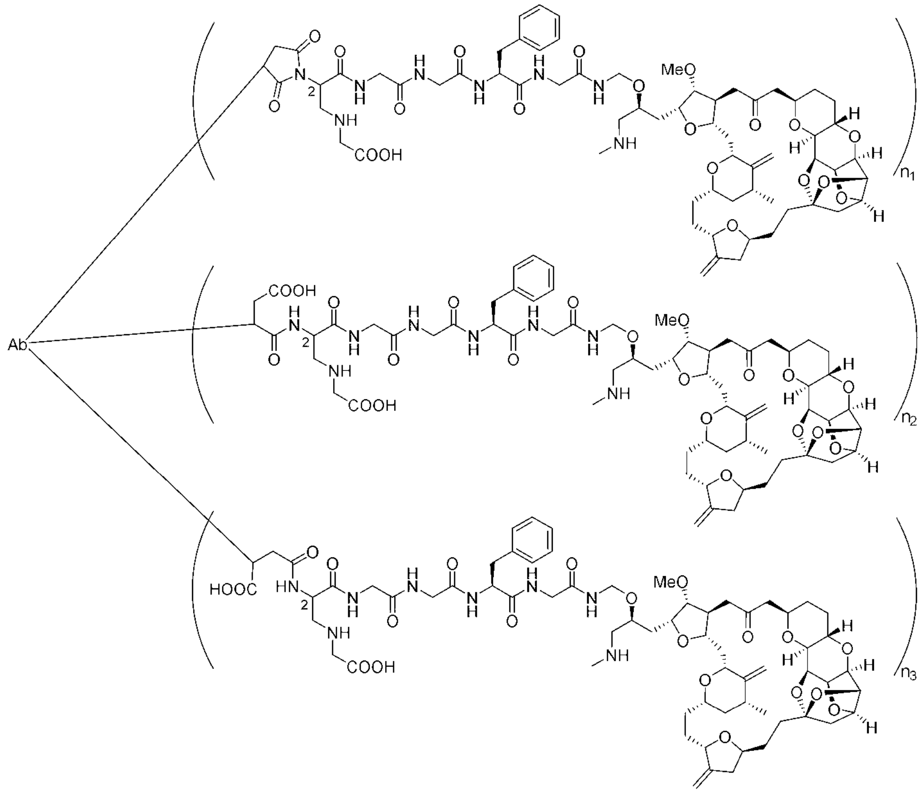

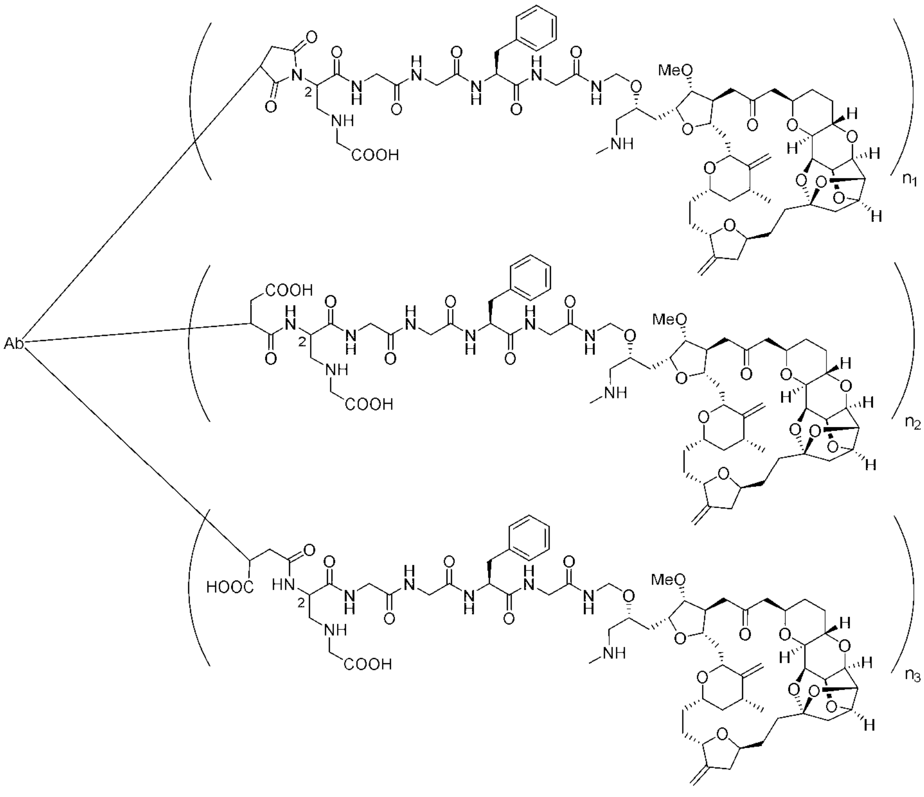

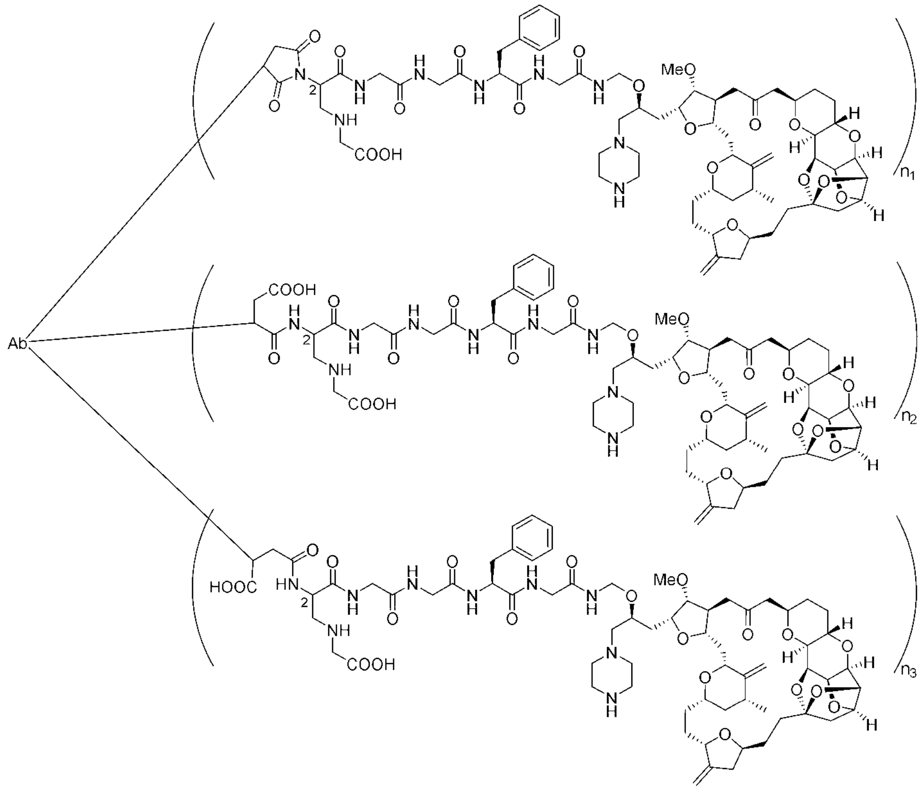

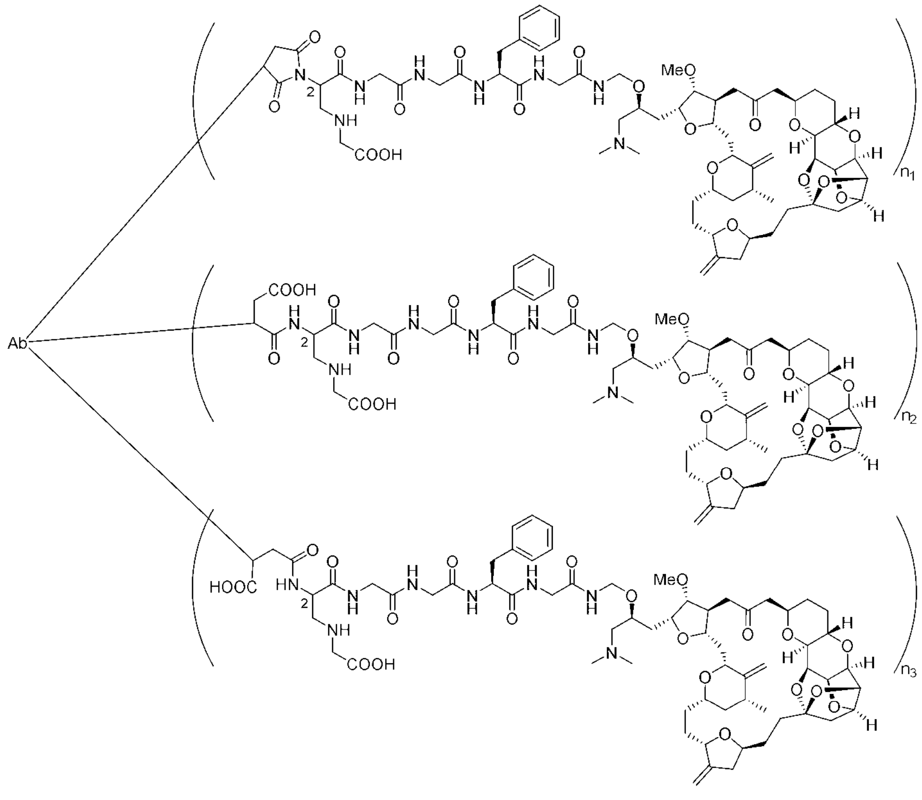

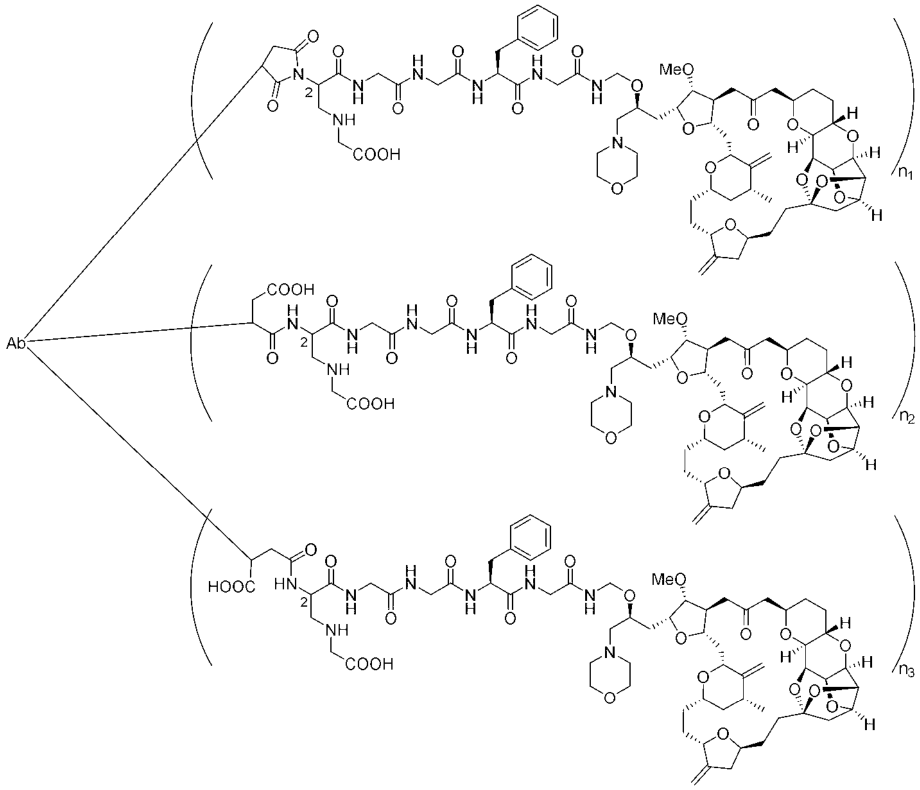

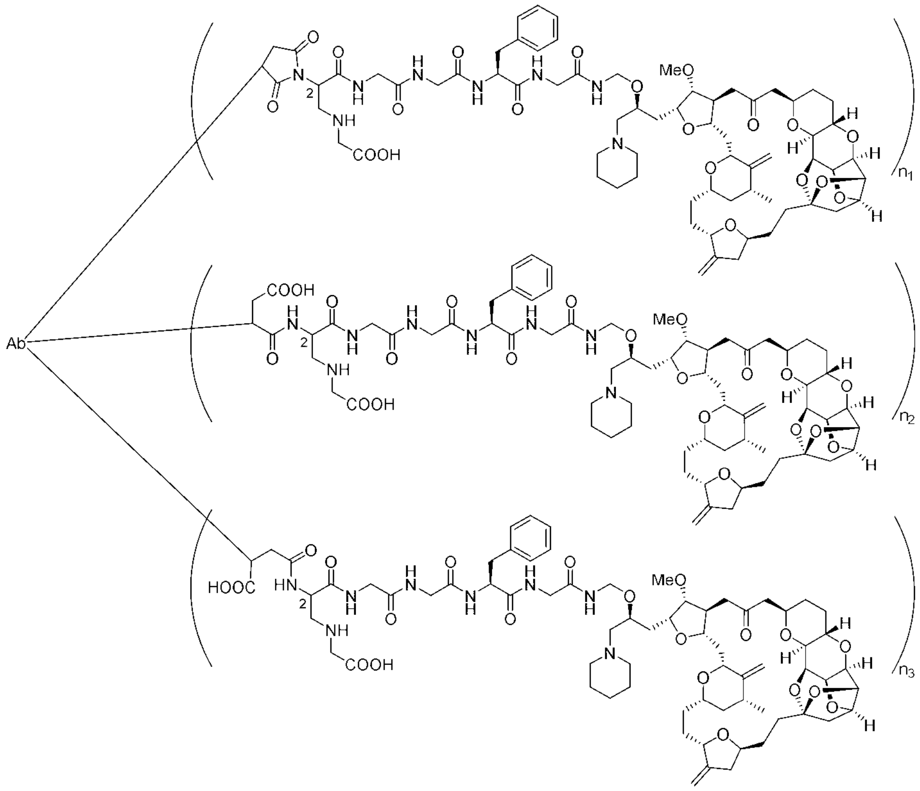

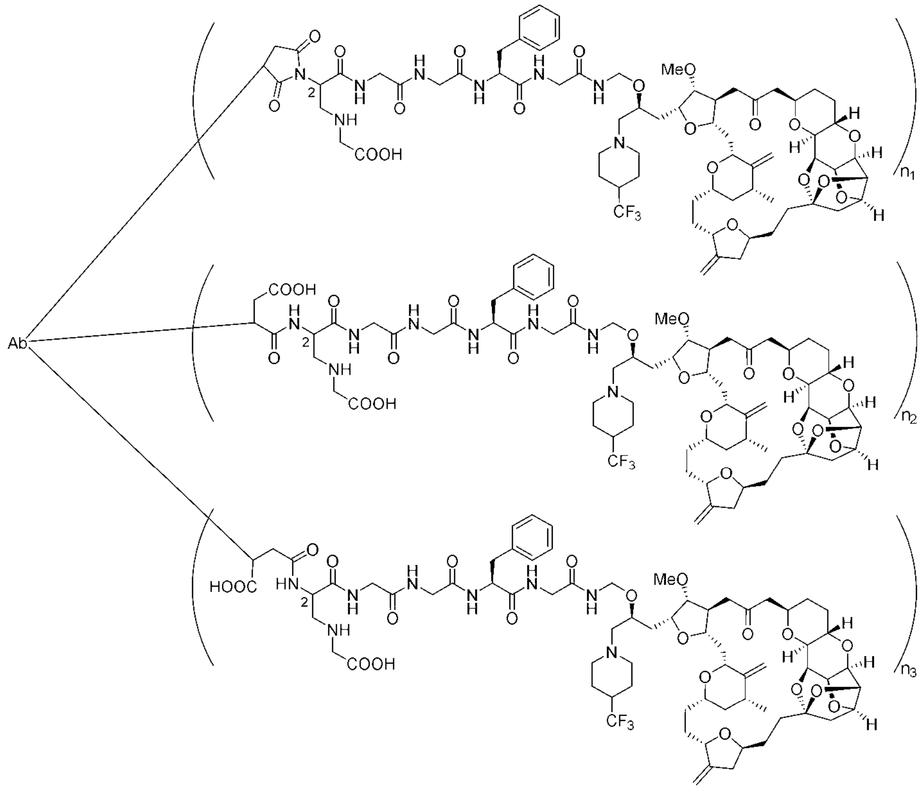

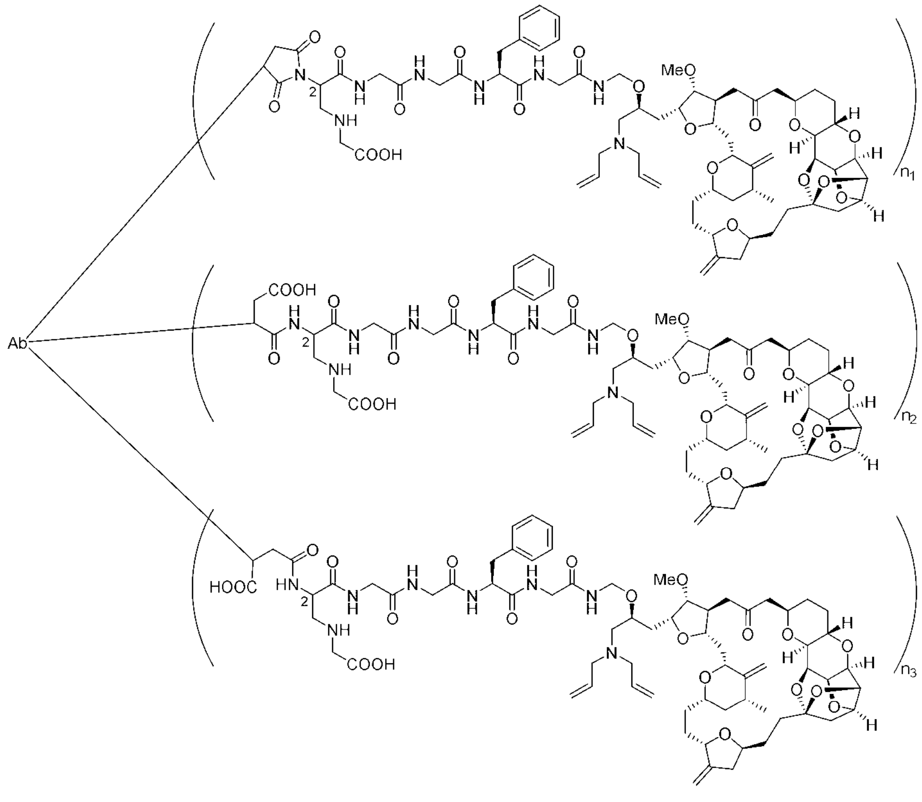

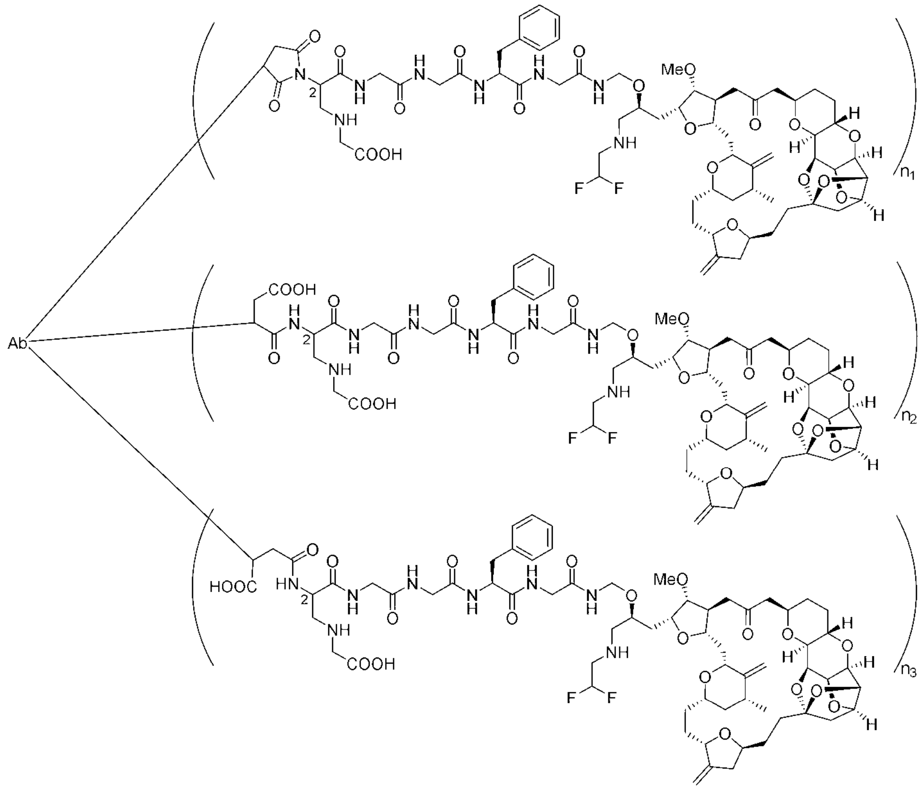

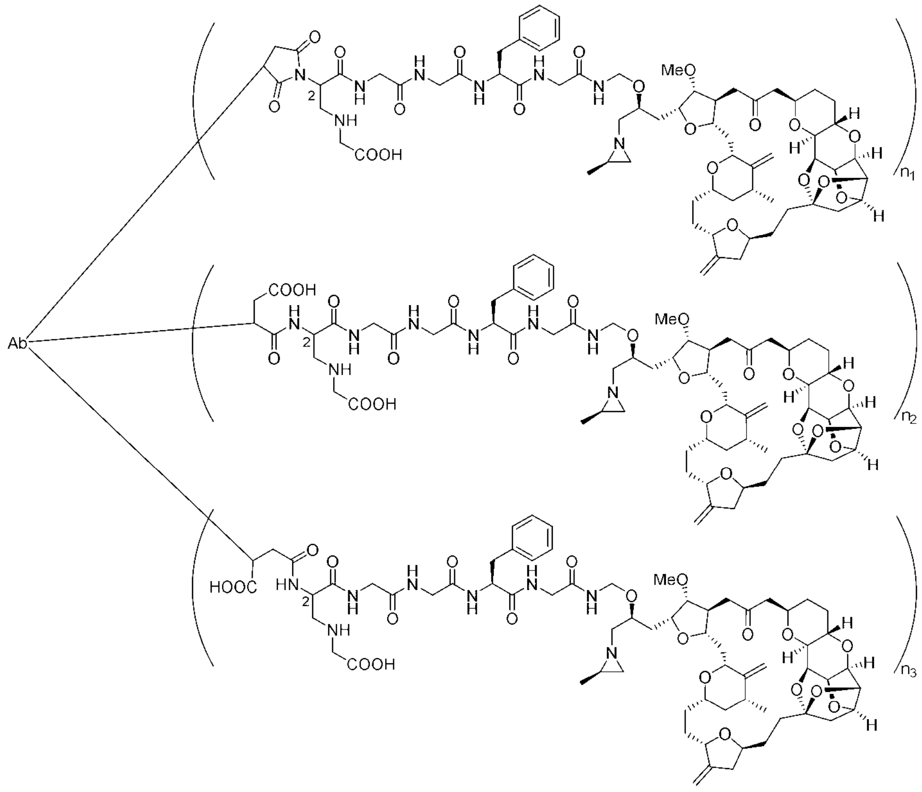

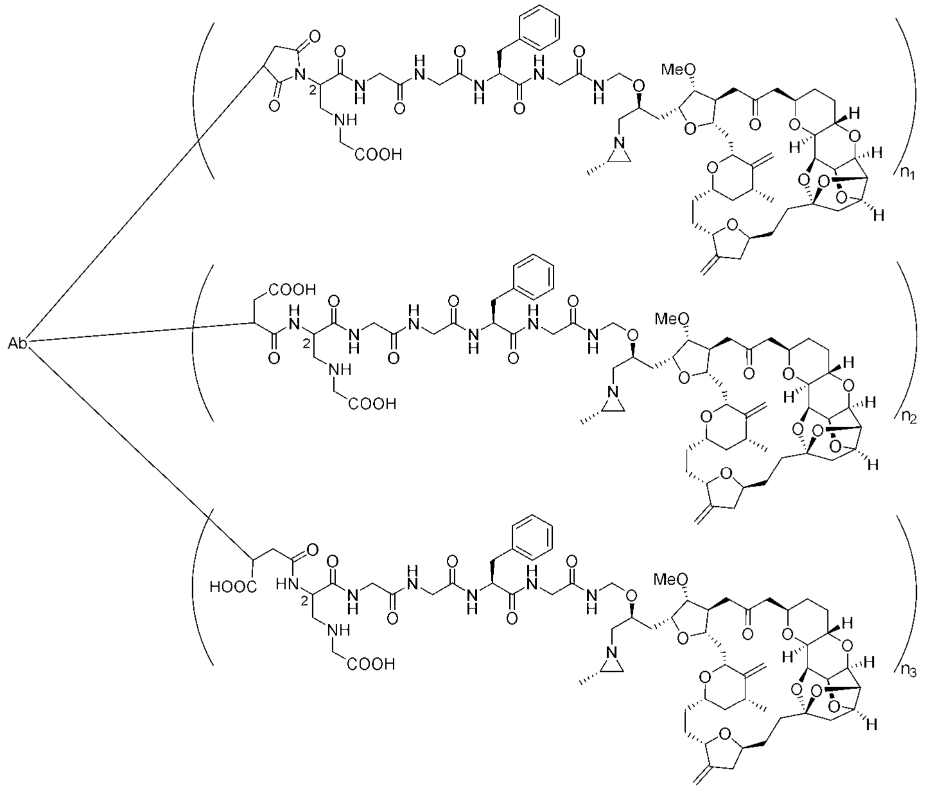

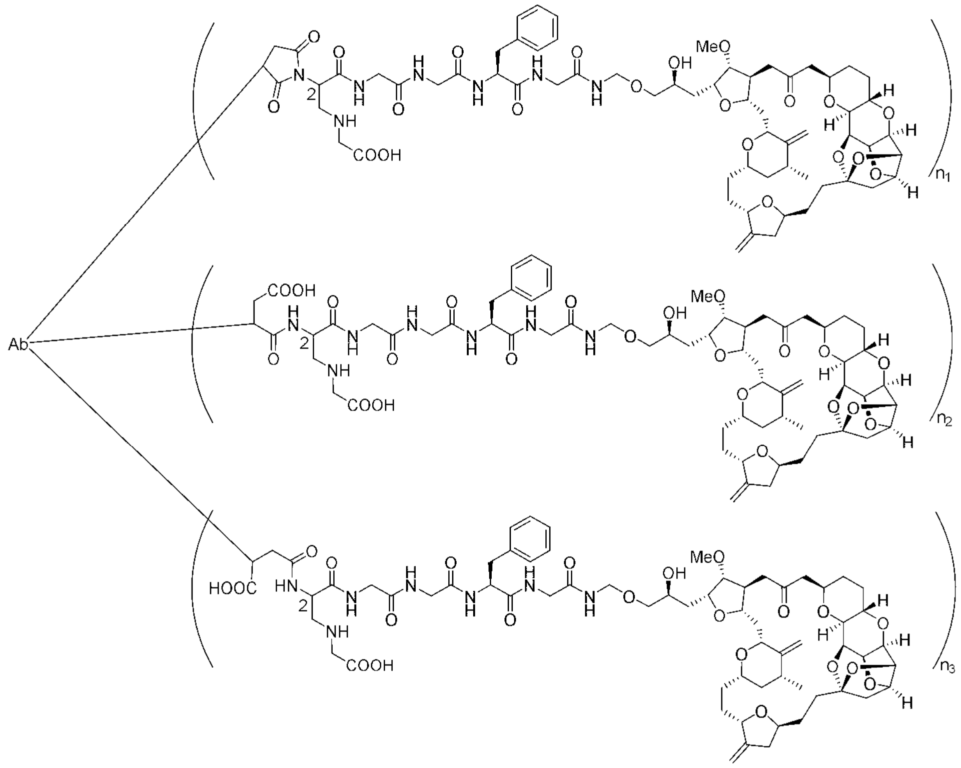

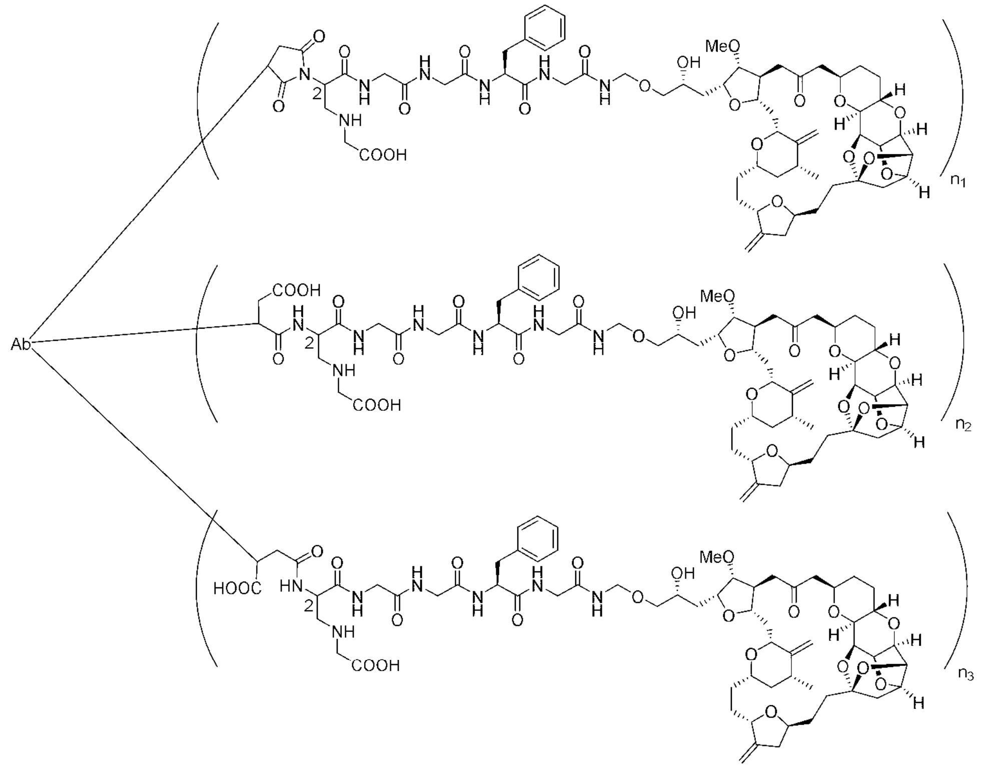

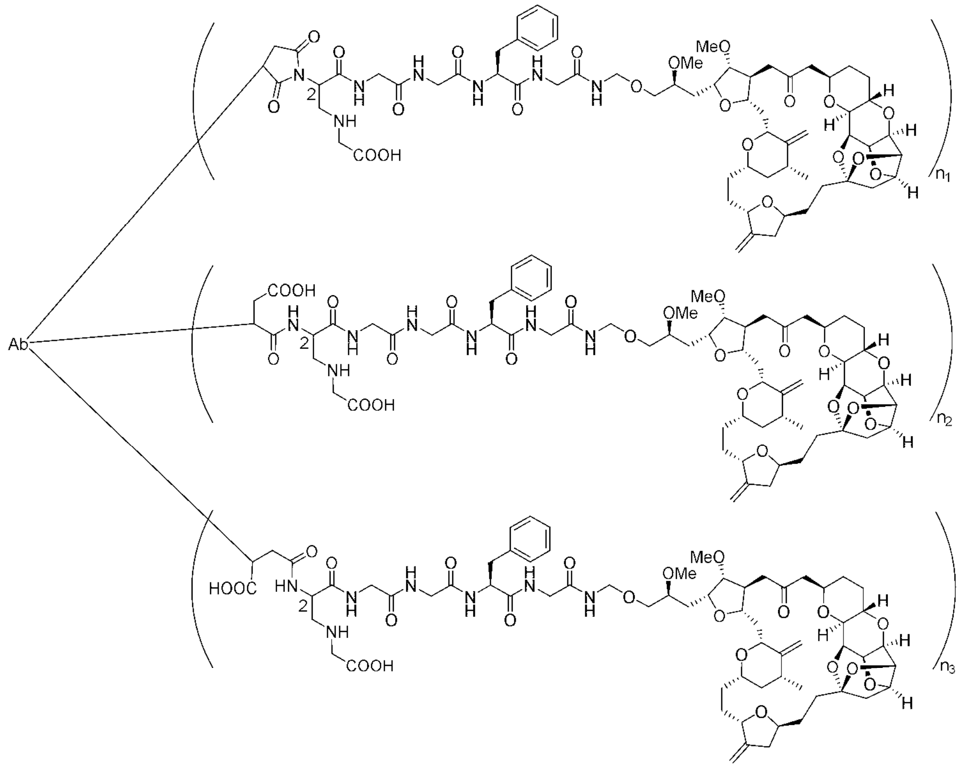

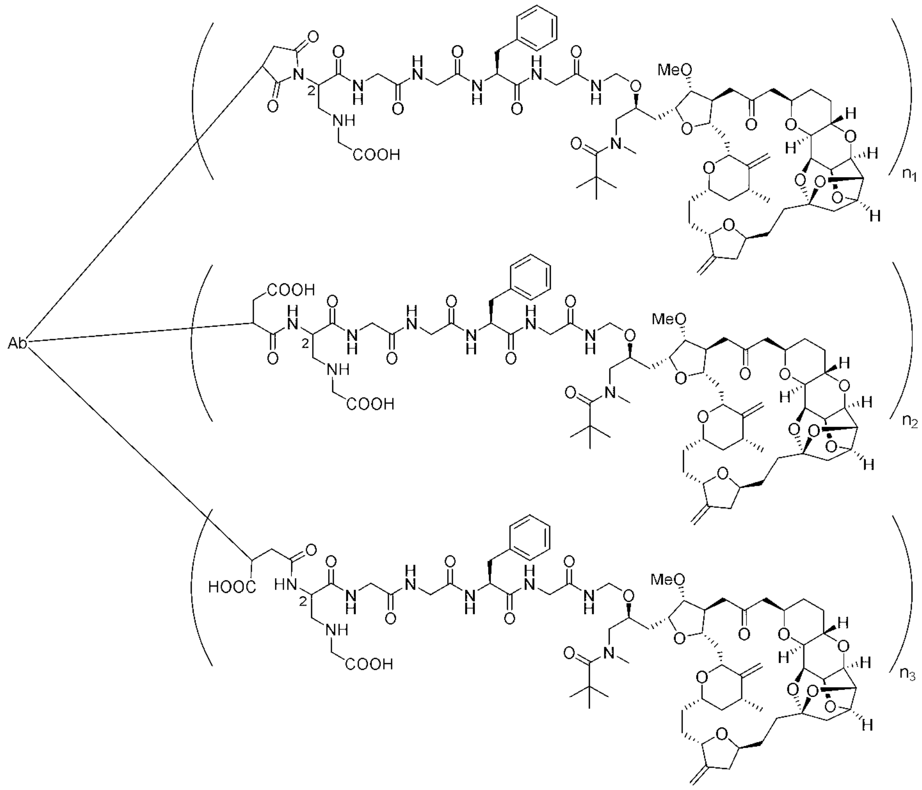

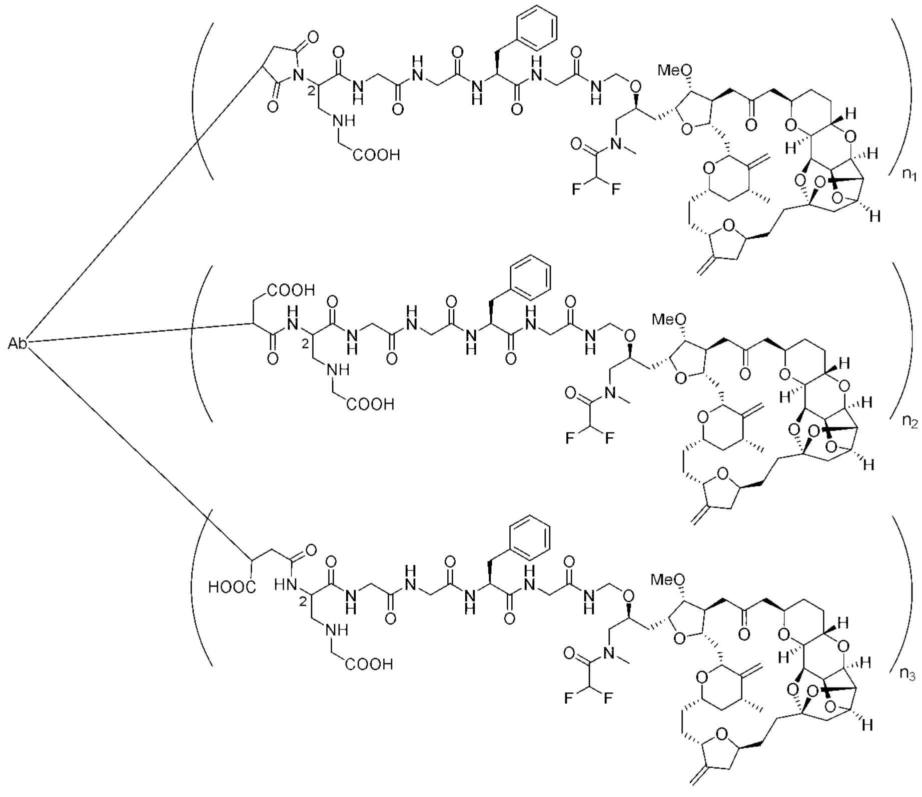

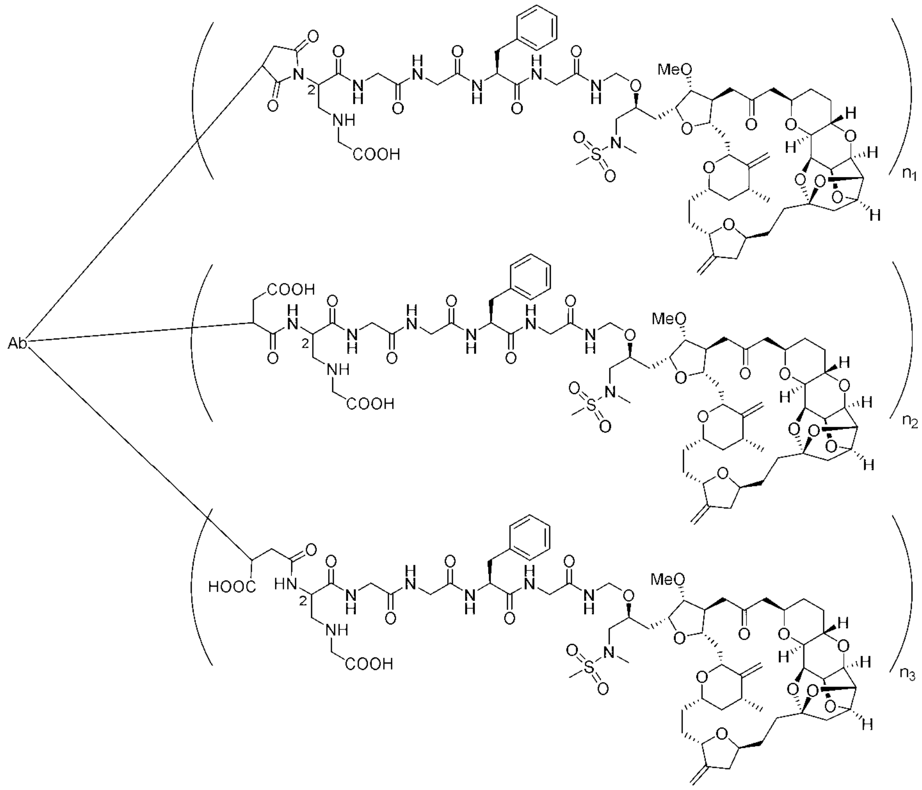

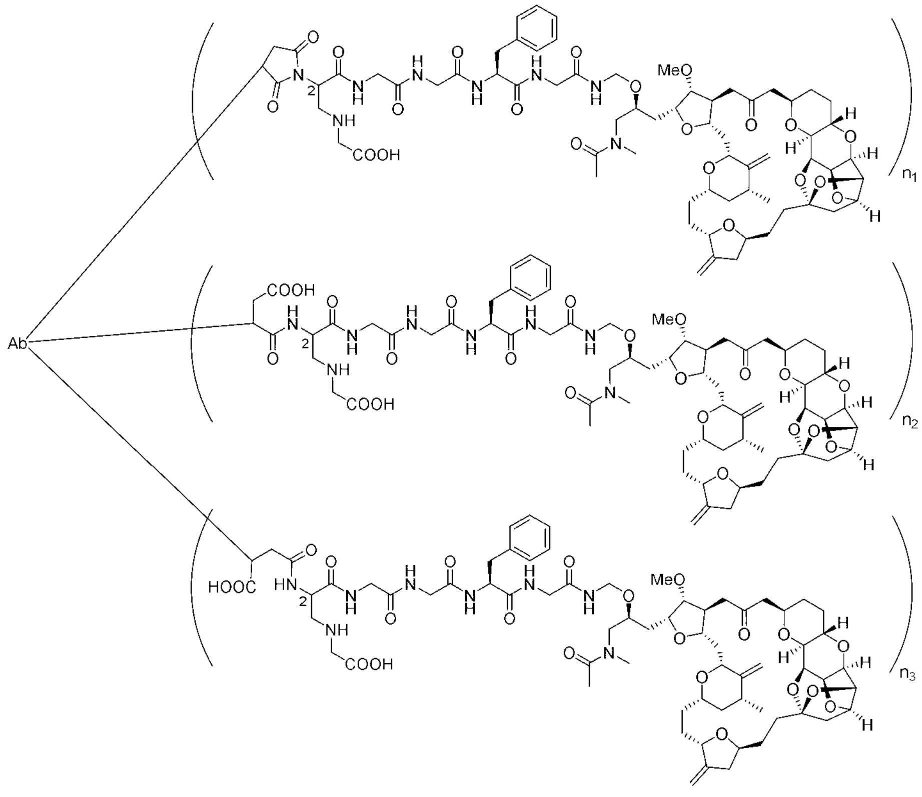

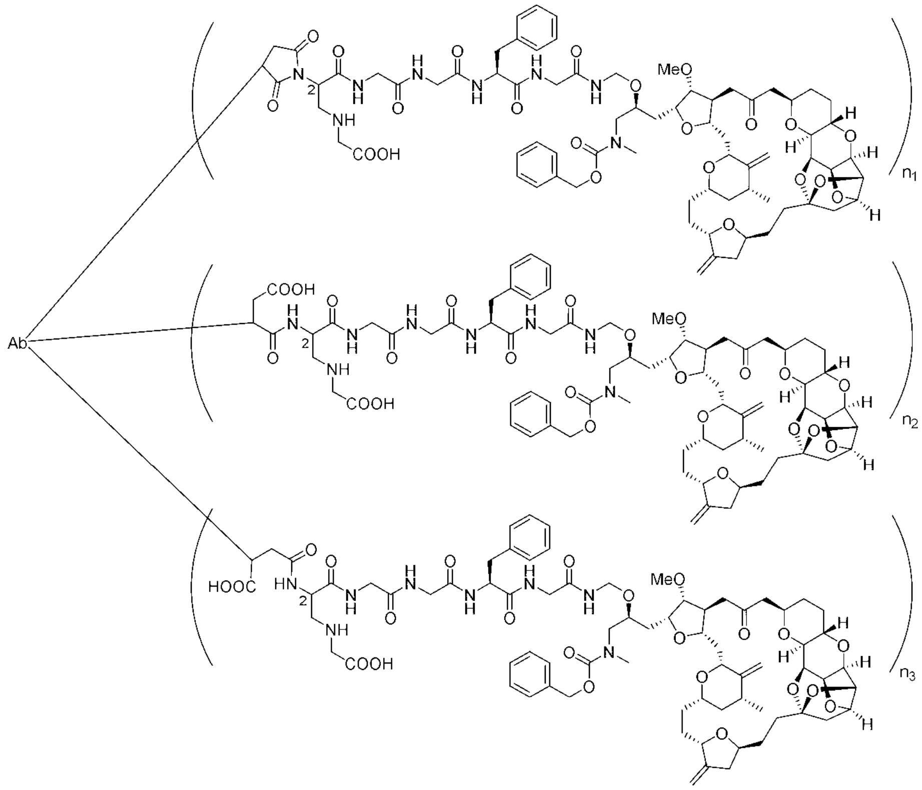

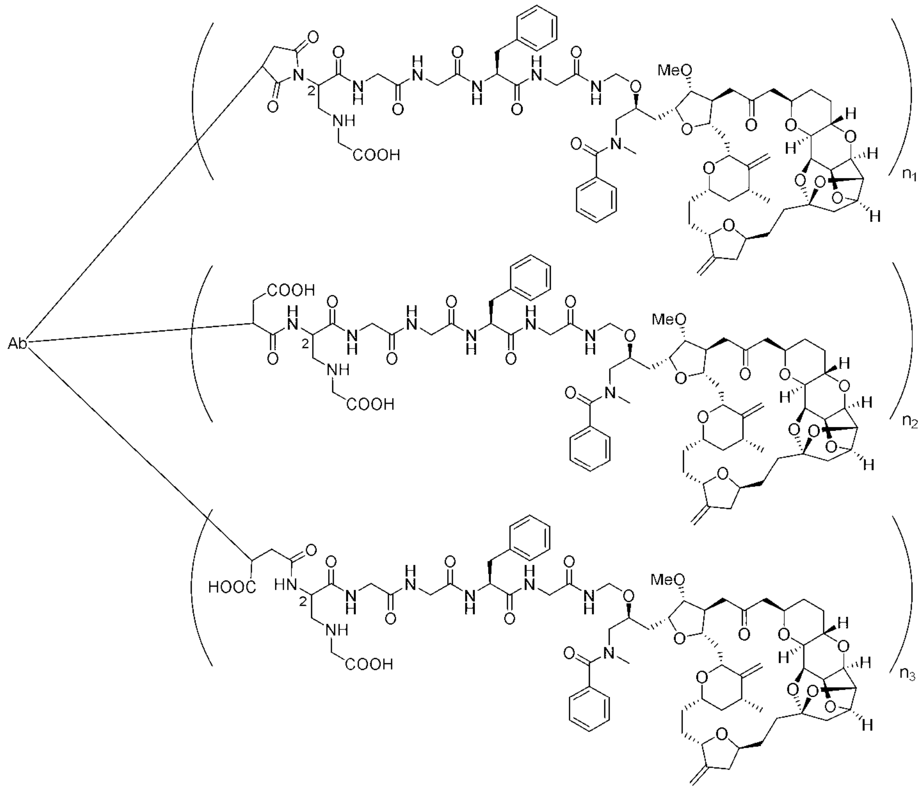

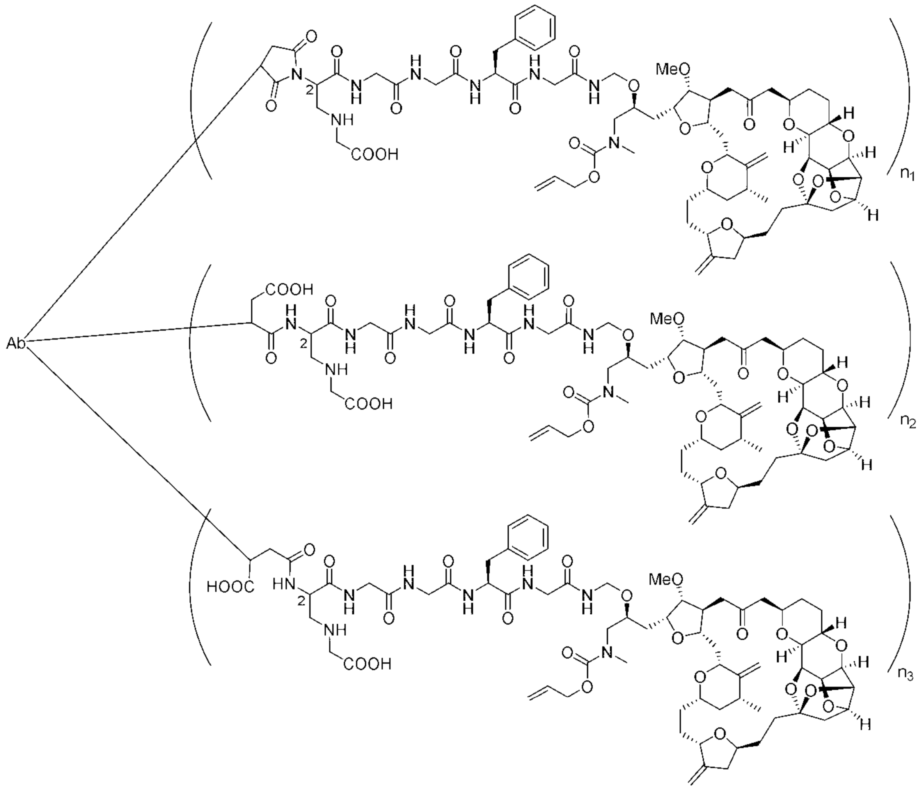

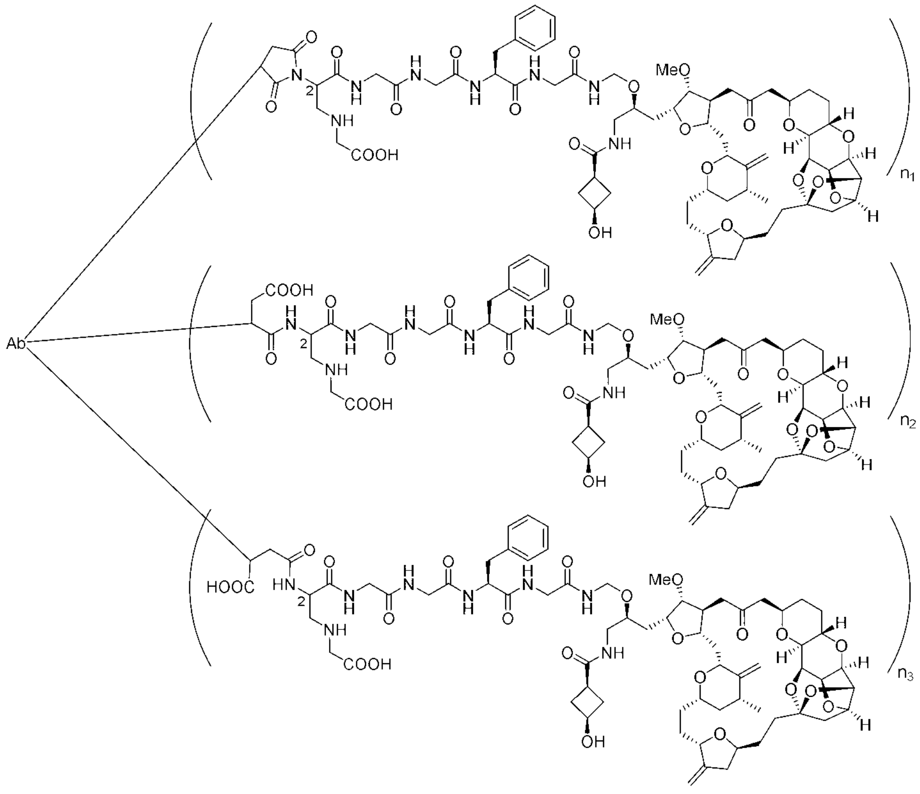

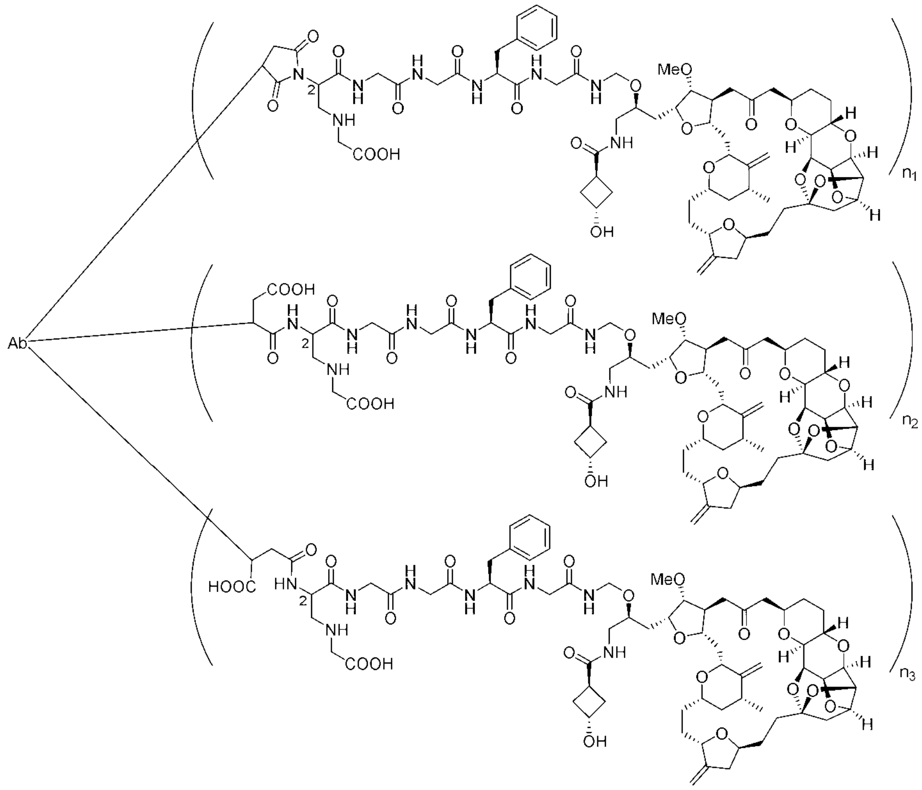

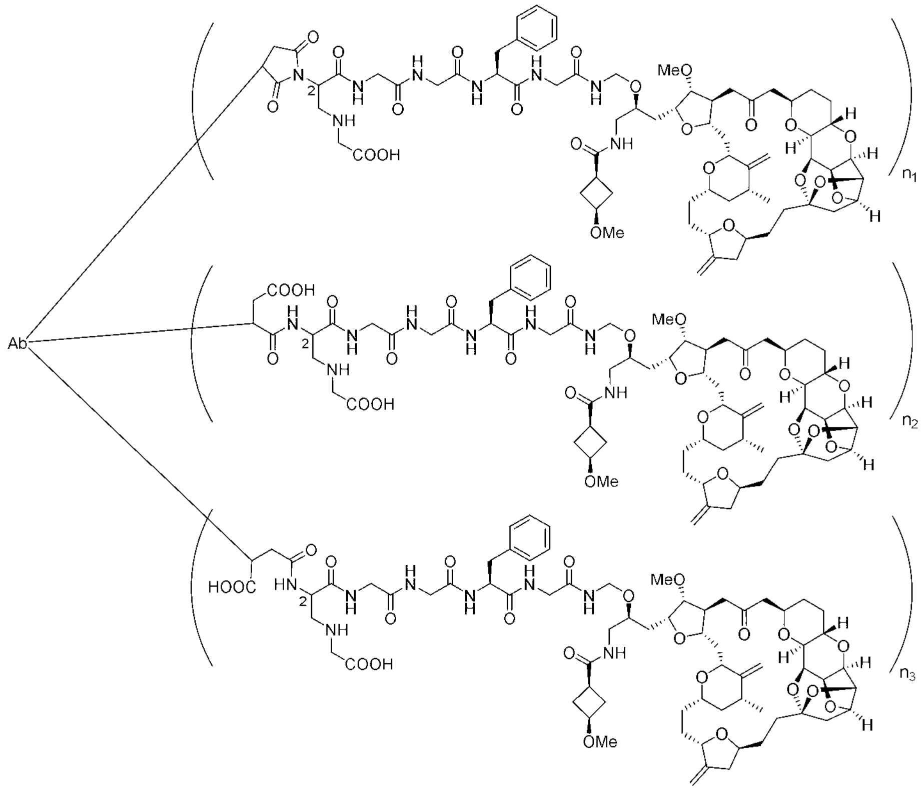

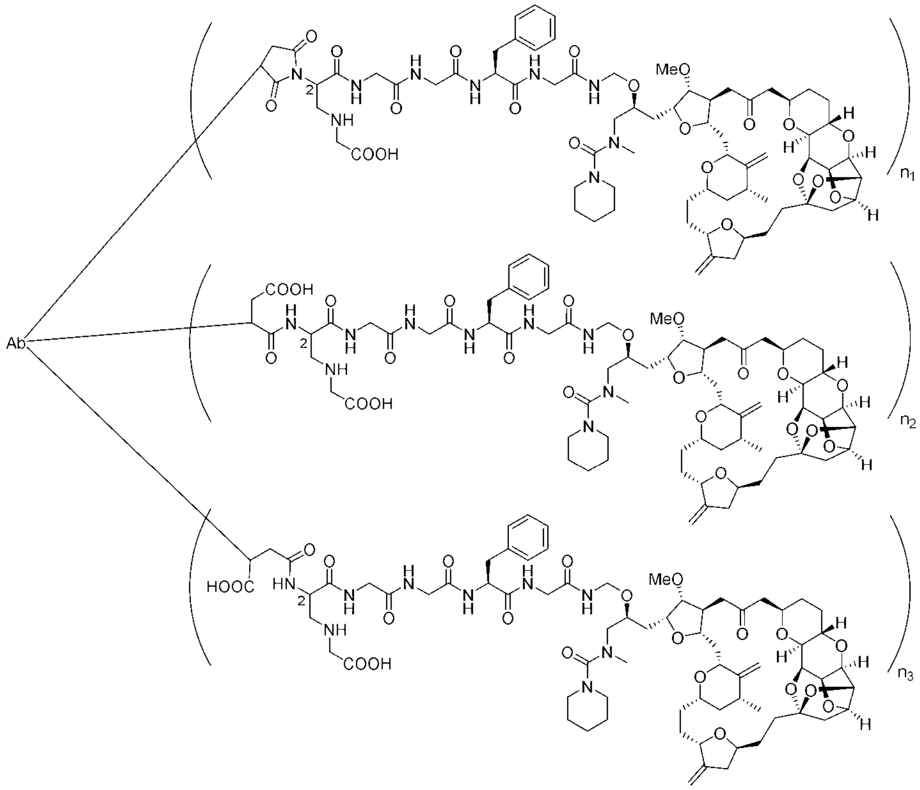

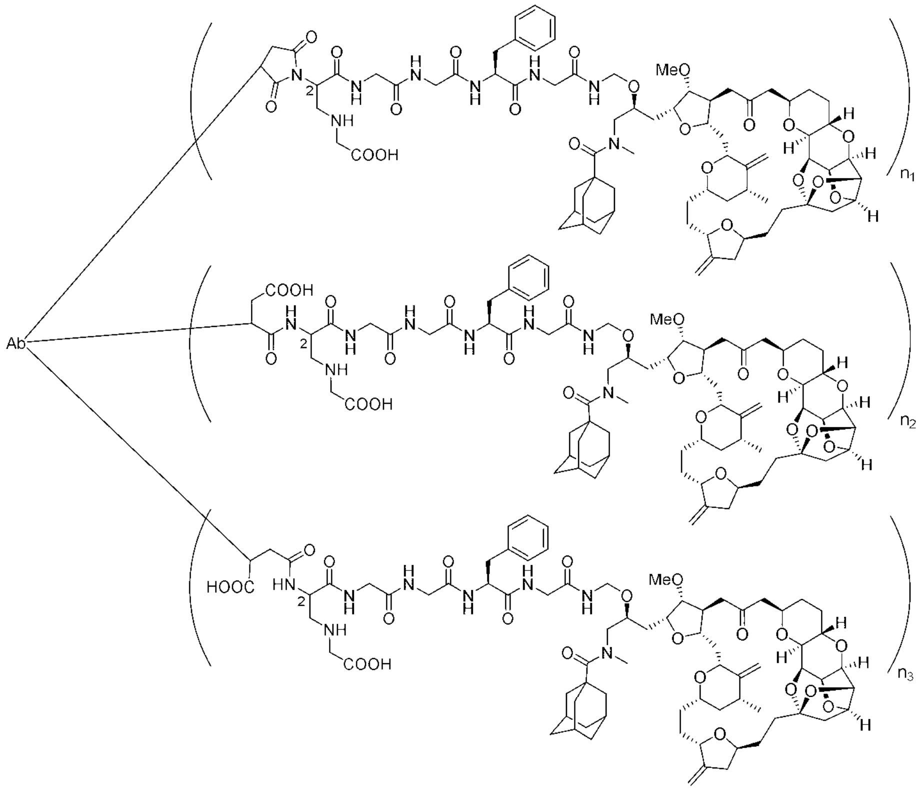

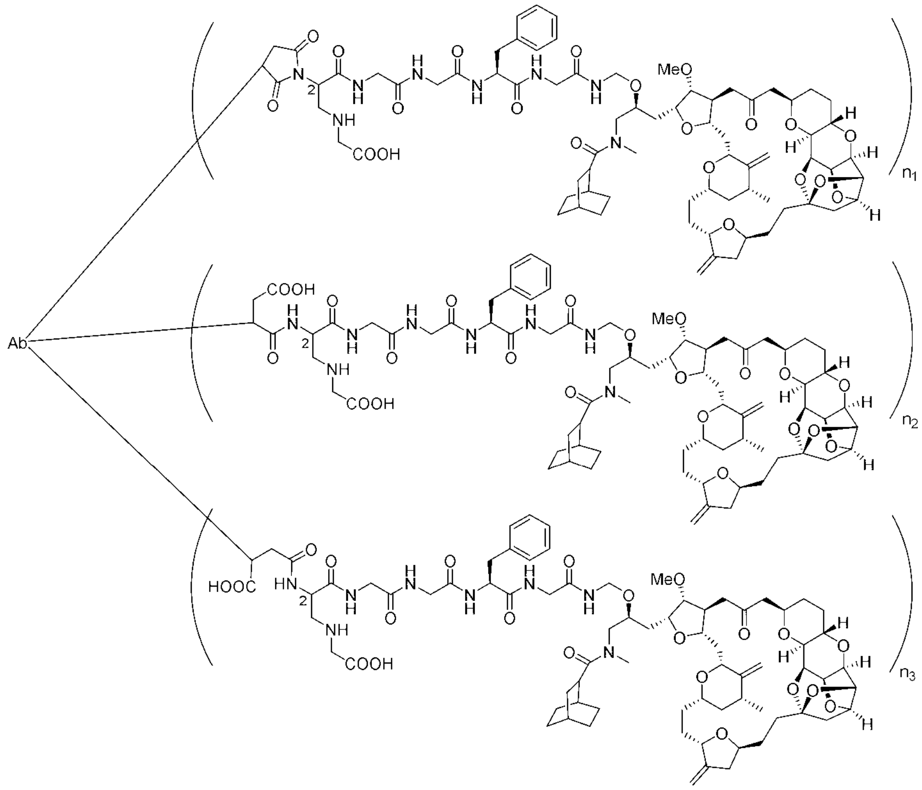

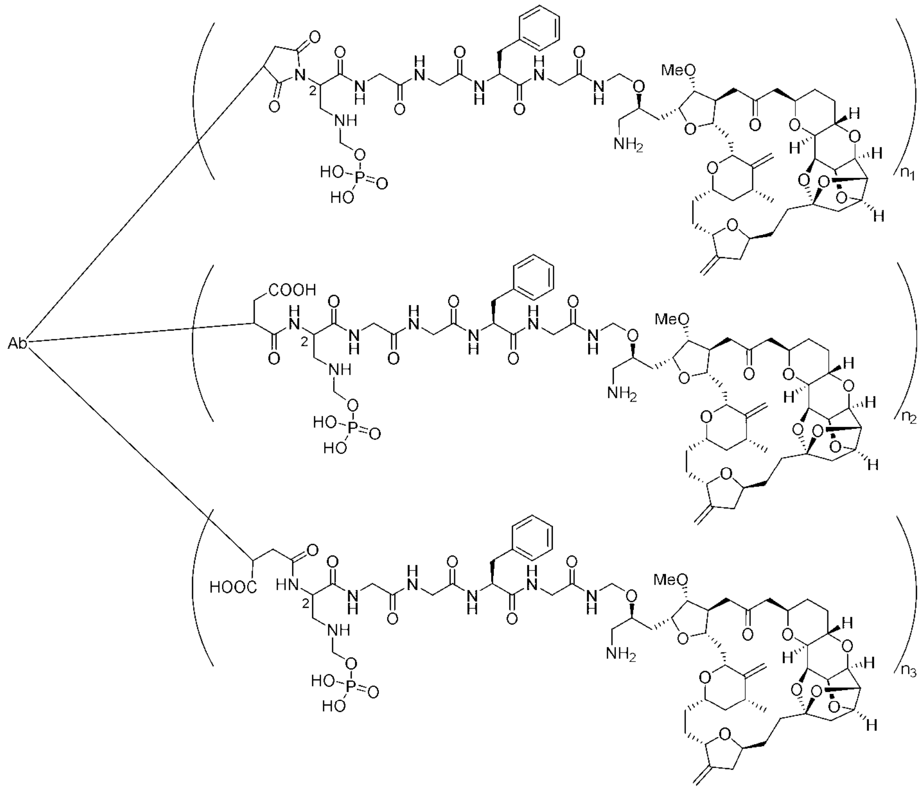

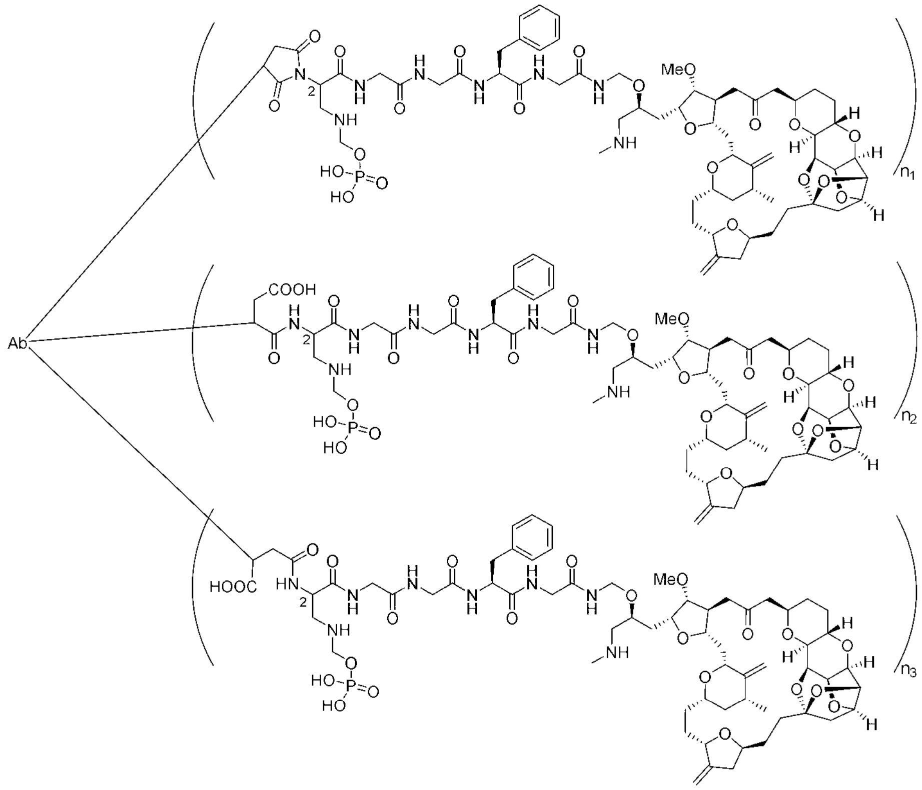

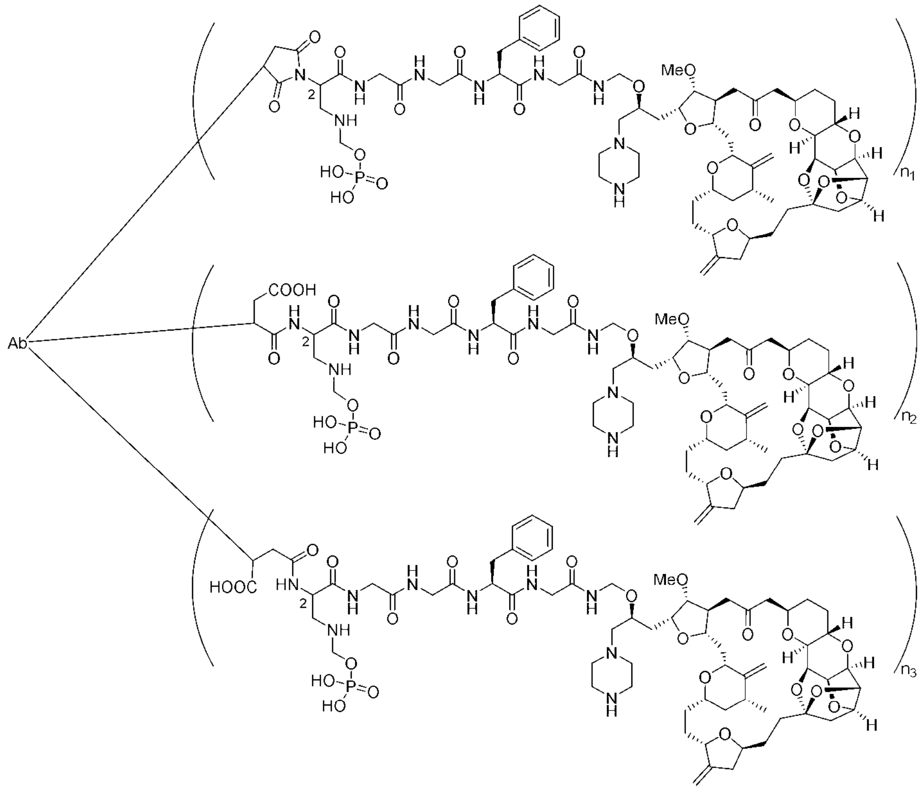

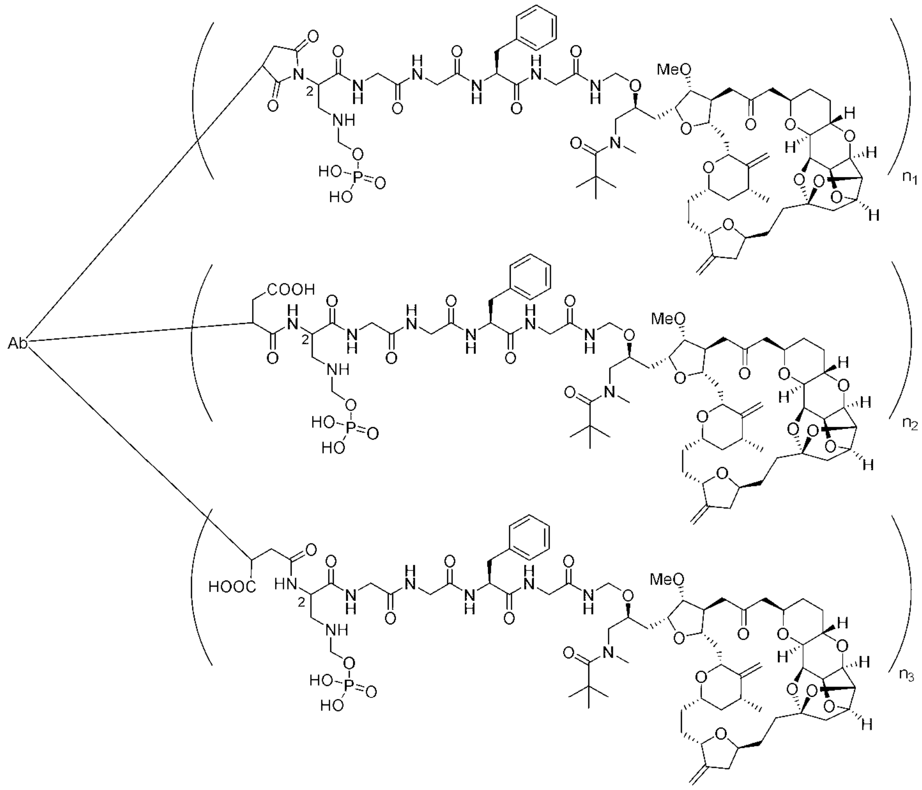

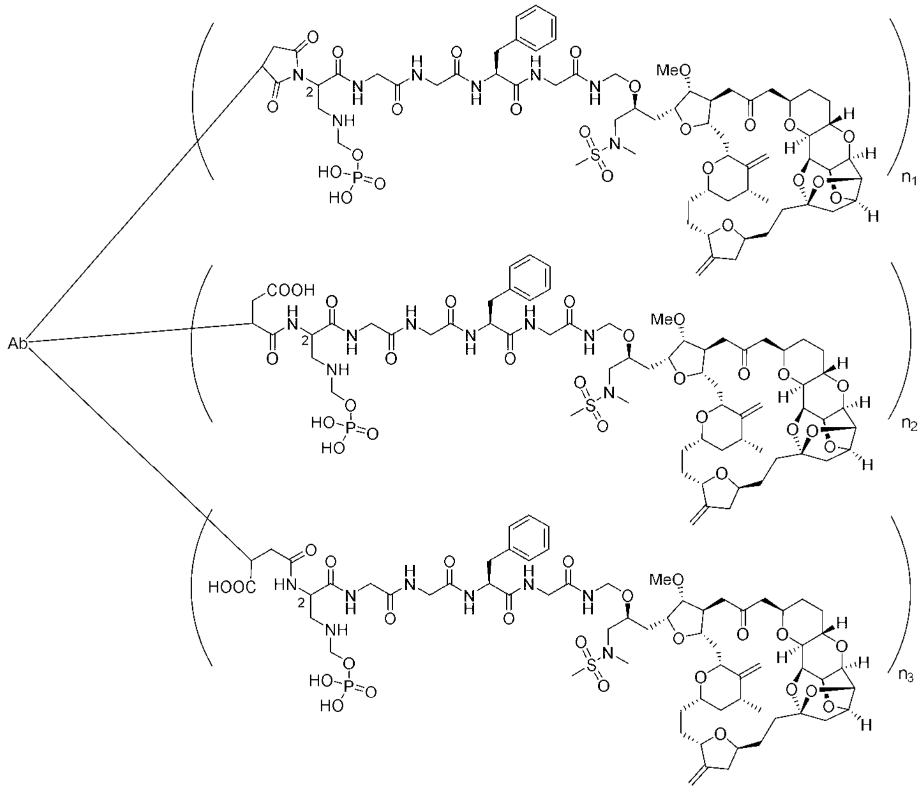

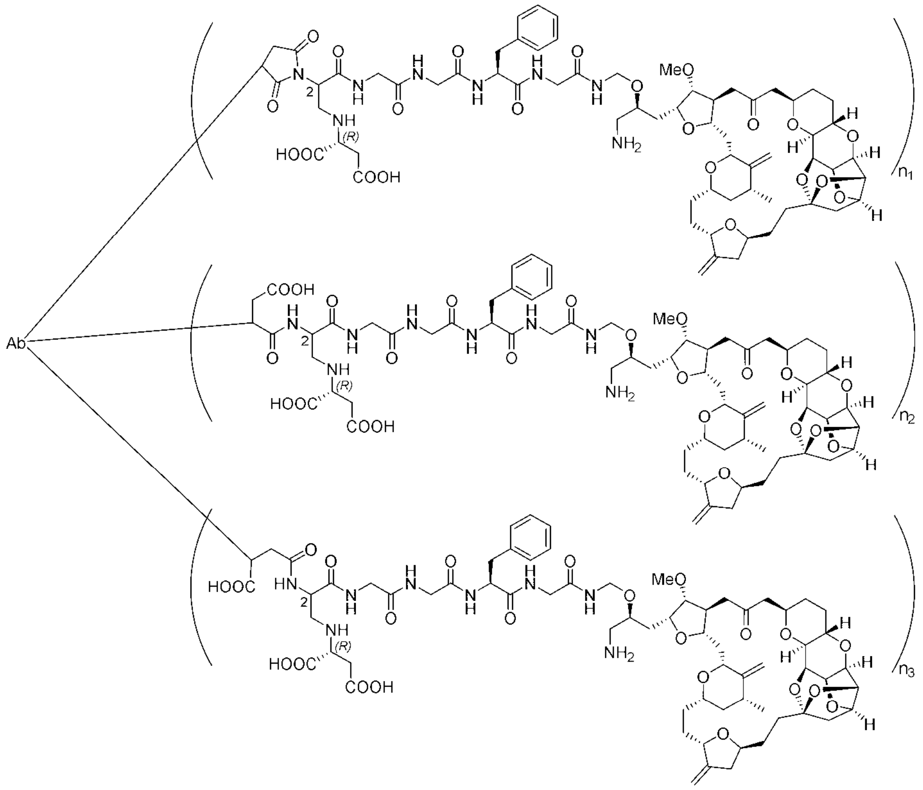

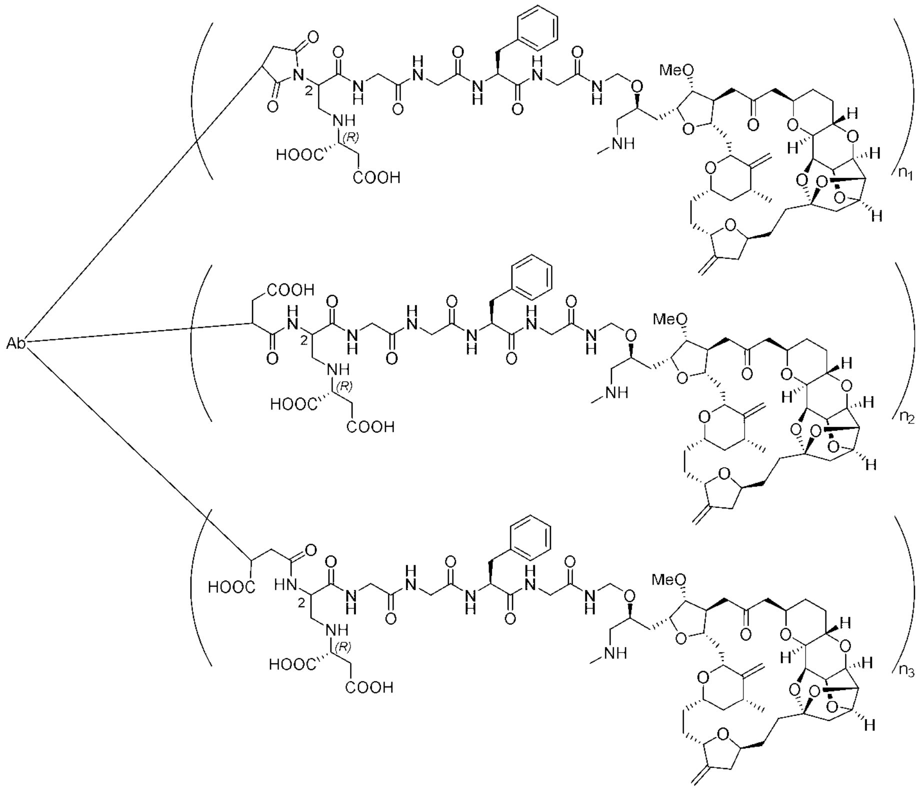

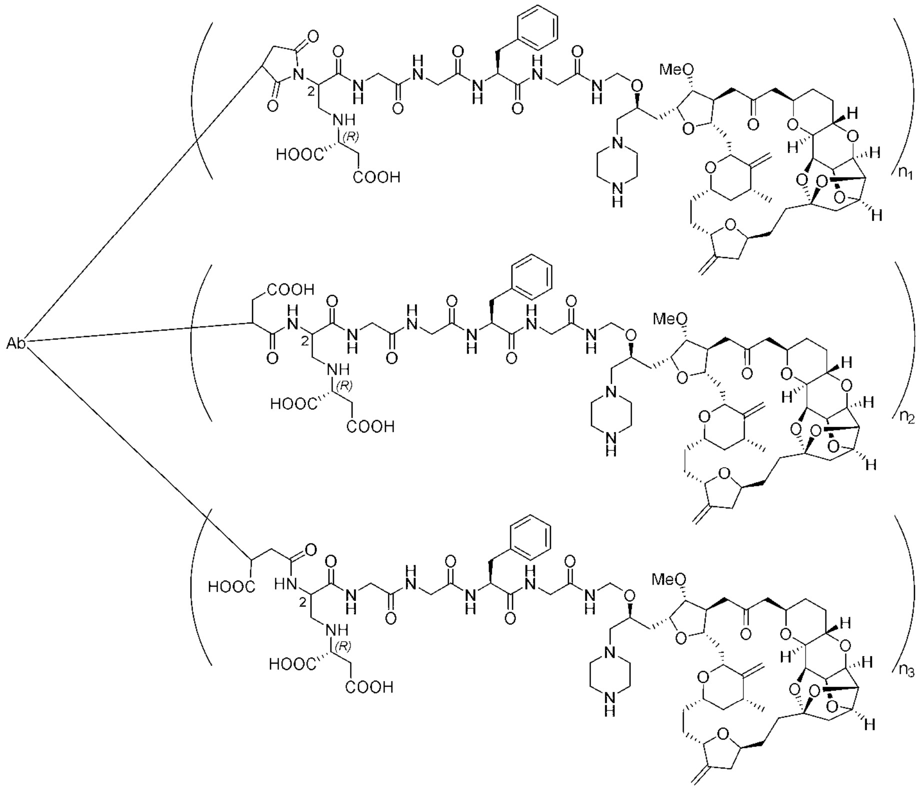

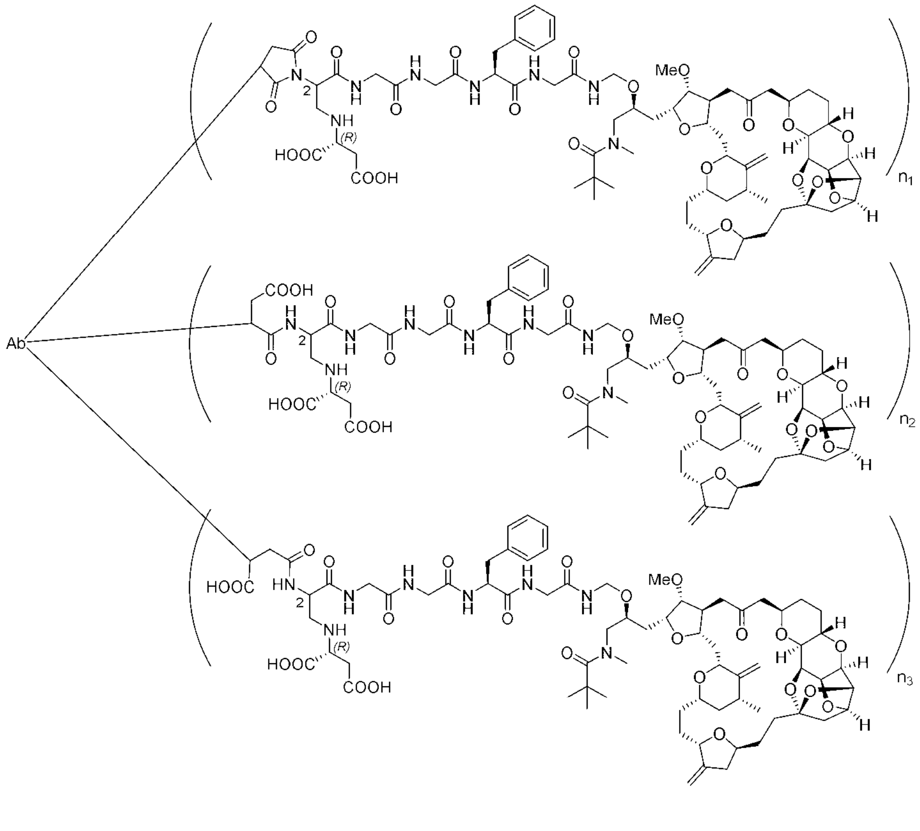

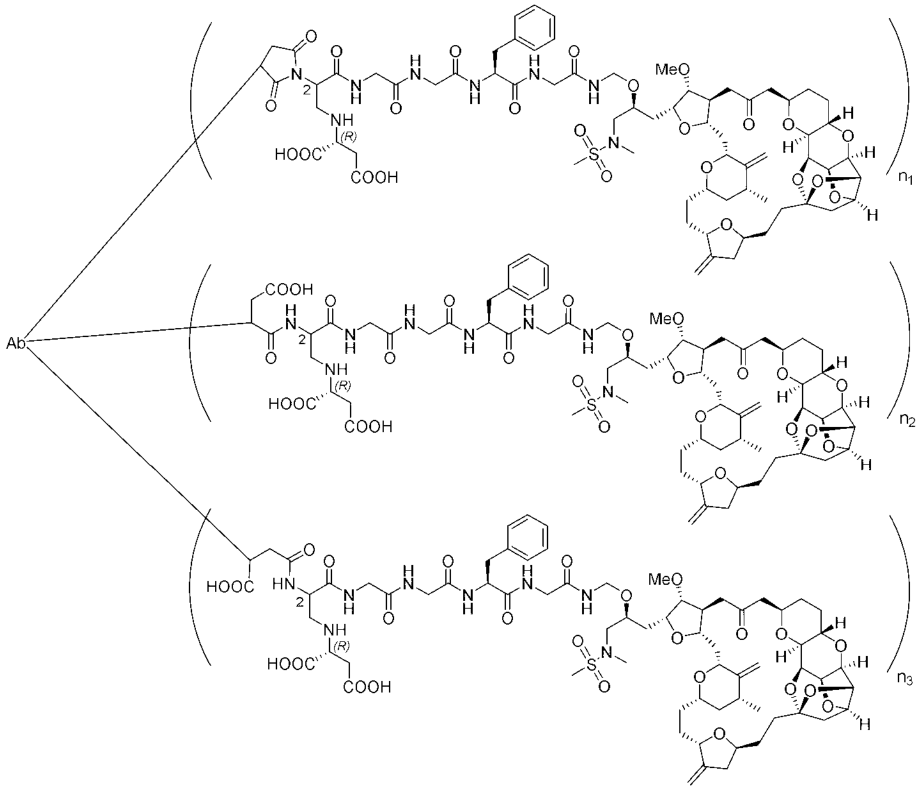

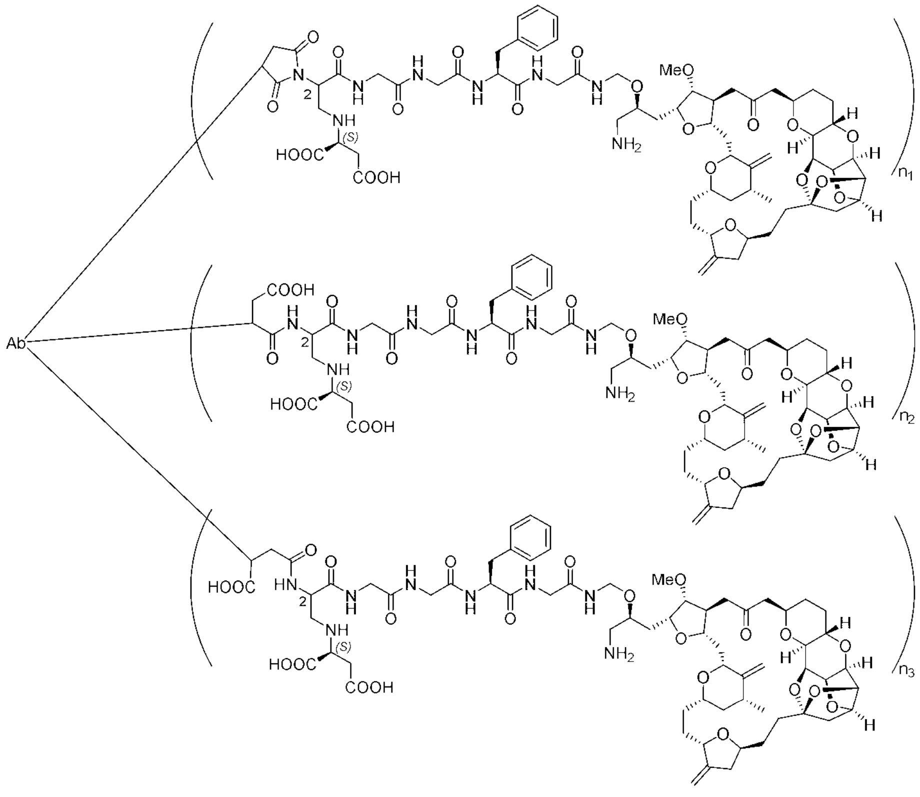

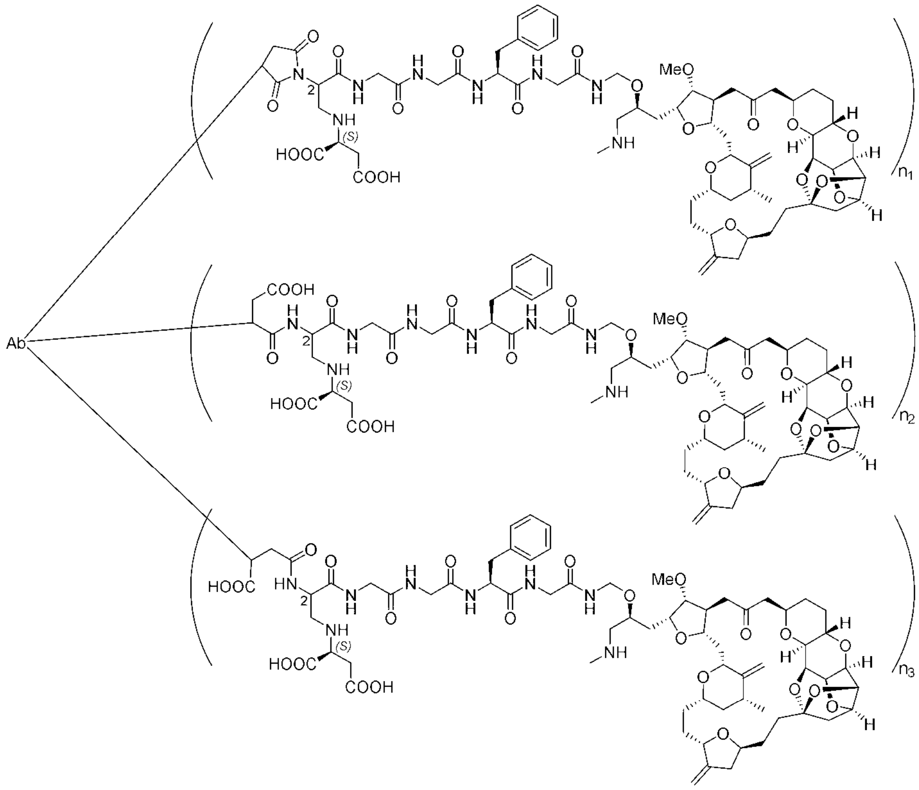

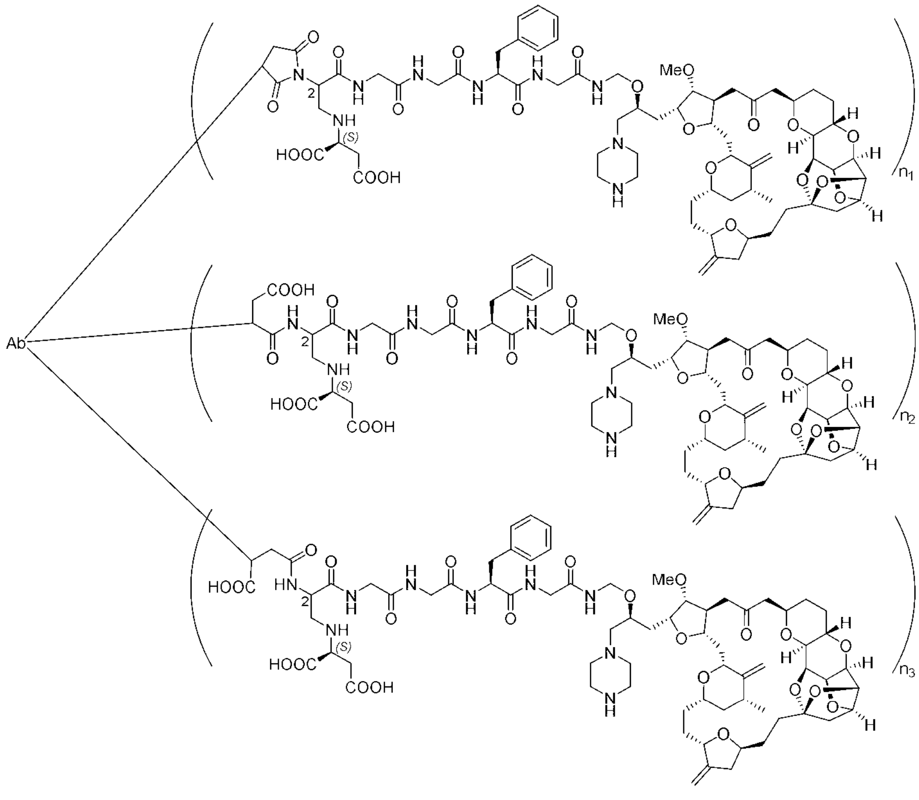

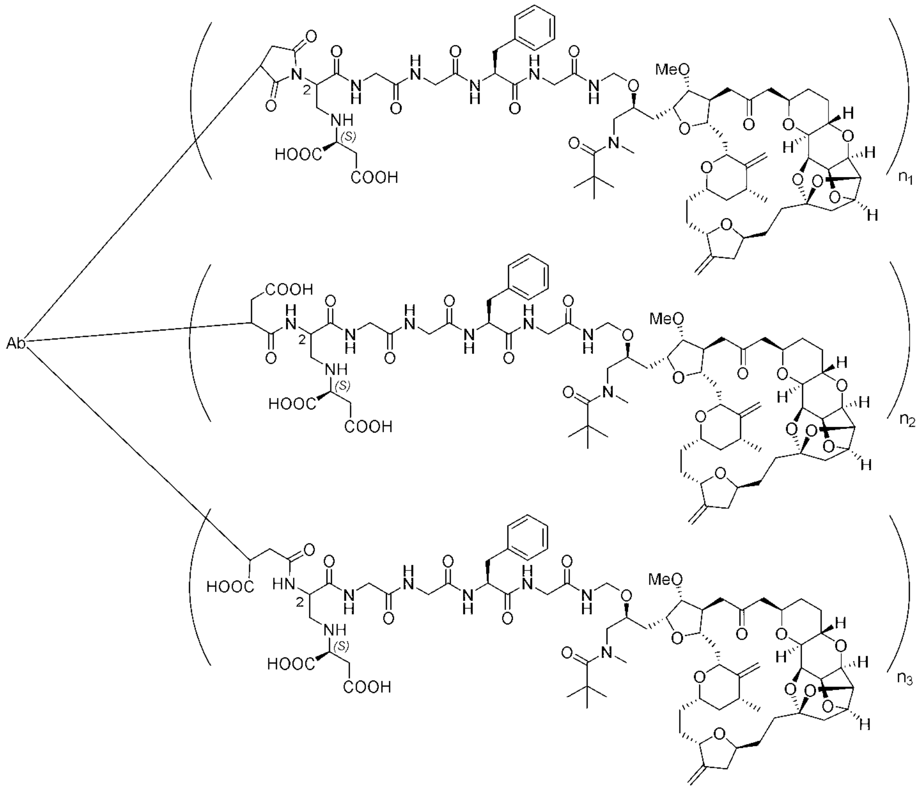

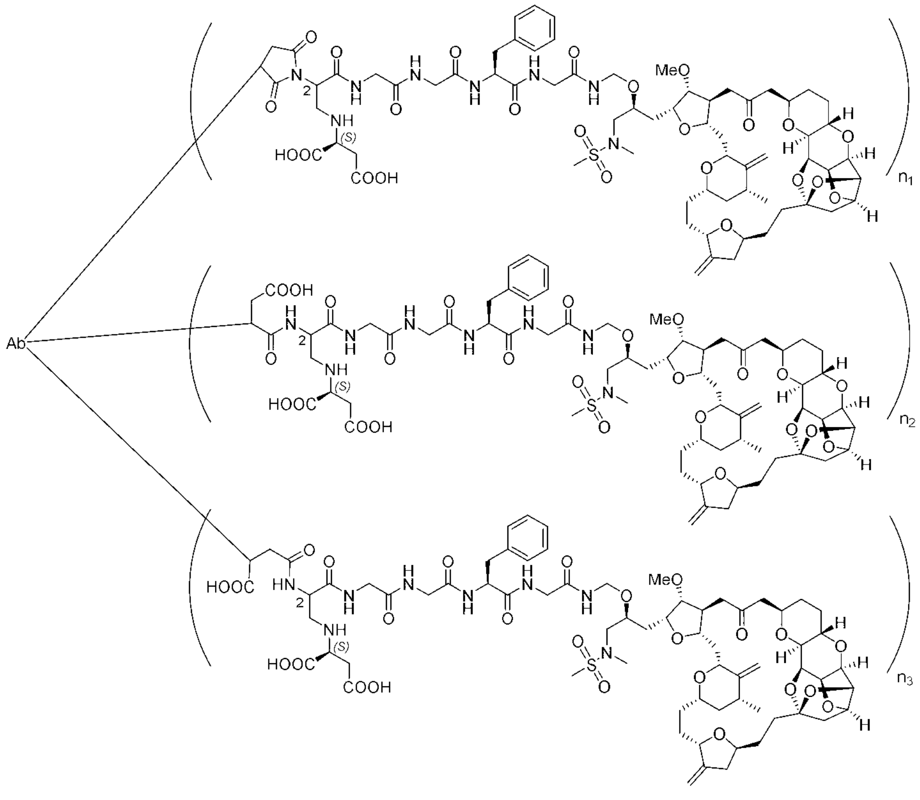

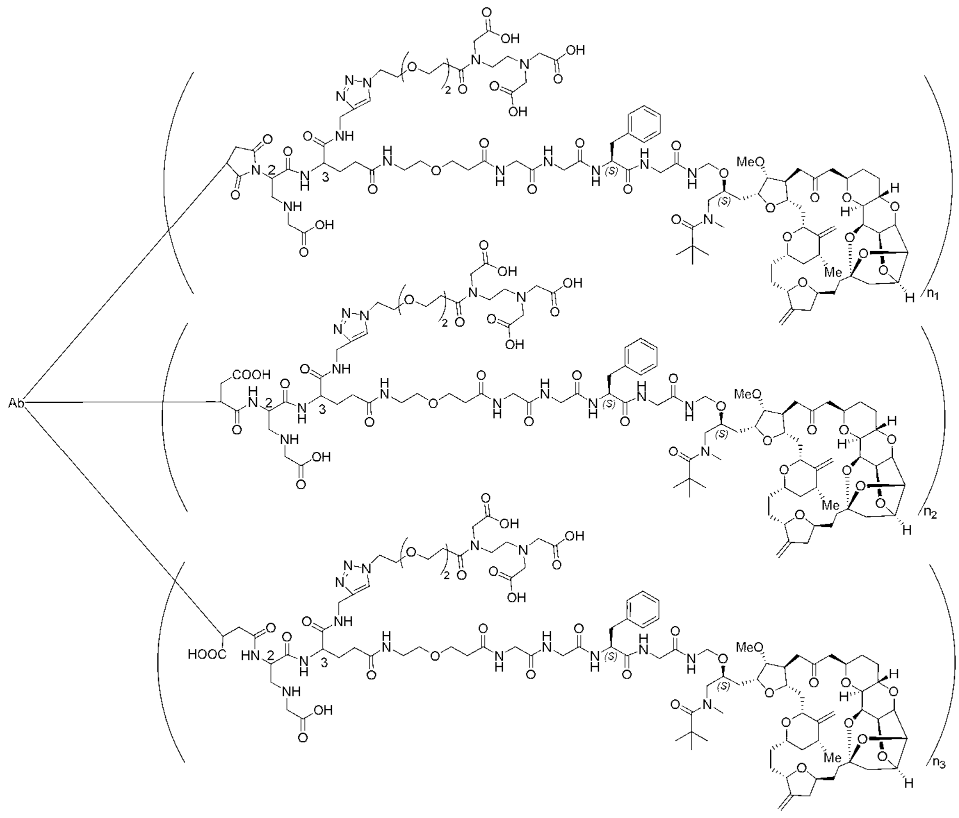

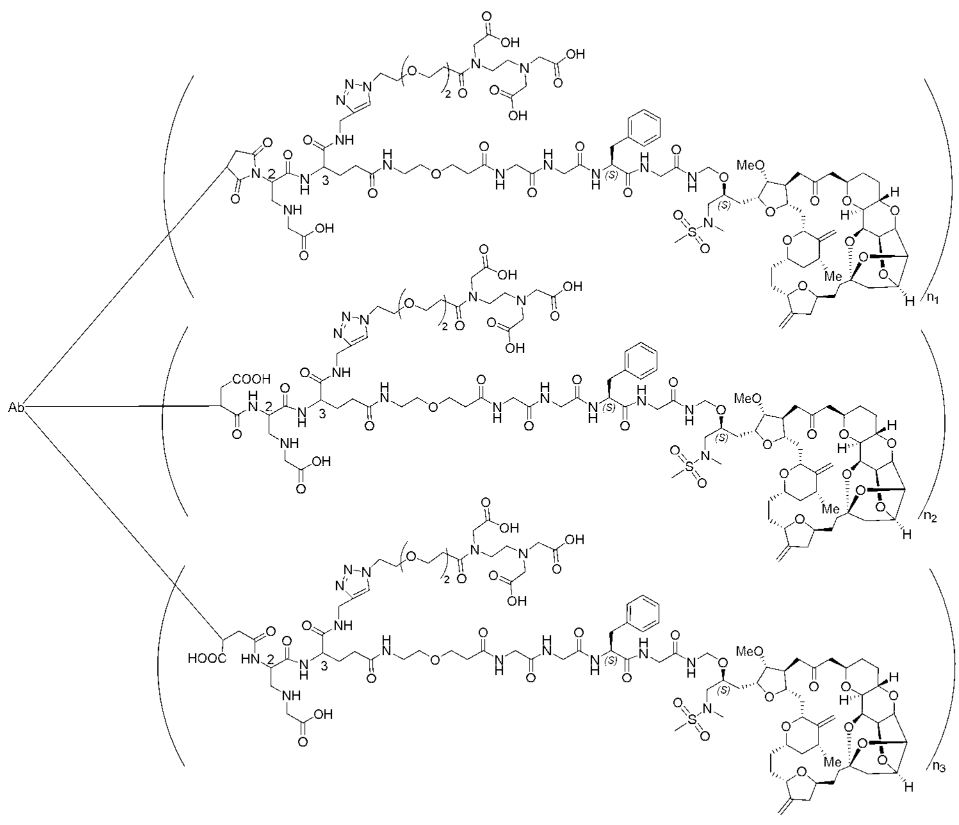

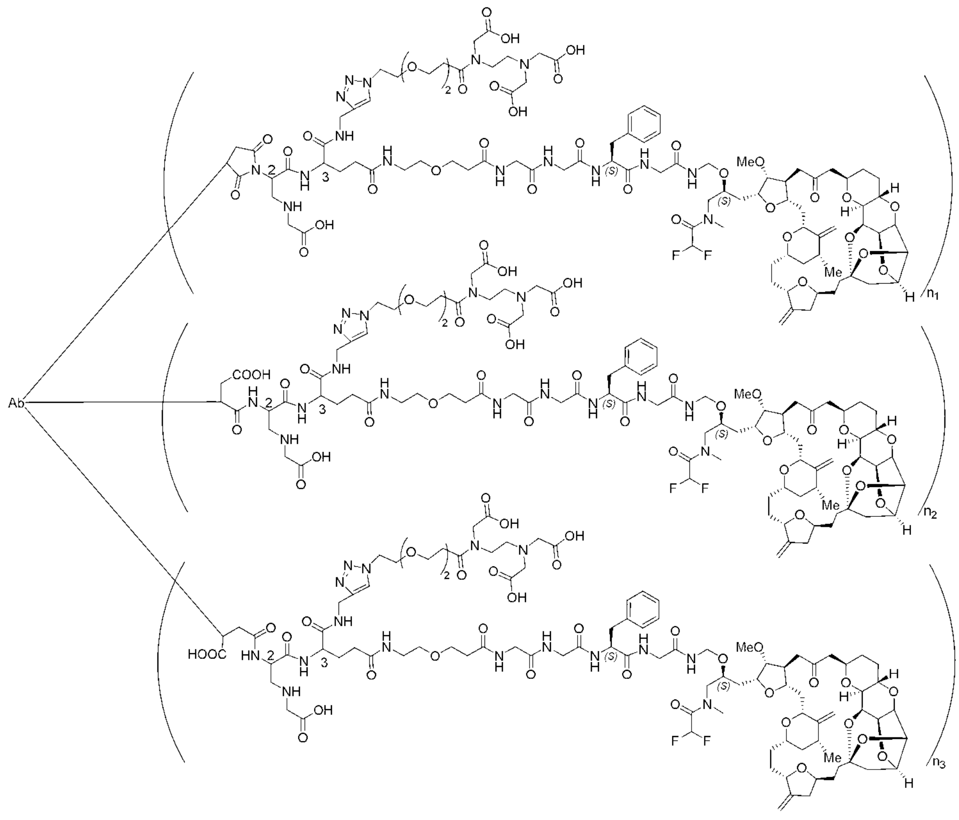

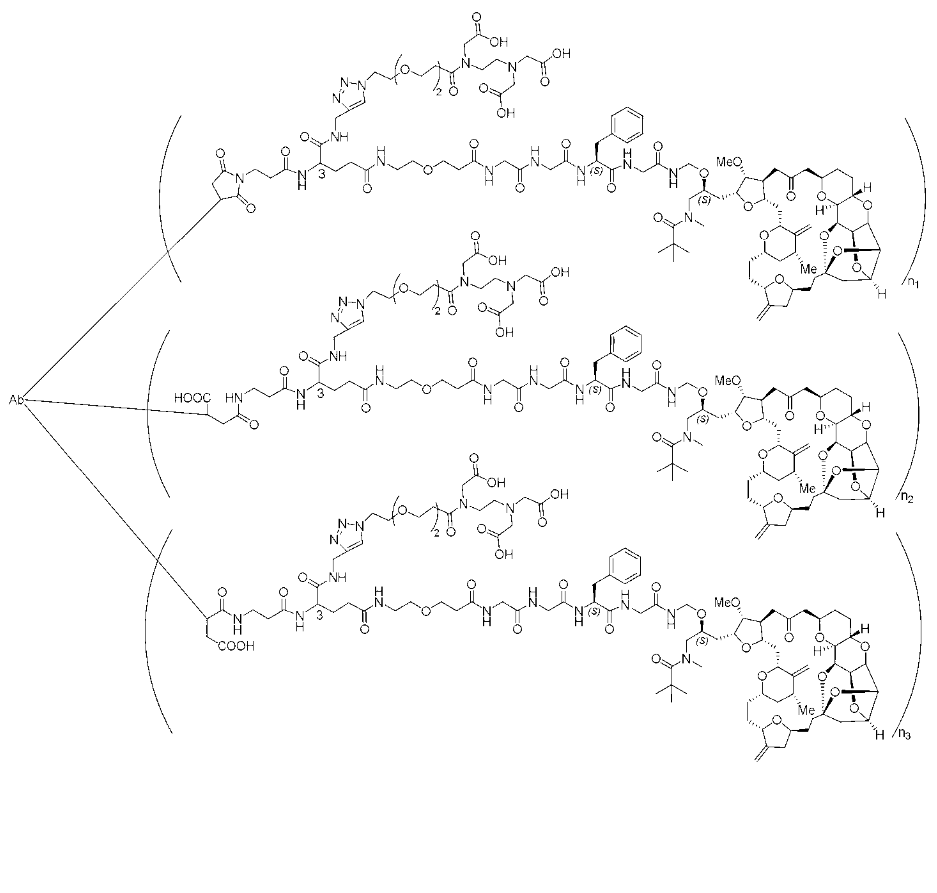

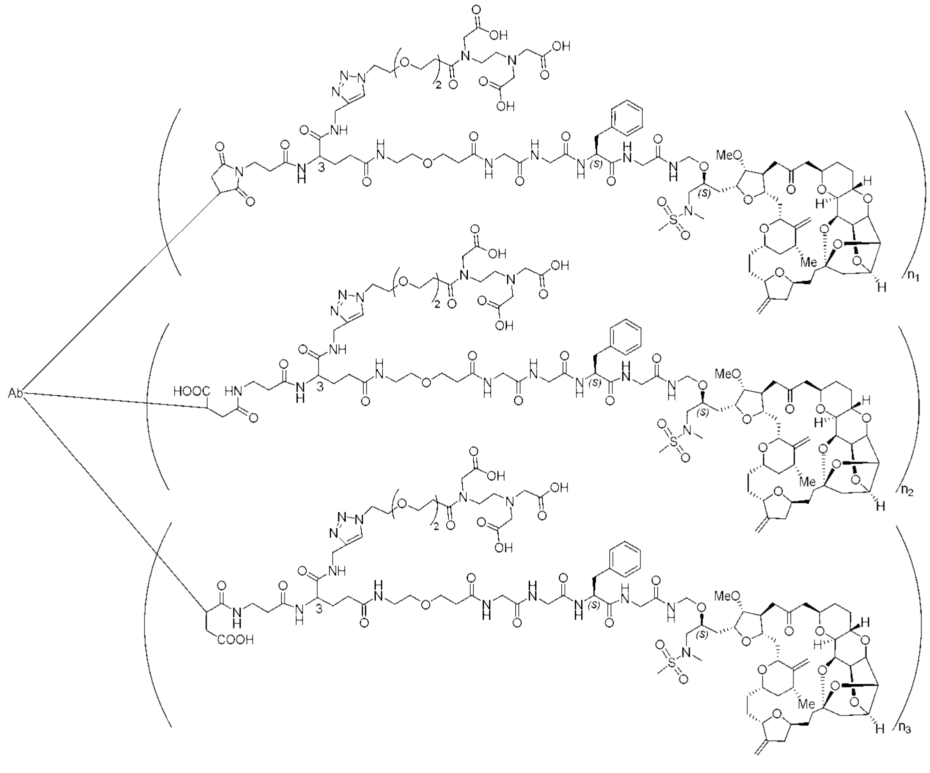

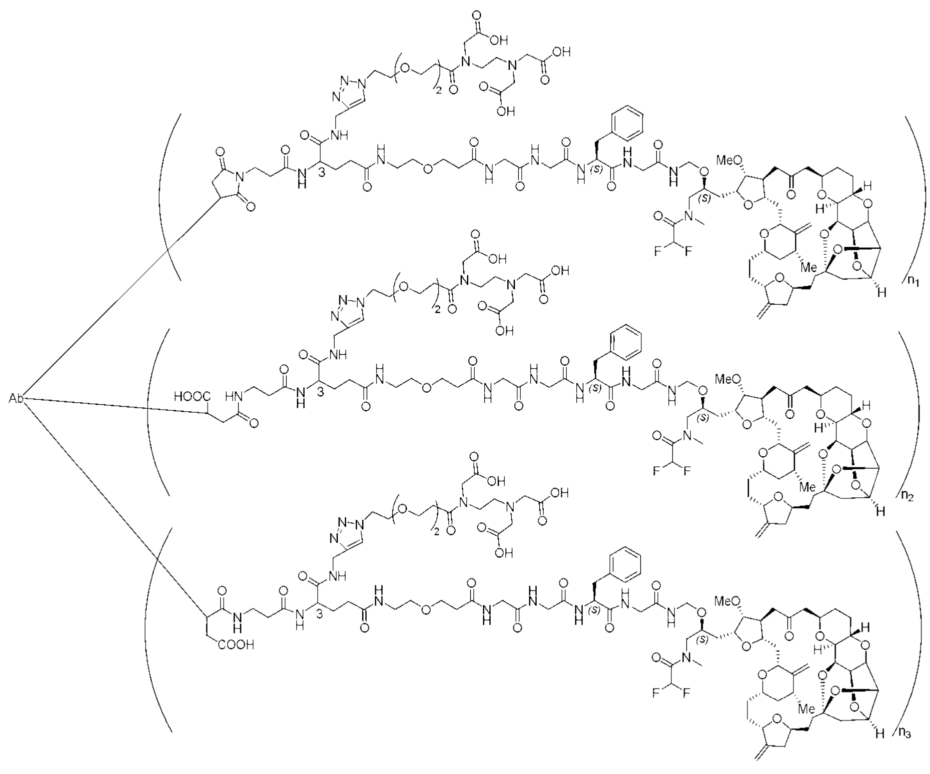

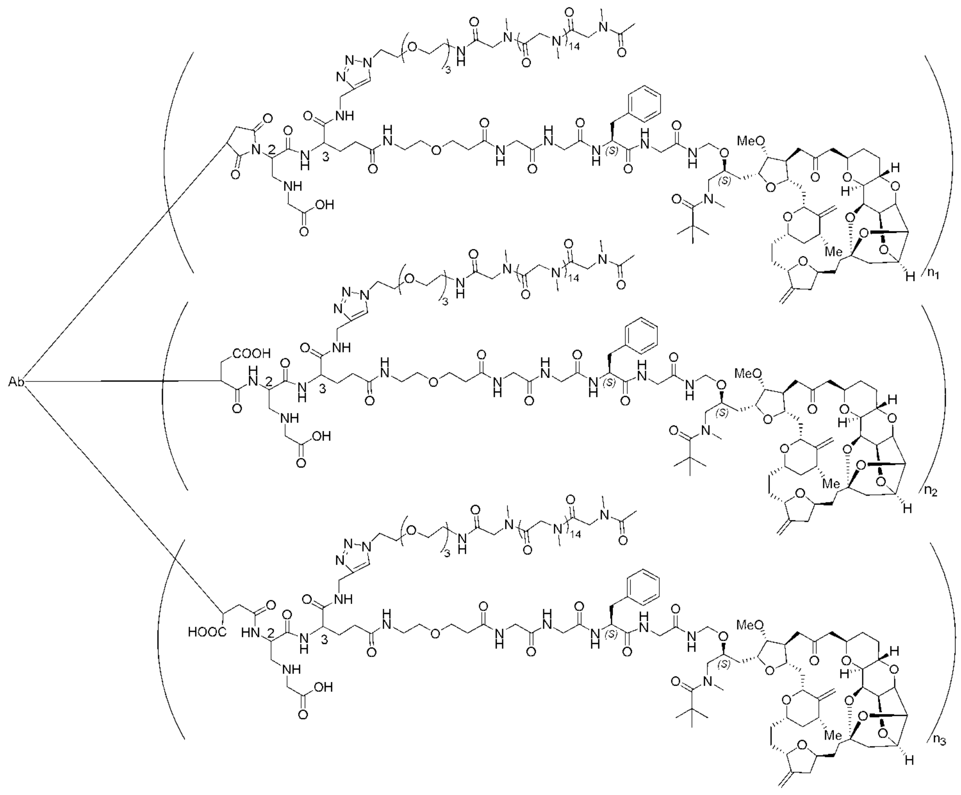

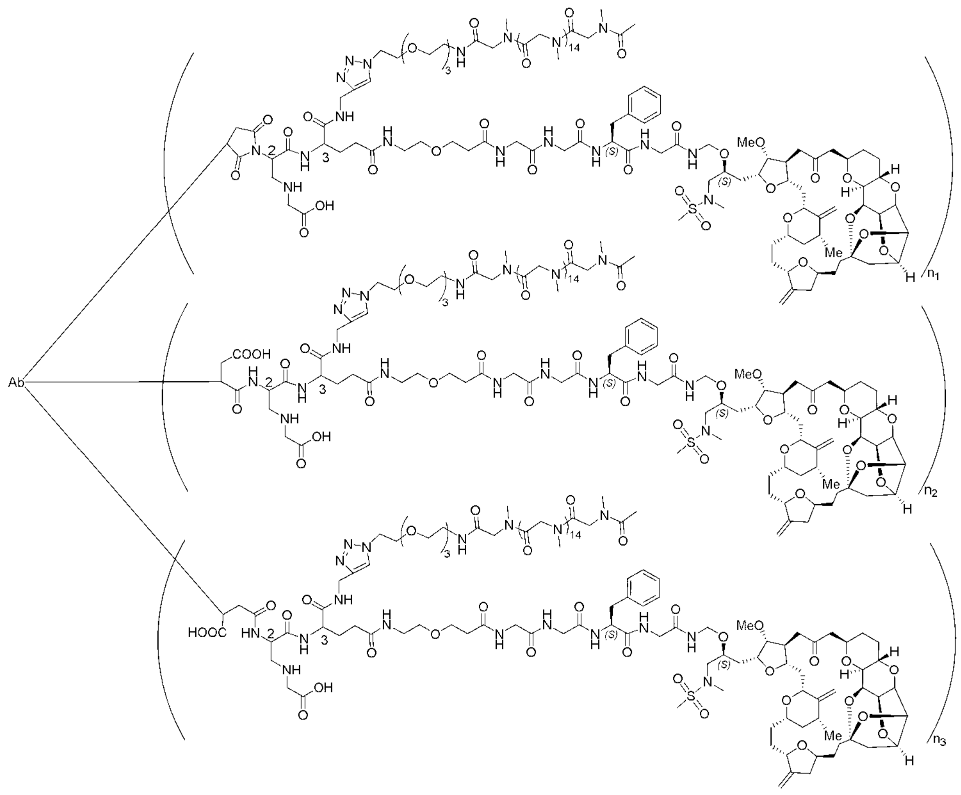

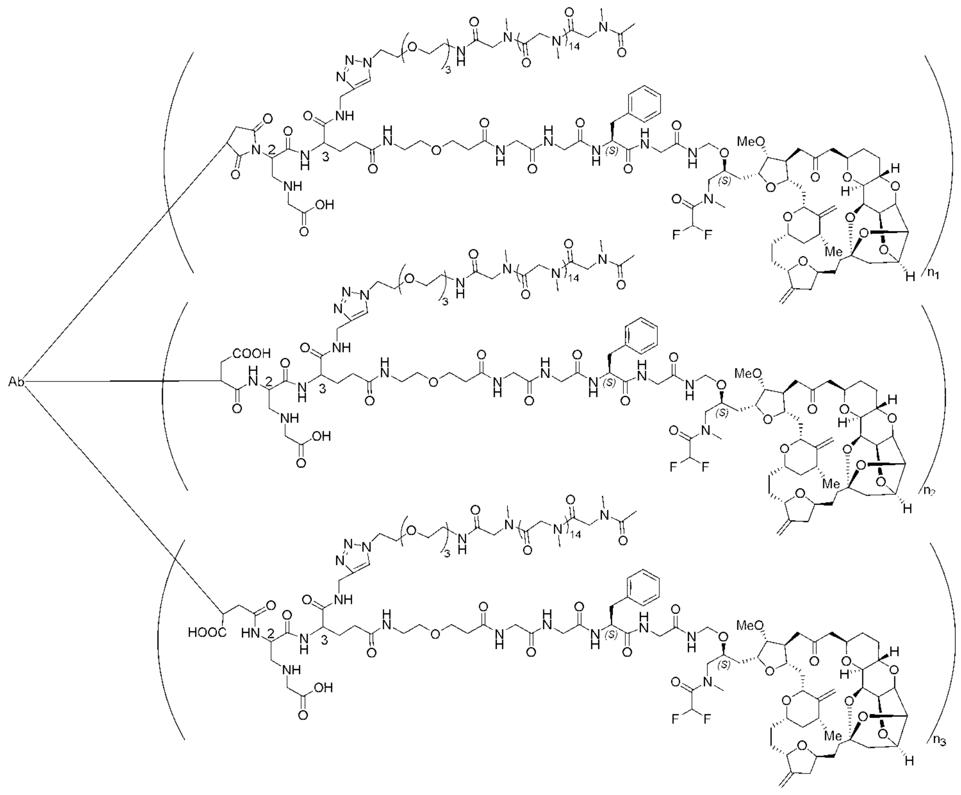

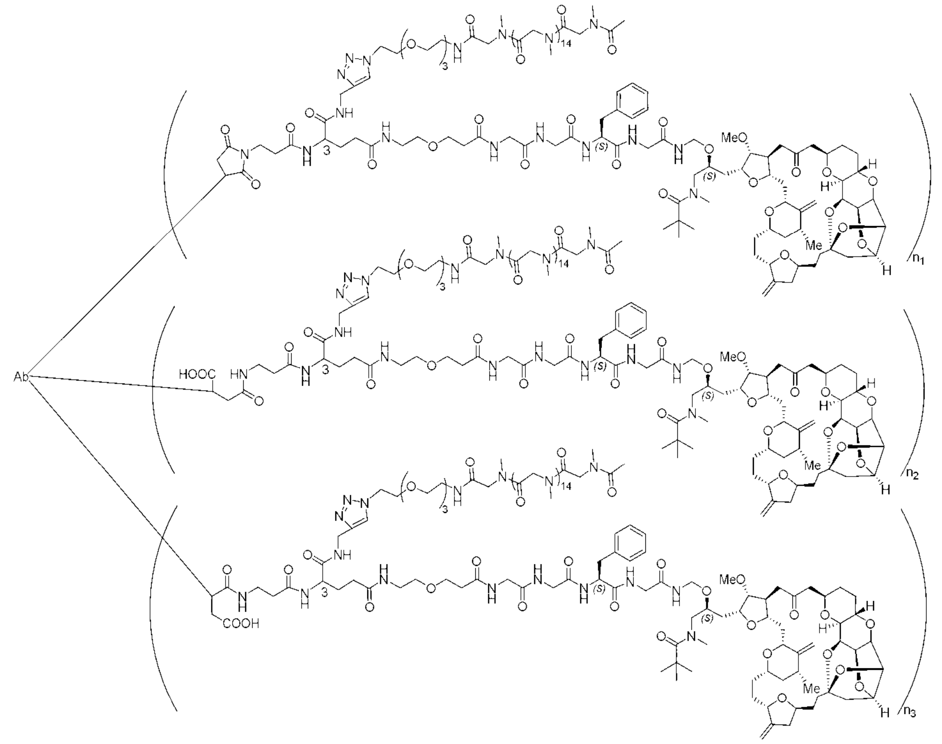

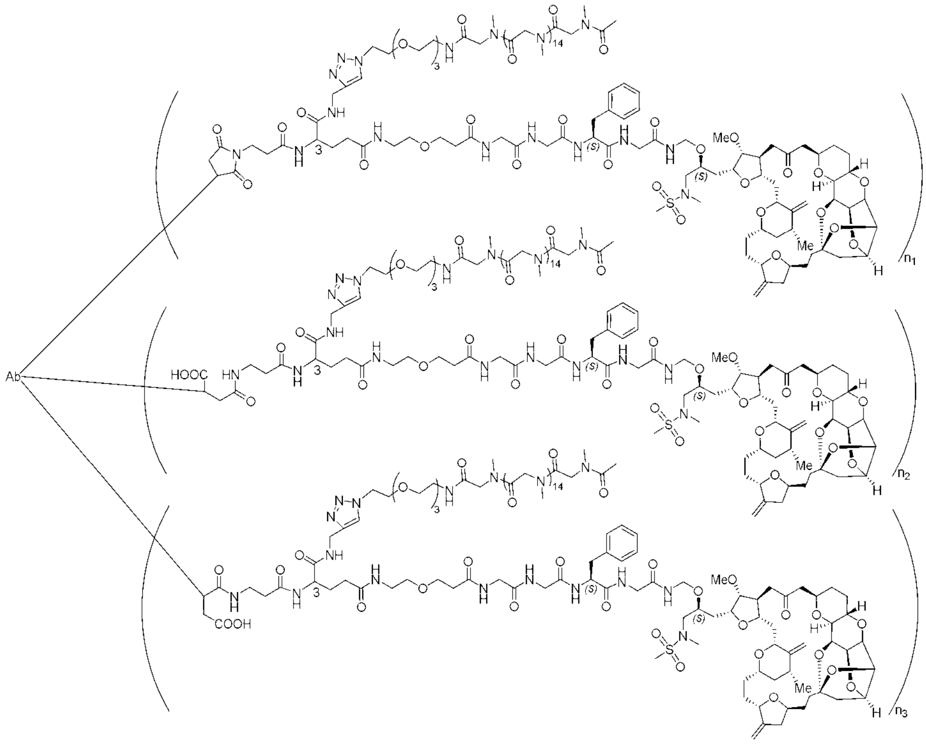

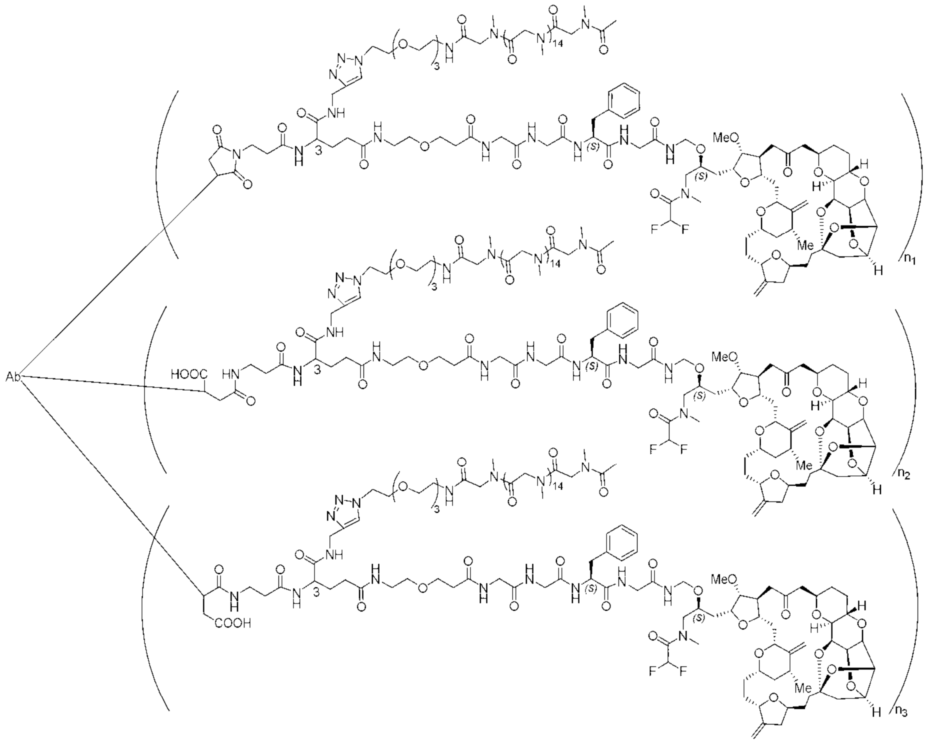

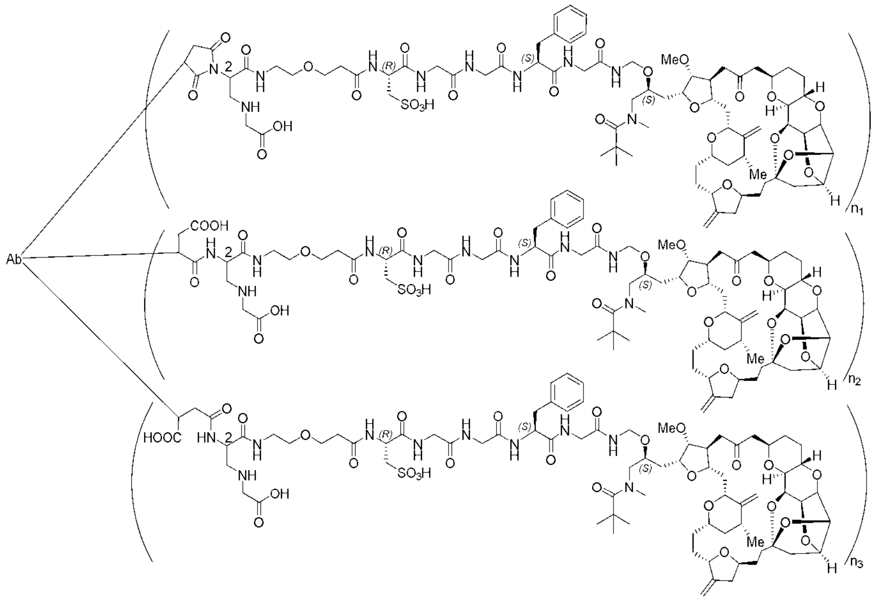

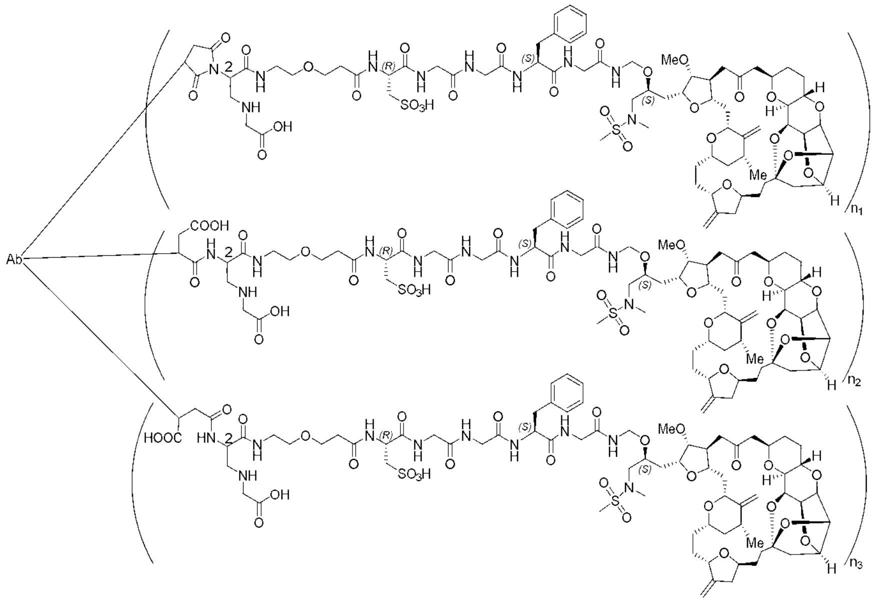

and

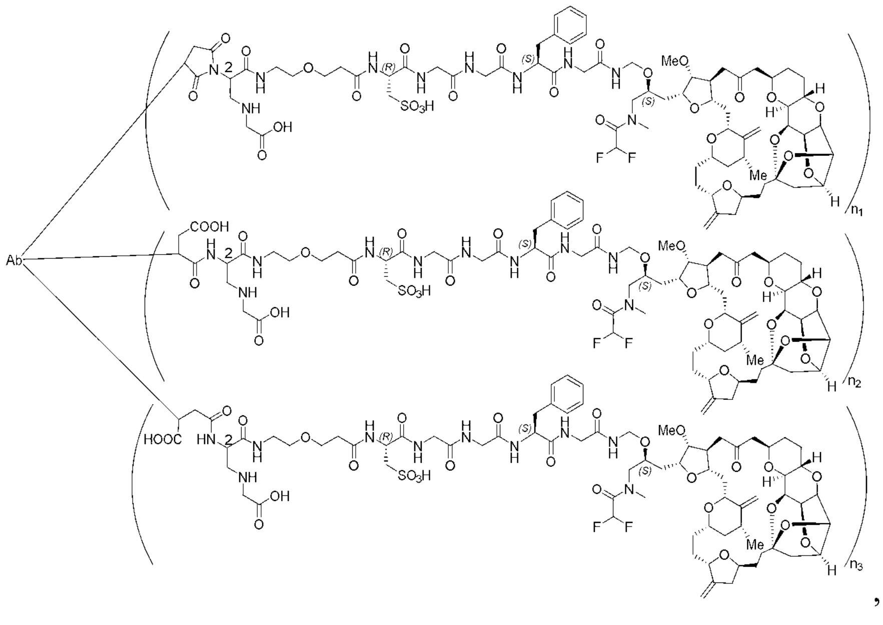

wherein chiral carbons at positions 2 and 3 independently have configuration R or S. In some embodiments, the chiral carbon at position 2 has configuration S, and the chiral carbon at position 3 has configuration S.

In the present application, the ligand unit Ab may be selected from the group consisting of an antibody, an antibody fragment or a protein; the antibody is preferably selected from the group consisting of a murine antibody, a rabbit antibody, a phage display-derived antibody, a yeast display-derived antibody, a chimeric antibody, a humanized antibody, a fully human antibody, an antibody fragment, a bispecific antibody and a multispecific antibody.

In some embodiments, the antibody is a monoclonal antibody, is selected, without limitation, from: anti-EGFRvIII antibody, anti-PD-1 antibody, anti-PD-L1 antibody, anti-DLL-3 antibody, anti-PSMA antibody, anti-CD70 antibody, anti-MUC16 antibody, anti-ENPP3 antibody, anti-TDGF1 antibody, anti-ETBR antibody, anti-MSLN antibody, anti-TIM-1 antibody, anti-LRRC15 antibody, anti-LIV-1 antibody, anti-CanAg/AFP antibody, anti-claudin 18.2 antibody, anti-Mesothelin antibody, anti-HER2(ErbB2) antibody, anti-EGFR antibody, anti-c-MET antibody, anti-SLITRK6 antibody, anti-KIT/CD117 antibody, anti-STEAP1 antibody, anti-SLAMF7/CS1 antibody, anti-NaPi2B/SLC34A2 antibody, anti-GPNMB antibody, anti-HER3(ErbB3) antibody, anti-MUC1/CD227 antibody, anti-AXL antibody, anti-CD166 antibody, anti-B7-H3(CD276) antibody, anti-PTK7/CCK4 antibody, anti-PRLR antibody, anti-EFNA4 antibody, anti-5T4 antibody, anti-NOTCH3 antibody, anti-Nectin 4 antibody, anti-TROP-2 antibody, anti-CD142 antibody, anti-CA6 antibody, anti-GPR20 antibody, anti-CD174 antibody, anti-CD71 antibody, anti-EphA2 antibody, anti-LYPD3 antibody, anti-FGFR2 antibody, anti-FGFR3 antibody, anti-FRα antibody, anti-CEACAMs antibody, anti-GCC antibody, anti-Integrin Av antibody, anti-CAIX antibody, anti-P-cadherin antibody, anti-GD3 antibody, anti-Cadherin 6 antibody, anti-LAMP1 antibody, anti-FLT3 antibody, anti-BCMA antibody, anti-CD79b antibody, anti-CD19 antibody, anti-CD33 antibody, anti-CD56 antibody, anti-CD74 antibody, anti-CD22 antibody, anti-CD30 antibody, anti-CD37 antibody, anti-CD47 antibody, anti-CD138 antibody, anti-CD352 antibody, anti-CD25 antibody or anti-CD123 antibody.

In some embodiments, the antibody is anti-TROP-2 antibody. In some embodiments, the antibody comprises heavy chains and light chains, the light chains comprise CDR-L1, CDR-L2 and CDR-L3 having amino acid sequences set forth in SEQ ID NO: 1, SEQ ID NO:2, and SEQ ID NO:3. In some embodiments, the heavy chains comprise CDR-H1, CDR-H2 and CDR-H3 having amino acid sequences set forth in SEQ ID NO:4, SEQ ID NO:5, and SEQ ID NO:6.

In some embodiments, the light chain comprises a light chain variable region having an amino acid sequence of SEQ ID NO:7. In some embodiments, the light chain further comprises a light chain constant region having an amino acid sequence of SEQ ID NO:8. In some embodiments, the amino acid sequence of the light chain is SEQ ID NO:9.

In some embodiments, the light chain comprises a light chain variable region having an amino acid sequence of SEQ ID NO:10. In some embodiments, the light chain further comprises a light chain constant region having an amino acid sequence of SEQ ID NO:11. In some embodiments, the amino acid sequence of the light chain is SEQ ID NO:12.

In some embodiments, the heavy chain comprises a heavy chain variable region having an amino acid sequence of SEQ ID NO:13. In some embodiments, the heavy chain further comprises a heavy chain constant region having an amino acid sequence of SEQ ID NO:14. In some embodiments, the amino acid sequence of the heavy chain is SEQ ID NO: 15.

In some embodiments, the antibody is anti-TROP-2 antibody. In some embodiments, the antibody comprises heavy chains and light chains, the light chains comprise CDR-L1, CDR-L2 and CDR-L3 having nucleic acid coding

sequences set forth in SEQ ID NO:16, SEQ ID NO:17, and SEQ ID NO:18. In some embodiments, the heavy chains comprise CDR-H1, CDR-H2 and CDR-H3 having nucleic acid coding sequences set forth in SEQ ID NO:19, SEQ ID NO:20, and SEQ ID NO:21.

In some embodiments, the light chain comprises a light chain variable region having a nucleic acid coding sequence of SEQ ID NO:22. In some embodiments, the light chain further comprises a light chain constant region having a nucleic acid coding sequence of SEQ ID NO:23. In some embodiments, the nucleic acid coding sequence of the light chain is SEQ ID NO:24.

In some embodiments, the light chain comprises a light chain variable region having a nucleic acid coding sequence of SEQ ID NO:25. In some embodiments, the light chain further comprises a light chain constant region having a nucleic acid coding sequence of SEQ ID NO:26. In some embodiments, the nucleic acid coding sequence of the light chain is SEQ ID NO:27.

In some embodiments, the heavy chain comprises a heavy chain variable region having a nucleic acid coding sequence of SEQ ID NO:28. In some embodiments, the heavy chain further comprises a heavy chain constant region having a nucleic acid coding sequence of SEQ ID NO: 29. In some embodiments, the nucleic acid coding sequence of the heavy chain is SEQ ID NO:30.

The information of partial sequences involved in the present invention is provided in Table 1.

Table 1: Description of the sequences

| SEQ ID NO: | Description | Sequences |
|---|---|---|
| 1 | Amino acid sequence of CDR-L1 | RASQDINKYLA |
| 2 | Amino acid sequence of CDR-L2 | STSTLQS |
| 3 | Amino acid sequence of CDR-L3 | LQYDDLFT |
| 4 | Amino acid sequence of CDR-H1 | SFDIN |
| 5 | Amino acid | WIFPGDGNTKYSQKFQG |
| | sequence of CDR-H2 | |
| 6 | Amino acid sequence of CDR-H3 | GEALYYFDY |
| 7 | Amino acid sequence of the light chain variable region of TR001 | DIQMTQSPSSLSASVGDRVTITCRASQDINKYLAWYQQKP GKVPKLLIYSTSTLQSGVPSRFSGSGSGTDFTLTISSLQPED VATYYCLQYDDLFTFGQGTKLEIK |
| 8 | Amino acid sequence of the light chain constant region of TR001 | RTVAAPSVFIFPPSDEQLKSGTASVVCLLNNFYPREAKVQ WKVDNALQSGNSQESVTEQDSKDSTYSLSSTLTLSKADY EKHKVYACEVTHQGLSSPVTKSFNRGEC |
| 9 | Amino acid sequence of the light chain of TR001 | DIQMTQSPSSLSASVGDRVTITCRASQDINKYLAWYQQKP GKVPKLLIYSTSTLQSGVPSRFSGSGSGTDFTLTISSLQPED VATYYCLQYDDLFTFGQGTKLEIKRTVAAPSVFIFPPSDEQ LKSGTASVVCLLNNFYPREAKVQWKVDNALQSGNSQES VTEQDSKDSTYSLSSTLTLSKADYEKHKVYACEVTHQGL SSPVTKSFNRGEC |
| 10 | Amino acid sequence of the light chain variable region of TR002 | DIQMTQSPSSLSASVGDRVTITCRASQDINKYLAWYQQKP GKVPKLLIYSTSTLQSGVPSRFSGSGSGTDFTLTISSLQPED VATYYCLQYDDLFTFGQGTKLEIK |

(continued)

| SEQ ID NO: | Description | Sequences |
|---|---|---|
| 11 | Amino acid sequence of the light chain constant region of TR002 | RTVAAPSVFIFPPSDEQLKSGTASVVCLLNNFYPREAKVQWKVDNALQSGNSQESVTEQDSKDSTYSLSSTLTLSKADYEKHKVYACEVTHQGLSSPVCTKSFNRGEC |
| 12 | Amino acid sequence of the light chain of TR002 | DIQMTQSPSSLSASVGDRVTITCRASQDINKYLAWYQQKPGKVPKLLIYSTSTLQSGVPSRFSGSGSGTDFTLTISSLQPEDVATYYCLQYDDLFTFGQGTKLEIKRTVAAPSVFIFPPSDEQLKSGTASVVCLLNNFYPREAKVQWKVDNALQSGNSQESVTEQDSKDSTYSLSSTLTLSKADYEKHKVYACEVTHQGLSSPVCTKSFNRGEC |
| 13 | Amino acid sequences of the heavy chain variable region of TR001 and TR002 | QVQLVQSGAEVKKPGASVKLSCKASGYTFTSFDINWVRQAPEQRLEWMGWIFPGDGNTKYSQKFQGRATITRDTSASTAYMELSSLRSEDTAVYYCVRGEALYYFDYWGQGTLVTVSS |
| 14 | Amino acid sequences of the heavy chain constant region of TR001 and TR002 | ASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKRVEPKSCDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSRDELTKNQVSLTCLVKGFYPSDIAVEWESNGQPENNYKTTP |
| | | PVLDSDGSFFLYSKLTVDKSRWQQGNVFSCSVMHEALHNHYTQKSLSLSPG |
| 15 | Amino acid sequences of the heavy chain of TR001 and TR002 | QVQLVQSGAEVKKPGASVKLSCKASGYTFTSFDINWVRQAPEQRLEWMGWIFPGDGNTKYSQKFQGRATITRDTSASTAYMELSSLRSEDTAVYYCVRGEALYYFDYWGQGTLVTVSSASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKRVEPKSCDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSRDELTKNQVSLTCLVKGFYPSDIAVEWESNGQPENNYKTTPPVLDSDGSFFLYSKLTVDKSRWQQGNVFSCSVMHEALHNHYTQKSLSLSPG |
| 16 | Nucleic acid coding sequence of CDR-L1 | AGAGCTTCTCAGGATATCAATAAGTATCTGGCT |
| 17 | Nucleic acid coding sequence of CDR-L2 | TCTACATCTACCCTGCAGTCT |
| 18 | Nucleic acid coding sequence of CDR-L3 | CTGCAGTATGATGATCTGTTCACC |
| 19 | Nucleic acid coding sequence of CDR-H1 | TCCTTCGACATTAAC |

(continued)

| SEQ ID NO: | Description | Sequences |
|---|---|---|
| 20 | Nucleic acid coding sequence of CDR-H2 | TGGATCTTCCCCGGCGACGGCAACACCAAGTACTCCCAGAAGTTCCAGGGA |
| 21 | Nucleic acid coding sequence of CDR-H3 | GGAGAGGCTCTGTACTATTTTGATTAT |
| 22 | Nucleic acid coding sequence of the light chain variable region of TR001 | GATATCCAGATGACCCAGTCTCCATCTAGCCTGTCCGCTTCTGTGGGCGATAGAGTGACCATCACATGCAGAGCTTCTCAGGATATCAATAAGTATCTGGCTTGGTATCAGCAGAAGCCTGGAAAGGTGCCTAAGCTGCTGATCTACTCTACATCTACCCTGCAGTCTGGAGTGCCTTCTAGATTTTCTGGATCTGGCTCTGGCACCGATTTTACACTGACAATCTCTTCTCTGCAGCCTGAGGATGTGGCTACATATTATTGTCTGCAGTATGATGATCTGTTCACCTTTGGCCAGGGCACCAAGCTGGAGATCAAG |
| 23 | Nucleic acid coding sequence of the light chain constant region of TR001 | CGTACGGTGGCTGCACCATCTGTCTTCATCTTCCCGCCATCTGATGAGCAGTTGAAATCTGGAACTGCCTCTGTTGTGTGCCTGCTGAATAACTTCTATCCCAGAGAGGCCAAAGTACAGTGGAAGGTGGATAACGCCCTCCAATCGGGTAACTCCCAGGAGAGTGTCACAGAGCAGGACAGCAAGGACAGCACCTACAGCCTCAGCAGCACCCTGACGCTGAGCAAAGCAGACTACGAGAAACACAAAGTCTACGCCTGCGAAGTCACCCATCAGGGCCTGAGCTCGCCCGTCACAAAGAGCTT |
| | | CAACAGGGGAGAGTGTTAG |
| 24 | Nucleic acid coding sequence of the light chain of TR001 | GATATCCAGATGACCCAGTCTCCATCTAGCCTGTCCGCTTCTGTGGGCGATAGAGTGACCATCACATGCAGAGCTTCTCAGGATATCAATAAGTATCTGGCTTGGTATCAGCAGAAGCCTGGAAAGGTGCCTAAGCTGCTGATCTACTCTACATCTACCCTGCAGTCTGGAGTGCCTTCTAGATTTTCTGGATCTGGCTCTGGCACCGATTTTACACTGACAATCTCTTCTCTGCAGCCTGAGGATGTGGCTACATATTATTGTCTGCAGTATGATGATCTGTTCACCTTTGGCCAGGGCACCAAGCTGGAGATCAAGCGTACGGTGGCTGCACCATCTGTCTTCATCTTCCCGCCATCTGATGAGCAGTTGAAATCTGGAACTGCCTCTGTTGTGTGCCTGCTGAATAACTTCTATCCCAGAGAGGCCAAAGTACAGTGGAAGGTGGATAACGCCCTCCAATCGGGTAACTCCCAGGAGAGTGTCACAGAGCAGGACAGCAAGGACAGCACCTACAGCCTCAGCAGCACCCTGACGCTGAGCAAAGCAGACTACGAGAAACACAAAGTCTACGCCTGCGAAGTCACCCATCAGGGCCTGAGCTCGCCCGTCACAAAGAGCTTCAACAGGGGAGAGTGTTAG |

(continued)

| SEQ ID NO: | Description | Sequences |
|---|---|---|
| 25 | Nucleic acid coding sequence of the light chain variable region of TR002 | GATATCCAGATGACCCAGTCTCCATCTAGCCTGTCCGCT TCTGTGGGCGATAGAGTGACCATCACATGCAGAGCTTC TCAGGATATCAATAAGTATCTGGCTTGGTATCAGCAGAA GCCTGGAAAGGTGCCTAAGCTGCTGATCTACTCTACATC TACCCTGCAGTCTGGAGTGCCTTCTAGATTTTCTGGATC TGGCTCTGGCACCGATTTTACACTGACAATCTCTTCTCT GCAGCCTGAGGATGTGGCTACATATTATTGTCTGCAGTA TGATGATCTGTTCACCTTTGGCCAGGGCACCAAGCTGG AGATCAAG |
| 26 | Nucleic acid coding sequence of the light chain constant region of TR002 | CGTACGGTGGCTGCACCATCTGTCTTCATCTTCCCGCCA TCTGATGAGCAGTTGAAATCTGGAACTGCCTCTGTTGTG TGCCTGCTGAATAACTTCTATCCCAGAGAGGCCAAAGT ACAGTGGAAGGTGGATAACGCCCTCCAATCGGGTAACT CCCAGGAGAGTGTCACAGAGCAGGACAGCAAGGACAG CACCTACAGCCTCAGCAGCACCCTGACGCTGAGCAAAG CAGACTACGAGAAACACAAAGTCTACGCCTGCGAAGTC ACCCATCAGGGCCTGAGCTCGCCCGTCTGTACAAAGAG CTTCAACAGGGGAGAGTGTTAG |
| 27 | Nucleic acid coding sequence of the light chain of TR002 | GATATCCAGATGACCCAGTCTCCATCTAGCCTGTCCGCT TCTGTGGGCGATAGAGTGACCATCACATGCAGAGCTTC TCAGGATATCAATAAGTATCTGGCTTGGTATCAGCAGAA GCCTGGAAAGGTGCCTAAGCTGCTGATCTACTCTACATC TACCCTGCAGTCTGGAGTGCCTTCTAGATTTTCTGGATC TGGCTCTGGCACCGATTTTACACTGACAATCTCTTCTCT GCAGCCTGAGGATGTGGCTACATATTATTGTCTGCAGTA TGATGATCTGTTCACCTTTGGCCAGGGCACCAAGCTGG AGATCAAGCGTACGGTGGCTGCACCATCTGTCTTCATCT TCCCGCCATCTGATGAGCAGTTGAAATCTGGAACTGCCT CTGTTGTGTGCCTGCTGAATAACTTCTATCCCAGAGAGG CCAAAGTACAGTGGAAGGTGGATAACGCCCTCCAATCG GGTAACTCCCAGGAGAGTGTCACAGAGCAGGACAGCA AGGACAGCACCTACAGCCTCAGCAGCACCCTGACGCTG |
| | | AGCAAAGCAGACTACGAGAAACACAAAGTCTACGCCT GCGAAGTCACCCATCAGGGCCTGAGCTCGCCCGTCTGT ACAAAGAGCTTCAACAGGGGAGAGTGTTAG |

(continued)

| SEQ ID NO: | Description | Sequences |
|---|---|---|
| 28 | Nucleic acid coding sequences of the heavy chain variable region of TR001 and TR002 | CAGGTGCAGCTGGTGCAGTCCGGCGCCGAGGTGAAGA<br>AGCCCGGCGCCTCCGTGAAGCTGAGCTGTAAGGCCTCC<br>GGCTACACCTTCACCTCCTTCGACATTAACTGGGTGCGG<br>CAGGCCCCCGAGCAGCGCCTGGAGTGGATGGGCTGGAT<br>CTTCCCCGGCGACGGCAACACCAAGTACTCCCAGAAGT<br>TCCAGGGAAGAGCTACCATCACCAGAGATACATCCGCT<br>TCTACAGCTTACATGGAGCTGTCTAGCCTGAGATCTGAG<br>GATACAGCTGTGTATTACTGTGTGAGAGGAGAGGCTCT<br>GTACTATTTTGATTATTGGGGCCAGGGCACCCTGGTGAC<br>AGTGTCTTCT |
| 29 | Nucleic acid coding sequences of the heavy chain constant region of TR001 and TR002 | GCTAGCACCAAGGGCCCATCGGTCTTCCCCCTGGCACC<br>CTCCTCCAAGAGCACCTCTGGGGGCACAGCGGCCCTGG<br>GCTGCCTGGTCAAGGACTACTTCCCCGAACCGGTGACG<br>GTGTCGTGGAACTCAGGCGCCCTGACCAGCGGCGTGCA<br>CACCTTCCCGGCTGTCCTACAGTCCTCAGGACTCTACTC<br>CCTCAGCAGCGTGGTGACCGTGCCCTCCAGCAGCTTGG<br>GCACCCAGACCTACATCTGCAACGTGAATCACAAGCCC<br>AGCAACACCAAGGTGGACAAGAGAGTTGAGCCCAAAT<br>CTTGTGACAAAACTCACACATGCCCACCGTGCCCAGCA<br>CCTGAACTCCTGGGGGGACCGTCAGTCTTCCTCTTCCC<br>CCCAAAACCCAAGGACACCCTCATGATCTCCCGGACCC<br>CTGAGGTCACATGCGTGGTGGTGGACGTGAGCCACGAA<br>GACCCTGAGGTCAAGTTCAACTGGTACGTGGACGGCGT<br>GGAGGTGCATAATGCCAAGACAAAGCCGCGGGAGGAG<br>CAGTACAACAGCACGTACCGTGTGGTCAGCGTCCTCAC<br>CGTCCTGCACCAGGACTGGCTGAATGGCAAGGAGTACA<br>AGTGCAAGGTCTCCAACAAAGCCCTCCCAGCCCCCATC<br>GAGAAAACCATCTCCAAAGCCAAAGGGCAGCCCCGAG<br>AACCACAGGTGTACACCCTGCCCCCATCCCGGGATGAG<br>CTGACCAAGAACCAGGTCAGCCTGACCTGCCTGGTCAA<br>AGGCTTCTATCCCAGCGACATCGCCGTGGAGTGGGAGA<br>GCAATGGGCAGCCGGAGAACAACTACAAGACCACGCC<br>TCCCGTGCTGGACTCCGACGGCTCCTTCTTCCTCTATAG<br>CAAGCTCACCGTGGACAAGAGCAGGTGGCAGCAGGGG<br>AACGTCTTCTCATGCTCCGTGATGCATGAGGCTCTGCAC<br>AACCACTACACGCAGAAGAGCCTCTCCCTGTCTCCGGG<br>TTAA |

(continued)

| SEQ ID NO: | Description | Sequences |
|---|---|---|
| 30 | Nucleic acid coding sequences of the heavy chain of TR001 and TR002 | CAGGTGCAGCTGGTGCAGTCCGGCGCCGAGGTGAAGA AGCCCGGCGCCTCCGTGAAGCTGAGCTGTAAGGCCTCC GGCTACACCTTCACCTCCTTCGACATTAACTGGGTGCGG CAGGCCCCCGAGCAGCGCCTGGAGTGGATGGGCTGGAT CTTCCCCGGCGACGGCAACACCAAGTACTCCCAGAAGT TCCAGGGAAGAGCTACCATCACCAGAGATACATCCGCT TCTACAGCTTACATGGAGCTGTCTAGCCTGAGATCTGAG GATACAGCTGTGTATTACTGTGTGAGAGGAGAGGCTCT GTACTATTTTGATTATTGGGGCCAGGGCACCCTGGTGAC AGTGTCTTCTGCTAGCACCAAGGGCCCATCGGTCTTCCC |
| | | CCTGGCACCCTCCTCCAAGAGCACCTCTGGGGGCACAG CGGCCCTGGGCTGCCTGGTCAAGGACTACTTCCCCGAA CCGGTGACGGTGTCGTGGAACTCAGGCGCCCTGACCAG CGGCGTGCACACCTTCCCGGCTGTCCTACAGTCCTCAG GACTCTACTCCCTCAGCAGCGTGGTGACCGTGCCCTCC AGCAGCTTGGGCACCCAGACCTACATCTGCAACGTGAA TCACAAGCCCAGCAACACCAAGGTGGACAAGAGAGTT GAGCCCAAATCTTGTGACAAAACTCACACATGCCCACC GTGCCCAGCACCTGAACTCCTGGGGGGACCGTCAGTCT TCCTCTTCCCCCCAAAACCCAAGGACACCCTCATGATCT CCCGGACCCCTGAGGTCACATGCGTGGTGGTGGACGTG AGCCACGAAGACCCTGAGGTCAAGTTCAACTGGTACGT GGACGGCGTGGAGGTGCATAATGCCAAGACAAAGCCG CGGGAGGAGCAGTACAACAGCACGTACCGTGTGGTCA GCGTCCTCACCGTCCTGCACCAGGACTGGCTGAATGGC AAGGAGTACAAGTGCAAGGTCTCCAACAAAGCCCTCC CAGCCCCCATCGAGAAAACCATCTCCAAAGCCAAAGGG CAGCCCCGAGAACCACAGGTGTACACCCTGCCCCCATC CCGGGATGAGCTGACCAAGAACCAGGTCAGCCTGACCT GCCTGGTCAAAGGCTTCTATCCCAGCGACATCGCCGTG GAGTGGGAGAGCAATGGGCAGCCGGAGAACAACTACA AGACCACGCCTCCCGTGCTGGACTCCGACGGCTCCTTC TTCCTCTATAGCAAGCTCACCGTGGACAAGAGCAGGTG GCAGCAGGGGAACGTCTTCTCATGCTCCGTGATGCATG AGGCTCTGCACAACCACTACACGCAGAAGAGCCTCTCC CTGTCTCCGGGTTAA |

In a second aspect, the present application provides a linker-drug conjugate of formula VIII or formula IX, or an isomer, mesomer, racemate, or enantiomer thereof, or a mixture thereof, or a pharmaceutically acceptable salt or solvate thereof,

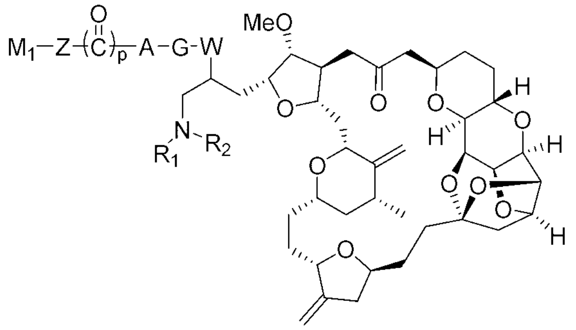

Formula VIII

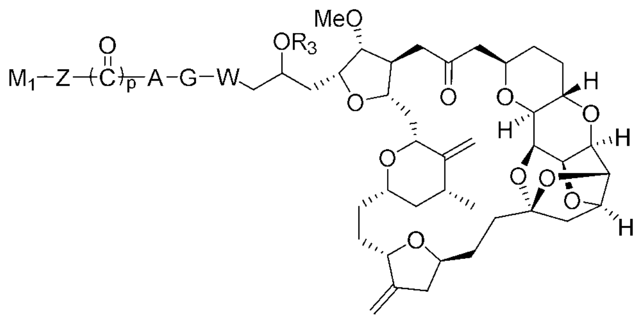

Formula IX

wherein,

$M_1$ is a linker unit,
the Z, p, A, G, W, $R_1$, $R_2$ and $R_3$ are as defined above.

In some embodiments, the linker-drug conjugate, or the isomer, mesomer, racemate, or enantiomer thereof, or the mixture thereof, or the pharmaceutically acceptable salt or solvate thereof of the present application has a structure represented by formula VIII-1 or formula IX-1,

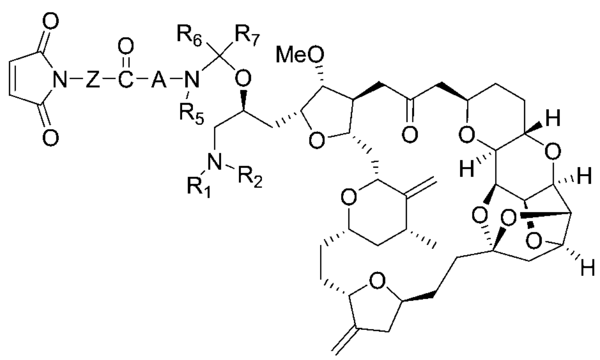

Formula VIII-1

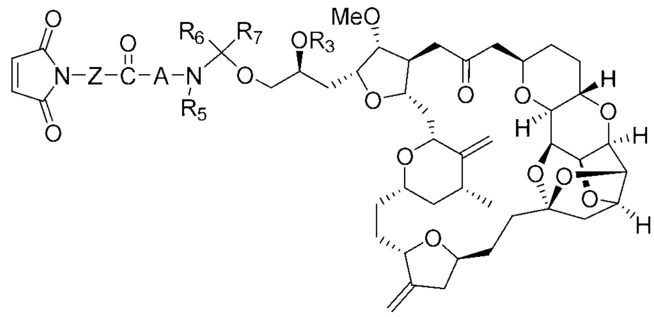

Formula IX-1

wherein,
the Z, A, $R_1$, $R_2$, $R_3$, $R_5$, $R_6$ and $R_7$ are as defined above.

In some embodiments, the linker-drug conjugate, or the isomer, mesomer, racemate, or enantiomer thereof, or the mixture thereof, or the pharmaceutically acceptable salt or solvate thereof of the present application has a structure represented by formula VIIIa or formula IXa:

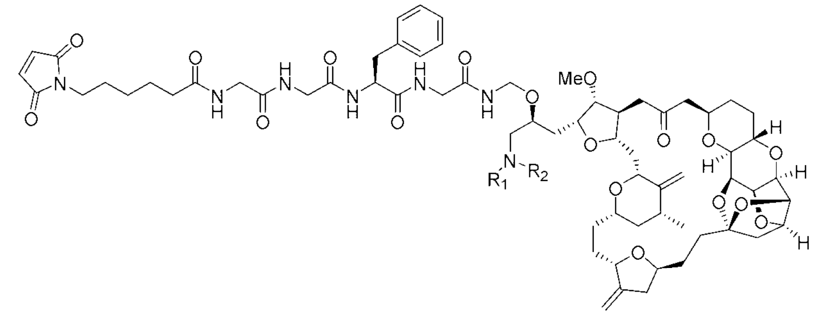

Formula VIIIa

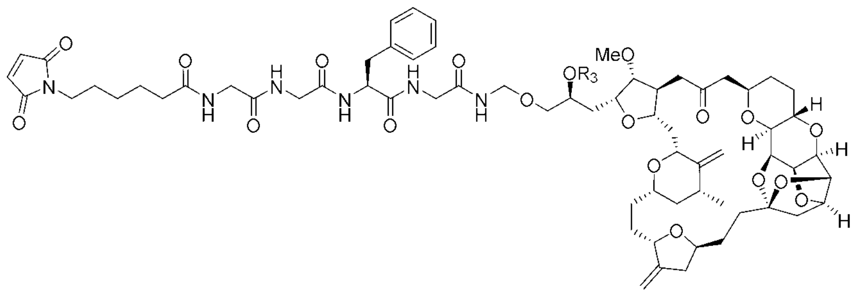

Formula IXa

wherein,
the $R_1$, $R_2$ and $R_3$ are as defined above.

In some embodiments, the linker-drug conjugate, or the isomer, mesomer, racemate, or enantiomer thereof, or the mixture thereof, or the pharmaceutically acceptable salt or solvate thereof of the present application has a structure represented by formula VIIIb or formula IXb:

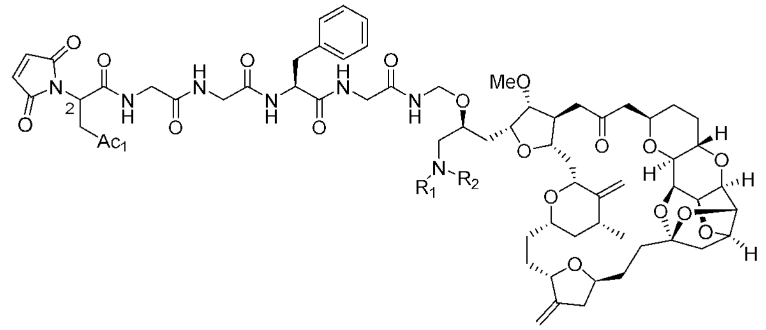

Formula VIIIb

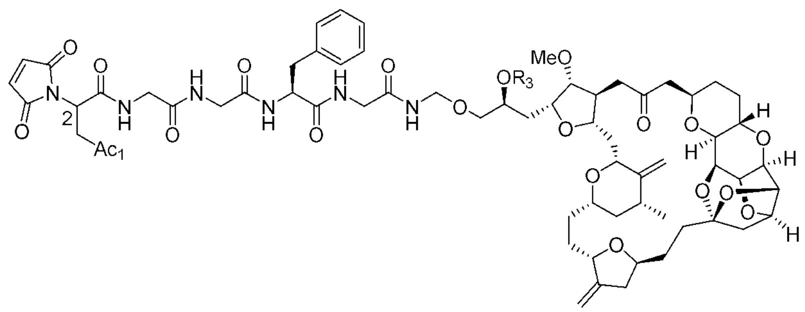

Formula IXb

wherein, Ac is a hydrophilic structural unit having the structure shown in formula d:

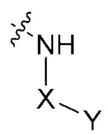

Formula d

the X and Y are as defined above. In some embodiments, the Ac is as defined above.

In some embodiments, the linker-drug conjugate, or the isomer, mesomer, racemate, or enantiomer thereof, or the mixture thereof, or the pharmaceutically acceptable salt or solvate thereof of the present application has a structure represented by formula VIIIc:

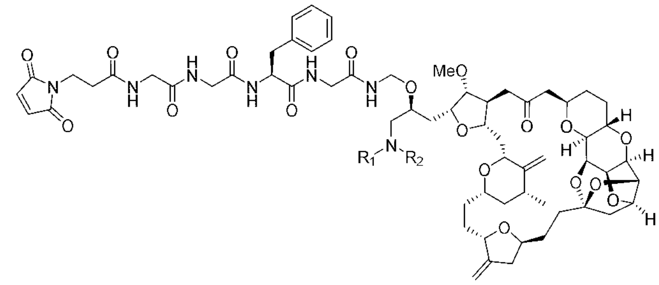

VIIIc

wherein, the $R_1$ and $R_2$ are as defined above.

In some embodiments, the linker-drug conjugate, or the isomer, mesomer, racemate, or enantiomer thereof, or the mixture thereof, or the pharmaceutically acceptable salt or solvate thereof of the present application has a structure represented by formula VIIId:

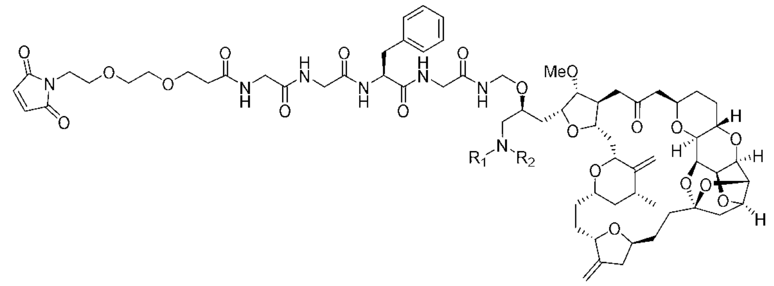

Formula VIIId

wherein, the $R_1$ and $R_2$ are as defined above.

In some embodiments, the linker-drug conjugate, or the isomer, mesomer, racemate, or enantiomer thereof, or the mixture thereof, or the pharmaceutically acceptable salt or solvate thereof of the present application has a structure represented by formula VIIIe:

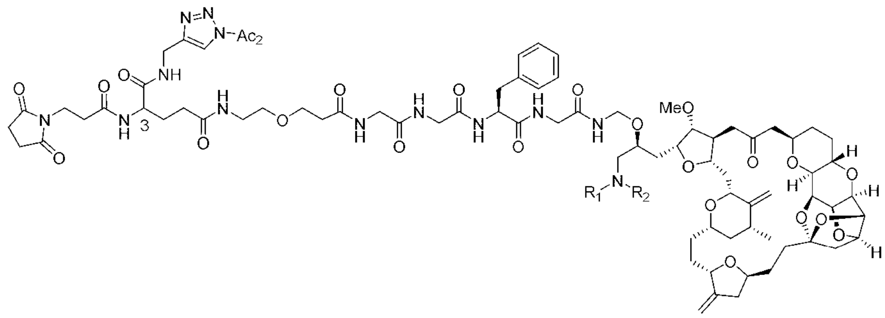

Formula VIIIe

wherein, the $R_1$ and $R_2$ are as defined above; $Ac_2$ is a hydrophilic structural unit composed of $-X_2-Y_2$, and the $Ac_2$, $X_2$ and $Y_2$ are as defined above.

In some embodiments, the linker-drug conjugate, or the isomer, mesomer, racemate, or enantiomer thereof, or the mixture thereof, or the pharmaceutically acceptable salt or solvate thereof of the present application has a structure represented by formula VIIIf:

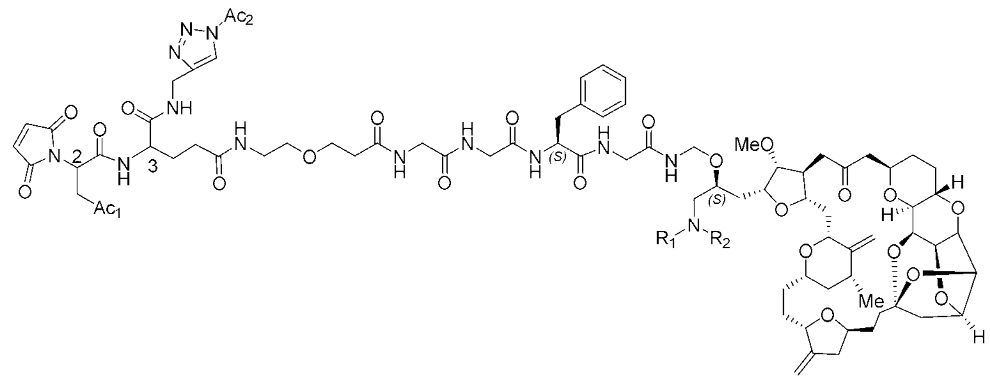

Formula VIIIf

wherein, the $R_1$ and $R_2$ are as defined above;

$Ac_1$ is a hydrophilic structural unit composed of $-X_1-Y_1$, and the $X_1$ and $Y_1$ are as defined above.

In some embodiments, the $Ac_1$ is selected, without limitation, from the group consisting of (D/L) alanine, (D/L) leucine, (D/L) isoleucine, (D/L) valine, (D/L) phenylalanine, (D/L) proline, (D/L) tryptophan, (D/L) serine, (D/L) tyrosine, (D/L) cysteine, (D/L) cystine, (D/L) arginine, (D/L) histidine, (D/L) methionine, (D/L) asparagine, (D/L) glutamine, (D/L) threonine, (D/L) aspartic acid, (D/L) glutamic acid, natural or unnatural amino acid derivatives and the following structures:

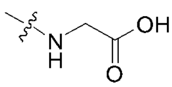 , 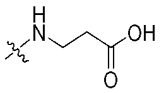 , 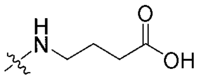 , 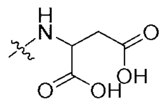 , 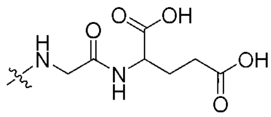 ,

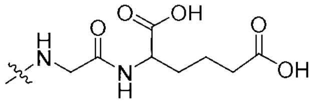 , 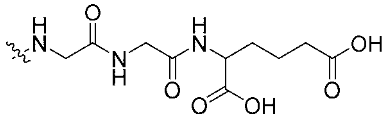 , 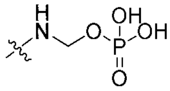 , 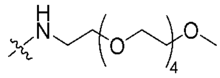

and

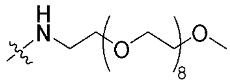 .

In some embodiments, $Ac_1$ is selected from the following structures:

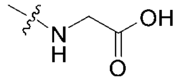 ,

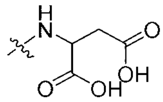 , and 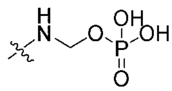 .

In some embodiments, $Ac_1$ is:

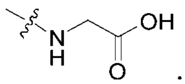 .

$Ac_2$ is a hydrophilic structural unit composed of $-X_2-Y_2$, and the $Ac_2$, $X_2$ and $Y_2$ are as defined above.

In some embodiments, the linker-drug conjugate, or the isomer, mesomer, racemate, or enantiomer thereof, or the mixture thereof, or the pharmaceutically acceptable salt or solvate thereof of the present application has a structure represented by formula VIIIg:

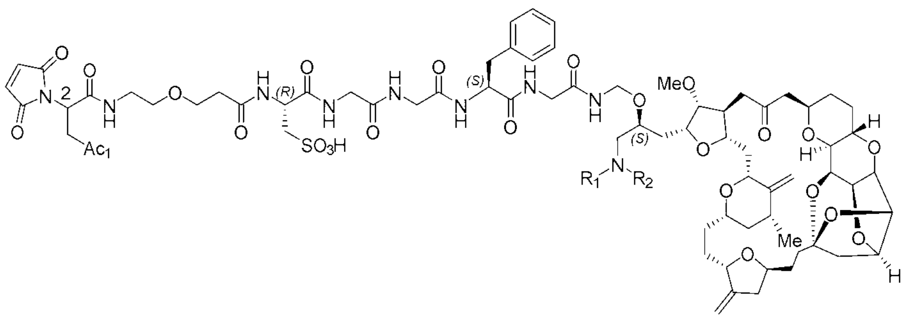

Formula VIIIg

wherein, the $R_1$ and $R_2$ are as defined above;
$Ac_1$ is a hydrophilic structural unit composed of $-X_1-Y_1$, and the $X_1$ and $Y_1$ are as defined above.

In some embodiments, the $Ac_1$ is selected, without limitation, from the group consisting of (D/L) alanine, (D/L) leucine, (D/L) isoleucine, (D/L) valine, (D/L) phenylalanine, (D/L) proline, (D/L) tryptophan, (D/L) serine, (D/L) tyrosine, (D/L) cysteine, (D/L) cystine, (D/L) arginine, (D/L) histidine, (D/L) methionine, (D/L) asparagine, (D/L) glutamine, (D/L) threonine, (D/L) aspartic acid, (D/L) glutamic acid, natural or unnatural amino acid derivatives and the following structures:

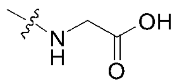 , 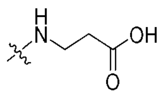 , 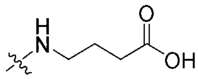 , 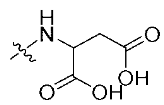 , 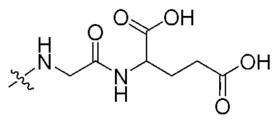 ,

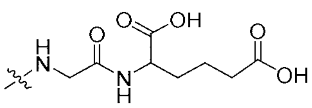 , 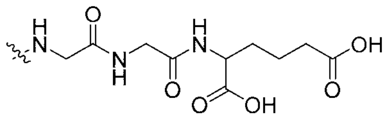 , 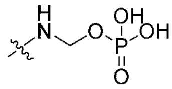 , 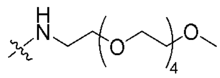

and

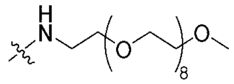 .

In some embodiments, $Ac_1$ is selected from the following structures:

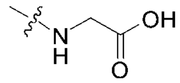 ,

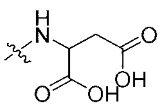 , and 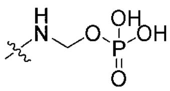 .

In some embodiments, $Ac_1$ is:

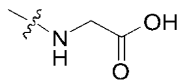

In some embodiments, the linker-drug conjugate, or the isomer, mesomer, racemate, or enantiomer thereof, or the mixture thereof, or the pharmaceutically acceptable salt or solvate thereof of the present application is selected from the following structures:

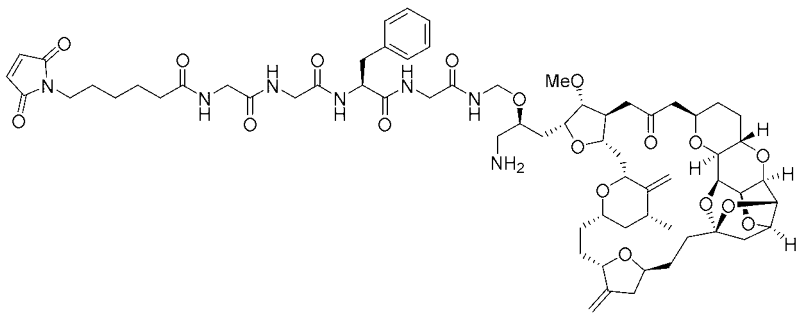

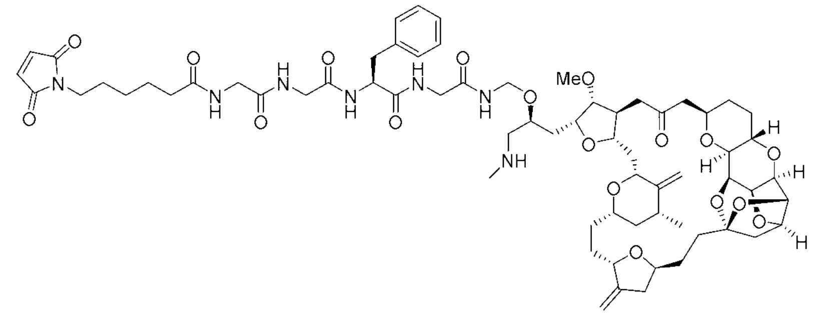

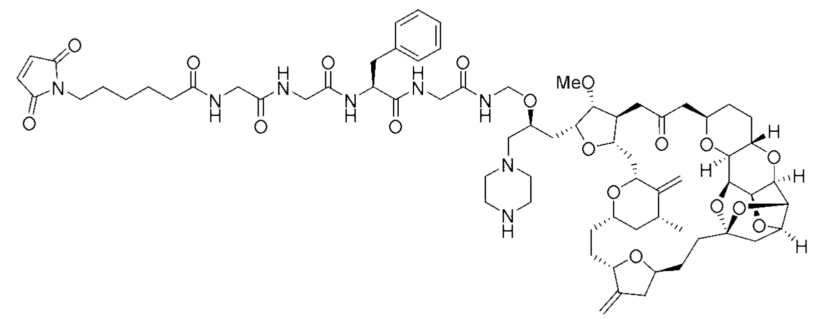

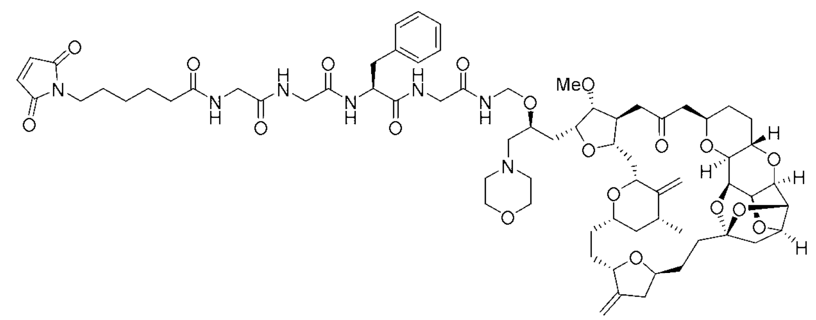

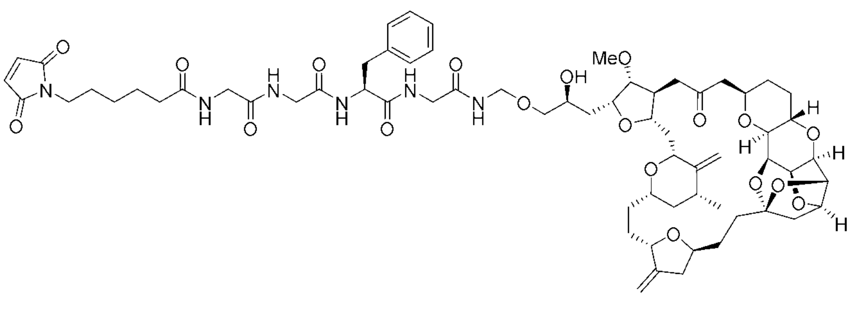

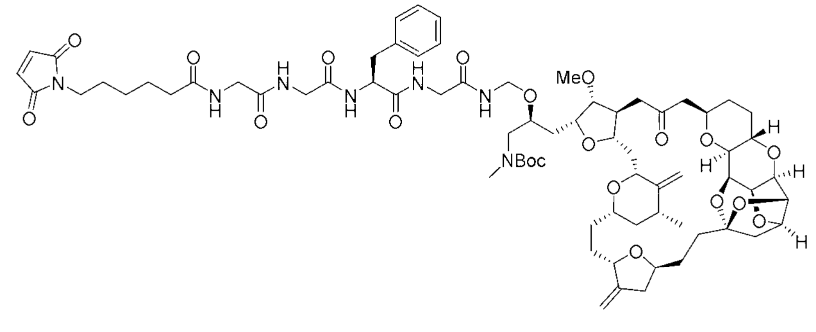

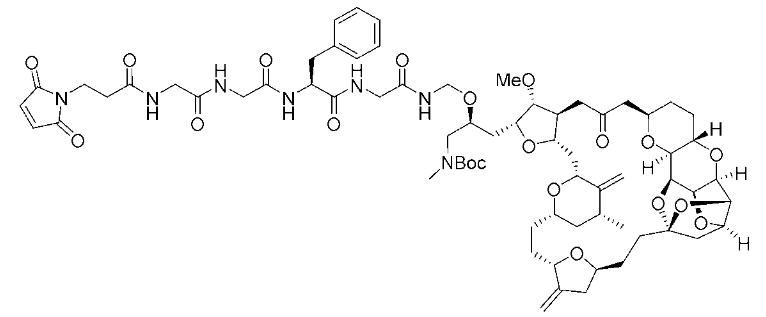

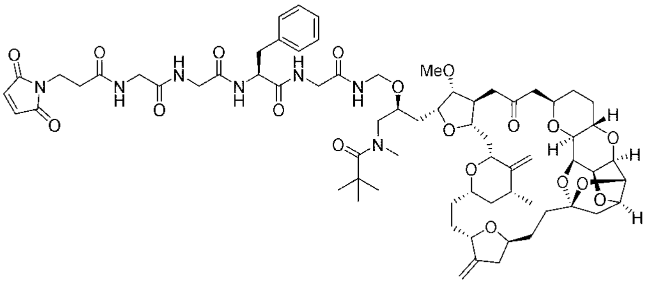

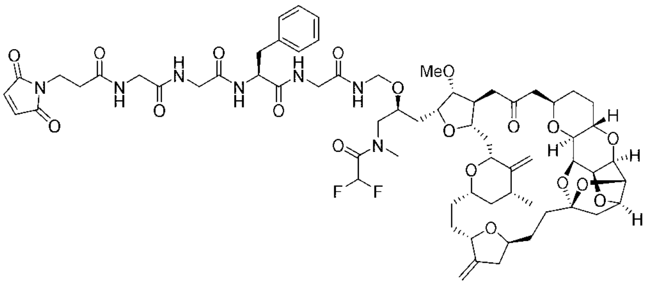

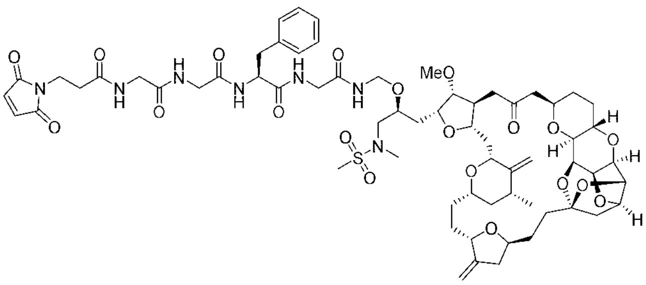

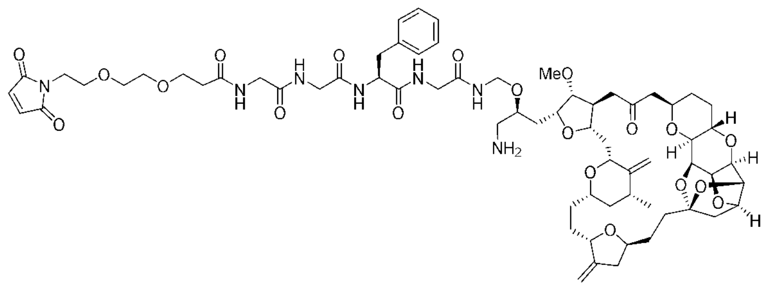

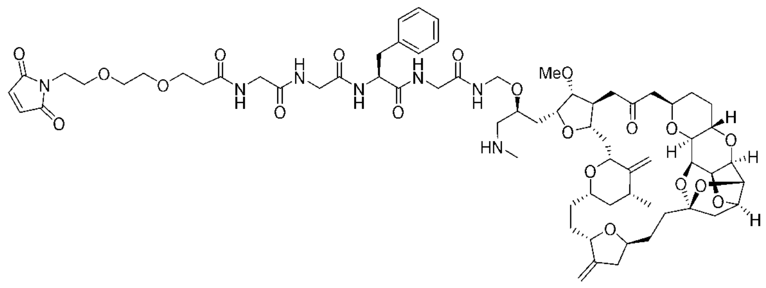

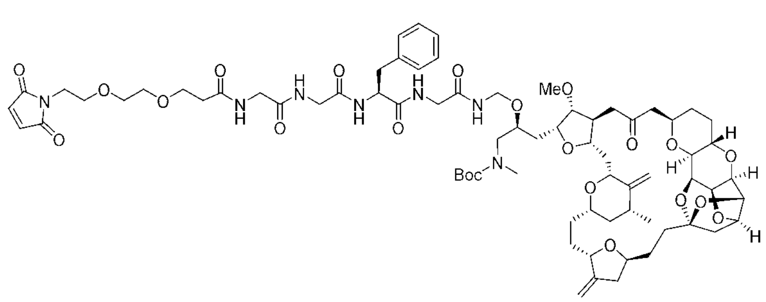

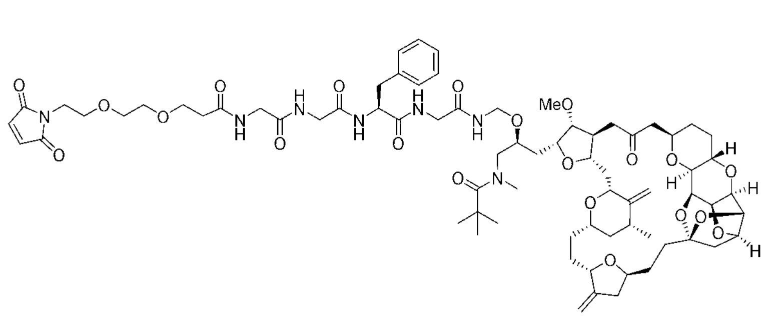

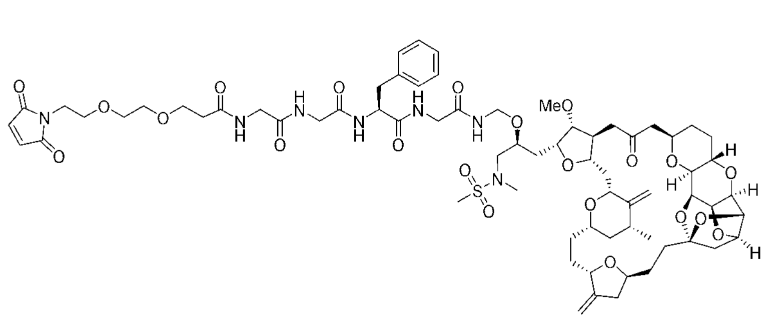

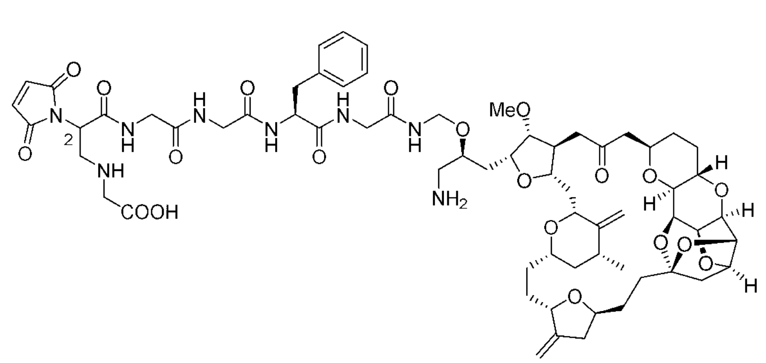

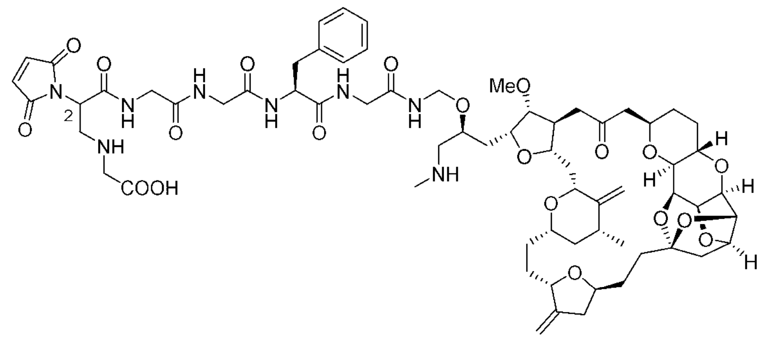

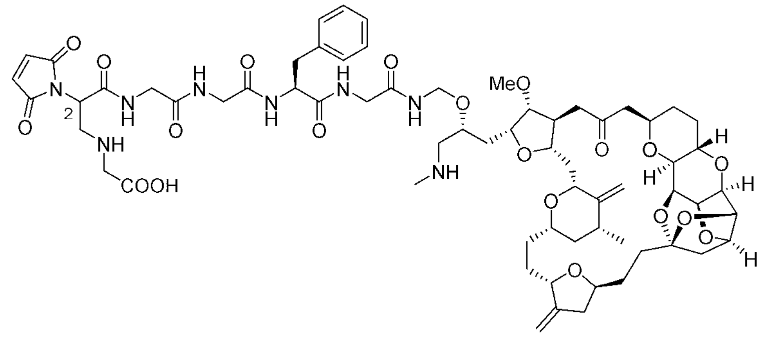

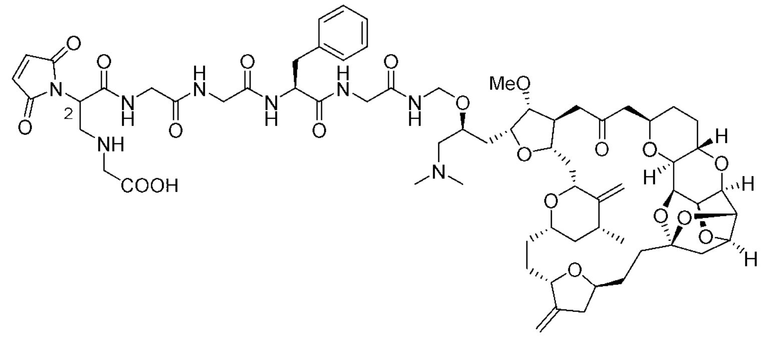

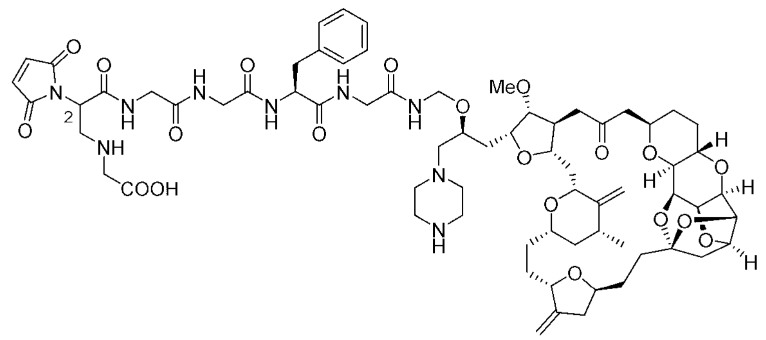

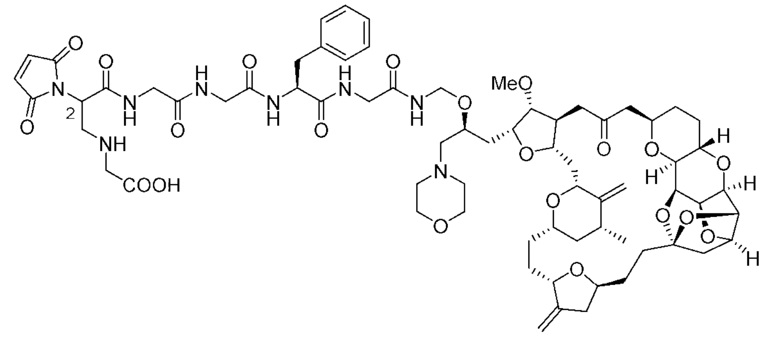

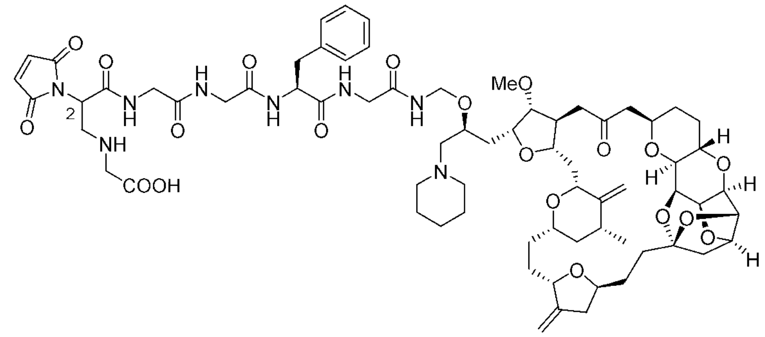

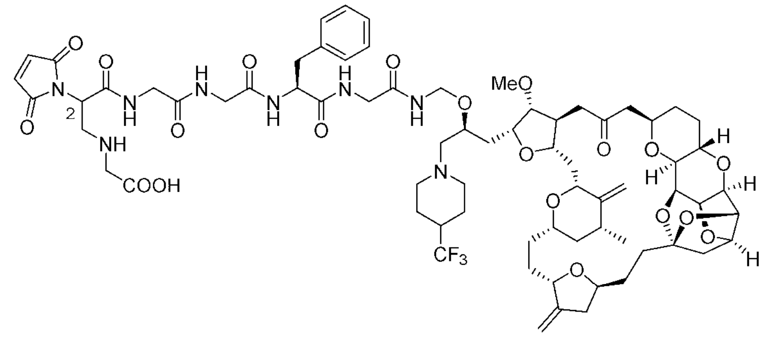

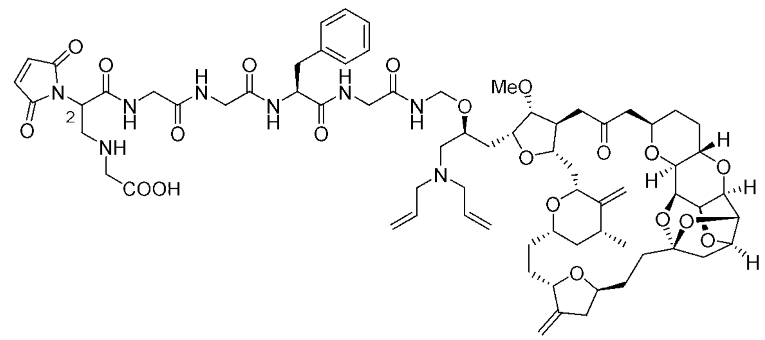

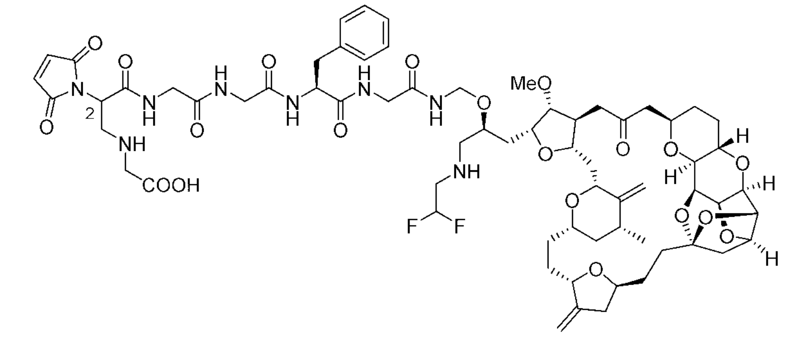

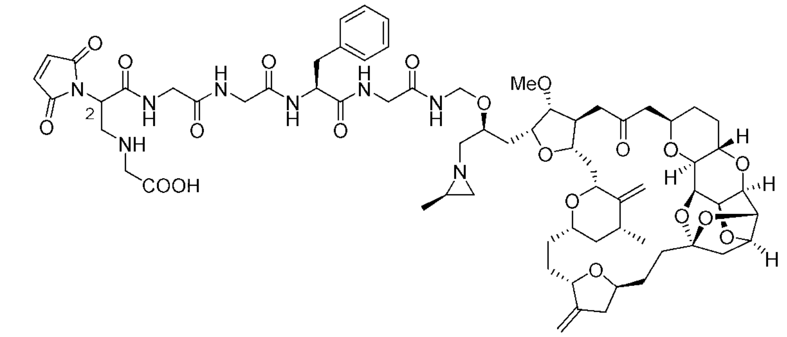

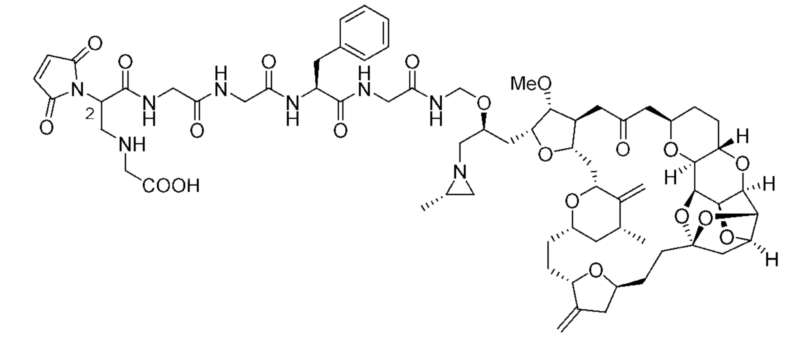

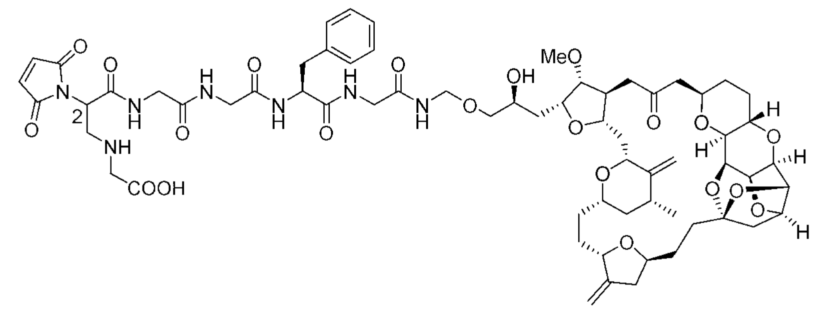

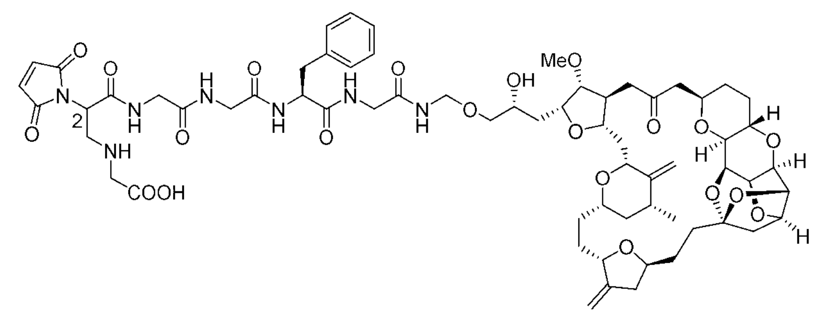

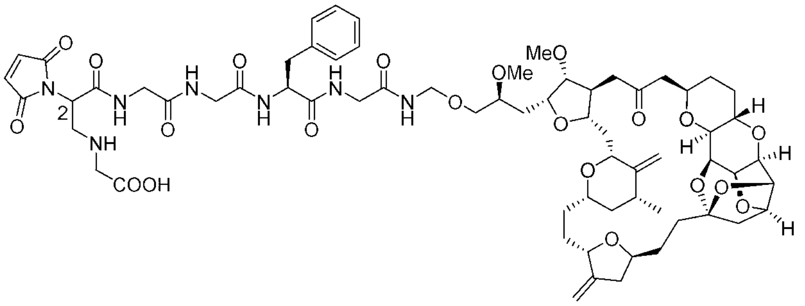

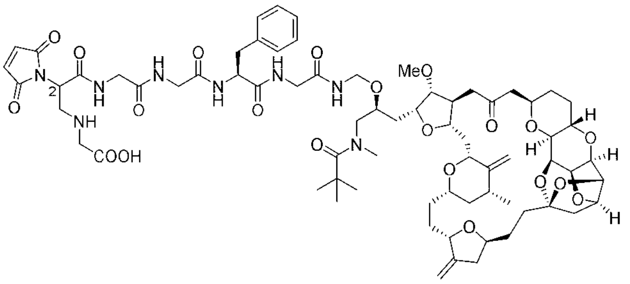

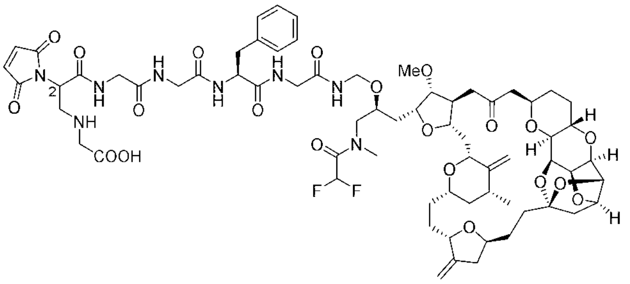

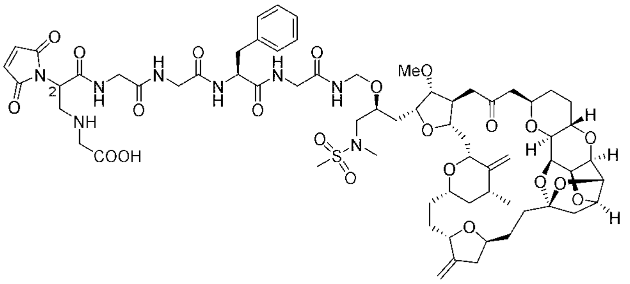

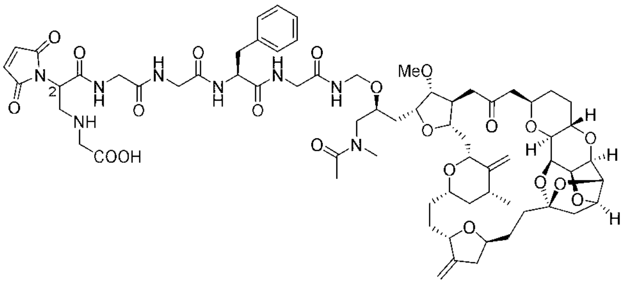

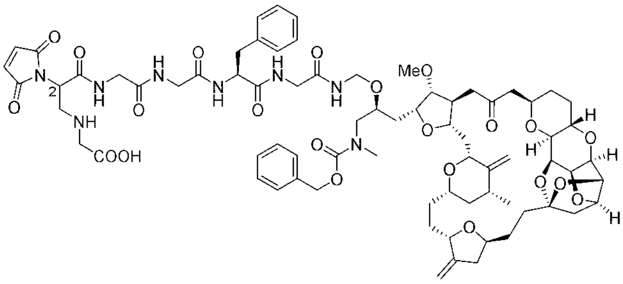

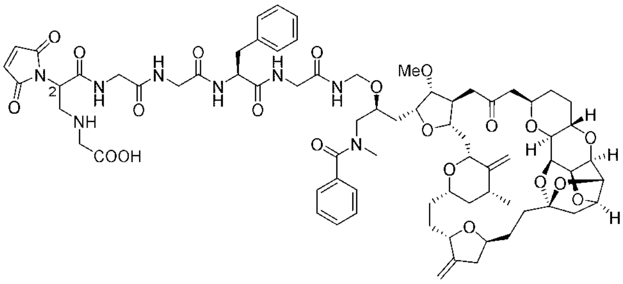

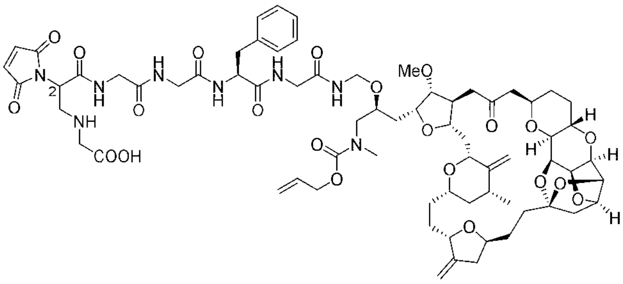

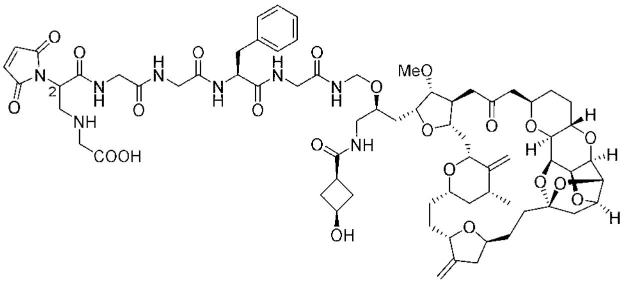

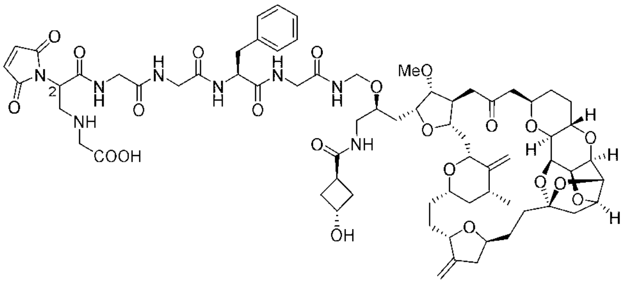

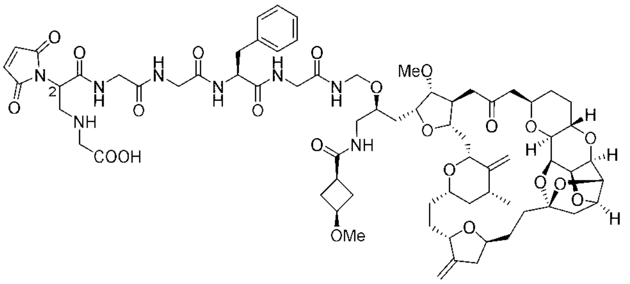

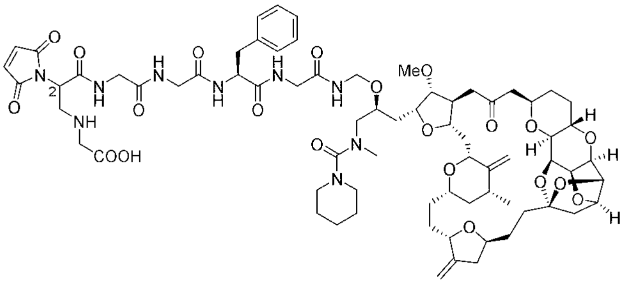

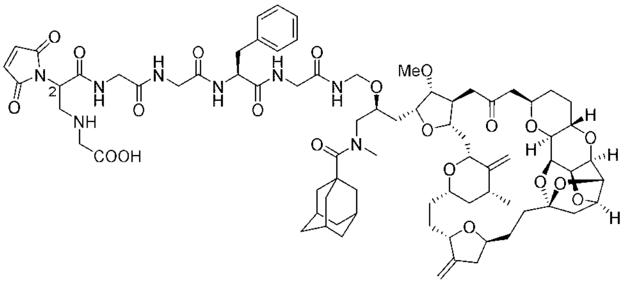

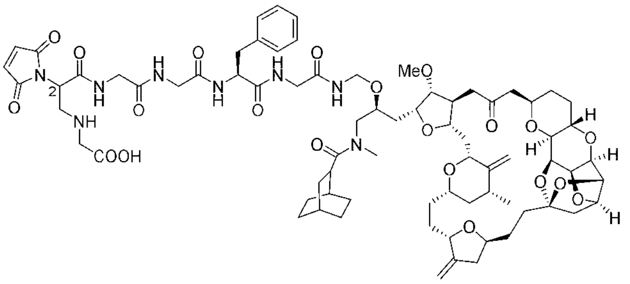

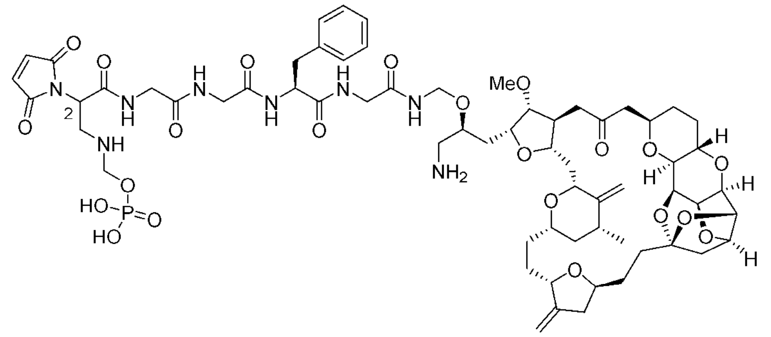

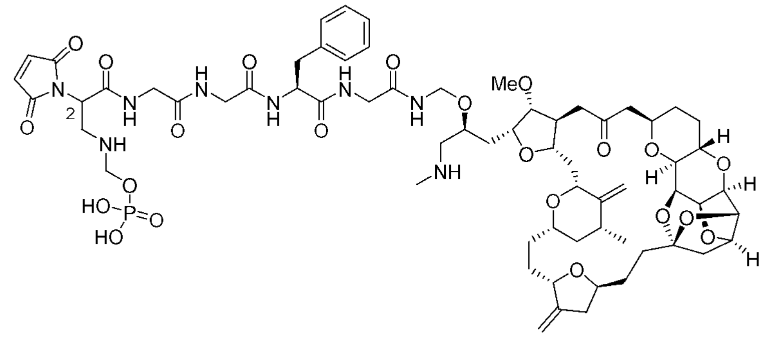

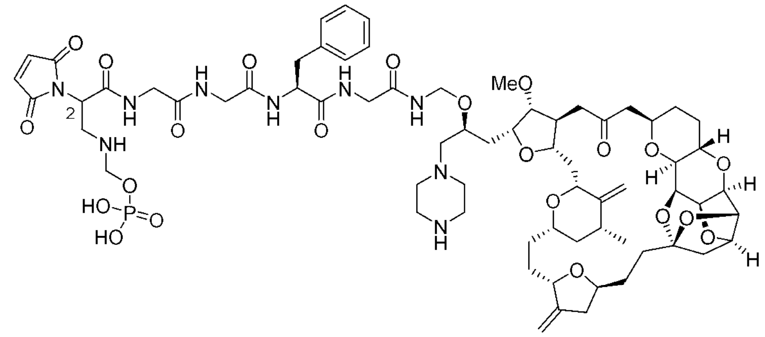

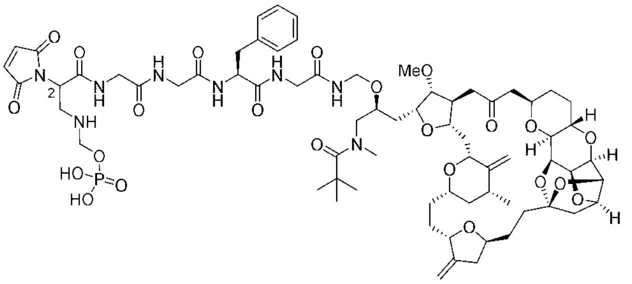

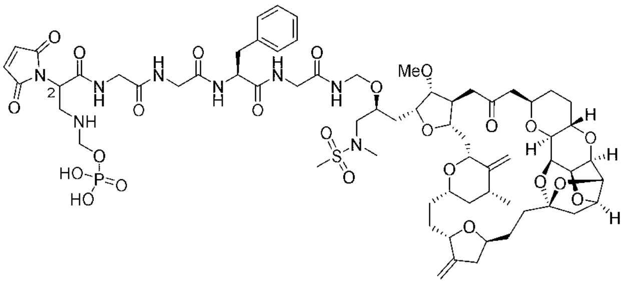

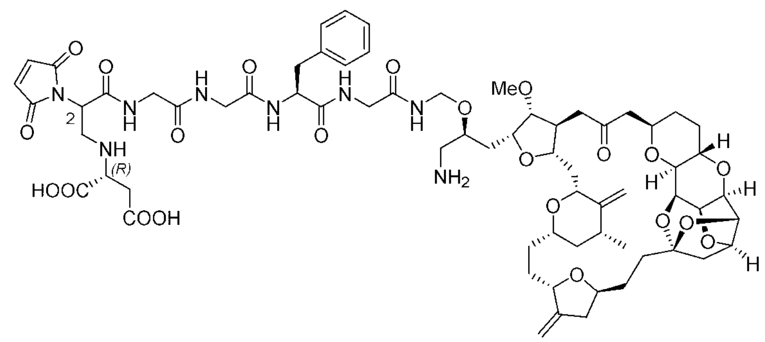

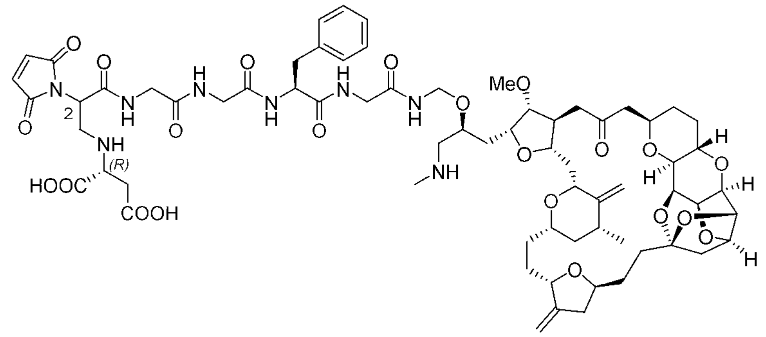

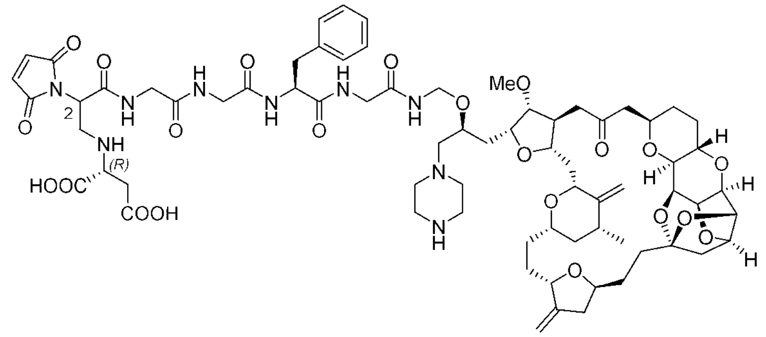

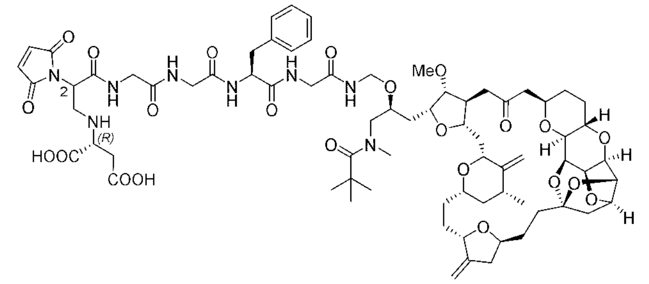

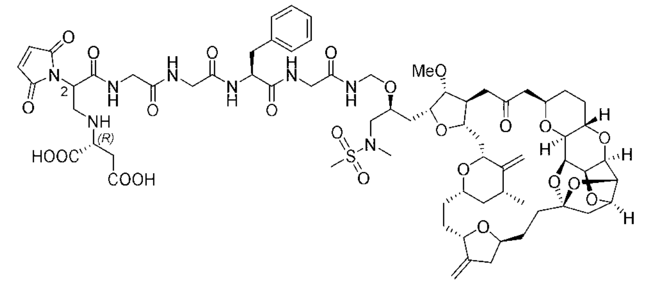

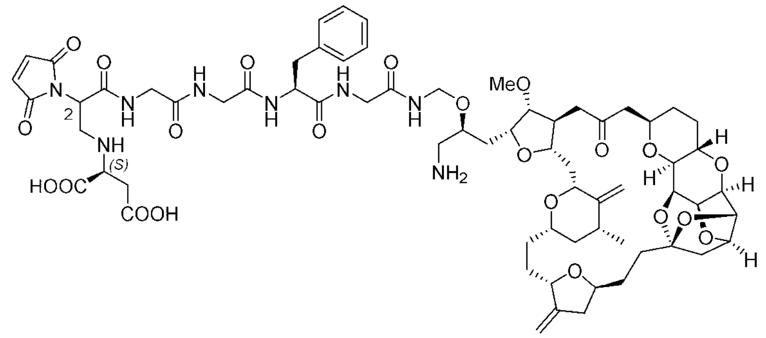

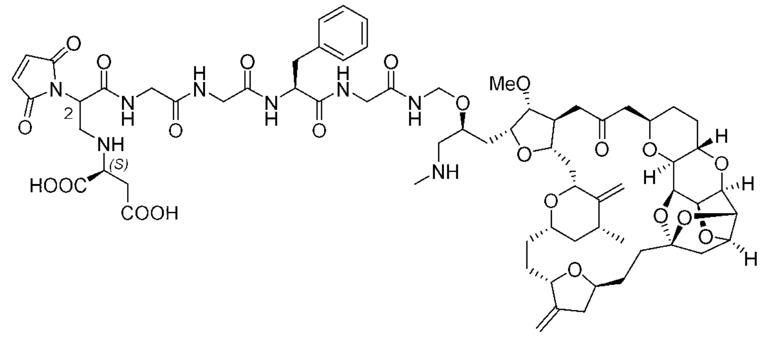

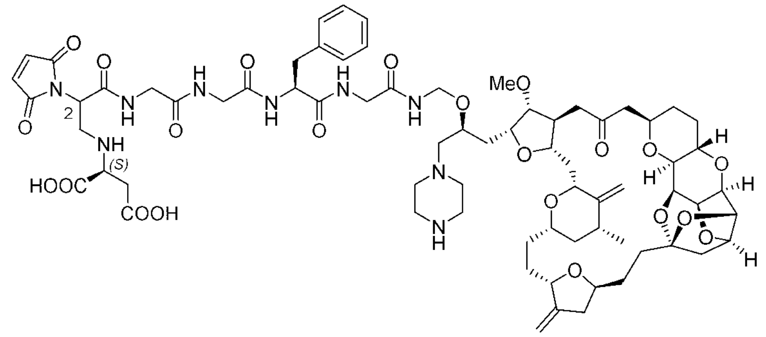

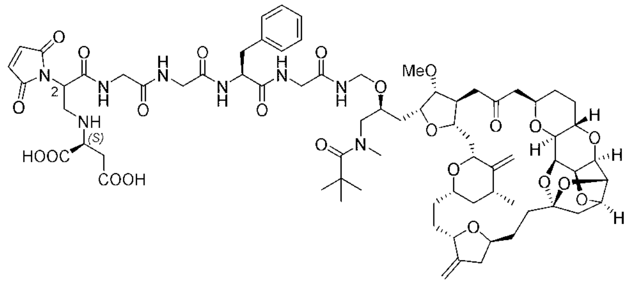

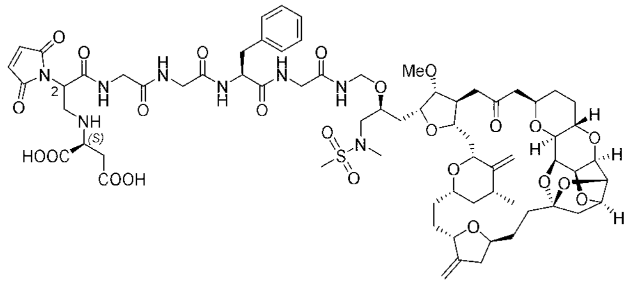

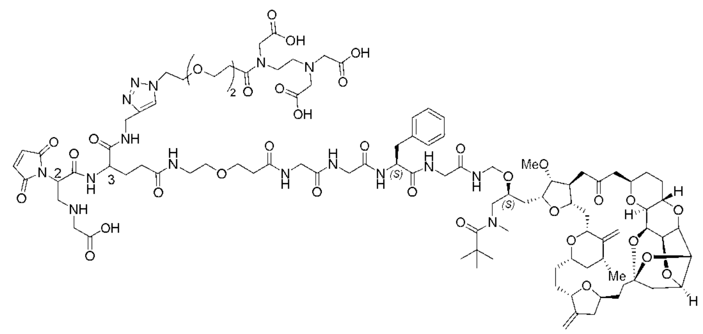

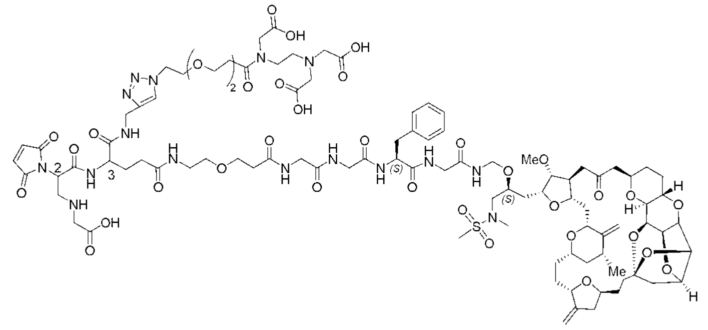

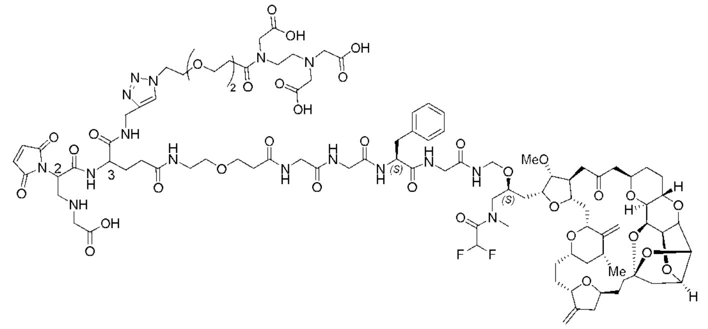

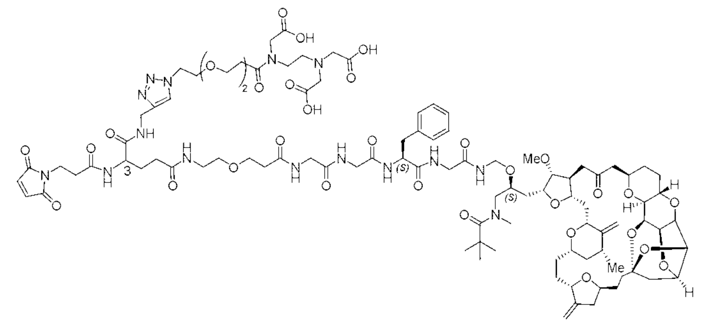

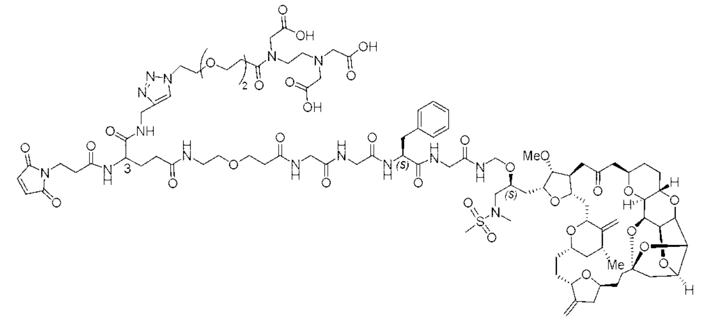

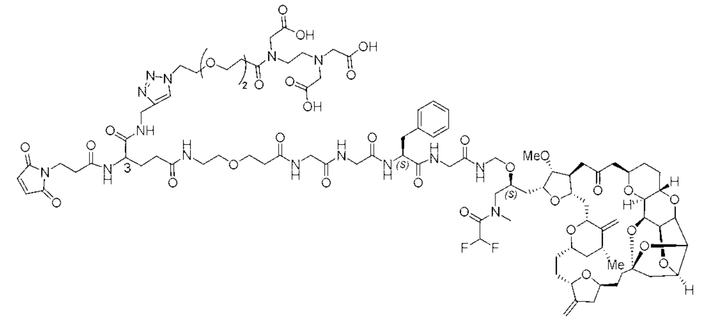

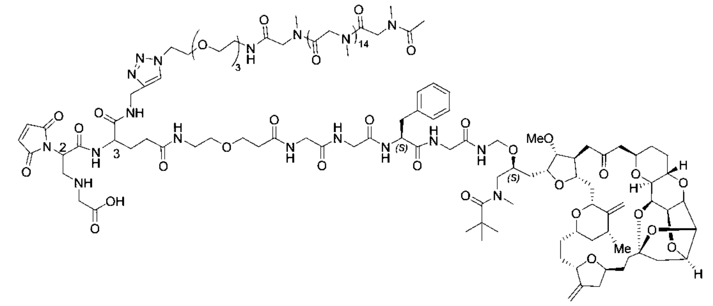

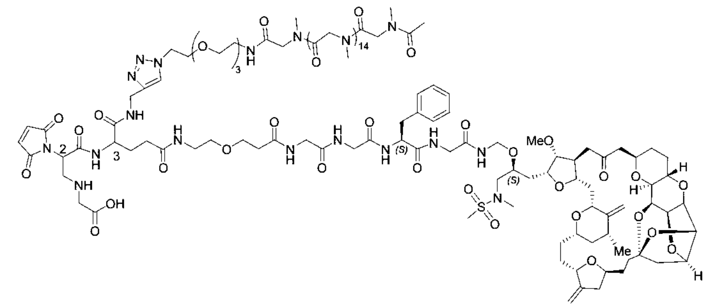

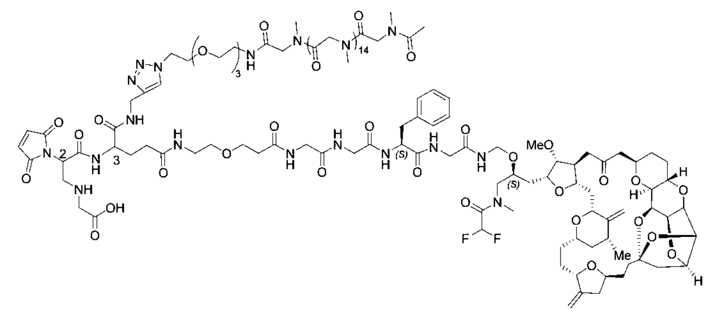

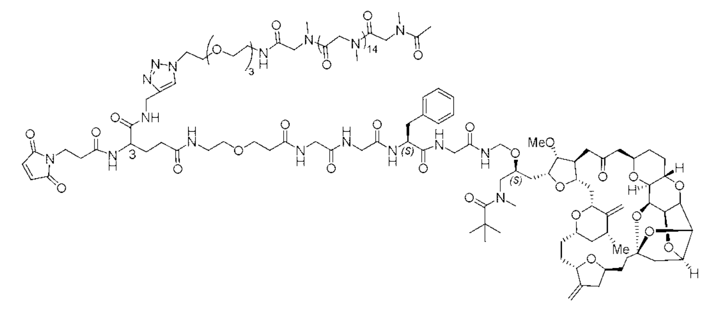

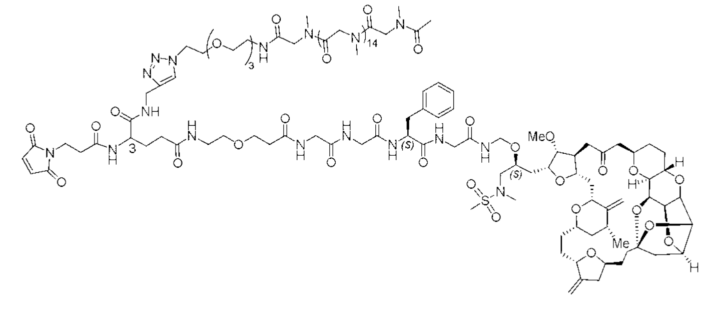

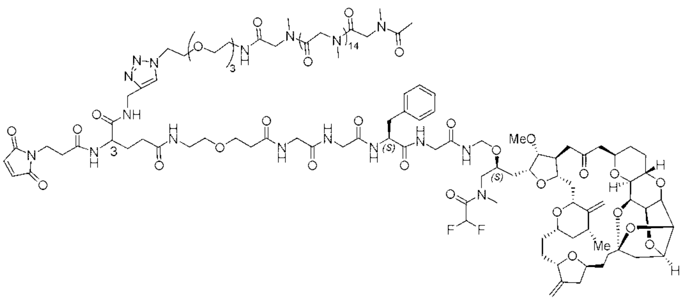

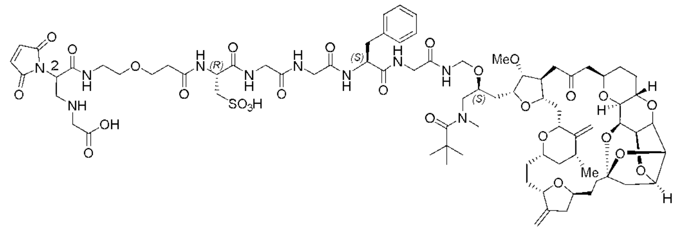

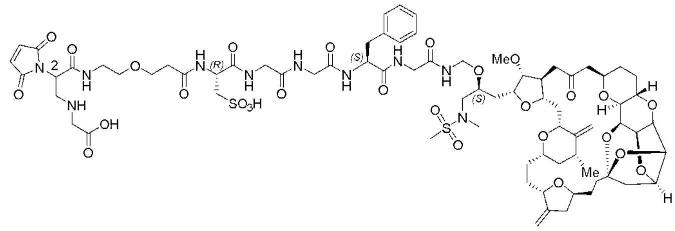

and

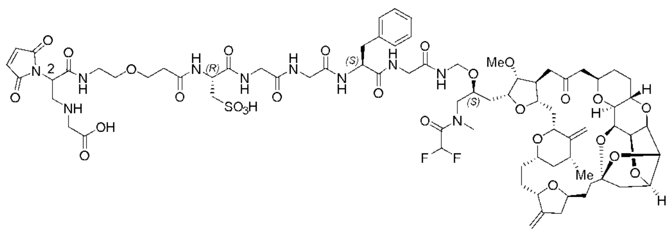

,

wherein chiral carbons at positions 2 and 3 independently have configuration R or S. In some embodiments, the chiral carbon at position 2 has configuration S, and the chiral carbon at position 3 has configuration S.

In a third aspect, the present application provides a compound of formula X, or an isomer, mesomer, racemate, or enantiomer thereof, or a mixture thereof, or a pharmaceutically acceptable salt or solvate thereof,

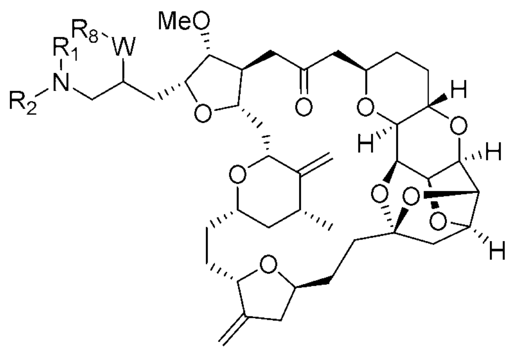

Formula X

wherein W is an oxygen atom or a sulfur atom, and preferably, W is an oxygen atom;

$R_1$ and $R_8$ are each independently selected from the group consisting of: hydrogen atom, alkyl, acyl, sulfonyl, cycloalkyl, heterocyclyl, aryl and heteroaryl, and are not simultaneously hydrogen; optionally, the alkyl, acyl, sulfonyl, cycloalkyl, aryl and heteroaryl are each independently substituted with one or more substituents selected from the group consisting of hydrogen atom, alkyl, alkoxy, halogen, deuterium, amino, cyano, hydroxy, mercapto, azido, nitro, carboxy, acyl, carbonyl, hydroxyalkyl, cycloalkyl, heterocyclyl, aryl and heteroaryl; preferably, $R_1$ is alkyl, and $R_8$ is H;

$R_2$ is -C(O)-$Q_1$-$Q_2$ or -$SO_2$-$Q_1$-$Q_2$, wherein $Q_1$ is selected from the group consisting of O, N and S atoms and a chemical bond;

in a case where $Q_1$ is selected from the group consisting of O, N and S atoms, $Q_2$ is selected from the group consisting of alkyl, cycloalkyl, heterocyclyl, spirocyclyl, bridged ring, alkenyl, aryl and heteroaryl, and optionally the alkyl, cycloalkyl, heterocyclyl, spirocyclyl, bridged ring, alkenyl, aryl and heteroaryl are each independently substituted with one or more substituents selected from the group consisting of hydrogen atom, alkyl, alkoxy, halogen, deuterium, amino, cyano, hydroxy, mercapto, azido, nitro, carboxy, acyl, carbonyl, hydroxyalkyl, cycloalkyl, heterocyclyl, aryl and heteroaryl;

in a case where $Q_1$ is a chemical bond, $Q_2$ is selected from the group consisting of alkyl, benzyl, cycloalkyl, heterocyclyl, spirocyclyl, bridged ring, alkenyl, aryl and heteroaryl;

in a case where $Q_2$ is an alkyl, the alkyl is substituted with one or more substituents selected from the group consisting of hydrogen atom, alkyl, alkoxy, halogen, deuterium, cyano, azido, nitro, carboxyl, acyl, carbonyl, cycloalkyl, heterocyclyl, aryl and heteroaryl;

in a case where $Q_2$ is selected from the group consisting of cycloalkyl, heterocyclyl, spirocyclyl, bridged ring, alkenyl, aryl and heteroaryl, the cycloalkyl, heterocyclyl, spirocyclyl, bridged ring, alkenyl, aryl and heteroaryl are each independently substituted with one or more substituents selected from the group consisting of hydrogen atom, alkyl, alkoxy, halogen, deuterium, amino, cyano, hydroxy, mercapto, azido, nitro, carboxyl, acyl, carbonyl, hydroxyalkyl, cycloalkyl, heterocyclyl, aryl and heteroaryl, and in a case where $Q_2$ is a cycloalkyl with the substituent being a hydroxyl, an amino or a mercapto, the substituent is at any position except a carbon atom connected to -C(O)-.

In some embodiments, the compound of formula X, or the isomer, mesomer, racemate, or enantiomer thereof, or the mixture thereof, or the pharmaceutically acceptable salt or solvate thereof is a compound of formula XIa or formula XIb, or an isomer, mesomer, racemate, or enantiomer thereof, or a mixture thereof,

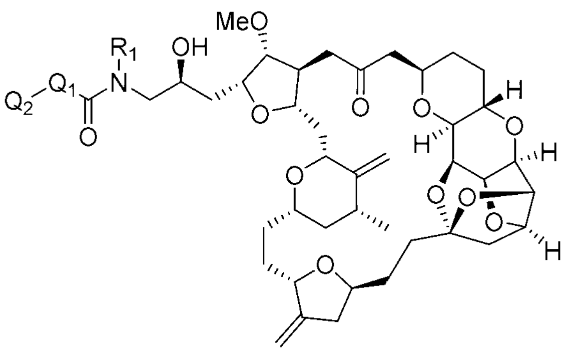

Formula XIa

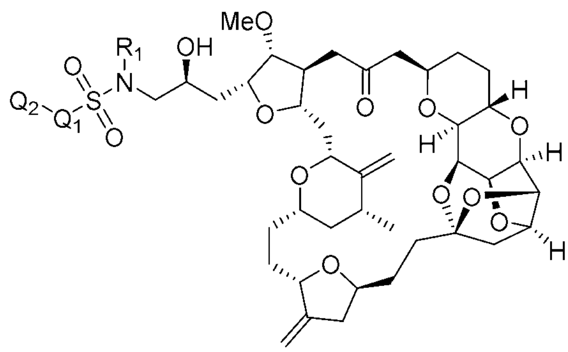

Formula XIb

wherein $R_1$ is an alkyl, and preferably $R_1$ is a methyl,

$Q_1$ is an O atom or a chemical bond;

in a case where $Q_1$ is an O atom, $Q_2$ is selected from the group consisting of alkyl, cycloalkyl, alkenyl and aryl, and optionally the alkyl, cycloalkyl, alkenyl and aryl are each independently substituted with one or more substituents selected from the group consisting of hydrogen atom, alkyl, halogen, hydroxy, hydroxyalkyl and aryl;

in a case where $Q_1$ is a chemical bond, $Q_2$ is selected from the group consisting of alkyl, benzyl, cycloalkyl, alkenyl and aryl;

in a case where $Q_2$ is an alkyl, the alkyl is substituted with one or more substituents selected from the group consisting of hydrogen atom, alkyl, halogen, deuterium, azido, nitro and aryl;

in a case where $Q_2$ is selected from the group consisting of cycloalkyl, alkenyl and aryl, the cycloalkyl, alkenyl and aryl are each independently substituted with one or more substituents selected from the group consisting of hydrogen atom, alkyl, halogen, hydroxyl, hydroxyalkyl and aryl, and in a case where $Q_2$ is a cycloalkyl with the substituent being a hydroxyl, an amino or a mercapto, the substituent is at any position except a carbon atom connected to -C(O)-.

In some embodiments, the compound of formula X, or the isomer, mesomer, racemate, or enantiomer thereof, or the mixture thereof, or the pharmaceutically acceptable salt or solvate thereof of the present application is selected from the following structures:

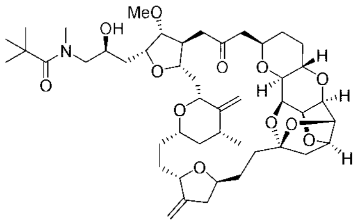 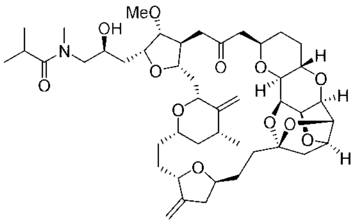 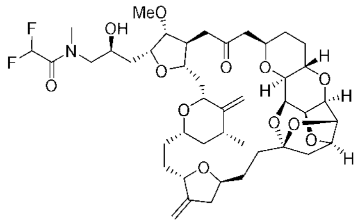

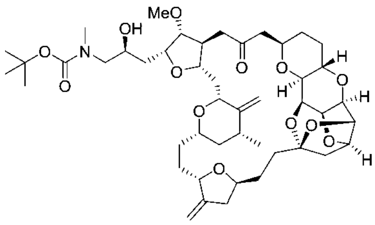 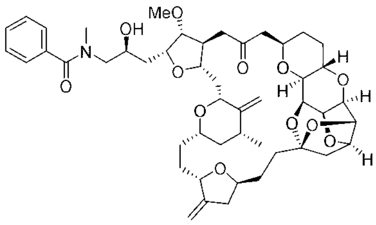 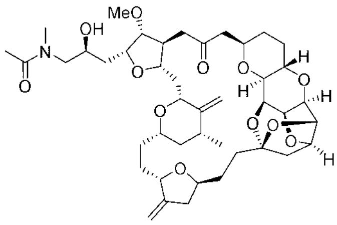

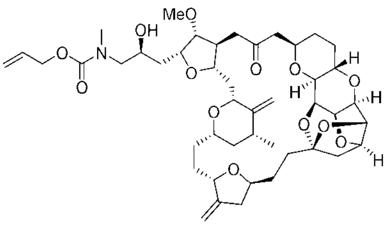 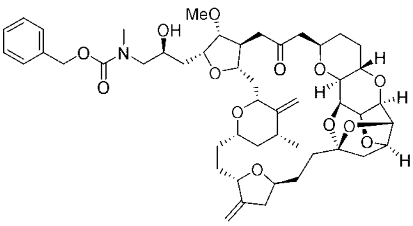 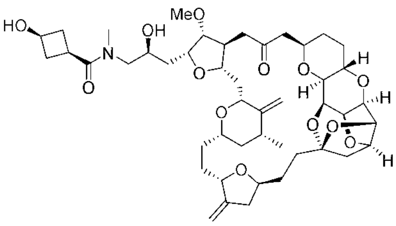

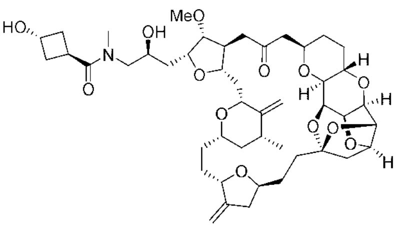 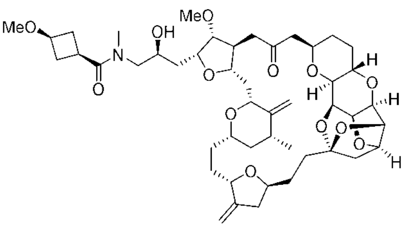 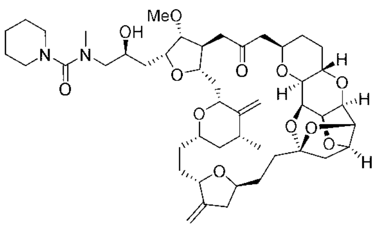

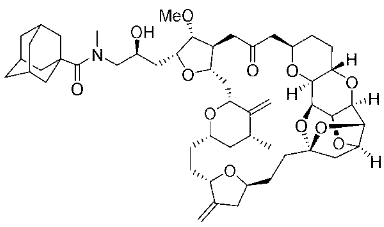 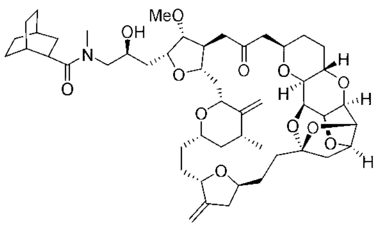 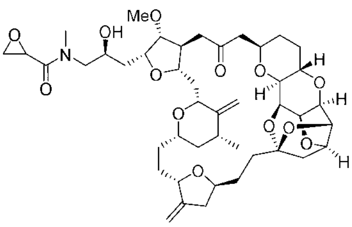

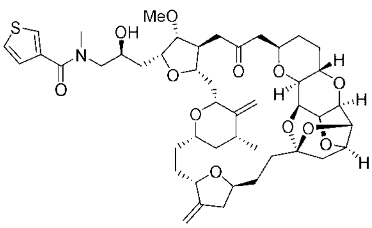 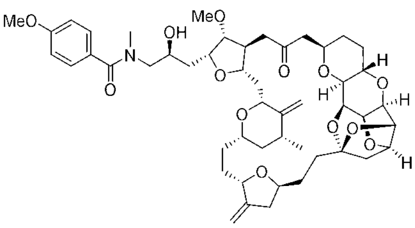 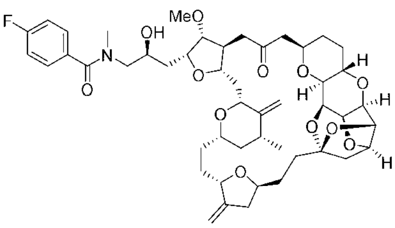

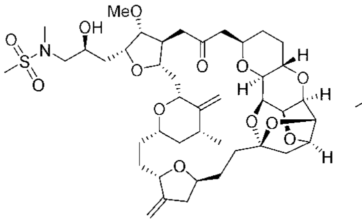 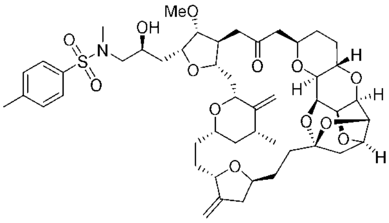 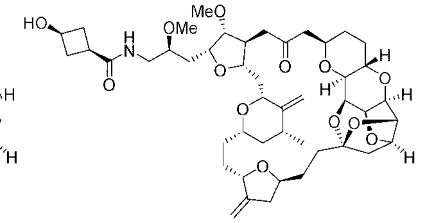

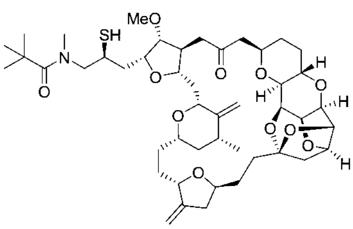 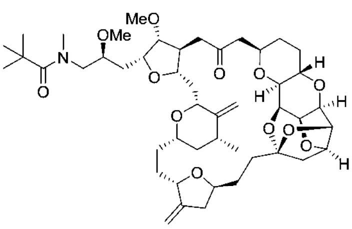 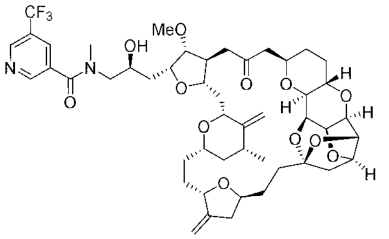

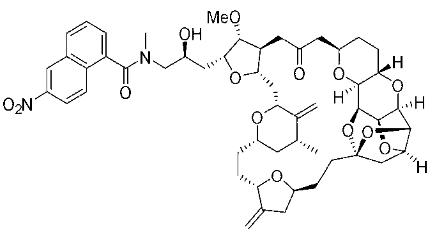 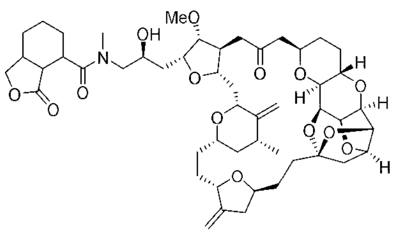 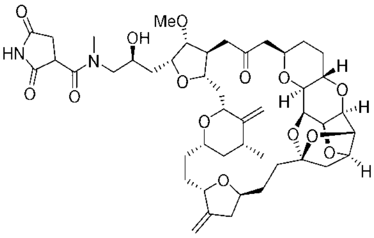

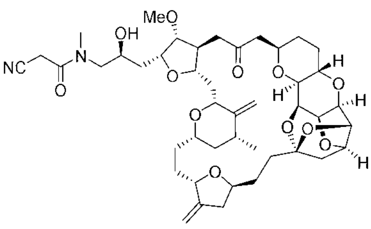 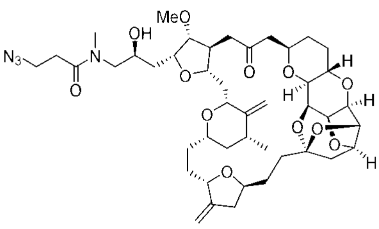 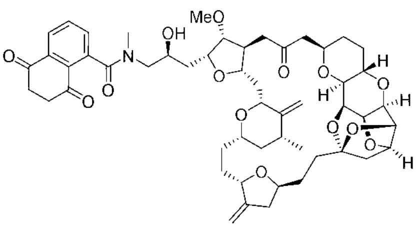

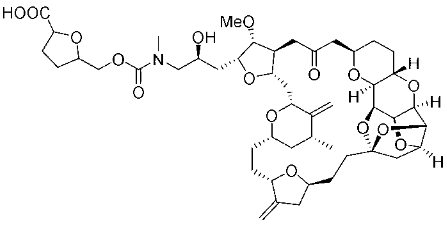 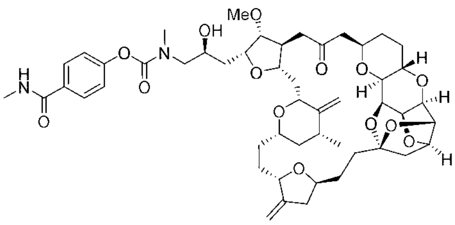

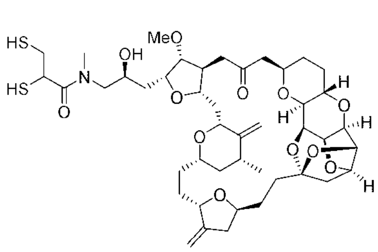 and 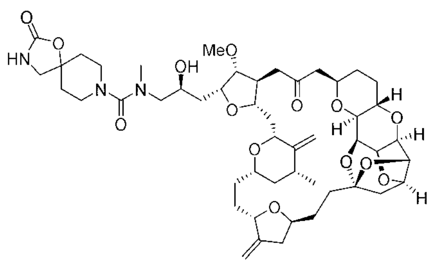 .

some embodiments of the present application, pharmaceutically acceptable salts of the ligand-drug conjugate, or the linker-drug conjugate or the isomer, mesomer, racemate, or enantiomer thereof or the mixture thereof, or the compound of formula X or the isomer, mesomer, racemate or enantiomer thereof or the mixture thereof include sodium, potassium, calcium or magnesium salts formed by acidic functional groups in the structural formula and acetates, trifluoroacetates, citrates, oxalates, tartrates, malates, nitrates, chlorides, bromides, iodides, sulfates, bisulfates, phosphates, lactates,

oleates, ascorbates, salicylates, formates, glutamates, methanesulfonates, ethanesulfonates, benzenesulfonates or p-toluenesulfonates formed by basic functional groups in the structure.

In a fourth aspect, the present application provides use of the linker-drug conjugate or the isomer, mesomer, racemate or enantiomer thereof or the mixture thereof or the pharmaceutically acceptable salt or solvate thereof according to any of the solutions described above, or the compound of formula X or the isomer, mesomer, racemate, or enantiomer thereof or the mixture thereof or the pharmaceutically acceptable salt or solvate thereof according to any of the solutions described above as an intermediate for the preparation of the ligand-drug conjugate of the present application.

In a fifth aspect, the present application provides a pharmaceutical composition comprising the ligand-drug conjugate or the pharmaceutically acceptable salt or solvate thereof of the present application, or the linker-drug conjugate or the isomer, mesomer, racemate, enantiomer thereof or the mixture thereof or the pharmaceutically acceptable salt or solvate thereof of the present application, or the compound of formula X or the isomer, mesomer, racemate or enantiomer thereof or the mixture thereof or the pharmaceutically acceptable salt or solvate thereof, and optionally further comprising a pharmaceutically acceptable carrier.

In a sixth aspect, the present application provides a pharmaceutical formulation comprising the ligand-drug conjugate or the pharmaceutically acceptable salt or solvate thereof of the present application, or the linker-drug conjugate or the isomer, mesomer, racemate, enantiomer thereof or the mixture thereof or the pharmaceutically acceptable salt or solvate thereof of the present application, or the compound of formula X or the isomer, mesomer, racemate or enantiomer thereof or the mixture thereof or the pharmaceutically acceptable salt or solvate thereof.

In a seven aspect, the present application provides use of the ligand-drug conjugate or the pharmaceutically acceptable salt or solvate thereof according to any of the solutions described above, or the linker-drug conjugate or the isomer, mesomer, racemate, enantiomer thereof or the mixture thereof or the pharmaceutically acceptable salt or solvate thereof according to any of the solutions described above, or the compound of formula X or the isomer, mesomer, racemate or enantiomer thereof or the mixture thereof or the pharmaceutically acceptable salt or solvate thereof according to any of the solutions described above in the preparation of a medicament for the treatment or prevention of a cancer or tumor.

In an eighth aspect, the present application provides a method of preventing or treating a cancer or tumor, comprising administering to a subject in need thereof a prophylactic or therapeutically effective amount of the ligand-drug conjugate or the pharmaceutically acceptable salt or solvate thereof of the present application, or the linker-drug conjugate or the isomer, mesomer, racemate, enantiomer thereof or the mixture thereof or the pharmaceutically acceptable salt or solvate thereof of the present application, or the compound of formula X or the isomer, mesomer, racemate or enantiomer thereof or the mixture thereof or the pharmaceutically acceptable salt or solvate thereof.

In the present application, the cancer or tumor may be a solid tumor or a hematological tumor, including but not limited to: adenocarcinoma, ovarian cancer, cervical cancer, uterine cancer, prostate cancer, kidney cancer, urinary tract cancer, bladder cancer, liver cancer, stomach cancer, endometrial cancer, salivary gland cancer, esophageal cancer, lung cancer (e.g., lung adenocarcinoma), colon cancer, rectal cancer, colorectal cancer, bone cancer, skin cancer (e.g., epidermal cancer), thyroid cancer, pancreatic cancer (e.g., pancreatic adenocarcinoma), melanoma, glioma, neuroblastoma, glioblastoma multiforme, sarcoma, lymphoma, leukemia, and hypopharyngeal cancer (e.g., hypopharyngeal squamous cell carcinoma).

## Definitions

Unless otherwise defined, all technical and scientific terms used herein are consistent with the common understanding of those of ordinary skill in the art to which the invention belongs. Although any methods and materials similar or equivalent to those described herein may be used to practice or test the invention, the invention describes preferred methods and materials. In describing and claiming the invention, the following terms are used in accordance with the definitions below.

When a trade name is used herein, the applicants intend to include formulations of a product of the trade name, generic drugs and active drug portions of the trade name.

Unless otherwise stated, the terms used in the specification and claims have the following meanings.

The term "ligand" is a targeting agent that specifically binds to a target moiety. The ligand is capable of specifically binding to a cellular component or to other target molecules of interest. The targeting moiety or target is usually on the cell surface. In some aspects, the ligand functions to deliver the drug unit to a specific target cell population with which the ligand unit interacts. The ligands include, but are not limited to, proteins, polypeptides and peptides, as well as non-proteins such as sugars. Suitable ligand units include, for example, antibodies, such as full-length (intact) antibodies and antigen-binding fragments thereof. In embodiments where the ligand unit is a non-antibody targeting agent, it may be a peptide or polypeptide, or a non-protein molecule. Examples of such targeting agents include interferons, lymphokines, hormones, growth factors and colony-stimulating factors, vitamins, nutrient transport molecules, or any other cell-binding molecules or substances. In some embodiments, the linker is covalently connected to the sulfur atom of the ligand. In some aspects, the sulfur atom is that of a cysteine residue that forms an interchain disulfide bond of the antibody. In another

aspect, the sulfur atom is a sulfur atom that has been introduced into a cysteine residue of the ligand unit, which forms an interchain disulfide bond of the antibody. In another aspect, the sulfur atom is a sulfur atom of a cysteine residue introduced into the ligand unit by, for example, site-directed mutagenesis or chemical reaction.

The term "drug" refers to a cytotoxic drug, i.e., a molecule that has a strong ability to destroy the normal growth of tumor or cancer cells. In principle, cytotoxic drugs can kill tumor cells at sufficiently high concentrations, but due to the lack of specificity, while killing tumor or cancer cells, they can also cause apoptosis of normal cells, thus easily leading to serious side effects.

The term "ligand-drug conjugate" refers to a molecule formed by connecting a ligand to a drug by a stable linking unit. In the present invention, the "ligand-drug conjugate" is preferably an antibody-drug conjugate (ADC), which refers to connecting a monoclonal antibody or a functional antibody fragment or a targeted protein to a cytotoxic drug by a stable linking unit.

The term "antibody" or "functional antibody fragment" includes, within its scope, any part of the antibody structure. This unit can bind, reactively associate, or complex a receptor, antigen, or other receptor unit present in the target cell population. The antibody may be any protein or proteinaceous molecule that binds, complexes, or reacts with a portion of the cell population to be treated or biomodified.

The antibodies of the present invention include but are not limited to mouse antibodies, chimeric antibodies, humanized antibodies and fully human antibodies, preferably humanized antibodies and fully human antibodies.

The three-letter and single-letter codes for amino acids used in the present invention are as described in J. boil. Chem. 1968, 243, 3558.

The term "natural amino acid" refers to amino acids that can be synthesized by organisms. Natural amino acids are generally L-type, but there are a few exceptions, such as glycine, including natural and biosynthesized glycine.

The term "unnatural amino acid" refers to amino acids that can only be synthesized artificially.

The term "alkyl" refers to a saturated aliphatic hydrocarbon group, which is a linear or branched chain group containing 1 to 20 carbon atoms, preferably an alkyl containing 1 to 12 carbon atoms, more preferably an alkyl containing 1 to 10 carbon atoms, most preferably an alkyl containing 1 to 6 or 1 to 4 carbon atoms. Non-limiting examples include methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl,n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, n-heptyl, 2-methylhexyl, 3-methylhexyl, 4-methylhexyl, 5-methylhexyl, 2,3-dimethylpentyl, 2,4-dimethylpentyl, 2,2-dimethylpentyl, 3,3-dimethylpentyl, 2-ethylpentyl, 3-ethylpentyl, n-octyl, 2,3-dimethylhexyl, 2,4-dimethylhexyl, 2,5-dimethylhexyl, 2,2-dimethylhexyl, 3,3-dimethylhexyl, 4,4-dimethylhexyl, 2-ethylhexyl, 3-ethylhexyl, 4-ethylhexyl, 2-methyl-2-ethylpentyl, 2-methyl-3-ethylpentyl, n-nonyl, 2-methyl-2-ethylhexyl, 2-methyl-3-ethylhexyl, 2,2-diethylpentyl, n-decyl, 3,3-diethylhexyl, 2,2-diethylhexyl, and various branched isomers thereof. More preferably, the alkyl group is a lower alkyl having 1 to 6 (e.g. 1 to 4) carbon atoms, and non-limiting examples include methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl and the like. The alkyl can be substituted or unsubstituted. When substituted, the substituent group(s) can be substituted at any available connection point. The substituent group(s) is preferably one or more groups independently selected from the group consisting of alkyl, alkenyl, alkynyl, alkoxy, alkylthio, alkylamino, halogen, thiol, hydroxy, nitro, cyano, cycloalkyl, heterocycloalkyl, aryl, heteroaryl, cycloalkoxy, heterocycloalkoxy, cycloalkylthio, heterocycloalkylthio, and oxo.

The term "substituted alkyl" means that the hydrogen in an alkyl is replaced by a substituent. Unless otherwise specified in the text, the substituent of the alkyl may be one or more groups selected from the group consisting of:-halogen, -OR', -NR'R", -SR', -SiR'R"R"', -OC(O)R', -C(O)R', $-CO_2R'$, -CONR'R", -OC(O)NR'R", -NR"C(O)R', -NR'-C(O)NR' 'R"', $-NR"C(O)_2R'$, $-NH-C(NH_2)=NH$, $-NR'C(NH_2)=NH$, $-NH-C(NH_2)=NR'$, -S(O)R', $-S(O)_2R'$, $-S(O)_2NR'R"$, $-NR'S(O)_2R"$, -CN and $-NO_2$, the number of substituents is 1 to (2m'+1), where m' is the total number of carbon atoms in the group, such as 1, 2, 3, 4, 5 or 6. R', R" and R"' respectively refer to hydrogen, $C_{1-8}$ alkyl, aryl, aryl substituted by 1-3 halogens, $C_{1-8}$ alkyl substituted by 1-3 halogens, $C_{1-8}$ alkoxy or $C_{1-8}$ thioalkoxy, or unsubstituted aryl-$C_{1-4}$ alkyl. When R' and R" are connected to the same nitrogen atom, they can form a 3-, 4-, 5-, 6- or 7-membered ring together with the nitrogen atom. For example, -NR'R" includes 1-pyrrolidinyl and 4-morpholinyl.

The term "heteroalkyl" refers to a group formed by substitution of one or more carbon atoms on an alkyl group with N, O, or S. The heteroalkyl preferably contains 1 to 12 carbon atoms, more preferably 1 to 10 carbon atoms, and most preferably 1 to 6 or 1 to 4 carbon atoms.

The term "cycloalkyl" refers to a saturated or partially unsaturated monocyclic or polycyclic ring-shaped hydrocarbon group, and the ring of the cycloalkyl contains 3 to 20 carbon atoms, preferably 3 to 12 carbon atoms, more preferably 3 to 10 carbon atoms, and most preferably 3 to 8 carbon atoms. Non-limiting examples of monocyclic cycloalkyl include cyclopropyl, cyclobutyl, cyclopentyl, cyclopentenyl, cyclohexyl, cyclohexenyl, cyclohexadienyl, cycloheptyl,

cycloheptatrienyl, and cyclooctyl. Non-limiting examples of polycyclic cycloalkyl include spiro, fused and bridged cycloalkyl groups, and each of the spiro, fused and bridged rings preferably contain 5 to 12 ring atoms. Optionally, one or more carbon atoms on the cycloalkyl may be replaced with one or more carbonyls.

The term "alkoxy" refers to an -O-(alkyl) and an -O-(cycloalkyl) group, wherein the alkyl and cycloalkyl are as defined above. The alkyl preferably contains 1 to 12 carbon atoms, more preferably 1 to 10 carbon atoms, most preferably 1 to 6 or 1 to 4 carbon atoms; the cycloalkyl contains 3 to 20 carbon atoms, preferably 3 to 12 carbon atoms, more preferably 3 to 10 carbon atoms, most preferably 3 to 8 carbon atoms. Non-limiting examples of alkoxy include: methoxy, ethoxy, propoxy, butoxy, cyclopropyloxy, cyclobutyloxy, cyclopentyloxy, and cyclohexyloxy. The alkoxy can be optionally substituted or unsubstituted. When substituted, the substituent group(s) is preferably one or more group(s) independently selected from the group consisting of alkyl, alkenyl, alkynyl, alkoxy, alkylthio, alkylamino, halogen, thiol, hydroxy, nitro, cyano, cycloalkyl, heterocycloalkyl, aryl, heteroaryl, cycloalkoxy, heterocycloalkoxy, cycloalkylthio, and heterocycloalkylthio.

The term "heterocycle" refers to a saturated or partially unsaturated monocyclic or polycyclic ring-shaped hydrocarbon containing 3 to 20 ring atoms, of which one or more (such as 1, 2, 3 or 4) ring atoms are heteroatoms selected from nitrogen, oxygen or $S(O)_m$ (wherein m is 0, 1 or 2), and the remaining ring atoms are carbon. Preferably, the heterocyclyl contains 3 to 12 ring atoms, of which 1 to 4 are heteroatoms. More preferably, the heterocyclyl contains 3 to 10 or 3 to 8 ring atoms. Non-limiting examples of monocyclic heterocyclyl include pyrrolidinyl, piperidinyl, piperazinyl, morpholinyl, thiomorpholinyl, and homopiperazinyl. Non-limiting examples of polycyclic heterocyclyl include spiro, fused and bridged heterocyclyls, and each of the spiro, fused and bridged rings preferably contain 5 to 12 ring atoms. Optionally, one or more carbon atoms on the heterocyclyl may be replaced with one or more carbonyls.

The term "aryl" refers to a 6- to 14-membered all-carbon monocyclic or fused polycyclic (i.e., rings that shares adjacent pairs of carbon atoms) group having a conjugated $\pi$ electron system, preferably 6- to 10-membered group, such as phenyl. The aryl may be substituted or unsubstituted. When the aryl is substituted, the substituent may be one or more of the following groups, selected from, but not limited to, alkyl, alkenyl, alkynyl, alkoxy, alkylthio, alkylamino, halogen, deuterium atom, mercapto, hydroxyl, nitro, cyano, cycloalkyl, heterocycloalkyl, aryl, heteroaryl, cycloalkyloxy, heterocycloalkyloxy, cycloalkylthio, and heterocycloalkylthio.

The term "heteroaryl" includes 5- to 8-membered monocyclic heteroaryls and 8- to 12-membered fused heteroaryls.

The term "5- to 8-membered monocyclic heteroaryl" refers to an aromatic monocyclic ring group containing 5 to 8 ring atoms (at least one of which is a heteroatom, such as a nitrogen atom, an oxygen atom or a sulfur atom). Optionally, the ring atoms (such as carbon atoms, nitrogen atoms or sulfur atoms) in the ring structure may be oxo-substituted. "5- to 8-membered monocyclic heteroaryl" includes, such as, "5- to 7-membered monocyclic heteroaryl", "5- to 6-membered monocyclic heteroaryl", "5- to 6-membered monocyclic nitrogen-containing heteroaryl", "6-membered monocyclic nitrogen-containing heteroaryl", etc., wherein the heteroatom in the "nitrogen-containing heteroaryl" contains at least one nitrogen atom, for example, only 1 or 2 nitrogen atoms, or, contains one nitrogen atom and other 1 or 2 heteroatoms (for example, oxygen atoms and/or sulfur atoms), or, contains 2 nitrogen atoms and other 1 or 2 heteroatoms (for example, oxygen atoms and/or sulfur atoms). Specific examples of "5- to 8-membered monocyclic heteroaryl" include but are not limited to furyl, thienyl, pyrrolyl, thiazolyl, isothiazolyl, thiadiazolyl, oxazolyl, isoxazolyl, oxadiazolyl, imidazolyl, pyrazolyl, 1,2,3-triazole base, 1,2,4-triazolyl,1,2,3-oxadiazolyl,1,2,4-oxadiazolyl,1,2,5-oxadiazolyl,1,3,4 -oxadiazolyl, pyridyl, 2-pyridonyl, 4-pyridonyl, pyrimidinyl, pyridazinyl, pyrazinyl, 1,2,3-triazinyl, 1,3,5-triazine base, 1,2,4,5-tetrazinyl, azepantrienyl, 1,3-diazacycloheptatrienyl, azepantetraenyl, etc.

The term "8- to12-membered fused heteroaryl" refers to an unsaturated aromatic ring structure containing 8 to 12 ring atoms (at least one of which is a heteroatom, such as a nitrogen atom, an oxygen atom or a sulfur atom), which is formed by two or more ring structures sharing two adjacent atoms. Optionally, the ring atoms (such as carbon atoms, nitrogen atoms or sulfur atoms) in the ring structure may be oxo-substituted. "8- to 12-membered fused heteroaryl" includes "8- to 10-membered fused heteroaryl" and "8- to 9-membered fused heteroaryl", etc.; specific examples include but are not limited to: pyrrolopyrrole, pyrrolofuran, pyrazopyrrole, pyrazothiophene, furothiophene, pyrazoxazole, benzofuryl, benzisofuryl, benzothienyl, indolyl, isoindolyl, benzoxazolyl, benzimidazolyl, indazolyl, benzotriazolyl, quinolinyl, 2-quinolinonyl, 4-quinolinonyl, 1-isoquinolinonyl, Isoquinolinyl, acridinyl, phenanthridinyl, benzopyridazinyl, phthalazinyl, quinazolinyl, quinoxalinyl, purinyl, and naphthyridinyl.

The term "haloalkyl" refers to an alkyl substituted by one or more halogens, where the alkyl is as defined above.

The term "deuterated alkyl" refers to an alkyl substituted by one or more deuterium atoms, where the alkyl is as defined above.

The term "hydroxy" refers to an -OH group.

The term "halogen" refers to fluorine, chlorine, bromine or iodine.

The term "amino" refers to a $-NH_2$ group.

The term "nitro" refers to a $-NO_2$ group.

The term "acyl" refers to the group formed by a carbonyl with an alkyl, alkoxy or amido, including alkylcarbonyl

groups, ester groups and amide groups.

The term "substituted" means that the hydrogen in a group is replaced with a substituent, and "substituted" includes the implicit condition that such substitution conforms to the allowed valences of the substituted atom and the substituent, and that the substitution results in a stable compound. The substituent can be found in the groups listed above. Optionally, two substituents on the same substituted atom may form a cyclic group, which may be a cycloalkyl or a heterocyclyl, such as a lactam group or a lactone group.

The term "derivative" refers to a substance that has a chemical structure similar to that of a compound but also contains at least one chemical group that is not present in the compound and/or lacks at least one chemical group that is present in the compound. The compound to which the derivative is compared is called "parent" compound. Typically, a "derivative" may be produced from a parent compound in one or more chemical reaction steps.

The term "pharmaceutically acceptable salt" refers to a pharmaceutically acceptable organic or inorganic salt of a compound (such as, a drug, a linker-drug compound, or a ligand-drug conjugate). The compound or conjugate may contain at least one amino or carboxyl group and can therefore form addition salts with the corresponding acid or alkali. Exemplary salts include, but are not limited to sulfate, trifluoroacetate, citrate, acetate, oxalate, chloride, bromide, iodide, nitrate, bisulfate, phosphate, acid phosphate, isonicotinate, lactate, salicylate, acid citrate, tartrate, oleate, tannate, pantothenate, bitartrate, ascorbate, salicylate, formate, benzoate, glutamate, methanesulfonate, ethanesulfonate, benzenesulfonate, p-toluenesulfonate, potassium salt, and sodium salt.

The term "solvate" refers to a compound formed by a linker-drug compound or ligand-drug conjugate of the present invention and one or more solvent molecules, where the solvent molecules include but are not limited to water, ethanol, acetonitrile, isopropanol, DMSO, and ethyl acetate.

The term "pharmaceutical composition" refers to a mixture containing one or more compounds of the present invention or physiologically/pharmaceutically acceptable salts or prodrugs thereof and other chemical components, as well as other components such as physiologically/pharmaceutically acceptable carriers and/or excipients. The purpose of the pharmaceutical composition is to promote the administration of drugs to organisms, facilitate the absorption of active ingredients and thus exert biological activity.

The term "carrier" refers to a system that can change the way drugs enter the human body and their distribution in the body, control the release rate of drugs and deliver drugs to the target. Drug carrier release and targeting systems can reduce drug degradation and loss, reduce side effects and improve bioavailability.

The term "excipient" refers to an additive or excipient other than the main drug in a pharmaceutical preparation. Such as adhesives, fillers, disintegrants, lubricants in tablets; matrix backup in semi-solid preparations ointments and creams; preservatives, antioxidants, flavoring agents, fragrances, cosolvents, emulsifiers, permeability enhancers, osmotic pressure regulators, colorants, etc. in liquid preparations can all be called excipients.

The term "diluent" or "filler" is mainly used to increase the weight and/or volume of the preparation. The addition of diluent not only ensures a certain volume, but also reduces the dosage deviation of the main ingredients and improves the compression molding of the drug.

## BRIEF DESCRIPTION OF THE DRAWINGS

FIG. 1A, FIG. 1B, FIG. 1C, FIG. 1D, FIG. 1E and FIG. 1F show the anti-tumor effects of the compounds in BxPC-3, SW620, H1975, HCC827, FaDu and A431 cell models, respectively.

FIG. 2A, FIG. 2B, FIG. 2C, FIG. 2D and FIG. 2E show the anti-tumor effect of ADCs in A431, HCC827, FaDu, N87 and SW620 cell models, respectively.

FIG. 3 shows the anti-tumor effect of ADCs in the heterogeneous tumor cell line A431+ SW620.

## DETAILED DESCRIPTION OF THE EMBODIMENTS

The present invention is further described below in conjunction with specific embodiments. It should be understood that these embodiments are only used to illustrate the invention and are not used to limit the scope of the invention. The experimental methods in the following embodiments that do not specify specific conditions are usually carried out under conventional conditions or under conditions recommended by the manufacturers. Unless otherwise specified, all percentages, proportions, ratios, or parts are calculated by weight. Unless otherwise defined, all professional and scientific terms used herein have the same meaning as is familiar to those skilled in the art. In addition, any methods and materials similar or equivalent to those described can be applied to the methods of the present invention. The preferred implementation methods and materials described herein are for demonstration purposes only.

The general procedures taken in the following examples of the present invention are:

General procedure A

Preparation of ADCs by conjugation

Antibody molecules with a monomer rate of more than 95% after preliminary purification were exchanged into phosphate buffer containing EDTA using an ultrafiltration centrifuge tube, the concentration being 10 mg/mL. TCEP with an amount 10 times the number of moles of the antibody was added for reaction at room temperature for 6 h. The interchain disulfide bonds of the antibody were opened, and the number of free thiol groups was determined using Ellman's method to determine whether all disulfide bonds were opened. Then, the payload with an amount 10 times the number of moles of the antibody was added for reaction at room temperature for 6 h. After the reaction ended, the reaction system was exchanged into PBS using an ultrafiltration centrifuge tube with a molecular weight cut-off of 30 KDa, and the unconjugated payload was removed, thus obtaining a conjugated ADC (DAR=8).

General procedure B

Preparation of ADCs by site-specific conjugation

Antibody molecules with a monomer rate of more than 95% after preliminary purification were exchanged into phosphate buffer containing EDTA using an ultrafiltration centrifuge tube, the concentration being 10 mg/mL. TCEP with an amount of 8 times the number of moles of the antibody was added for reaction at room temperature for 3 h. The reaction system was exchanged into phosphate buffer at pH 6.5 using an ultrafiltration centrifuge tube, and then DHAA (dehydroascorbic acid) with an amount 8 times the number of moles of the antibody was added for reaction at room temperature for 3 h. Then, the payload with an amount 5 times the number of moles of the antibody was added for reaction at room temperature for 3 h. After the reaction ended, the reaction system was exchanged into PBS using an ultrafiltration centrifuge tube with a molecular weight cut-off of 30 KDa, and the unconjugated payload was removed, thus obtaining a site-specific conjugated ADC (DAR=2).

General procedure C

Preparation of ADCs by conjugation

Antibody molecules with a monomer rate of more than 95% after preliminary purification were exchanged into 20 mM/sodium acetate/pH 6.0 buffer solution using an ultrafiltration centrifuge tube, the concentration being greater than 5 mg/mL. During the preparation of an ADC, an appropriate amount of the antibody after buffer exchange was placed in a reaction tube, TCEP (0.1M) with an amount 3.6 times the molar amount of the antibody was then added, the reaction tube was placed in a 25 °C constant temperature incubator for reaction for 2 h, the reaction tube was then taken out, a Linker-drug with an amount 5 times the molar amount was added and mixed well, and the reaction tube was placed in a 25 °C constant temperature incubator for reaction for 2 h. After the reaction ended, the sample solution was taken out and exchanged into 20 mM/histidine/pH 5.3 storage buffer using an ultrafiltration centrifuge tube with a molecular weight cutoff of 30 KDa, and the unconjugated payload was removed, thus obtaining a conjugated ADC (DAR=4).

Example 1 Synthesis of compound D-2

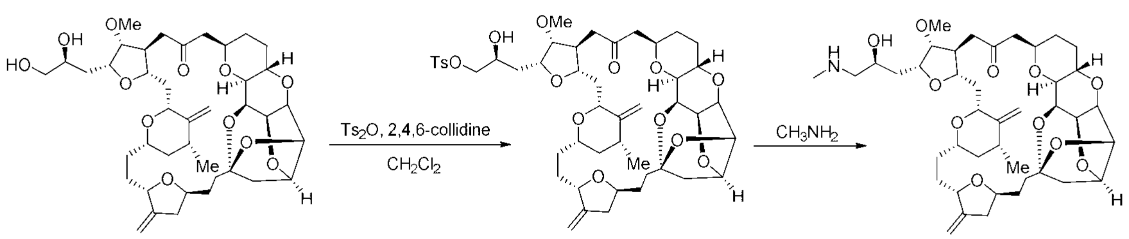


D-1

D-2

In a 25 mL single-neck flask, compound D-1 (80 mg, 0.109 mmol, 1.0 eq) was added and dissolved in dried dichloromethane (4 mL), followed by addition of 2,4,6-trimethylpyridine (58 μL, 0.438 mmol, 4.0 eq) and pyridine (0.44 μL, 0.005 mmol, 0.05 eq), and the reaction solution was cooled to -10 °C. P-toluenesulfonic anhydride (89.3 mg, 0.274 mmol,

2.5 eq) was added under the protection of nitrogen, and the reaction was continued at -10 °C for 20 h. Then, water (310 μL) was added to the reaction solution. After the reaction solution was brought naturally to room temperature and was stirred for 30 min, methylamine (methanol solution, 4 mL) was added, and the reaction was continued at room temperature for 46 h. The reaction solution was then concentrated under reduced pressure. The purification was performed by reverse-phase preparative column chromatography, and the preparation solution was lyophilized to give compound **D-2** (white solid, 58 mg, 71%). LC-MS m/z(ES+): [M+H]+: 744.2.

Example 2 Synthesis of compound D-3

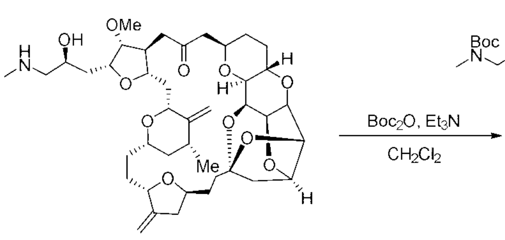


D-2 D-3

In a 25 mL single-neck flask, compound D-2 (55 mg, 0.074 mmol, 1.0 eq) was added and dissolved in dried dichloromethane (3 mL), followed by addition of triethylamine (15.4 μL, 0.111 mmol, 1.5 eq) and di-tert-butyl decarbonate (20.4 μL, 0.089 mmol, 1.2 eq), and the reaction was allowed at room temperature for 2 h. After the reaction was found to be completed from the monitoring of the TLC, the reaction solution was concentrated under reduced pressure. The purification was performed by reverse-phase preparative column chromatography, and the preparation solution was lyophilized to give compound **D-3** (white solid, 43 mg, 69%). LC-MS m/z(ES+): [M+H]+: 844.2.

Example 3 Synthesis of compound **D-4** (eribulin)

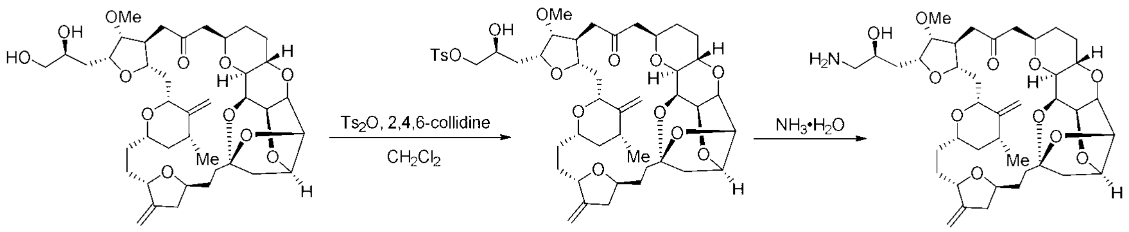


D-1 D-4

In a 25 mL single-neck flask, compound D-1 (80 mg, 0.109 mmol, 1.0 eq) was added and dissolved in dried dichloromethane (4 mL), followed by addition of 2,4,6-trimethylpyridine (58 μL, 0.438 mmol, 4.0 eq) and pyridine (0.44 μL, 0.005 mmol, 0.05 eq), and the reaction solution was cooled to -10 °C. P-toluenesulfonic anhydride (89.3 mg, 0.274 mmol, 2.5 eq) was added under the protection of nitrogen, and the reaction was continued at -10°C for 20 h. Then, water (310 μL) was added to the reaction solution. After the reaction solution was brought naturally to room temperature and was stirred for 30 min, isopropanol (4 mL) and ammonium hydroxide (4 mL) were added, and the reaction was continued at room temperature for 3 days. The reaction solution was then concentrated under reduced pressure. The purification was performed by reverse-phase preparative column chromatography, and the preparation solution was lyophilized to give compound **D-4** (white solid, 56 mg, 70%). LC-MS m/z(ES+): [M+H]+: 730.2.

Example 4 Synthesis of compound **D-5**

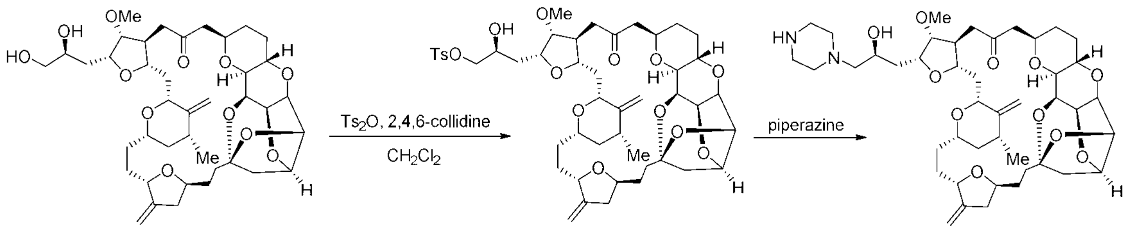


D-1

D-5

In a 25 mL single-neck flask, compound **D-1** (80 mg, 0.109 mmol, 1.0 eq) was added and dissolved in dried dichloromethane (4 mL), followed by addition of 2,4,6-trimethylpyridine (58 μL, 0.438 mmol, 4.0 eq) and pyridine (0.44 μL, 0.005 mmol, 0.05 eq), and the reaction solution was cooled to -10 °C. P-toluenesulfonic anhydride (89.3 mg, 0.274 mmol, 2.5 eq) was added under the protection of nitrogen, and the reaction was continued at -10 °C for 20 h. Then, water (310 μL) was added to the reaction solution. After the reaction solution was brought naturally to room temperature and was stirred for 30 min, methanol (4 mL) and piperazine (943 mg, 10.9 mmol, 100.0 eq) were added, and the reaction was continued at room temperature for 3 days. The reaction solution was then concentrated under reduced pressure. The purification was performed by reverse-phase preparative column chromatography, and the preparation solution was lyophilized to give compound **D-5** (white solid, 66 mg, 75%). LC-MS m/z(ES$^+$): [M+H]$^+$: 799.2.

Example 5 Synthesis of compound **D-6**

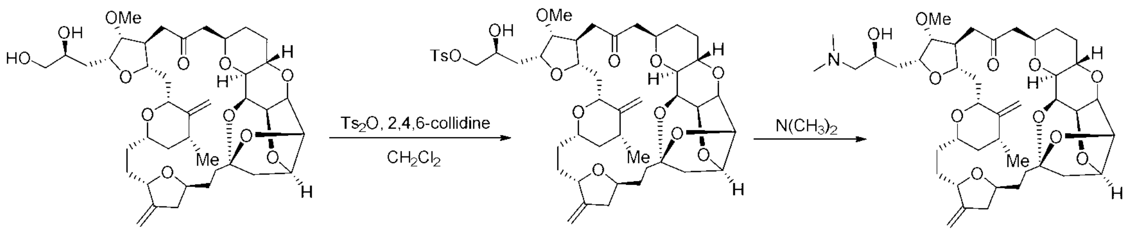


D-1

D-6

In a 25 mL single-neck flask, compound **D-1** (80 mg, 0.109 mmol, 1.0 eq) was added and dissolved in dried dichloromethane (4 mL), followed by addition of 2,4,6-trimethylpyridine (58 μL, 0.438 mmol, 4.0 eq) and pyridine (0.44 μL, 0.005 mmol, 0.05 eq), and the reaction solution was cooled to -10 °C. P-toluenesulfonic anhydride (89.3 mg, 0.274 mmol, 2.5 eq) was added under the protection of nitrogen, and the reaction was continued at -10 °C for 20 h. Then, water (310 μL) was added to the reaction solution. After the reaction solution was warmed to room temperature and was stirred for 30 min, dimethylamine (methanol solution, 8 mL) was added, and the reaction was continued at room temperature for 40 h. The reaction solution was then concentrated under reduced pressure. The purification was performed by reverse-phase preparative column chromatography, and the preparation solution was lyophilized to give compound **D-6** (white solid, 60 mg, 72%). LC-MS m/z(ES$^+$): [M+H]$^+$: 758.4.

Example 6 Synthesis of compound **D-7**

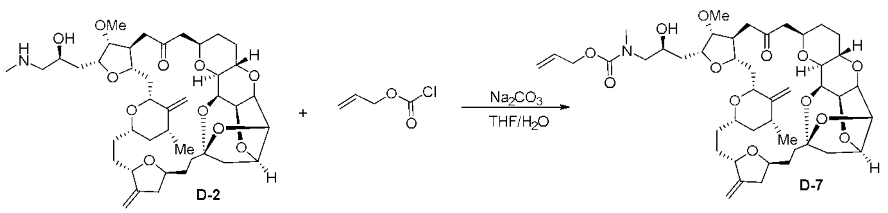


In a 25 mL single-neck flask, compound **D-2** (20 mg, 0.027 mmol, 1.0 eq) was added and dissolved in tetrahydrofuran and water (2 mL), followed by addition of sodium carbonate (28 mg, 0.269 mmol, 10 eq) and allyl

chloroformate (5.7 μL, 0.054 mmol, 2.0 eq), and the reaction was allowed at room temperature for 2 h. After the reaction was found to be completed from the monitoring of the TLC, the reaction solution was purified by reverse-phase preparative column chromatography, and the preparation solution was lyophilized to give compound **D-7** (white solid, 17 mg, 76%). LC-MS m/z(ES$^+$): [M+H]$^+$: 828.4.

Example 7 Synthesis of compound **D-8**

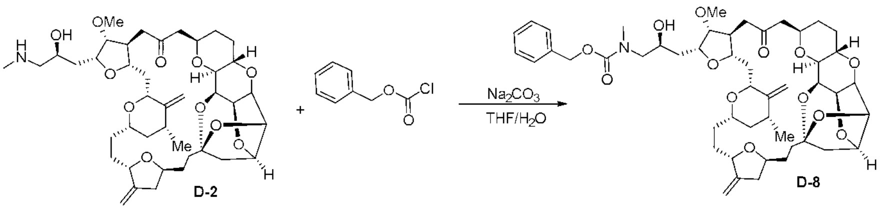


Compound **D-8** was synthesized from compound **D-2** and benzyl chloroformate according to the synthesis method of Example 6. LC-MS m/z(ES$^+$): [M+H]$^+$:878.3.

Example 8 Synthesis of compound **D-9**

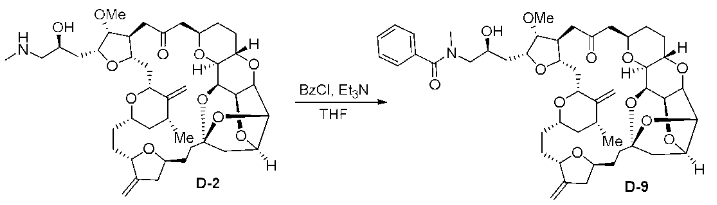


In a 25 mL single-neck flask, compound **D-2** (20 mg, 0.027 mmol, 1.0 eq) was added and dissolved in dry tetrahydrofuran (2 mL), followed by addition of triethylamine (7.5 μL, 0.054 mmol, 2.0 eq) and benzoyl chloride (6.2 μL, 0.054 mmol, 2.0 eq), and the reaction was allowed at room temperature for 2 h. After the reaction was found to be completed from the monitoring of the TLC, the reaction solution was purified by reverse-phase preparative column chromatography, and the preparation solution was lyophilized to give compound **D-9** (white solid, 18 mg, 79%). LC-MS m/z(ES$^+$): [M+H]$^+$: 848.4.

Example 9 Synthesis of compound **D-10**

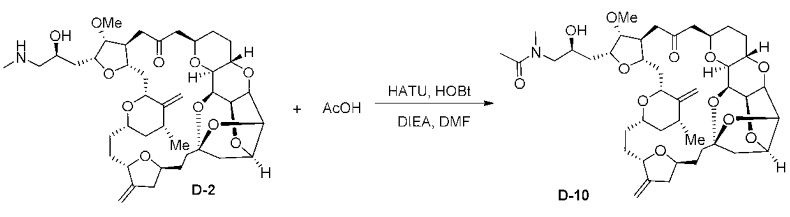


In a 25 mL single-neck flask, compound **D-2** (20 mg, 0.027 mmol, 1.0 eq), HATU (20 mg, 0.054 mmol, 2.0 eq), HOBt (7.3 mg, 0.054 mmol, 2.0 eq), and dried DMF (2 mL) were added and dissolved uniformly. In condition of an ice water bath, glacial acetic acid (2.3 μL, 0.040 mmol, 1.5 eq) and DIEA (9.4 μL, 0.054 mmol, 2.0 eq) were added sequentially dropwise, and reaction was allowed at room temperature. After 30 min, HPLC showed the reaction was complete. The reaction solution was purified by reverse-phase preparative column chromatography. The preparation solution was lyophilized to give compound **D-10** (white solid, 14 mg, 66%). LC-MS m/z(ES$^+$): [M+H]$^+$: 886.3.

Example 10 Synthesis of compound **D-11**

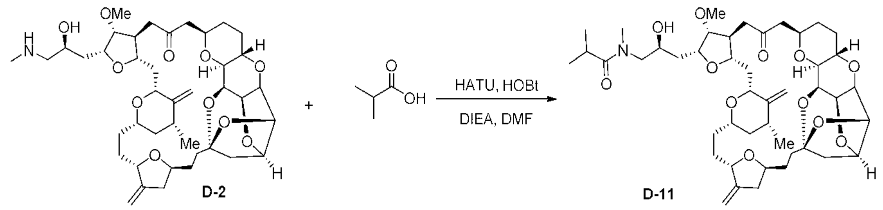


Compound **D-11** was synthesized from compound **D-2** and isobutyric acid according to the synthesis method of Example 9. LC-MS m/z(ES$^+$): [M+H]$^+$:814.4.

Example 11 Synthesis of compound **D-12**

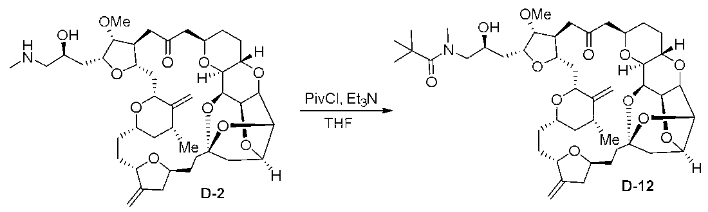


Compound **D-12** was synthesized from compound **D-2** and pivaloyl chloride according to the synthesis method of Example 8. LC-MS m/z(ES$^+$): [M+H]$^+$:828.3.

Example 12 Synthesis of compound **D-13**

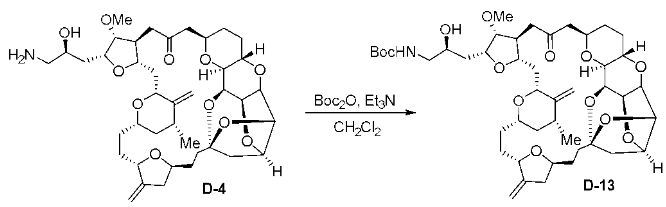


Compound **D-13** was synthesized from compound **D-4** according to the synthesis method of Example 2. LC-MS m/z(ES$^+$): [M+H]$^+$:830.3.

Example 13 Synthesis of compound **D-14**

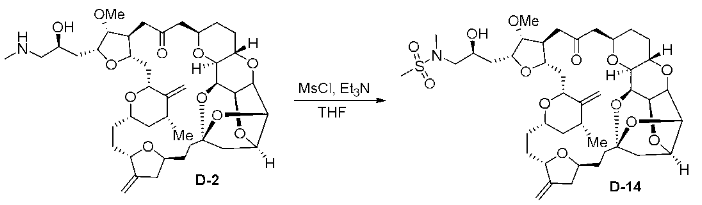


Compound **D-14** was synthesized from compound **D-2** and methanesulfonyl chloride according to the synthesis method of Example 8. LC-MS m/z(ES$^+$): [M+H]$^+$:822.3.

Example 14 Synthesis of compound **D-15**

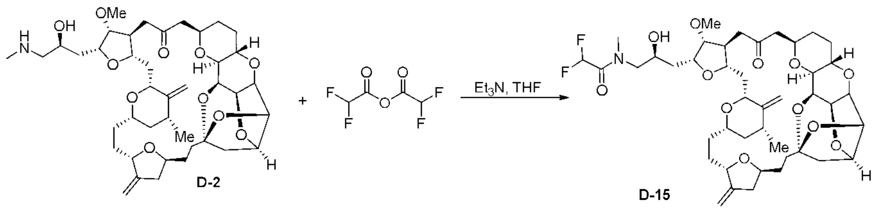


Compound **D-15** was synthesized from compound **D-2** and difluoroacetic anhydride according to the synthesis method of Example 8. LC-MS m/z(ES$^+$): [M+H]$^+$:822.3.

Example 15 Synthesis of compound **D-16**

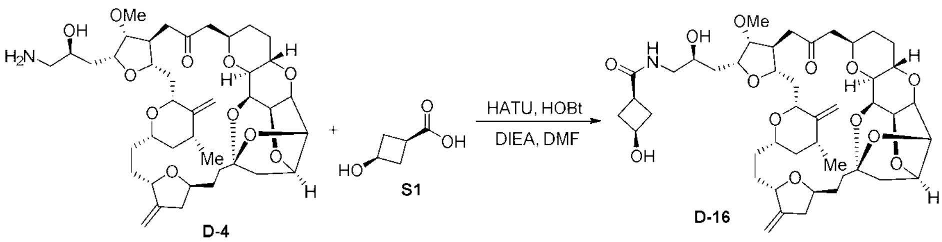


Compound **D-16** was synthesized from compound **D-4** and cis-3-hydroxycyclobutanecarboxylic acid **S1** according to the synthesis method of Example 9. LC-MS m/z(ES$^+$): [M+H]$^+$:828.3.

Example 16 Synthesis of compound **D-17**

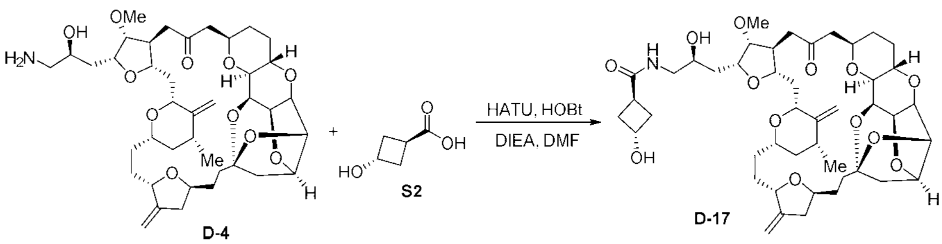


Compound **D-17** was synthesized from compound **D-4** and trans-3-hydroxycyclobutanecarboxylic acid **S2** according to the synthesis method of Example 9. LC-MS m/z(ES$^+$): [M+H]$^+$:828.3.

Example 17 Synthesis of compound **D-18**

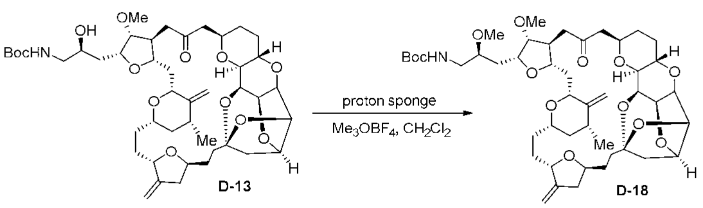

Compound **D-13** (61 mg, 0.074 mmol, 1.0 eq) was weighed and placed into a 25 mL single-neck flask and dissolved in dried dichloromethane (6 mL), followed by addition of 1,8-bis(dimethylamino)naphthalene (48 mg, 0.222 mmol, 3.0 eq) and trimethyloxonium tetrafluoroborate (33 mg , 0.222 mmol, 3.0 eq), and the reaction was allowed at room temperature overnight. After the reaction was found to be completed from the monitoring of the TLC, the reaction solution was filtered, concentrated under reduced pressure, and purified by TLC to give compound **D-18** (a white vesicular solid, 56 mg, 90%). LC-MS m/z(ES$^+$): [M+H]$^+$: 844.4.

Example 18 Synthesis of compound **D-19**

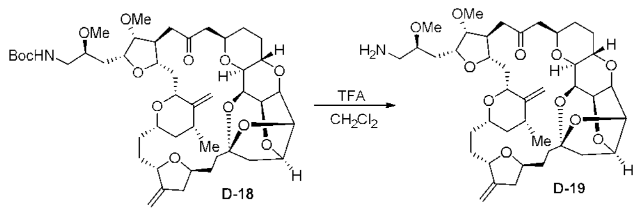


Compound **D-18** (56 mg, 0.066 mmol, 1.0 eq) was dissolved in dried dichloromethane (4 mL), followed by addition of trifluoroacetic acid (200 μL), and the reaction was allowed at room temperature for 40 min. After the reaction was found to be completed from the monitoring of the TLC, the reaction solution was adjusted to pH 8 with sodium bicarbonate, extracted with dichloromethane, dried, filtered, and concentrated to give a crude product of compound D-19.

Example 19 Synthesis of compound **D-20**

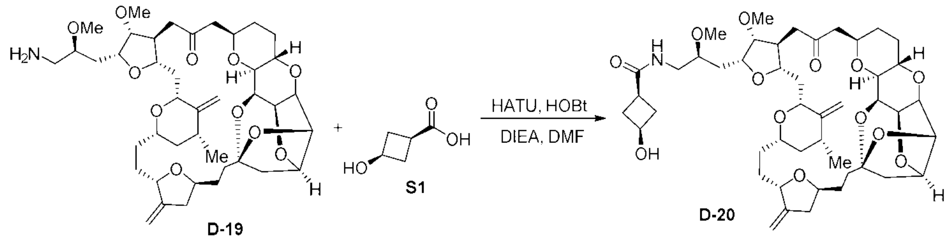


Compound **D-20** was synthesized from compound **D-19** and cis-3-hydroxycyclobutanecarboxylic acid **S1** according to the synthesis method of Example 9. LC-MS m/z(ES$^+$): [M+H]$^+$:842.3.

Example 20 Synthesis of compound **S5**

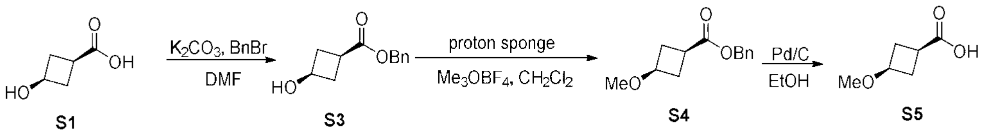


Step 1: compound **S3**

Cis-3-hydroxycyclobutanecarboxylic acid **S1** (150 mg, 1.29 mmol, 1.0 eq) and potassium carbonate (536 mg, 3.88 mmol, 3.0 eq) were weighed and placed into a 25 mL single-neck flask, followed by addition of DMF (3 mL) and benzyl bromide (230 μL, 1.94 mmol, 1.5 eq), and the reaction was allowed at room temperature for 2 h. After the reaction was found to be completed from the monitoring of the TLC, water was added to the reaction solution, which was then extracted with ethyl acetate, dried, filtered, concentrated, and purified by column chromatography (dichloromethane/methanol = 30:1) to give compound **S3** (yellow oil, 207 mg, 78%).

Step 2: compound **S4**

Compound **S3** (173 mg, 0.839 mmol, 1.0 eq) was weighed and placed into a 25 mL single-neck flask and dissolved in dried dichloromethane (5 mL), followed by addition of 1,8-bis(dimethylamino)naphthalene (539 mg, 2.52 mmol, 3.0 eq) and trimethyloxonium tetrafluoroborate (372 mg , 2.52 mmol, 3.0 eq), and the reaction was allowed at room temperature overnight. After the reaction was found to be completed from the monitoring of the TLC, The reaction solution was filtered, concentrated under reduced pressure, and purified by column chromatography (petroleum ether: ethyl acetate = 6:1) to give compound **S4** (colorless oily substance, 168 mg, 91%).

Step **2:** compound **S5**

Compound **S4** (6 mg, 0.027 mmol, 1.0 eq) was weighed and placed into a 25 mL single-neck flask and dissolved in anhydrous ethanol (1 mL), followed by addition of 5% Pd/C (3 mg) and the reaction was allowed at room temperature for 2 h. After the reaction was found to be completed from the monitoring of the TLC, the resulting reaction solution was filtered and concentrated to give a crude product of compound **S5.**

Example 21 Synthesis of compound **D-21**

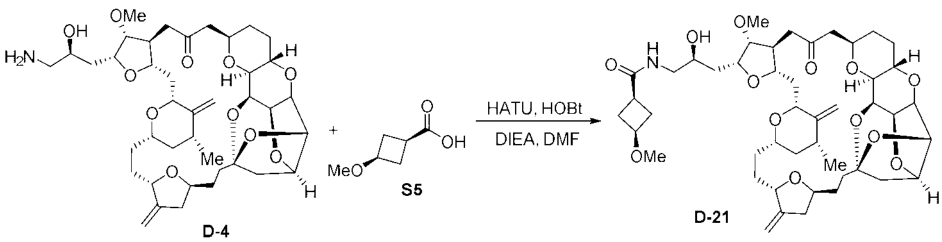


Compound **D-21** was synthesized from compound **D-4** and cis-3-methoxycyclobutanecarboxylic acid **S5** according to the synthesis method of Example 9. LC-MS m/z(ES$^+$): [M+H]$^+$:842.3.

Example 22 Synthesis of compound **S7**

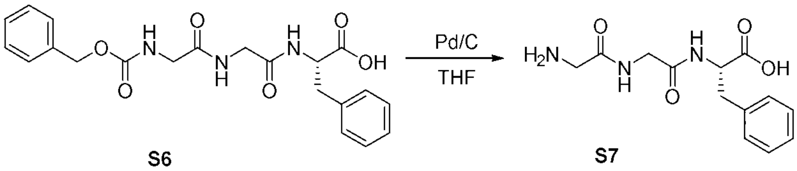


In a 25 mL single-neck flask, compound **S6** (2.0 g, 4.84 mmol, 1.0 eq, synthesis method refers to the patent CN111051330 A) and dry tetrahydrofuran (4 mL) were added and dissolved completely, then 5% Pd/C (100 mg) was added, and the hydrogenation reaction was carried out overnight. After completion, the mixture was filtered, and the filtrate was concentrated to give compound **S7** (white solid, 1.17 g, 87%). LC-MS m/z(ES$^+$): [M+H]$^+$: 280.1.

Example 23 Synthesis of compound **S9**

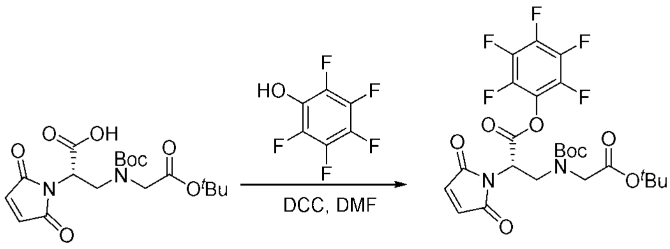


S8 S9

In a 25 mL single-neck flask, compound **S8** (1.0 g, 2.51 mmol, 1.0 eq, synthesis method refers to the synthesis of compound M2 in patent application CN113827736 A), pentafluorophenol (508 mg, 2.76 mmol, 1.1 eq), DCC (570 mg, 2.76 mmol, 1.1 eq) and DMF (8 mL) were added, and the reaction was allowed at room temperature for 2 h. After the reaction was found to be completed from the monitoring of the TLC, the reaction solution was filtered to give the crude filtrate of compound S9 for later use. LC-MS m/z(ES$^+$): [M+H]$^+$: 565.1.

Example 24 Synthesis of compound **S10**

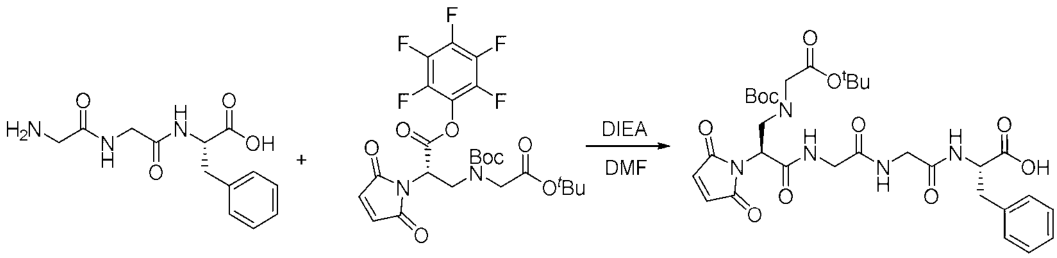


S7 S9 S10

Compound **S7** (771 mg, 2.76 mmol, 1.1 eq), DMF (8 mL) and DIEA (656 μL, 3.76 mmol, 1.5 eq) were added in a 50 mL single-neck flask, and the crude filtrate of the above compound **S9** was added in an ice-water bath, and the reaction was allowed at room temperature overnight. The reaction solution was purified by reverse-phase preparative column chromatography, and the preparation solution was lyophilized to give compound **S10** (white solid, 778 mg, 47%). LC-MS m/z(ES$^+$): [M+H]$^+$: 660.2.

Example 25 Synthesis of compound **S12**

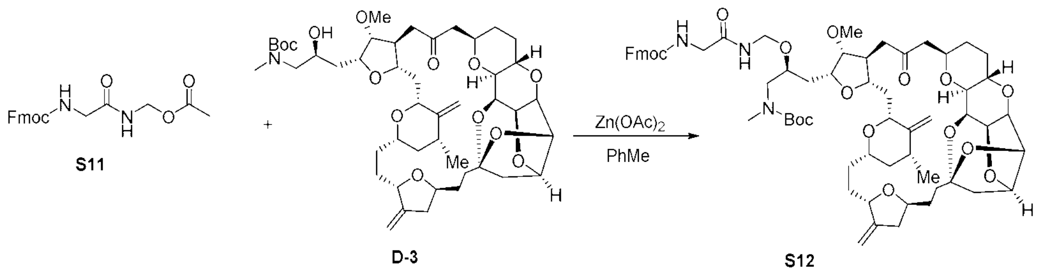


S11 D-3 S12

Compound **D-3** (40 mg, 0.047 mmol, 1.0 eq), compound **S11** (52.4 mg, 0.142 mmol, 3.0 eq, synthesis method refers to the synthesis of compound **1** in patent CN111686259 A), zinc acetate (26.1 mg, 0.142 mmol, 3.0 eq) and 4Å molecular sieve (60 mg) were added in sequence in a 25 mL single-neck flask, followed by addition of dry toluene (4 mL), and the reaction mixture was replaced with nitrogen three times, then heated with stirring at 100 °C. After 2 hours, the reaction was terminated, allowed to cool to room temperature, filtered, and the filtrate was concentrated under reduced pressure. The purification was performed by reverse-phase preparative column chromatography, and the preparation solution was lyophilized to give compound **S12** (white solid, 37 mg, 68%). LC-MS m/z(ES$^+$): [M+Na]$^+$: 1174.2.

Example 26 Synthesis of compound **S13**

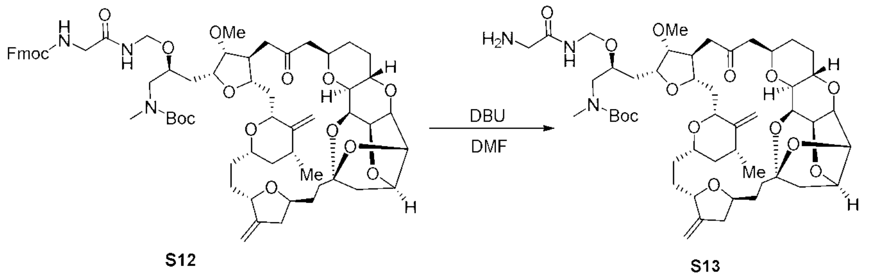


S12 S13

In a 25 mL single-neck flask, compound **S12** (30 mg, 0.026 mmol, 1.0 eq) and dried DMF (1.5 mL) were added and dissolved completely, then DBU (4.3 μL, 0.029 mmol, 1.1 eq) was added, and the reaction was allowed at room temperature. The consumption of compound **S12** was found to be completed from the monitoring of the TLC, and no post-treatment was needed for reaction. The next reaction was carried out directly with a crude solution of compound **S13.** LC-MS m/z(ES$^+$): [M+Na]$^+$: 953.3.

Example 27 Synthesis of compound **S14**

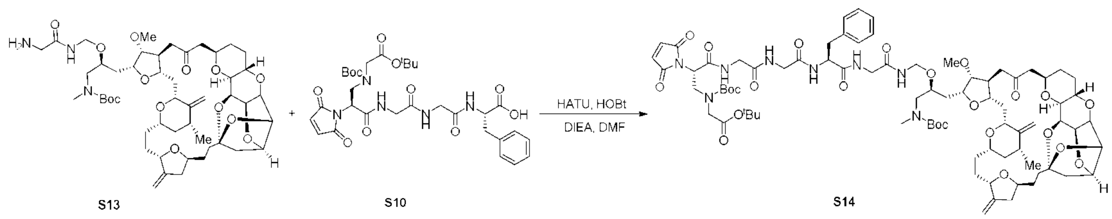


S13 S10 S14

In another 25 mL single-neck flask, compound **S10** (18.9 mg, 0.029 mmol, 1.1 eq), HATU (11.9 mg, 0.031 mmol, 1.2 eq), HOBt (4.2 mg, 0.031 mmol, 1.2 eq) and dried DMF (1.5 mL) were added sequentially and dissolved uniformly, then the above crude solution of compound **S13** and DIEA (5.4 μL, 0.031 mmol, 1.2 eq) was added dropwise in condition of an ice water bath, and the reaction was allowed at room temperature. After 30 min, HPLC showed the completion of the reaction. The reaction solution was purified by reverse-phase preparative column chromatography, and the preparation solution was lyophilized to give compound **S14** (white solid, 23 mg, 56%). LC-MS m/z(ES$^+$): [M-99]$^+$: 1472.2 (fragment ion of compound **S14** after removing the Boc protecting group and adding hydrogen ion).

Example 28 Synthesis of compound **L1**

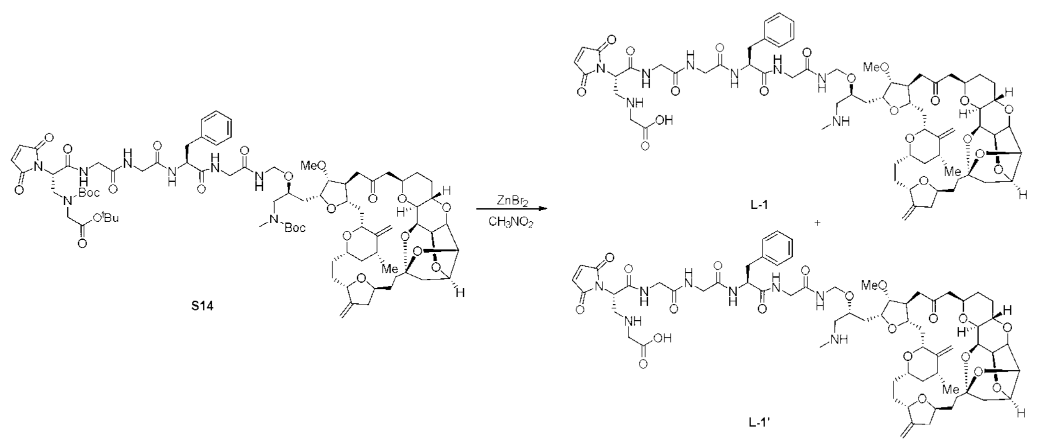


S14 L-1 + L-1'

In a 25 mL single-neck flask, compound **S14** (20 mg, 0.013 mmol, 1.0 eq) was completely dissolved in nitromethane (2 mL), then zinc bromide (57 mg, 0.25 mmol, 20.0 eq) was added, and the reaction was allowed at room temperature for 30 min and monitored by HPLC. After the reaction was found to be completed, the resulting reaction solution was concentrated under reduced pressure at 45 °C using a water pump to give a crude product. The reaction solution was purified by reverse-phase preparative column chromatography, and the preparation solution was lyophilized to give compound **L-1** (white solid, 9.2 mg, 55%) and compound **L-1'** (white solid, 5.2 mg, 31%). **L-1:** LC-MS m/z(ES$^+$): [M+Na]$^+$: 1337.3. **L-1':** LC-MS m/z(ES$^+$): [M+Na]$^+$: 1337.3.

Example 29 Synthesis of compound **S15**

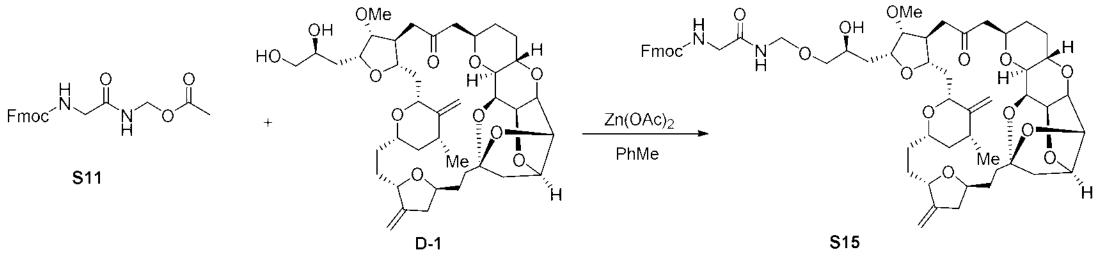


S11 + D-1 → S15

Compound **D-1** (50 mg, 0.068 mmol, 1.0 eq), compound **S11** (27.7 mg, 0.075 mmol, 1.1 eq), zinc acetate (25.1 mg, 0.137 mmol, 2.0 eq) and 4Å molecular sieve (100 mg) were added in sequence in a 25 mL single-neck flask, and then dry toluene (5 mL) was added. The reaction mixture was replaced with nitrogen three times, then heated with stirring at 100 °C. After 3 hours, the reaction was quenched, allowed to cool to room temperature, filtered, and the filtrate was concentrated under reduced pressure. The purification was performed by reverse-phase preparative column chromatography, and the preparation solution was lyophilized to give compound **S15** (white solid, 42 mg, 59%). LC-MS m/z(ES$^+$): [M+Na]$^+$: 1061.3.

Example 30 Synthesis of compound **S16**

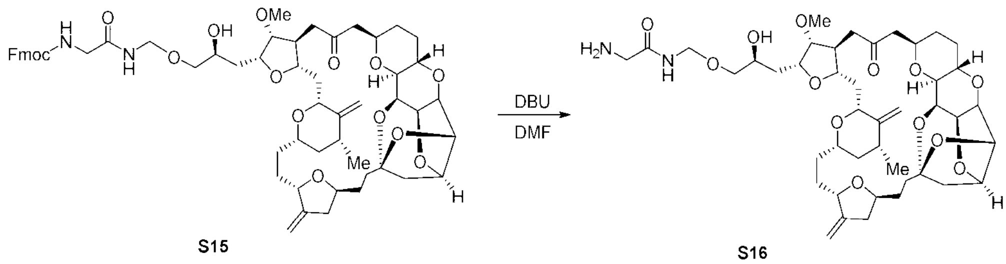


S15 → S16

In a 25 mL single-neck flask, compound **S15** (37 mg, 0.036 mmol, 1.0 eq) and dried DMF(1.5 mL) were added and dissolved completely, then DBU (5.9 μL, 0.039 mmol, 1.1 eq) was added, and the reaction was allowed at room temperature. The consumption of compound **S15** was found to be completed from the monitoring of the TLC, and no post-treatment was needed for reaction. The next reaction was carried out directly with a crude solution of compound **S16.** LC-MS m/z(ES$^+$): [M+Na]$^+$: 839.1.

Example 31 Synthesis of compound **S17**

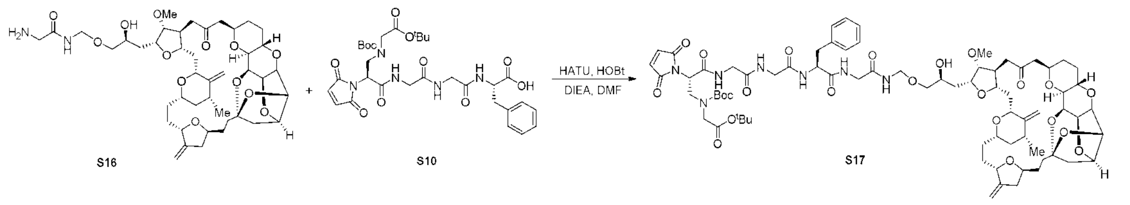

S16 S10 S17

In another 25 mL single-neck flask, compound **S10** (25.8 mg, 0.039 mmol, 1.1 eq), HATU (16.2 mg, 0.043 mmol, 1.2 eq), HOBt (5.8 mg, 0.043 mmol, 1.2 eq) and dried DMF (1.5 mL) were added sequentially and dissolved uniformly. The above crude solution of compound **S16** and DIEA (7.4 μL, 0.043 mmol, 1.2 eq) was added dropwise in condition of an ice water bath, and the reaction was allowed at room temperature. After 30 min, HPLC showed the completion of the reaction. The reaction solution was purified by reverse-phase preparative column chromatography, and the preparation solution was lyophilized to give compound **S17** (white solid, 26 mg, 50%). LC-MS m/z(ES$^+$): [M+H]$^+$: 1459.2.

Example 32 Synthesis of compound **L-2**

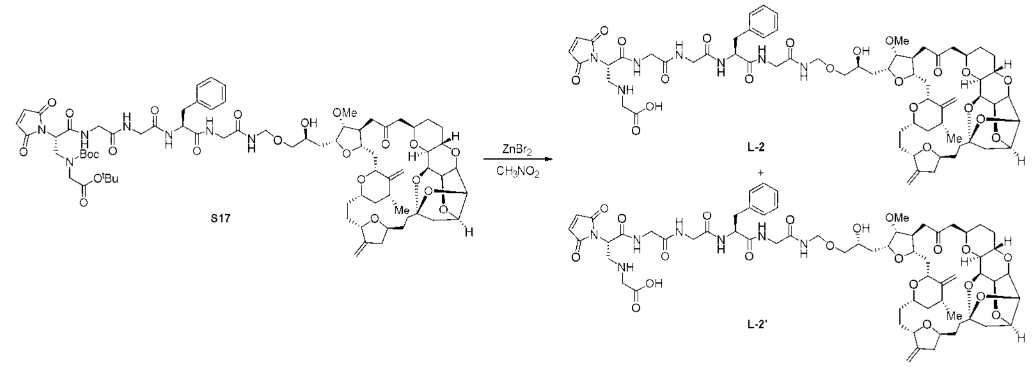

S17 L-2 + L-2'

In a 25 mL single-neck flask, compound **S17** (21 mg, 0.014 mmol, 1.0 eq) was completely dissolved in nitromethane (2 mL), then zinc bromide (65 mg, 0.288 mmol, 20.0 eq) was added, and the reaction was allowed at room temperature for 30 min and monitored by HPLC. After the reaction was found to be completed, the resulting reaction solution was concentrated under reduced pressure at 45 °C using a water pump to give a crude product. The reaction solution was purified by reverse-phase preparative column chromatography, and the preparation solution was lyophilized to give compound **L-2** (white solid, 10.7 mg, 57%) and compound L-2' (white solid, 5.4 mg, 29%). **L-2:** LC-MS m/z(ES$^+$): [M+Na]$^+$: 1324.3. **L-2':** LC-MS m/z(ES$^+$): [M+Na]$^+$: 1324.3.

Example 33 Synthesis of compound **L-3**

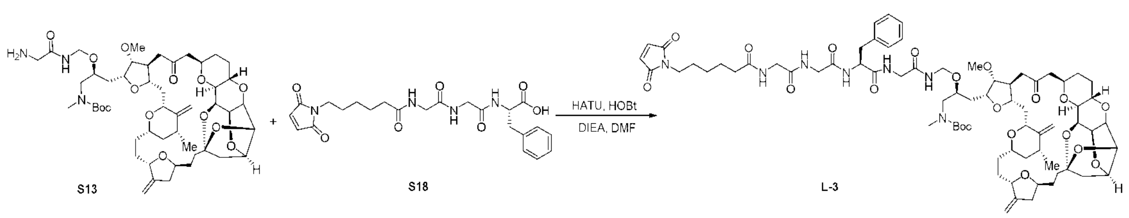

S13 S18 L-3

Compound **L-3** was synthesized from compound **S13** and compound **S18** (see patent WO2022171101 for synthesis method) according to the synthesis method of Example 27. LC-MSm/z(ES+): [M-99]+: 1285.1 (fragment ion of compound **L-3** after removing the Boc protecting group and adding hydrogen ion).

Example 34 Synthesis of compound **D-22**

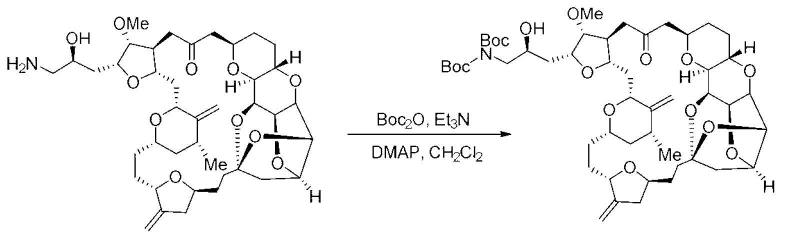


D-4 D-22

In a 25 mL single-neck flask, compound **D-4** (53 mg, 0.073 mmol, 1.0 eq) was added and dissolved in dried dichloromethane (3 mL), followed by addition of triethylamine (30 μL, 0.218 mmol, 3.0 eq), DMAP (1.8 mg, 0.015 mmol, 0.2 eq) and di-tert-butyl dicarbonate (42 μL, 0.182 mmol, 2.5 eq), and the reaction was allowed at room temperature for 2 h. After the reaction was found to be completed from the monitoring of the TLC, the reaction solution was concentrated under reduced pressure. The purification was performed by reverse-phase preparative column chromatography, and the preparation solution was lyophilized to give compound **D-22** (white solid, 36 mg, 53%). LC-MS m/z(ES$^+$): [M+H]$^+$: 930.2.

Example 35 Synthesis of compound **S19**

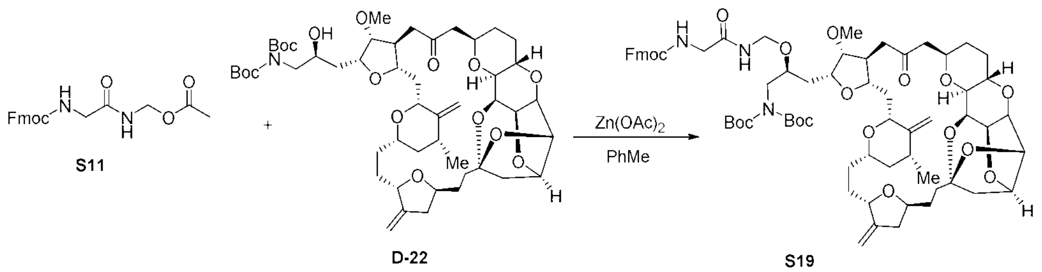


S11 D-22 S19

Compound **S19** was synthesized from compound **D-22** and compound **S11** according to the synthesis method of Example 25. LC-MS m/z(ES$^+$): [M+Na]$^+$:1260.2.

Example 36 Synthesis of compound **S20**

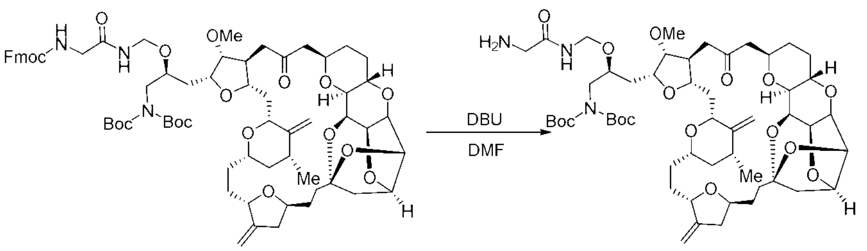


S19 S20

A crude solution of compound **S20** was synthesized from compound **S19** according to the synthesis method of Example 26, and put into the next step of reaction directly without post-treatment. LC-MS m/z(ES$^+$): [M+Na]$^+$:1038.3.

Example 37 Synthesis of compound **S21**

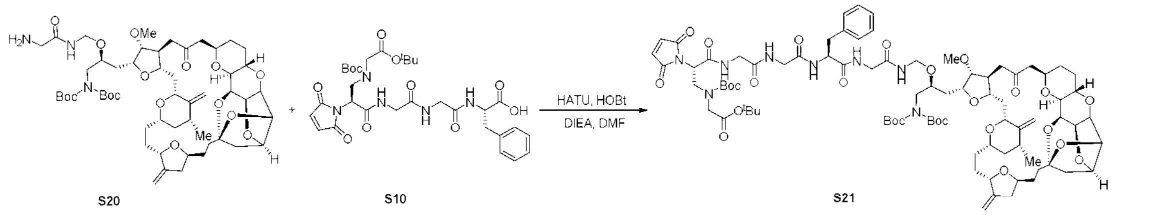


S20 S10 S21

Compound S21 was synthesized from compound **S20** and compound **S10** according to the synthesis method of Example 27. TOF m/z: $[M+H]^+$:1657.5.

Example 38 Synthesis of compound **L-4**

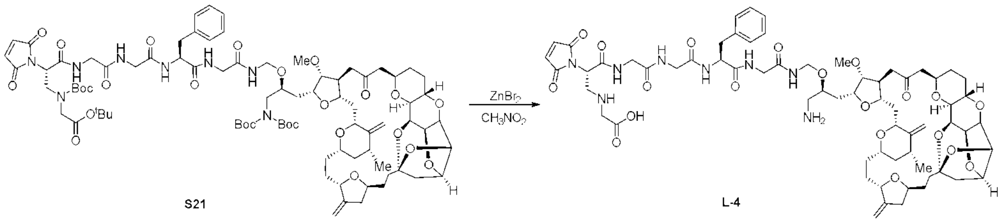


S21 L-4

Compound **L-4** was synthesized from compound **S21** according to the synthesis method of Example 28. LC-MS m/z($ES^+$): $[M+Na]^+$:1323.3.

Example 39 Synthesis of compound S22

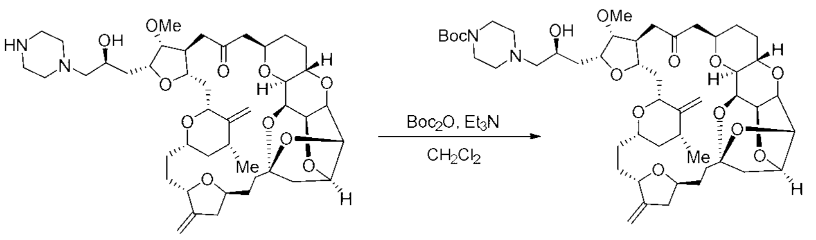


D-5 S22

Compound **S22** was synthesized from compound **D-5** according to the synthesis method of Example 2. LC-MS m/z($ES^+$): $[M+H]^+$:898.3.

Example 40 Synthesis of compound **S23**

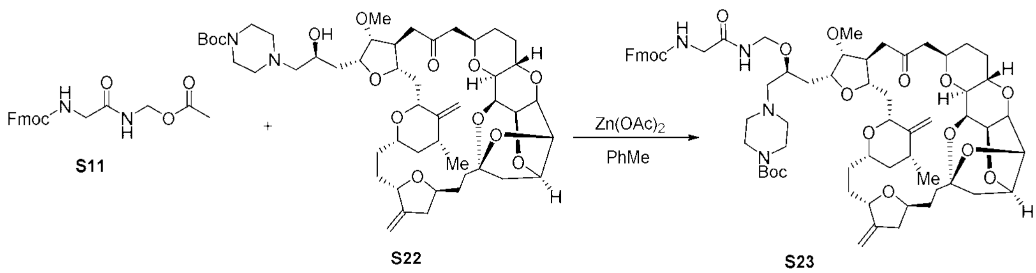


S11 S22 S23

Compound **S23** was synthesized from compound **S22** and compound **S11** according to the synthesis method of Example 25. LC-MS m/z($ES^+$): $[M+Na]^+$:1229.3.

Example 41 Synthesis of compound **S24**

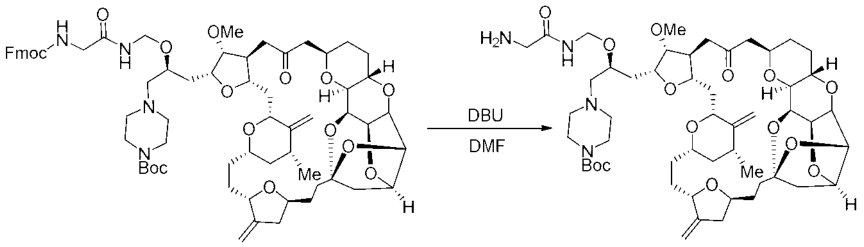


S23 S24

A crude solution of compound **S24** was synthesized from compound **S23** according to the synthesis method of Example 26, and put into the next step of reaction directly without post-treatment. LC-MS m/z(ES$^+$): [M+Na]$^+$:1007.3.

Example 42 Synthesis of compound **S25**

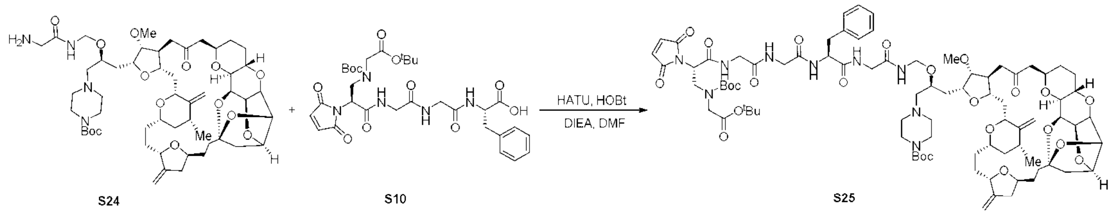


S24 S10 S25

Compound **S25** was synthesized from compound **S24** and compound **S10** according to the synthesis method of Example 27. TOF m/z: [M+H]$^+$:1626.5.

Example 43 Synthesis of compound **L-5**

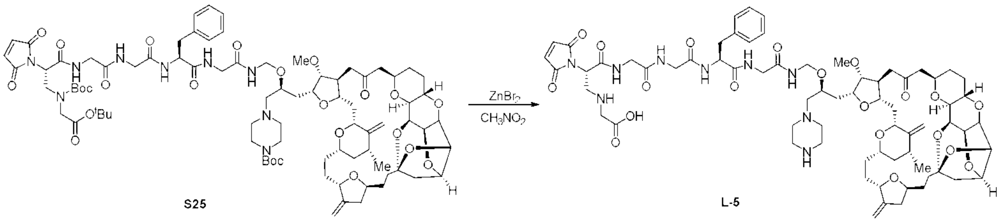


S25 L-5

Compound **L-5** was synthesized from compound **S25** according to the synthesis method of Example 28. LC-MS m/z(ES$^+$): [M+Na]$^+$:1392.4.

Example 44 Synthesis of compound **S26**

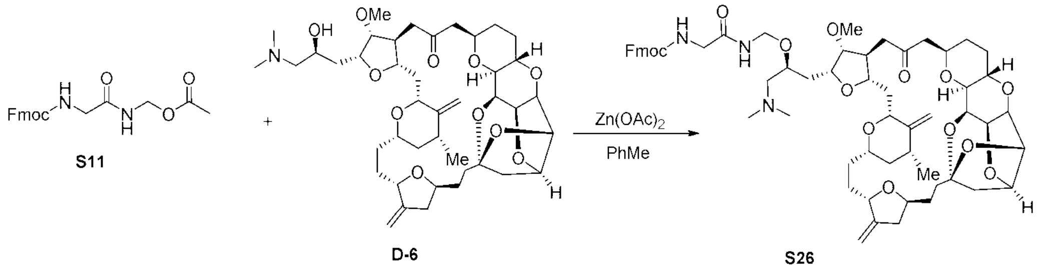


S11 D-6 S26

Compound **S26** was synthesized from compound **D-6** and compound **S11** according to the synthesis method of Example 25. LC-MS m/z(ES$^+$): [M+Na]$^+$:1088.3.

Example 45 Synthesis of compound **S27**

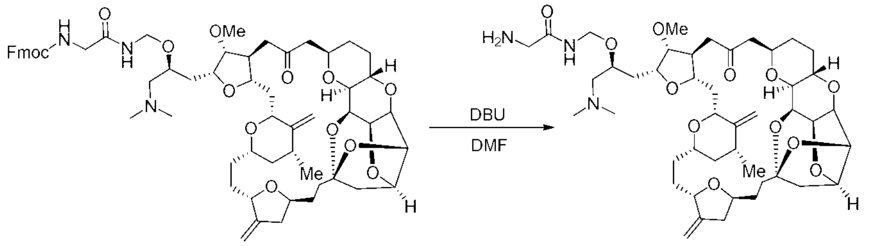


S26 S27

A crude solution of compound **S27** was synthesized from compound **S26** according to the synthesis method of Example 26, and put into the next step of reaction directly without post-treatment. LC-MS m/z(ES$^+$): [M+Na]$^+$:866.2.

Example 46 Synthesis of compound **S28**

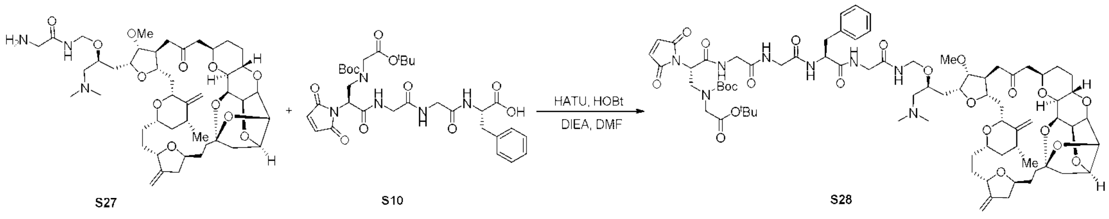


S27 S10 S28

Compound **S28** was synthesized from compound **S27** and compound **S10** according to the synthesis method of Example 27. LC-MS m/z(ES$^+$): [M+Na]$^+$:1485.5.

Example 47 Synthesis of compound **L-6**

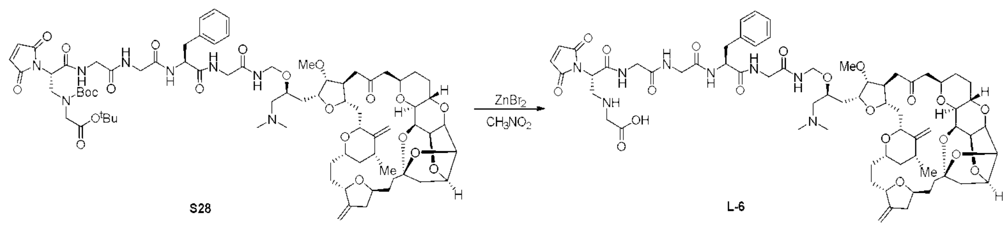


S28 L-6

Compound **L-6** was synthesized from compound **S28** according to the synthesis method of Example 28. LC-MS

m/z(ES+): [M+Na]+:1351.3.

Example 48 Synthesis of compound **L-7**

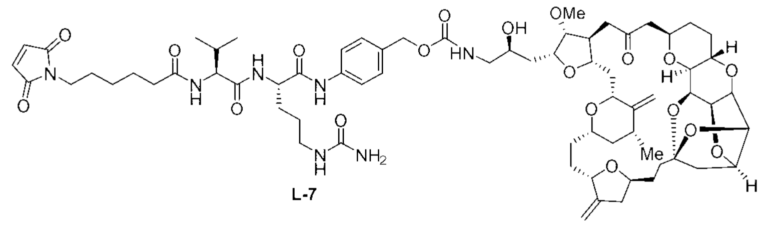

L-7

Compound **L-7** was synthesized according to the synthesis method of compound ER-001235638 in patent CNl08883198A.

Example 49 Synthesis of compound **L-8**

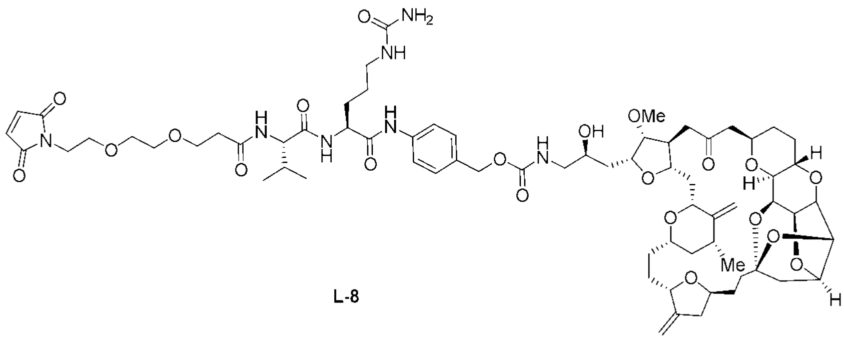

L-8

Compound **L-8** was synthesized according to the synthesis method of ER-001159569 in patent CN108883198A.

Example 50 Synthesis of compound **S29**

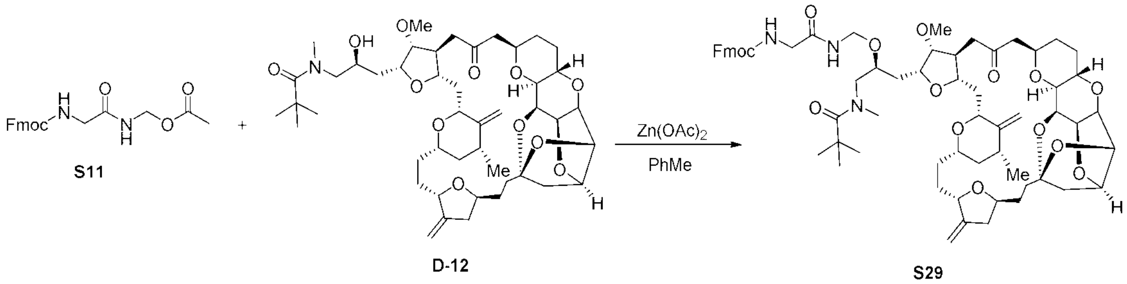


S11 D-12 S29

Compound **S29** was synthesized from compound **D-12** and compound **S11** according to the synthesis method of Example 25. LC-MS m/z(ES+): [M+Na]+:1158.4.

Example 51 Synthesis of compound **S30**

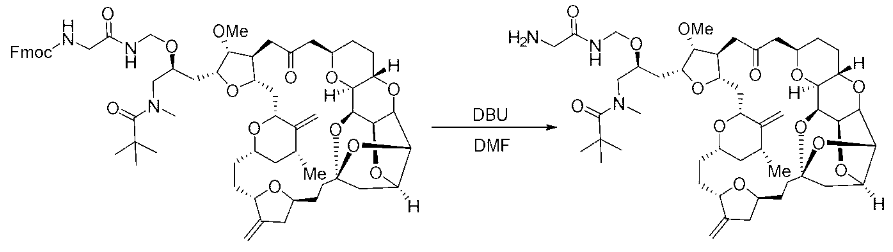


S29 S30

A crude solution of compound **S30** was synthesized from compound **S29** according to the synthesis method of Example 26, and put into the next step of reaction directly without post-treatment. LC-MS m/z(ES$^+$): [M+Na]$^+$:936.5.

Example 52 Synthesis of compound **S31**

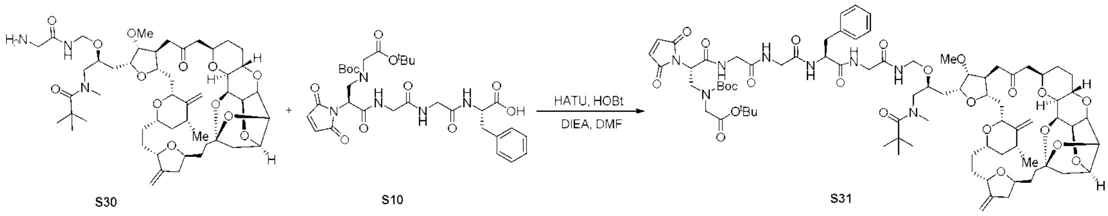


S30 S10 S31

Compound **S31** was synthesized from compound **S30** and compound **S10** according to the synthesis method of Example 27. TOF m/z: [M+H]$^+$:1555.7.

Example 53 Synthesis of compound **L-9**

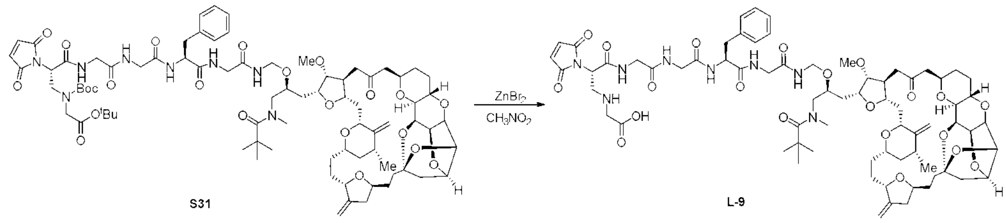


S31 L-9

Compound **L-9** was synthesized from compound **S31** according to the synthesis method of Example 28. LC-MS m/z(ES$^+$): [M+Na]$^+$:1421.7.

Example 54 Synthesis of compound **S32**

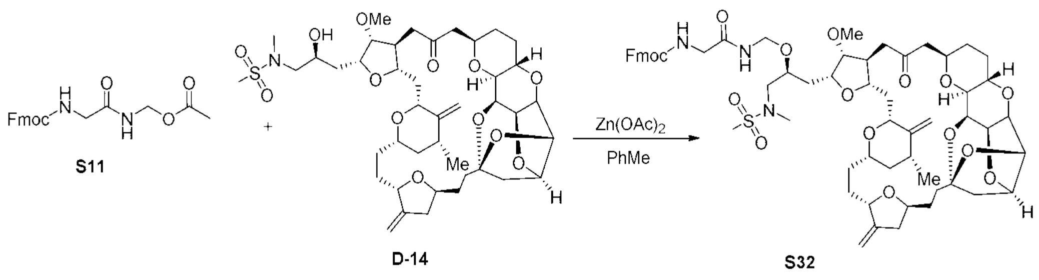


S11 D-14 S32

Compound **S32** was synthesized from compound **D-14** and compound **S11** according to the synthesis method of

Example 25. LC-MS m/z(ES$^+$): [M+Na]$^+$:1152.5.

Example 55 Synthesis of compound **S33**

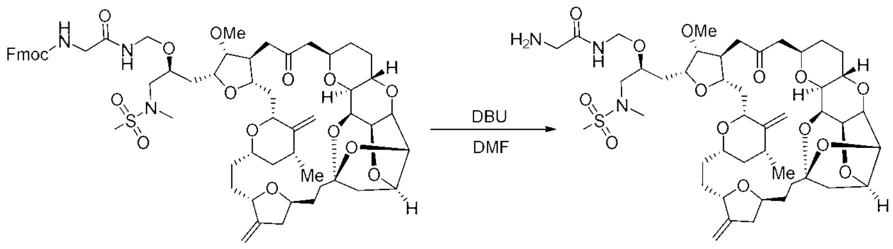


S32 S33

A crude solution of compound **S33** was synthesized from compound **S32** according to the synthesis method of Example 26, and put into the next step of reaction directly without post-treatment. LC-MS m/z(ES$^+$): [M+Na]$^+$:930.4.

Example 56 Synthesis of compound **S33**

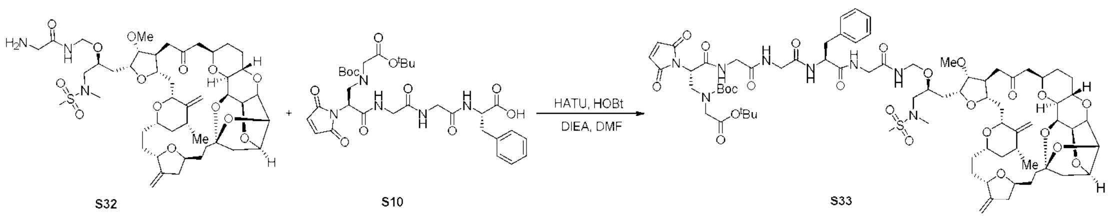


S32 S10 S33

Compound **S33** was synthesized from compound **S32** and compound **S10** according to the synthesis method of Example 27. TOF m/z: [M+H]$^+$:1549.7.

Example 57 Synthesis of compound **L-10**

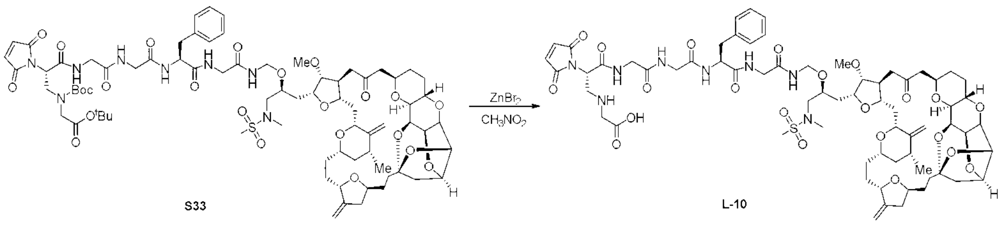


S33 L-10

Compound **L-10** was synthesized from compound **S33** according to the synthesis method of Example 28. LC-MS m/z(ES$^+$): [M+Na]$^+$:1415.6.

Example 58 Synthesis of compound **S34**

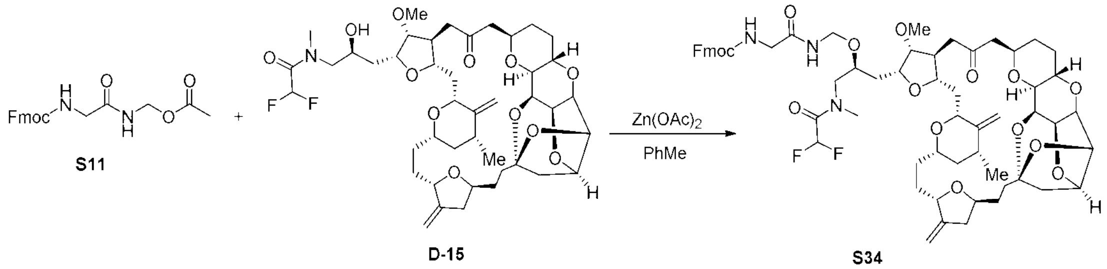


S11 D-15 S34

Compound **S34** was synthesized from compound **D-15** and compound **S11** according to the synthesis method of Example 25. LC-MS m/z(ES$^+$): [M+Na]$^+$:1152.4.

Example 59 Synthesis of compound **S35**

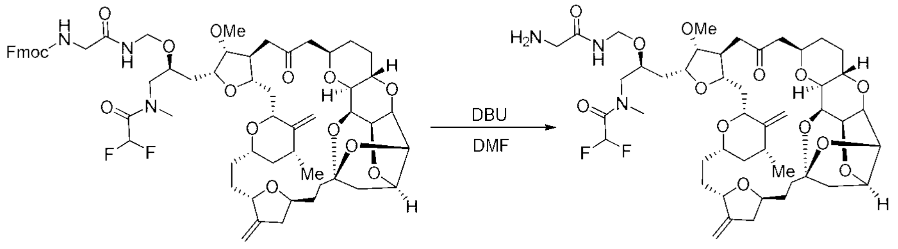


S34 S35

A crude solution of compound **S35** was synthesized from compound **S34** according to the synthesis method of Example 26, and put into the next step of reaction directly without post-treatment. LC-MS m/z(ES$^+$): [M+Na]$^+$:930.3.

Example 60 Synthesis of compound **S36**

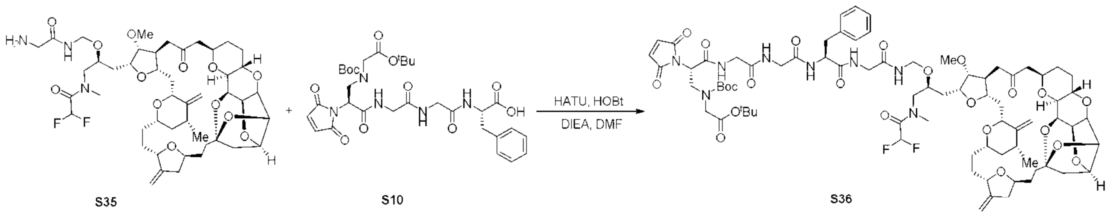


S35 S10 S36

Compound **S36** was synthesized from compound **S35** and compound **S10** according to the synthesis method of Example 27. TOF m/z: [M+H]$^+$:1549.6.

Example 61 Synthesis of compound **L-11**

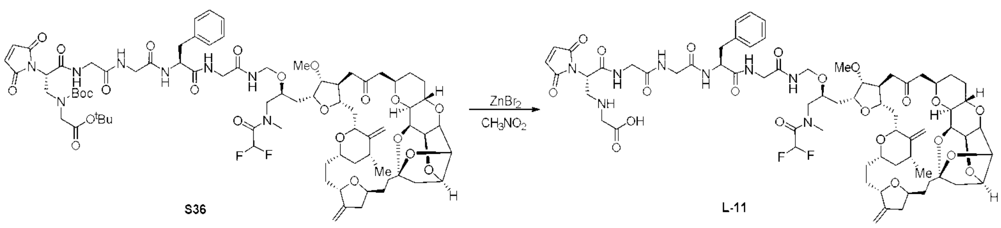


S36 L-11

Compound **L-11** was synthesized from compound **S36** according to the synthesis method of Example 28. LC-MS

m/z(ES⁺): [M+Na]⁺:1415.6.

Example 62 Synthesis of compound **S39**

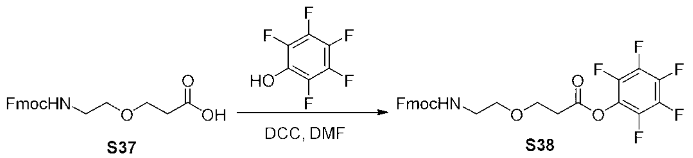


S37 S38

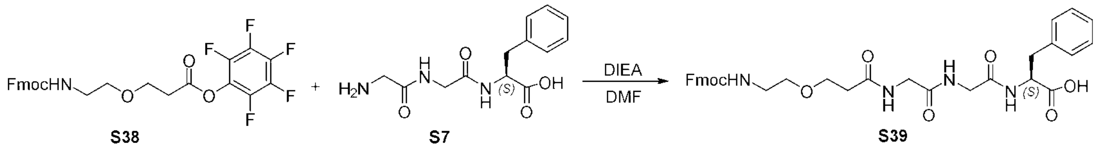


S38 S7 S39

Step 1: Synthesis of compound **S38**

In a 25 mL single-neck flask, compound **S37** (400 mg, 1.13 mmol, 1.0 eq), pentafluorophenol (228 mg, 1.24 mmol, 1.1 eq), DCC (255 mg, 1.24 mmol, 1.1 eq) and DMF (4 mL) were added, and the reaction was allowed at room temperature overnight. After the reaction was found to be completed from the monitoring of the TLC, the reaction solution was filtered to give the crude filtrate of compound **S38** for later use.

Step 2: Synthesis of compound **S39**

Compound **S7** (377mg, 1.35 mmol, 1.2 eq), DMF (4 mL) and DIEA (235 μL, 1.35 mmol, 1.2 eq) were added in a 25 mL single-neck flask, and the crude filtrate of the above compound **S38** was added in an ice-water bath, and the reaction was allowed at room temperature overnight. The reaction solution was purified by reverse-phase preparative column chromatography, and the preparation solution was lyophilized to give compound **S39** (white solid, 543 mg, 78%). LC-MS m/z(ES⁺): [M+H]⁺: 617.1.

Example 63 Synthesis of compound **S44**

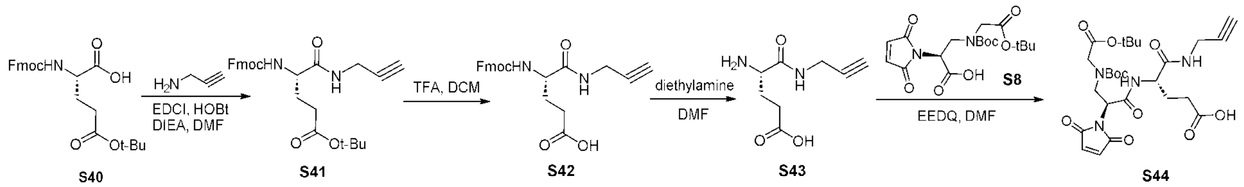


S40 S41 S42 S43 S8 S44

Step 1: Synthesis of compound **S41**

In a 2 L single-neck flask, Fmoc-L-glutamic acid-5-tert-butyl ester (**S40**, 200 g, 470 mmol, 1.0 eq), propargylamine (28.5 g, 517 mmol, 1.1 eq) and 400 mL DMF were added and dissolved completely, then the resulting solution was cooled to 0 °C in an ice water bath. EDCI (108.2 g, 564 mmol, 1.2 eq), HOBt (76.2 g, 564 mmol) and DIEA (117 mL, 705 mmol, 1.5 eq) were added, and the reaction was allowed at room temperature for 2 h and the reaction was monitored by TLC until the endpoint. After the reaction was completed, the reaction solution was poured into 2 L of water. Extraction was carried out three times with ethyl acetate (500 mL × 3). The organic phases were combined, washed twice with saturated sodium chloride solution, and dried over anhydrous sodium sulfate. The resulting reaction solution was then filtered to remove the sodium sulfate, and the filtrate was concentrated to give a crude product. The crude product was purified via petroleum ether/ethyl acetate (2/1) to give compound **S41** (100.9 g, 48% yield). LC-MS m/z(ES⁺): [M+H]⁺: 463.2.

Step 2: Synthesis of compound **S42**

In a 1 L single-neck flask, **S41** (100.0 g) was added and dissolved in 500 mL dichloromethane, followed by addition of 200 mL of trifluoroacetic acid. The reaction was allowed at room temperature for 2 h and the reaction was monitored by TLC until the endpoint. After the reaction was completed, the reaction solution was concentrated to remove dichloromethane and a portion of trifluoroacetic acid, and a crude product was obtained. Methyl tert-butyl ether was added to the crude product, and a white solid was precipitated, filtered, and dried to give compound **S42** (77.5 g, 87% yield), LC-MS m/z(ES$^-$): [M-H]$^-$: 405.1.

Step 3: Synthesis of compound **S43**

In a 2 L single-neck flask, **S42** (77.0 g) was added and dissolved in 150 mL of DMF, followed by addition of 38 mL of diethylamine. The reaction was allowed at room temperature for 2 h and the reaction was monitored by TLC until the endpoint. After the reaction was completed, 800 mL of methyl tert-butyl ether was added to the reaction solution. A white solid was precipitated, filtered, and dried to give compound **S43** (33.7 g, 96.6% yield), LC-MS m/z(ES$^-$): [M-H]$^-$: 183.1.

Step 4: Synthesis of compound **S44**

In a 100 mL single-neck flask, compound **S43** (10.02 g, 54.4 mmol, 1.5 eq), compound **S8** (14.48 g, 36.3 mmol, 1.0 eq) and 100 mL DMF were added and dissolved, followed by addition of EEDQ (13.5 g, 54.8 mmol, 1.5 eq). The reaction was allowed at room temperature for 2 h and the reaction was monitored by HPLC until the endpoint. After the reaction was completed, the reaction solution was purified by preparative liquid chromatography to give the product preparation solution, which was then lyophilized to give compound **S44** (9.94 g, 48% yield), LC-MS m/z(ES$^-$): [M-H]$^-$: 563.2.

Example 64 Synthesis of compound **S47**

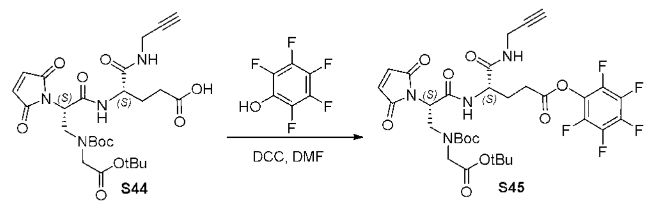

S44 S45

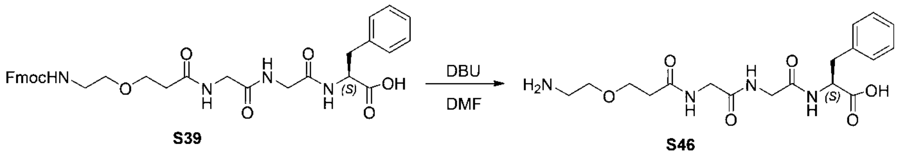

S39 S46

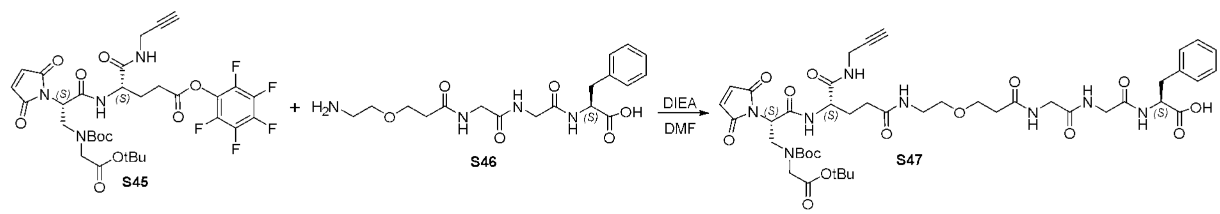

S45 S46 S47

Step 1: Synthesis of compound **S45**

In a 25 mL single-neck flask, compound **S44** (230 mg, 0.407 mmol, 1.0 eq), pentafluorophenol (83 mg, 0.448 mmol, 1.1 eq), DCC (93 mg, 0.448 mmol, 1.1 eq) and DMF (3.5 mL) were added, and the reaction was allowed at room temperature overnight. After the reaction was found to be completed from the monitoring of the TLC, the reaction solution was filtered to give the crude filtrate of compound **S45** for later use.

Step 2: Synthesis of compound **S46**

In a 25 mL single-neck flask, compound **S39** (301 mg, 0.489 mmol, 1.2 eq) and dried DMF (3 mL) were added and dissolved completely, then DBU (79 μL, 0.529 mmol, 1.3 eq) was added, and the reaction was allowed at room temperature. The consumption of compound **S39** was found to be completed from the monitoring of the TLC, and no post-treatment was needed for reaction. The next reaction was carried out directly with a crude solution of compound **S46.**

Step 3: Synthesis of compound **S47**

Under an ice-water bath, the crude filtrate of compound **S45** was mixed with the crude solution of **S46,** and DIEA (71 μL, 0.407 mmol, 1.0 eq) was slowly added dropwise, and the reaction was allowed at room temperature for 40 min. The reaction solution was purified by reverse-phase preparative column chromatography, and the preparation solution was lyophilized to give compound **S47** (white solid, 258 mg, 67%). LC-MS m/z(ES$^+$): [M+H]$^+$: 941.3.

Example 65 Synthesis of compound **S50**

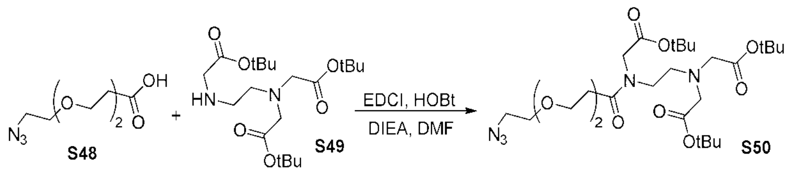


In a 150 mL single-neck flask, compound **S48** (667 mg, 3.28 mmol, 1.0 eq), **S49** (1.58 g, 3.94 mmol, 1.2 eq, synthesis method refers to the synthesis of compound **11** in patent WO 0152900 A2), EDCI (944 mg, 4.92 mmol, 1.5 eq), HOBt (665 mg, 4.92 mmol, 1.5 eq) and dried dichloromethane (60 mL) were added sequentially and dissolved uniformly. DIEA (1.14 mL , 6.56 mmol, 2.0 eq) was added dropwise in condition of an ice water bath, and the reaction was allowed at room temperature. After 2 h, the reaction was found to be completed from the monitoring of the TLC, the reaction solution was quenched with saturated aqueous ammonium chloride, extracted with dichloromethane, dried, filtered, concentrated, and purified by column chromatography (petroleum ether/ethyl acetate = 30:1) to give compound **S50** (a yellow oil 1.3 g, 67%). LC-MS m/z(ES$^+$): [M+H]$^+$: 588.4.

Example 66 Synthesis of compound **S51**

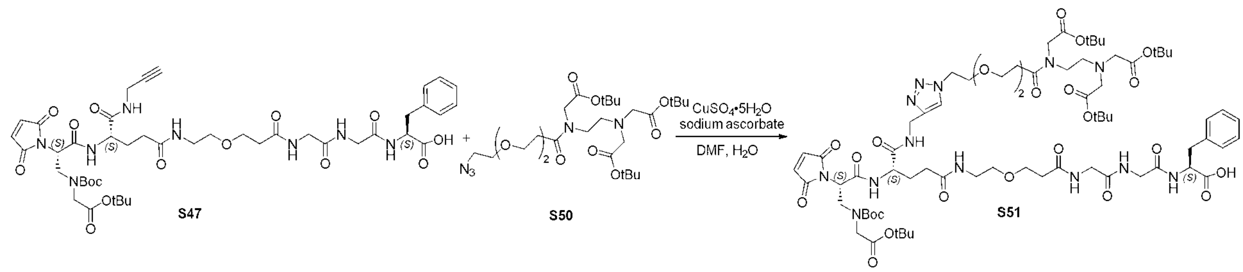


In a 25 mL single-neck flask, compound **S47** (58 mg, 0.061 mmol, 1.0 eq), compound **S50** (36 mg, 0.061 mmol, 1.0 eq), copper (II) sulfate pentahydrate (31 mg, 0.123 mmol, 2.0 eq), sodium ascorbate (24 mg, 0.123 mmol, 2.0 eq) and DMF (2 mL) were added, then water (1 mL) was added in an ice-water bath, and the reaction was allowed at room temperature. After 30 min, HPLC showed the completion of the reaction. The reaction solution was purified by reverse-phase preparative column chromatography, and the preparation solution was lyophilized to give compound **S51** (white solid, 85 mg, 91%). LC-MS m/z(ES$^+$): [M+2H]$^{2+}$: 764.7.

Example 67 Synthesis of compound **S52**

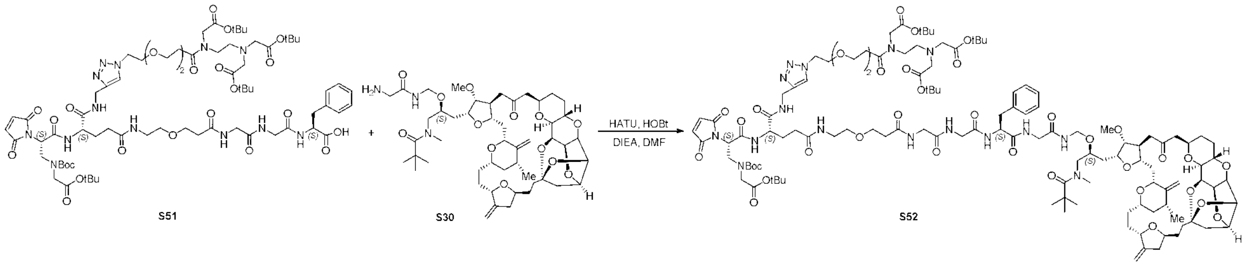


S51 + S30 → S52

Compound **S52** was synthesized from compound **S51** and compound **S30** according to the synthesis method of Example 27. LC-MS m/z(ES$^+$): [M+H+Na]$^{2+}$: 1223.8.

Example 68 Synthesis of compound **L-12**

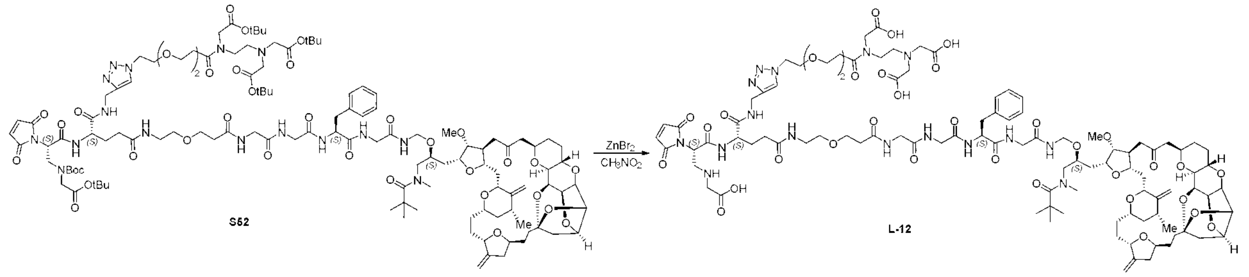


S52 → L-12

In a 25 mL single-neck flask, compound **S52** (19 mg, 0.0078 mmol, 1.0 eq) and nitromethane (2 mL) were added and dissolved completely, then zinc bromide (124 mg, 0.548 mmol, 70.0 eq) was added, and the reaction was allowed at room temperature for 45 min. The reaction solution was concentrated under reduced pressure to give a crude product. The crude product was purified by reverse-phase preparative column chromatography, and the preparation solution was lyophilized to give compound **L-12** (white solid, 9.2 mg, 55%)**L-12:** LC-MS m/z(ES$^+$): [M+2H]$^{2+}$: 1050.3.

Example 69 Synthesis of compound **S53**

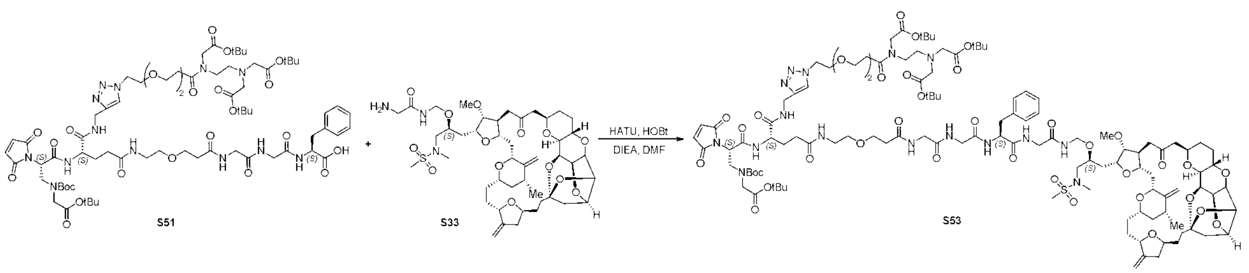


S51 + S33 → S53

Compound **S53** was synthesized from compound **S51** and compound **S33** according to the synthesis method of Example 27. LC-MS m/z(ES$^+$): [M+2H]$^{2+}$: 1209.9.

Example 70 Synthesis of compound **L-13**

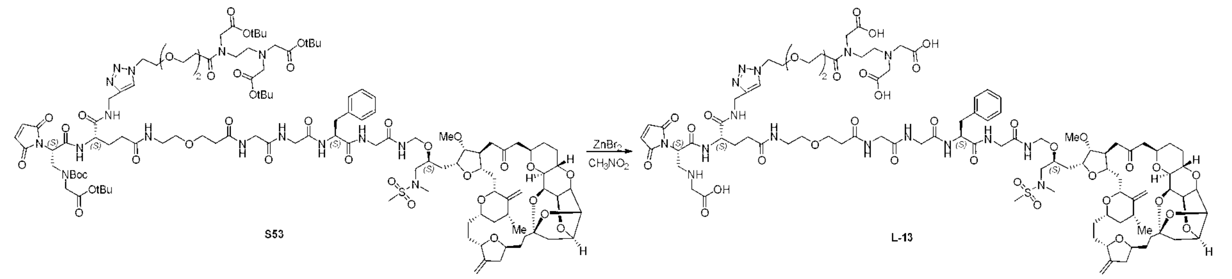


S53 L-13

Compound **L-13** was synthesized from compound **S53** according to the synthesis method of Example 68. LC-MS m/z(ES$^+$): [M+2H]$^{2+}$: 1047.4.

Example 71 Synthesis of compound **S54**

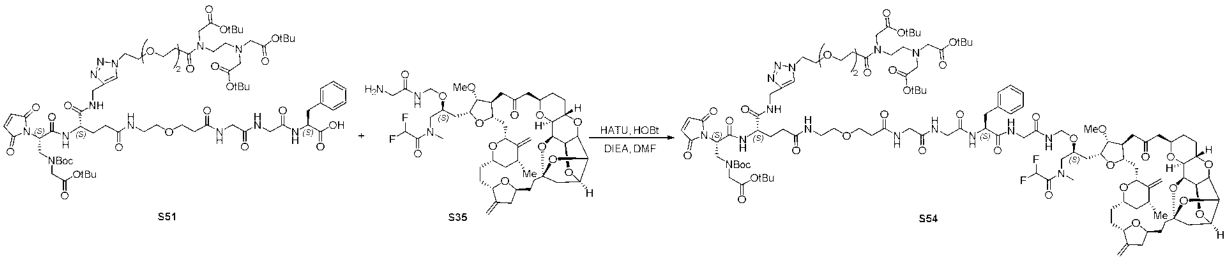


S51 + S35 S54

Compound **S54** was synthesized from compound **S51** and compound **S35** according to the synthesis method of Example 27. LC-MS m/z(ES$^+$): [M+2H+Na]$^{2+}$: 1220.8.

Example 72 Synthesis of compound **L-14**

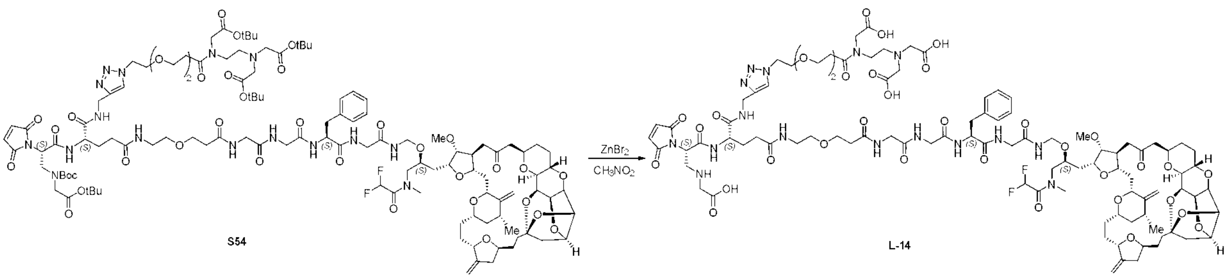


S54 L-14

Compound **L-14** was synthesized from compound **S54** according to the synthesis method of Example 68. LC-MS m/z(ES$^+$): [M+2H]$^{2+}$: 1047.4.

Example 73 Synthesis of compound **S57**

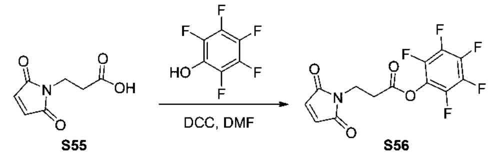


S55 S56

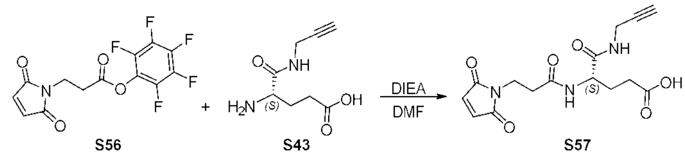


S56 S43 S57

Step 1: Synthesis of compound **S56**

In a 25 mL single-neck flask, compound **S55** (400 mg, 2.36 mmol, 1.0 eq), pentafluorophenol (479 mg, 2.60 mmol, 1.1 eq), DCC (537 mg, 2.60 mmol, 1.1 eq) and DMF (4 mL) were added, and the reaction was allowed at room temperature for 4 h. After the reaction was found to be completed from the monitoring of the TLC, the reaction solution was filtered to give the crude filtrate of compound **S56** for later use.

Step 2: Synthesis of compound **S57**

Compound **S43** (436 mg, 2.36 mmol, 1.0 eq) and DMF(7 mL) were added in the crude filtrate of the above compound **S56,** and DIEA (412 μL, 2.36 mmol, 1.0 eq) was added in an ice-water bath, and the reaction was allowed at room temperature for 2 h. After the reaction was found to be completed from the monitoring of the HPLC, the reaction solution was purified by reverse-phase preparative column chromatography, and the preparation solution was lyophilized to give compound **S57** (white solid, 649 mg, 82%). LC-MS m/z(ES$^+$): [M+Na]$^+$: 358.2.

Example 74 Synthesis of compound **S59**

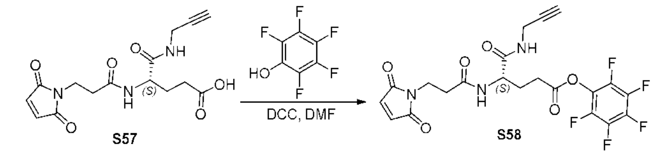


S57 S58

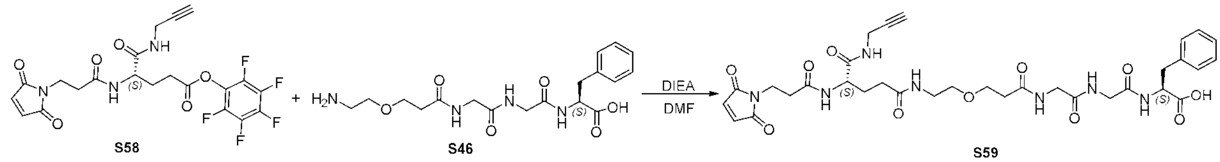


S58 S46 S59

Compound **S59** was synthesized from compound **S57** and compound **S46** according to the synthesis method of Example 64. LC-MS m/z(ES$^+$): [M+H]$^+$: 712.0.

Example 75 Synthesis of compound **S60**

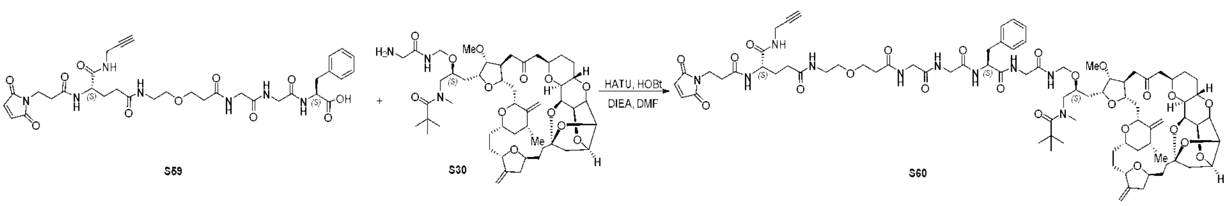


S59 S30 S60

Compound **S60** was synthesized from compound **S59** and compound **S30** according to the synthesis method of Example 27. LC-MS m/z(ES$^+$): [M+2H]$^{2+}$: 804.4.

Example 76 Synthesis of compound **S61**

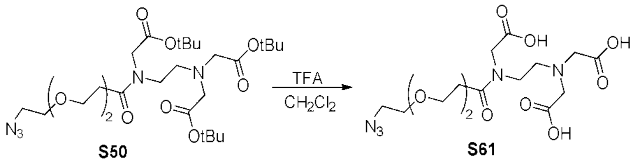


Compound **S50** (201 mg, 0.342 mmol, 1.0 eq) was dissolved in dried dichloromethane (4 mL), then trifluoroacetic acid (800 μL) was added, and the reaction was allowed at room temperature. After 30 h, the reaction solution was concentrated under reduced pressure. The crude product was purified by reverse-phase preparative column chromatography, and the preparation solution was lyophilized to give compound **S61** (white solid, 98 mg, 69%). LC-MS m/z(ES$^-$): [M-H]$^-$: 418.2.

Example 77 Synthesis of compound **L-15**

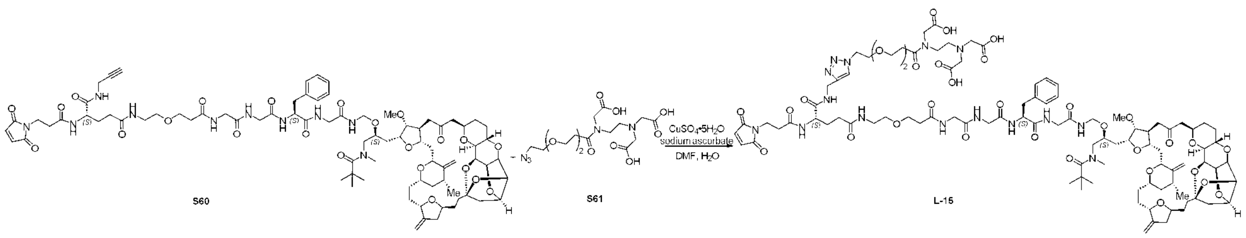


In a 25 mL single-neck flask, compound **S60** (25 mg, 0.016 mmol, 1.0 eq), compound S61 (7.8 mg, 0.019 mmol, 1.2 eq), copper (II) sulfate pentahydrate (19 mg, 0.078 mmol, 5.0 eq), sodium ascorbate (15 mg, 0.078 mmol, 5.0 eq) and DMF (2 mL) were added, then water (1 mL) was added in an ice-water bath, and the reaction was allowed at room temperature. After 30 min, HPLC showed the completion of the reaction. The reaction solution was purified by reverse-phase preparative column chromatography, and the preparation solution was lyophilized to give compound **L-15** (white solid, 21 mg, 66%). LC-MS m/z(ES$^+$): [M+H+Na]$^{2+}$: 1024.9.

Example 78 Synthesis of compound **S62**

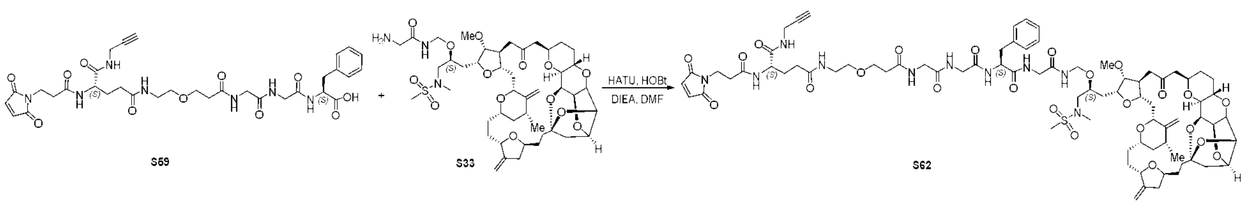


Compound **S62** was synthesized from compound **S59** and compound **S33** according to the synthesis method of Example 27. LC-MS m/z(ES$^+$): [M+2H]$^{2+}$: 801.3.

Example 79 Synthesis of compound **L-16**

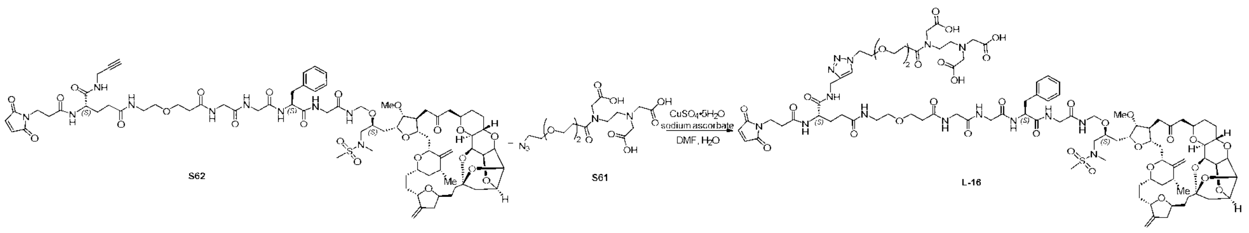


S62 S61 L-16

Compound **L-16** was synthesized from compound **S62** and compound **S61** according to the synthesis method of Example 77. LC-MS m/z(ES+): $[M+H+NH_4]^{2+}$: 1019.5.

Example 80 Synthesis of compound **S63**

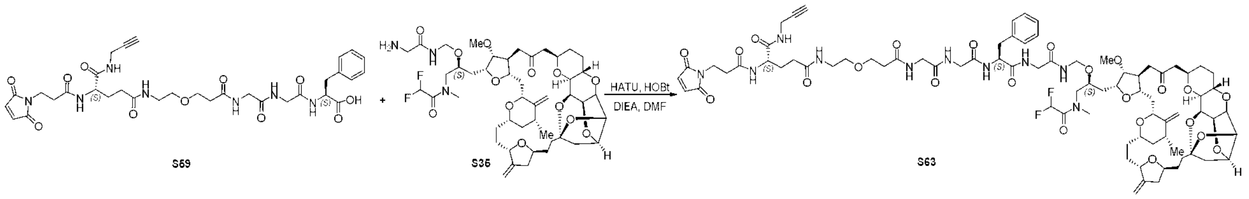


S59 S36 S63

Compound **S63** was synthesized from compound **S59** and compound **S35** according to the synthesis method of Example 27. LC-MS m/z(ES+): $[M+2H]^{2+}$: 801.0.

Example 81 Synthesis of compound **L-17**

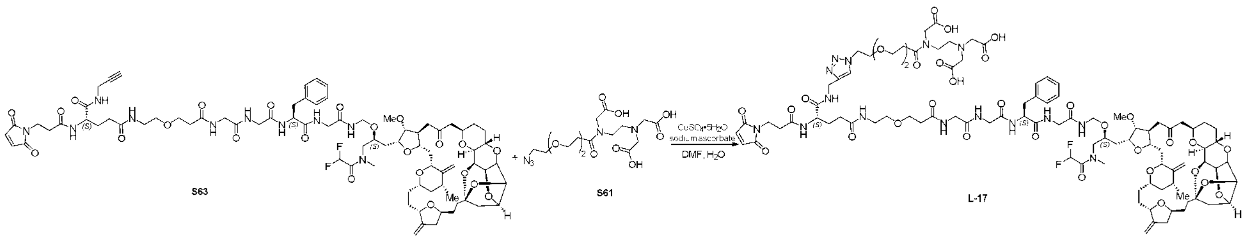


S63 S61 L-17

Compound **L-17** was synthesized from compound **S63** and compound **S61** according to the synthesis method of Example 77. LC-MS m/z(ES+): $[M+2H]^{2+}$:1010.9.

**Example** 82 Synthesis of compound **S68**

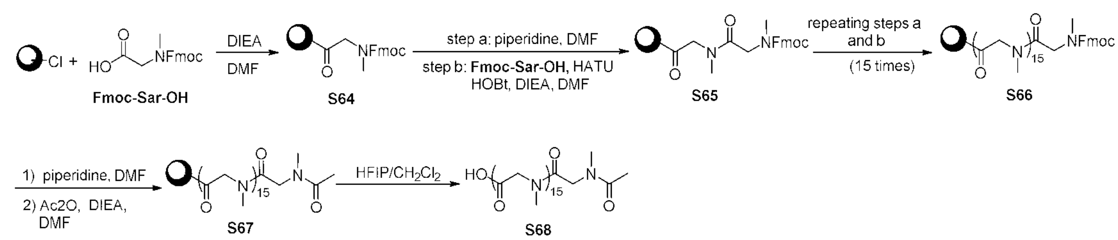


Fmoc-Sar-OH S64 S65 S66 S67 S68

Step 1: Resin loading: 4.6 g of 2-chlorotrityl chloride resin beads (1.05 mmol/g) were weighed and placed into a resin reactor and swollen in DMF for 1 h. After swelling, the solvent was filtered off, and a solution (30 mL) of Fmoc-sarcosine (1.5 g, 4.8 mmol, 1.0 eq) and DIEA (1.7 mL, 9.6 mmol, 2.0 eq) in DMF was added to the resin, and the resin reactor was shaken at room temperature for 4 h. After filtration, the resin was blocked with MeOH/DIEA/DMF (5 mL/2.5

mL/25 mL) for 30 min, and then the resin was thoroughly washed with DMF (8 times) to give compound **S64.**

Step 2: Extension of polysarcosine compounds

Step a: The resin was treated with piperidine/DMF (6 mL/24 mL) twice, 20 min each time. The resin was then washed with DMF (8 times).

Step b: A solution (30 mL) of Fmoc-sarcosine (4.5 g, 14.4 mmol, 3.0 eq), HATU (5.5 g, 14.4 mmol, 3.0 eq), HOBt (1.9 g, 14.4 mmol, 3.0 eq) and DIEA (4.2 mL, 24 mmol, 5.0 eq) in DMF was added to the resin, and the resin reactor was shaken at room temperature for 2 h. The resin was then washed with DMF (8 times).

Steps a and b were repeated until the length of the polysarcosine compound on the resin reached 16 peptides, thus obtaining compound **S66.**

Step 3: Acetylation

The resin was treated with piperidine/DMF (6 mL/24 mL) twice for20 min each time. The resin was then washed with DMF (8 times).

The resin was then treated with acetic anhydride/DIEA/DMF (5 mL/10 mL/15 mL) twice, 1 h each time. The resin was then washed with DMF (8 times) to give compound **S67.**

Step 4: Resin cleavage

Poly-sarcosine oligomers were cleaved from the resin with an HFIP/$CH_2Cl_2$ (6mL/24mL) solution for 30 min. The resin was filtered, the filtrate was concentrated under reduced pressure, and the crude product was purified by a reverse phase preparative column, and the preparation was lyophilized to give compound **S68** (white solid, 1.7 g, 30%). LC-MS m/z(ES$^+$): $[M+2NH_4]^{2+}$: 616.4.

Example 83 Synthesis of compound **S70**

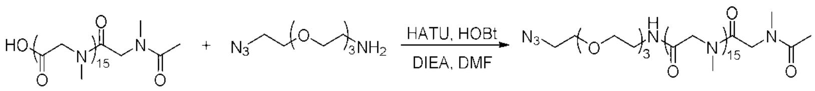


S68 S69 S70

In a 25 mL single-neck flask, compound **S68** (93 mg, 0.078 mmol, 1.0 eq), **S69** (17 mg, 0.078 mmol, 1.0 eq), HATU (45 mg, 0.117 mmol, 1.5 eq), HOBt (16 mg, 0.117 mmol, 1.5 eq) and dried DMF (2.5 mL) were added sequentially and dissolved uniformly. DIEA (27 μL, 0.156 mmol, 2.0 eq) was added dropwise in condition of an ice water bath, and the reaction was allowed at room temperature. After 1 h, HPLC showed the completion of the reaction. The reaction solution was purified by reverse-phase preparative column chromatography, and the preparation solution was lyophilized to give compound **S70** (white solid, 80 mg, 73%). LC-MS m/z(ES$^+$): $[M+H]^+$: 1397.1.

Example 84 Synthesis of compound **L-18**

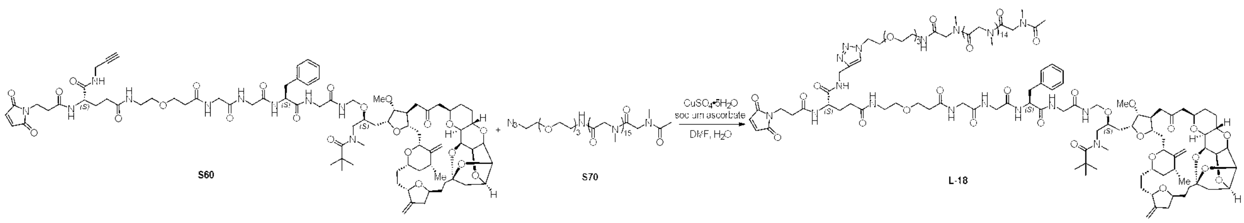


S60 S70 L-18

Compound **L-18** was synthesized from compound **S60** and compound **S70** according to the synthesis method of Example 77. TOF m/z: $[M+Na]^+$: 3026.5.

Example 85 Synthesis of compound **L-19**

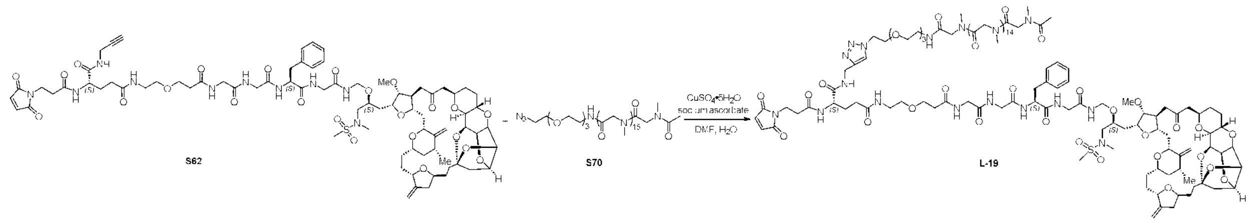


S62 S70 L-19

Compound **L-19** was synthesized from compound **S62** and compound **S70** according to the synthesis method of Example 77. TOF m/z: $[M+Na]^+$: 3020.4.

Example 86 Synthesis of compound **L-20**

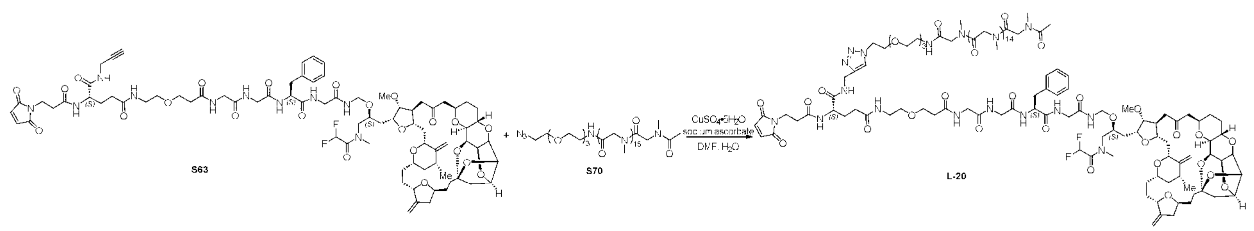


S63 S70 L-20

Compound **L-20** was synthesized from compound **S63** and compound **S70** according to the synthesis method of Example 77. TOF m/z: $[M+Na]^+$: 3020.4.

Example 87 Synthesis of compound **S76**

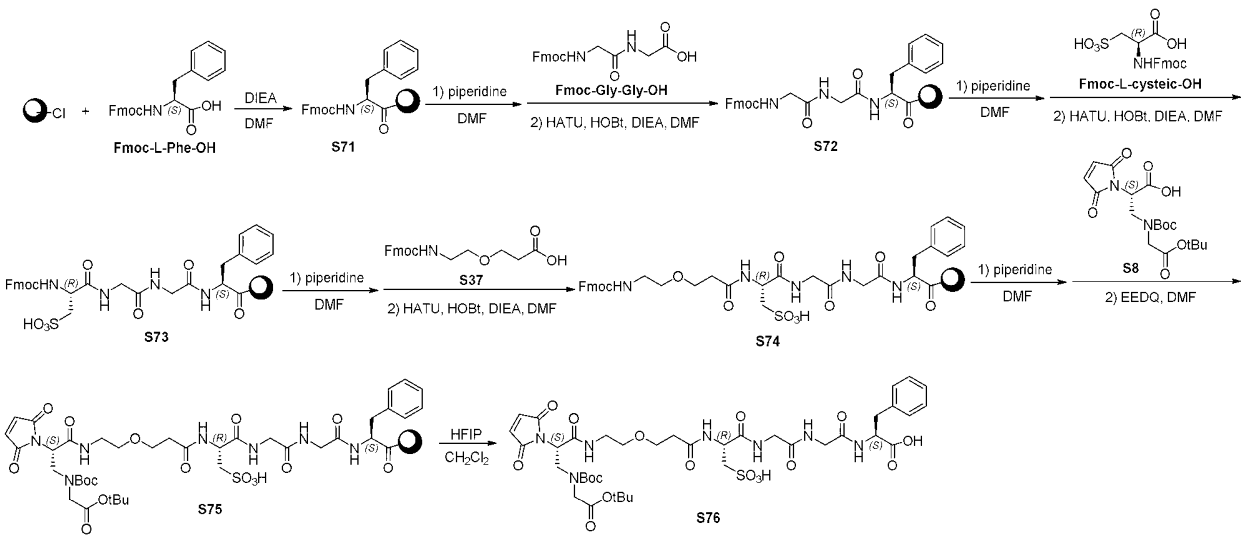


Fmoc-L-Phe-OH S71 Fmoc-Gly-Gly-OH S72 Fmoc-L-cysteic-OH S73 S37 S74 S8 S75 S76

Step 1: Resin loading

3.7 g of 2-chlorotrityl chloride resin beads (1.05 mmol/g) were weighed and placed into a resin reactor and swollen in DMF for 1 h. After swelling, the solvent was filtered off, and a solution (30 mL) of Fmoc-L-phenylalanine (1.5 g, 3.9 mmol, 1.0 eq) and DIEA (1.3 mL, 7.7 mmol, 2.0 eq) in DMF was added to the resin, and the resin reactor was shaken at

room temperature for 4 h. After filtration, the resin was blocked with MeOH/DIEA/DMF (5 mL/2.5 mL/25 mL) for 30 min, and then the resin was thoroughly washed with DMF (8 times) to give compound **S71.**

Step 2: Extension of peptide chains

The resin was treated with piperidine/DMF (6 mL/24 mL) twice, 20 min each time, and then thoroughly washed with DMF (8 times). A solution (30 mL) of Fmoc-glycyl-glycine (4.1 g, 11.6 mmol, 3.0 eq), HATU (4.4 g, 11.6 mmol, 3.0 eq), HOBt (1.6 g, 11.6 mmol, 3.0 eq) and DIEA (3.4 mL, 19.4 mmol, 5.0 eq) in DMF was added to the resin, and the resin reactor was shaken at room temperature for 2 h. The resin was then washed with DMF (8 times) to give compound **S72.**

The resin was treated with piperidine/DMF (6 mL/24 mL) twice, 20 min each time, and then thoroughly washed with DMF (8 times). A solution (30 mL) of Fmoc-cysteic acid (4.5 g, 11.6 mmol, 3.0 eq), HATU (4.4 g, 11.6 mmol, 3.0 eq), HOBt (1.6 g, 11.6 mmol, 3.0 eq) and DIEA (3.4 mL, 19.4 mmol, 5.0 eq) in DMF was added to the resin, and the resin reactor was shaken at room temperature for 4 h. The resin was then washed with DMF (8 times) to give compound **S73.**

The resin was treated with piperidine/DMF (6 mL/24 mL) twice, 20 min each time, and then thoroughly washed with DMF (8 times). A solution (30 mL) of compound **S37** (4.1 g, 11.6 mmol, 3.0 eq), HATU (4.4 g, 11.6 mmol, 3.0 eq), HOBt (1.6 g, 11.6 mmol, 3.0 eq) and DIEA (3.4 mL, 19.4 mmol, 5.0 eq) in DMF was added to the resin, and the resin reactor was shaken at room temperature for 4 h. The resin was then washed with DMF (8 times) to give compound **S74.**

The resin was treated with piperidine/DMF (6 mL/24 mL) twice, 20 min each time, and then thoroughly washed with DMF (8 times). A solution (30 mL) of compound **S8** (4.6 g, 11.6 mmol, 3.0 eq) and EEDQ (2.9 mL, 11.6 mmol, 3.0 eq) in DMF was added to the resin, and the resin reactor was shaken at room temperature for 4 h. The resin was then washed with DMF (8 times) to give compound **S75.**

Step 3: Resin cleavage

The resin with an HFIP/$CH_2Cl_2$ (6 mL/24 mL) solution for 30 min. The resin was filtered, the filtrate was concentrated under reduced pressure, and the crude product was purified by reverse-phase preparative column chromatography, and the preparation was lyophilized to give compound **S76** (white solid, 836 mg, 23%). LC-MS m/z(ES$^-$): [M-H]$^-$: 924.2.

Example 88 Synthesis of compound **S77**

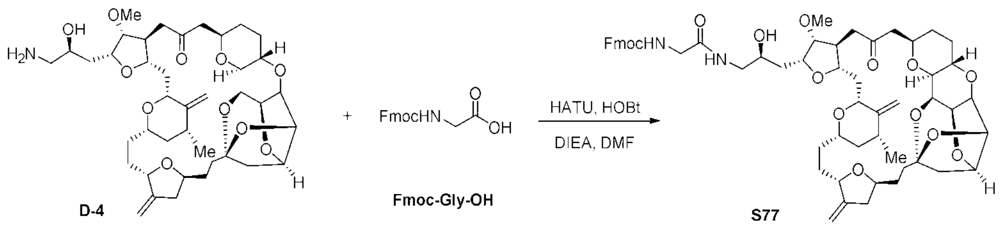


D-4 Fmoc-Gly-OH S77

In a 25 mL single-neck flask, compound **D-4** (50 mg, 0.069 mmol, 1.0 eq), Fmoc-glycine (31 mg, 0.103 mmol, 1.5 eq), HATU (39 mg, 0.103 mmol, 1.5 eq), HOBt (14 mg, 0.103 mmol, 1.5 eq) and dried DMF (3 mL) were added sequentially and dissolved uniformly, then DIEA (24 μL, 0.137 mmol, 2.0 eq) was added dropwise in condition of an ice water bath, and the reaction was allowed at room temperature. After 1 h, HPLC showed the reaction was complete. The reaction solution was purified by reverse-phase preparative column chromatography, and the preparation solution was lyophilized to give compound **S77** (white solid, 58 mg, 84%). LC-MS m/z(ES$^+$): [M+H]$^+$: 1009.2.

Example 89 Synthesis of compound **S79**

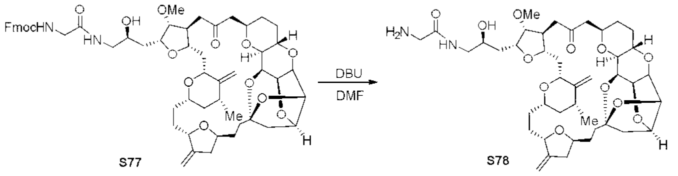

S77 S78

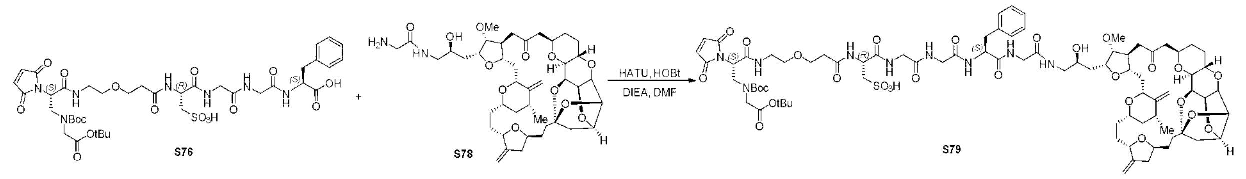

S76 S78 S79

Compound **S79** was synthesized from compound **S77** and compound **S76** according to the synthesis method of Example 26 and Example 27. LC-MS m/z(ES+): [M-100+2H]2+: 797.9 (fragment ion response of compound **S79** after removing the Boc protecting group and adding hydrogen ion).

Example 90 Synthesis of compound **L-21**

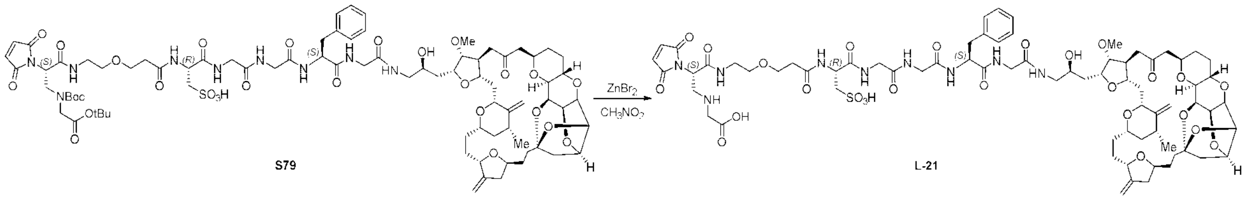

S79 L-21

Compound **L-21** was synthesized from compound **S79** according to the synthesis method of Example 28. LC-MS m/z(ES$^+$): [M+2H]$^{2+}$: 769.8.

Example 91 Synthesis of compound **S80**

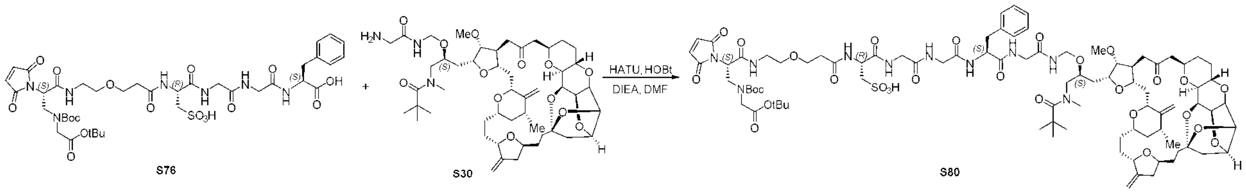

S76 S30 S80

Compound **S80** was synthesized from compound **S76** and compound **S30** according to the synthesis method of Example 27. LC-MS m/z(ES$^+$): [M+2H]$^{2+}$: 911.5.

Example 92 Synthesis of compound **L-22**

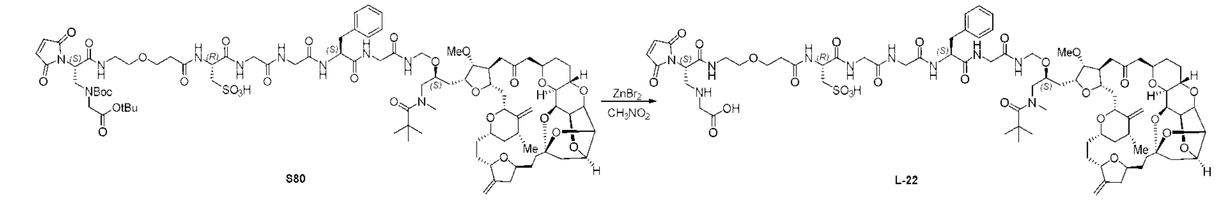

S80 L-22

Compound L-22 was synthesized from compound **S80** according to the synthesis method of Example 28. LC-MS

m/z(ES+): $[M+2H]^{2+}$: 833.4.

Example 93 Synthesis of compound **S81**

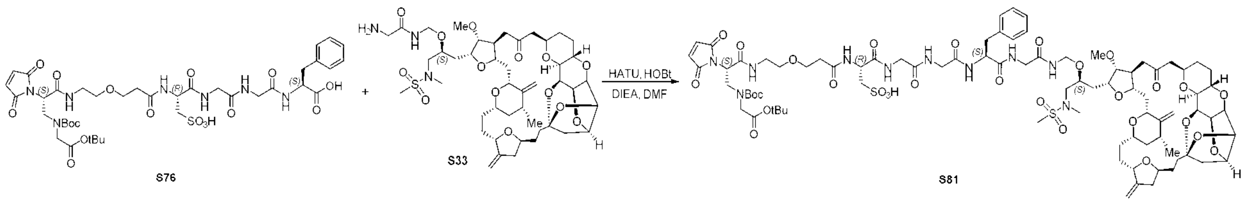


S76 S33 S81

Compound **S81** was synthesized from compound **S76** and compound **S33** according to the synthesis method of Example 27. LC-MS m/z(ES+): $[M+2H]^{2+}$: 908.4.

Example 94 Synthesis of compound **L-23**

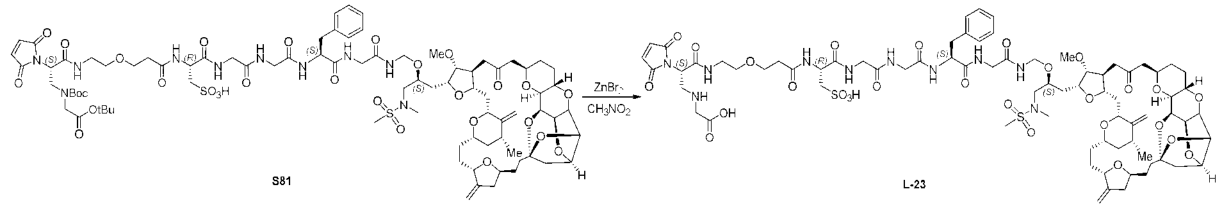


S81 L-23

Compound **L-23** was synthesized from compound **S81** according to the synthesis method of Example 28. LC-MS m/z(ES+): $[M+2H]^{2+}$: 830.3.

Example 95 Synthesis of compound **S82**

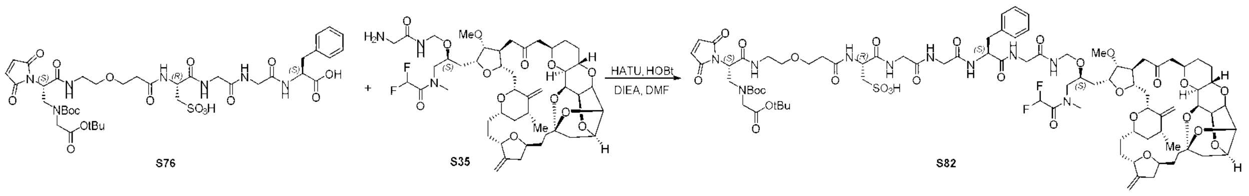


S76 S35 S82

Compound **S82** was synthesized from compound **S76** and compound **S35** according to the synthesis method of Example 27. LC-MS m/z(ES+): $[M+2H]^{2+}$: 908.3.

Example 96 Synthesis of compound **L-24**

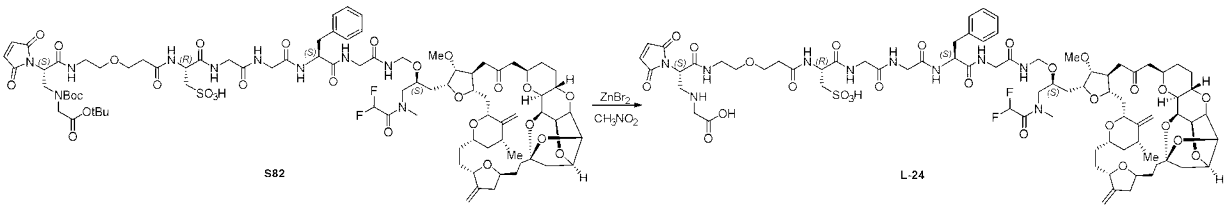


S82 L-24

Compound **L-24** was synthesized from compound **S82** according to the synthesis method of Example 28. LC-MS

m/z(ES$^+$): [M+2H]$^{2+}$: 830.3.

Example 97 Preparation of **ADC-1**

The **ADC-1** was prepared by using compound **L-1** and anti-Trop2 antibody TR001 according to the method in general procedure A. The sequence information of TR001 is shown in Table 2.

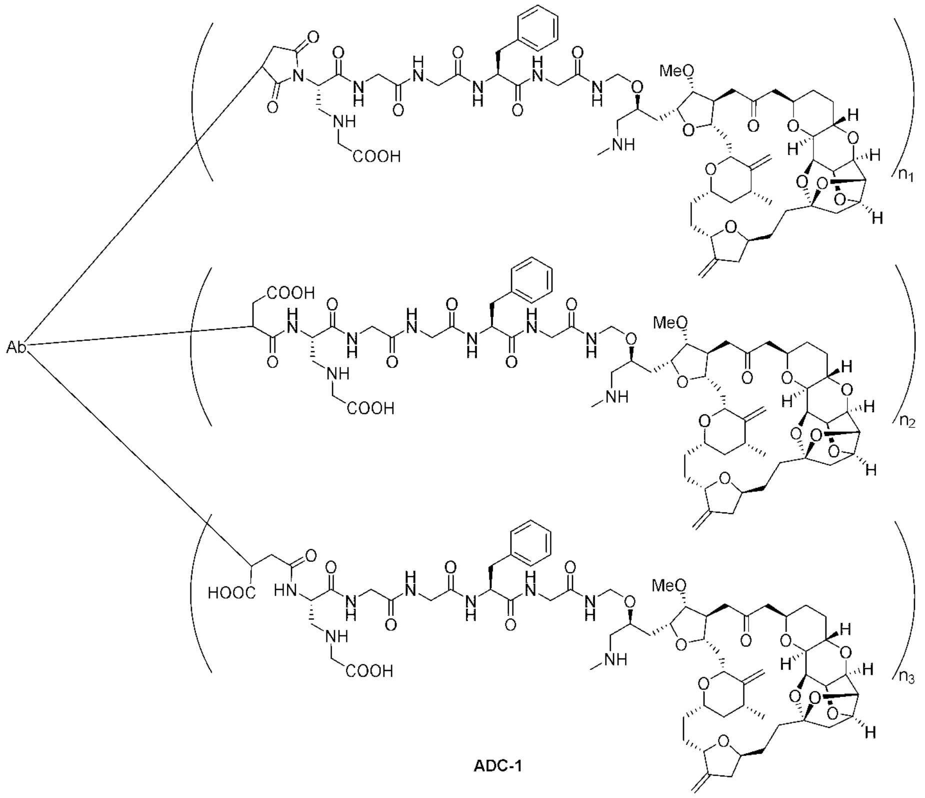

**ADC-1**

Table 2: Description of sequence of TR001

| | |
|---|---|
| Amino acid sequence of the light chain of TR001 | DIQMTQSPSSLSASVGDRVTITCRASQDINKYLAWYQQKPGKVPKLLIYSTSTLQSGVPSRFSGSGSGTDFTLTISSLQPEDVATYYCLQYDDLFTFGQGTKLEIKRTVAAPSVFIFPPSDEQLKSGTASVVCLLNNFYPREAKVQWKVDNALQSGNSQESVTEQDSKDSTYSLSSTLTLSKADYEKHKVYACEVTHQGLSSPVTKSFNRGEC |
| Amino acid sequence of the heavy chain of TR001 | QVQLVQSGAEVKKPGASVKLSCKASGYTFTSFDINWVRQAPEQRLEWMGWIFPGDGNTKYSQKFQGRATITRDTSASTAYMELSSLRSEDTAVYYCVRGEALYYFDYWGQGTLVTVSSASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKRVEPKSCDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSRDELTKNQVSLTCLVKGFYPSDIAVEWESNGQPENNYKTTPPVLDSDGSFFLYSKLTVDKSRWQQGNVFSCSVMHEALHNHYTQKSLSLSPG |

Example 98 Preparation of **ADC-2**

The **ADC-2** was prepared by using compound **L-2** and anti-Trop2 antibody TR001 according to the method in general procedure A.

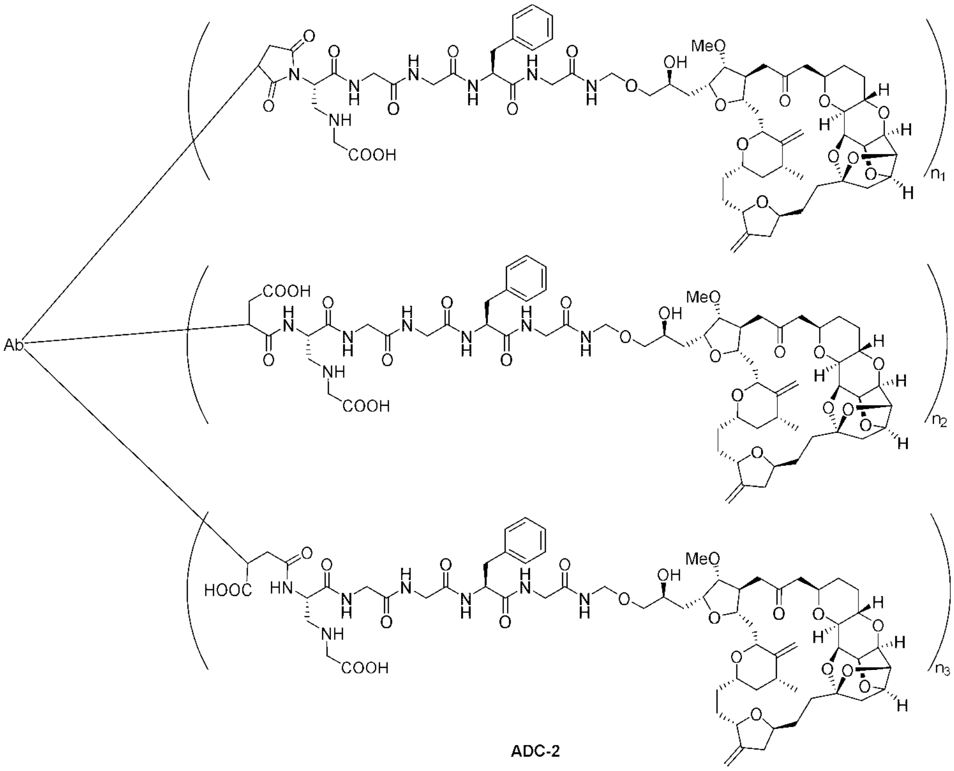

ADC-2

Example 99 Preparation of **ADC-3**

The **ADC-3** was prepared by using compound **L-1** and anti-Trop2 antibody TR002 according to the method in general procedure B. The sequence information of TR002 is shown in Table 3.

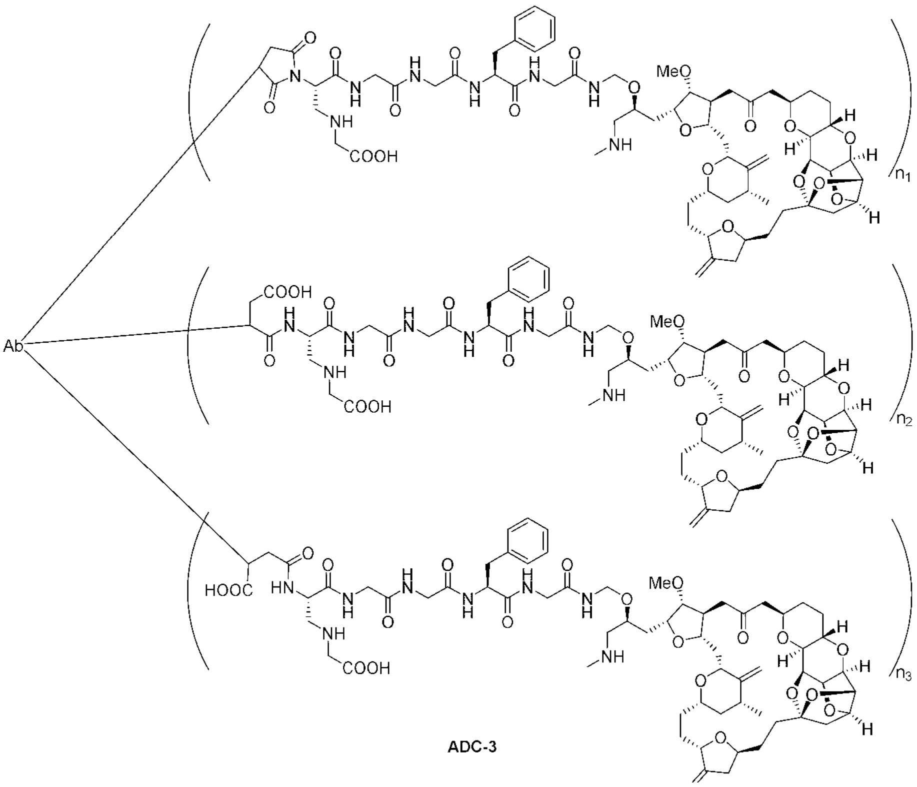

ADC-3

Table 3: Description of sequence of TR002

| | |
|---|---|
| Amino acid sequence of the light chain of TR002 | DIQMTQSPSSLSASVGDRVTITCRASQDINKYLAWYQQKPGKVPKLLIYSTSTLQSGVPSRFSGSGSGTDFTLTISSLQPEDVATYYCLQYDDLFTFGQGTKLEIKRTVAAPSVFIFPPSDEQLKSGTASVVCLLNNFYPREAKVQWKVDNALQSGNSQESVTEQDSKDSTYSLSSTLTLSKADYEKHKVYACEVTHQGLSSPVCTKSFNRGEC |
| Amino acid sequence of the heavy chain of TR002 | QVQLVQSGAEVKKPGASVKLSCKASGYTFTSFDINWVRQAPEQRLEWMGWIFPGDGNTKYSQKFQGRATITRDTSASTAYMELSSLRSEDTAVYYCVRGEALYYFDYWGQGTLVTVSSASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKRVEPKSCDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSRDELTKNQVSLTCLVKGFYPSDIAVEWESNGQPENNYKTTPPVLDSDGSFFLYSKLTVDKSRWQQGNVFSCSVMHEALHNHYTQKSLSLSPG |

Example 100 Preparation of **ADC-4**

The **ADC-4** was prepared by using compound **L-2** and anti-Trop2 antibody TR002 according to the method in general procedure B.

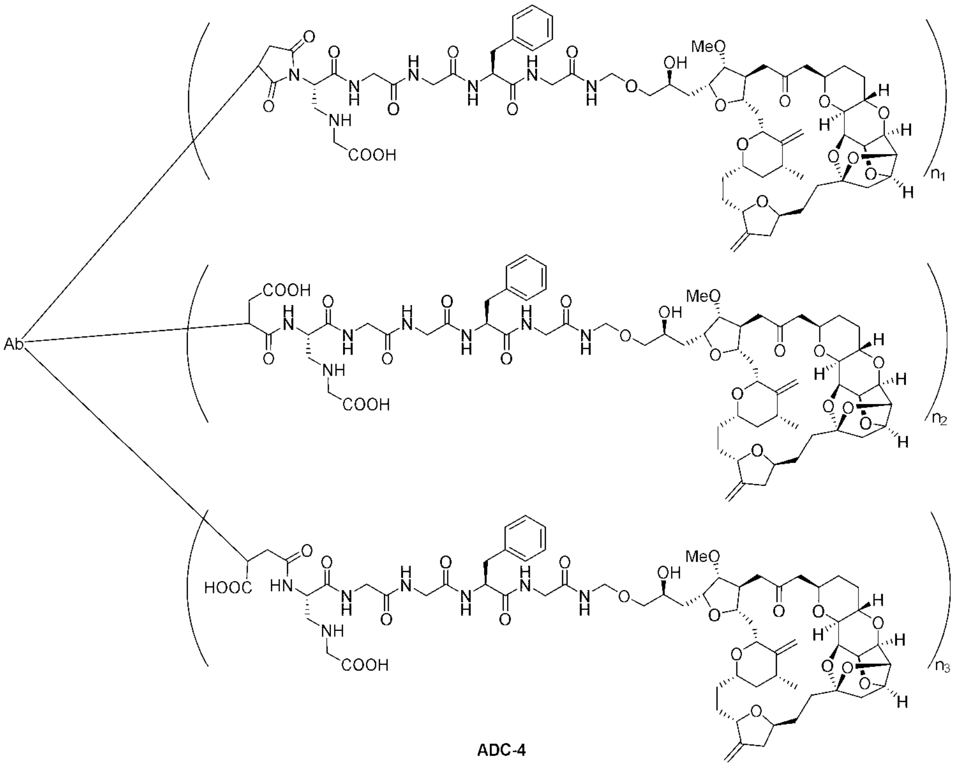

ADC-4

Example 101 Preparation of **ADC-5**

The **ADC-5** was prepared by using compound **L-3** and anti-Trop2 antibody TR001 according to the method in general procedure A.

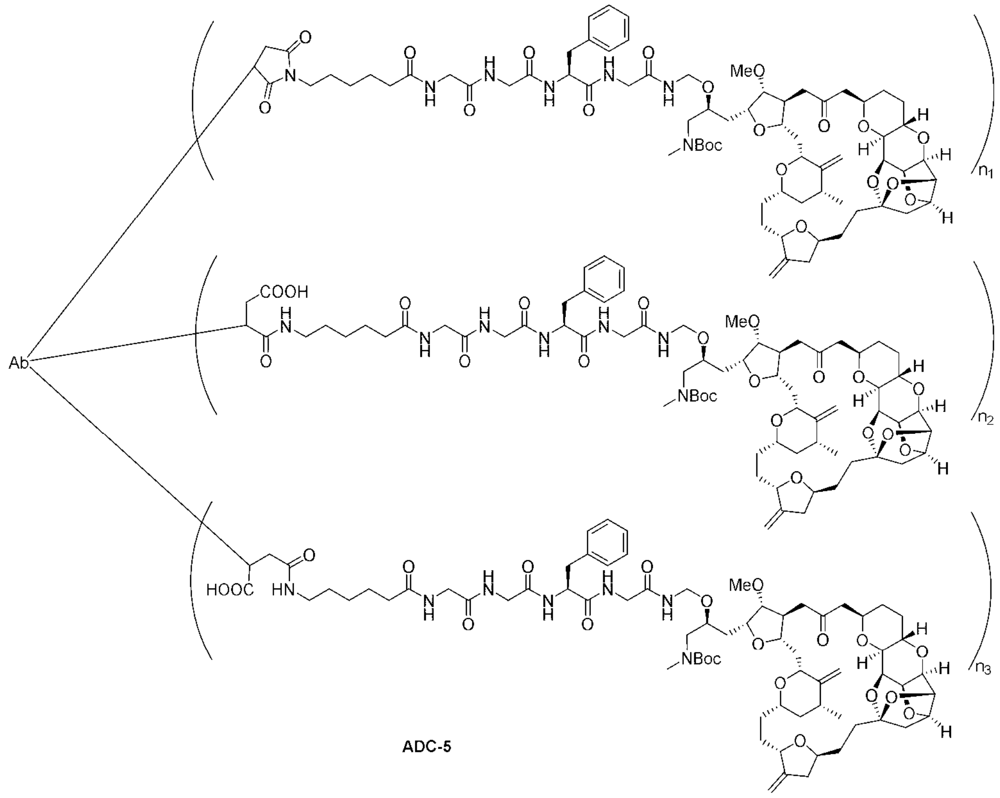

ADC-5

Example 102 Preparation of **ADC-6**

The **ADC-6** was prepared by using compound **L-7** and anti-Trop2 antibody TR001 according to the method in general procedure A.

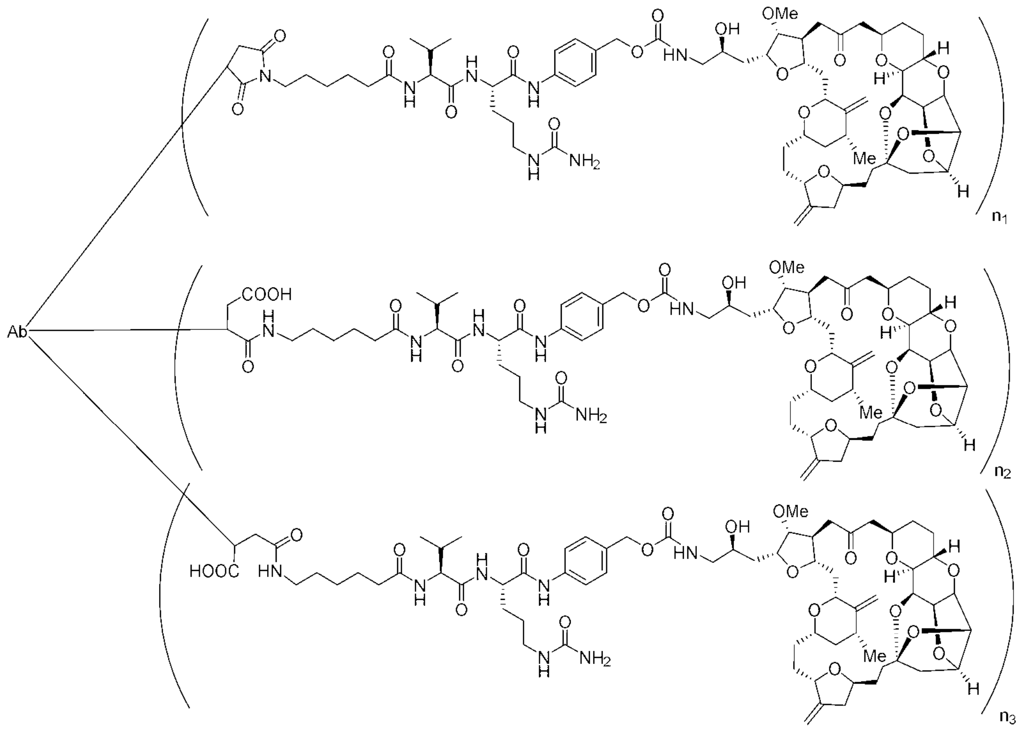

ADC-6

Example 103 Preparation of **ADC-7**

The **ADC-7** was prepared by using compound **L-8** and anti-Trop2 antibody TR001 according to the method in general procedure A.

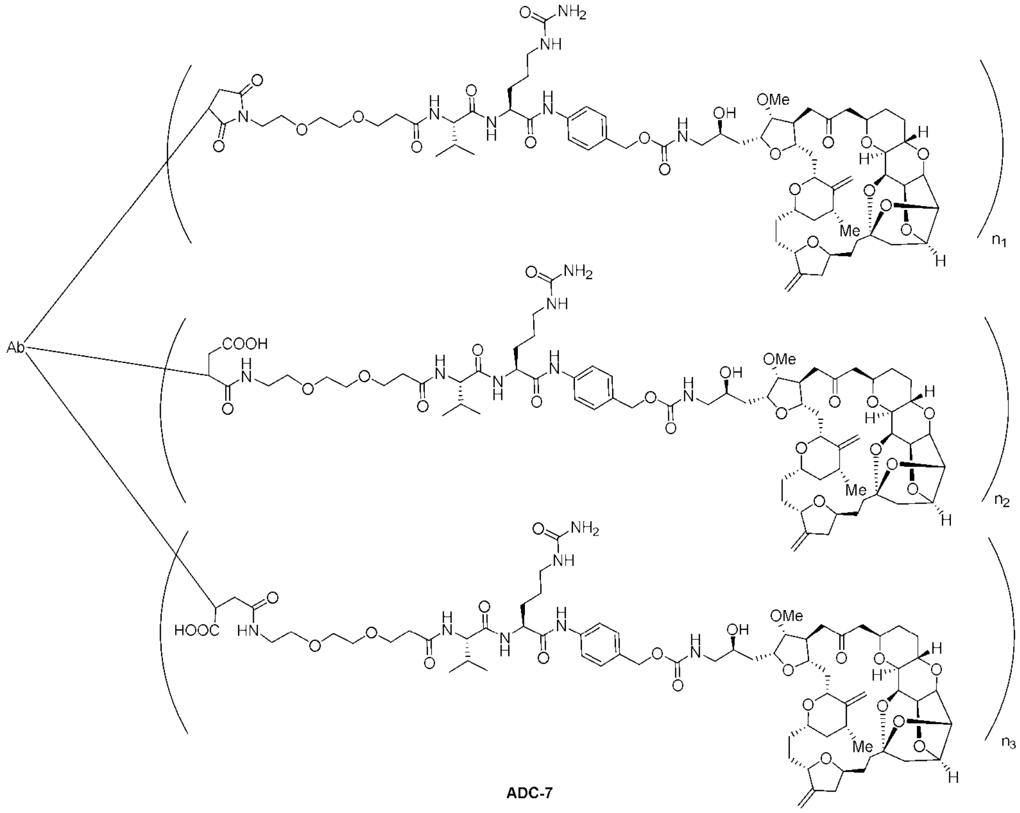

**ADC-7**

Example 104 Preparation of **ADC-8**

The **ADC-8** was prepared by using compound **L-9** and anti-Trop2 antibody TR001 according to the method in general procedure A.

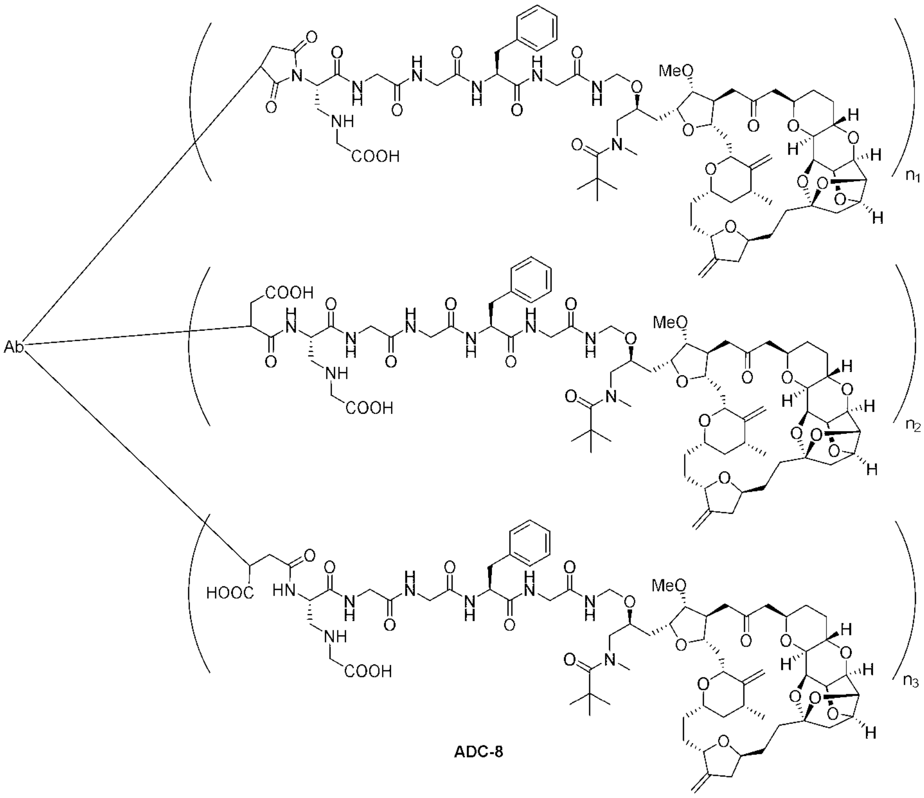

ADC-8

Example 105 Preparation of **ADC-9**

The **ADC-9** was prepared by using compound **L-10** and anti-Trop2 antibody TR001 according to the method in general procedure A.

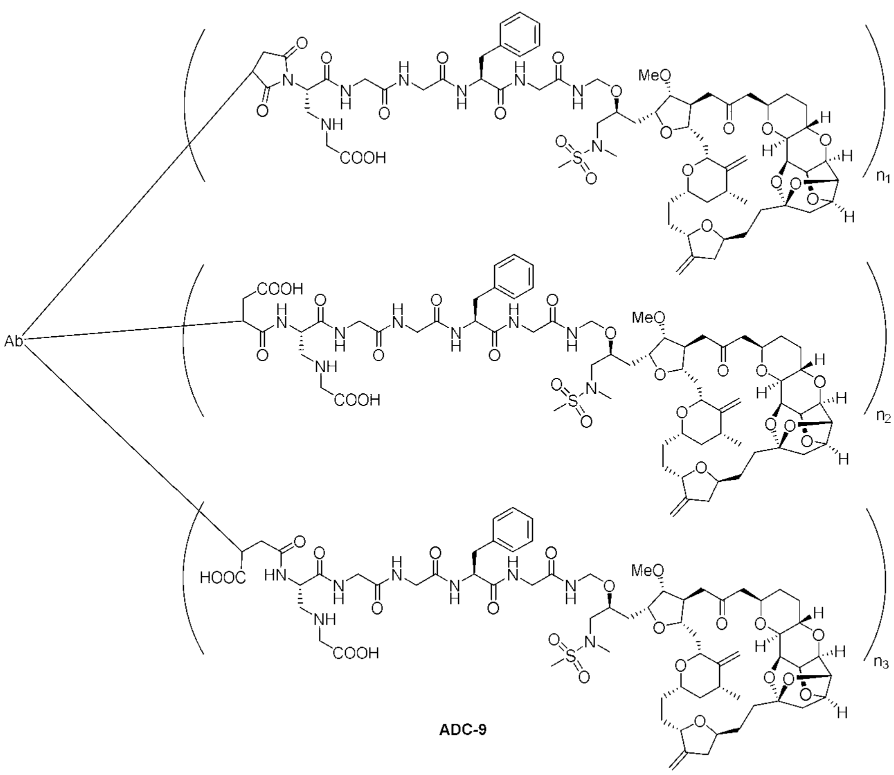

ADC-9

Example 106 Preparation of **ADC-10**

The **ADC-10** was prepared by using compound **L-11** and anti-Trop2 antibody TR001 according to the method in general procedure A.

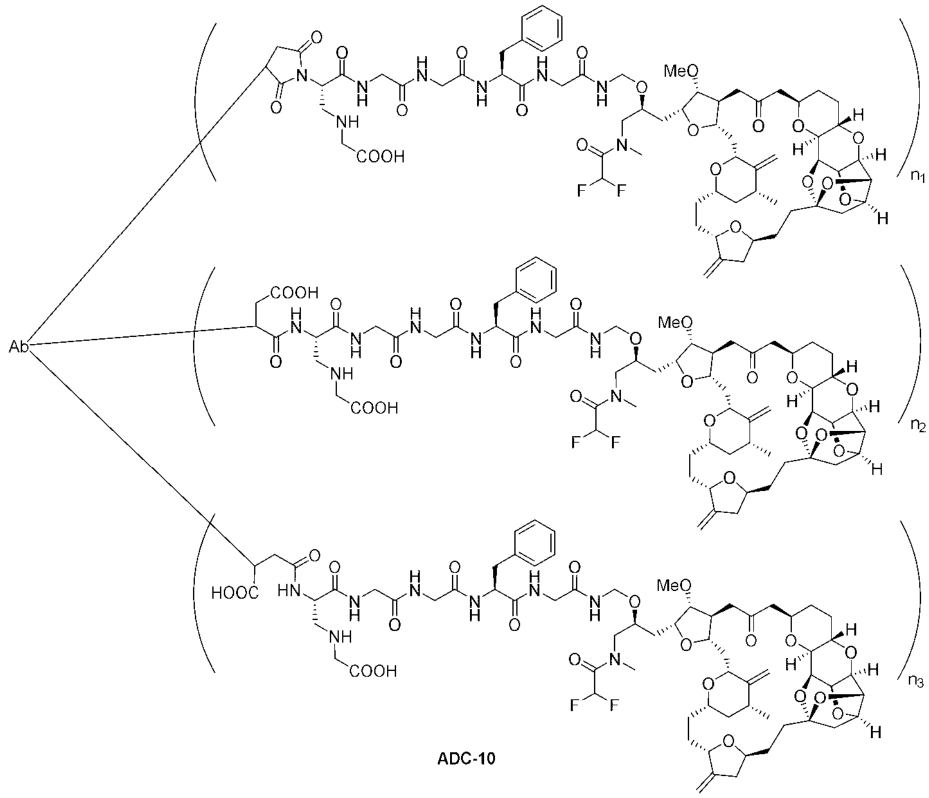

ADC-10

Example 107 Preparation of **ADC-11**

The **ADC-11** was prepared by using compound **L-1'** and anti-Trop2 antibody TR001 according to the method in general procedure A.

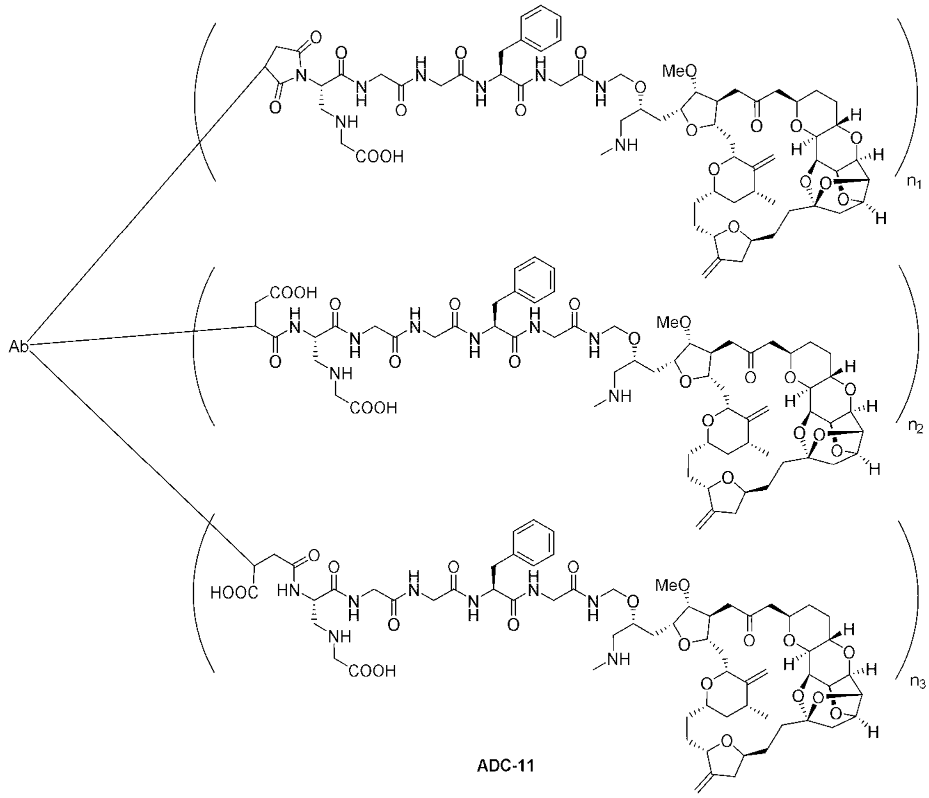

ADC-11

Example 108 Preparation of **ADC-12**

The **ADC-12** was prepared by using compound **L-2'** and anti-Trop2 antibody TR001 according to the method in general procedure A.

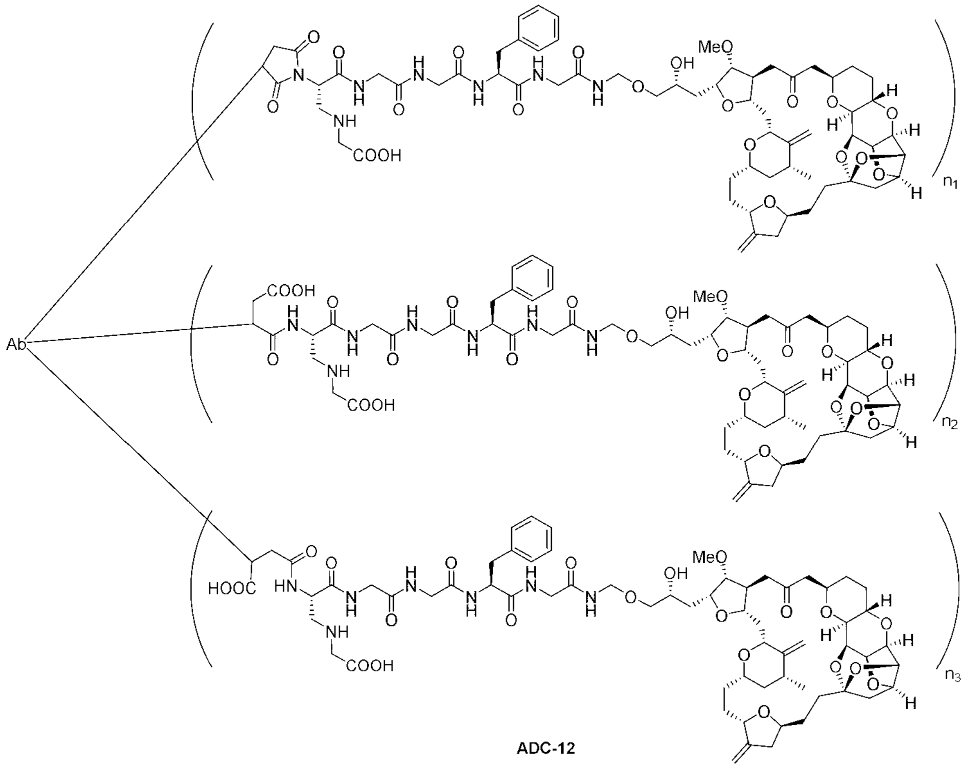

ADC-12

Example 109 Preparation of **ADC-13**

The **ADC-13** was prepared by using compound **L-15** and anti-Trop2 antibody TR001 according to the method in general procedure C.

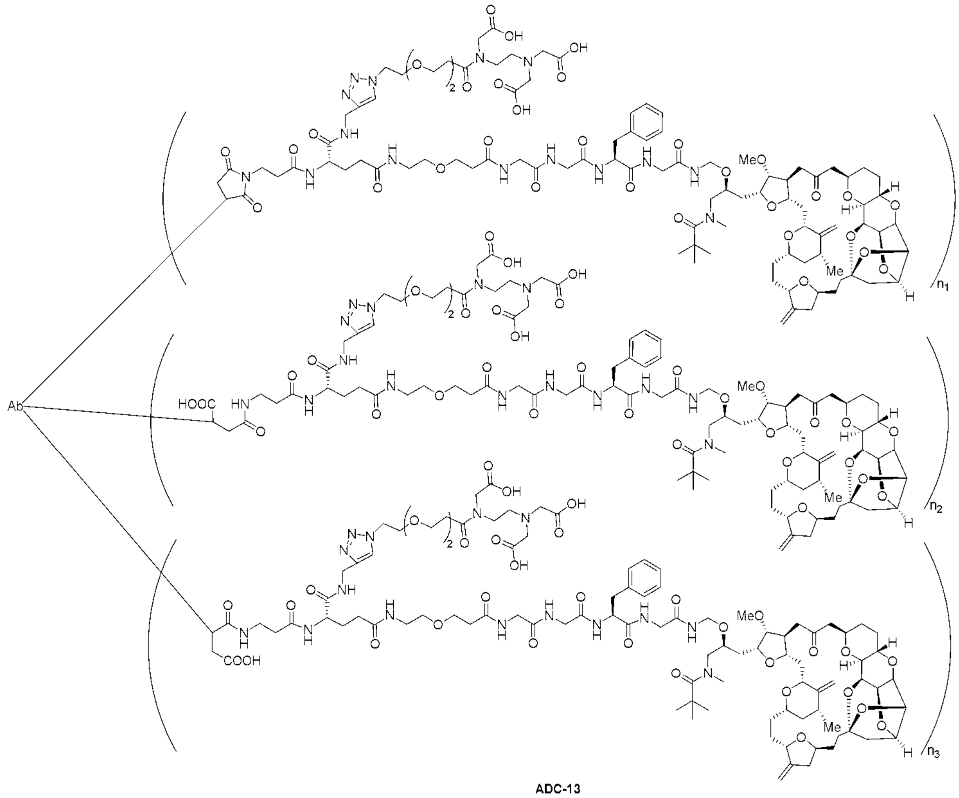

ADC-13

Example 110 Preparation of **ADC-14**

The **ADC-14** was prepared by using compound **L-16** and anti-Trop2 antibody TR001 according to the method in general procedure C.

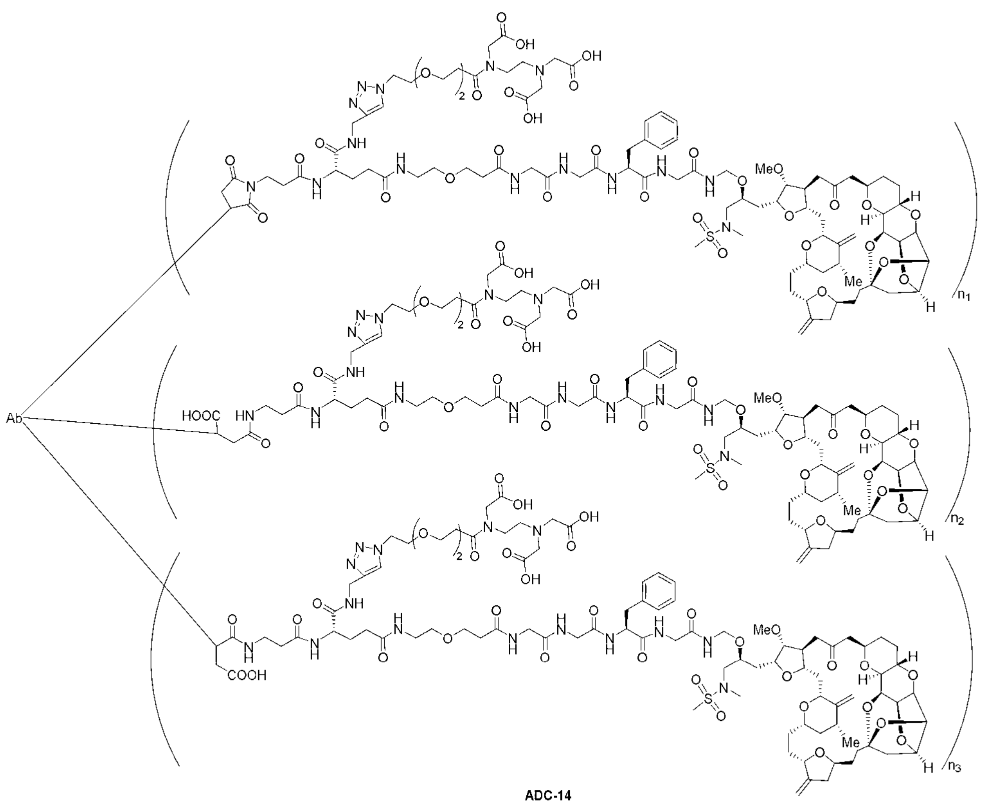

ADC-14

Example 111 Preparation of **ADC-15**

The **ADC-15** was prepared by using compound **L-17** and anti-Trop2 antibody TR001 according to the method in general procedure C.

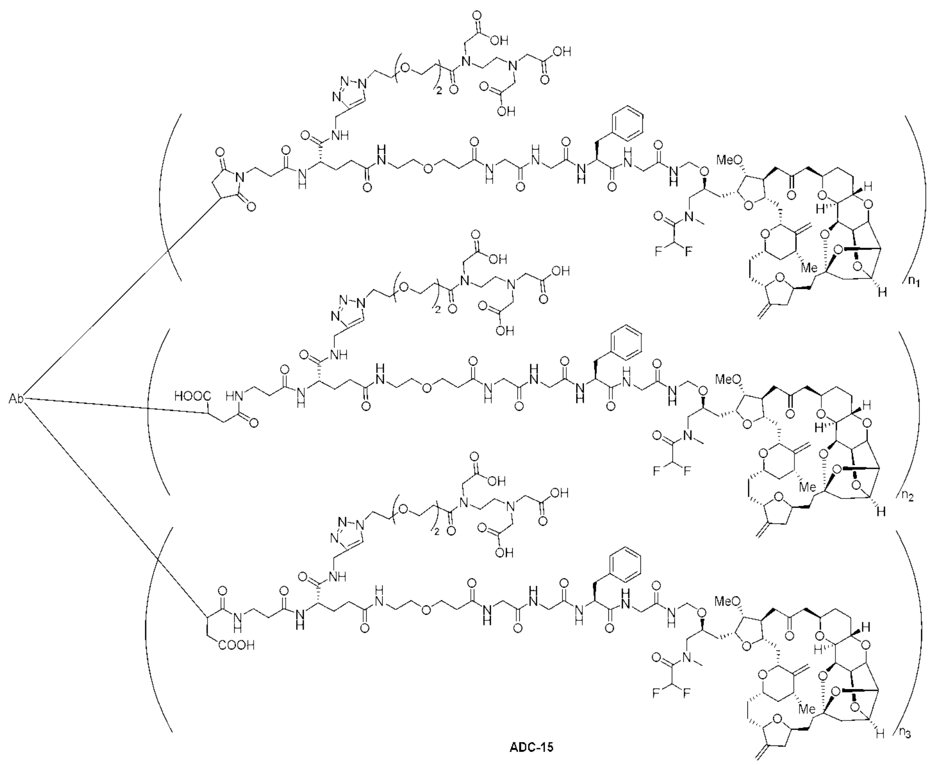

ADC-15

Example 112 Preparation of **ADC-16**

The **ADC-16** was prepared by using compound **L-18** and anti-Trop2 antibody TR001 according to the method in general procedure C.

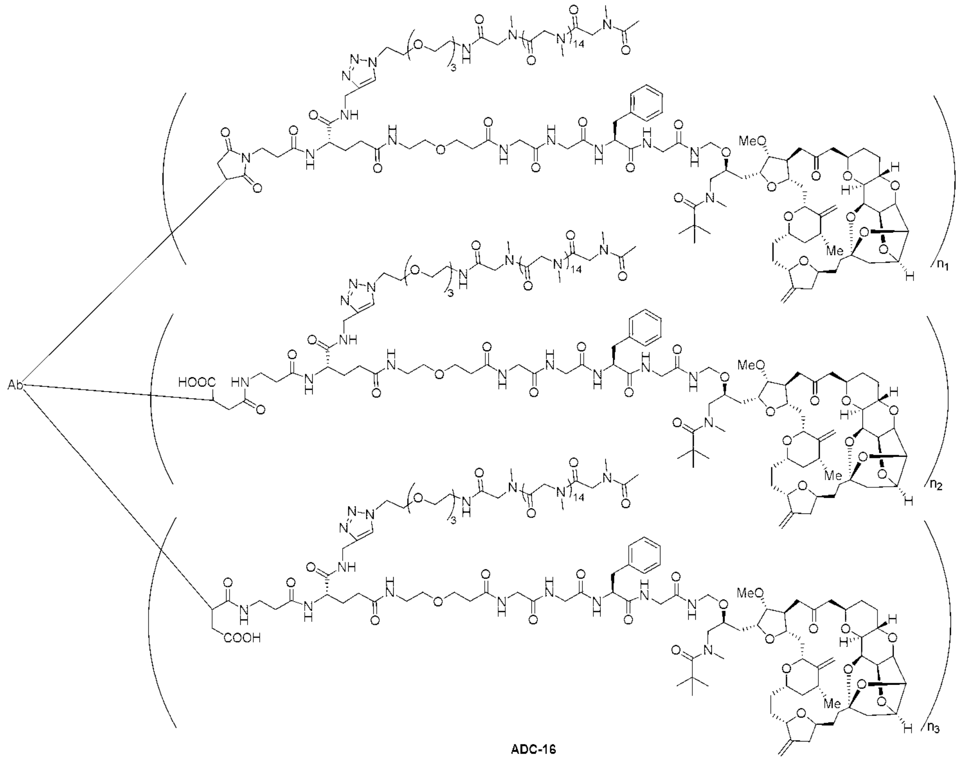

ADC-16

Example 113 Preparation of **ADC-17**

The **ADC-17** was prepared by using compound **L-19** and anti-Trop2 antibody TR001 according to the method in general procedure C.

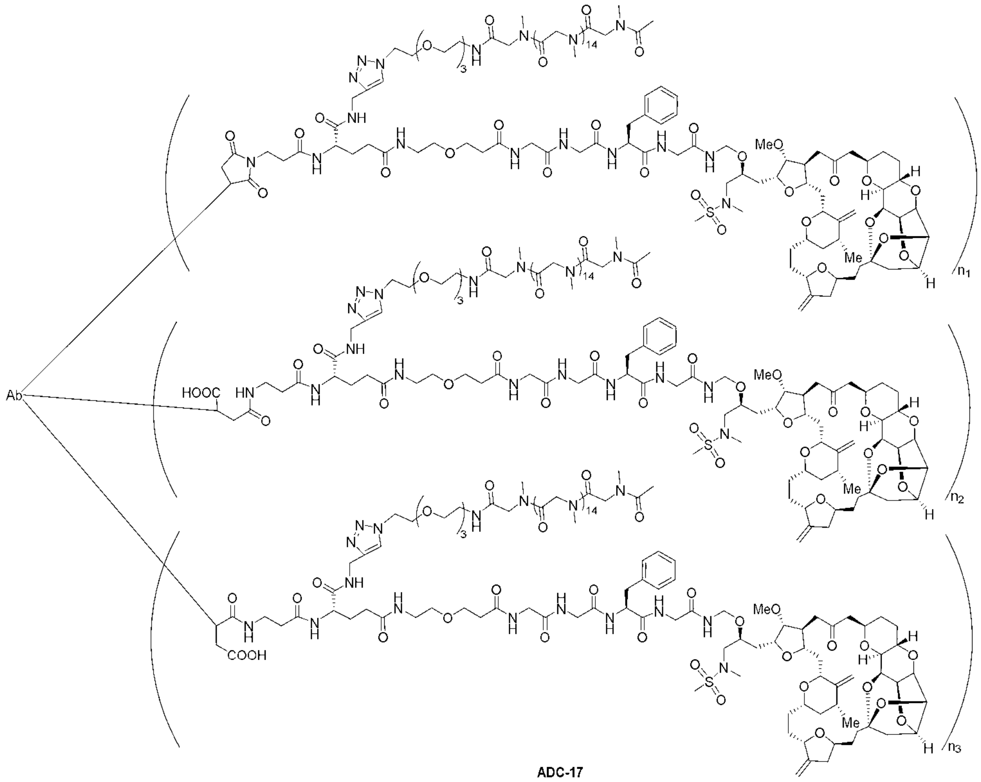

ADC-17

Example 114 Preparation of **ADC-18**

The **ADC-18** was prepared by using compound **L-20** and anti-Trop2 antibody TR001 according to the method in general procedure C.

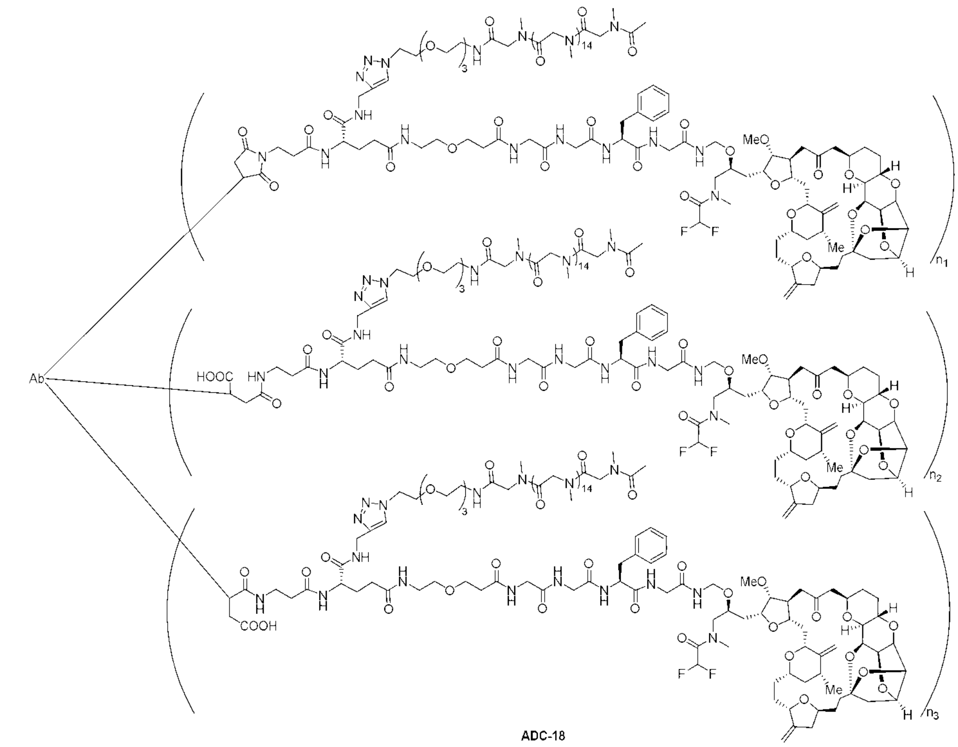

ADC-18

Example 115 Preparation of **ADC-19**

The **ADC-19** was prepared by using compound **L-8** and anti-Trop2 antibody TR001 according to the method in general procedure C.

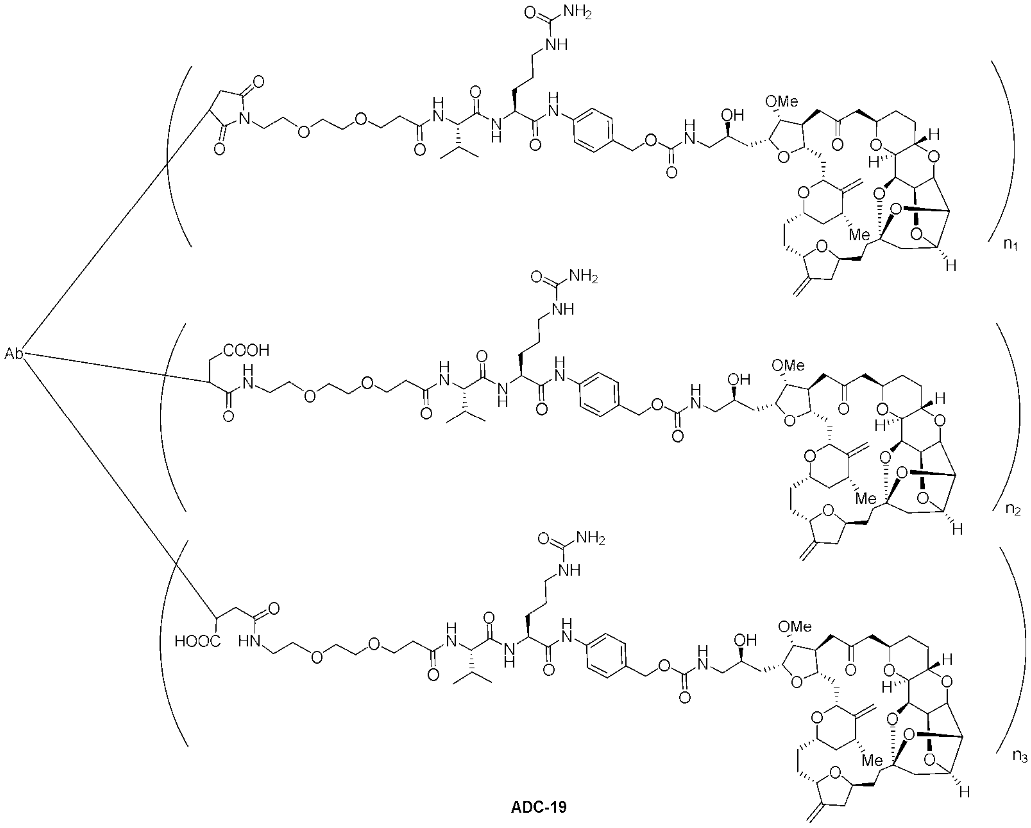

**ADC-19**

Example 116 Preparation of **ADC-20**

The **ADC-20** was prepared by using compound **L-21** and anti-Trop2 antibody TR001 according to the method in general procedure C.

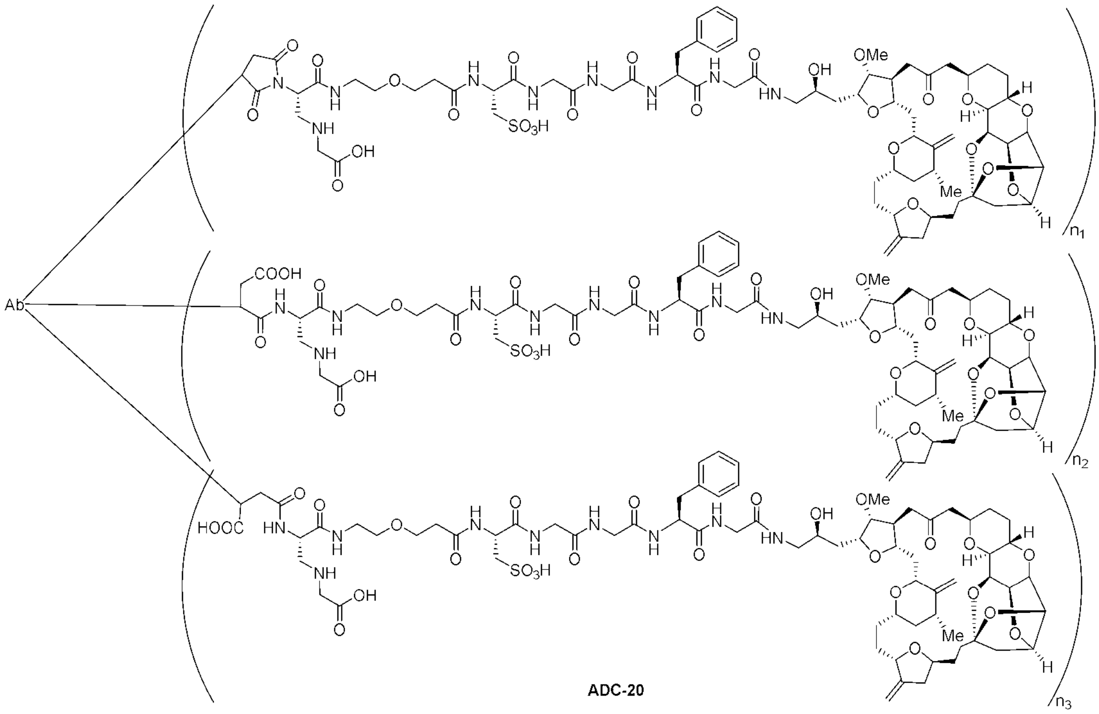

ADC-20

Example 117 Preparation of **ADC-21**

The **ADC-21** was prepared by using compound **L-22** and anti-Trop2 antibody TR001 according to the method in general procedure C.

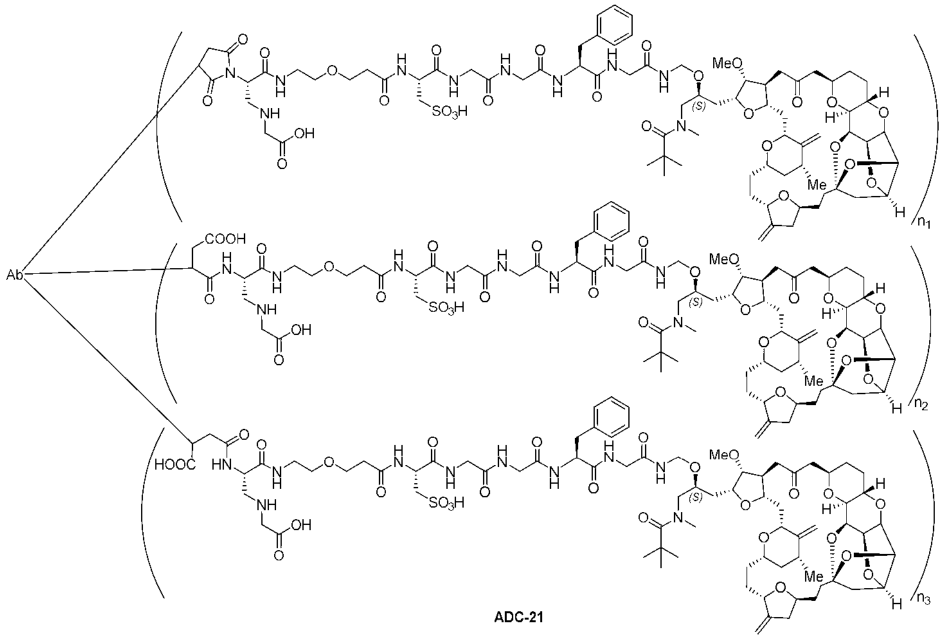

ADC-21

Example 118 Preparation of **ADC-22**

The **ADC-22** was prepared by using compound **L-23** and anti-Trop2 antibody TR001 according to the method in general procedure C.

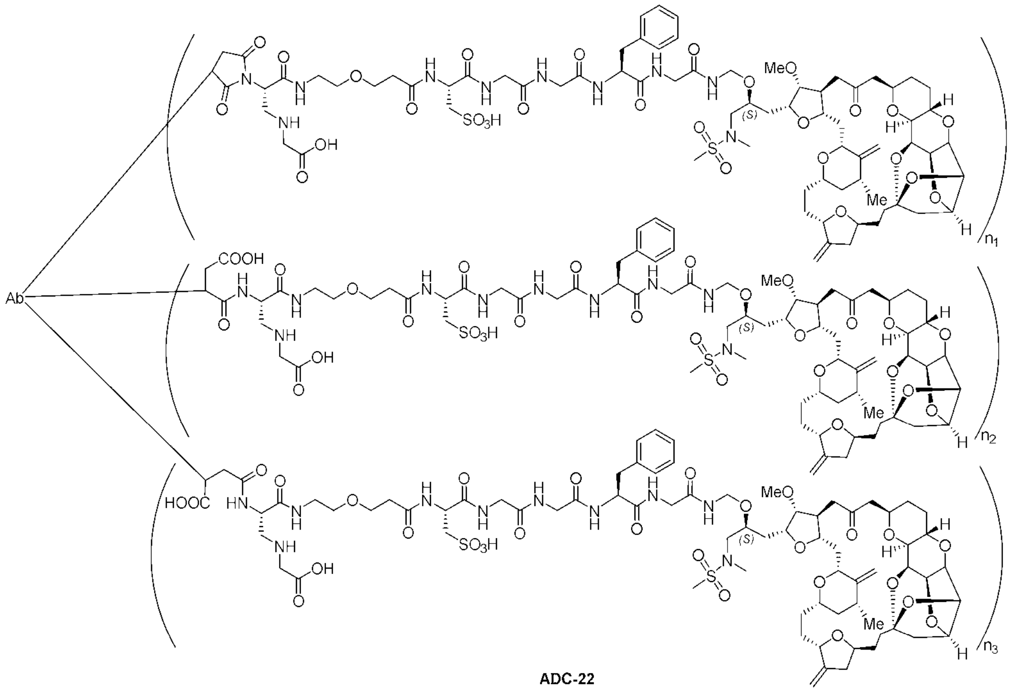

ADC-22

Example 119 Preparation of **ADC-23**

The **ADC-23** was prepared by using compound **L-24** and anti-Trop2 antibody TR001 according to the method in general procedure C.

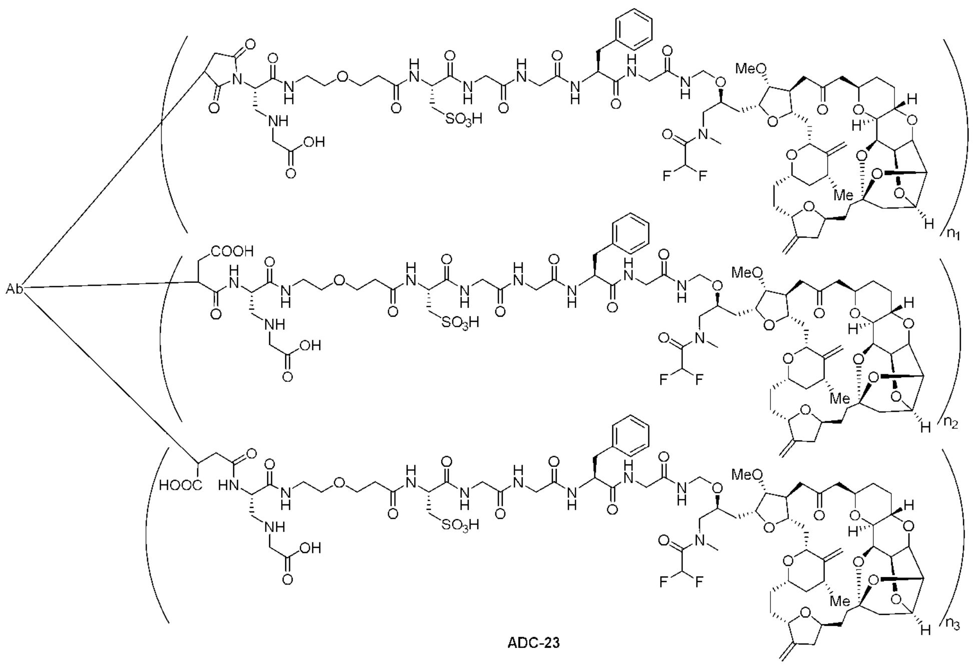

ADC-23

Example 120 Determination of drug-antibody ratio (DAR)

Reversed-phase high performance liquid chromatography (RP-HPLC) for detecting DAR Preparation of RP-HPLC mobile phase:

RP mobile phase A: 0.1% TFA in water; RP mobile phase B: 0.1% TFA in acetonitrile

The sample to be tested and the corresponding antibody control were diluted with sample diluent to 1 mg/mL; 2 μL of DTT stock solution was added to every 98 μL of diluted sample, and a blank control of 98 μL of sample diluent + 2 μL of

DTT stock solution was prepared. Each sample was mixed well and then heated in a 65 °C metal bath for 30 min. The processed samples were centrifuged at 14000 rpm for 5 min or filtered with a 0.22 μm filter to remove large particles from the samples, and an inner cannula was placed in each sample bottle and the sample bottles were then capped.

The sample bottles filled with samples were placed on a sample plate, and the corresponding position, injection volume, number of injection needles and injection method for each sample were set in accordance with UPLC Standard Operating Procedures. The chromatographic column model was Proteomix RP-1000 (4.6*100 mm, 5 μm), Sepax.

The test wavelengths were 214 nm and 280 nm. The sample running method was edited as follows:

| Time (min) | Flow rate (mL/min) | Volume percentage of RP mobile phase A (%): | Volume percentage of RP mobile phase B (%): |
|---|---|---|---|
| 0 | 0.5 | 75 | 25 |
| 3 | 0.5 | 75 | 25 |
| 28 | 0.5 | 50 | 50 |
| 30 | 0.5 | 5 | 95 |
| 32 | 0.5 | 5 | 95 |
| 33 | 0.5 | 75 | 25 |
| 40 | 0.5 | 75 | 25 |

Table 4: Detailed data of DAR of ligand-drug conjugates (ADCs) disclosed:

| Number of compound | Number of ADC prepared by conjugation | DAR |
|---|---|---|
| **L-1** | **ADC-1** | 6.82 |
| **L-2** | **ADC-2** | 6.08 |
| **L-1** | **ADC-3** | 1.87 |
| **L-2** | **ADC-4** | 1.72 |
| **L-3** | **ADC-5** | 7.20 |
| **L-7** | **ADC-6** | 6.81 |
| **L-8** | **ADC-7** | 7.63 |
| **L-9** | **ADC-8** | 6.97 |
| **L-10** | **ADC-9** | 7.15 |
| **L-11** | **ADC-10** | 7.86 |
| **L-1'** | **ADC-11** | 7.58 |
| **L-2'** | **ADC-12** | 7.30 |
| **L-15** | **ADC-13** | 4.07 |
| **L-16** | **ADC-14** | 4.35 |
| **L-17** | **ADC-15** | 4.12 |
| **L-18** | **ADC-16** | 3.94 |
| **L-19** | **ADC-17** | 3.88 |
| **L-20** | **ADC-18** | 3.74 |
| **L-8** | **ADC-19** | 3.75 |
| **L-21** | **ADC-20** | 3.63 |
| **L-22** | **ADC-21** | 3.71 |
| **L-23** | **ADC-22** | 3.84 |
| **L-24** | **ADC-23** | 3.96 |

Example 121 Screening of compounds for in vitro pharmacodynamic activity

In the present invention, various human-derived tumor cell lines (BxPC-3 (human orthotopic pancreatic adenocarcinoma cell), SW620 (human colon cancer cell), H1975 (human lung adenocarcinoma cell), HCC827 (human lung adenocarcinoma cell), FaDu (human pharyngeal squamous cell carcinoma cell) and A431(human epidermal carcinoma cells)) were used as experimental models to evaluate the cytotoxicity of compound to tumor cell lines. A certain number of tumor cells were seeded into a 96-well plate. After the cells adhered to the wall, gradient dilutions of tested compounds were added to the test cells, followed by incubation at 37 °C and 5% $CO_2$ for 5 days. Cell viability was measured using MTS, and the inhibitory effect of the control compounds and the tested compounds on the tumor cell lines was evaluated by$IC_{50}$. The compound with a starting concentration of 4000 nM was diluted 7 folds, with a total of 8 concentration points. Finally, the survival rate was given by the equation: survival rate = (experimental group - blank) / (control - blank) × 100%, and then the curve was fitted using the Graph Pad Prism 9 four-parameter model to calculate $IC_{50}$.

Table 5: In vitro pharmacodynamic activity of control compounds and tested compounds in human-derived tumor cell lines BxPC-3, SW620 and H1975:

| Compound | BxPC-3 | SW620 | H1975 |
|---|---|---|---|
| | $IC_{50}$(nM) | | |
| **D-4(eribulin)** | 0.06 | 2.56 | 0.39 |
| **D-2** | 0.03 | 1.25 | 0.25 |
| **D-3** | <0.005 | <0.005 | <0.005 |
| **D-7** | <0.005 | <0.005 | 0.01 |
| **D-8** | <0.005 | <0.005 | <0.005 |
| **D-9** | <0.005 | <0.005 | <0.005 |
| **D-10** | 0.02 | 0.2 | 0.13 |
| **D-11** | 0.01 | 0.15 | 0.06 |
| **D-12** | <0.005 | <0.005 | <0.005 |
| **D-13** | <0.005 | <0.005 | <0.005 |
| **D-14** | 0.01 | 0.19 | 0.1 |
| **D-15** | 0.03 | 0.69 | 0.14 |
| **D-21** | 0.05 | 0.24 | 0.24 |

Table 6: In vitro pharmacodynamic activity of control compounds and tested compounds in human-derived tumor cell lines HCC827, FaDu and A431:

| Compound | HCC827 | \| FaDu | A431 |
|---|---|---|---|
| | $IC_{50}$(nM) | | |
| **D-4(eribulin)** | 0.05 | 0.73 | 0.03 |
| **D-2** | 0.025 | 0.37 | 0.02 |
| **D-3** | <0.005 | <0.005 | <0.005 |
| **D-7** | <0.005 | 0.01 | <0.005 |
| **D-8** | <0.005 | <0.005 | <0.005 |
| **D-9** | <0.005 | <0.005 | <0.005 |
| **D-10** | 0.01 | 0.16 | 0.03 |
| **D-11** | 0.01 | 0.08 | 0.02 |
| **D-12** | <0.005 | <0.005 | <0.005 |
| **D-13** | <0.005 | <0.005 | <0.005 |

(continued)

| Compound | HCC827 | \| FaDu | A431 |
|---|---|---|---|
| | $IC_{50}$(nM) | | |
| **D-14** | 0.01 | 0.16 | 0.01 |
| **D-15** | 0.02 | 0.33 | 0.02 |
| **D-21** | 0.04 | 0.39 | 0.12 |

The results are shown in the table above and FIGs. 1A-1F.

Conclusion: In BxPC-3, SW620, H1975, HCC827, FaDu and A431 cell models, various compounds synthesized herein had obvious anti-tumor effects, and compounds D-3, D-7, D-8, D-9, D-12, and D-13 showed the best effects.

Example 122 Evaluation on stability of ADCs

ADCs were diluted with sterile human plasma to prepare samples with a drug concentration of 0.6 mg/ml and incubated in a 37 °C water bath for 0, 3 and 7 days respectively. The samples incubated in plasma for 0, 3 and 7 days and the samples serving as controls, diluted with PBS with a final drug concentration of 0.6 mg/ml were purified and extracted respectively to give ADCs. Finally, the purified and extracted ADCs were tested accordingly to analyze their stability in plasma.

Determination of monomer rate using SEC-HPLC:

Chromatographic column: Biocore SEC-300 5 μm, 4.6×300 mm
Manufacturer: NanoChrom, Product code: B213-050030-04630S
Mobile phase: 50 mM PB+300 mM NaCl+200 mM Arg+5% IPA, pH=6.5

Method parameters are listed below:

| Parameter | Setting |
|---|---|
| Flow rate | 0.3 mL/min |
| Wavelength | 280 nm |
| Column temperature | 30 °C |
| Sample tray temperature | Room temperature |
| Sample load | 20 ug |
| Max. pressure | 150 bar/15 MPa/2175 PSI |
| Gradient | Isocratic |
| Run time | 20 min |

Table 7: Evaluation on stability of tested ADCs of the invention in plasma:

| Sample | Day 0 | | | | Day 1 | | | | Day 5 | | | |
|---|---|---|---|---|---|---|---|---|---|---|---|---|
| | Aggregation % | Monomer % | Degradation rate % | RP-DAR | Aggregation % | Monomer % | Degradation rate % | RP-DAR | Aggregation % | Monomer % | Degradation rate % | RP-DAR |
| **TR001** | 0.51 | 98.78 | 0.71 | N/A | 0.45 | 99.29 | 0.26 | N/A | 0.4 | 99.33 | 0.27 | N/A |
| **ADC-13** | 9.62 | 88.81 | 1.58 | 4.07 | 5.83 | 93.34 | 0.83 | 3.88 | 5.28 | 93.72 | 1.00 | 3.80 |
| **ADC-14** | 4.78 | 93.91 | 1.31 | 4.35 | 4.78 | 94.54 | 0.69 | 4.19 | 4.28 | 94.89 | 0.83 | 4.02 |
| **ADC-15** | 7.50 | 91.02 | 1.48 | 4.12 | 6.47 | 92.39 | 1.14 | 3.73 | 5.81 | 92.91 | 1.27 | 3.51 |
| **ADC-16** | 4.94 | 94.56 | 0.50 | 3.94 | 4.79 | 94.45 | 0.76 | 4.15 | 4.61 | 94.52 | 0.87 | 4.06 |
| **ADC-17** | 6.41 | 92.88 | 0.71 | 3.88 | 6.46 | 92.42 | 1.12 | 4.15 | 6.29 | 92.45 | 1.26 | 3.75 |
| **ADC-18** | 4.44 | 95.23 | 0.33 | 3.74 | 4.21 | 95.27 | 0.52 | 3.88 | 4.07 | 95.33 | 0.59 | 3.73 |

Conclusion: The above results show that the ADCs synthesized in the present invention have good stability in human plasma.

Example 123 In vitro pharmacodynamic activity testing of ADCs-single tumor model

In the present invention, various human-derived tumor cell lines (A431, HCC827, N87 (human gastric cancer cells), SW620 and FaDu) were used as experimental models to evaluate the in vitro pharmacodynamic activity of the ADCs. A certain number of tumor cells were seeded into a 96-well plate, gradient dilutions of test antibodies and corresponding ADCs were added to the cells, and treated for 5 days. Cell viability was detected using MTS, and the inhibitory effect of the test antibodies and ADCs on the tumor cell lines was evaluated by $IC_{50}$. The antibodies and the ADCs, having a starting concentration of 500 nM, were diluted 7 folds, with a total of 8 concentration points, and the treatment was carried out for 5 days. The final calculation was based on the equation: survival rate = (experimental group - blank) / (control - blank) × 100%, and then Graph Pad Prism was used to fit the curve and calculate $IC_{50}$.

Table 8: In vitro pharmacodynamic activity of tested ADCs in human-derived tumor cell lines A431, HCC827 and FaDu:

| No. | A431 | HCC827 | FaDu |
|---|---|---|---|
| | $IC_{50}$ (nM) | | |
| **TR001** | >500 | >500 | >500 |
| **ADC-1** | 0.013 | 0.004 | 0.091 |
| **ADC-2** | 0.212 | 5.365 | - |
| **ADC-5** | 0.033 | 0.146 | 2.542 |
| **ADC-13** | 0.142 | 0.230 | 4.012 |
| **ADC-14** | 0.126 | 0.461 | 9.020 |
| **ADC-15** | 0.125 | 0.657 | 11.342 |
| **ADC-16** | 0.199 | 0.953 | 17.285 |
| **ADC-17** | 0.257 | 2.205 | - |
| **ADC-18** | 0.194 | 3.000 | - |

Table 9: In vitro pharmacodynamic activity of tested ADCs in human-derived tumor cell lines N87 and SW620:

| No. | N87 | SW620 |
|---|---|---|
| | $IC_{50}$ (nM) | |
| **TR001** | >500 | >500 |
| **ADC-1** | 0.093 | 13.417 |
| **ADC-5** | 1.421 | 14.724 |

The results are shown in the table above and FIGs. 2A-2E.

Conclusion: In A431, HCC827, N87, SW620 and Fadu cell models, naked anti-TR001 had no cytotoxic activity to tumor cells, and the ADCs synthesized according to the invention had obvious anti-tumor effect.

Example 124 In vitro pharmacodynamic activity testing of ADCs-heterogeneous tumor model

In the present invention, heterogeneous tumor cell lines (A431+SW620) were used as experimental models to evaluate the in vitro pharmacodynamic activity of the ADCs. A certain number of tumor cells were seeded into a 96-well plate, gradient dilutions of tested ADCs were added to the cells, and treated for 5 days. Cell viability was detected using MTS, and the inhibitory effect of the test antibody and ADCs on the tumor cell lines was evaluated by $IC_{50}$. The antibody and the ADCs, having a starting concentration of 500 nM, were diluted 7 folds, with a total of 8 concentration points, and the treatment was carried out for 5 days. The final calculation was based on the equation: survival rate = (experimental group - blank) / (control - blank) × 100%, and then Graph Pad Prism was used to fit the curve and calculate $IC_{50}$.

Table 10: In vitro pharmacodynamic activity of tested ADCs in heterogeneous tumor cell lines A431+SW620:

| **No.** | **A431+SW620** |
|---|---|
| | $IC_{50}$ (nM) |
| **ADC-19 (control)** | 39.908 |
| **ADC-13** | 2.884 |
| **ADC-14** | 6.667 |
| **ADC-15** | 7.617 |
| **ADC-16** | 11.280 |

The results are shown in the table above and FIG. 3.

Conclusion: In the A431+ SW620 heterotumor cell model, the ADCs synthesized according to the present invention had significant anti-tumor effect and were superior to the control ADC-19.

Example 125 In vivo pharmacodynamic activity testing of ADCs

In the present invention, *BALB*/*c-nu* subcutaneously inoculated a human-derived tumor cell line (A431) was used as an experimental model to evaluate the in vivo pharmacodynamics of ADCs. Suspension containing a certain number of tumor cells was inoculated subcutaneously into *BALB*/*c-nu.* When the tumor volume grew to about 180 $mm^3$, the vehicle and ADC-5 were injected into the tail vein once a week for three times. Observation was continued. Tumor size and body weight were measured twice a week to evaluate the inhibitory effect of ADCs on tumor growth.

The results are shown in the table below.

Table 11: Efficacy of the tested ADC on A431 mouse xenograft:

| Compound | Mean tumor volume ($mm^3$) | | | | | | T/C % | TGI % |
|---|---|---|---|---|---|---|---|---|
| | D0 | D3 | D7 | D10 | D14 | D17 | | |
| Blank | 175.01 | 456.78 | 828.16 | 1164.44 | 1438.74 | 1671.07 | 100 | 0 |
| ADC-5 (3 mg/Kg) | 171.24 | 283.15 | 199.84 | 198.75 | 178.75 | 158.06 | 9.67 | 90.33 |

$$T/C\ \% = T_{RTV} / C_{RTV} \times 100\%;\ TGI\% = (1-T/C)\times 100\%$$

Table 12: Effect of tested ADC on body weight of A431 transplanted mice:

| Compound | D0 | D3 | | D7 | | D10 | | D14 | | D17 | |
|---|---|---|---|---|---|---|---|---|---|---|---|
| | Mean Body weigh t (g) | Mean Body weigh t (g) | BWC % | Mean Body weigh t (g) | BWC % | Mean Body weigh t (g) | BWC % | Mean Body weigh t (g) | BWC % | Mean Body weigh t (g) | BWC % |
| Blank | 20.45 | 22.09 | 8.06 | 23.11 | 13.05 | 23.28 | 13.86 | 24.40 | 19.35 | 24.19 | 18.33 |
| ADC-5 (3 mg/Kg) | 19.85 | 21.47 | 8.17 | 22.24 | 12.13 | 21.78 | 9.86 | 22.44 | 13.14 | 21.98 | 10.93 |

Conclusion: In mice transplanted with A431 single tumor, the ADC synthesized according to the invention had significant anti-tumor effects.

The above embodiments are only used to illustrate but not limit the technical solutions of the invention. Any modification or equivalent substitution of the technical solutions of the invention without departing from the purpose and scope of the technical solutions of the invention should be covered within the scope of the invention.

## Claims

1. A ligand-drug conjugate of formula I or a pharmaceutically acceptable salt or solvate thereof,

$$Ab\text{-}(L\text{-}D)_n \qquad \text{Formula I}$$

wherein,

Ab is a ligand unit;
L is a linker for covalently connecting Ab to D;
n is selected from integers or decimals between 1 and 40;
-D is as shown in formula II or formula III:

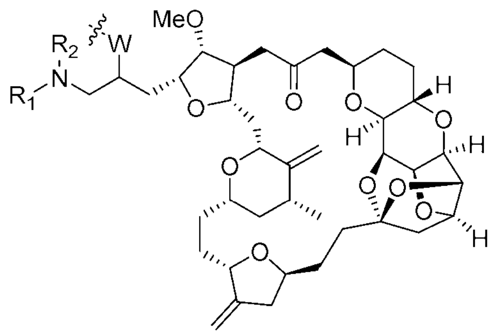

Formula II

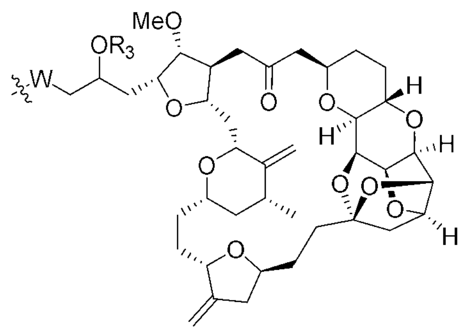

Formula III

wherein W is an oxygen atom or a sulfur atom,
$R_1$ and $R_2$ are the same or different, and are each independently selected from: hydrogen atom, alkyl, alkoxy, alkenyl, cycloalkyl, aryl, heteroaryl, heterocyclyl, $-C(O)-Q_1-Q_2$ and $-SO_2-Q_1-Q_2$, wherein $Q_1$ is selected from the group consisting of O, N, S atoms and a chemical bond, $Q_2$ is selected from the group consisting of alkyl, cycloalkyl, heterocyclyl, spirocyclyl, bridged ring, alkenyl, aryl and heteroaryl; optionally, the alkyl, alkoxy, alkenyl, cycloalkyl, heterocyclyl, spirocyclyl, bridged ring, aryl and heteroaryl are each independently substituted by one or more substituents selected from the group consisting of hydrogen atom, alkyl, alkoxy, halogen, deuterium, amino, cyano, hydroxy, mercapto, azido, nitro, carboxyl, acyl, carbonyl, hydroxyalkyl, cycloalkyl, heterocyclyl, aryl and heteroaryl; or $R_1$ and $R_2$, together with a nitrogen atom connected thereto, form a 3- to 8-membered heterocyclyl; optionally, the heterocyclyl is substituted by one or more substituents selected from the group consisting of hydrogen atom, alkyl, alkoxy, halogen, deuterium, amino, cyano, hydroxy, mercapto, azido, nitro, carboxyl, acyl, carbonyl, hydroxyalkyl, cycloalkyl, heterocyclyl, aryl and heteroaryl; $R_3$ is selected from the group consisting of hydrogen atom, alkyl, acyl, sulfonyl, cycloalkyl, heterocyclyl, aryl and heteroaryl; optionally, the alkyl, acyl, sulfonyl, cycloalkyl, heterocyclyl, aryl and heteroaryl are each independently substituted by one or more substituents selected from the group consisting of hydrogen atom, alkyl, alkoxy, halogen, deuterium, amino, cyano, hydroxy, mercapto, azido, nitro, carboxyl, acyl, carbonyl, hydroxyalkyl, cycloalkyl, heterocyclyl, aryl and heteroaryl.

2. The ligand-drug conjugate or the pharmaceutically acceptable salt or solvate thereof according to claim 1, wherein the ligand unit Ab is selected from the group consisting of an antibody, an antibody fragment or a protein; the antibody is preferably selected from the group consisting of a murine antibody, a rabbit antibody, a phage display-derived antibody, a yeast display-derived antibody, a chimeric antibody, a humanized antibody, a fully human antibody, an antibody fragment, a bispecific antibody and a multispecific antibody.

3. The ligand-drug conjugate or the pharmaceutically acceptable salt or solvate thereof according to claim 1 or 2, wherein the antibody is a monoclonal antibody, is selected, without limitation, from: anti-EGFRvIII antibody, anti-PD-1 antibody, anti-PD-L1 antibody, anti-DLL-3 antibody, anti-PSMA antibody, anti-CD70 antibody, anti-MUC16 antibody, anti-ENPP3 antibody, anti-TDGF1 antibody, anti-ETBR antibody, anti-MSLN antibody, anti-TIM-1 antibody, anti-LRRC15 antibody, anti-LIV-1 antibody, anti-CanAg/AFP antibody, anti-claudin 18.2 antibody, anti-Mesothelin antibody, anti-HER2(ErbB2) antibody, anti-EGFR antibody, anti-c-MET antibody, anti-SLITRK6 antibody, anti-KIT/CD117 antibody, anti-STEAP1 antibody, anti-SLAMF7/CS1 antibody, anti-NaPi2B/SLC34A2 antibody, anti-GPNMB antibody, anti-HER3(ErbB3) antibody, anti-MUC1/CD227 antibody, anti-AXL antibody, anti-CD166 antibody, anti-B7-H3(CD276) antibody, anti-PTK7/CCK4 antibody, anti-PRLR antibody, anti-EFNA4 antibody, anti-5T4 antibody, anti-NOTCH3 antibody, anti-Nectin 4 antibody, anti-TROP-2 antibody, anti-CD142 antibody, anti-CA6 antibody, anti-GPR20 antibody, anti-CD174 antibody, anti-CD71 antibody, anti-EphA2 antibody, anti-LYPD3 antibody, anti-FGFR2 antibody, anti-FGFR3 antibody, anti-FRα antibody, anti-CEACAMs antibody, anti-GCC antibody, anti-Integrin Av antibody, anti-CAIX antibody, anti-P-cadherin antibody, anti-GD3 antibody, anti-Cadherin 6 antibody, anti-LAMP1 antibody, anti-FLT3 antibody, anti-BCMA antibody, anti-CD79b antibody, anti-CD19 antibody, anti-CD33 antibody, anti-CD56 antibody, anti-CD74 antibody, anti-CD22 antibody, anti-CD30 antibody, anti-CD37 antibody, anti-CD47 antibody, anti-CD138 antibody, anti-CD352 antibody, anti-CD25 antibody or anti-CD123 antibody.

**4.** The ligand-drug conjugate or the pharmaceutically acceptable salt or solvate thereof according to claim 1, wherein L has a structure shown in formula IV:

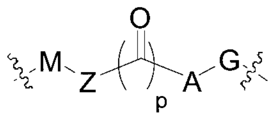

Formula IV

wherein,

M is a linking unit covalently connected to Ab;
Z is selected from the group consisting of -C1-C10 alkylene-, -C3-C8 carbocycle-, -arylene-, -3- to 8-membered heterocyclyl-, $-(CH_2CH_2O)_r-$, sulfonyl, amide, a chemical bond,

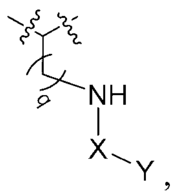,
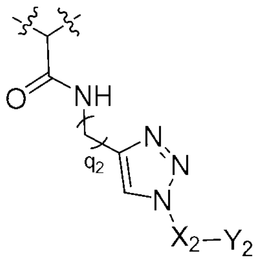

and any combinations thereof, wherein $X_1$ and $X_2$ are selected from the group consisting of -C1-C10 alkylene-, -C3-C8 carbocycle-, -arylene-, -3- to 8-membered heterocyclyl-, $-(CH_2CH_2O)_r-$, $-NR_4-$, carbonyl and any combinations thereof, wherein $R_4$ is selected from the group consisting of hydrogen atom, deuterium atom, alkyl and substituted alkyl; optionally, the -C1-C10 alkylene-, - C3-C8 carbocycle-, and -3- to 8-membered heterocyclyl- are each independently substituted with one or more substituents selected from the group consisting of hydrogen atom, deuterium atom, halogen, hydroxyl, cyano, nitro, amino, alkyl, heteroalkyl, substituted alkyl, alkoxy, carboxy and cycloalkyl; each heterocyclyl independently contains 1 to 3 atoms selected from the group consisting of N, O and S; $Y_1$ is a hydrophilic structure selected from the group consisting of carboxyl, phosphoric acid, polyphosphoric acid, phosphorous acid, sulfonic acid, sulfinic acid, polyethylene glycol (PEG), and any combinations thereof; $Y_2$ is selected from the following structures:

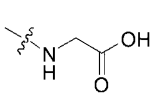, 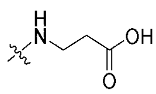, 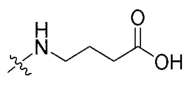, 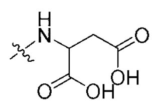, 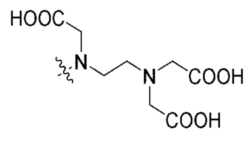,

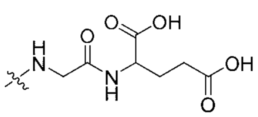, 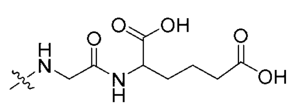, 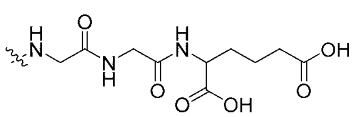, 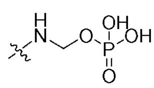,

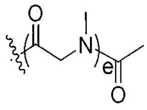, 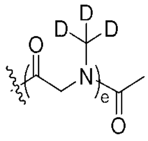 and 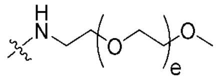 ;

r is selected from integers between 1 and 10; $q_1$ and $q_2$ are selected from integers between 1 and 8; e is selected from integers between 1 and 20;
A is a peptide residue consisting of 2 to 7 amino acids;
G is a spacer unit connected to D; and
p is 0 or1.

**5.** The ligand-drug conjugate or the pharmaceutically acceptable salt or solvate thereof according to claim 4, wherein the linking unit M has a succinimide structure as represented by formula a, or a structure as represented by formula b or formula c in which the ring in succinimide group is in open form:

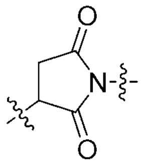
Formula a

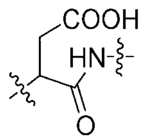
Formula b

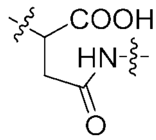
Formula c

In formula a, formula b or formula c, a wavy line on the left indicates connecting to a linking site on Ab, and a wavy line on the right indicates connecting to a linking site on Z.

**6.** The ligand-drug conjugate or the pharmaceutically acceptable salt or solvate thereof according to claim 4 or 5, wherein A is a polypeptide residue composed of 2 to 7 amino acids selected from the group consisting of phenylalanine, glycine, valine, lysine, citrulline, serine, glutamic acid, aspartic acid, and cysteic acid.

**7.** The ligand-drug conjugate or the pharmaceutically acceptable salt or solvate thereof according to any one of claims 4-6, wherein the spacer unit G is selected from the group consisting of structures represented by formulas Va, Vb, Vc and Vd and any combinations thereof:

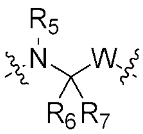
Formula Va

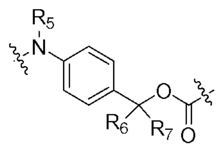
Formula Vb

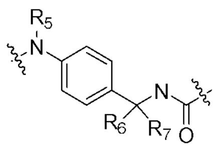
Formula Vc

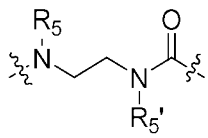
Formula Vd

wherein a wavy line on the left represents a linking site between a nitrogen atom and peptide residue A, a wavy line on the right represents a linking site between an oxygen atom and drug D, and W is an oxygen atom or a sulfur atom and is a joint group between the drug D and the spacer unit G; and $R_5$, $R_5$', $R_6$ and $R_7$ are each independently selected from the group consisting of hydrogen atom, deuterium atom, alkyl and substituted alkyl.

**8.** The ligand-drug conjugate or the pharmaceutically acceptable salt or solvate thereof according to any one of claims 1-7, wherein it has a structure represented by formula VI or formula VII:

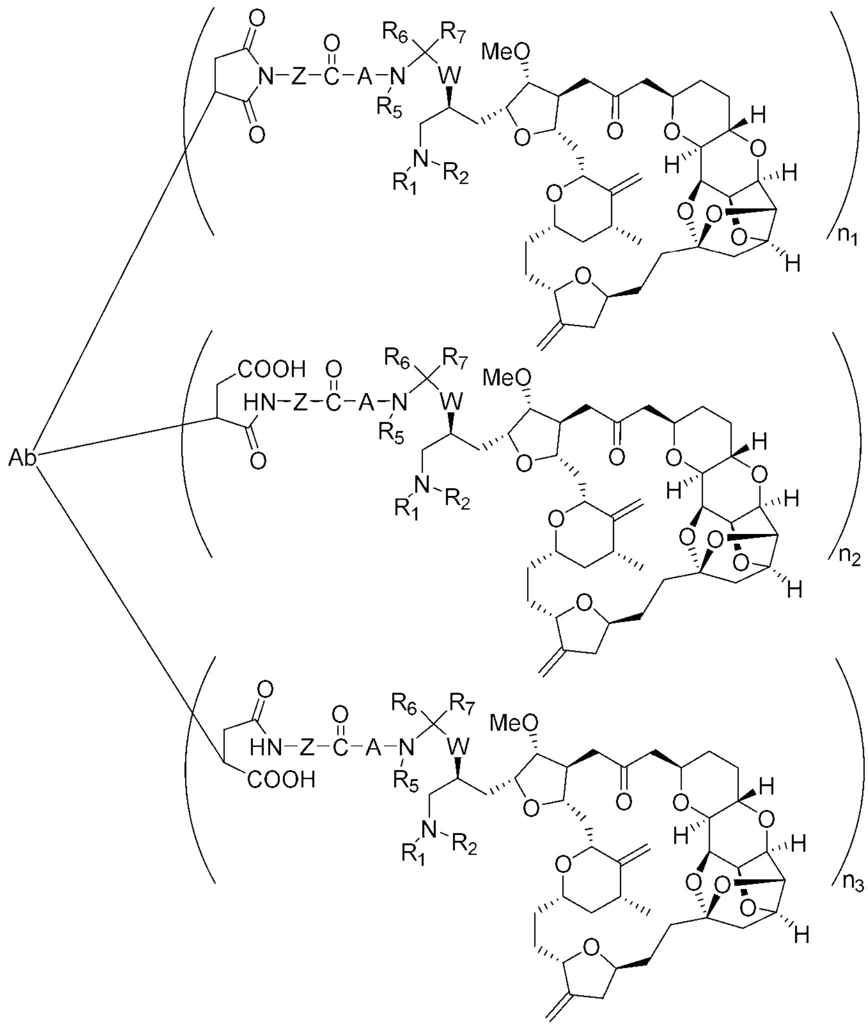

Formula VI

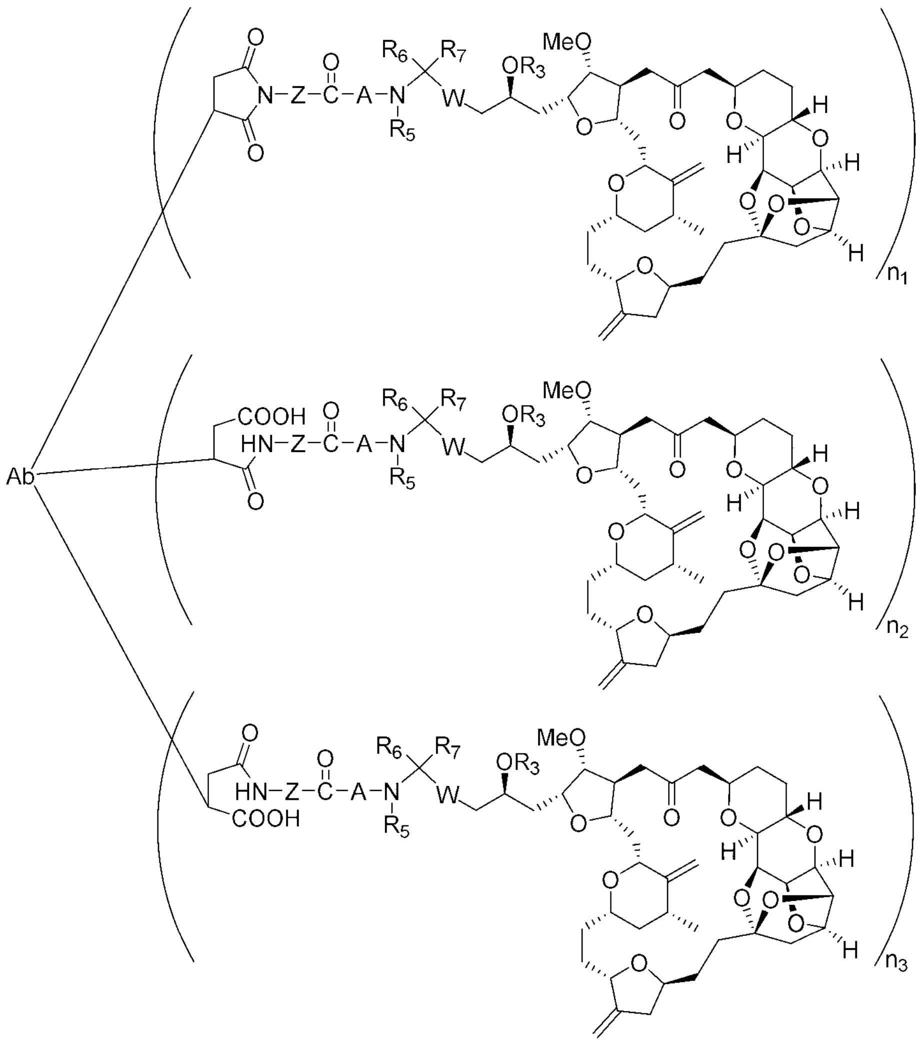

Formula VII

wherein, the Ab, Z, A, $R_5$, $R_6$, $R_7$, $R_3$ and W are as defined in any one of claims 1-7; $n_1$, $n_2$ and $n_3$ are independently selected from integers or decimals between 0 and 40, $n_1$, $n_2$ and $n_3$ are not 0 at the same time, and $n_1+n_2+n_3 \leq 40$.

**9.** The ligand-drug conjugate or the pharmaceutically acceptable salt or solvate thereof according to any one of claims 4-8, wherein Z is selected from the group consisting of -C1-C10 alkylene-, - $(CH_2CH_2O)_r$-, amide,

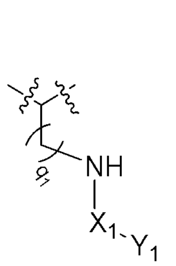 , 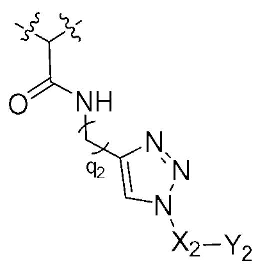 ,

and any combinations thereof; preferably, Z is -C2-alkylene or -C5-alkylene;

preferably, Z is

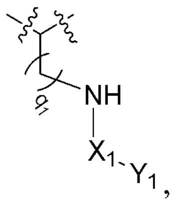

wherein, $q_1$ is selected from integers between 1 and 8; preferably, $q_1$ is 1;

preferably, $q_1$ is 1, and -NH-$X_1$-$Y_1$ composes the hydrophilic structural unit $Ac_1$, the $Ac_1$ is selected, without limitation, from the group consisting of (D/L) alanine, (D/L) leucine, (D/L) isoleucine, (D/L) valine, (D/L) phenylalanine, (D/L) proline, (D/L) tryptophan, (D/L) serine, (D/L) tyrosine, (D/L) cysteine, (D/L) cystine, (D/L) arginine, (D/L) histidine, (D/L) methionine, (D/L) asparagine, (D/L) glutamine, (D/L) threonine, (D/L) aspartic acid, (D/L) glutamic acid, natural or unnatural amino acid derivatives, and the following structures:

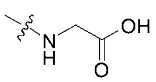 , 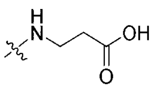 , 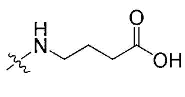 , 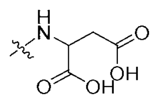 , 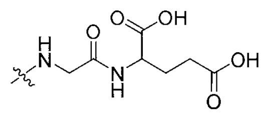 ,

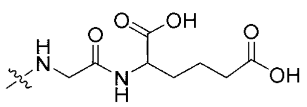 , 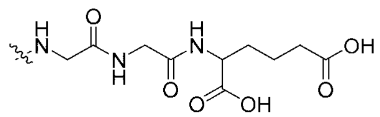 , 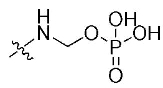 , 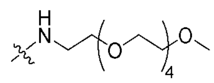

and

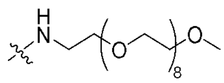 ;

preferably, $q_1$ is 1, and -NH-$X_1$-$Y_1$ composes the hydrophilic structural unit $Ac_1$, the $Ac_1$ is selected from the following structures:

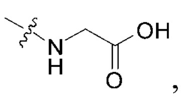 , 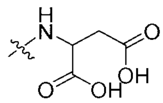 , and 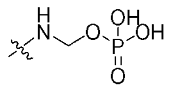 ;

preferably, Z is

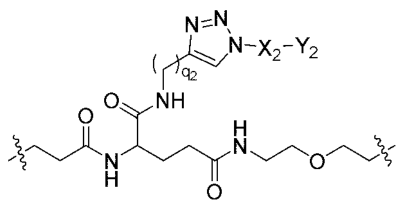 ,

wherein, $q_2$ is selected from integers between 1 and 8; preferably, $q_2$ is 1; preferably, $X_2$ is -(C1-C10 alkylene)-$(CH_2CH_2O)r$-(C=O)- or -(C1-C10 alkylene)-$(CH_2CH_2O)_r$-$NR_4$-;

preferably, $q_2$ is 1, and -$X_2$-$Y_2$ composes the hydrophilic structural unit $Ac_2$, the $Ac_2$ is selected, without limitation, from

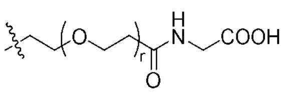 , 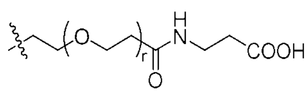 ,

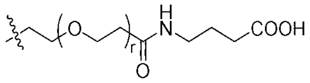 , 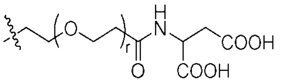 , 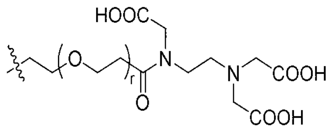 ,

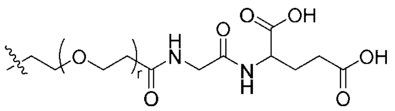 , 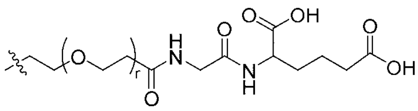 ,

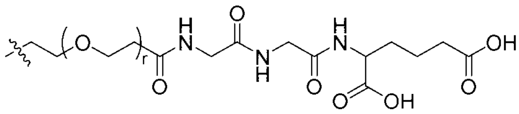 , 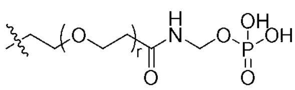 , 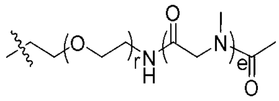

and

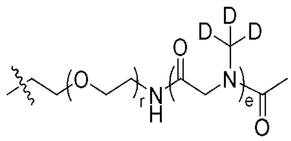 ;

r is selected from integers between 1 and 10 ; e is selected from integers between 1 and 20;
preferably, $q_2$ is 1, and $-X_2-Y_2$ composes the hydrophilic structural unit $Ac_2$, the $Ac_2$ is selected from the following structures:

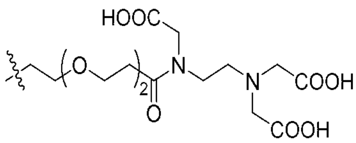 , 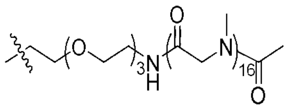

and

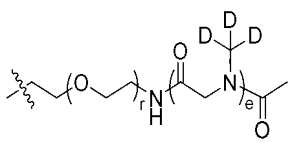 .

**10.** The ligand-drug conjugate or the pharmaceutically acceptable salt or solvate thereof according to any one of claims 4-9, wherein A is a polypeptide residue composed of 2 to 5 amino acids selected from the group consisting of phenylalanine, glycine, valine, lysine, citrulline, serine, glutamic acid, aspartic acid, and cysteic acid;

preferably, A is a peptide residue formed by 2 to 4 amino acids selected from phenylalanine and glycine;
more preferably, A is a tetrapeptide residue composed of glycine (G), glycine (G), phenylalanine (F), and glycine (G);
and more preferably, A is -GGFG-.

**11.** The ligand-drug conjugate or the pharmaceutically acceptable salt or solvate thereof according to any one of claims 1-10, wherein W is an oxygen atom or a sulfur atom, $R_1$ and $R_2$ are the same or different, and are each independently selected from the group consisting of: hydrogen atom, alkyl, alkoxy, alkenyl, cycloalkyl, $-C(O)-Q_1-Q_2$ and $-SO_2-Q_1-Q_2$, wherein $Q_1$ is selected from the group consisting of O and a chemical bond, and $Q_2$ is selected from the group consisting of alkyl, heterocyclyl, alkenyl, aryl, heteroaryl and cycloalkyl; optionally, the alkyl, heterocyclyl, alkenyl, aryl, heteroaryl and cycloalkyl are each independently substituted with one or more substituents selected from the group consisting of hydrogen atom, alkyl, hydroxyalkyl, halogen, deuterium, mercapto, amino, aryl and hydroxy; or $R_1$ and $R_2$ together with a nitrogen atom connected thereto, form a 3- to 6-membered heterocyclyl, and optionally the

heterocyclyl is substituted with one or more substituents selected from the group consisting of hydrogen atom, alkyl, alkoxy, halogen, deuterium, amino, hydroxyl, cycloalkyl and heterocyclyl; $R_3$ is selected from the group consisting of hydrogen atom and alkyl, and optionally the alkyl is substituted with one or more substituents selected from the group consisting of hydrogen atom, alkyl, alkoxy, halogen, deuterium, amino, hydroxyl, cycloalkyl and heterocyclyl; preferably, W is an oxygen atom, $R_1$ and $R_2$ are the same or different, and are each independently selected from the group consisting of: hydrogen atom, alkyl, alkenyl, haloalkyl, -C(O)-$Q_1$-$Q_2$ and -$SO_2$-$Q_1$-$Q_2$, wherein $Q_1$ is selected from the group consisting of O and a chemical bond, $Q_2$ is selected from the group consisting of alkyl, alkenyl, aryl and 3- to 8-membered cycloalkyl, and optionally, the alkyl, alkenyl, aryl and cycloalkyl are each independently substituted with one or more substituents selected from the group consisting of hydrogen atom, alkyl, hydroxyalkyl, halogen, aryl, and hydroxyl; or $R_1$ and $R_2$ together with a nitrogen atom connected thereto, form a 3- to 6-membered heterocyclyl containing one to two nitrogen atoms and optionally one oxygen atom; optionally, the 3- to 6-membered heterocyclyl is substituted with one or more substituents selected from the group consisting of hydrogen atom, alkyl, and haloalkyl; $R_3$ is selected from the group consisting of hydrogen atom and alkyl, and the alkyl is substituted with one or more substituents selected from the group consisting of hydrogen atom, alkyl, halogen, deuterium, amino and hydroxyl.

**12.** The ligand-drug conjugate or the pharmaceutically acceptable salt or solvate thereof according to any one of claims 1-11, wherein W is an oxygen atom, $R_1$ and $R_2$ are the same or different, and are each independently selected from the group consisting of: hydrogen atom, $C_1$-$C_4$ alkyl (such as methyl, ethyl), $C_2$-$C_4$ alkenyl (such as allyl), $C_1$-$C_4$ haloalkyl (such as difluoroethyl) and the following structures:

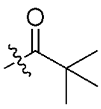 , 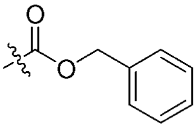 , 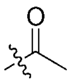 , 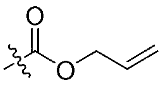 , 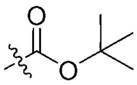 , 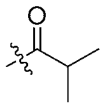 , 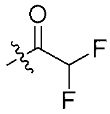 , 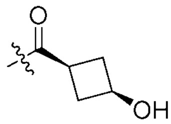 ,

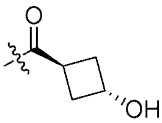 , 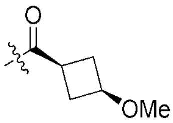 , 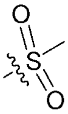 , 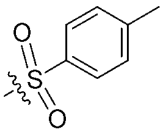 , 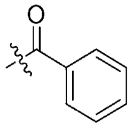 , 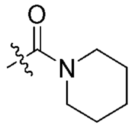 , 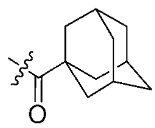 ,

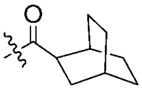 , 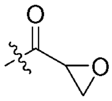 , 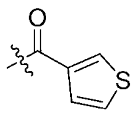 , 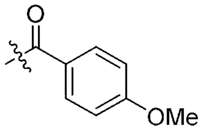 , 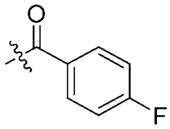 , 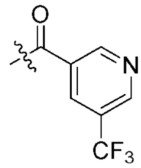 ,

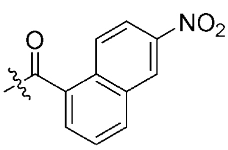 , 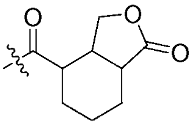 , 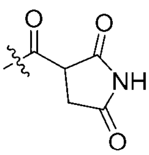 , 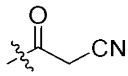 , 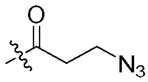 , 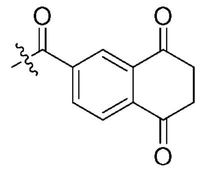 ,

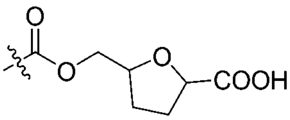 , 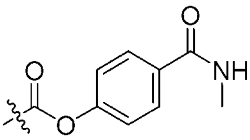 , 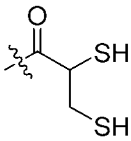 , and 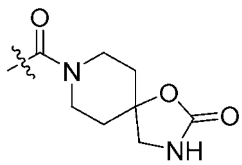 ;

or $R_1$ and $R_2$, together with a nitrogen atom connected thereto, form a 3- to 6-membered heterocyclyl containing one to two nitrogen atoms and optionally one oxygen atom (such as azyclopropyl, piperidinyl, piperazinyl, morpholinyl); optionally, the 3- to 6-membered heterocyclyl is substituted with one or more substituents selected from the group consisting of hydrogen atom, $C_1$-$C_4$ alkyl (e.g., methyl), and $C_1$-$C_4$ haloalkyl (e.g., trifluoromethyl); and $R_3$ is selected from the group consisting of hydrogen atom and methyl.

**13.** The ligand-drug conjugate or the pharmaceutically acceptable salt or solvate thereof according to any one of claims 7-12, wherein $R_5$, $R_6$ and $R_7$ are each independently selected from the group consisting of hydrogen atom, deuterium atom, alkyl, haloalkyl, deuterated alkyl and hydroxyalkyl; and
preferably, $R_5$, $R_6$ and $R_7$ are simultaneously hydrogen atoms.

**14.** The ligand-drug conjugate or the pharmaceutically acceptable salt or solvate thereof according to any one of claims 1-13, wherein it has a structure represented by formula VIa or formula VIIa:

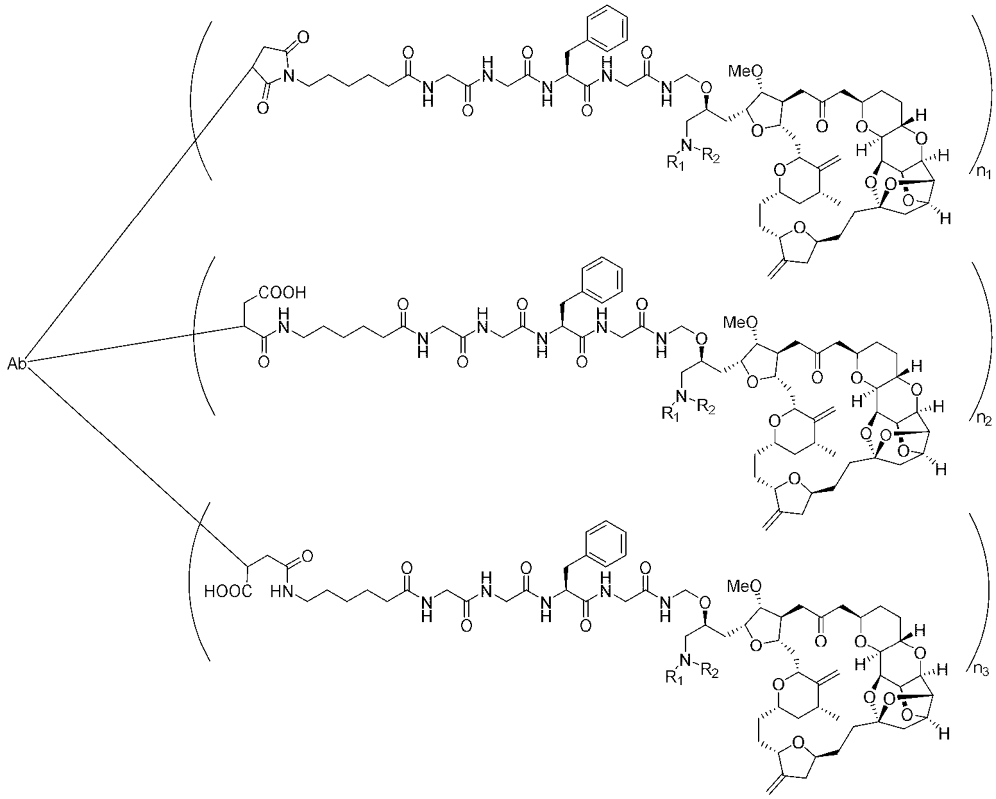

Formula VIa

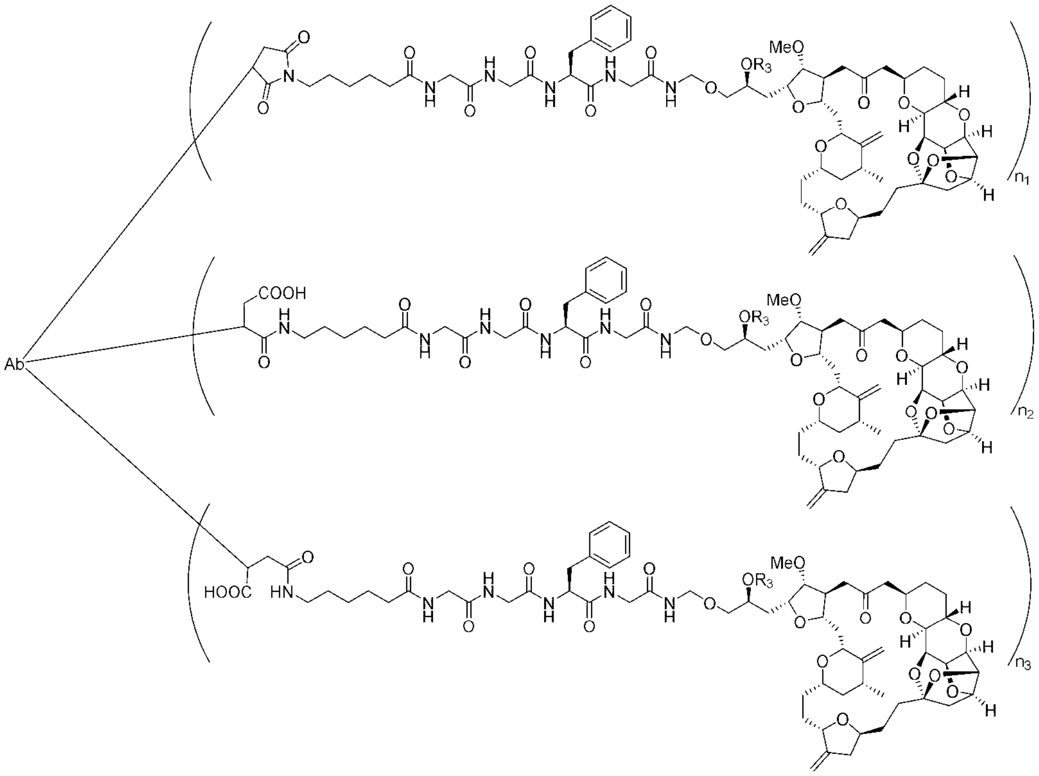

Formula VIIa

wherein,
the $R_1$, $R_2$, $R_3$, $n_1$, $n_2$ and $n_3$ are as defined in any one of claims 1-13.

**15.** The ligand-drug conjugate or the pharmaceutically acceptable salt or solvate thereof according to any one of claims 1-13, wherein it has a structure represented by formula VIb or formula VIIb:

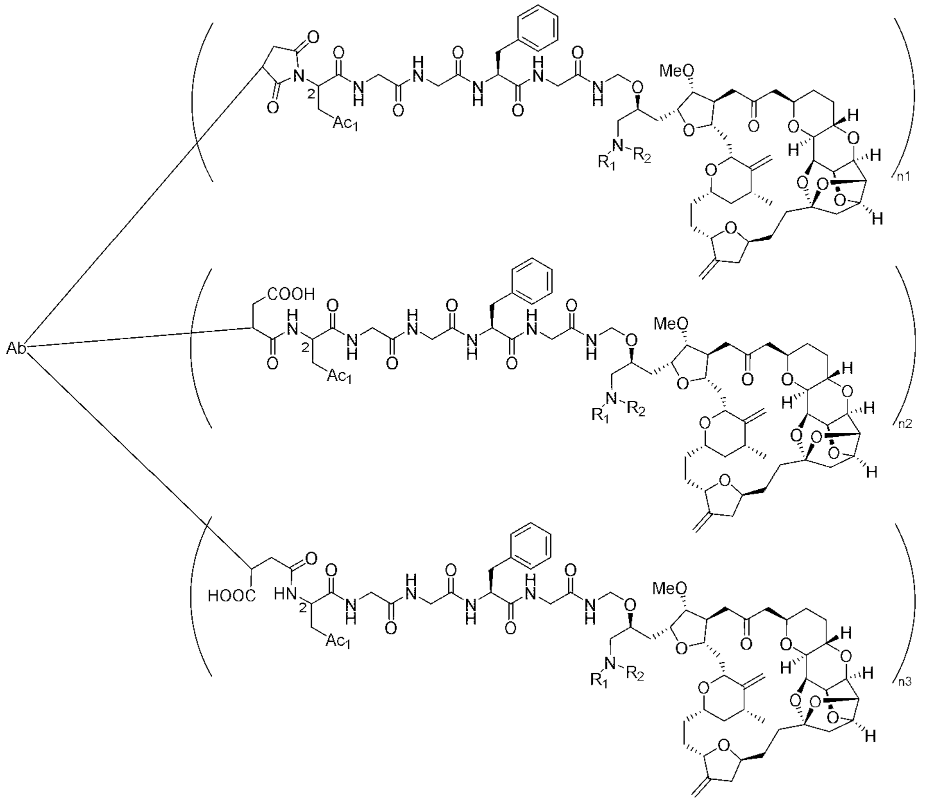

Formula VIb

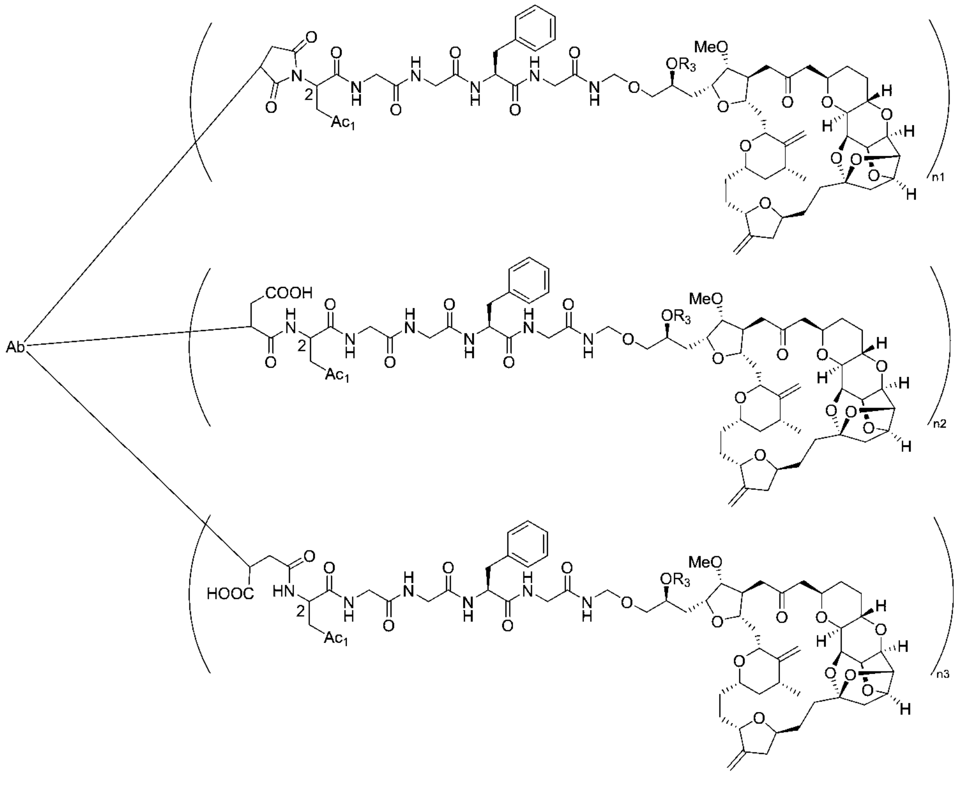

Formula VIIb

wherein,

the $R_1$, $R_2$, $R_3$, $n_1$, $n_2$ and $n_3$ are defined as in any one of claims 1-13;
$Ac_1$ is a hydrophilic structural unit having the structure shown in formula d:

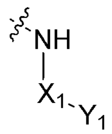

Formula d

the $X_1$ and $Y_1$ are as defined in claim 4;
preferably, the $Ac_1$ is selected, without limitation, from the group consisting of (D/L) alanine, (D/L) leucine, (D/L) isoleucine, (D/L) valine, (D/L) phenylalanine, (D/L) proline, (D/L) tryptophan, (D/L) serine, (D/L) tyrosine, (D/L) cysteine, (D/L) cystine, (D/L) arginine, (D/L) histidine, (D/L) methionine, (D/L) asparagine, (D/L) glutamine, (D/L) threonine, (D/L) aspartic acid, (D/L) glutamic acid, natural or unnatural amino acid derivatives and the following structures:

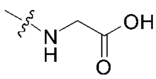 , 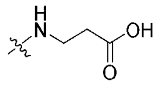 , 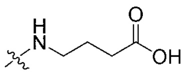 , 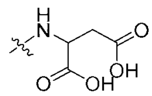 , 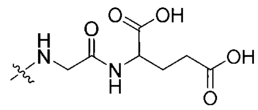 ,

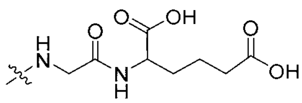 , 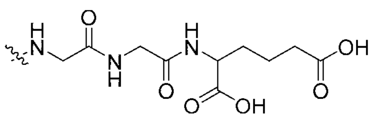 , 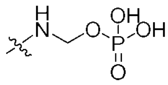 , 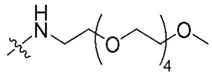

and

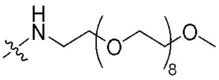 ;

preferably, $Ac_1$ is selected from the following structures:

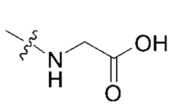 , 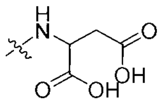 ,

and

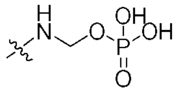 .

**16.** The ligand-drug conjugate or the pharmaceutically acceptable salt or solvate thereof according to any one of claims 1-13, wherein it has a structure represented by formula VIc:

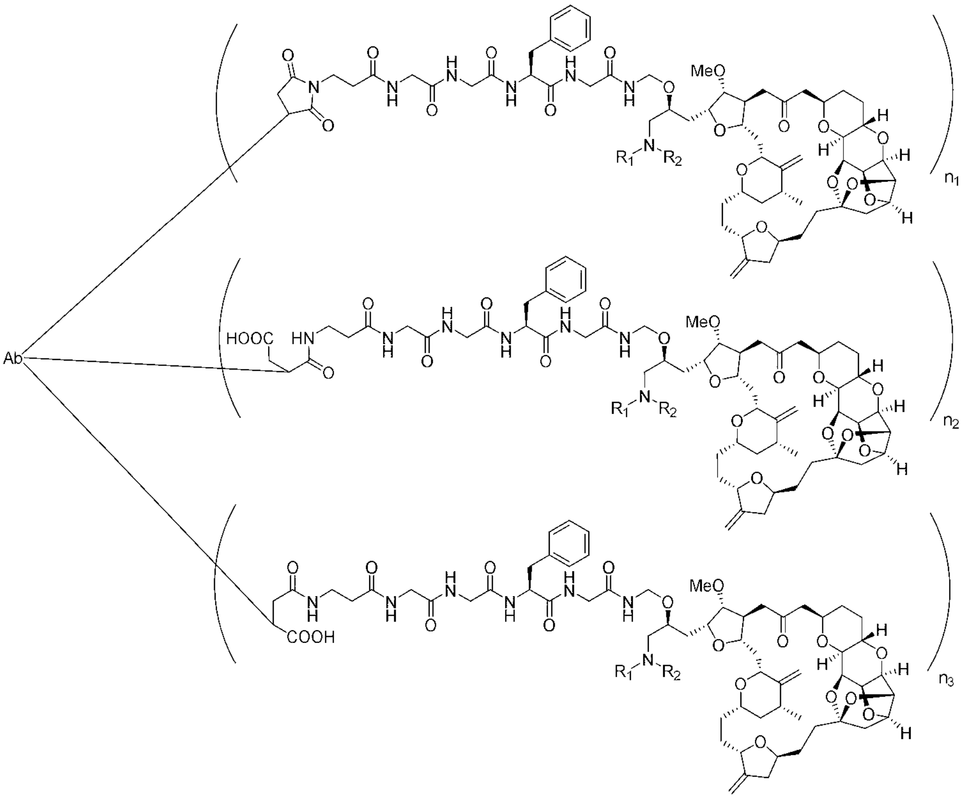

Formula VIc

wherein,
the $R_1$, $R_2$, $n_1$, $n_2$ and $n_3$ are defined in any one of claims1-13.

**17.** The ligand-drug conjugate or the pharmaceutically acceptable salt or solvate thereof according to any one of claims 1-13, wherein it has a structure represented by formula VId:

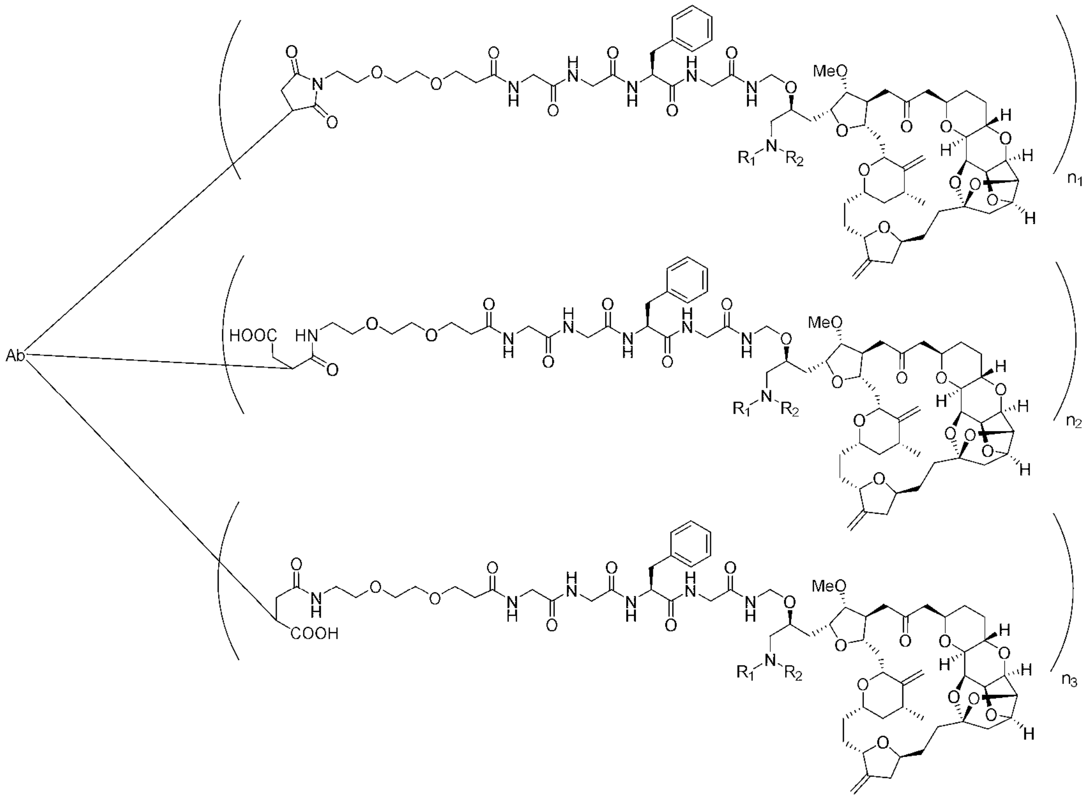

Formula VId

wherein,
the $R_1$, $R_2$, $n_1$, $n_2$ and $n_3$ are as defined in any one of claims 1-13.

**18.** The ligand-drug conjugate or the pharmaceutically acceptable salt or solvate thereof according to any one of claims 1-13, wherein it has a structure represented by formula VIe:

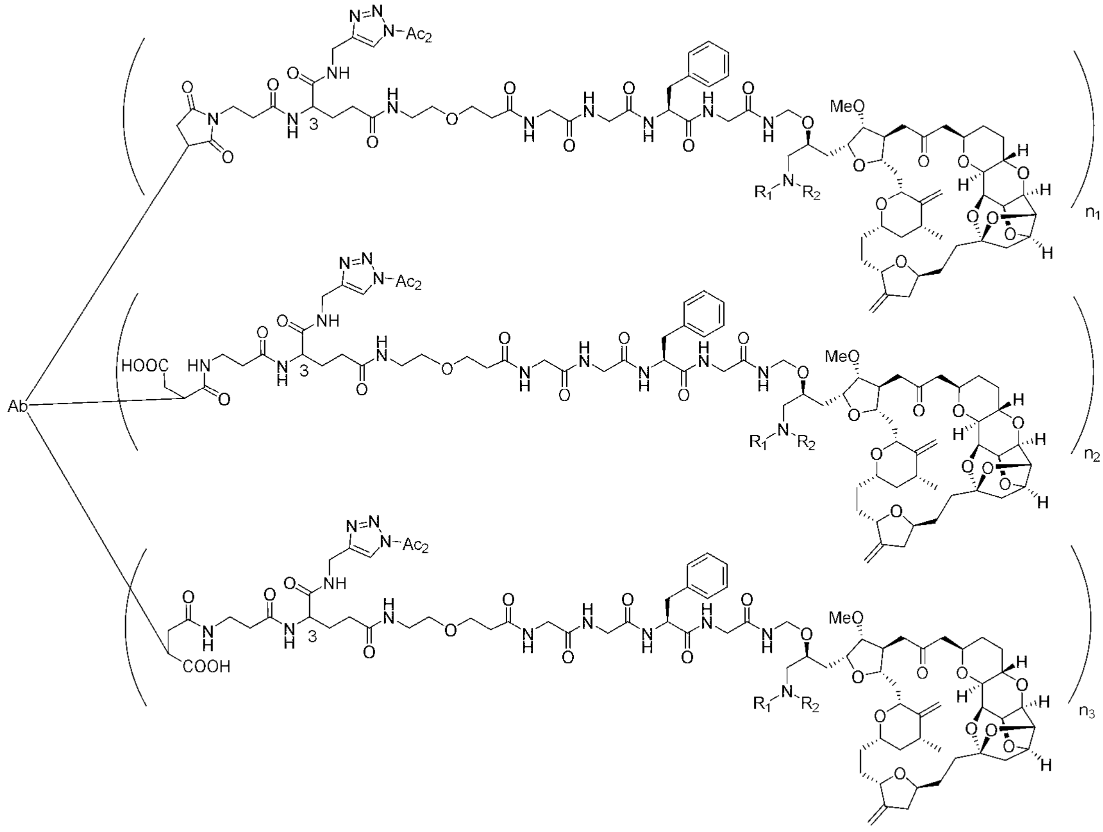

Formula VIe

wherein, the $R_1$, $R_2$, $n_1$, $n_2$ and $n_3$ are as defined in any one of claims 1-13;
$Ac_2$ is a hydrophilic structural unit composed of $-X_2-Y_2$, and $X_2$ and $Y_2$ are as defined in claim 4; preferably, the $Ac_2$ is selected, without limitation, from

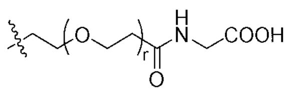

,

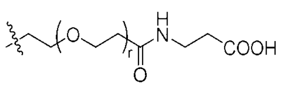

,

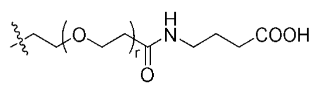

,

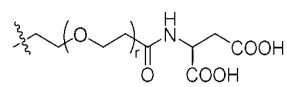

,

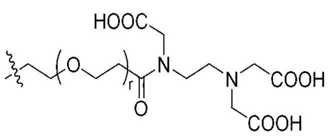

,

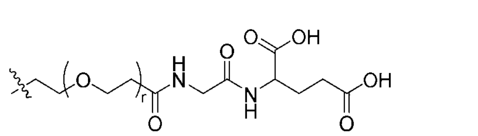

,

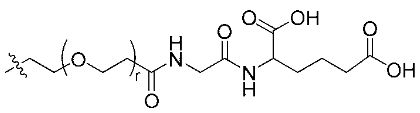

,

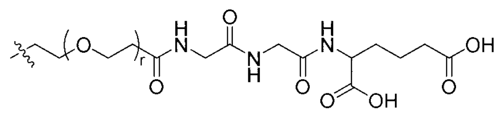

,

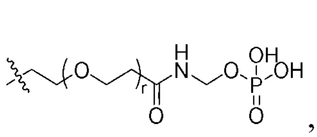

,

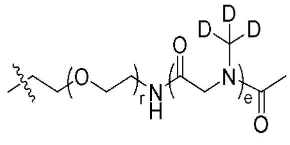

and

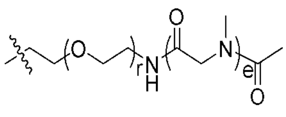

;

wherein, r is selected from integers between 1 and 10; e is selected from integers between 1 and 20; preferably, $Ac_2$ is selected from the following structures:

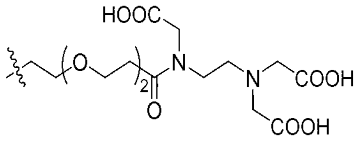

,

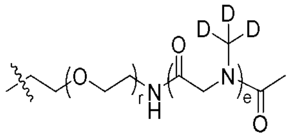

and

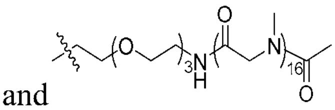

.

**19.** The ligand-drug conjugate or the pharmaceutically acceptable salt or solvate thereof according to any one of claims 1-13, wherein it has a structure represented by formula VIf:

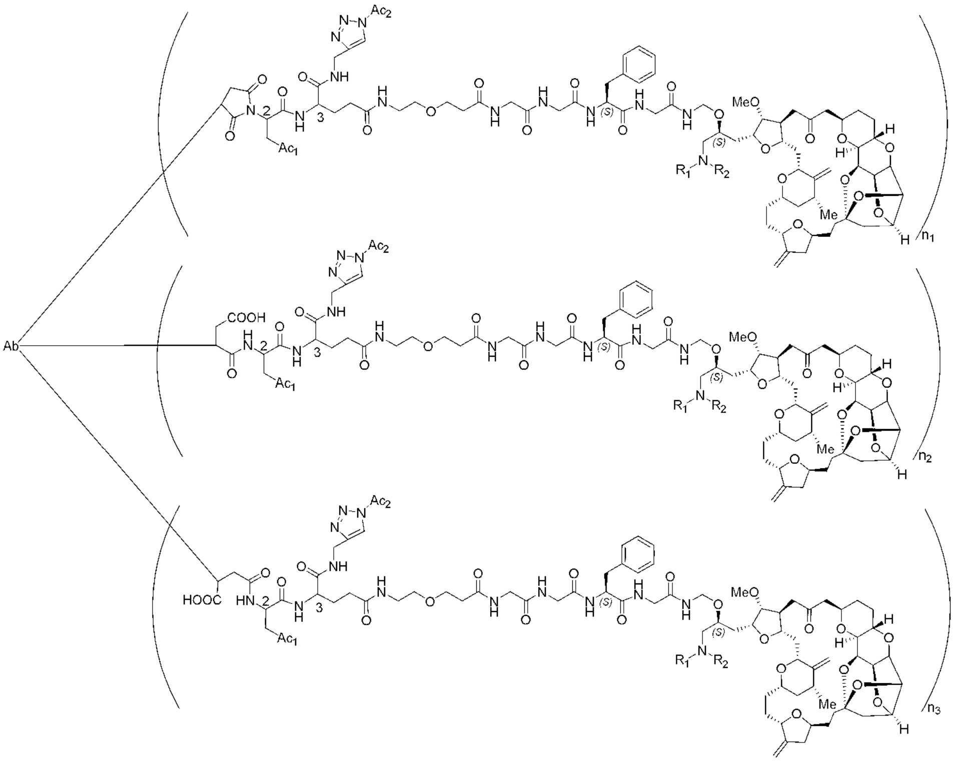

Formula VIf

wherein, the $R_1$, $R_2$, $n_1$, $n_2$ and $n_3$ are as defined in any one of claims 1-13;
$Ac_1$ is a hydrophilic structural unit having the structure shown in formula d:

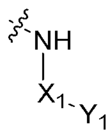

Formula d

$X_1$ and$Y_1$ are as defined in claim 4;
preferably, $Ac_1$ is as defined in claim 15;
$Ac_2$ is a hydrophilic structural unit composed of $-X_2-Y_2$, and $X_2$ and $Y_2$ are as defined in claim 4;

preferably, the $Ac_2$ is selected, without limitation, from

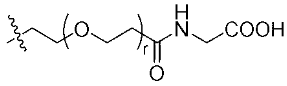,

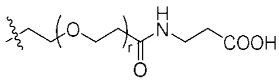, 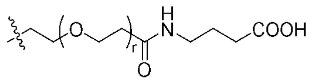, 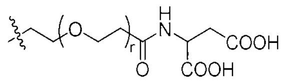,

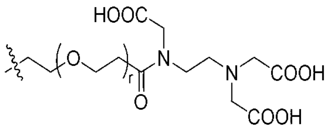, 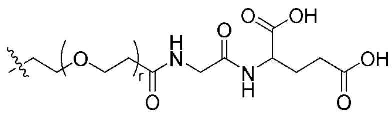,

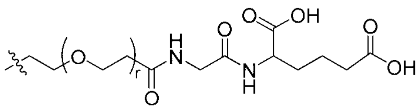, 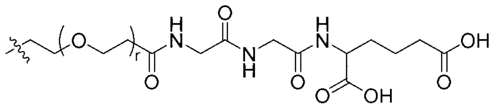,

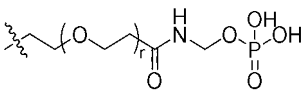, 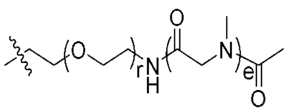 or 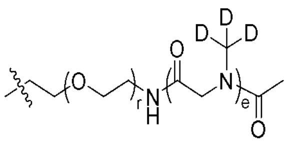;

wherein, r is selected from integers between 1 and 10; e is selected from integers between 1 and 20; and preferably, $Ac_2$ is selected from the following structures:

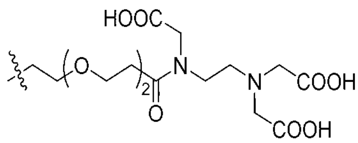,

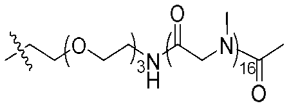 and 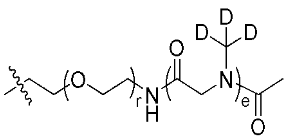.

**20.** The ligand-drug conjugate or the pharmaceutically acceptable salt or solvate thereof according to any one of claims 1-13, wherein it has a structure represented by formula VIg:

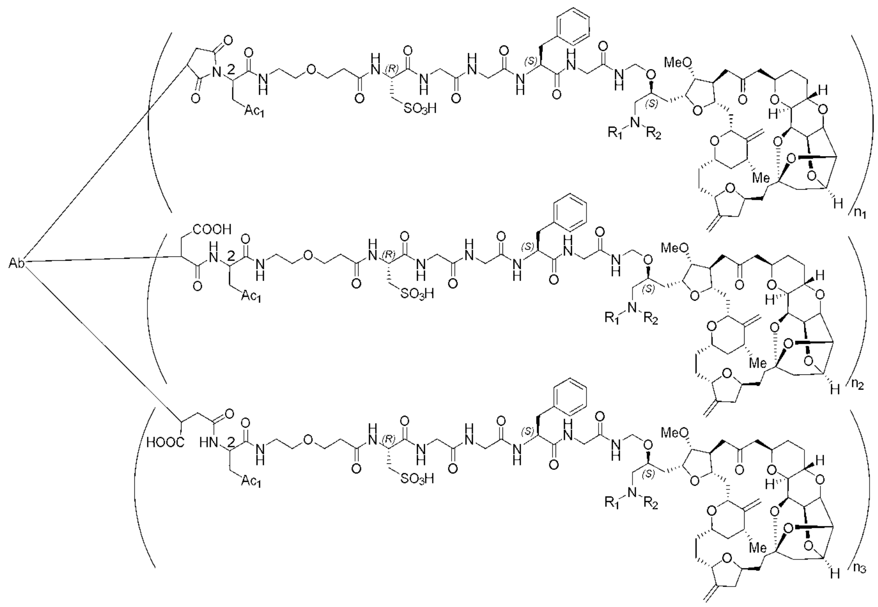

Formula VIg

wherein, the $R_1$, $R_2$, $n_1$, $n_2$ and $n_3$ are as defined in any one of claims 1-13;
$Ac_1$ is a hydrophilic structural unit having the structure shown in formula d:

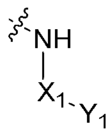

Formula d

$X_1$ and $Y_1$ are as defined in claim 4; and
preferably, $Ac_1$ is as defined in claim 15.

**21.** The ligand-drug conjugate or the pharmaceutically acceptable salt or solvate thereof according to any one of claims 1-20, wherein it is selected from the following structures:

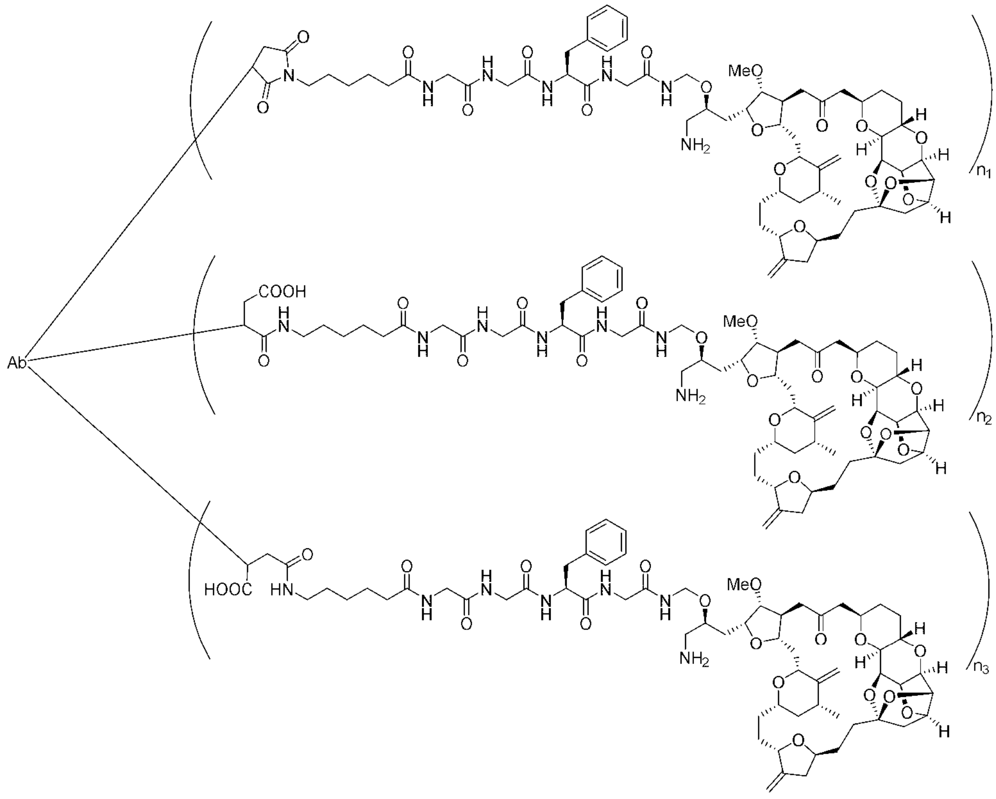

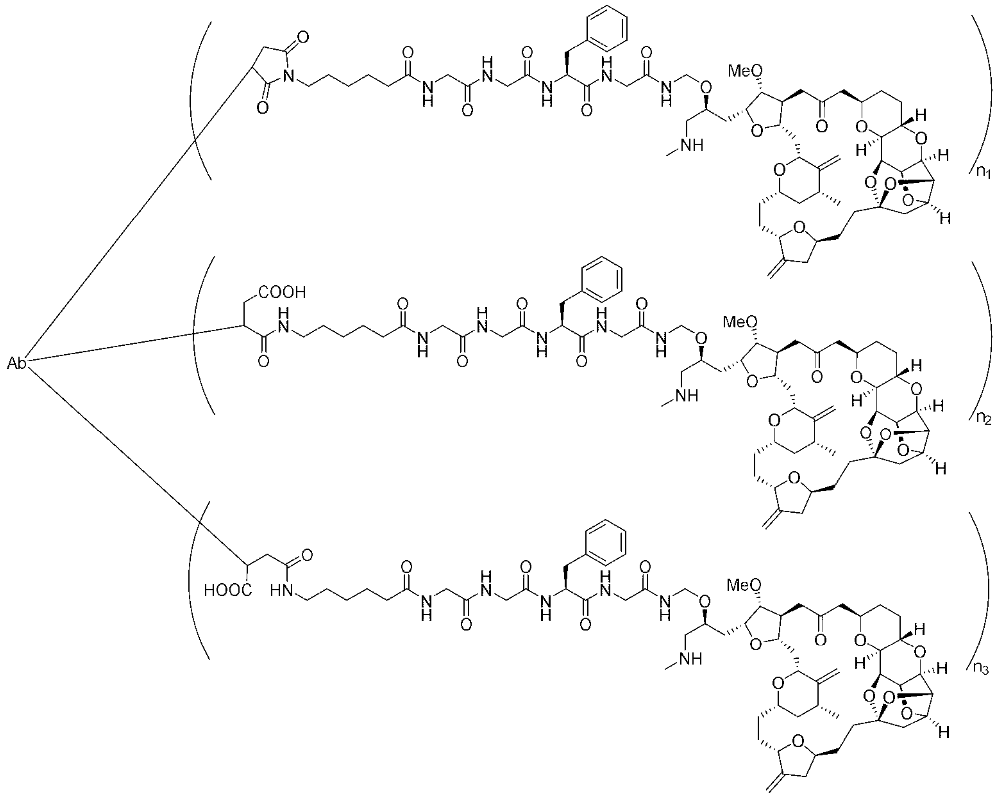

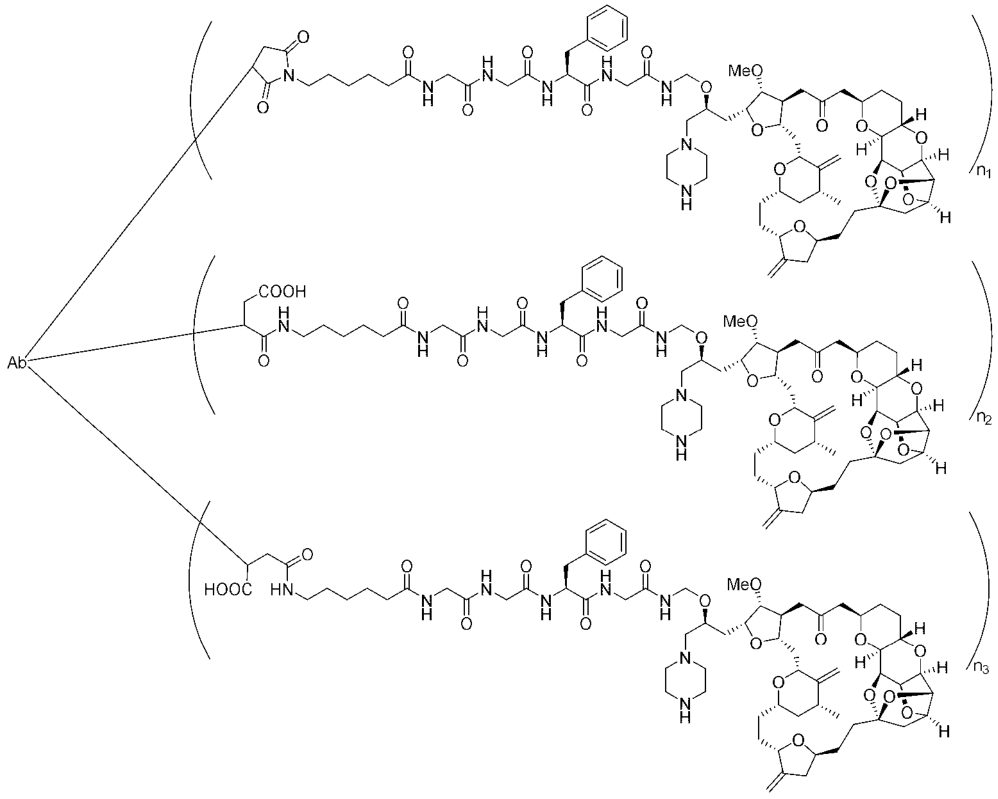

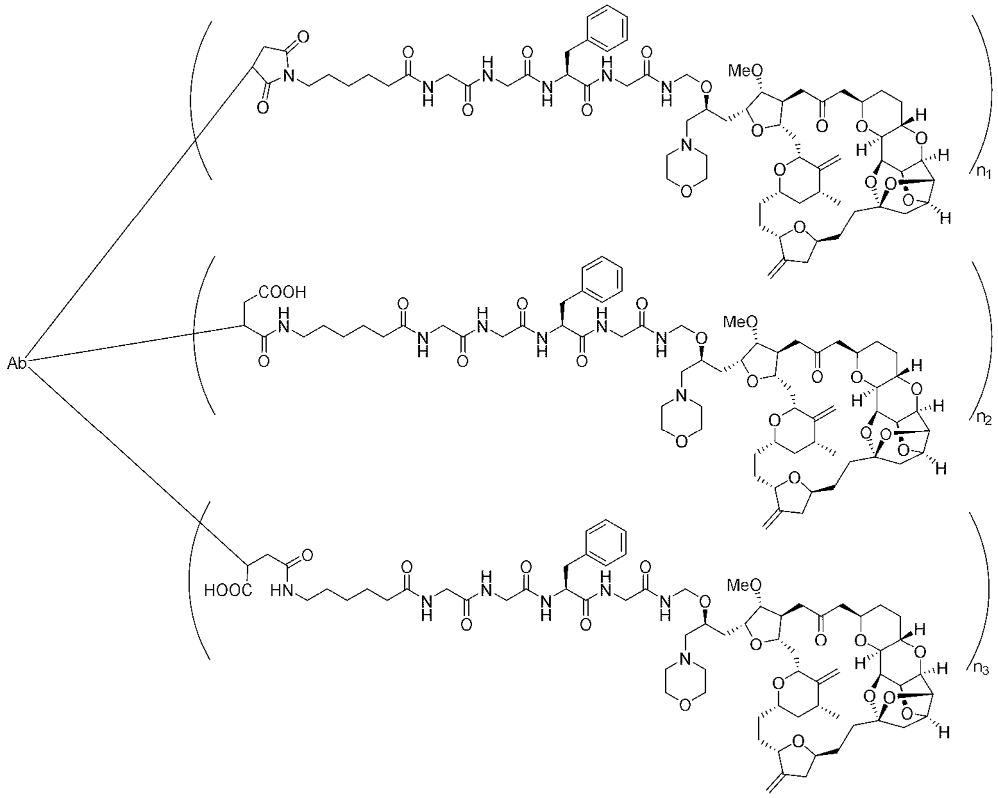

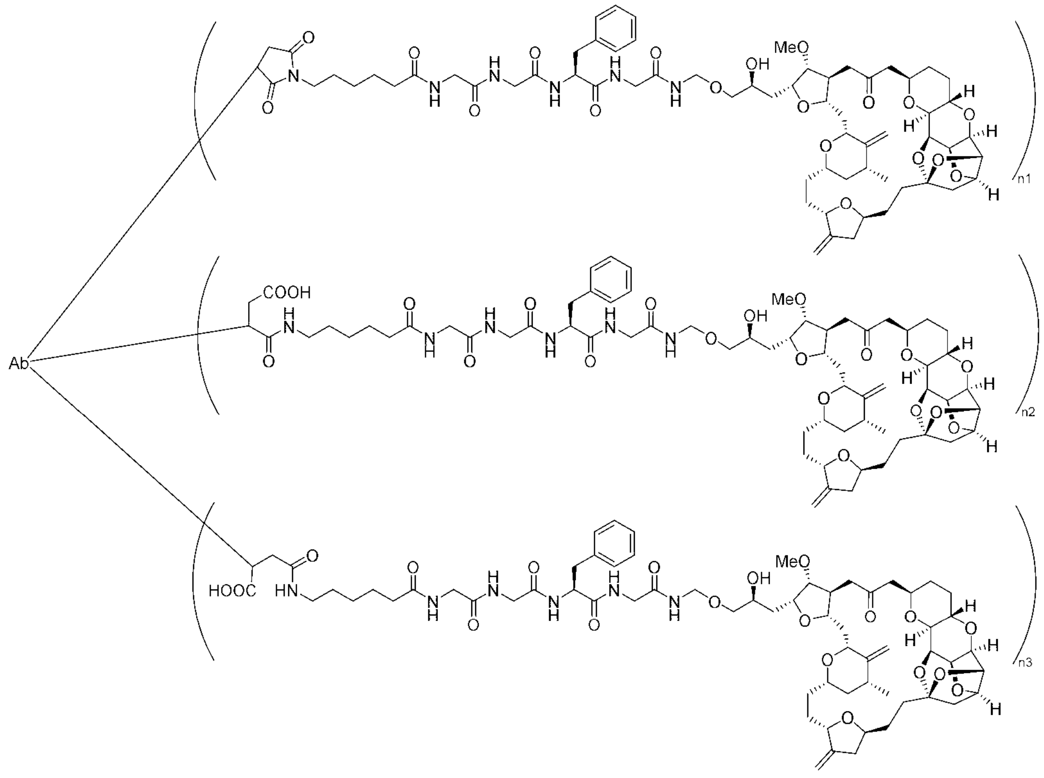

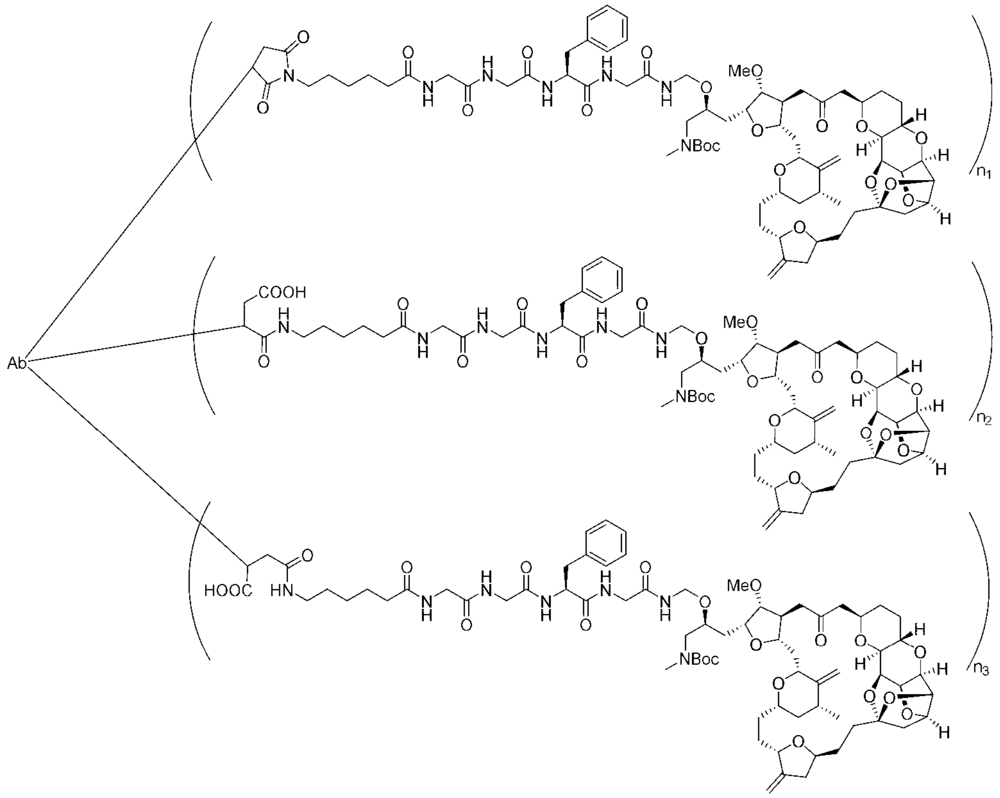

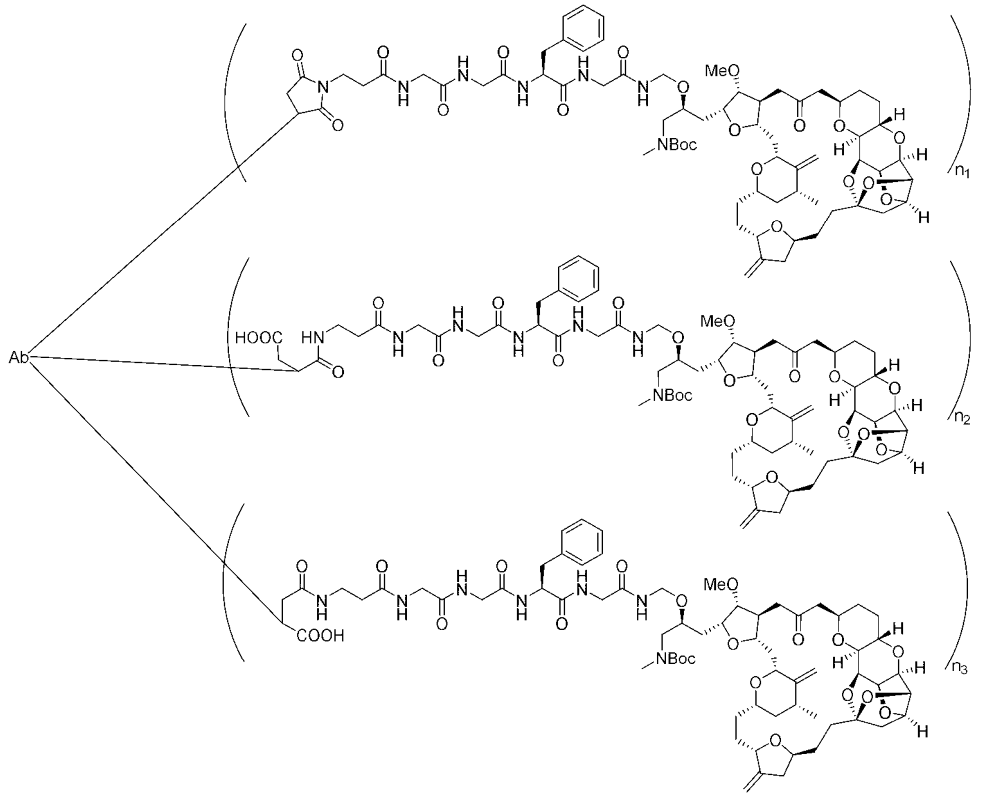

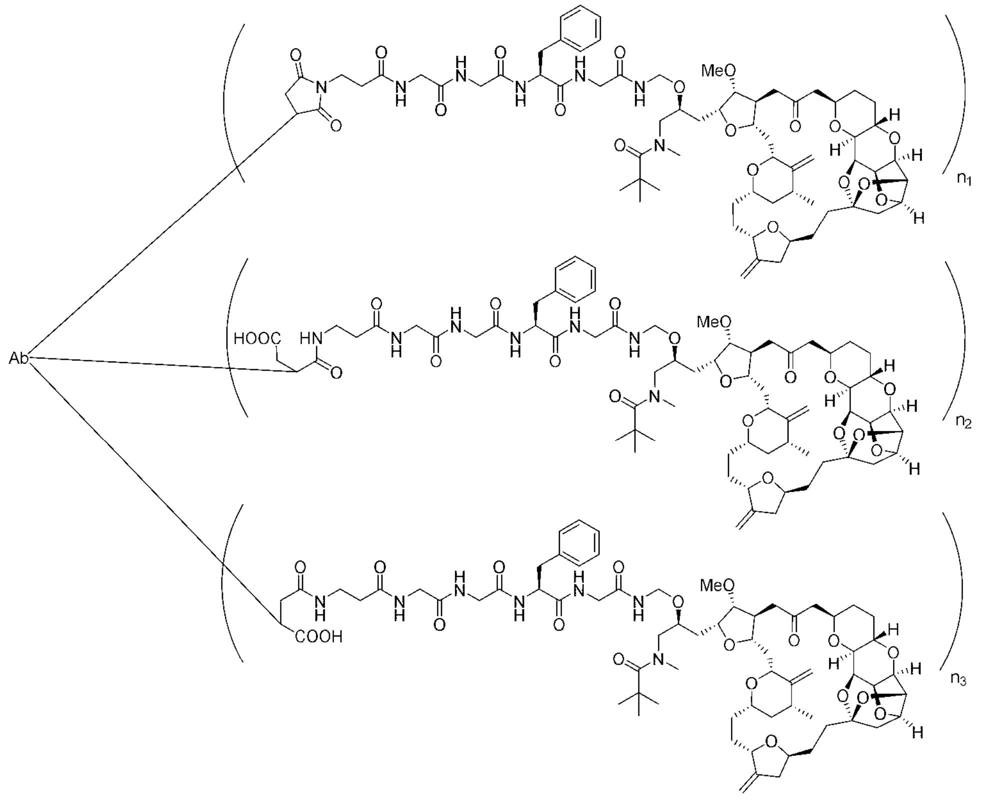

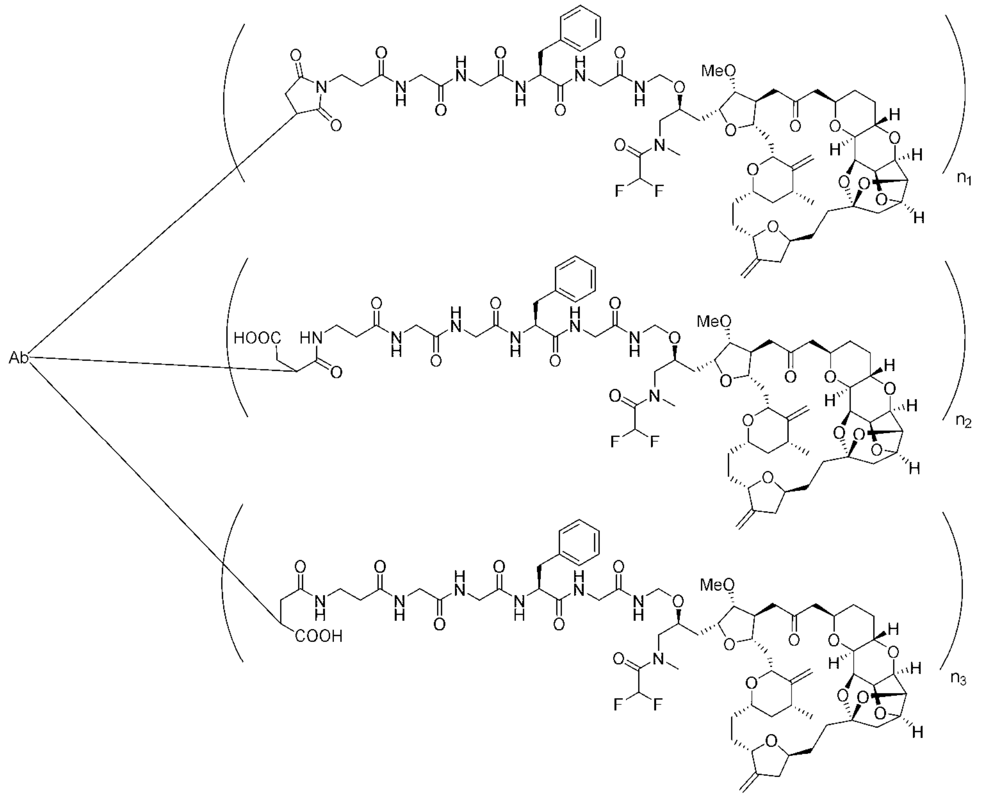

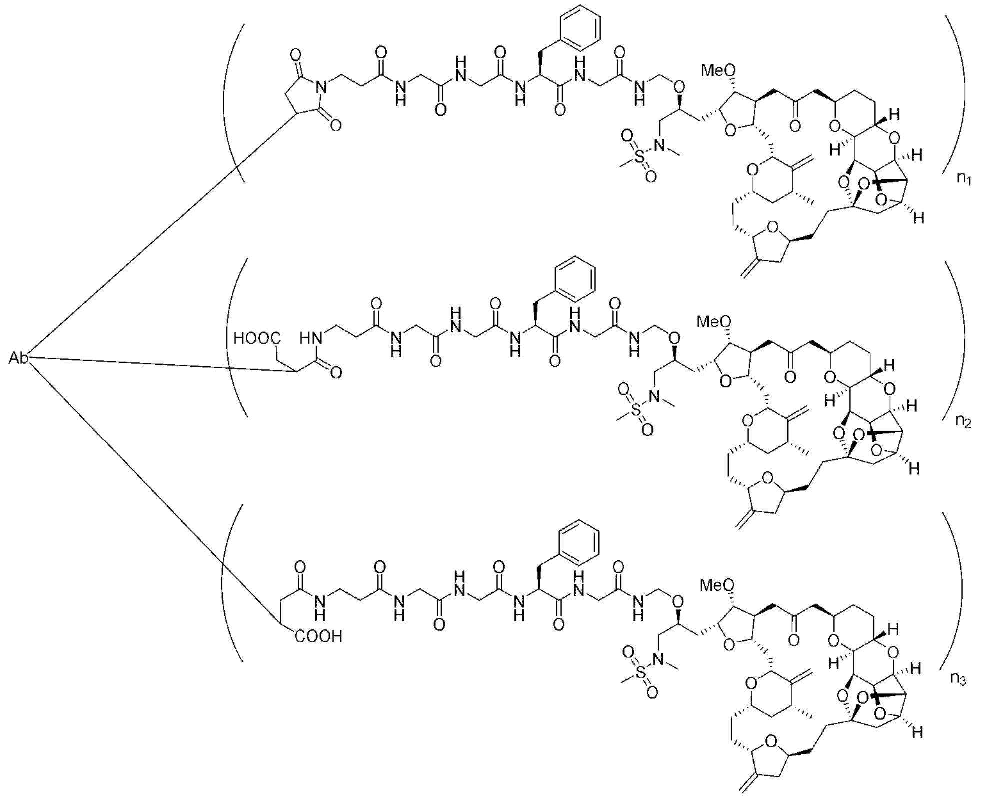

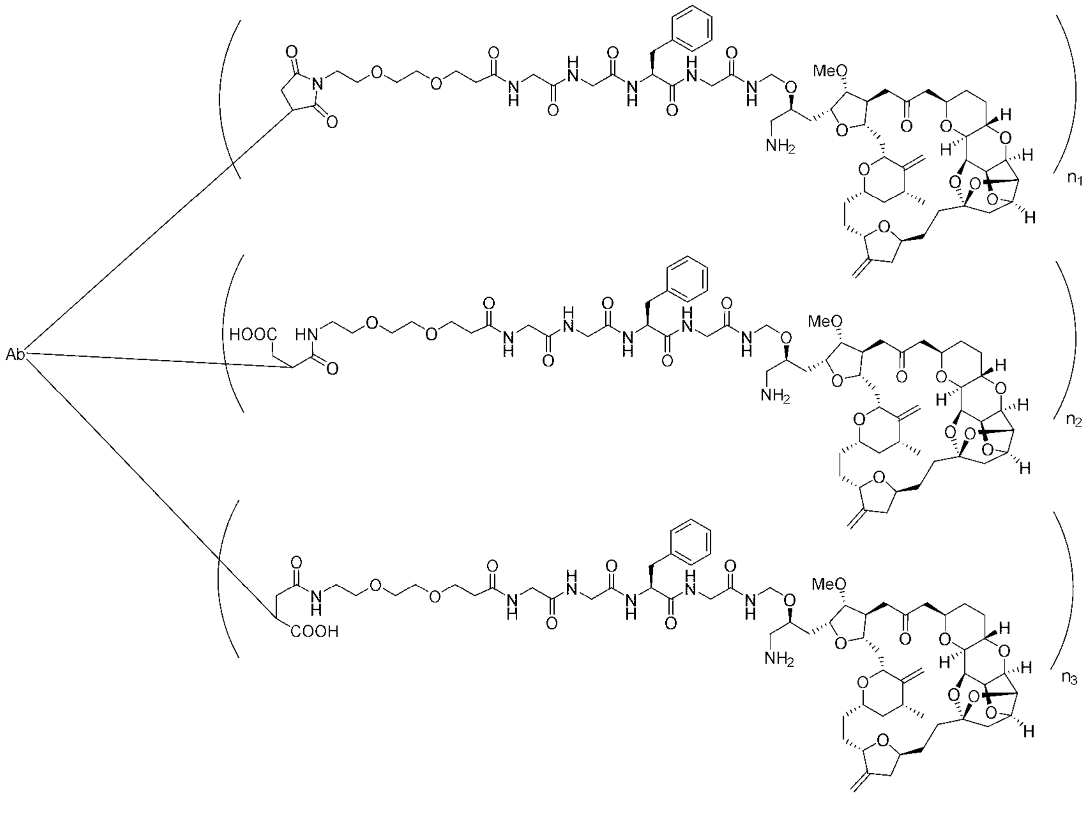

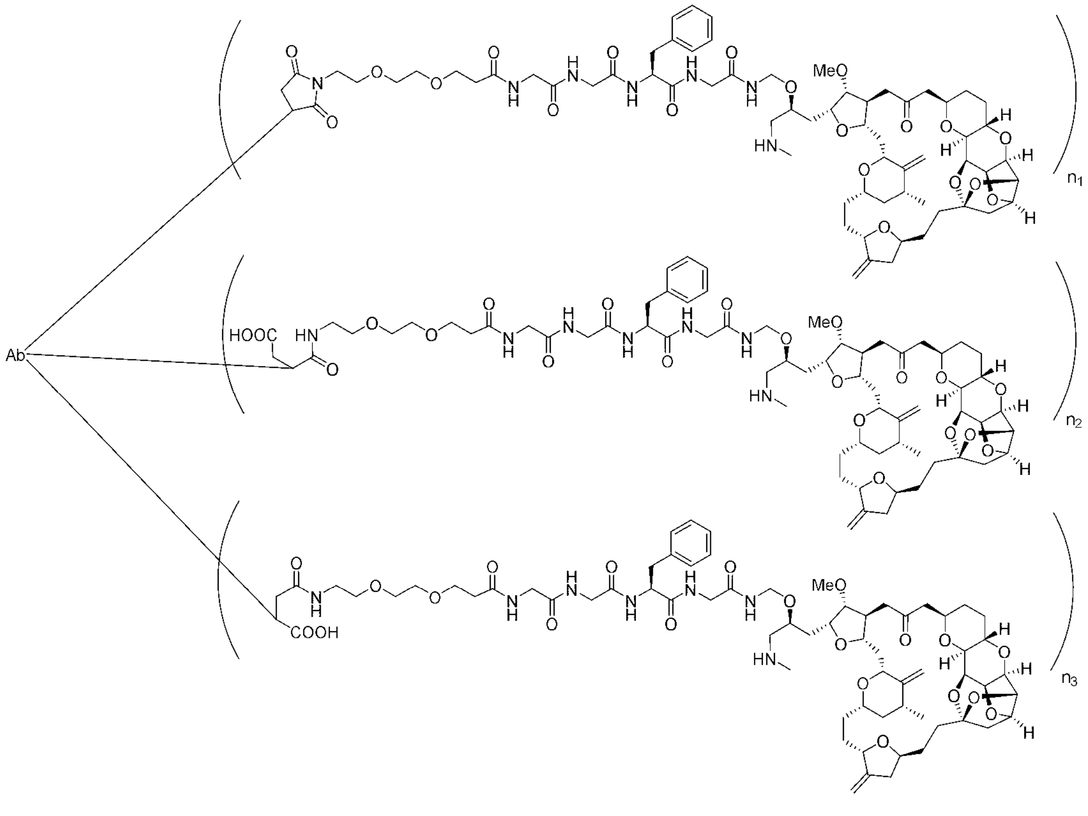

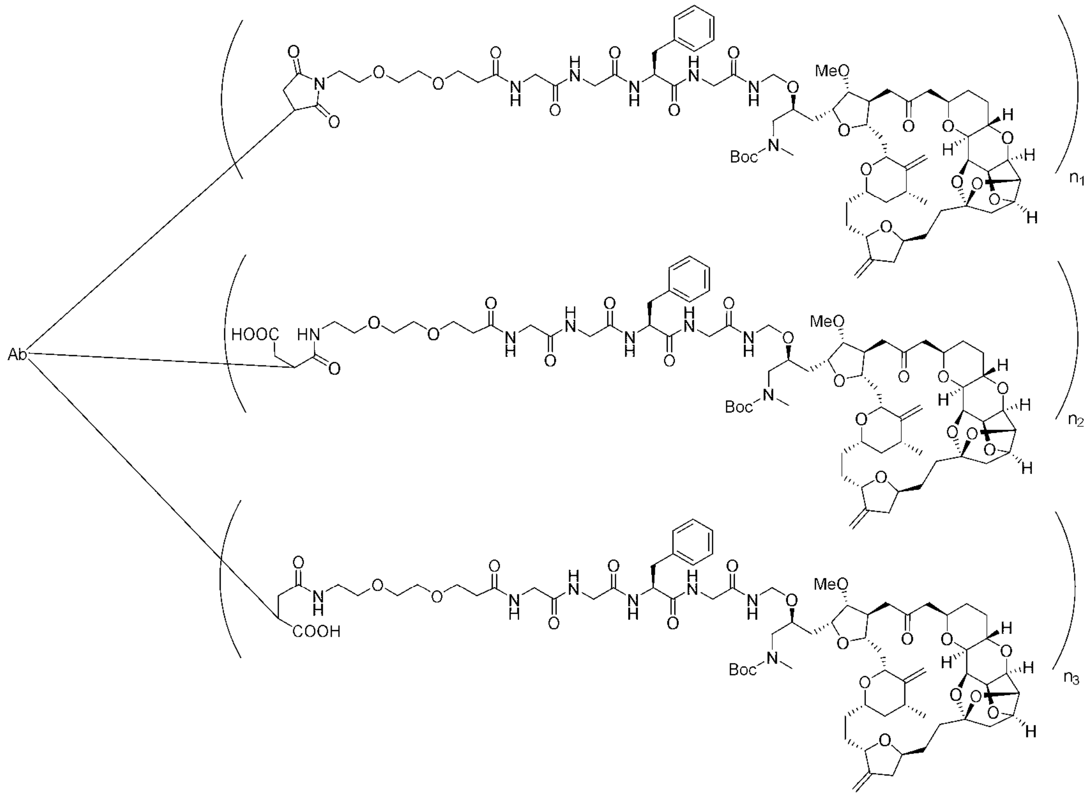

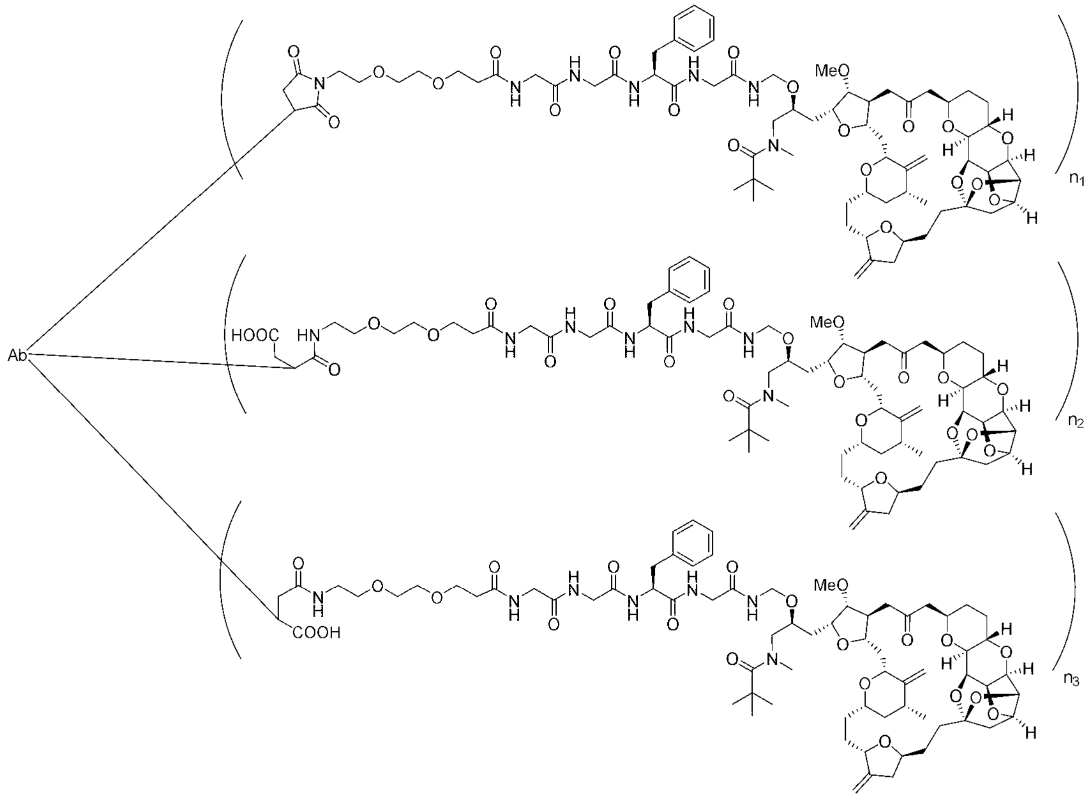

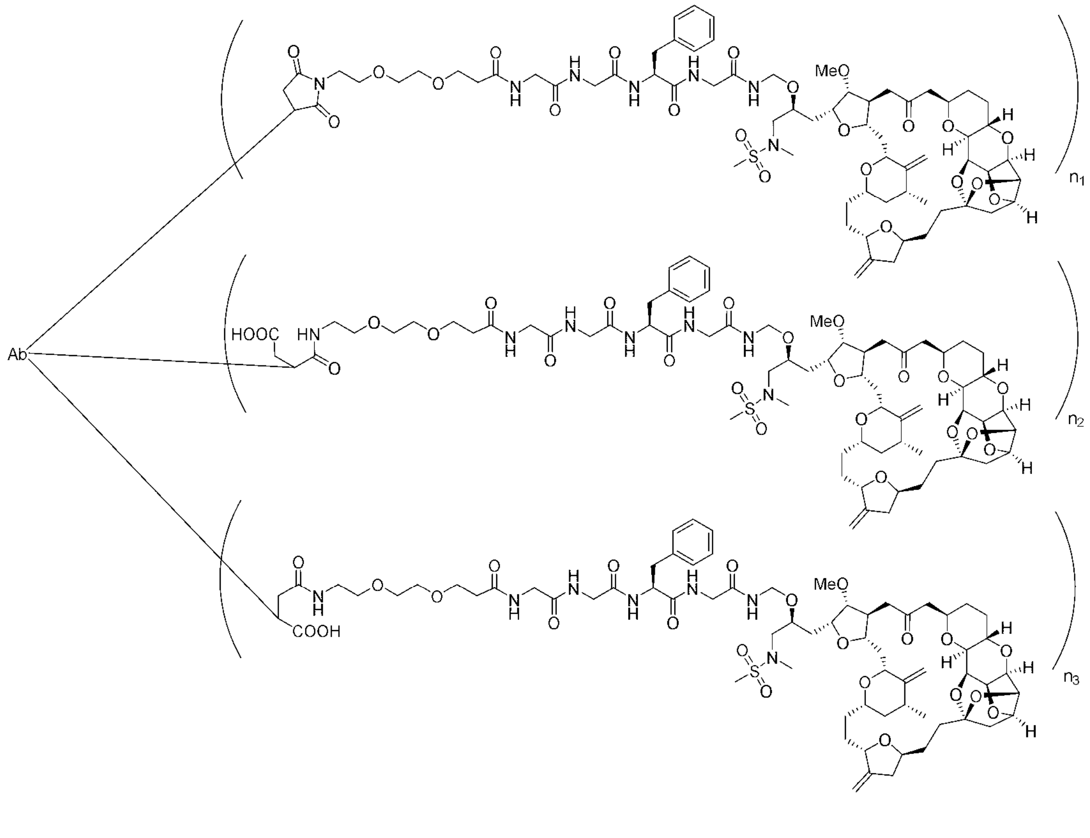

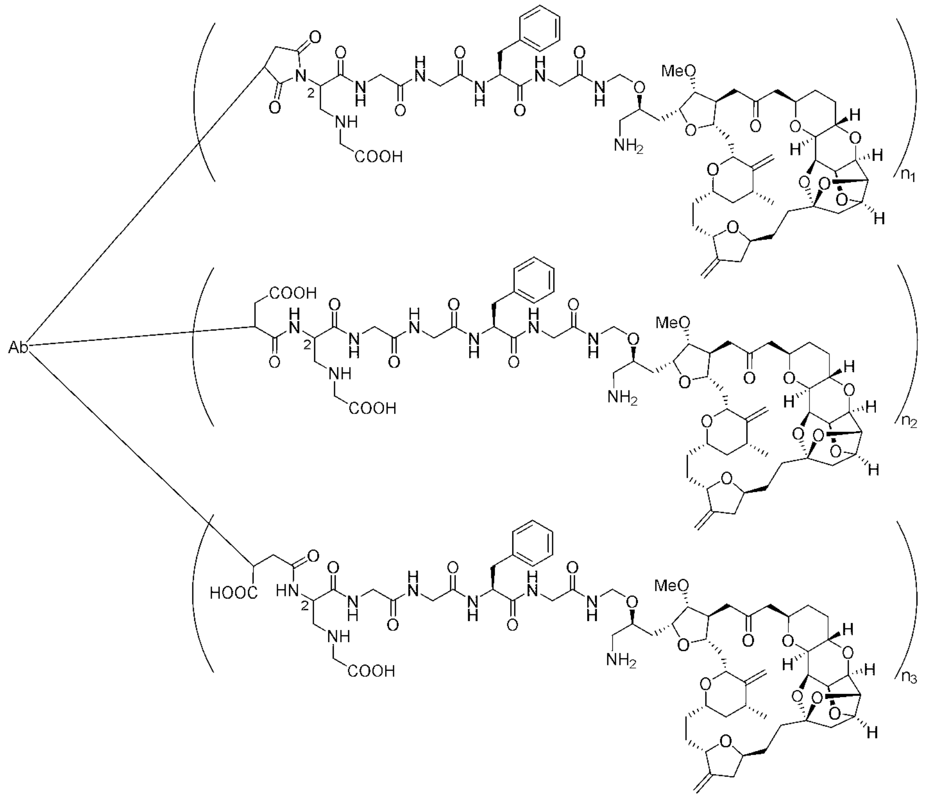

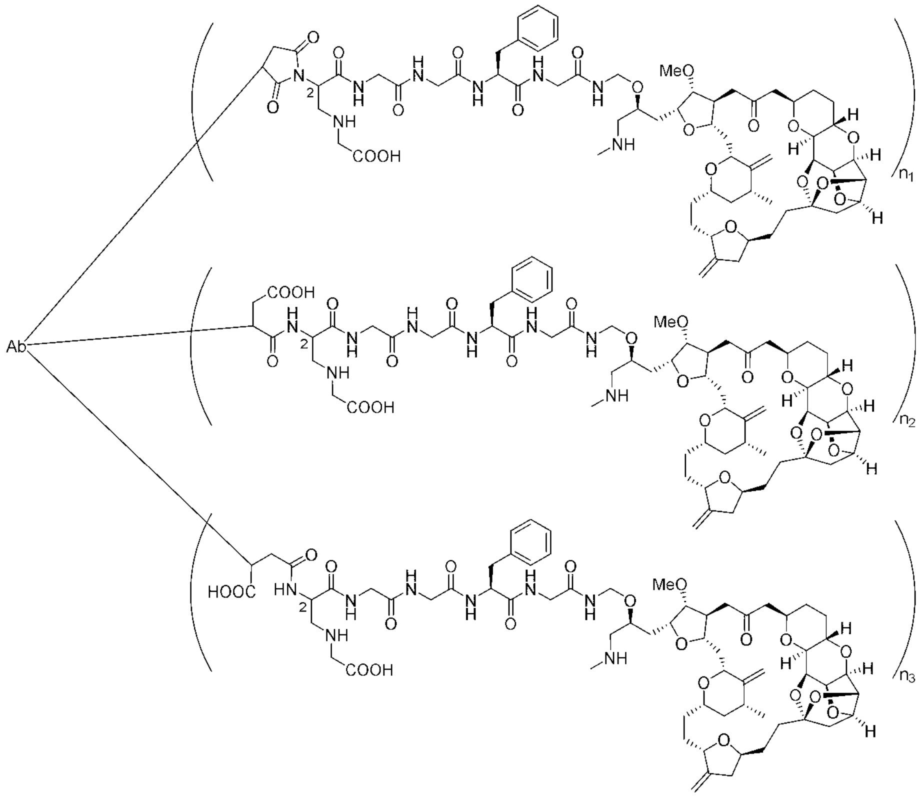

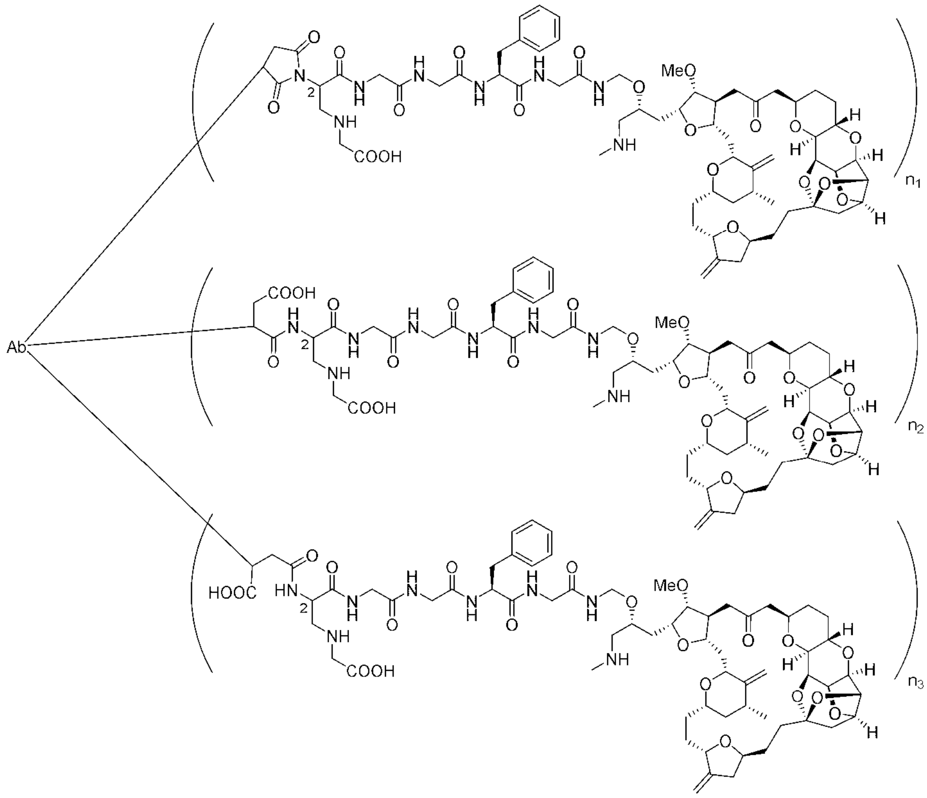

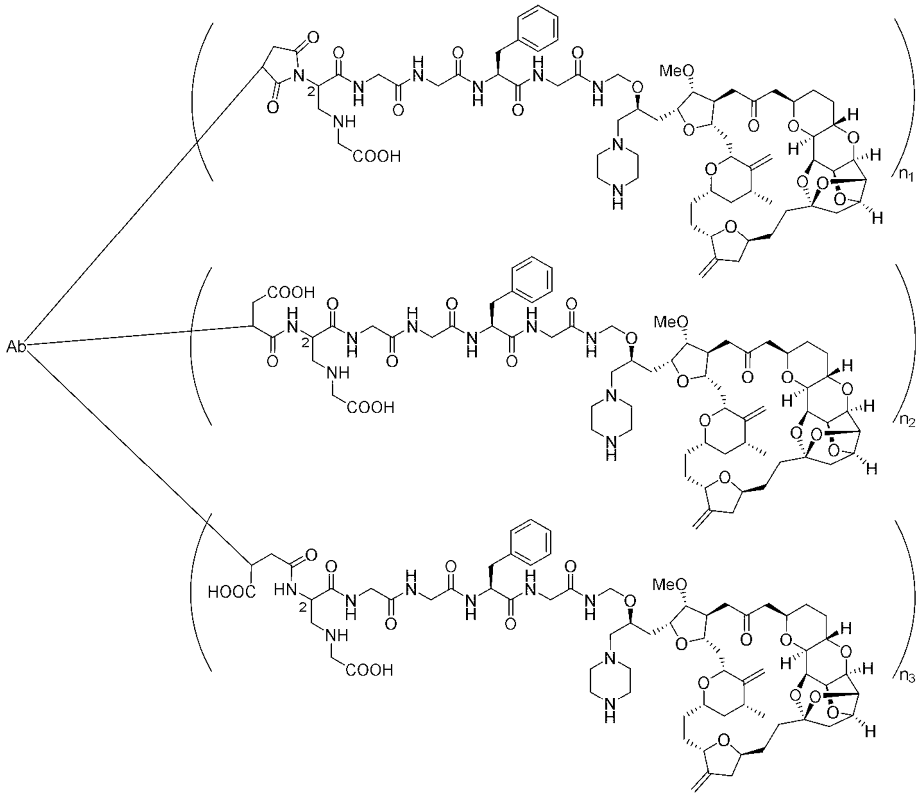

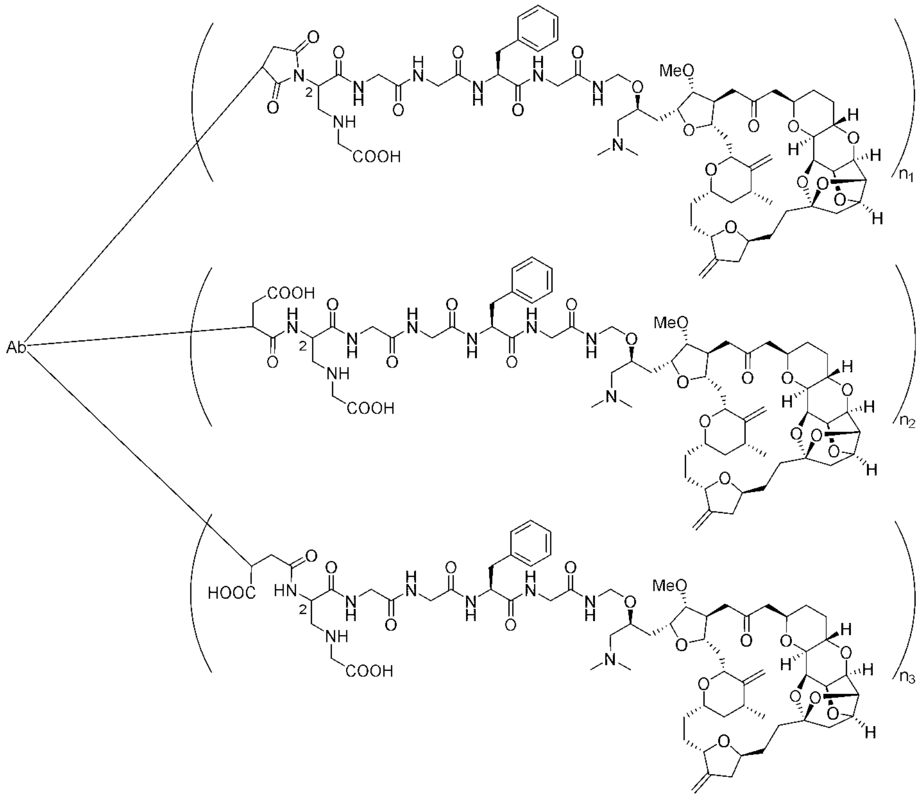

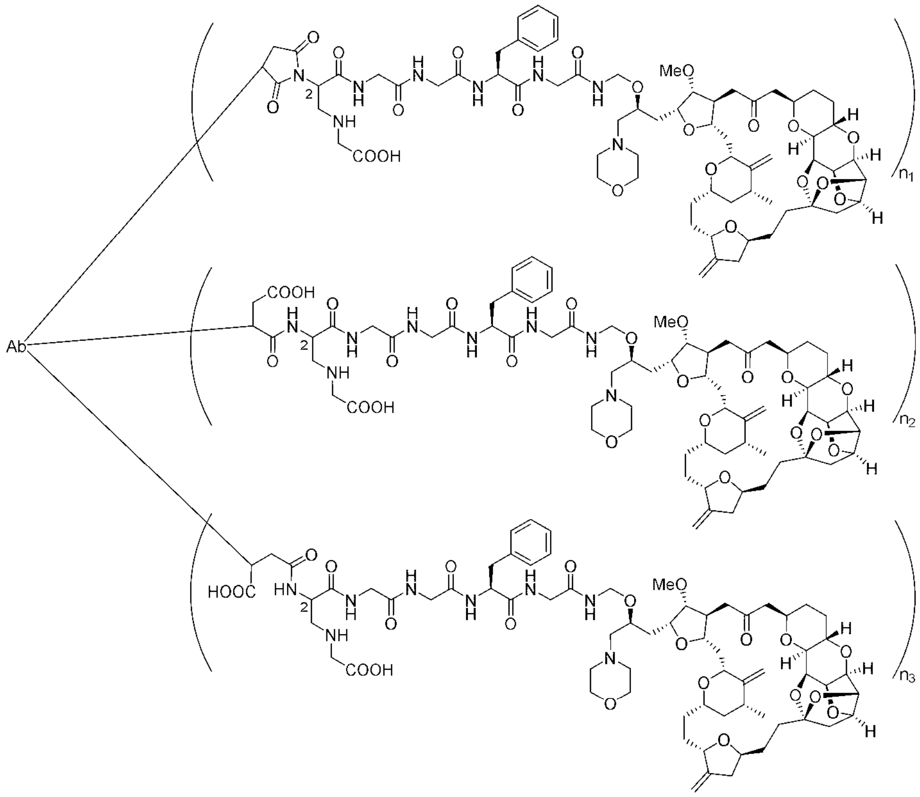

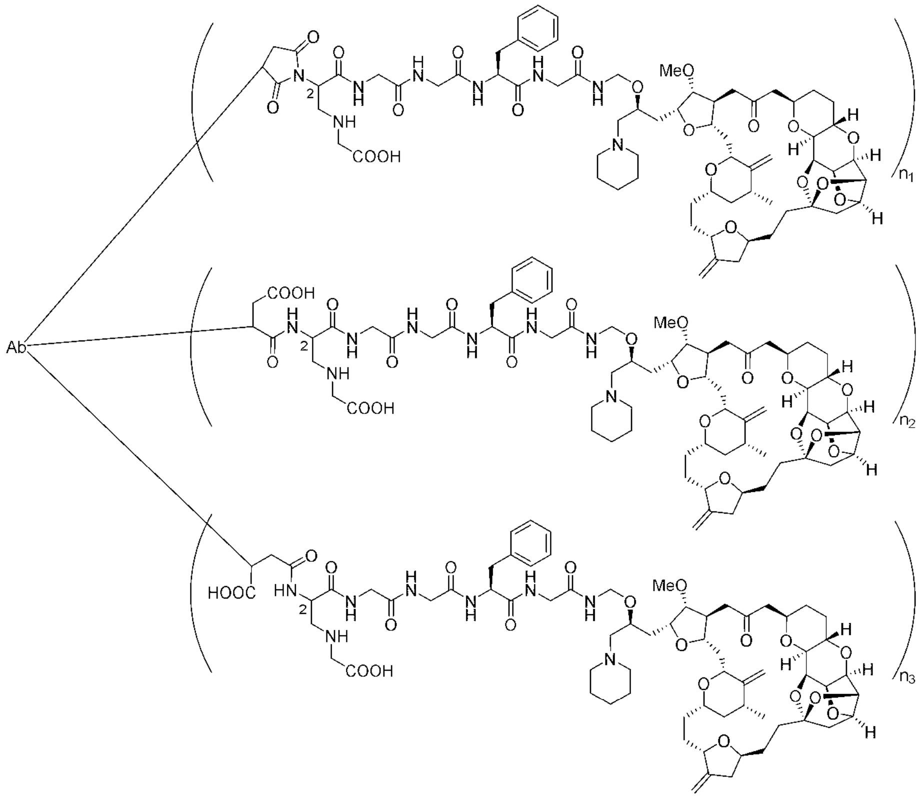

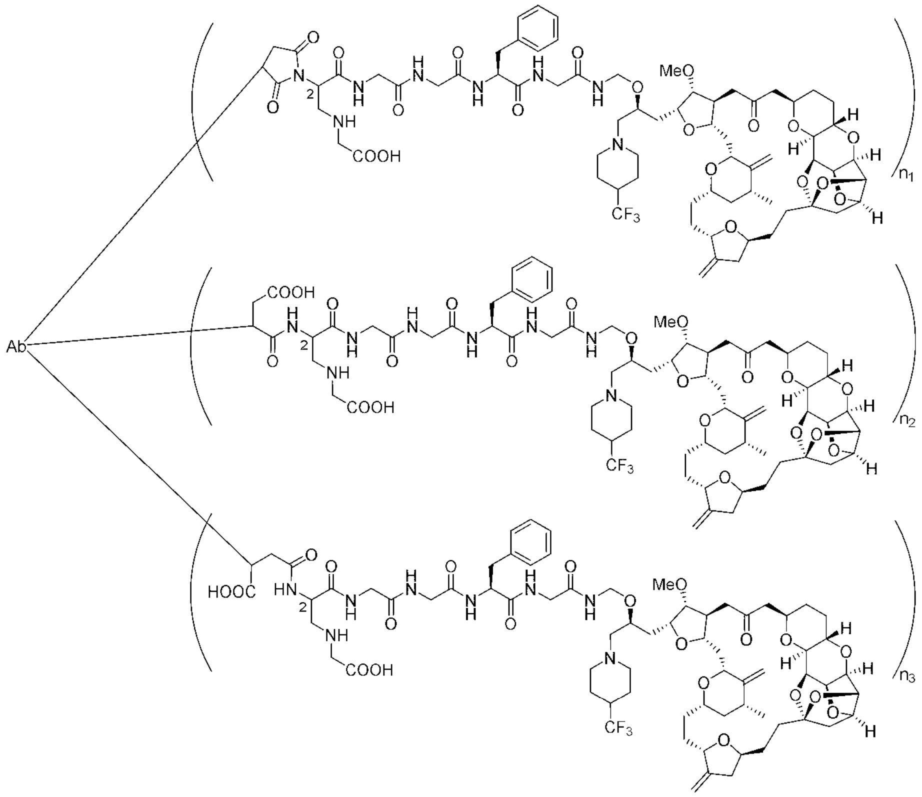

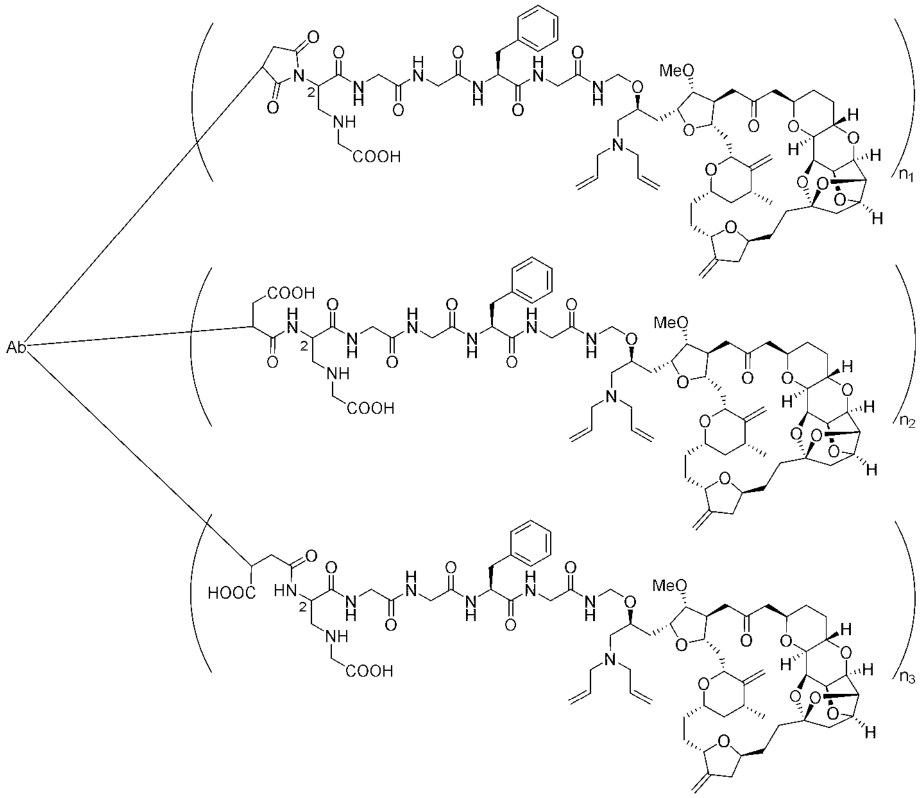

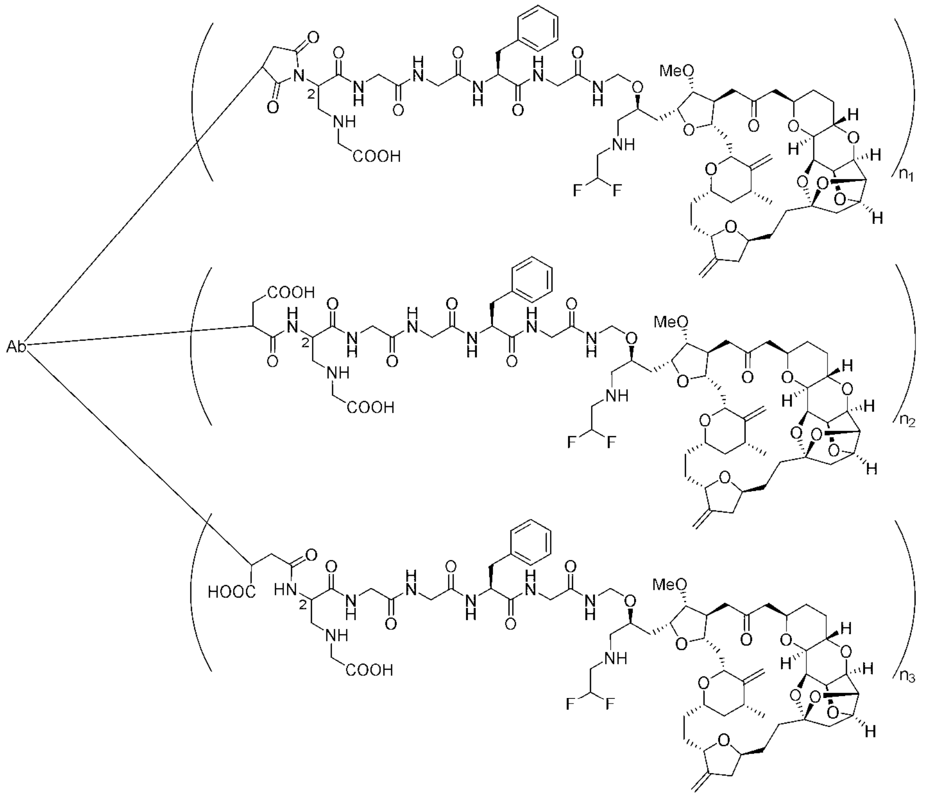

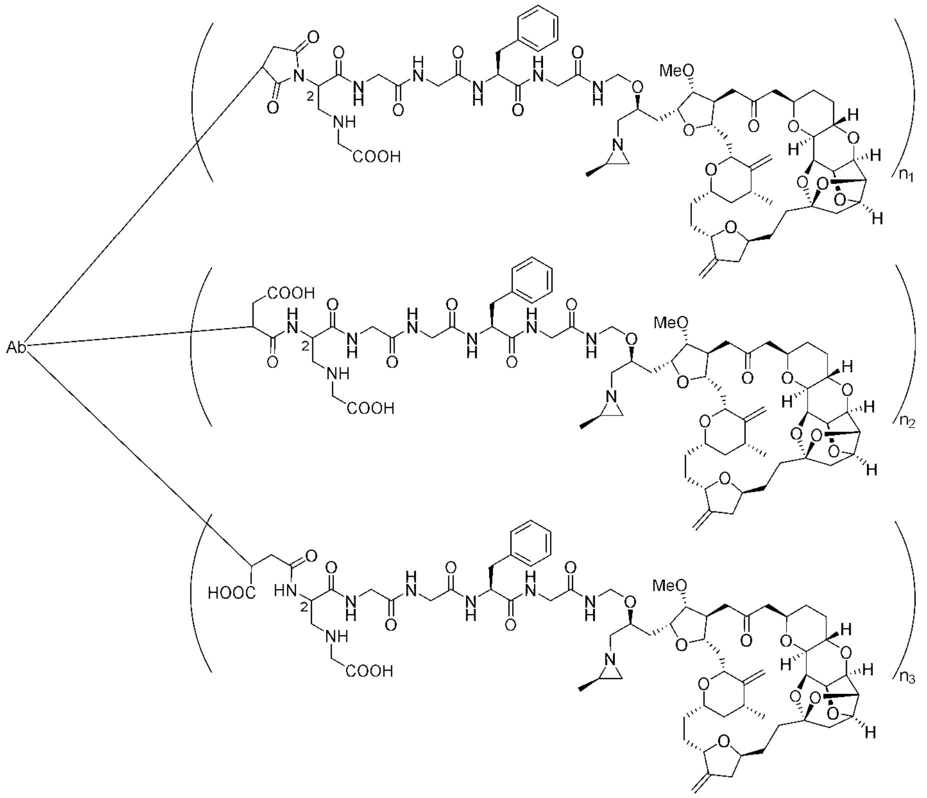

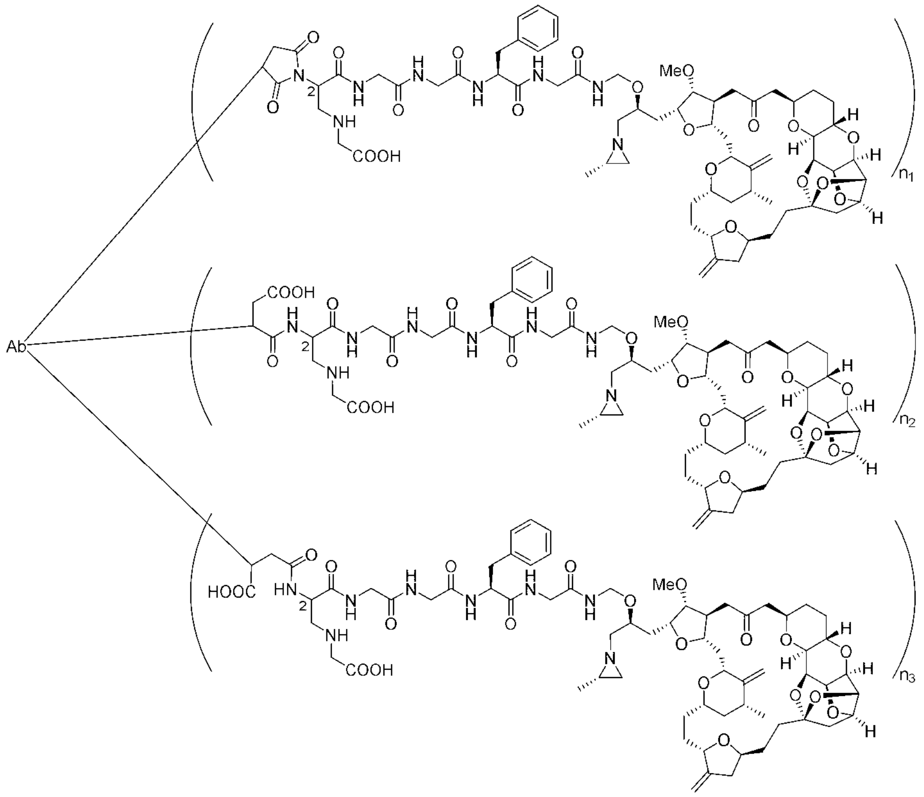

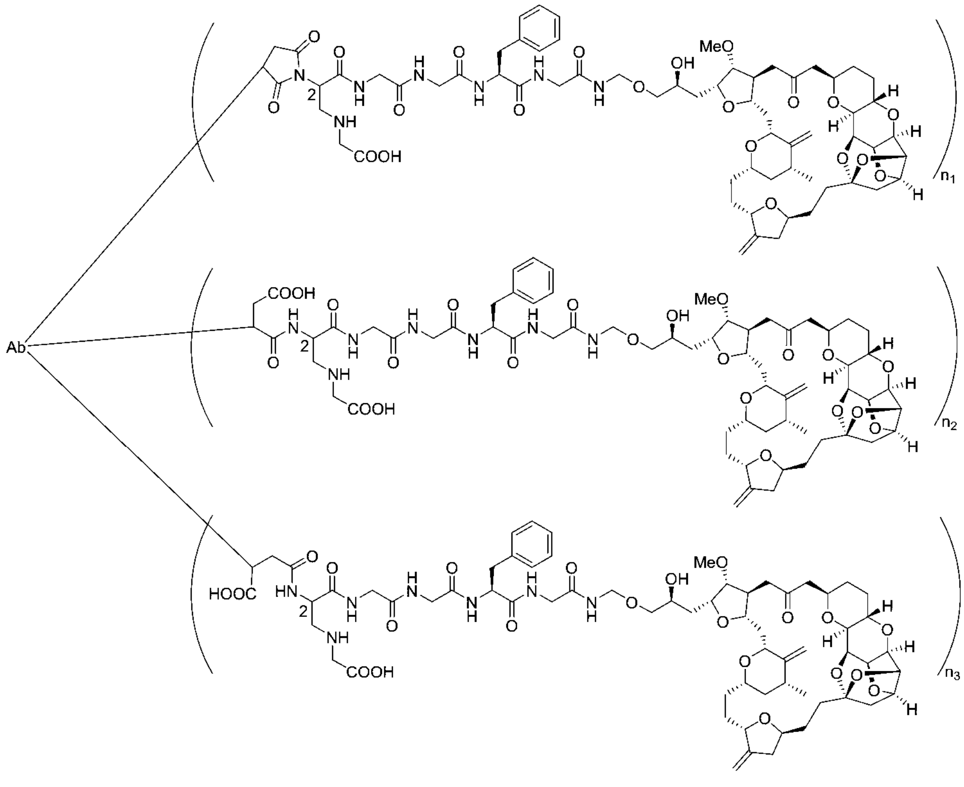

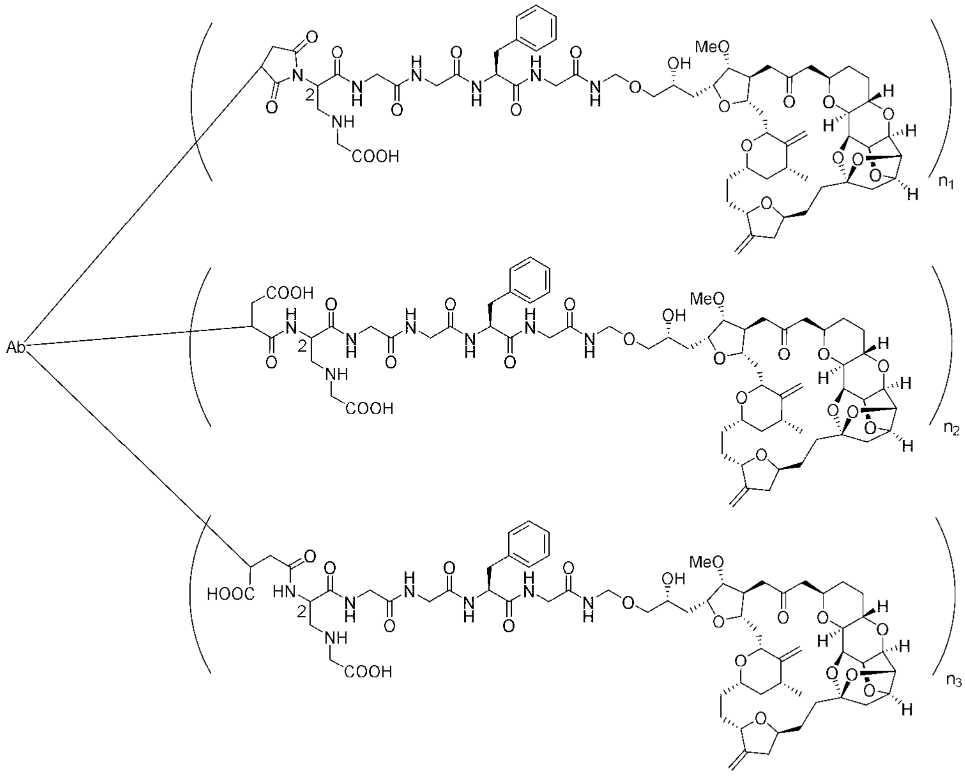

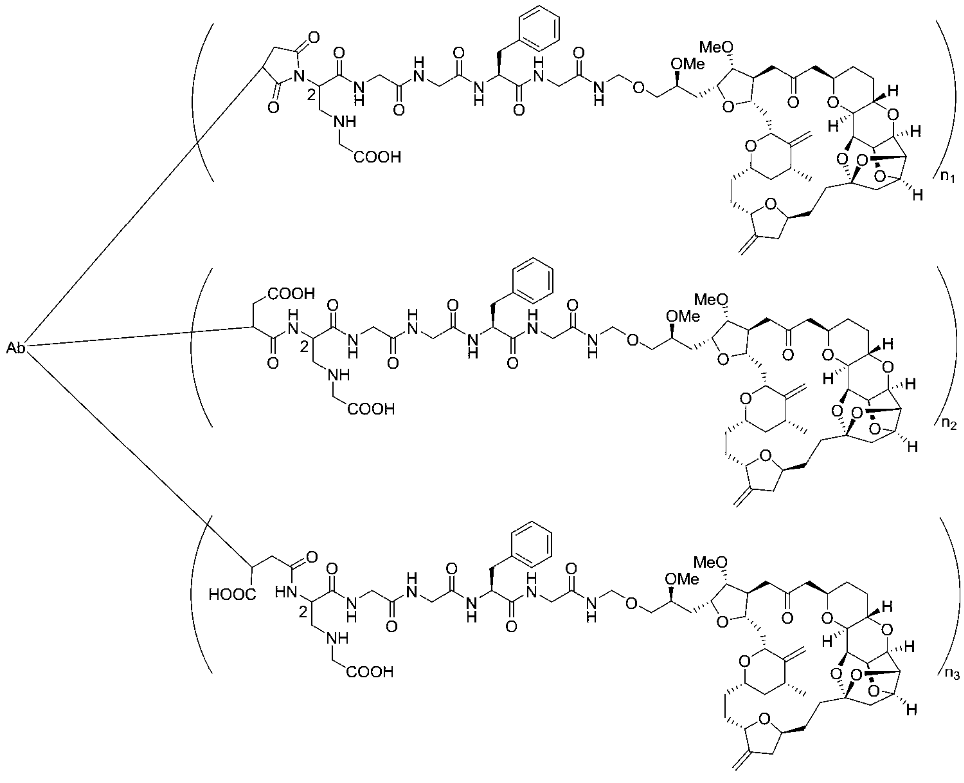

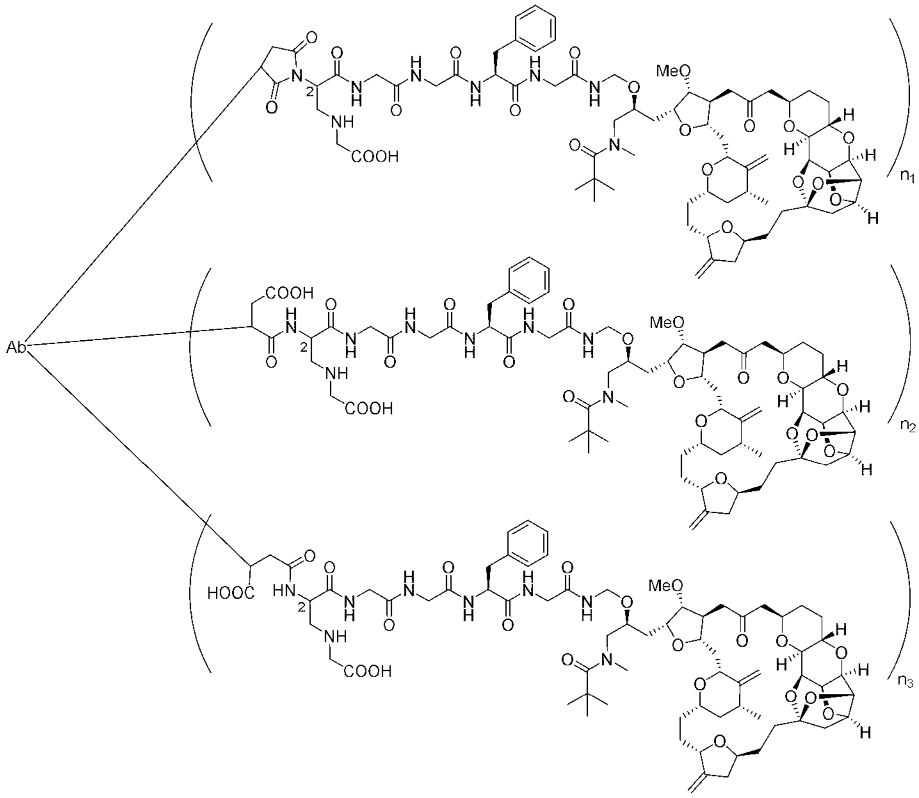

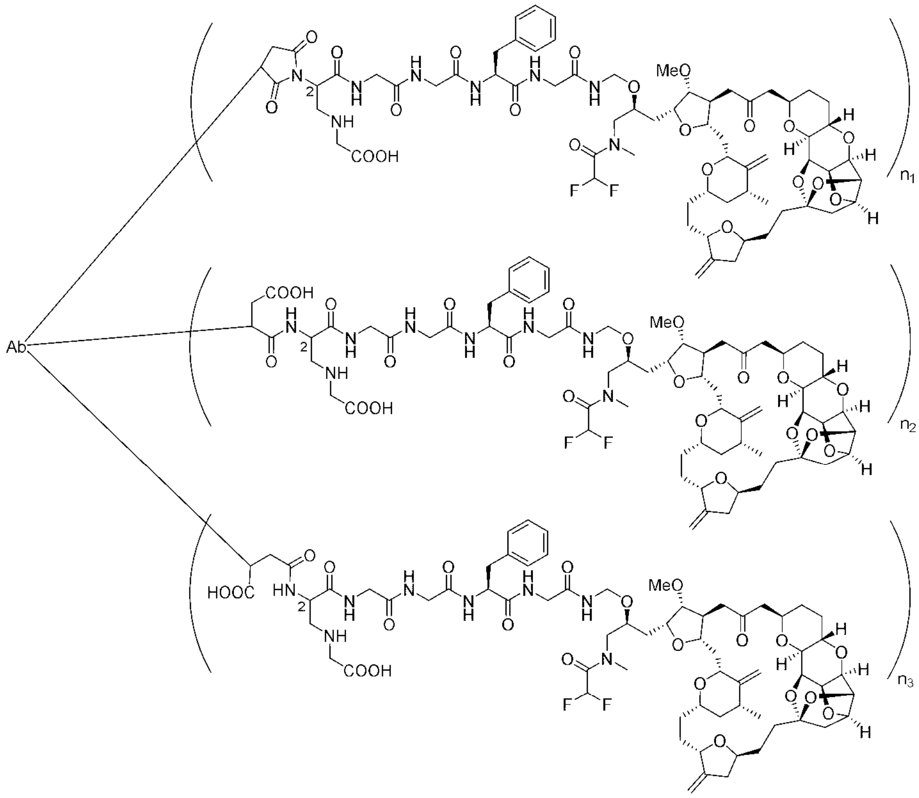

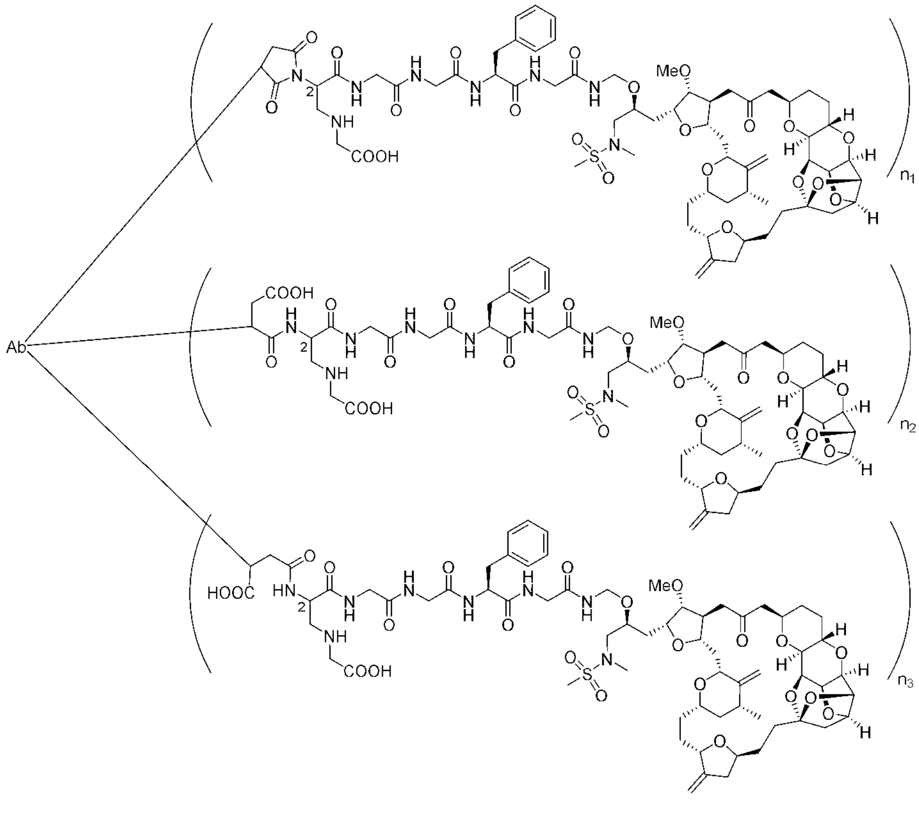

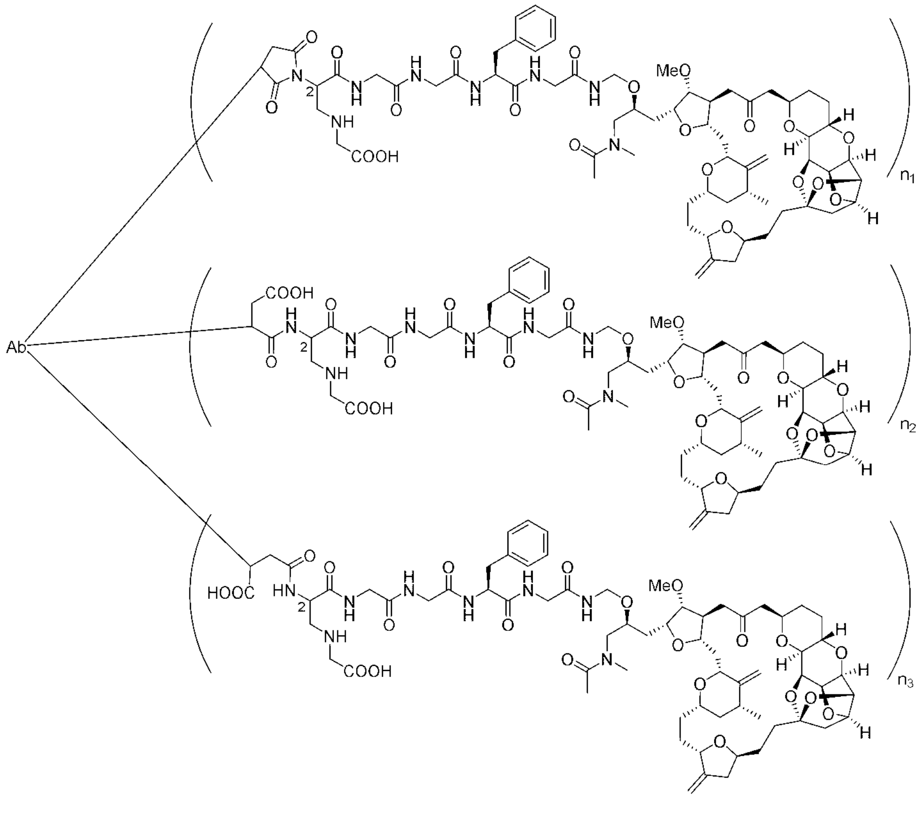

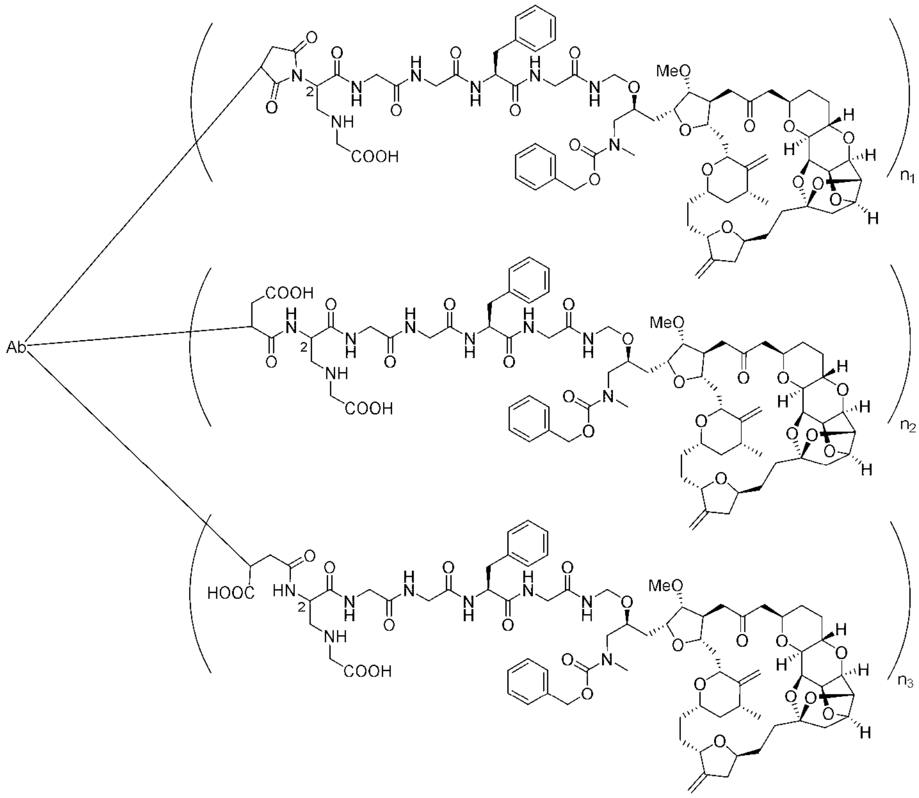

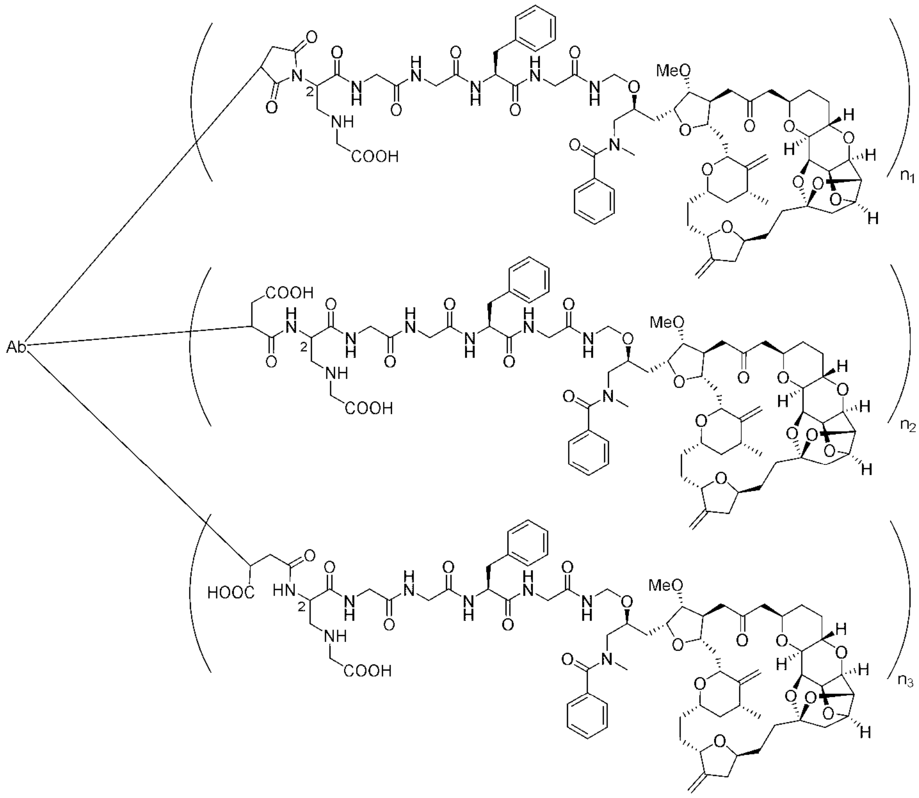

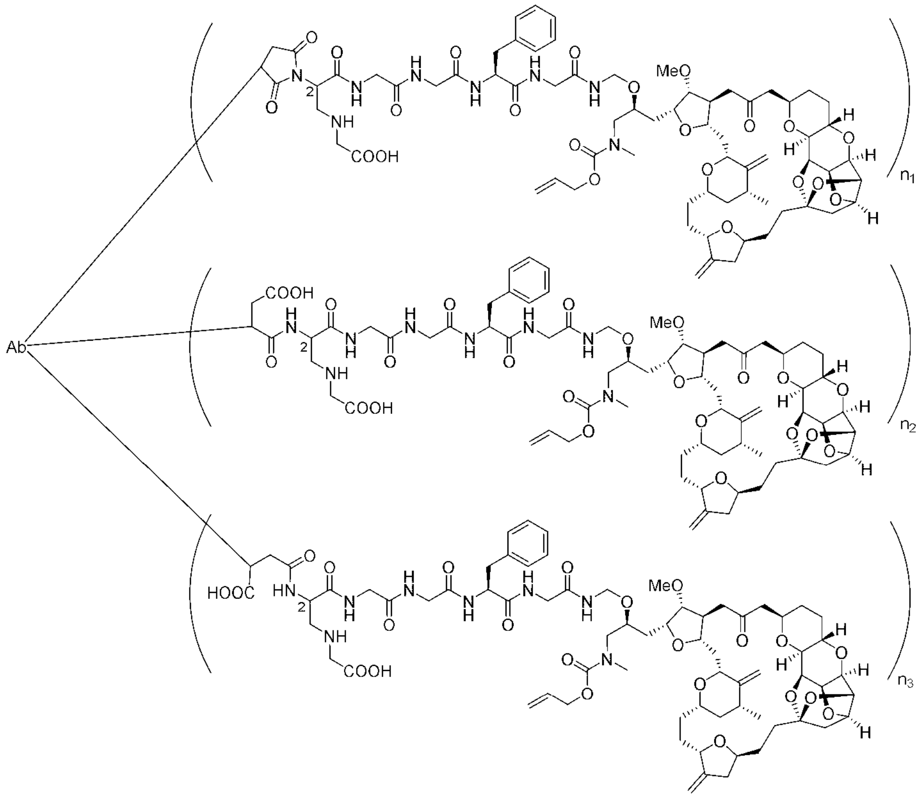

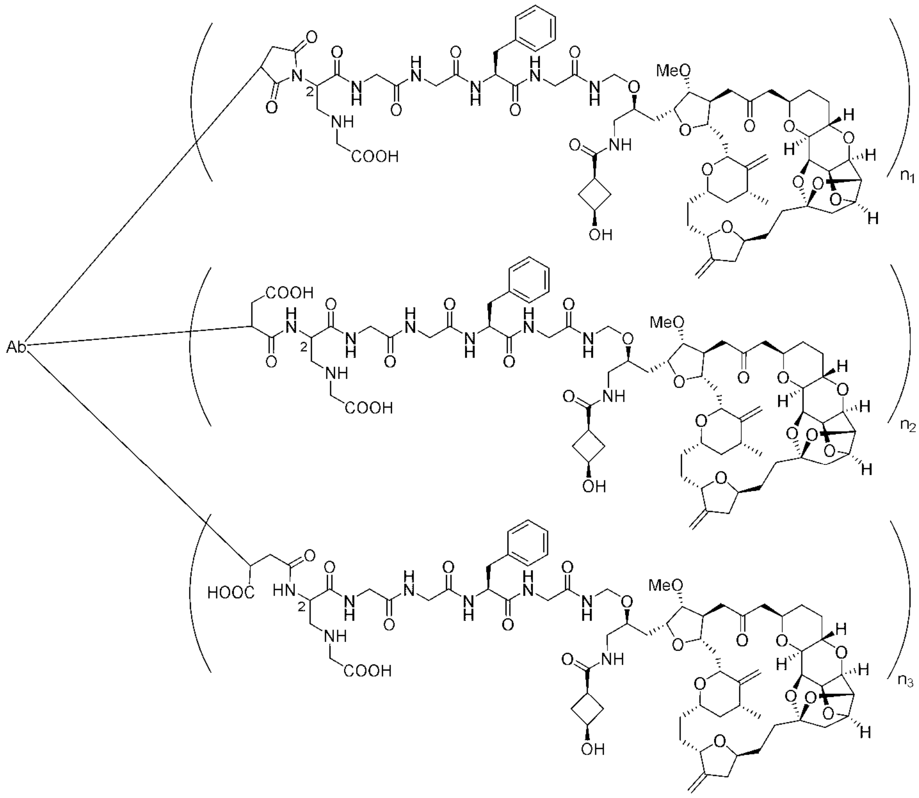

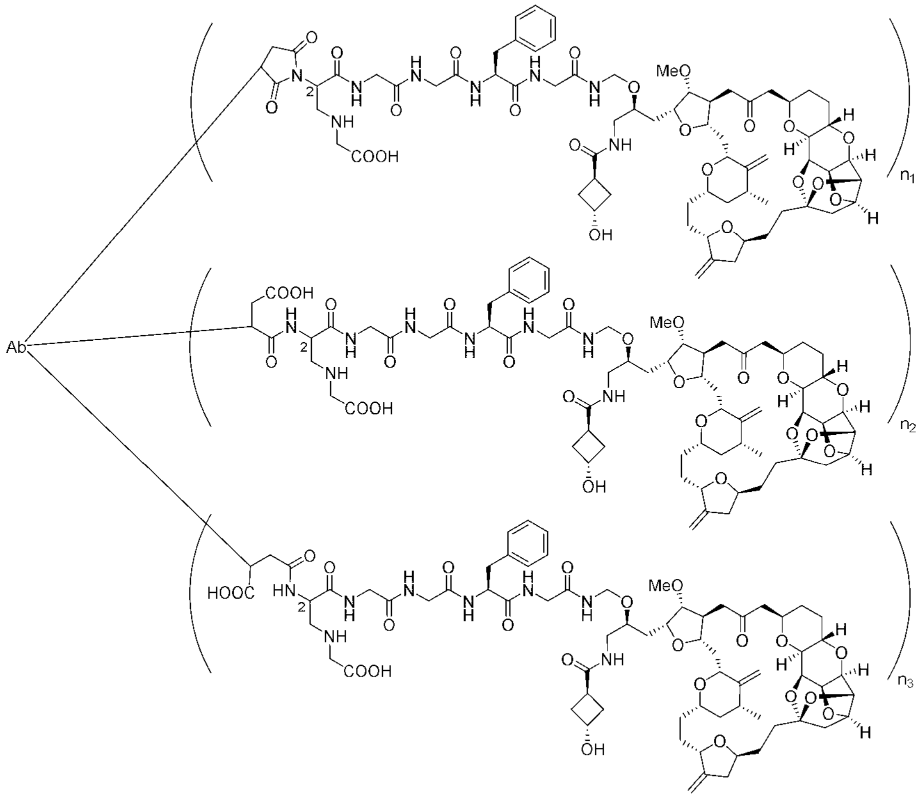

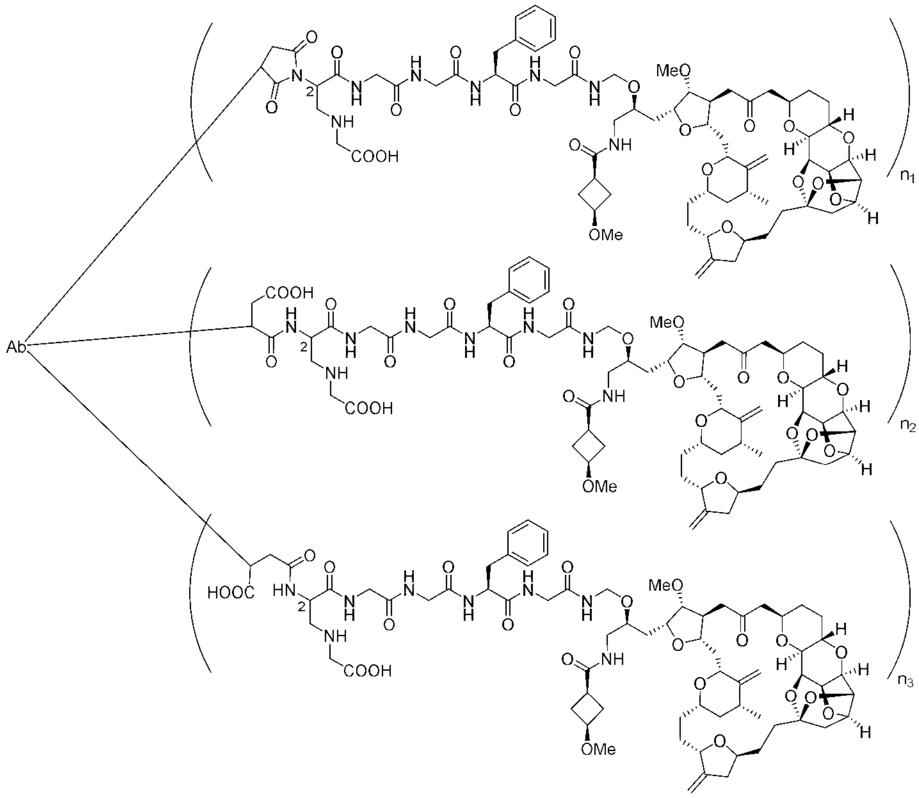

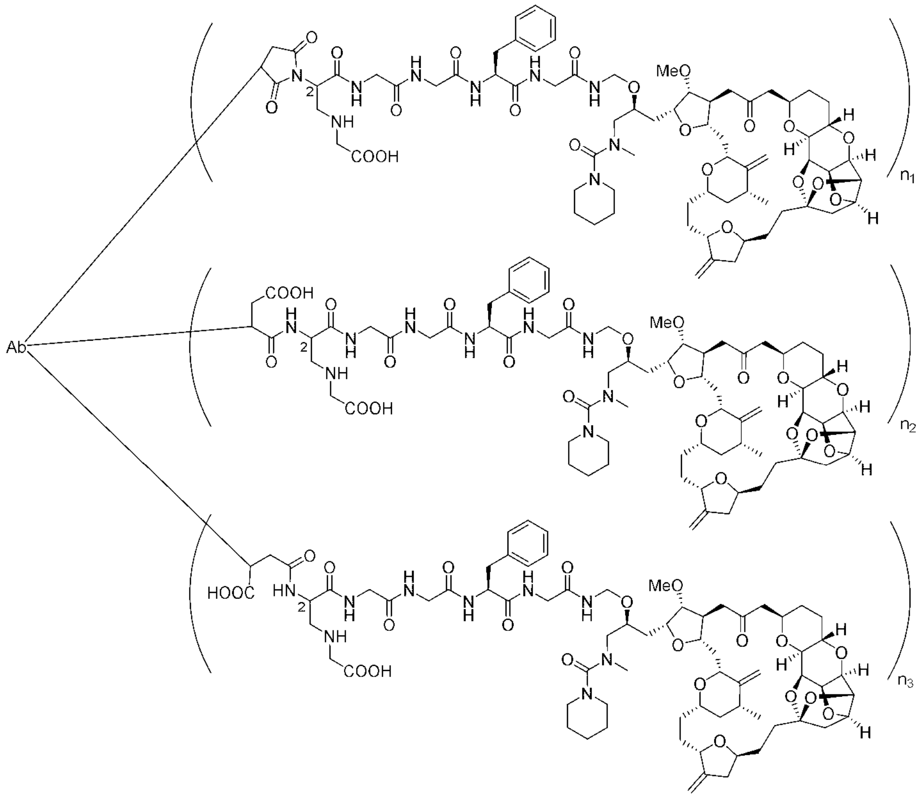

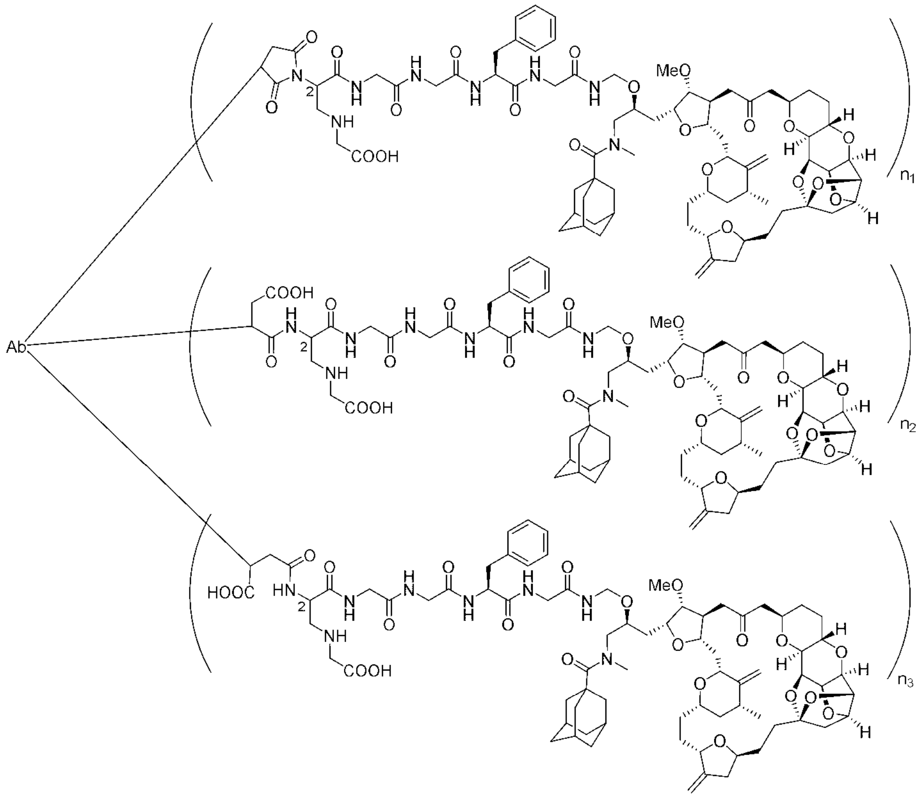

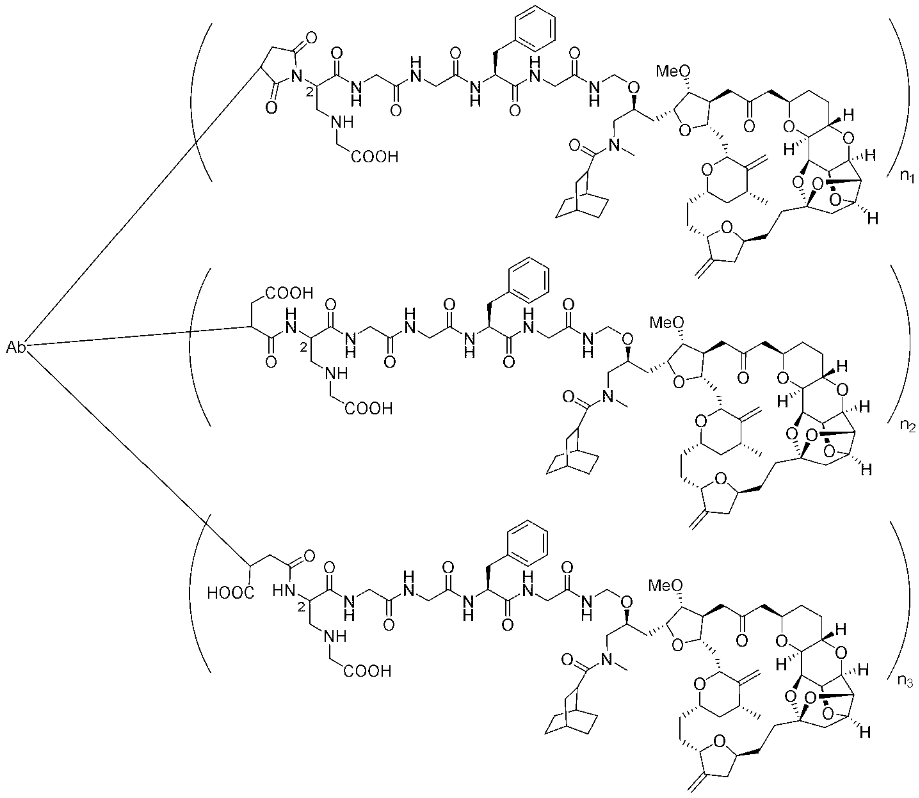

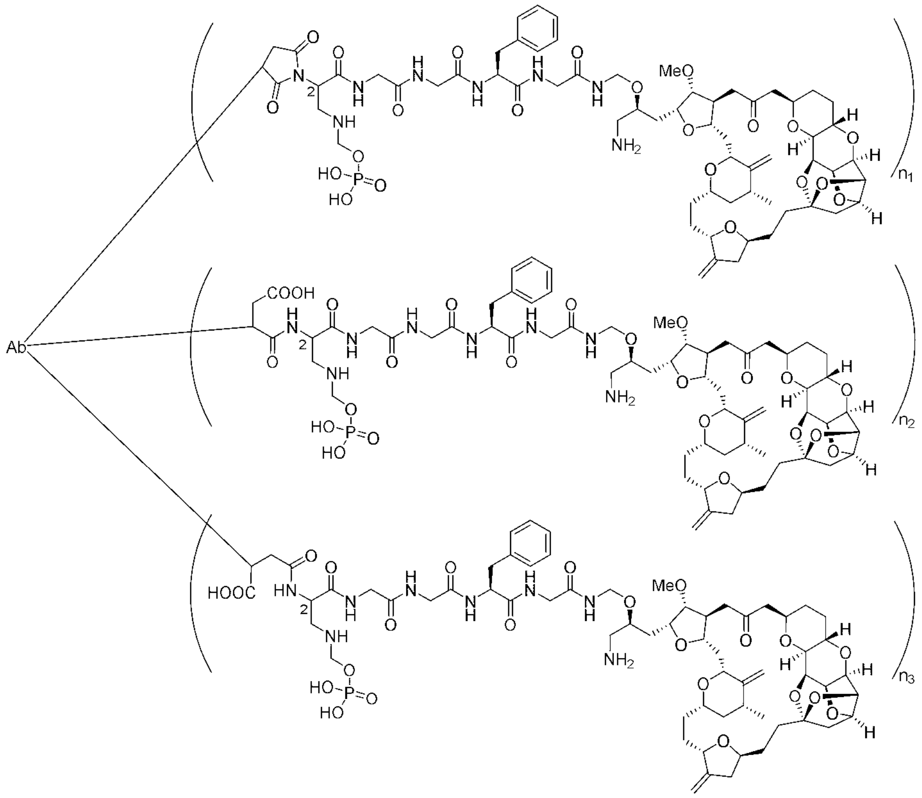

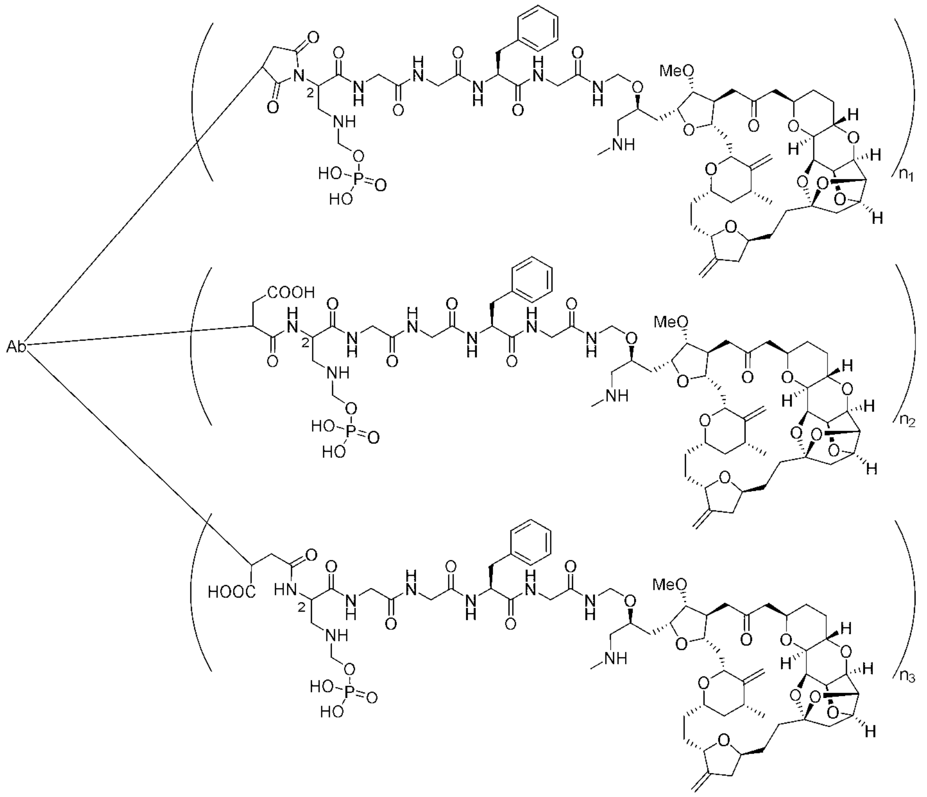

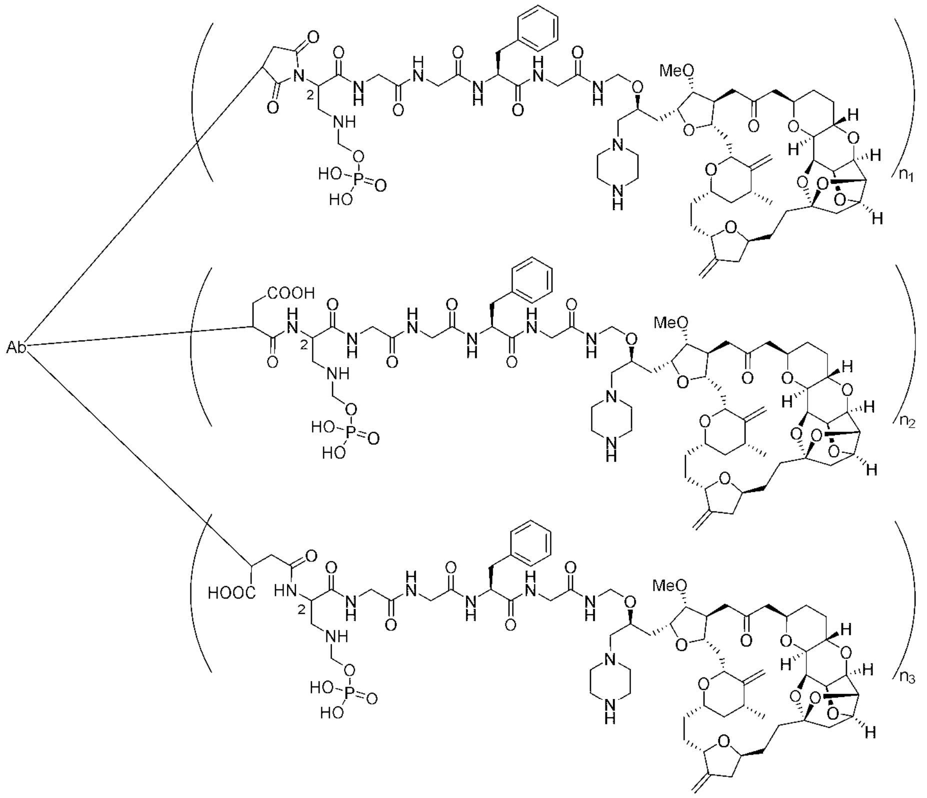

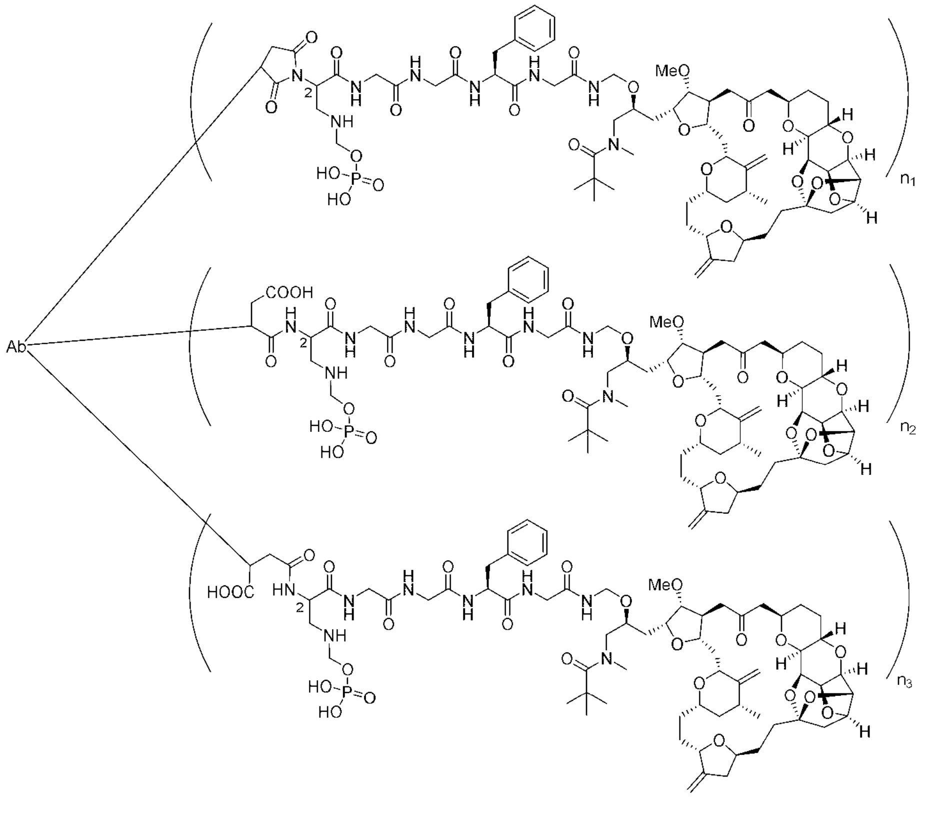

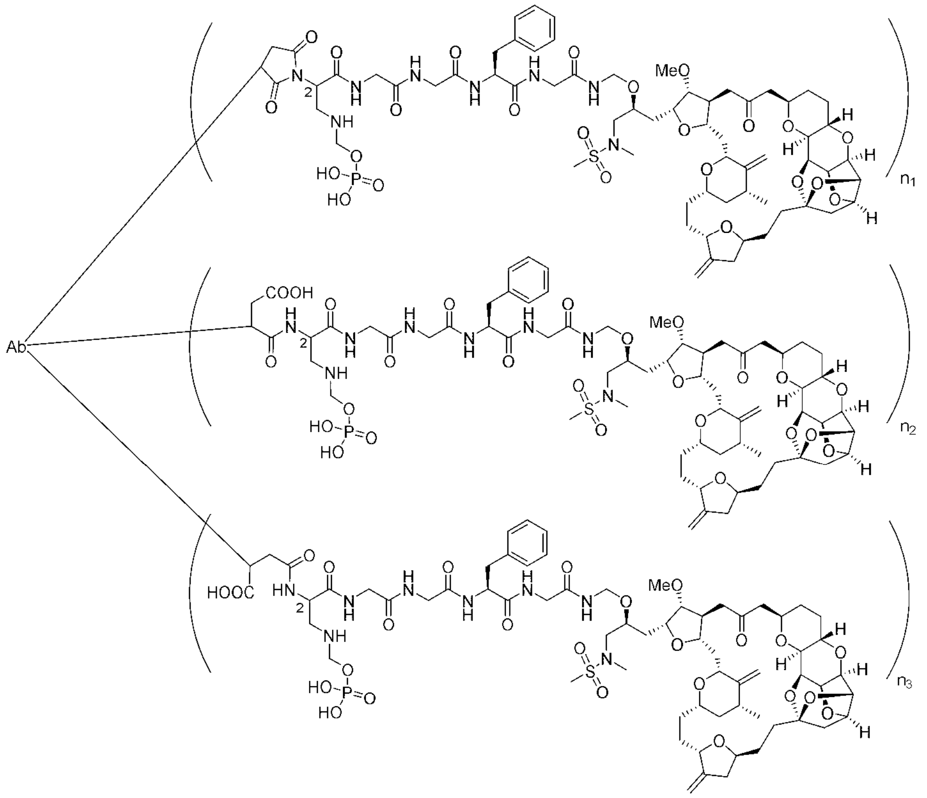

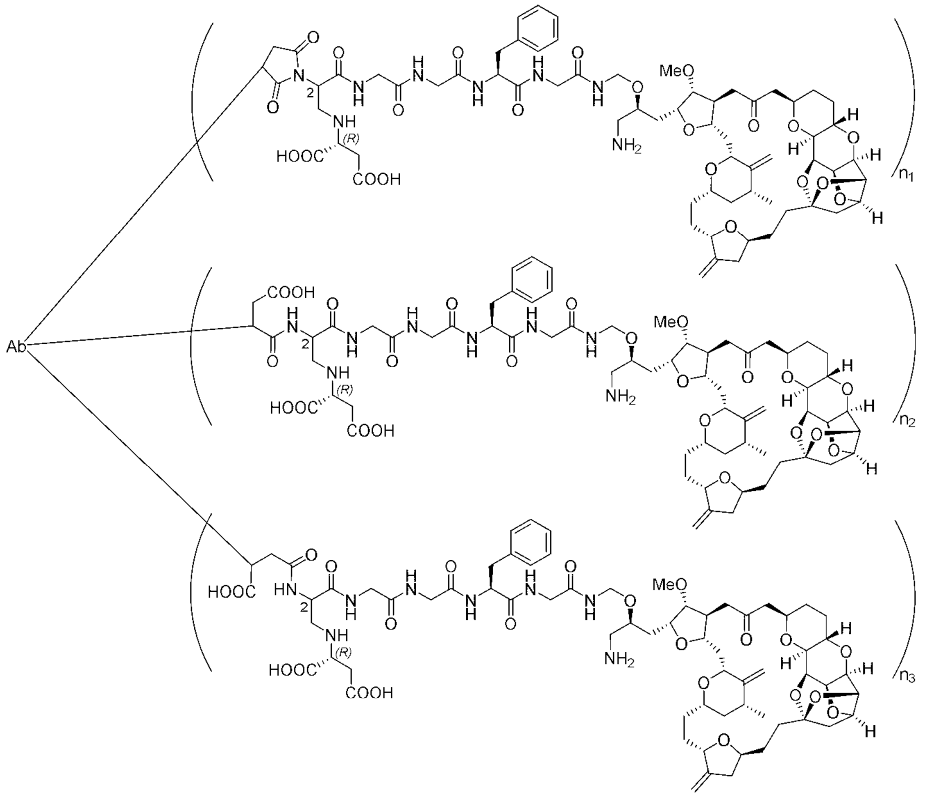

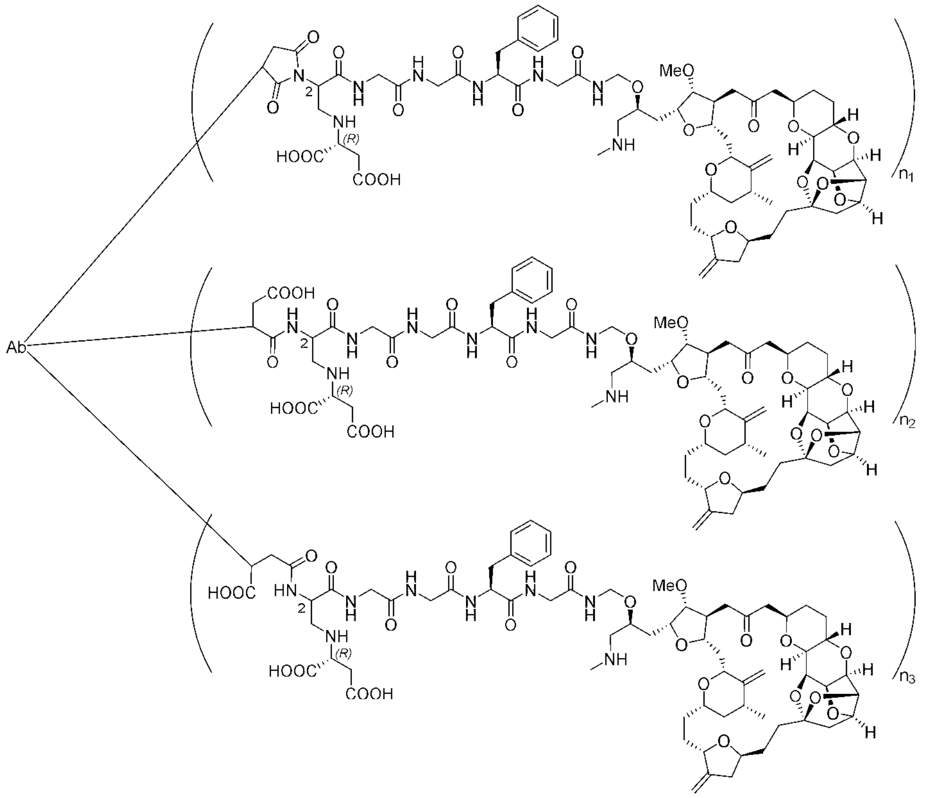

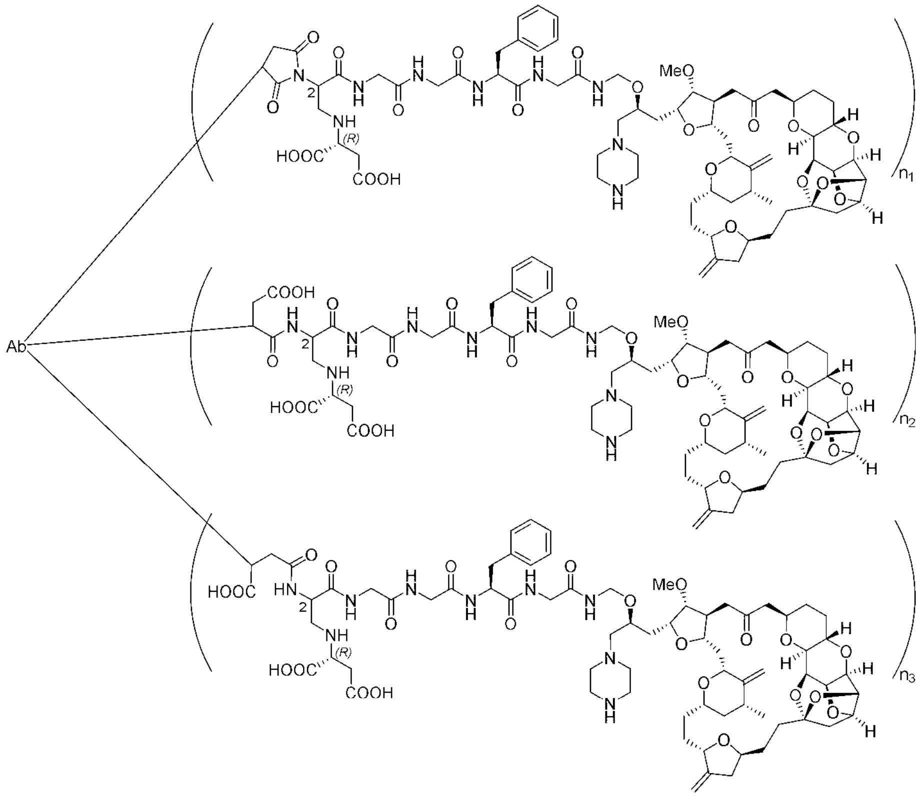

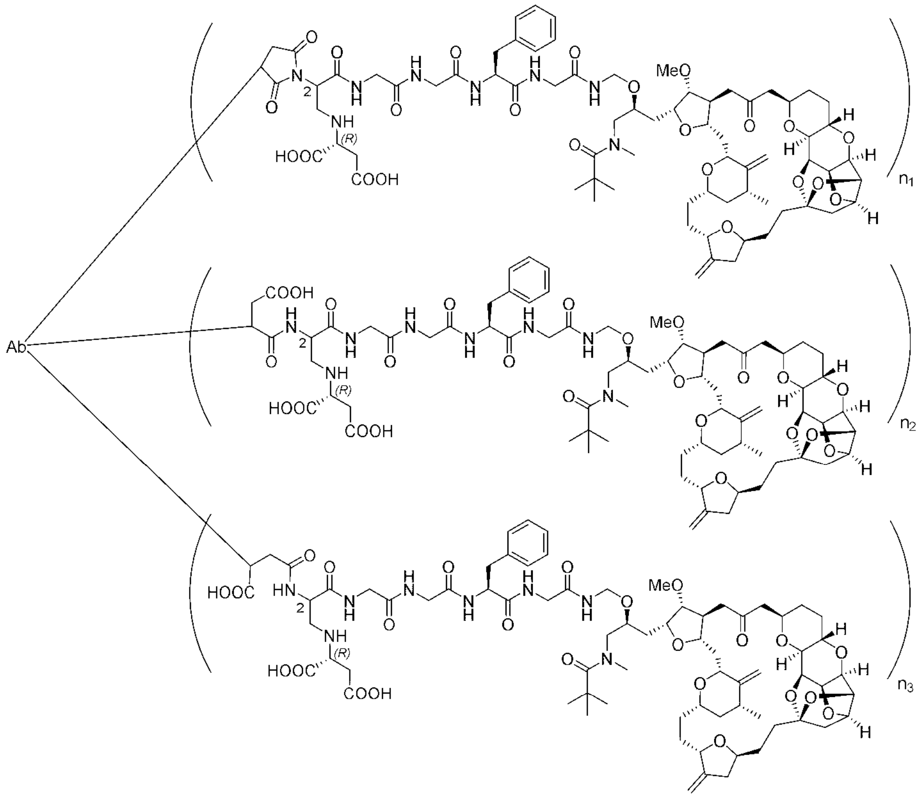

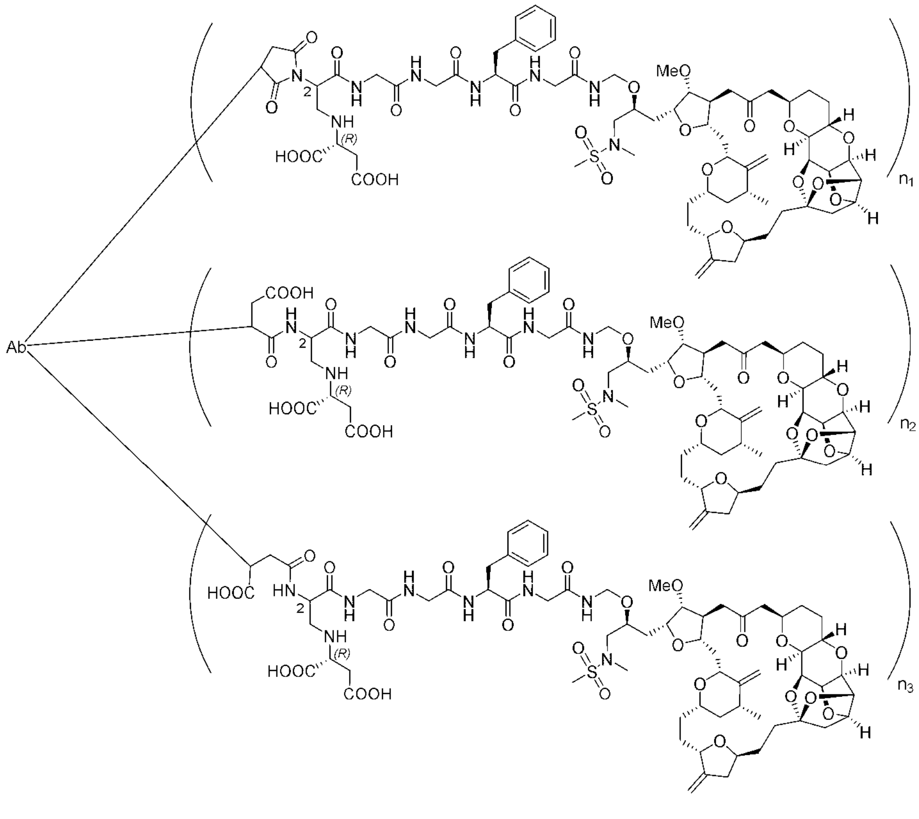

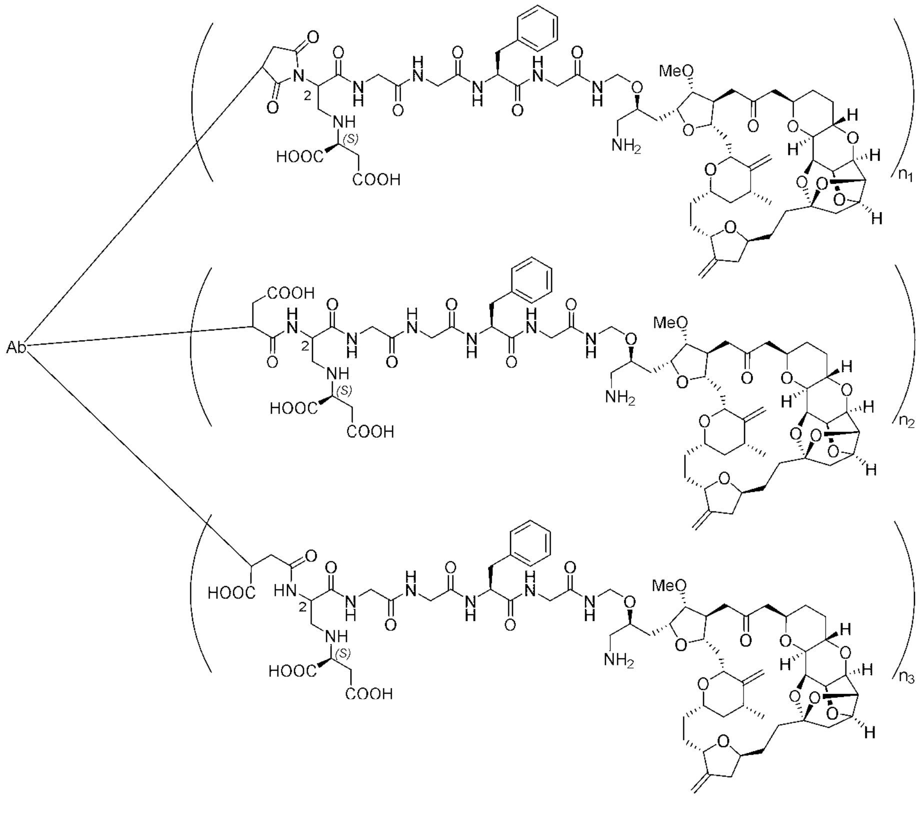

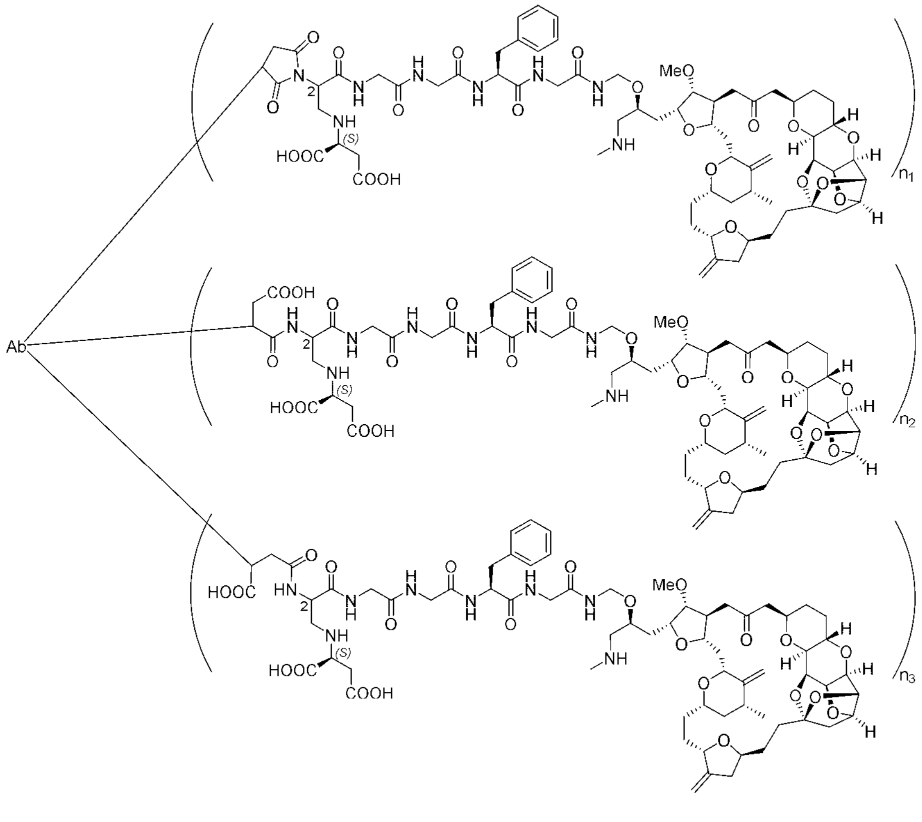

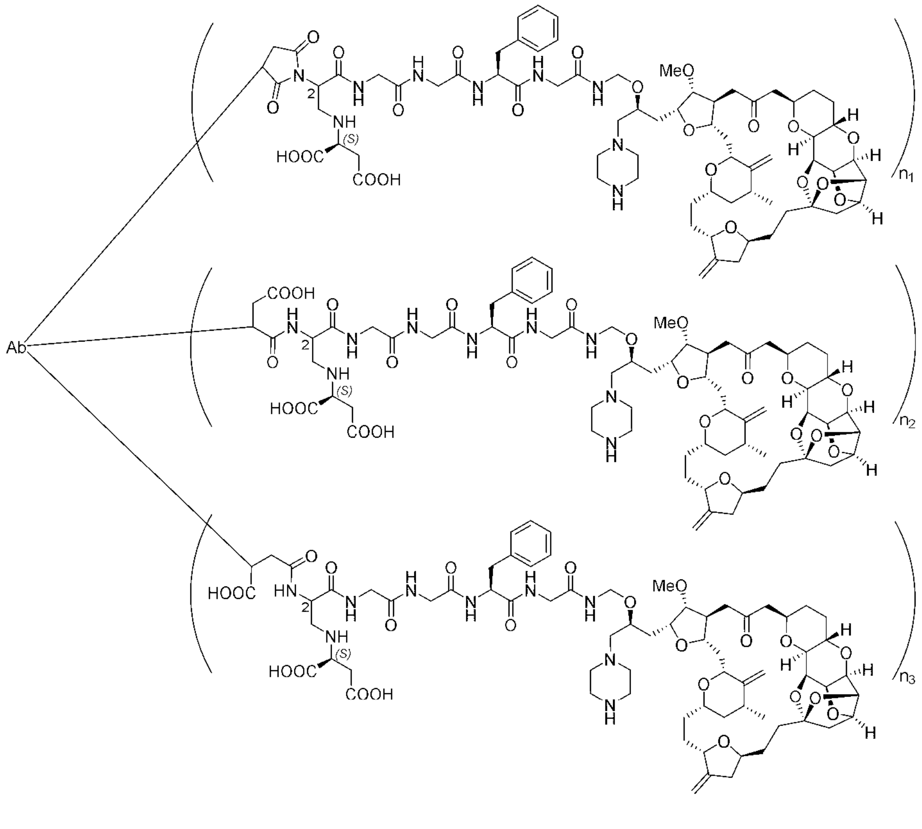

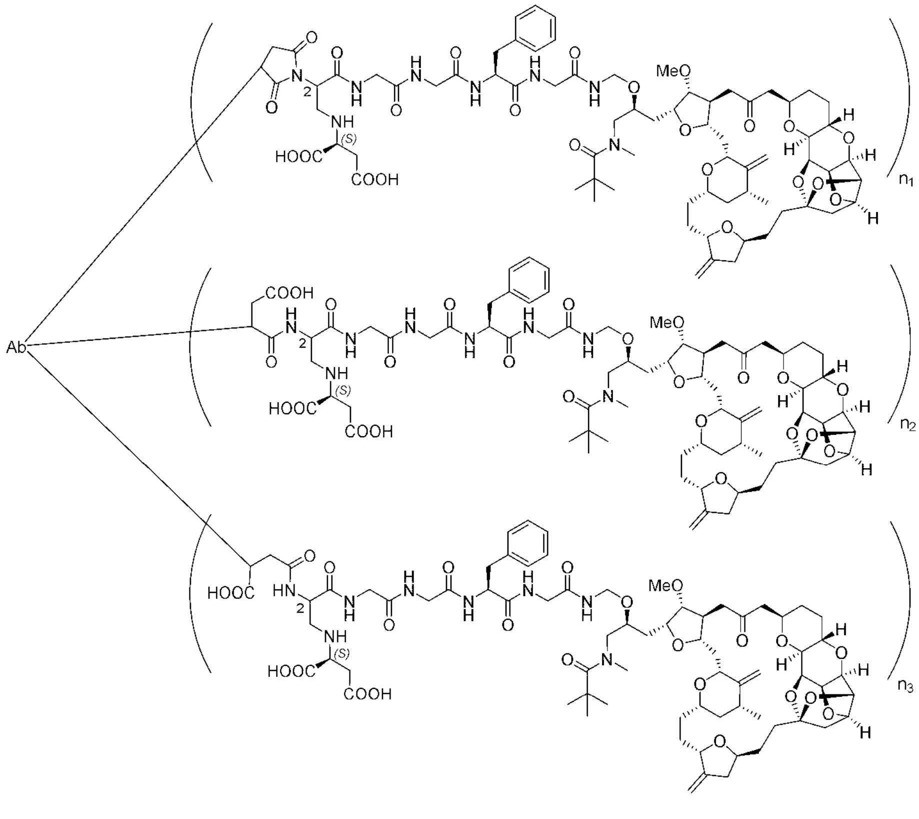

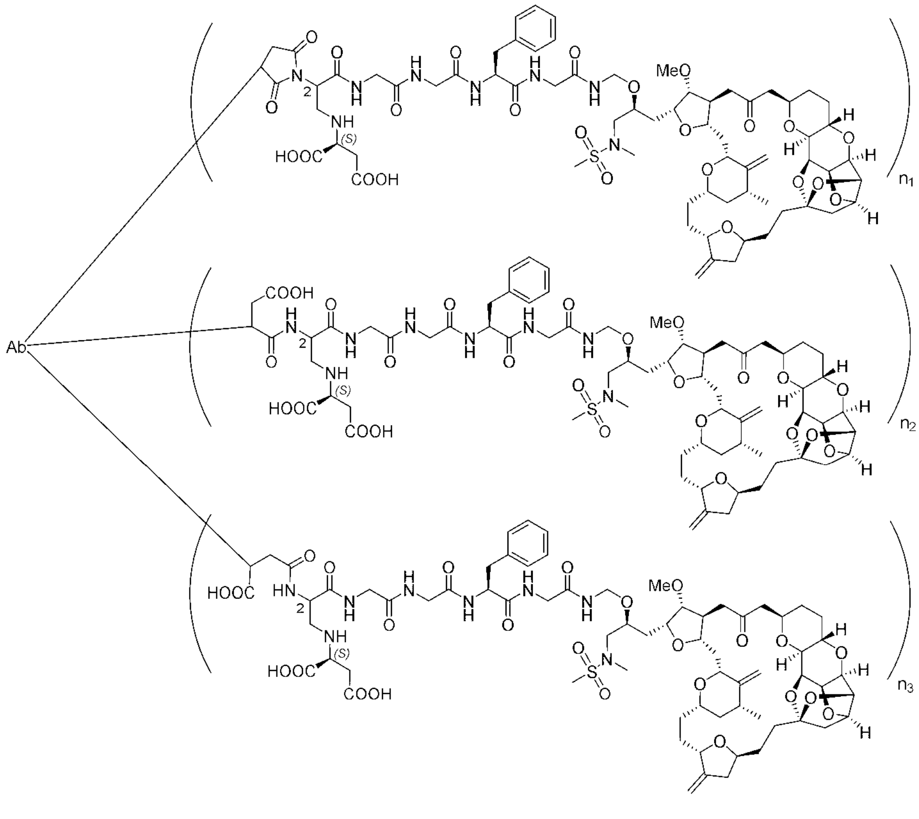

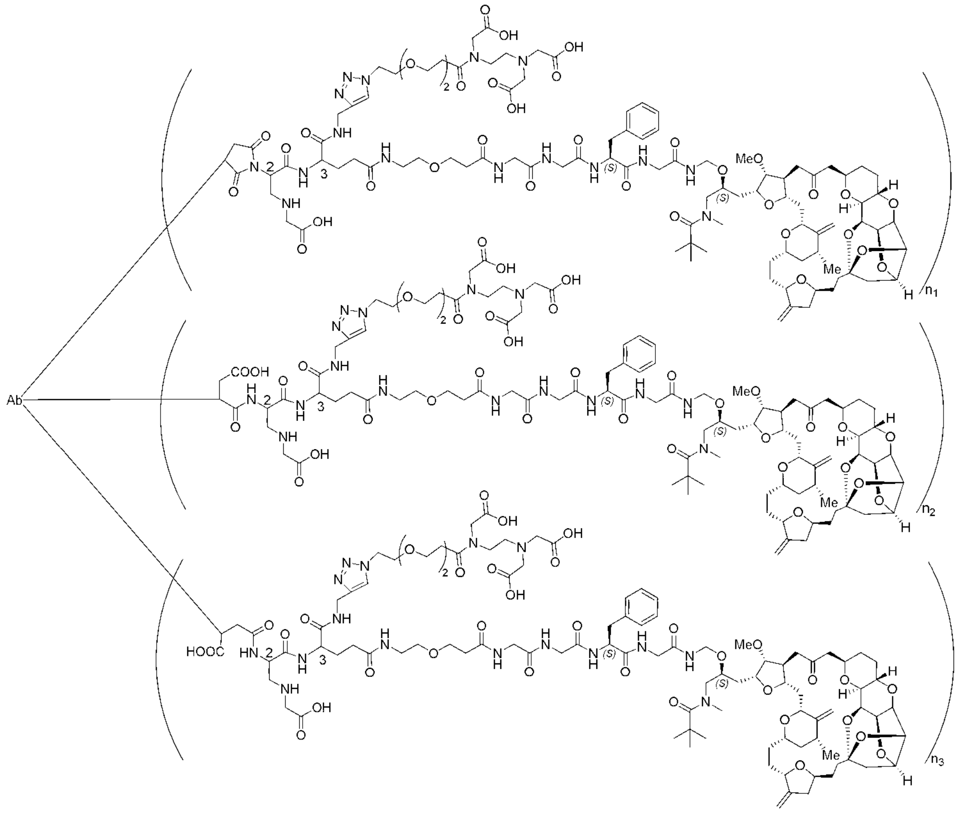

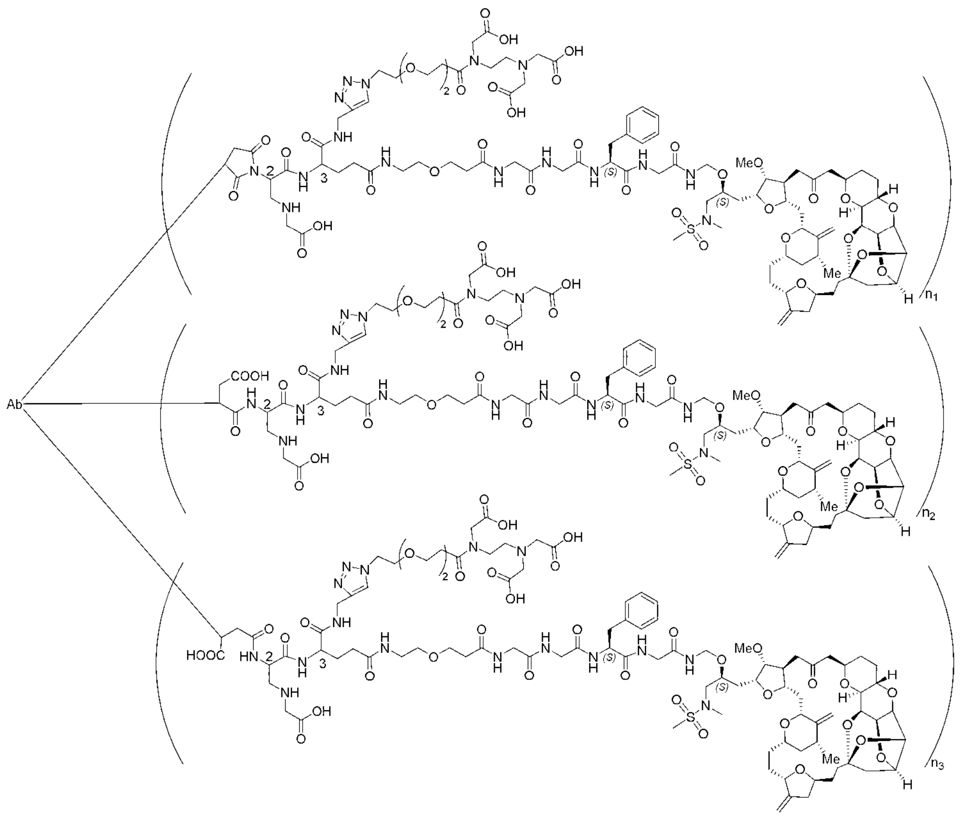

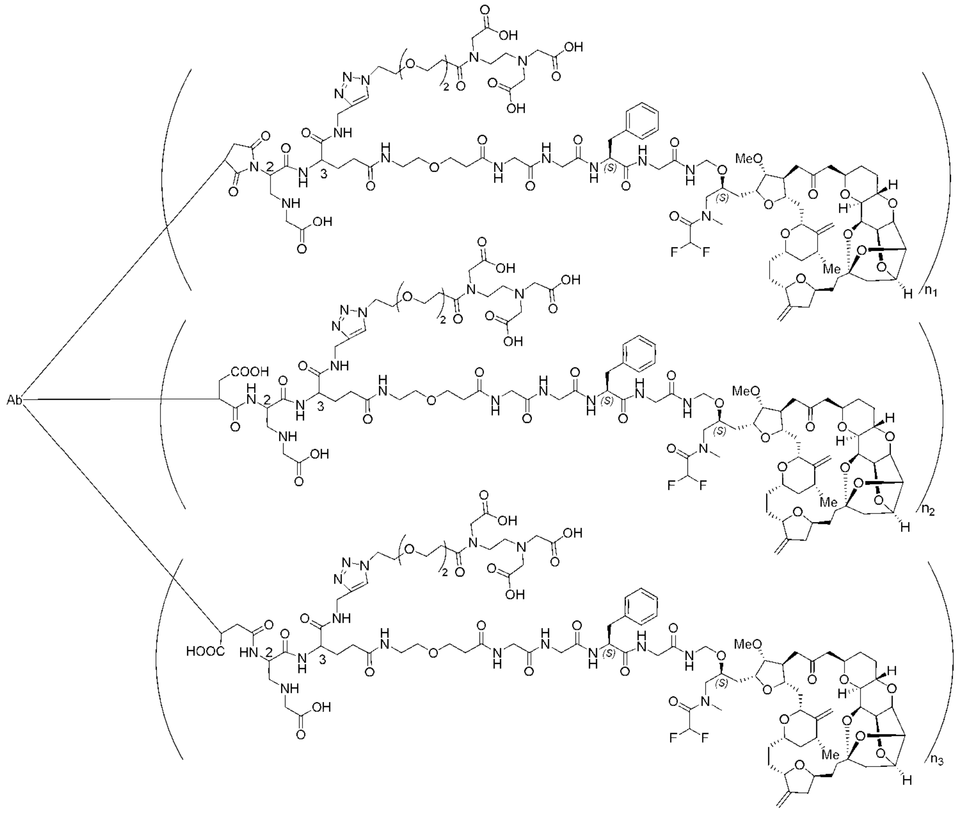

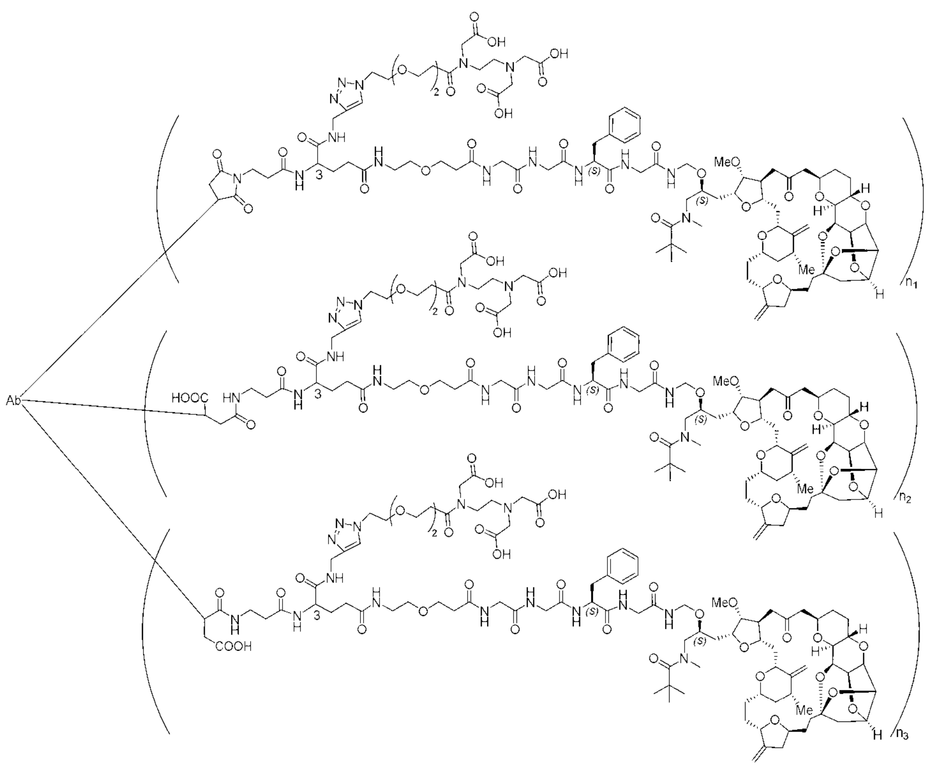

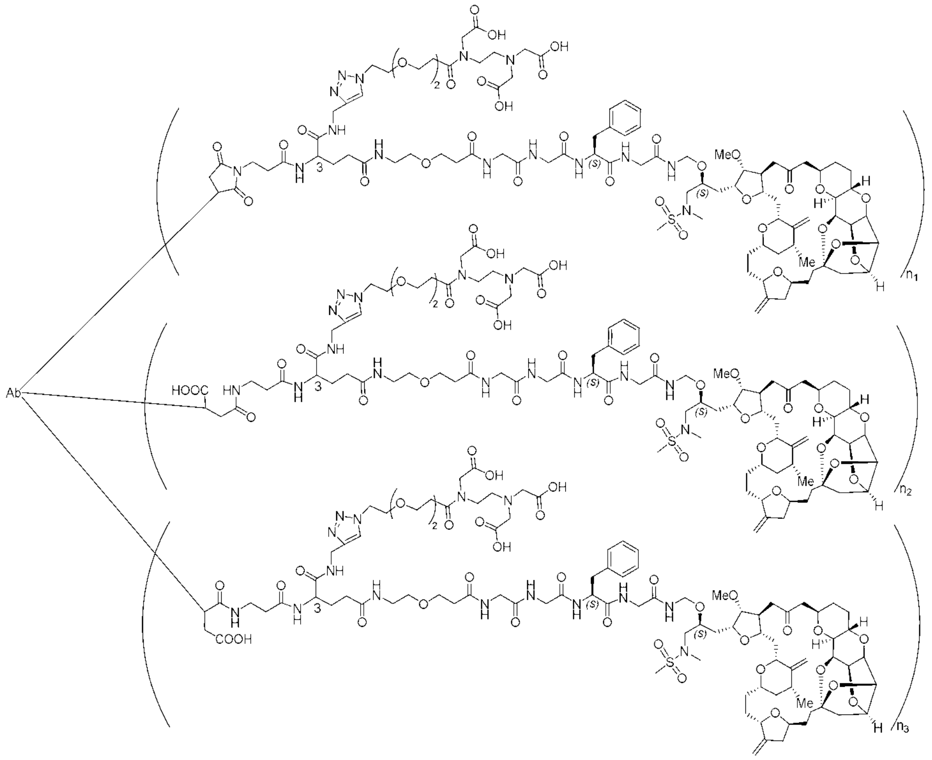

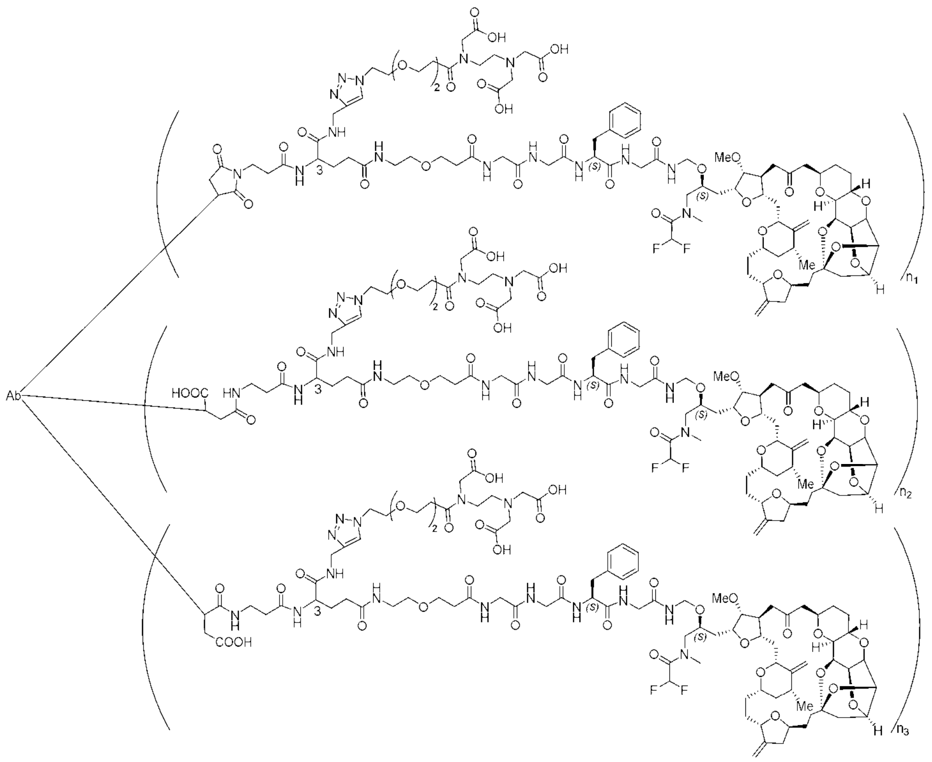

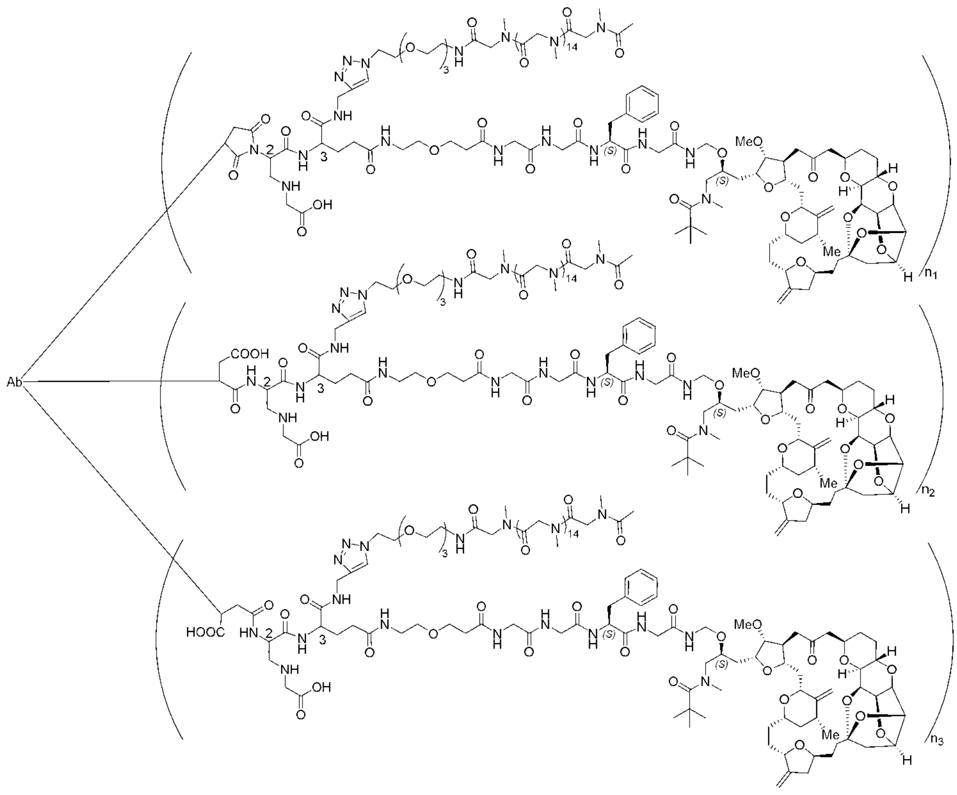

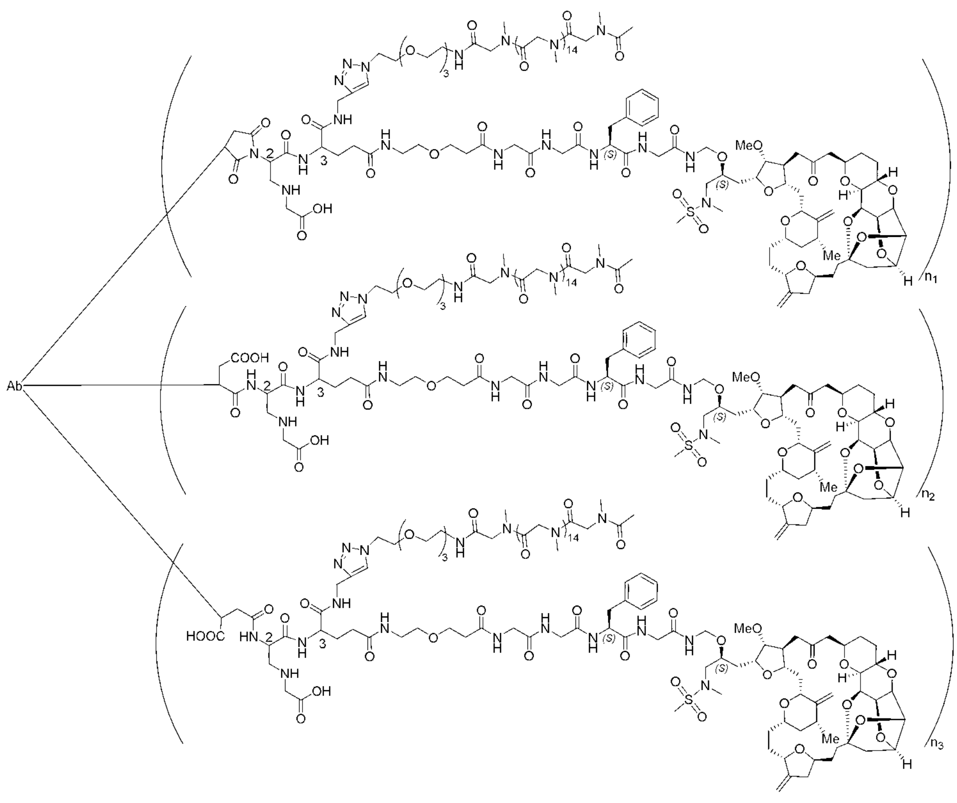

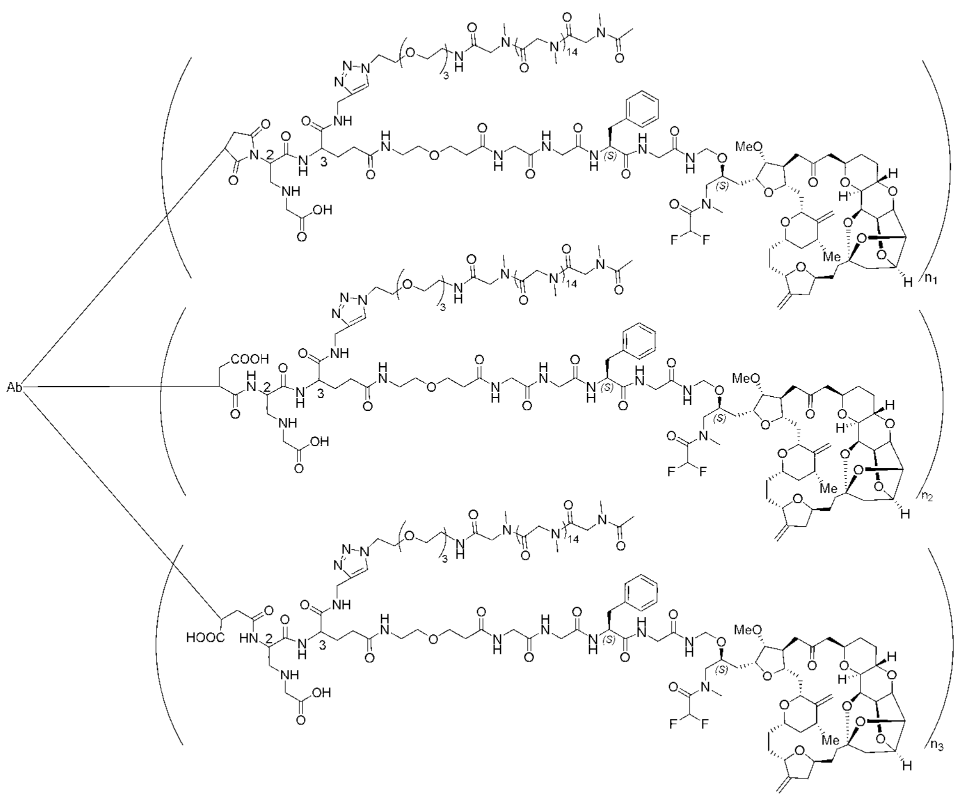

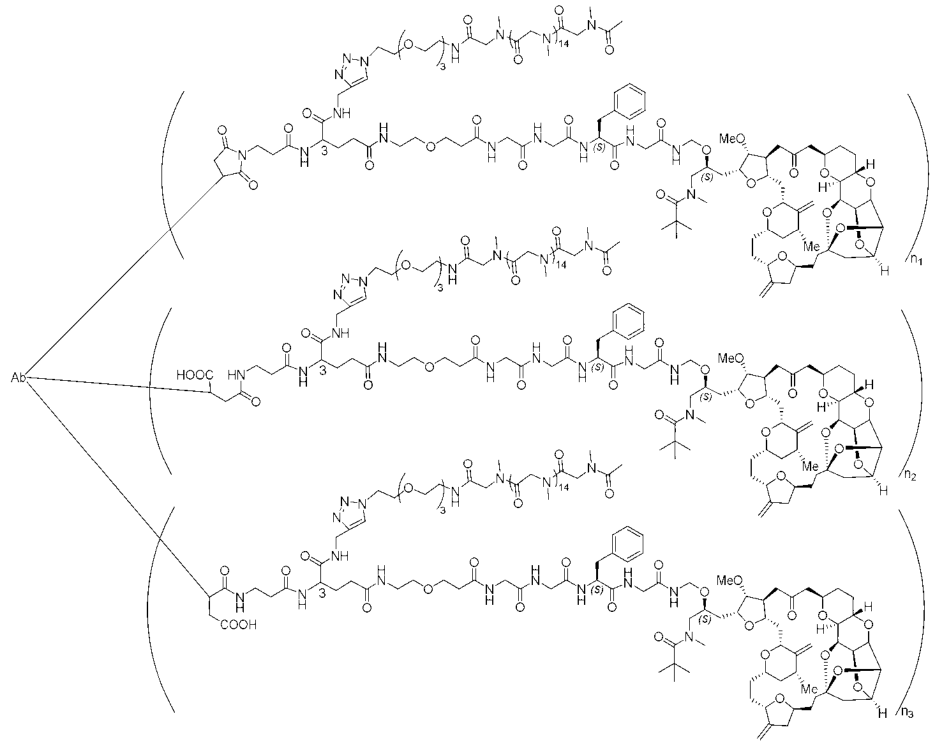

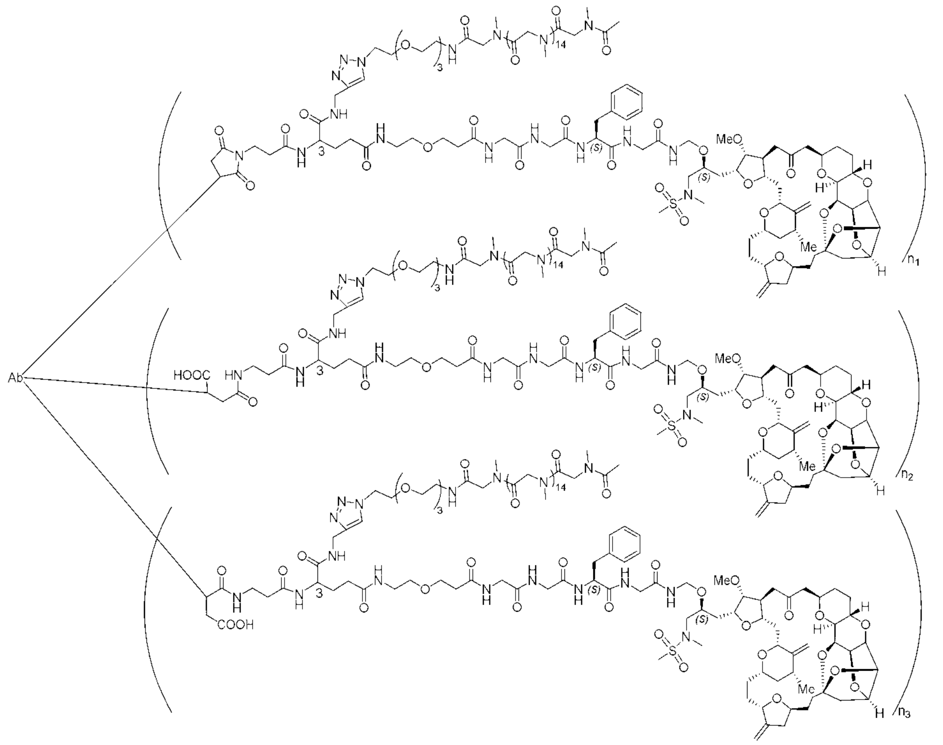

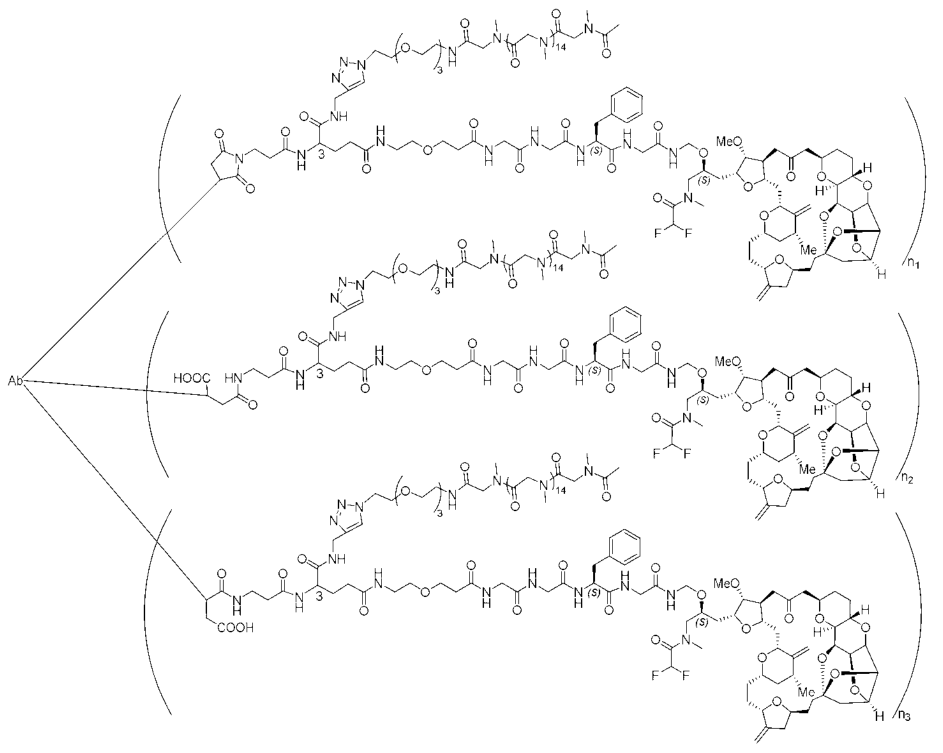

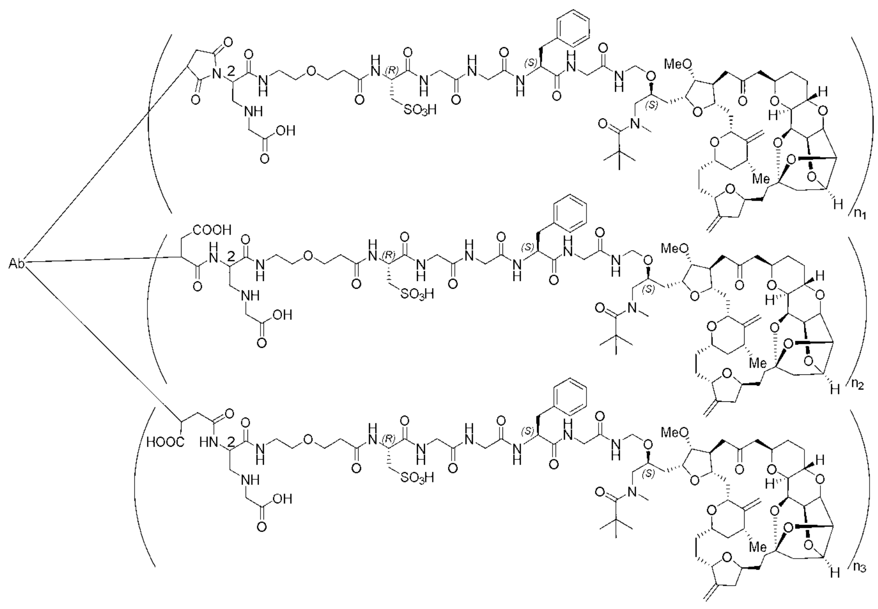

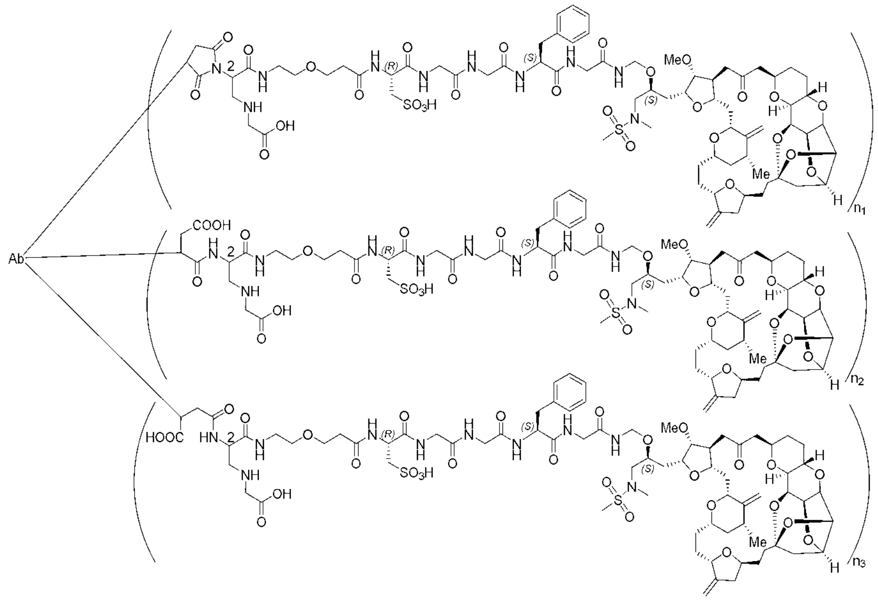

and

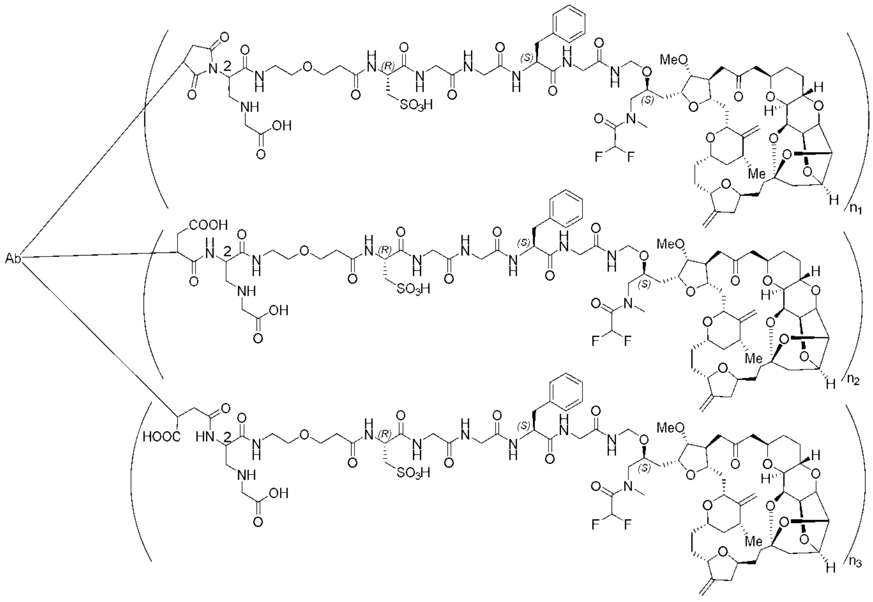

,

wherein chiral carbons at positions 2 and 3 independently have configuration R or S; preferably, the chiral carbon at position 2 has configuration S, and the chiral carbon at position 3 has configuration S.

**22.** The ligand-drug conjugate or the pharmaceutically acceptable salt or solvate thereof according to any one of claims 1-21, wherein the ligand unit Ab is an antibody, the antibody is anti-TROP-2 antibody;

preferably, the antibody consists of heavy chains and light chains, the light chains comprise CDR-L1, CDR-L2 and CDR-L3 having amino acid sequences set forth in SEQ ID NO:1, SEQ ID NO:2, and SEQ ID NO:3;
preferably, the heavy chains comprise CDR-H1, CDR-H2 and CDR-H3 having amino acid sequences set forth in SEQ ID NO:4, SEQ ID NO:5, and SEQ ID NO:6;
preferably, the light chain comprises a light chain variable region having an amino acid sequence of SEQ ID NO:7; more preferably, the light chain further comprises a light chain constant region having an amino acid sequence of SEQ ID NO:8;
preferably, the amino acid sequence of the light chain is SEQ ID NO:9;
preferably, the light chain comprises a light chain variable region having an amino acid sequence of SEQ ID NO:10; more preferably, the light chain further comprises a light chain constant region having an amino acid sequence of SEQ ID NO:11;
preferably, the amino acid sequence of the light chain is SEQ ID NO:12;
preferably, the heavy chain comprises a heavy chain variable region having an amino acid sequence of SEQ ID NO:13; more preferably, the heavy chain further comprises a heavy chain constant region having an amino acid sequence of SEQ ID NO:14;
preferably, the amino acid sequence of the heavy chain is SEQ ID NO:15.

**23.** A linker-drug conjugate of formula VIII or formula IX, or an isomer, mesomer, racemate, or enantiomer thereof, or a mixture thereof, or a pharmaceutically acceptable salt or solvate thereof,

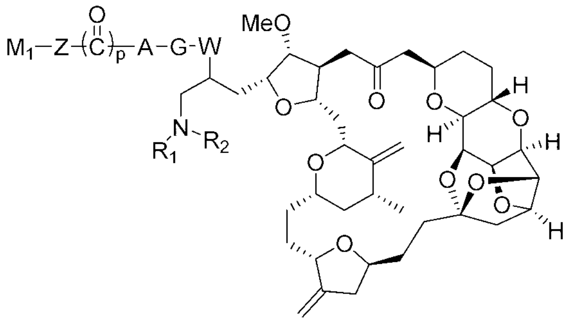

Formula VIII

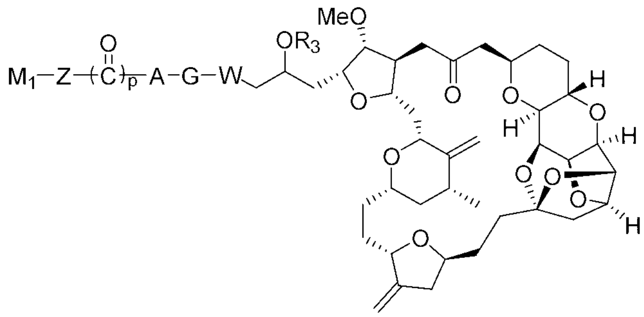

Formula IX

wherein,

$M_1$ is a linker unit, and
the Z, p, A, G, W, $R_1$, $R_2$ and $R_3$ are as defined in any one of claims 1-13.

**24.** The linker-drug conjugate, or the isomer, mesomer, racemate, or enantiomer thereof, or the mixture thereof, or the pharmaceutically acceptable salt or solvate thereof according to claim 23, wherein it has a structure represented by formula VIII-1 or formula IX-1:

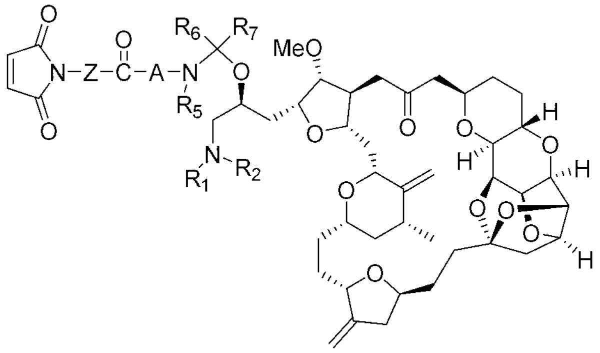

Formula VIII-1

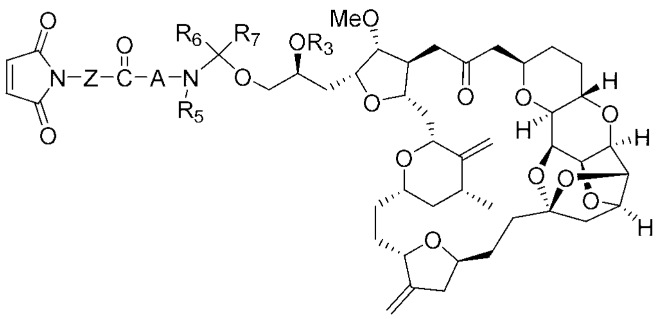

Formula IX-1

wherein,
the Z, A, $R_1$, $R_2$, $R_3$, $R_5$, $R_6$ and $R_7$ are as defined in any one of claims 1-13.

**25.** The linker-drug conjugate, or the isomer, mesomer, racemate, or enantiomer thereof, or the mixture thereof, or the pharmaceutically acceptable salt or solvate thereof according to claim 23 or 24, wherein it has a structure represented by formula VIIIa or formula IXa:

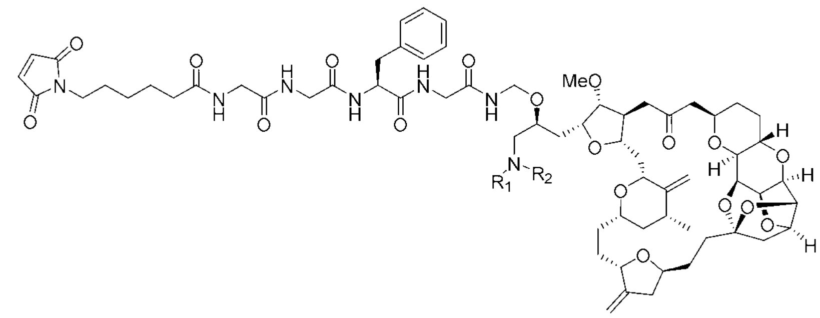

Formula VIIIa

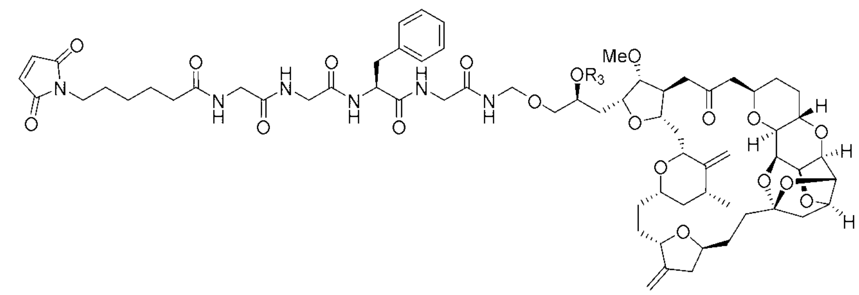

Formula IXa

wherein,
the $R_1$, $R_2$ and $R_3$ are as defined in any one of claims 1-13.

**26.** The linker-drug conjugate, or the isomer, mesomer, racemate, or enantiomer thereof, or the mixture thereof, or the pharmaceutically acceptable salt or solvate thereof according to claim 23 or 24, wherein it has a structure represented by formula VIIIb or formula IXb:

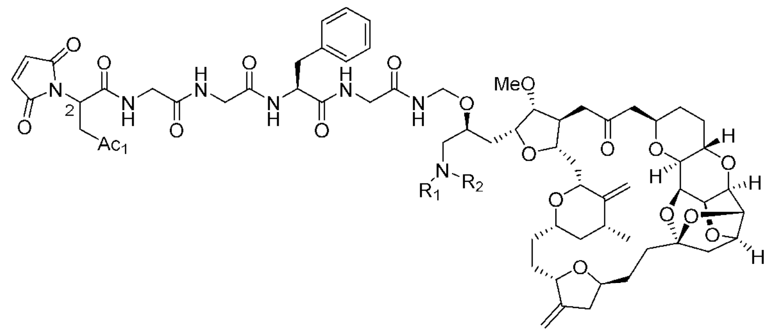

Formula VIIIb

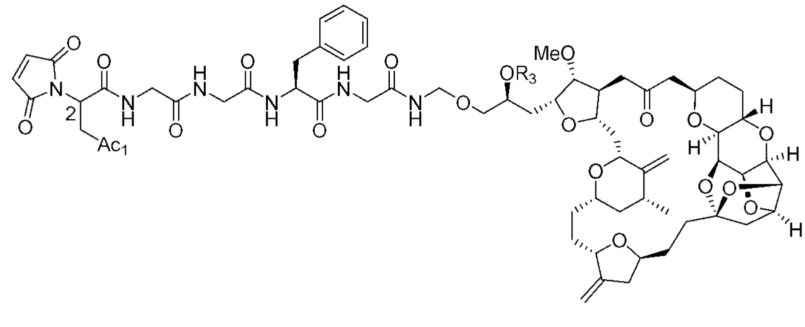

Formula IXb

wherein,

the $R_1$, $R_2$ and $R_3$ are as defined in any one of claims 1-13;
$Ac_1$ is a hydrophilic structural unit having the structure shown in formula d:

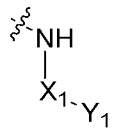

Formula d

$X_1$ and $Y_1$ are as defined in claim 4;
preferably, $Ac_1$ is as defined in claim 15.

**27.** The linker-drug conjugate, or the isomer, mesomer, racemate, or enantiomer thereof, or the mixture thereof, or the pharmaceutically acceptable salt or solvate thereof according to claim 23 or 24, wherein it has a structure represented by formula VIIIc:

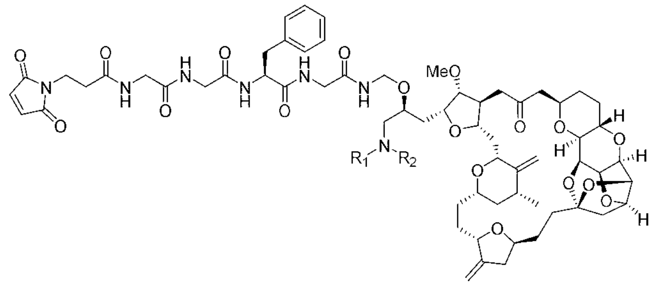

VIIIc

wherein,
the $R_1$ and $R_2$ are as defined in any one of claims 1-13.

**28.** The linker-drug conjugate, or the isomer, mesomer, racemate, or enantiomer thereof, or the mixture thereof, or the pharmaceutically acceptable salt or solvate thereof according to claim 23 or 24, wherein it has a structure represented by formula VIIId:

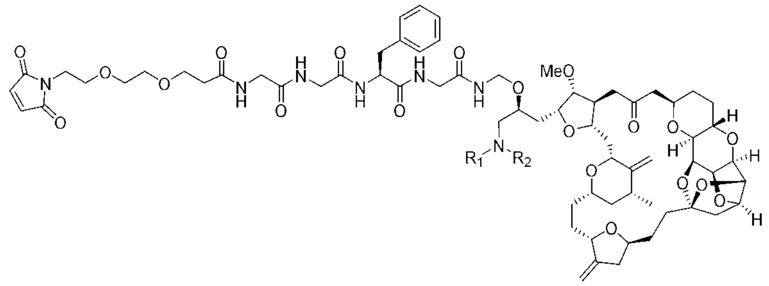

VIIId

wherein,
the $R_1$ and $R_2$ are as defined in any one of claims 1-13.

**29.** The linker-drug conjugate, or the isomer, mesomer, racemate, or enantiomer thereof, or the mixture thereof, or the pharmaceutically acceptable salt or solvate thereof according to claim 23 or 24, wherein it has a structure represented by formula VIIIe:

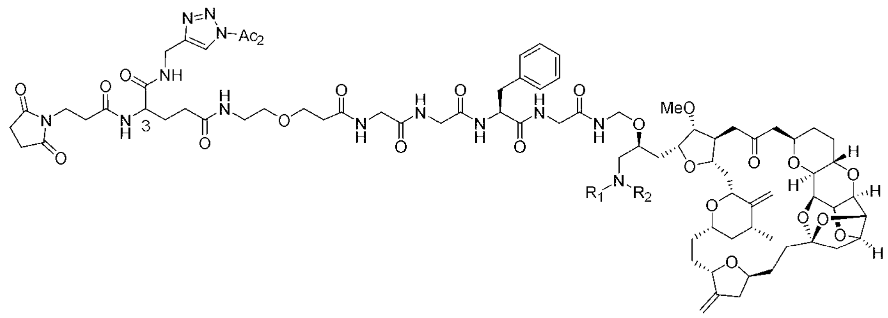

Formula VIIIe

wherein,

the $R_1$ and $R_2$ are as defined in any one of claims 1-13;
$Ac_2$ is a hydrophilic structural unit composed of $-X_2-Y_2$, $X_2$ and $Y_2$ are as defined in claim 4; preferably, $Ac_2$ is as defined in claim 9 or 18.

**30.** The linker-drug conjugate, or the isomer, mesomer, racemate, or enantiomer thereof, or the mixture thereof, or the pharmaceutically acceptable salt or solvate thereof according to claim 23 or 24, wherein it has a structure represented by formula VIIIf:

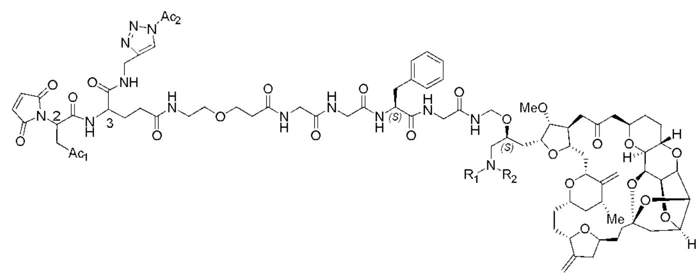

Formula VIIIf

wherein, the $R_1$ and $R_2$ are as defined in any one of claims 1-13;
$Ac_1$ is a hydrophilic structural unit composed of $-X_1-Y_1$, and $X_1$ and $Y_1$ are as defined in claim 4; preferably, $Ac_1$ is as defined in claim 15;
$Ac_2$ is a hydrophilic structural unit composed of $-X_2-Y_2$, and $X_2$ and $Y_2$ are as defined in claim 4; preferably, $Ac_2$ is as defined in claim 9 or 18.

**31.** The linker-drug conjugate, or the isomer, mesomer, racemate, or enantiomer thereof, or the mixture thereof, or the pharmaceutically acceptable salt or solvate thereof according to claim 23 or 24, wherein it has a structure represented by formula VIIIg:

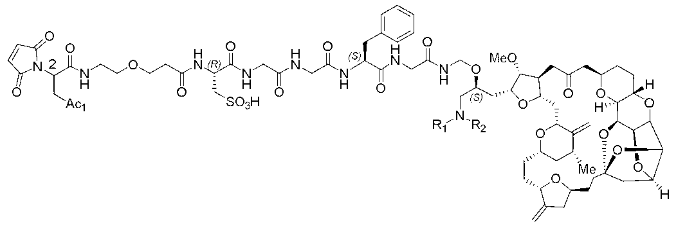

Formula VIIIg

wherein, the $R_1$ and $R_2$ are as defined in any one of claims 1-13;
$Ac_1$ is a hydrophilic structural unit composed of-$X_1-Y_1$, and $X_1$ and $Y_1$ are as defined in claim 4; preferably, $Ac_1$ is as defined in claim 15.

**32.** The linker-drug conjugate, or the isomer, mesomer, racemate, or enantiomer thereof, or the mixture thereof, or the pharmaceutically acceptable salt or solvate thereof according to any one of claims 23-31, wherein it is selected from the following structures:

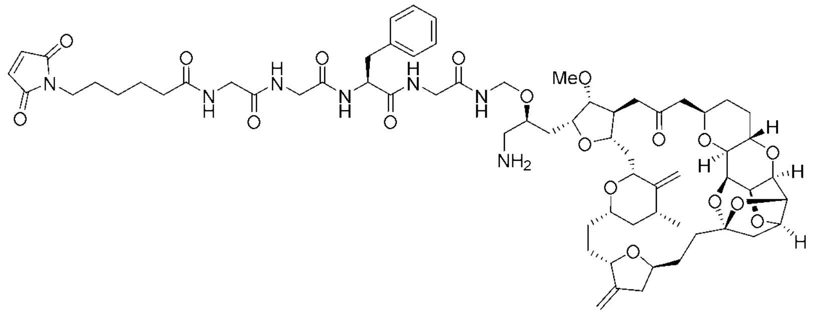

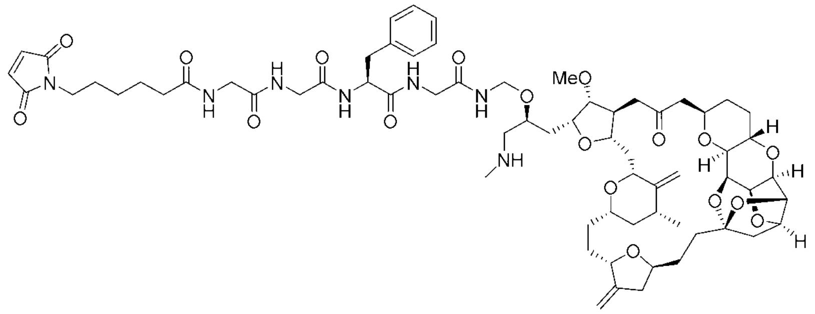

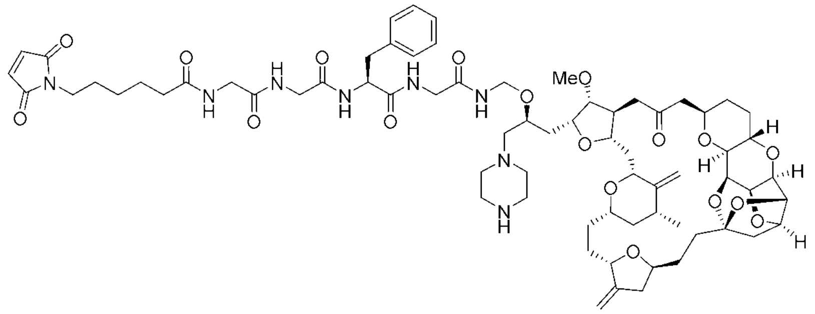

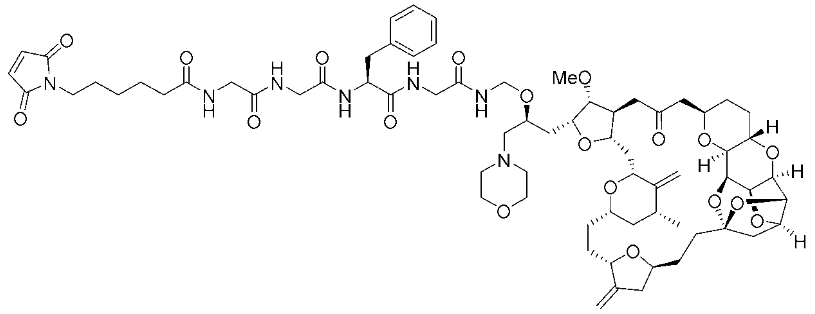

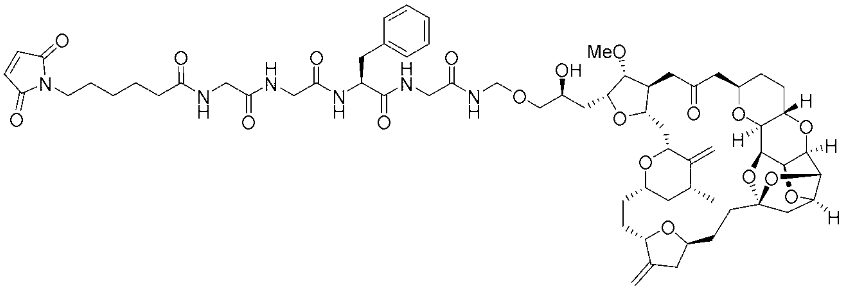

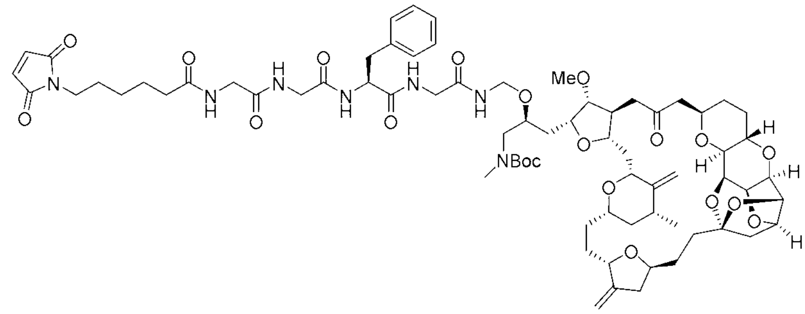

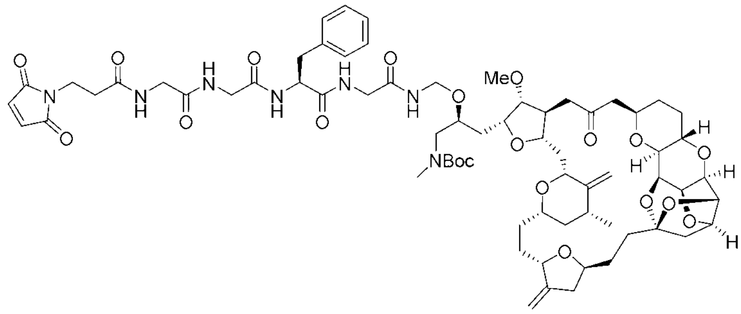

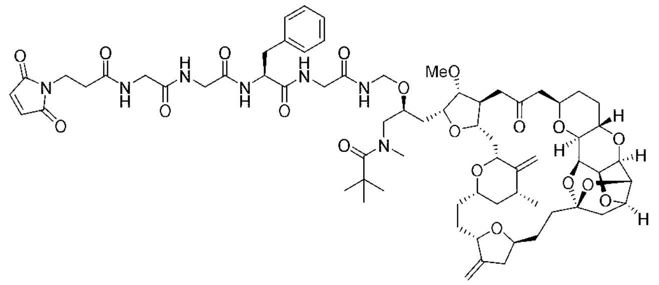

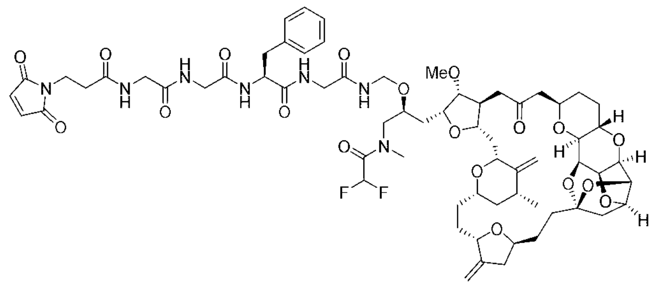

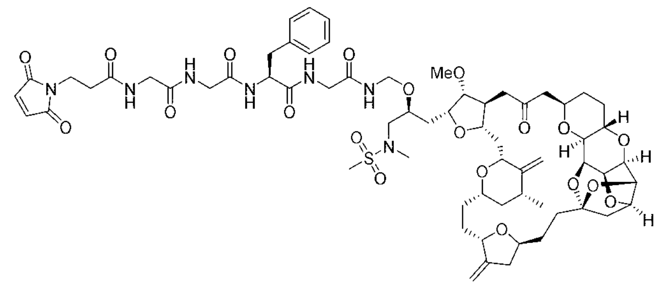

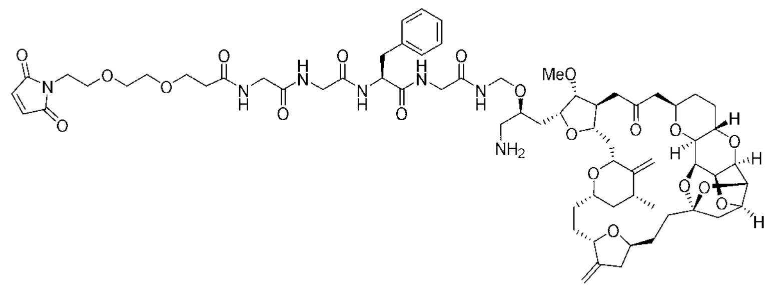

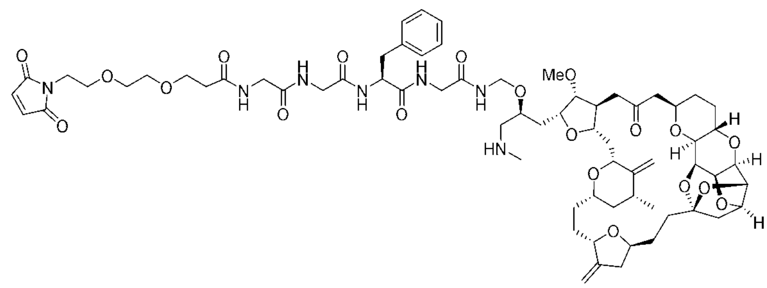

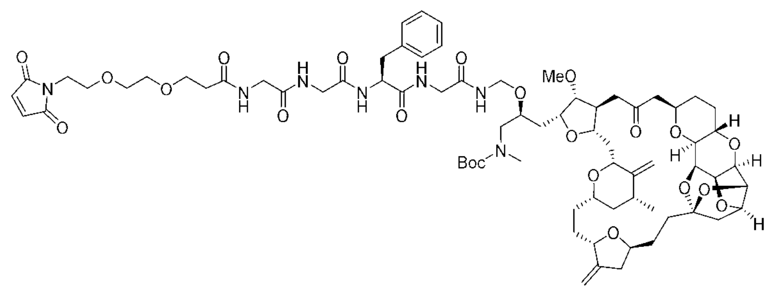

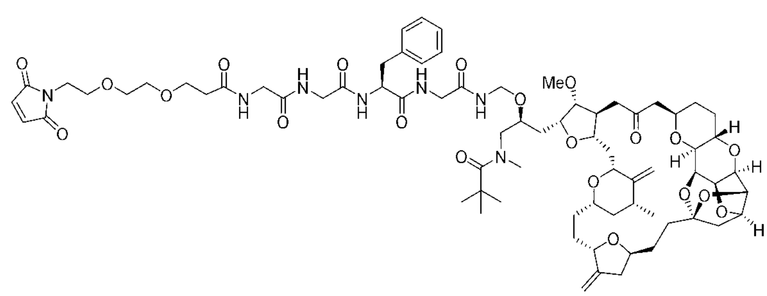

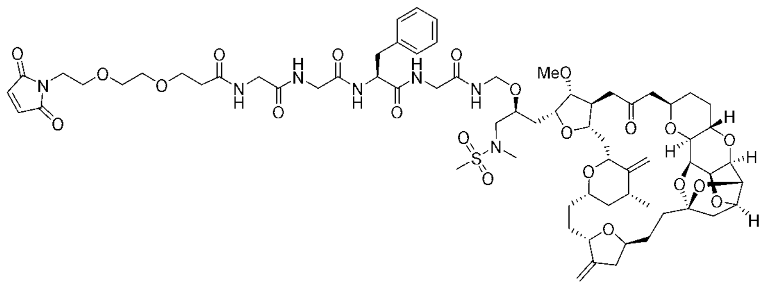

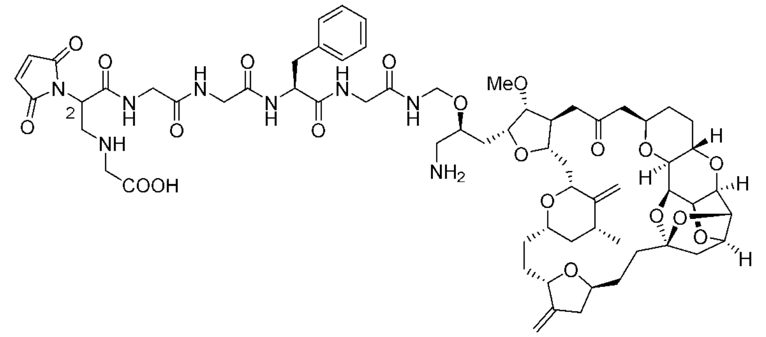

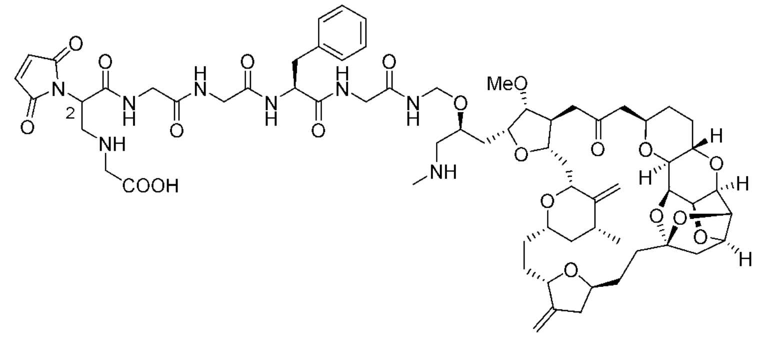

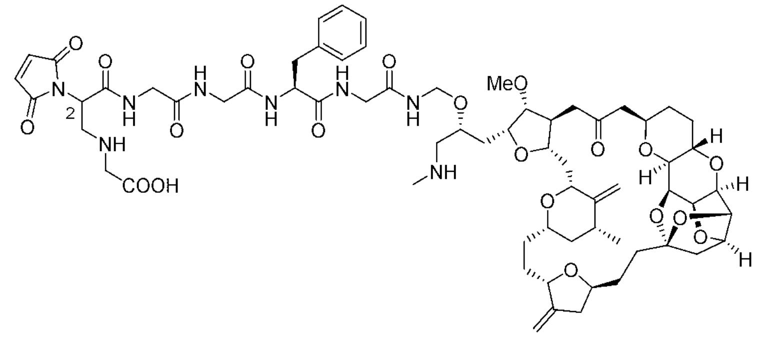

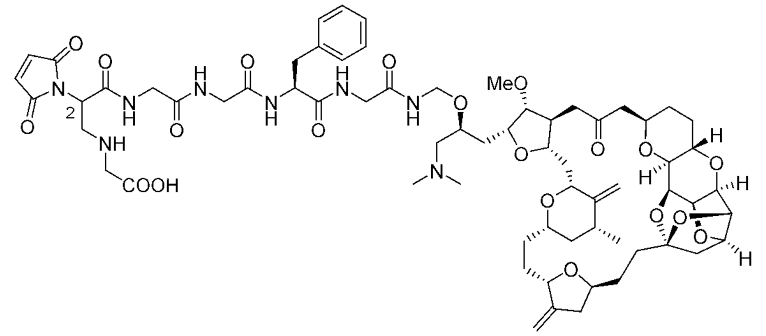

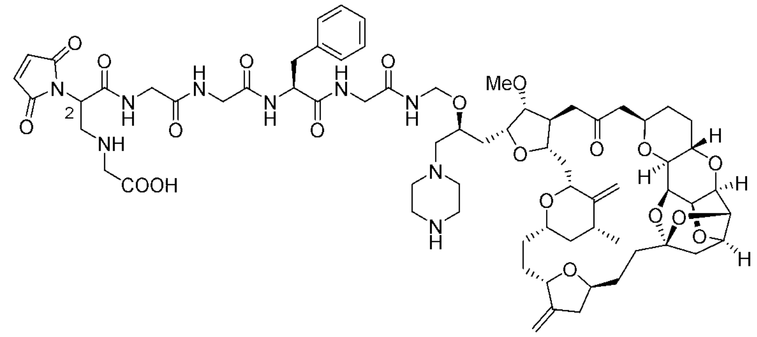

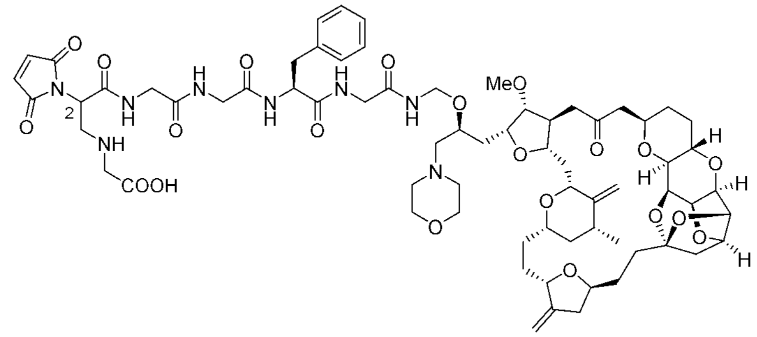

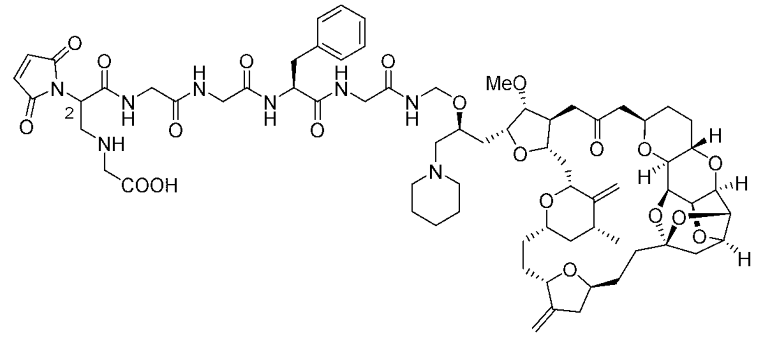

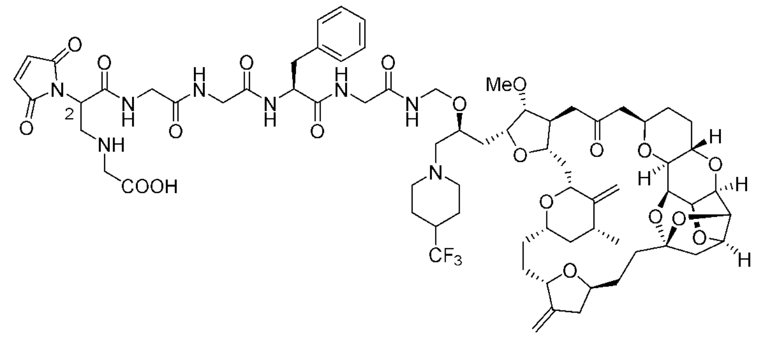

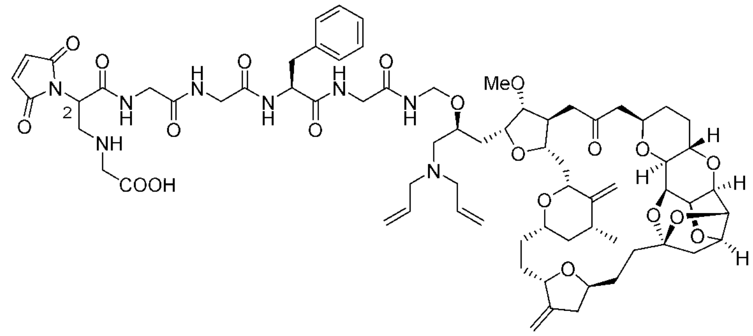

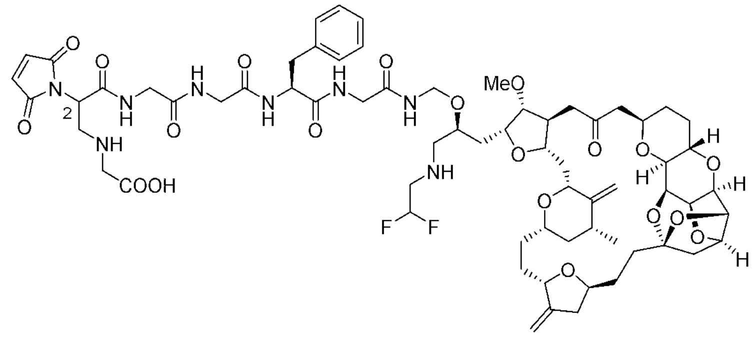

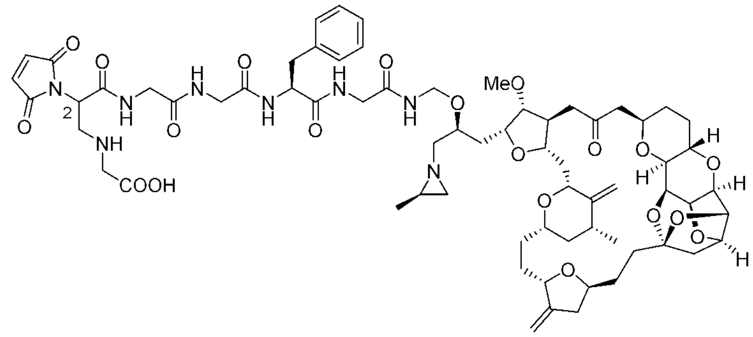

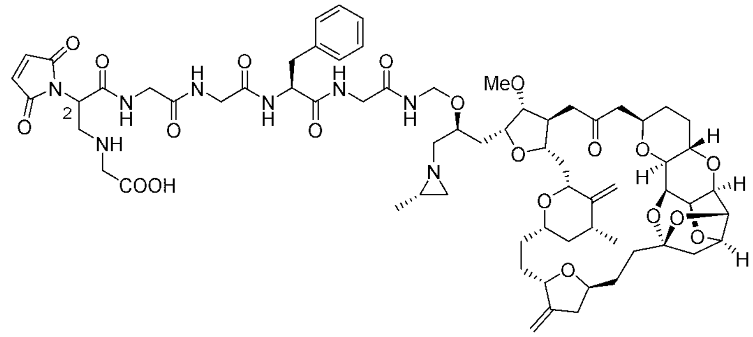

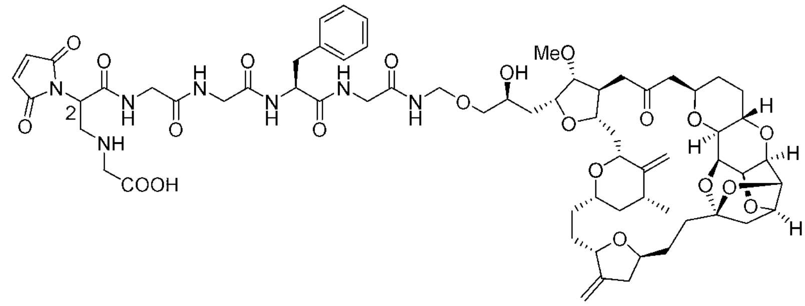

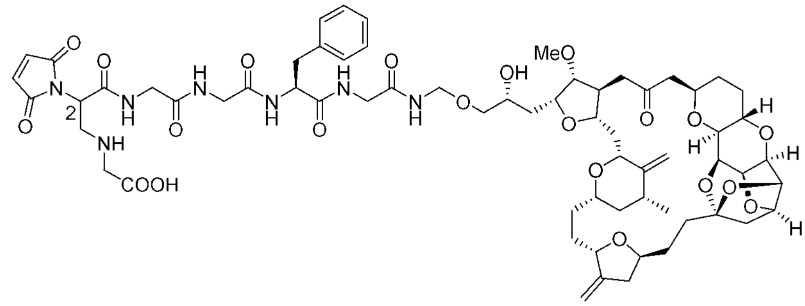

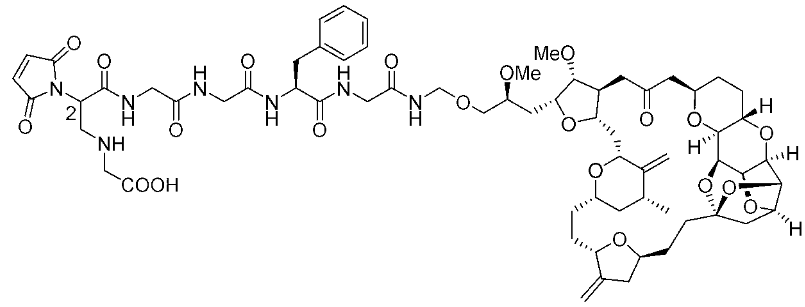

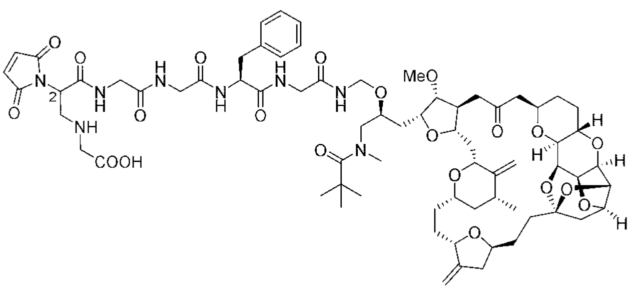

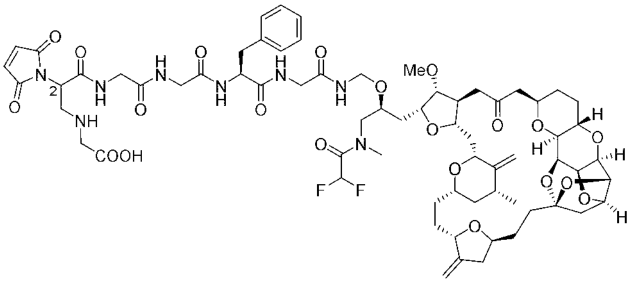

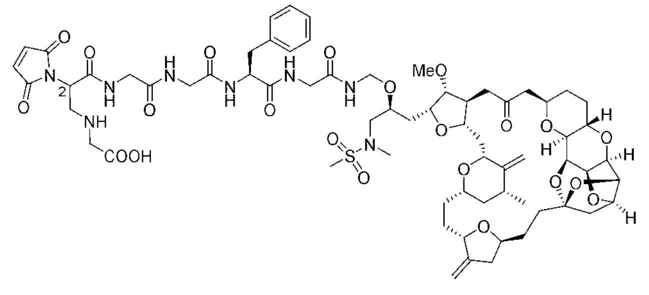

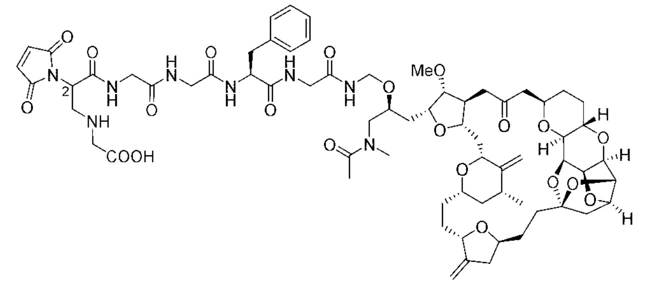

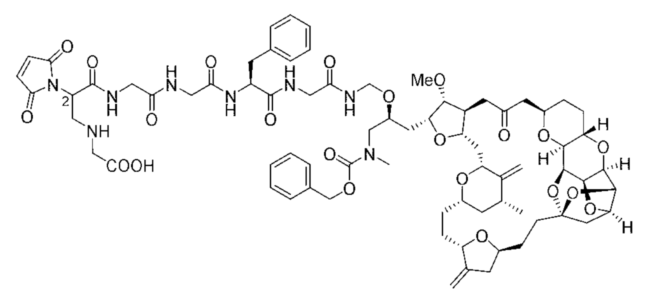

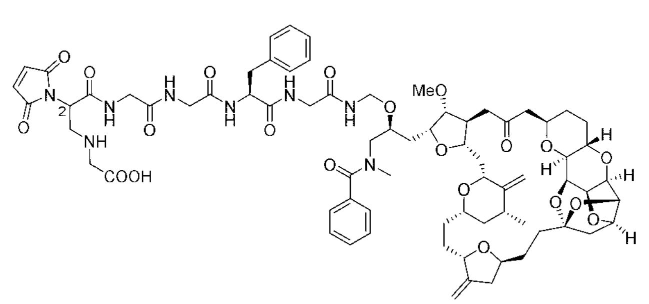

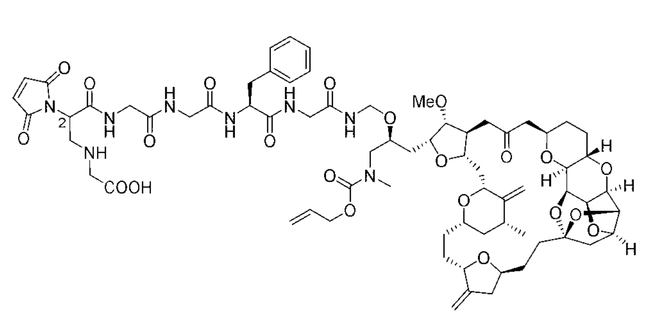

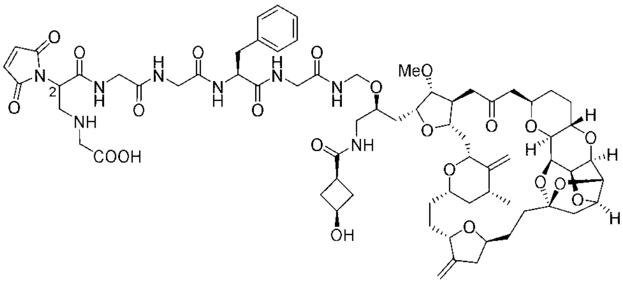

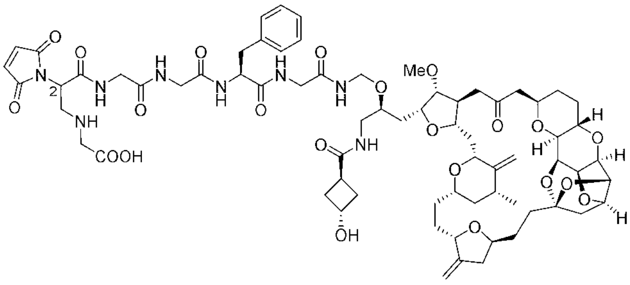

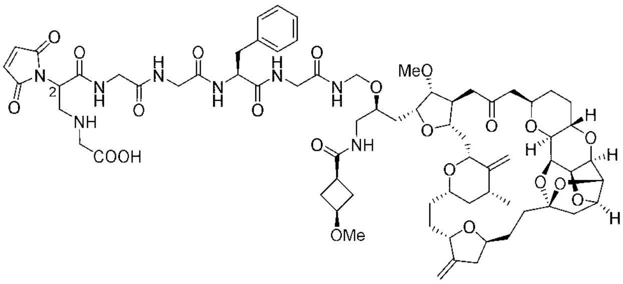

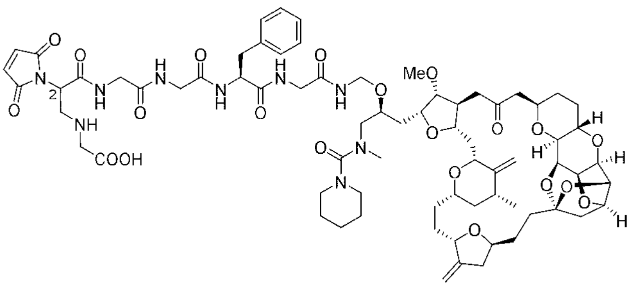

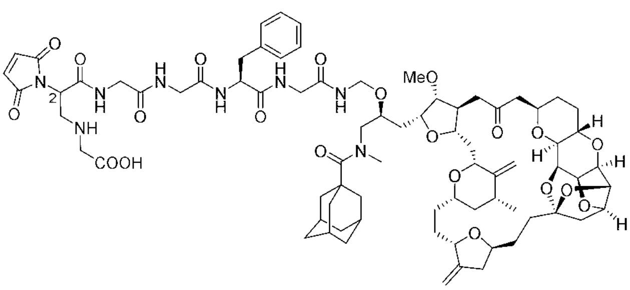

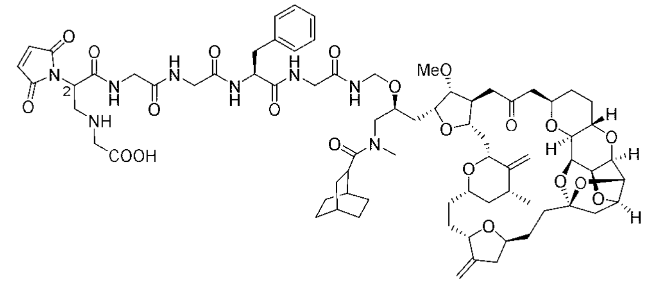

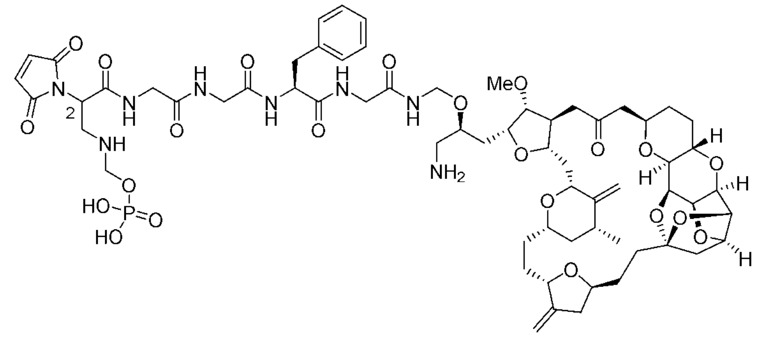

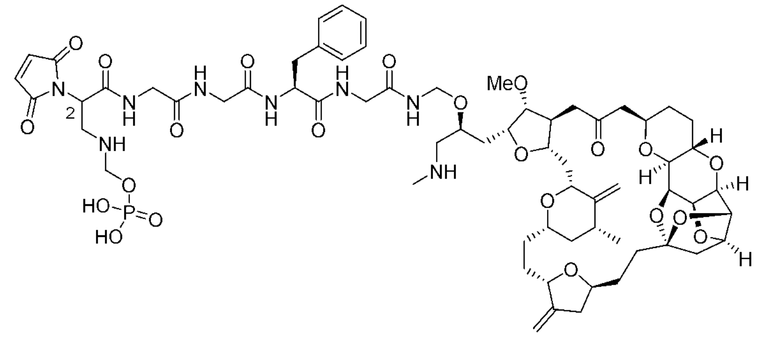

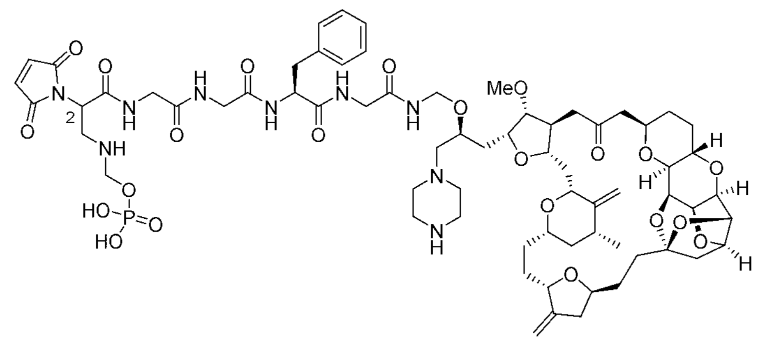

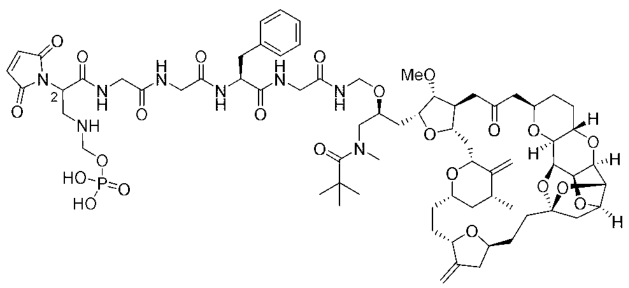

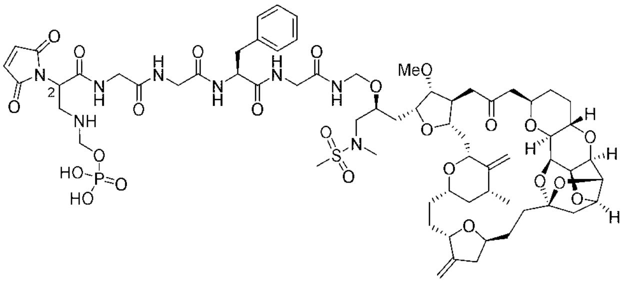

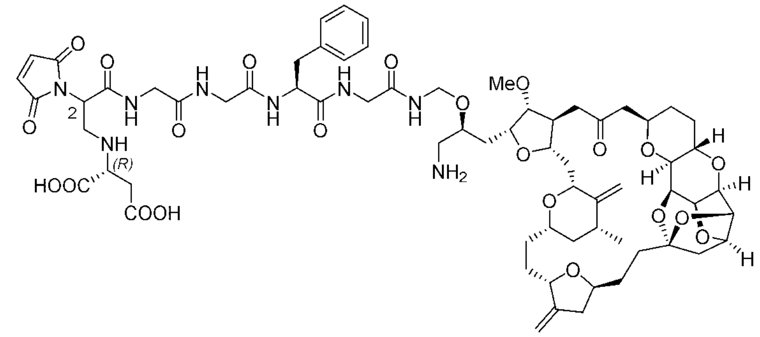

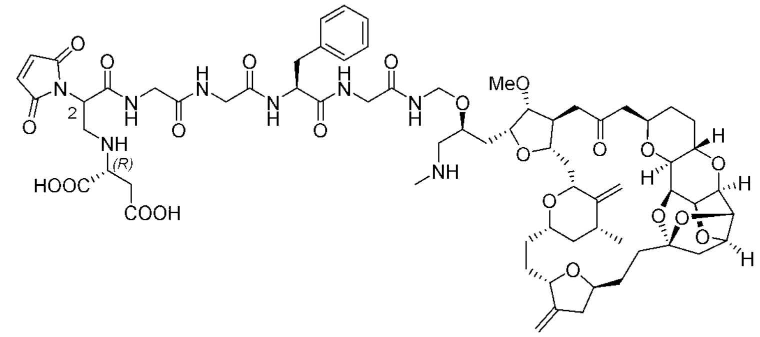

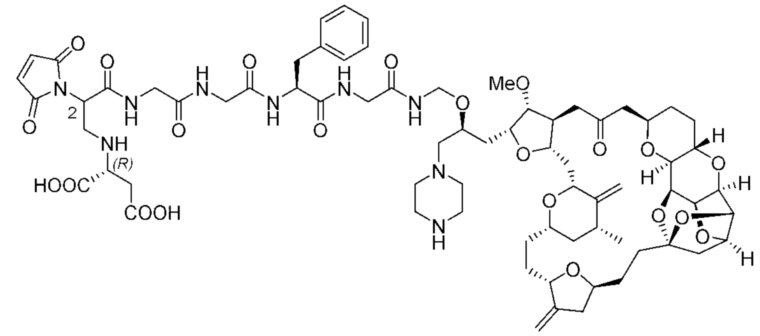

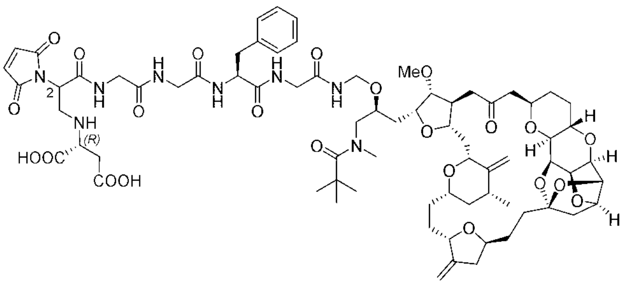

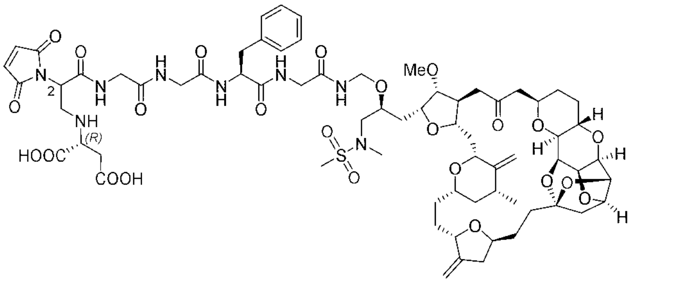

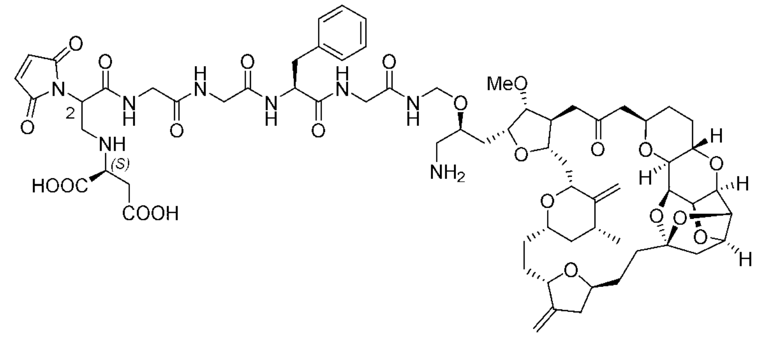

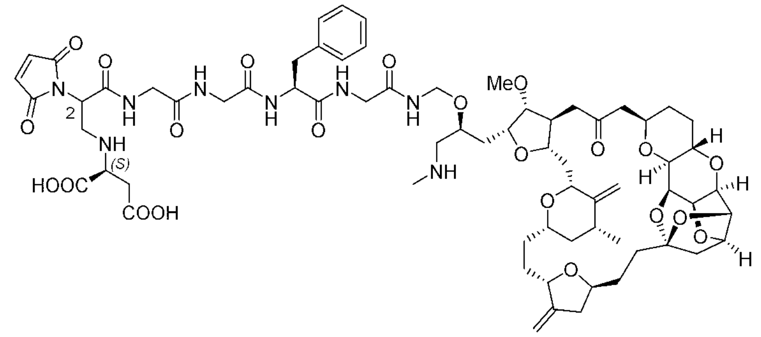

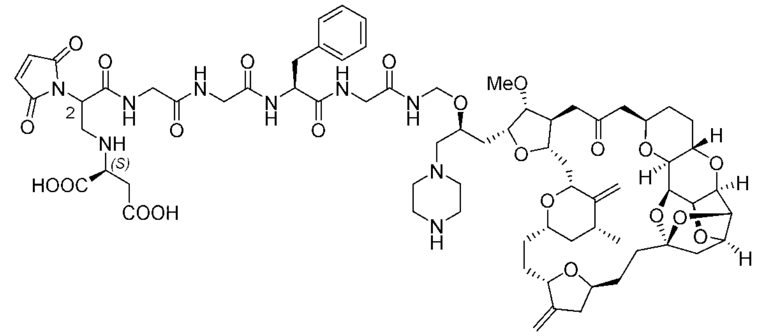

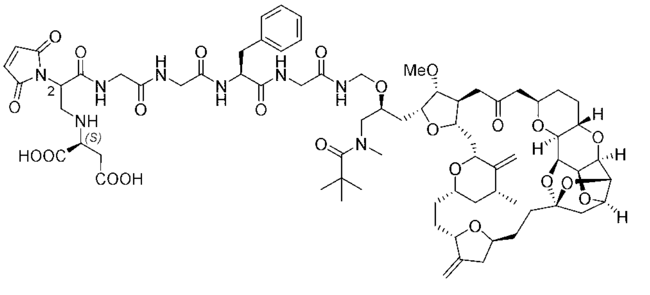

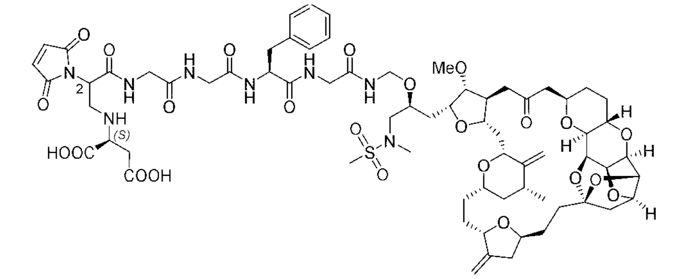

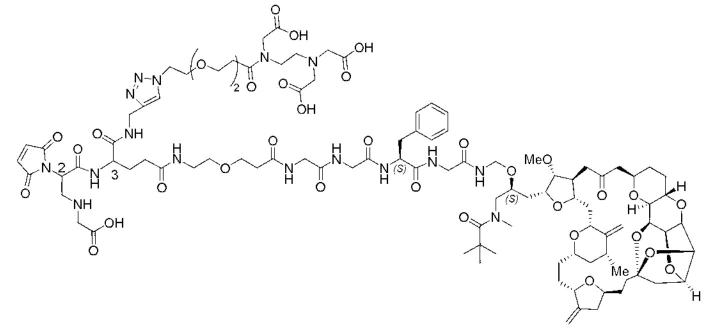

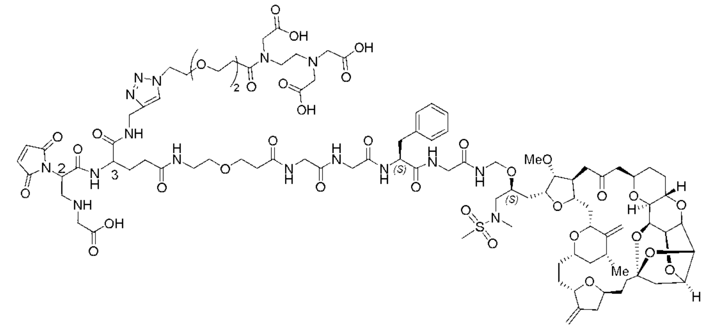

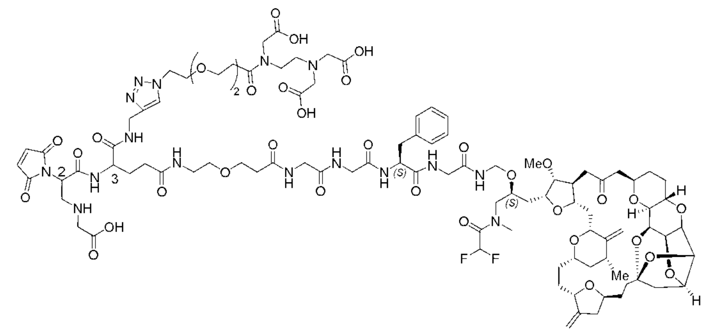

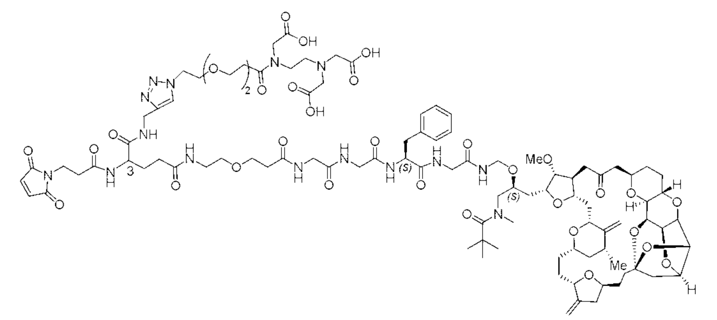

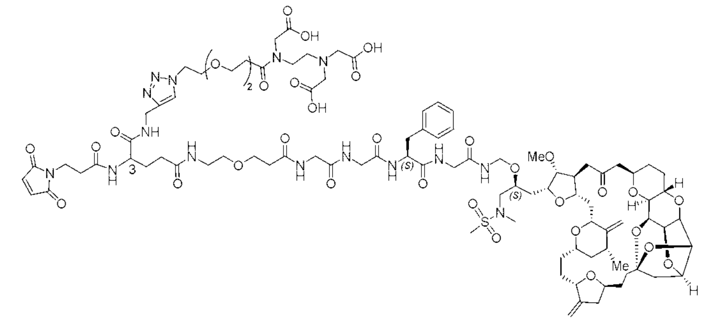

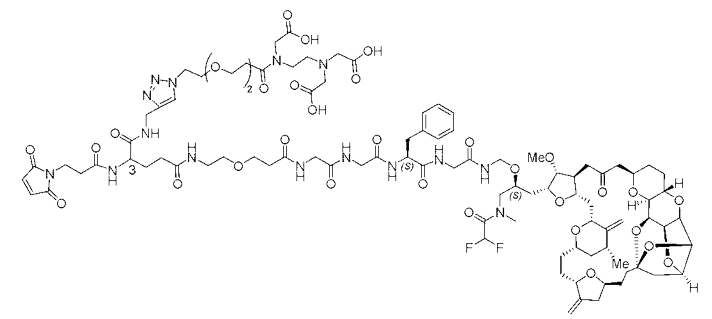

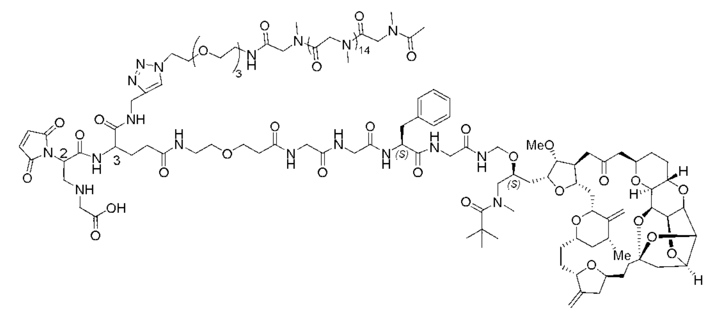

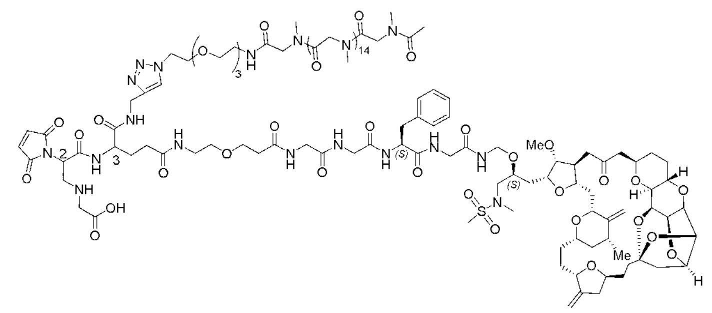

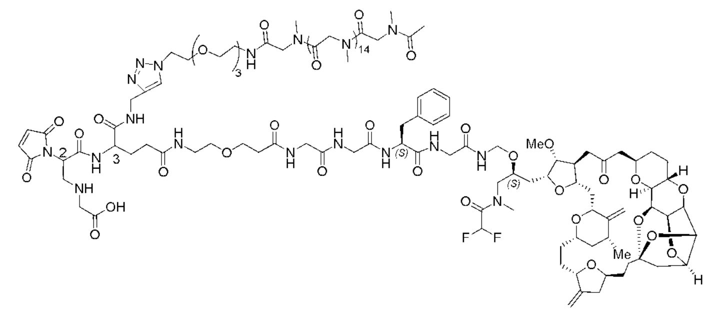

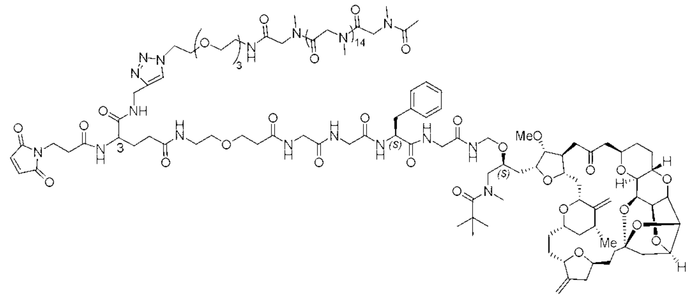

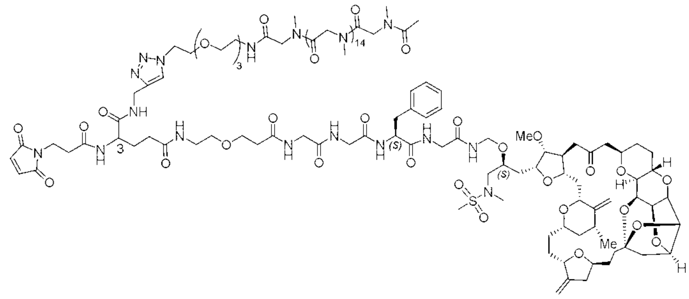

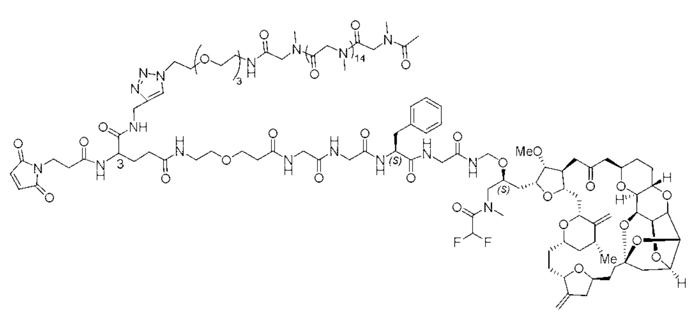

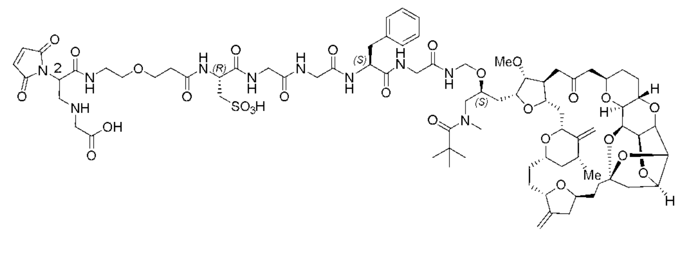

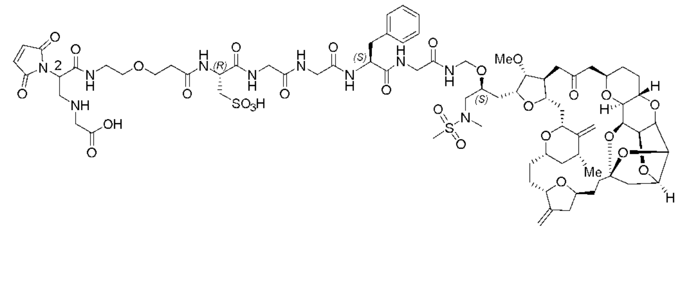

and

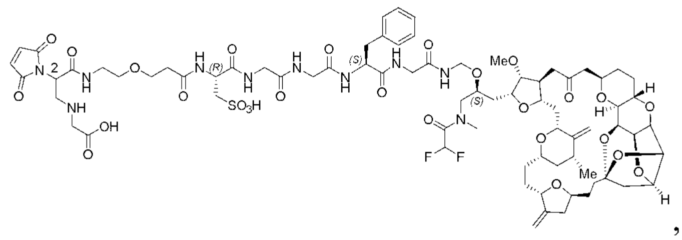

,

wherein chiral carbons at positions 2 and 3 independently have configuration R or S; preferably, the chiral carbon at position 2 has configuration S, and the chiral carbon at position 3 has configuration S.

**33.** A compound of formula X, or an isomer, mesomer, racemate, or enantiomer thereof, or a mixture thereof, or a pharmaceutically acceptable salt or solvate thereof,

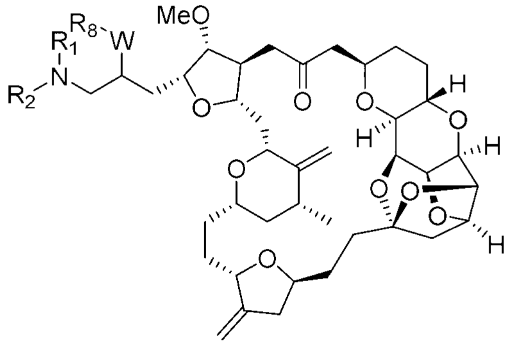

Formula X

wherein W is an oxygen atom or a sulfur atom, and preferably, W is an oxygen atom;

$R_1$ and $R_8$ are each independently selected from the group consisting of: hydrogen atom, alkyl, acyl, sulfonyl, cycloalkyl, heterocyclyl, aryl and heteroaryl, and are not simultaneously hydrogen; optionally, the alkyl, acyl, sulfonyl, cycloalkyl, aryl and heteroaryl are each independently substituted with one or more substituents selected from the group consisting of hydrogen atom, alkyl, alkoxy, halogen, deuterium, amino, cyano, hydroxy, mercapto, azido, nitro, carboxy, acyl, carbonyl, hydroxyalkyl, cycloalkyl, heterocyclyl, aryl and heteroaryl; preferably, $R_1$ is alkyl, and $R_8$ is H;

$R_2$ is -C(O)-$Q_1$-$Q_2$ or -$SO_2$-$Q_1$-$Q_2$, wherein $Q_1$ is selected from the group consisting of O, N and S atoms and a chemical bond;

in a case where $Q_1$ is selected from the group consisting of O, N and S atoms, $Q_2$ is selected from the group consisting of alkyl, cycloalkyl, heterocyclyl, spirocyclyl, bridged ring, alkenyl, aryl and heteroaryl, and optionally the alkyl, cycloalkyl, heterocyclyl, spirocyclyl, bridged ring, alkenyl, aryl and heteroaryl are each independently substituted with one or more substituents selected from the group consisting of hydrogen atom, alkyl, alkoxy, halogen, deuterium, amino, cyano, hydroxy, mercapto, azido, nitro, carboxy, acyl, carbonyl, hydroxyalkyl, cycloalkyl, heterocyclyl, aryl and heteroaryl;

in a case where $Q_1$ is a chemical bond, $Q_2$ is selected from the group consisting of alkyl, benzyl, cycloalkyl, heterocyclyl, spirocyclyl, bridged ring, alkenyl, aryl and heteroaryl;

in a case where $Q_2$ is an alkyl, the alkyl is substituted with one or more substituents selected from the group consisting of hydrogen atom, alkyl, alkoxy, halogen, deuterium, cyano, azido, nitro, carboxyl, acyl, carbonyl, cycloalkyl, heterocyclyl, aryl and heteroaryl;

in a case where $Q_2$ is selected from the group consisting of cycloalkyl, heterocyclyl, spirocyclyl, bridged ring, alkenyl, aryl and heteroaryl, the cycloalkyl, heterocyclyl, spirocyclyl, bridged ring, alkenyl, aryl and heteroaryl are each independently substituted with one or more substituents selected from the group consisting of hydrogen atom, alkyl, alkoxy, halogen, deuterium, amino, cyano, hydroxy, mercapto, azido, nitro, carboxyl, acyl, carbonyl, hydroxyalkyl, cycloalkyl, heterocyclyl, aryl and heteroaryl, and in a case where $Q_2$ is a cycloalkyl with the substituent being a hydroxyl, an amino or a mercapto, the substituent is at any position except a carbon atom connected to -C(O)-.

**34.** The compound of formula X, or the isomer, mesomer, racemate, or enantiomer thereof, or the mixture thereof, or the

pharmaceutically acceptable salt or solvate thereof according to claim 33, wherein it is a compound of formula XIa or formula XIb, or an isomer, mesomer, racemate, or enantiomer thereof, or a mixture thereof,

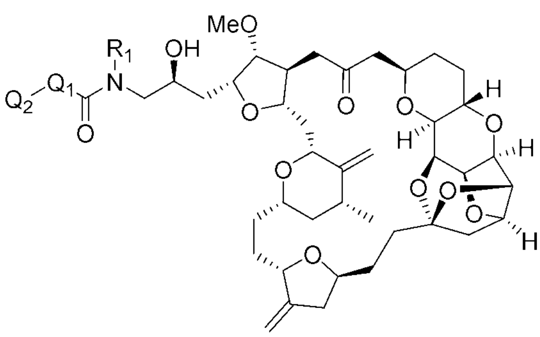

Formula XIa

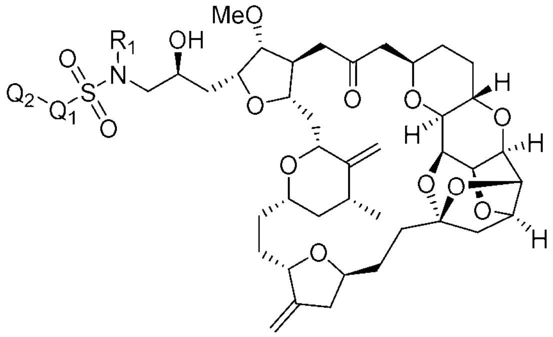

Formula XIb

wherein $R_1$ is an alkyl, and preferably $R_1$ is a methyl,
$Q_1$ is an O atom or a chemical bond;
in a case where $Q_1$ is an O atom, $Q_2$ is selected from the group consisting of alkyl, cycloalkyl, alkenyl and aryl, and optionally the alkyl, cycloalkyl, alkenyl and aryl are each independently substituted with one or more substituents selected from the group consisting of hydrogen atom, alkyl, halogen, hydroxy, hydroxyalkyl and aryl;
in a case where $Q_1$ is a chemical bond, $Q_2$ is selected from the group consisting of alkyl, benzyl, cycloalkyl, alkenyl and aryl;
in a case where $Q_2$ is an alkyl, the alkyl is substituted with one or more substituents selected from the group consisting of hydrogen atom, alkyl, halogen, deuterium, azido, nitro and aryl; and
in a case where $Q_2$ is selected from the group consisting of cycloalkyl, alkenyl and aryl, the cycloalkyl, alkenyl and aryl are each independently substituted with one or more substituents selected from the group consisting of hydrogen atom, alkyl, halogen, hydroxyl, hydroxyalkyl and aryl, and in a case where $Q_2$ is a cycloalkyl with the substituent being a hydroxyl, an amino or a mercapto, the substituent is at any position except a carbon atom connected to -C(O)-.

**35.** The compound of formula X, or the isomer, mesomer, racemate, or enantiomer thereof, or the mixture thereof, or the pharmaceutically acceptable salt or solvate thereof according to claim 33 or 34, wherein it is selected from the following structures:

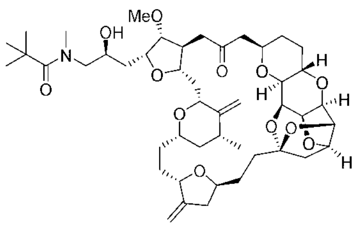

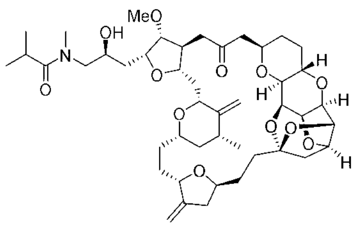

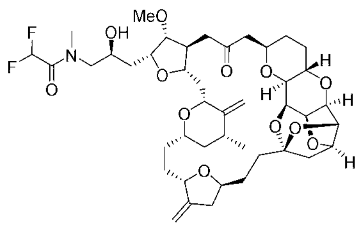

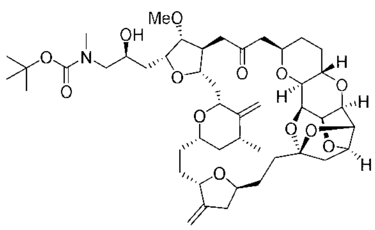

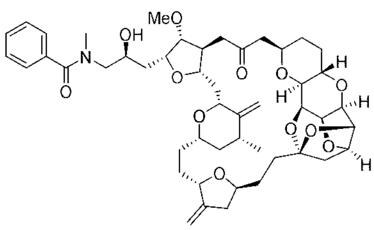

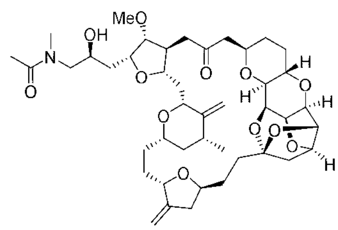

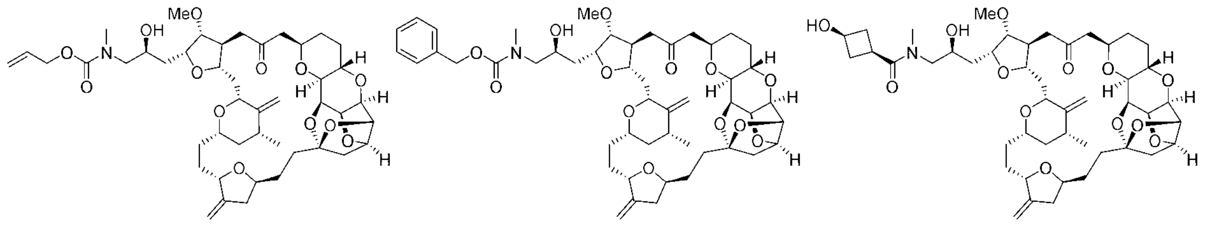

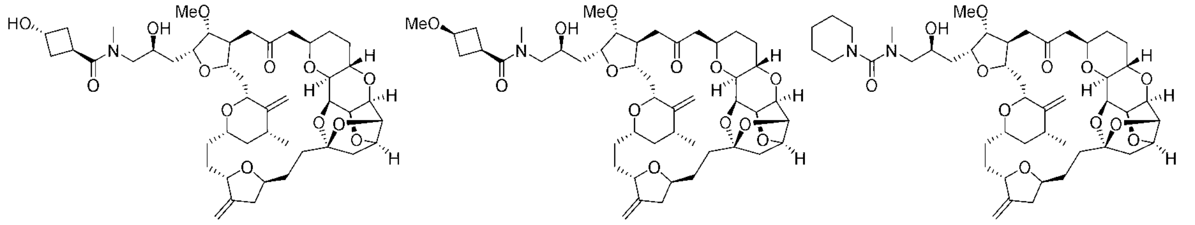

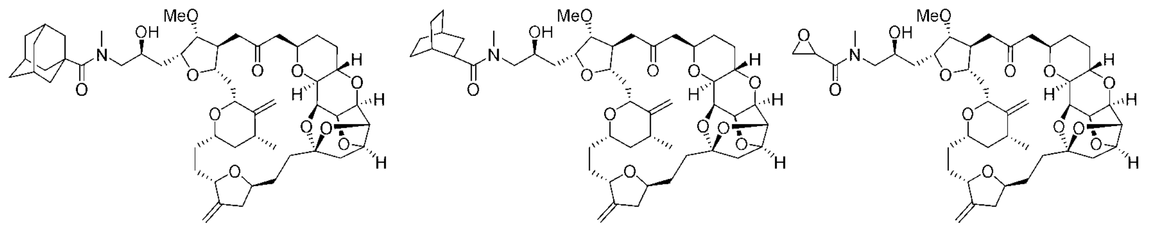

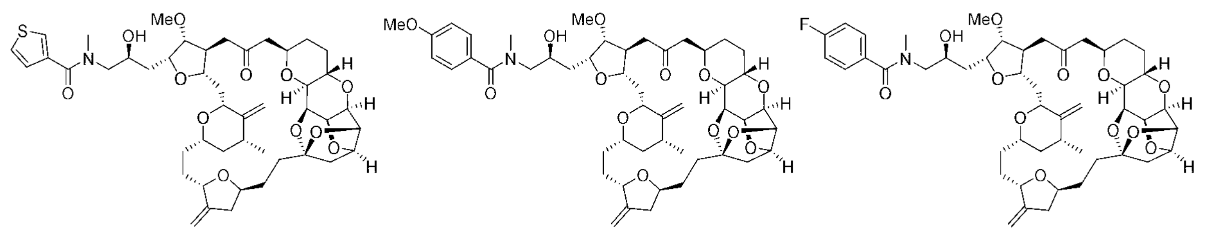

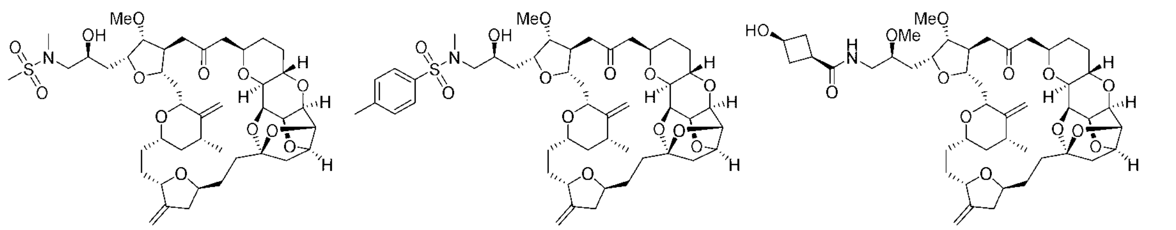

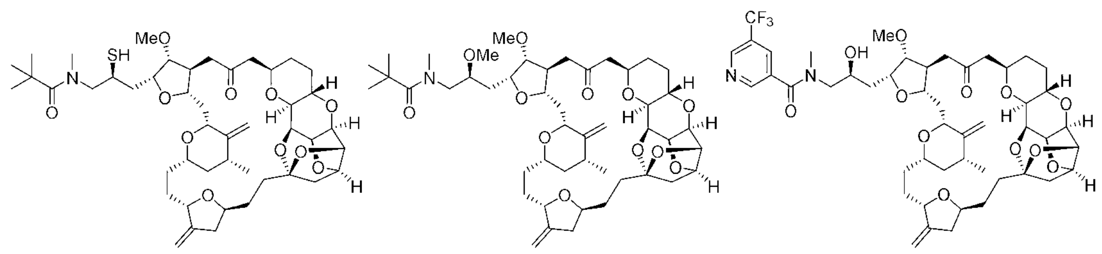

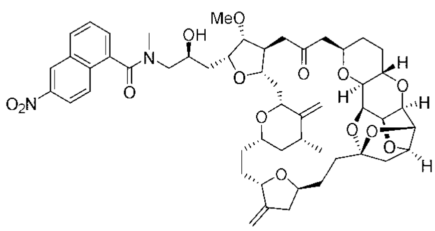
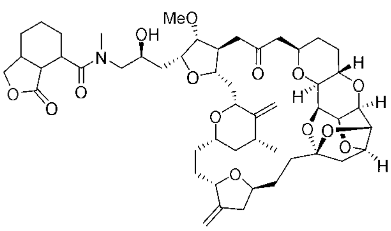
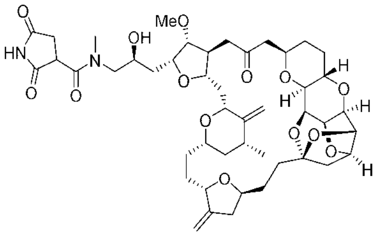

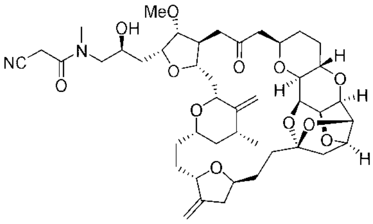
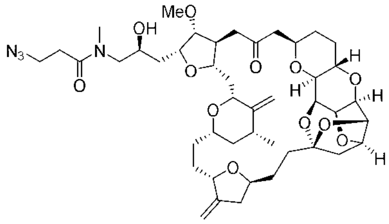
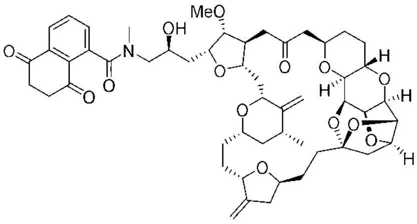

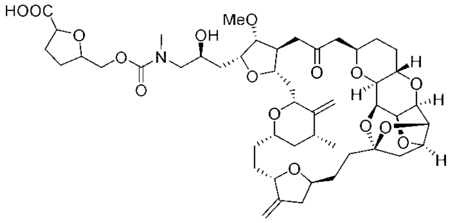
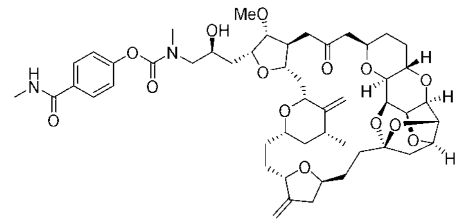

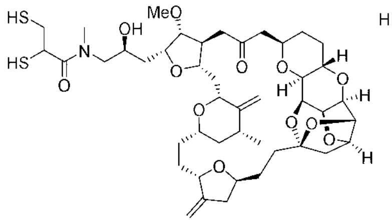
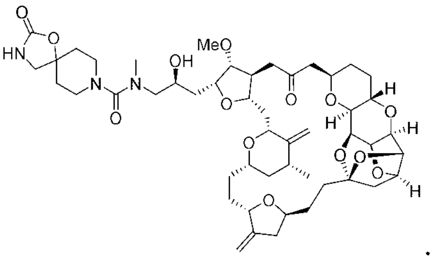

and

**36.** Use of the linker-drug conjugate, or the isomer, mesomer, racemate, or enantiomer thereof, or the mixture thereof, or the pharmaceutically acceptable salt or solvate thereof according to any one of claims 23-32, or the compound or the isomer, mesomer, racemate, or enantiomer thereof or the mixture thereof or the pharmaceutically acceptable salt or solvate thereof according to any one of claims 33-35 as an intermediate for the preparation of a ligand-drug conjugate, and the ligand-drug conjugate is described as in any one of claims 1-22.

**37.** The ligand-drug conjugate or the pharmaceutically acceptable salt or solvate thereof according to any one of claims 1-22, or the linker-drug conjugate, or the isomer, mesomer, racemate, or enantiomer thereof, or the mixture thereof, or the pharmaceutically acceptable salt or solvate thereof according to any one of claims 23-32, or the compound, or the isomer, mesomer, racemate, or enantiomer thereof, or the mixture thereof, or the pharmaceutically acceptable salt or solvate thereof according to any one of claims 33-35, wherein: the pharmaceutically acceptable salts include sodium, potassium, calcium or magnesium salts formed by acidic functional groups in the structural formula and acetates, trifluoroacetates, citrates, oxalates, tartrates, malates, nitrates, chlorides, bromides, iodides, sulfates, bisulfates, phosphates, lactates, oleates, ascorbates, salicylates, formates, glutamates, methanesulfonates, ethanesulfonates, benzenesulfonates or p-toluenesulfonates formed by basic functional groups in the structure.

**38.** A pharmaceutical composition, comprising the ligand-drug conjugate or the pharmaceutically acceptable salt or solvate thereof according to any one of claims 1-22, or the linker-drug conjugate, or the isomer, mesomer, racemate, or enantiomer thereof, or the mixture thereof, or the pharmaceutically acceptable salt or solvate thereof according to any one of claims 23-32, or the compound, or the isomer, mesomer, racemate, or enantiomer thereof, or the mixture thereof, or the pharmaceutically acceptable salt or solvate thereof according to any one of claims 33-35, and optionally

further comprising a pharmaceutically acceptable carrier.

**39.** A pharmaceutical formulation, comprising the ligand-drug conjugate or the pharmaceutically acceptable salt or solvate thereof according to any one of claims 1-22, or the linker-drug conjugate, or the isomer, mesomer, racemate, or enantiomer thereof, or the mixture thereof, or the pharmaceutically acceptable salt or solvate thereof according to any one of claims 23-32, or the compound, or the isomer, mesomer, racemate, or enantiomer thereof, or the mixture thereof, or the pharmaceutically acceptable salt or solvate thereof according to any one of claims 33-35.

**40.** Use of the ligand-drug conjugate or the pharmaceutically acceptable salt or solvate thereof according to any one of claims 1-22, or the linker-drug conjugate, or the isomer, mesomer, racemate, or enantiomer thereof, or the mixture thereof, or the pharmaceutically acceptable salt or solvate thereof according to any one of claims 23-32, or the compound, or the isomer, mesomer, racemate, or enantiomer thereof, or the mixture thereof, or the pharmaceutically acceptable salt or solvate thereof according to any one of claims 33-35 in the preparation of a medicament for the treatment or prevention of a cancer or tumor;

preferably, the cancer or tumor is a solid tumor or a hematological tumor;
preferably, the cancer or tumor is selected from adenocarcinoma, ovarian cancer, cervical cancer, uterine cancer, prostate cancer, kidney cancer, urinary tract cancer, bladder cancer, liver cancer, stomach cancer, endometrial cancer, salivary gland cancer, esophageal cancer, lung cancer, colon cancer, rectal cancer, colorectal cancer, bone cancer, skin cancer, thyroid cancer, pancreatic cancer, melanoma, glioma, neuroblastoma, glioblastoma multiforme, sarcoma, lymphoma, leukemia, and hypopharyngeal cancer.

**41.** A method of preventing or treating a cancer or tumor, comprising administering to a subject in need thereof a prophylactic or therapeutically effective amount of the ligand-drug conjugate or the pharmaceutically acceptable salt or solvate thereof according to any one of claims 1-22, or the linker-drug conjugate, or the isomer, mesomer, racemate, or enantiomer thereof, or the mixture thereof, or the pharmaceutically acceptable salt or solvate thereof according to any one of claims 23-32, or the compound, or the isomer, mesomer, racemate, or enantiomer thereof, or the mixture thereof, or the pharmaceutically acceptable salt or solvate thereof according to any one of claims 33-35;

preferably, the cancer or tumor is a solid tumor or a hematological tumor;
preferably, the cancer or tumor is selected from adenocarcinoma, ovarian cancer, cervical cancer, uterine cancer, prostate cancer, kidney cancer, urinary tract cancer, bladder cancer, liver cancer, stomach cancer, endometrial cancer, salivary gland cancer, esophageal cancer, lung cancer, colon cancer, rectal cancer, colorectal cancer, bone cancer, skin cancer, thyroid cancer, pancreatic cancer, melanoma, glioma, neuroblastoma, glioblastoma multiforme, sarcoma, lymphoma, leukemia, and hypopharyngeal cancer.

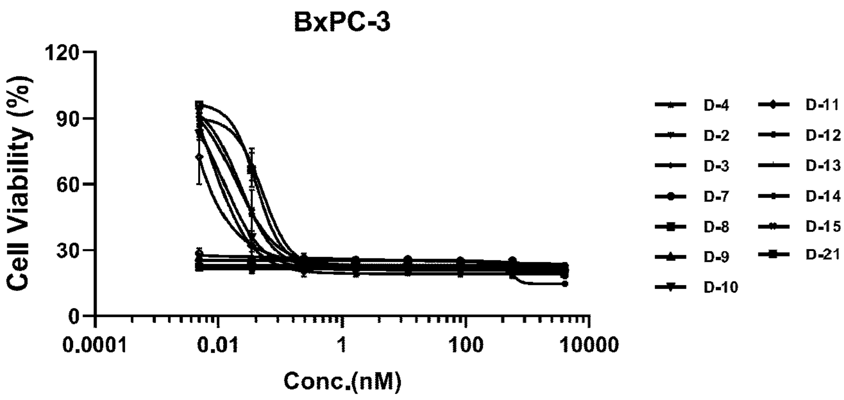


FIG. 1A

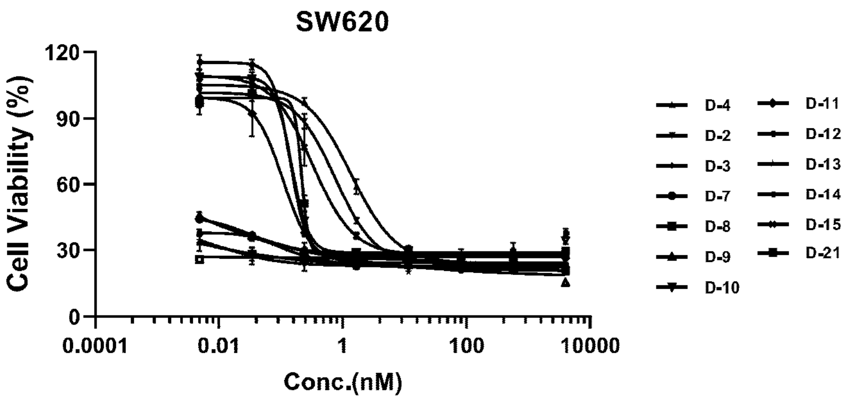


FIG. 1B

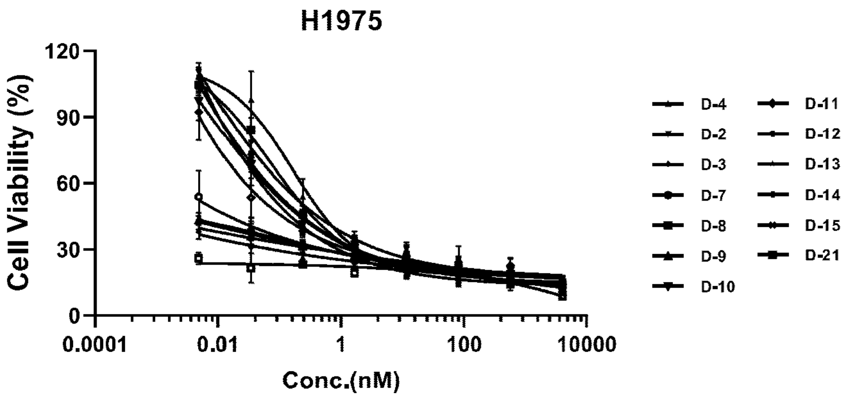


FIG. 1C

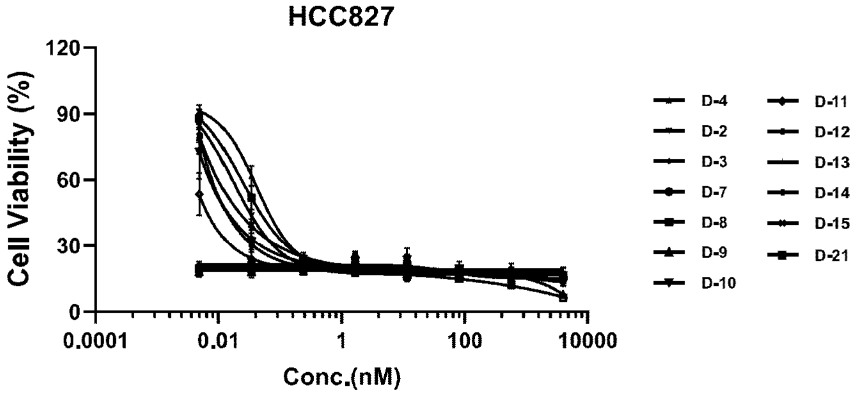


FIG. 1D

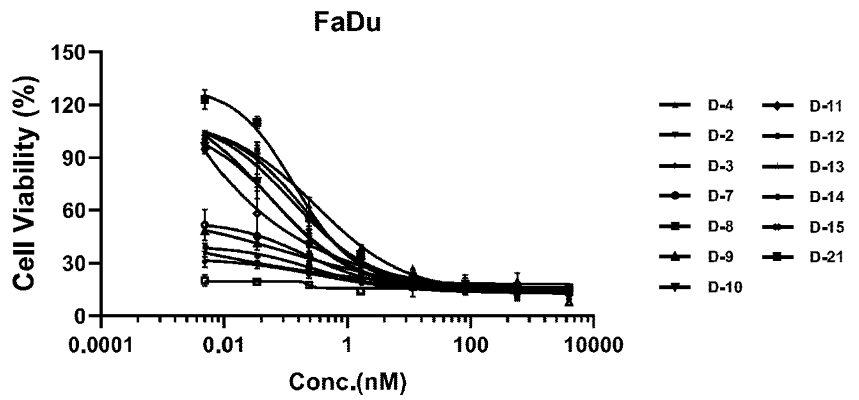


FIG. 1E

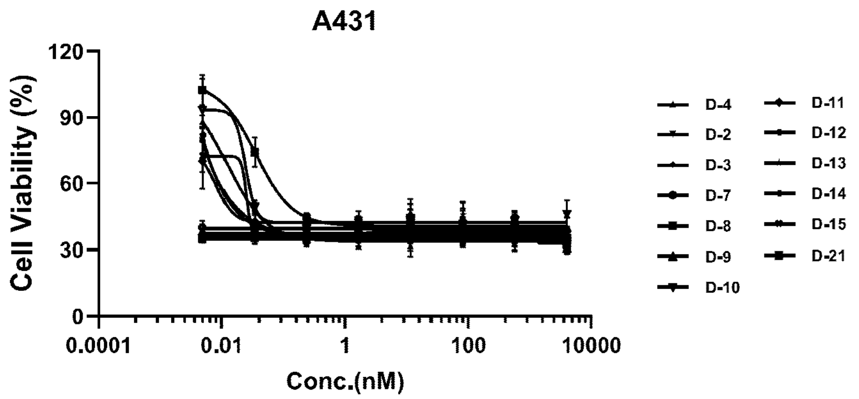


FIG. 1F

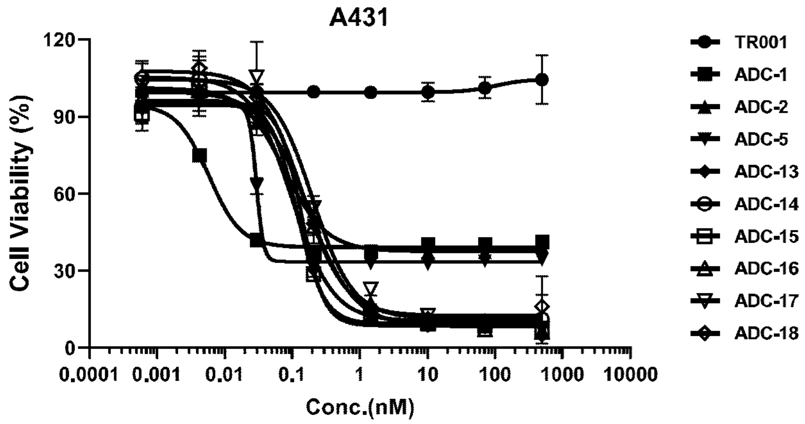


FIG. 2A

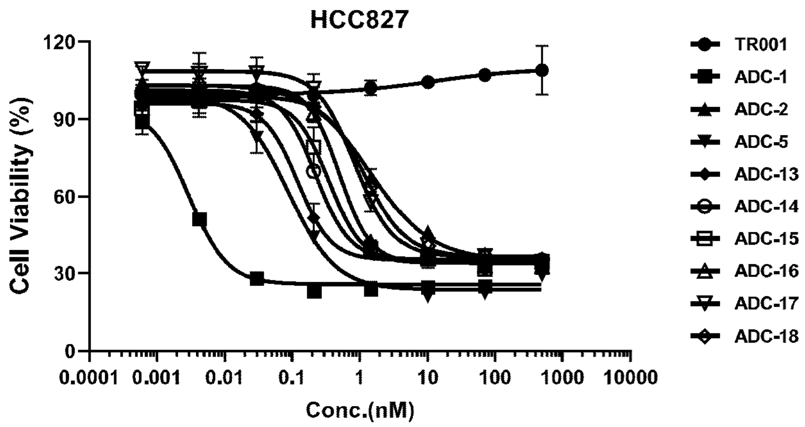


FIG. 2B

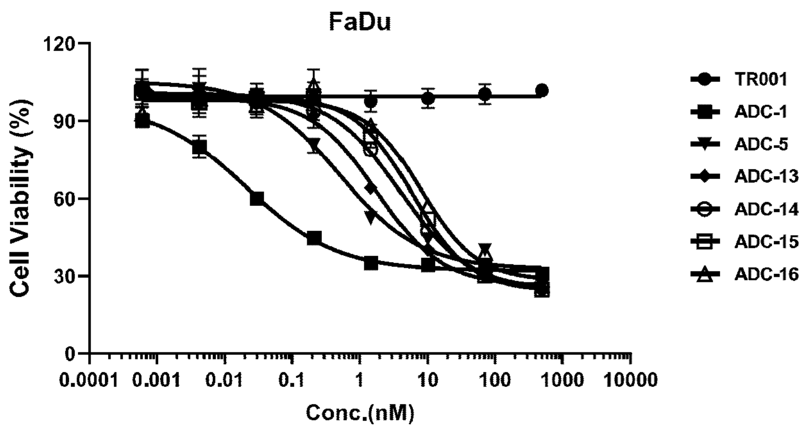


FIG. 2C

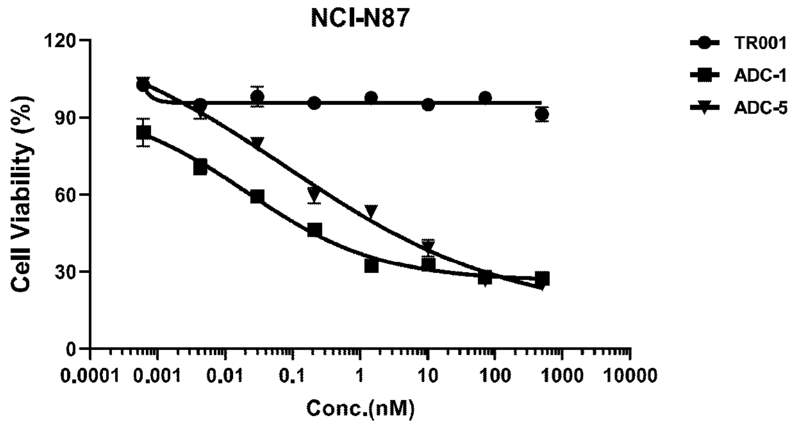


FIG. 2D

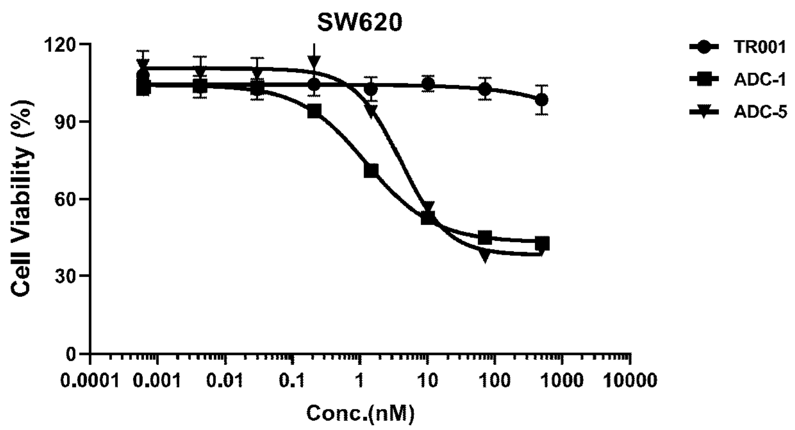


FIG. 2E

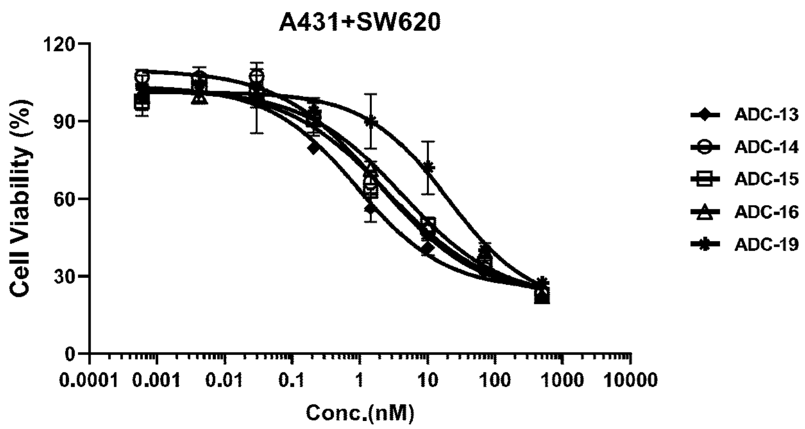


FIG. 3

## INTERNATIONAL SEARCH REPORT

International application No.
**PCT/CN2024/072749**

**A. CLASSIFICATION OF SUBJECT MATTER**

A61K 47/68(2017.01)i； C07K 16/28(2006.01)i； C07D 307/20(2006.01)i； A61P 35/00(2006.01)i

According to International Patent Classification (IPC) or to both national classification and IPC

**B. FIELDS SEARCHED**

Minimum documentation searched (classification system followed by classification symbols)

A61K； C07K； C07D； A61P

Documentation searched other than minimum documentation to the extent that such documents are included in the fields searched

Electronic data base consulted during the international search (name of data base and, where practicable, search terms used)

CNABS, CNTXT, CNKI, VEN, ENTXT, TWTXT, ISI Web of Science, PUBMED, BING, STNext: 艾日布林, 艾立布林, 艾瑞布林, 百利多特, 百利药业, 西雅图免疫公司, 抗体, 偶联, eribulin, E7389, B1939, ER086526, antibody, conjugate, BAILI, SYSTIMMUNE, 253128-41-5, 结构检索, structure search

**C. DOCUMENTS CONSIDERED TO BE RELEVANT**

| Category* | Citation of document, with indication, where appropriate, of the relevant passages | Relevant to claim No. |
|---|---|---|
| X | CN 1312804 A (EISAI CO., LTD.) 12 September 2001 (2001-09-12) description, page 1, paragraph 3 to page 3, paragraph 3, and page 62, last paragraph to page 64, last paragraph | 33-35, 37-41 |
| X | ZHENG. W. et al. "Macrocyclic Ketone Analogues of Halichondrin B" *Bioorganic & Medicinal Chemistry Letters,* Vol. 14, No. (22), 21 September 2004 (2004-09-21), pages 5551-5554, abstract, scheme 4, and table 1 | 33-35, 37-41 |
| X | CN 104334562 A (ALPHORA RES INC.) 04 February 2015 (2015-02-04) claim 33 | 33-34, 37 |
| X | CN 113166096 A (DR. REDDY'S LABORATORIES LTD.) 23 July 2021 (2021-07-23) description, paragraph 0099 | 33-34, 37 |
| A | WO 2021148003 A1 (SHANGHAI SENHUI MEDICINE CO., LTD. et al.) 29 July 2021 (2021-07-29) abstract, and claims 1-46 | 1-41 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **28 March 2024** | **12 April 2024** |
| Name and mailing address of the ISA/CN<br>**China National Intellectual Property Administration (ISA/CN)**<br>**China No. 6, Xitucheng Road, Jimenqiao, Haidian District, Beijing 100088** | Authorized officer<br><br>Telephone No. |

Form PCT/ISA/210 (second sheet) (July 2022)

INTERNATIONAL SEARCH REPORT

International application No.
**PCT/CN2024/072749**

**C. DOCUMENTS CONSIDERED TO BE RELEVANT**

| Category* | Citation of document, with indication, where appropriate, of the relevant passages | Relevant to claim No. |
|---|---|---|
| A | CN 108883198 A (EISAI INC.) 23 November 2018 (2018-11-23) abstract, and claims 1-183 | 1-41 |
| A | CN 114828895 A (EISAI R&D MANAGEMENT CO., LTD.) 29 July 2022 (2022-07-29) abstract, and claims 1-8 | 1-41 |
| A | CHENG. X. et al. "MORAb-202, an Antibody–Drug Conjugate Utilizing Humanized Anti-human FRa Farletuzumab and the Microtubule-targeting Agent Eribulin, has Potent Antitumor Activity" *Molecular Cancer Therapeutics,* Vol. 17, No. (12), 27 September 2018 (2018-09-27), pp. 2665-2675 | 1-41 |

Form PCT/ISA/210 (second sheet) (July 2022)

**INTERNATIONAL SEARCH REPORT**

International application No.
**PCT/CN2024/072749**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed.
   b. ☐ furnished subsequent to the international filing date for the purposes of international search (Rule 13*ter*.1(a)),
      ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**

International application No.
**PCT/CN2024/072749**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **41**
because they relate to subject matter not required to be searched by this Authority, namely:

Claim 41 relates to a method for preventing or treating cancer or tumors, which falls within methods for treatment of diseases as defined in PCT Rule 39.1(iv). However, a search is still performed on the basis of the pharmaceutical use thereof.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (July 2022)

**INTERNATIONAL SEARCH REPORT**
**Information on patent family members**

International application No.
**PCT/CN2024/072749**

| Patent document cited in search report | Publication date (day/month/year) | Patent family member(s) | Publication date (day/month/year) |
|---|---|---|---|
| CN 1312804 A | 12 September 2001 | ATE 502932 T1 | 15 April 2011 |
| | | CA 2632433 A1 | 23 December 1999 |
| | | CA 2632433 C | 07 February 2012 |
| | | LU 91854 I2 | 17 October 2011 |
| | | US 6365759 B1 | 02 April 2002 |
| | | WO 9965894 A1 | 23 December 1999 |
| | | NO 20093217 L | 15 February 2001 |
| | | NO 331183 B1 | 24 October 2011 |
| | | NO 2011026 I1 | 09 January 2012 |
| | | NO 2011026 I2 | 11 June 2012 |
| | | HK 1035534 A1 | 30 November 2001 |
| | | FR 1C0038 I1 | 14 October 2011 |
| | | FR 1C0038 I2 | 11 January 2013 |
| | | EP 2277873 A1 | 26 January 2011 |
| | | EP 2277873 B1 | 30 May 2012 |
| | | NZ 508597 A | 30 January 2004 |
| | | CA 2755266 A1 | 23 December 1999 |
| | | CA 2755266 C | 12 August 2014 |
| | | EP 2272840 A1 | 12 January 2011 |
| | | EP 2272840 B1 | 22 August 2012 |
| | | EP 1087960 A1 | 04 April 2001 |
| | | EP 1087960 A4 | 01 December 2004 |
| | | EP 1087960 B1 | 23 March 2011 |
| | | NO 2022019 I1 | 03 June 2022 |
| | | CY 1111516 T1 | 09 April 2014 |
| | | BR 9911326 A | 03 April 2001 |
| | | BR 9911326 B1 | 06 January 2015 |
| | | BRPI 9911326 B8 | 25 May 2021 |
| | | JP 2002518384 A | 25 June 2002 |
| | | JP 4454151 B2 | 21 April 2010 |
| | | US 6214865 B1 | 10 April 2001 |
| | | US 6469182 B1 | 22 October 2002 |
| | | IL 139960 A0 | 10 February 2002 |
| | | BE 2011C028 I2 | 24 August 2018 |
| | | HUP 0103357 A2 | 28 January 2002 |
| | | HUP 0103357 A3 | 28 October 2002 |
| | | HU 227912 B1 | 29 May 2012 |
| | | AU 762998 C | 05 January 2000 |
| | | AU 4573999 A | 10 July 2003 |
| | | RU 2245335 C2 | 27 January 2005 |
| | | NO 20006316 D0 | 12 December 2000 |
| | | NO 20006316 L | 15 February 2001 |
| | | NO 328280 B1 | 25 January 2010 |
| | | EP 2272839 A1 | 12 January 2011 |
| | | EP 2272839 B1 | 22 August 2012 |
| | | KR 20010083050 A | 31 August 2001 |
| | | KR 100798600 B1 | 28 January 2008 |
| | | DK 1087960 T3 | 14 June 2011 |
| | | CA 2335300 A1 | 23 December 1999 |
| | | CA 2335300 C | 07 October 2008 |

Form PCT/ISA/210 (patent family annex) (July 2022)

**INTERNATIONAL SEARCH REPORT**
**Information on patent family members**

International application No.
**PCT/CN2024/072749**

| Patent document cited in search report | | | Publication date (day/month/year) | Patent family member(s) | | | Publication date (day/month/year) |
|---|---|---|---|---|---|---|---|
| | | | | PT | 1087960 | E | 17 June 2011 |
| | | | | NO | 2011018 | I1 | 26 September 2011 |
| | | | | ZA | 200007159 | B | 04 December 2001 |
| | | | | DE | 69943296 | D1 | 05 May 2011 |
| | | | | DE | 122011100031 | I1 | 15 December 2011 |
| | | | | US | 2011172446 | A1 | 14 July 2011 |
| | | | | US | 8148554 | B2 | 03 April 2012 |
| CN | 104334562 | A | 04 February 2015 | IN | 2106MUMN2014 | A | 11 September 2015 |
| | | | | EP | 2831082 | A1 | 04 February 2015 |
| | | | | EP | 2831082 | A4 | 06 January 2016 |
| | | | | EP | 2831082 | B1 | 20 February 2019 |
| | | | | US | 2015065733 | A1 | 05 March 2015 |
| | | | | US | 9278979 | B2 | 08 March 2016 |
| | | | | AU | 2013239290 | A1 | 30 October 2014 |
| | | | | AU | 2013239290 | B2 | 03 August 2017 |
| | | | | WO | 2013142999 | A1 | 03 October 2013 |
| | | | | JP | 2015512897 | A | 30 April 2015 |
| | | | | JP | 6531911 | B2 | 19 June 2019 |
| | | | | US | 2016152631 | A1 | 02 June 2016 |
| | | | | US | 9695187 | B2 | 04 July 2017 |
| | | | | CA | 2868627 | A1 | 03 October 2013 |
| | | | | CA | 2868627 | C | 16 February 2021 |
| CN | 113166096 | A | 23 July 2021 | US | 2021340156 | A1 | 04 November 2021 |
| | | | | EP | 3864011 | A1 | 18 August 2021 |
| | | | | EP | 3864011 | A4 | 29 June 2022 |
| | | | | WO | 2020075027 | A1 | 16 April 2020 |
| WO | 2021148003 | A1 | 29 July 2021 | AU | 2021210079 | A1 | 25 August 2022 |
| | | | | BR | 112022014398 | A2 | 13 September 2022 |
| | | | | MX | 2022009044 | A | 11 August 2022 |
| | | | | EP | 4094779 | A1 | 30 November 2022 |
| | | | | EP | 4094779 | A4 | 22 November 2023 |
| | | | | TW | 202140078 | A | 01 November 2021 |
| | | | | JP | 2023511163 | A | 16 March 2023 |
| | | | | KR | 20220130160 | A | 26 September 2022 |
| | | | | CA | 3168654 | A1 | 29 July 2021 |
| | | | | US | 2023144203 | A1 | 11 May 2023 |
| CN | 108883198 | A | 23 November 2018 | UA | 125024 | C2 | 29 December 2021 |
| | | | | JOP | 20210074 | A1 | 30 January 2023 |
| | | | | TW | 202241524 | A | 01 November 2022 |
| | | | | TWI | 825834 | B | 11 December 2023 |
| | | | | JP | 2020019787 | A | 06 February 2020 |
| | | | | JP | 6870051 | B2 | 12 May 2021 |
| | | | | CA | 3013791 | A1 | 08 September 2017 |
| | | | | US | 2020297860 | A1 | 24 September 2020 |
| | | | | EP | 3824909 | A1 | 26 May 2021 |
| | | | | US | 2017252458 | A1 | 07 September 2017 |
| | | | | US | 10548986 | B2 | 04 February 2020 |
| | | | | SG | 10202007520 | WA | 29 September 2020 |
| | | | | AU | 2023285804 | A1 | 18 January 2024 |
| | | | | AR | 121302 | A2 | 04 May 2022 |

Form PCT/ISA/210 (patent family annex) (July 2022)

**INTERNATIONAL SEARCH REPORT**
**Information on patent family members**

International application No.
**PCT/CN2024/072749**

| Patent document cited in search report | Publication date (day/month/year) | Patent family member(s) | | | Publication date (day/month/year) |
|---|---|---|---|---|---|
| | | LT | 3423105 | T | 10 September 2021 |
| | | IL | 261428 | A | 31 October 2018 |
| | | JP | 2023082096 | A | 13 June 2023 |
| | | SG | 11201806515 | RA | 27 September 2018 |
| | | CO | 2018008667 | A2 | 31 August 2018 |
| | | BR | 112018067379 | A2 | 15 January 2019 |
| | | RS | 62108 | B1 | 31 August 2021 |
| | | KR | 20220101204 | A | 19 July 2022 |
| | | MX | 2023003809 | A | 12 April 2023 |
| | | PE | 20231049 | A1 | 11 July 2023 |
| | | KR | 20180115330 | A | 22 October 2018 |
| | | KR | 102445255 | B1 | 22 September 2022 |
| | | HUE | 054726 | T2 | 28 September 2021 |
| | | CL | 2021000048 | A1 | 28 May 2021 |
| | | TW | 201800110 | A | 01 January 2018 |
| | | TWI | 772288 | B | 01 August 2022 |
| | | JP | 2020128413 | A | 27 August 2020 |
| | | JP | 2019516664 | A | 20 June 2019 |
| | | JP | 6599019 | B2 | 30 October 2019 |
| | | KR | 20240007722 | A | 16 January 2024 |
| | | MY | 189113 | A | 26 January 2022 |
| | | PL | 3423105 | T3 | 25 October 2021 |
| | | MD | 3423105 | T2 | 31 October 2021 |
| | | ES | 2880402 | T3 | 24 November 2021 |
| | | HRP | 20211125 | T1 | 15 October 2021 |
| | | JOP | 20210073 | A1 | 30 January 2023 |
| | | EP | 3423105 | A1 | 09 January 2019 |
| | | EP | 3423105 | B1 | 05 May 2021 |
| | | JOP | 20170053 | B1 | 17 August 2021 |
| | | IL | 292946 | A | 01 July 2022 |
| | | AR | 107787 | A1 | 06 June 2018 |
| | | WO | 2017151979 | A1 | 08 September 2017 |
| | | US | 2018193478 | A1 | 12 July 2018 |
| | | US | 10322192 | B2 | 18 June 2019 |
| | | AU | 2017225982 | A1 | 16 August 2018 |
| | | AU | 2017225982 | B2 | 05 October 2023 |
| | | PE | 20181953 | A1 | 17 December 2018 |
| | | SI | 3423105 | T1 | 31 December 2021 |
| | | PH | 12018501847 | A1 | 15 May 2019 |
| | | US | 2023398228 | A1 | 14 December 2023 |
| | | AR | 121301 | A2 | 04 May 2022 |
| | | CY | 1124628 | T1 | 22 July 2022 |
| | | DK | 3423105 | T3 | 26 July 2021 |
| | | MX | 2018010562 | A | 20 February 2019 |
| | | PE | 20231050 | A1 | 11 July 2023 |
| | | KR | 20220101203 | A | 19 July 2022 |
| | | KR | 102456433 | B1 | 19 October 2022 |
| | | CL | 2018002456 | A1 | 21 December 2018 |
| | | RU | 2018134331 | A | 02 April 2020 |
| | | RU | 2018134331 | A3 | 14 August 2020 |

Form PCT/ISA/210 (patent family annex) (July 2022)

**INTERNATIONAL SEARCH REPORT**
**Information on patent family members**

International application No.
**PCT/CN2024/072749**

| Patent document cited in search report | Publication date (day/month/year) | Patent family member(s) | Publication date (day/month/year) |
|---|---|---|---|
| | | RU 2754369 C2 | 01 September 2021 |
| | | PT 3423105 T | 19 July 2021 |
| | | MX 2023003808 A | 12 April 2023 |
| | | RU 2021125492 A | 05 April 2022 |
| | | MX 2023003806 A | 12 April 2023 |
| | | CL 2021000049 A1 | 28 May 2021 |
| | | MA 45280 A | 09 January 2019 |
| | | MA 45280 B1 | 31 August 2021 |
| | | JP 2021185176 A | 09 December 2021 |
| | | JP 7254861 B2 | 10 April 2023 |
| CN 114828895 A | 29 July 2022 | WO 2021132166 A1 | 01 July 2021 |
| | | BR 112022012546 A2 | 06 September 2022 |
| | | IL 293040 A | 01 July 2022 |
| | | CA 3164797 A1 | 01 July 2021 |
| | | KR 20220120586 A | 30 August 2022 |
| | | MX 2022007798 A | 19 July 2022 |
| | | EP 4088739 A1 | 16 November 2022 |
| | | AU 2020414996 A1 | 14 July 2022 |
| | | JPWO 2021132166 A1 | 01 July 2021 |
| | | US 2023074385 A1 | 09 March 2023 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- CN 202310082754
- CN 202310563948
- CN 2024100454262
- CN 111051330 A
- CN 113827736 A
- CN 111686259 A
- WO 2022171101 A
- CN L08883198 A
- CN 108883198 A
- WO 0152900 A2

### Non-patent literature cited in the description

- *J. boil. Chem.*, 1968, vol. 243, 3558